# EUROPEAN PATENT SPECIFICATION

(11) **EP 3 408 407 B1**
(45) Date of publication and mention of the grant of the patent: **06.01.2021**
(21) Application number: 17702586.3
(22) Date of filing: 27.01.2017
(51) Int. Cl.: C12Q 1/6886

(54) **METHODS FOR DETECTING CPG METHYLATION OF TUMOR-DERIVED DNA IN BLOOD SAMPLES**
VERFAHREN FÜR DEN NACHWEIS VON CPG-METHYLIERUNG VON TUMORABGELEITETEM DNA IN BLUTPROBEN
PROCÉDÉS DE DÉTECTION DE LA MÉTHYLATION CPG DE L'ADN DÉRIVÉ DE TUMEURS DANS DES ÉCHANTILLONS DE SANG

(30) Priority: 29.01.2016 EP 16153452
(43) Date of publication of application: 05.12.2018
(73) Proprietor: Epigenomics AG, 10829 Berlin (DE)
(72) Inventor: LEWIN, Jörn, 10115 Berlin (DE); KOTTWITZ, Denise, 12109 Berlin (DE); ESCHE, Selina, 14482 Potsdam (DE)
(74) Representative: Zwicker, Jörk
(86) International application number: PCT/EP2017/051710
(87) International publication number: WO 2017/129716

(56) References cited:
- WO-A1-00/47600
- WO-A1-2013/177265
- WO-A2-2014/036314
- WO-A2-2015/187823
- REJANE HUGHES CARVALHO ET AL: "Genome-wide DNA methylation profiling of non-small cell lung carcinomas", EPIGENETICS & CHROMATIN, BIOMED CENTRAL LTD, LONDON, UK, vol. 5, no. 1, 22 June 2012 (2012-06-22), page 9, XP021127600, ISSN: 1756-8935, DOI: 10.1186/1756-8935-5-9
- A. VINCENT ET AL: "Genome-Wide Analysis of Promoter Methylation Associated with Gene Expression Profile in Pancreatic Adenocarcinoma", CLINICAL CANCER RESEARCH, vol. 17, no. 13, 24 May 2011 (2011-05-24), pages 4341-4354, XP055092899, ISSN: 1078-0432, DOI: 10.1158/1078-0432.CCR-10-3431
- ILLUMINA: "Infinium HumanMethylation27, RevB BeadChip Kits", INTERNET CITATION, 8 February 2011 (2011-02-08), pages 1-2, XP002621307, Retrieved from the Internet: URL:http://www.illumina.com/products/infin ium_humanmethylation27_beadchip_kits.ilmn [retrieved on 2011-02-08]
- HONG-PAN TIAN ET AL: "DNA Methylation Affects the SP1-regulated Transcription of FOXF2 in Breast Cancer Cells", JOURNAL OF BIOLOGICAL CHEMISTRY, vol. 290, no. 31, 12 June 2015 (2015-06-12), pages 19173-19183, XP055282490, US ISSN: 0021-9258, DOI: 10.1074/jbc.M114.636126

## Description

### FIELD OF THE INVENTION

The present invention relates to the field of pharmacogenomics and in particular to detecting the presence or absence of methylated ANKRD13B and/or FOXF2 DNA derived from a tumor in blood or blood-derived samples or in other body fluids that contain DNA released from a tumor. This detection is useful for a minimally invasive diagnosis of cancers and the invention provides methods and oligonucleotides suitable for this purpose.

### BACKGROUND OF THE INVENTION

More than 65 years ago Mandel and Metais described for the first time their observation of the presence of extracellular nucleic acids in humans (Mandel P, Metais P. Les acides nucleiques du plasma sanguin chez l'homme. C.R.Acad.Sci.Paris 142, 241-243. 1948) and more than four decades later it could be clearly demonstrated that tumor-associated genetic alterations can be found in cell-free nucleic acids isolated from plasma, serum and other body fluids (Fleischhacker M, Schmidt B. (2007) Circulating nucleic acids (CNAs) and cancer--a survey. Biochim Biophys Acta 1775: 181-232; Jung K, Fleischhacker M, Rabien A. (2010) Cell-free DNA in the blood as a solid tumor biomarker-a critical appraisal of the literature. Clin Chim Acta 411: 1611-1624). This includes epigenetic alterations observed in different forms of malignant tumors. A hallmark of mammalian chromatin is DNA methylation and it is known that cytosine methylation in the context of a CpG dinucleotide plays a role in the regulation of development and is important in basic biological processes like embryogenesis and cell differentiation (Smith ZD, Meissner A. (2013) DNA methylation: roles in mammalian development. Nat Rev Genet 14: 204-220; Gibney ER, Nolan CM. (2010) Epigenetics and gene expression. Heredity (Edinb) 105: 4-13). As such, methylation not only regulates gene transcription, but also plays a role in maintaining genome stability, imprinting and X-chromosome inactivation. Epigenetic alterations in oncogenes and tumor suppressor genes are of key importance in the development of cancer (Suva ML, Riggi N, Bernstein BE. (2013) Epigenetic reprogramming in cancer. Science 339: 1567-1570). DNA methylation patterns are largely modified in cancer cells and can therefore be used to distinguish cancer cells from normal tissues. As such, DNA methylation patterns are being used to diagnose all sorts of cancers. A relatively recent concept is the use of free circulating tumor DNA that is released from the tumor for example into the blood for methylation analysis as an indicator for tumor load in the body of the patient. This ability to isolate and to characterize extracellular nucleic acids from tumor patients with very sensitive and highly specific methods led to the term "liquid biopsy". As a result, physicians no longer solely depend on the examination of tissue biopsies and body scans.

The less advanced the cancer is, the better the treatment options and the chances of curing the patient are. Thus, it is highly desirable to diagnose a cancer as early as possible. However, a less advanced cancer, which means smaller tumor size and less cancer cells, releases less free circulating tumor DNA. This is exacerbated by the fact that the half-life of extracellular nucleic acids is rather short, for example less than six hours in plasma (Rago C, Huso DL, Diehl F, Karim B, Liu G, et al. (2007) Serial assessment of human tumor burdens in mice by the analysis of circulating DNA. Cancer Res 67: 9364-9370). A further difficulty is that small amounts of methylated tumor marker DNA have to be detected at high backgrounds of non-tumor DNA, which greatly challenges the sensitivity of the detection methods. Very good results have been achieved using technologies based on a selective amplification of methylated tumor DNA after bisulfite conversion, like methylation-specific PCR (MSP) and especially the HeavyMethyl™ (HM) technology (Cottrell et al., A real-time PCR assay for DNA-methylation using methylation-specific blockers. Nucleic Acids Res. 2004 Jan 13;32(1):e10).

Another difficulty is the presence of methylated non-tumor DNA in the background DNA of liquid biopsies that often occurs, depending on marker and tumor to be detected. Unless this background is very low or most desirably non-existent, it can be difficult to distinguish between healthy subjects and those having cancer, because the amount of methylated tumor DNA in liquid biopsies is usually very low. So far this second difficulty has been accepted as given by marker and tumor and addressed mostly by increasing specificity, which comes with a loss of sensitivity of detection. The present inventors have, however, focused on the methylation markers in liquid biopsies of healthy subjects instead of primarily cancer patients to increase sensitivity and specificity of cancer detection. To this end, they have, rather than screening for markers methylated in samples from tumor patients, screened for potential cancer markers which are not methylated in liquid biopsies of healthy subjects, to achieve a minimal or non-existent amount of methylated non-tumor DNA in the samples. The inventors have found that, surprisingly, methylated genomic DNA of the markers ANKRD13B and FOXF2 is not or virutally not found in blood or blood-derived samples of healthy subjects, and that these markers show a clear methylation signal in cancer tissues. This property is very rare for a marker and allows for an unusually sensitive and specific detection, which could not have been expected from Hughes Carvalho et al. (Epigenetics & Chromatin 2012, 5:9) or Vincent et al. (Clin Cancer Res; 17(13) July 1, 2011), which disclose the differential methylation of ANKRD13B in lung cancer cells and of FOXF2 in pancres cancer cells. Also, the inventors identified a variety of cancers which can be dectected with these markers. Methods for detecting cancer which are adapted according to these findings will allow for an improved care for cancer patients by providing the possibility of the most promising time window for treatment.

### SUMMARY OF THE INVENTION

In a first aspect, the present invention relates to a method for detecting DNA methylation within genomic DNA having a sequence comprised in SEQ ID NO: 11 and/or 16 and/or within genomic DNA having a sequence comprised in SEQ ID NO: 36 from a sample comprising cell-free DNA from blood or a sample derived therefrom of a subject, wherein the subject has an increased risk for or is suspected of having a cancer selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

In a second aspect, the present invention relates to a method for detecting the presence or absence of cancer or a risk thereof in a subject, comprising detecting DNA methylation within genomic DNA having a sequence comprised in SEQ ID NO: 11 and/or 16, and/or within genomic DNA having a sequence comprised in SEQ ID NO: 36 from a sample comprising cell-free DNA from blood or a sample derived therefrom of a subject, wherein the cancer is selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

In a third aspect, the present invention relates to the use of an oligonucleotide selected from the group consisting of a primer, blocker or probe, which has a sequence that is substantially identical or complementary to a stretch of contiguous nucleotides of SEQ ID NOs 12-15 or 17-20, or which has a sequence that is substantially identical or complementary to a stretch of contiguous nucleotides of SEQ ID NOs 37-40, in a method for detecting DNA methylation for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer for SEQ ID NOs 12-15 and 17-20 and/or from the group consisting of lung, colon, breast, stomach and esophagus cancer for SEQ ID NOs 37-40.

In a fourth aspect, the present invention relates to the use of a kit comprising at least a first and a second oligonucleotide, wherein the first and the second oligonucleotides are oligonucleotides of aspect 3 in a method for detecting DNA methylation for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer for SEQ ID NOs 12-15 or 17-20 and/or from the group consisting of lung, colon, breast, stomach and esophagus cancer for SEQ ID NOs 37-40.

In a fifth aspect, the present invention relates to the use of the method of the first or the second aspect, of the oligonucleotide of the third aspect or of the kit of the fourth aspect for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

### LEGENDS TO THE FIGURES

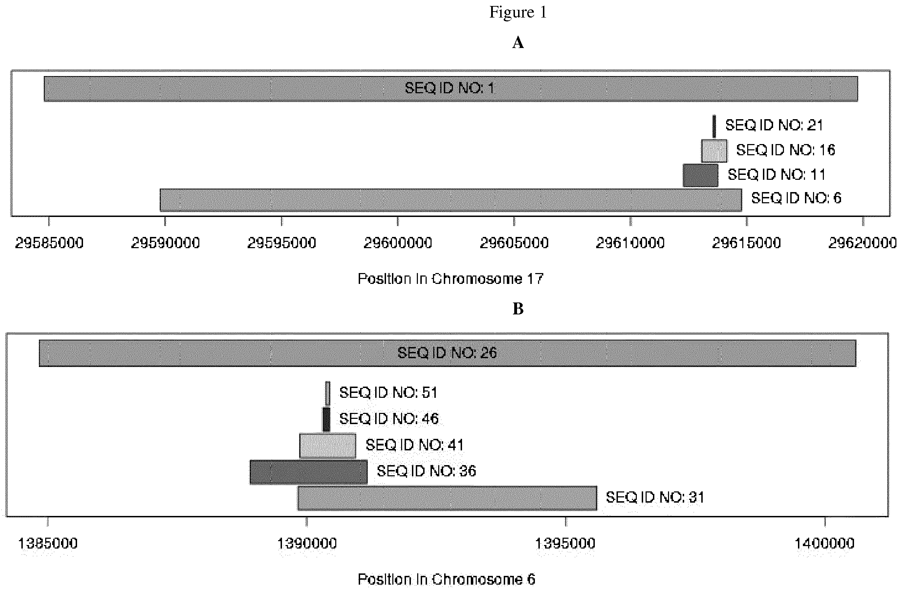

**Figure 1****:** Map of preferred target regions. **A**: ANKRD13B, **B**: FOXF2. See Table 1 for an explanation of the SEQ ID NOs.

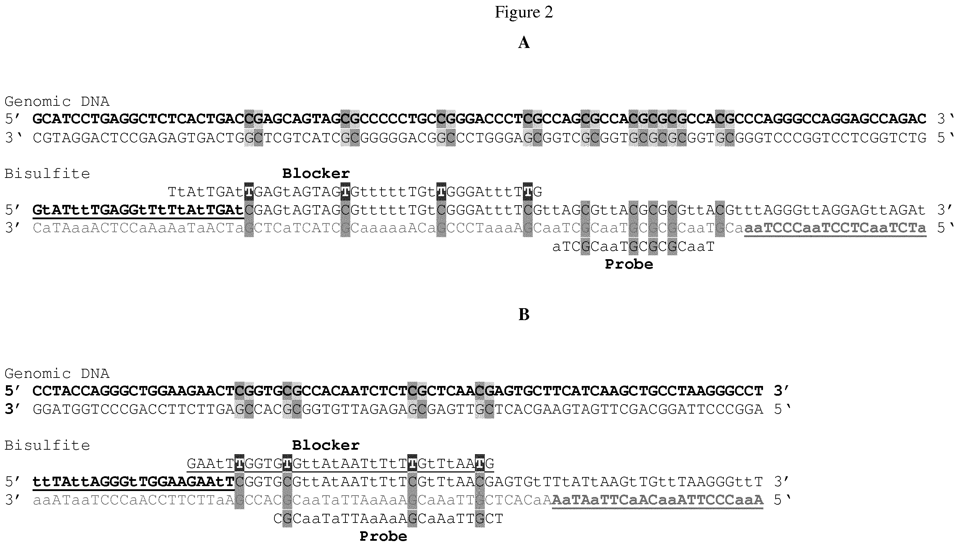

**Figure 2****:** Assay examples. **A**: ANKRD13B, **B**: FOXF2. DNA sequences are in the sense orientation of the GRCh38 genome build. Assays and their elements are aligned with the genomic reference sequence (genomic, both strands shown) on the top. Below the genomic sequence the bisulfite and PCR converted sense strand of the genomic DNA is shown, wherein C in non-CpG context is converted to T (bisulfite converts to U, which pairs with A and is converted to T by amplification). The bisulfite strand is the initial template for amplification. The reverse complement strand shown in grey below is unavailable until it is synthesized by amplification. All DNA single strand sequences are annotated with 3' and 5' on both ends according to their orientation. Primer sequences are shown by underlined sequences. Other oligomers like blockers and probes are shown in alignment with the assay and genomic sequences. Cytosine in CpG context, their counterparts on the opposite strand and/or any base in probe or blocker covering them indirectly or directly are shown with grey background. Capital letters are used in different context: For all genomic reference sequences and for the bases in other strands that are not converted and are identical to the genomic sequence. Lower case letters show converted bases.

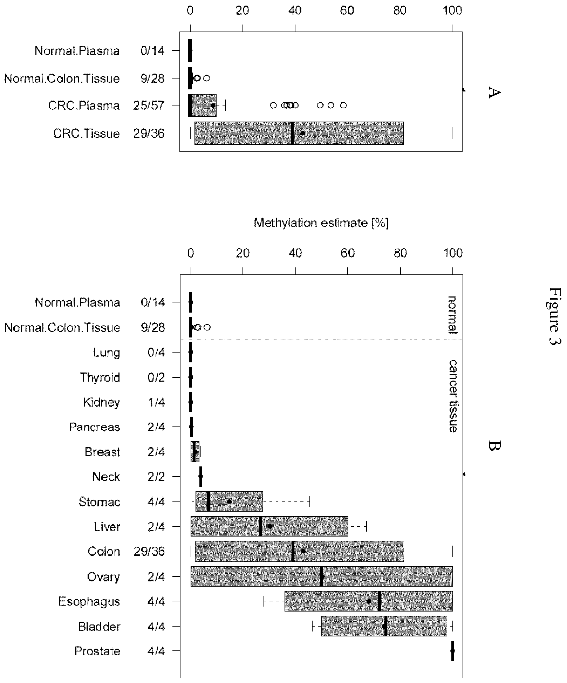

**Figure 3****:** Boxplots of DNA methylation measured with the ANKRD13B assay on different kinds of samples. Numbers below the x-axis provide information how many PCR reactions were done and how many of them lead to any signal (>0%), which is important information for a qualitative analysis of the data. Mean methylation is shown as dark points. Boxplot A provides example information for the diagnostic value in colorectal cancer (CRC): The marker showed no signal in plasma from normal patients, very few and low signals in normal colon tissue but high signals in most colon cancer tissue and was positive in 44% of the PCR reactions on blood plasma from cancer patients. Boxplot **B** shows two normal and 13 cancer tissue types. It provides evidence that the ANKRD13B marker is a cancer marker that is usable as a diagnostic and/or prognostic marker in stomach, liver, colon, ovary, esophagus, bladder and prostate cancer.

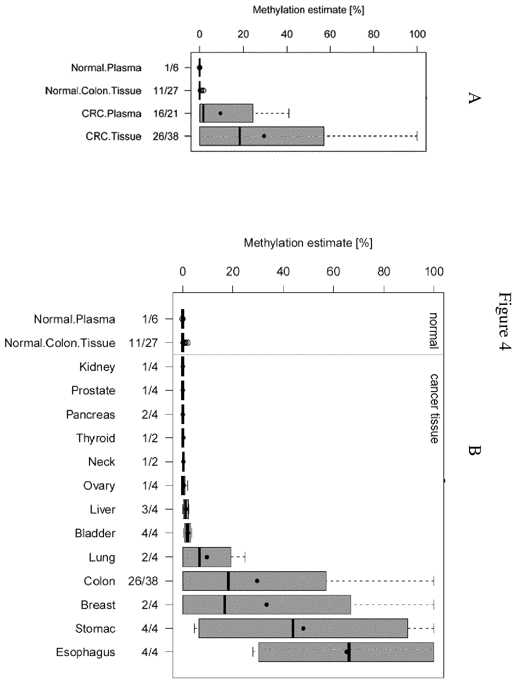

**Figure 4****:** Boxplots of DNA methylation measured with the FOXF2 assay on different kinds of samples. Mean methylation is shown as dark points. Numbers below the x-axis provide information how many PCR reactions were done and how many of them lead to any signal (>0%), which is important information for a qualitative analysis of the data. Mean methylation is shown as dark points. Boxplot **A** provides example information for the diagnostic value in colorectal cancer (CRC): The marker showed virtually no signal in plasma from normal patients, very few and low signals in normal colon tissue but high signals in most colon cancer tissue and was positive in 76% of the PCR reactions on blood plasma from cancer patients. Boxplot **B** shows two normal and 13 cancer tissue types. It provides evidence that the FOXF2 marker is a cancer marker that is usable as a diagnostic and/or prognostic marker in lung, colon, breast, stomach and esophagus cancer.

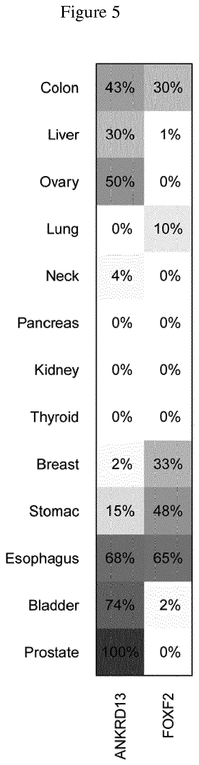

**Figure 5****:** Average methylation [%] of the two cancer markers ANKRD13B and FOXF2 measured over tissue samples from different cancer types. The clustering of the two markers demonstrates in how far a paneling can be of use to diagnose cancer and specify the organ of origin for a series of different cancers in parallel.

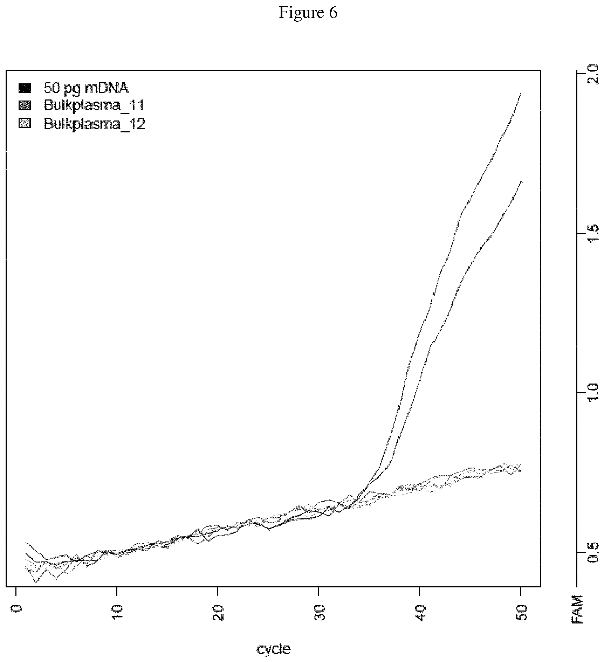

**Figure 6**: ANKRD13B assay realtime PCR amplification curves for bulk plasma (pooled plasma) from healthy individuals in comparison to a sample with very low amount of methylated DNA (50 pg).

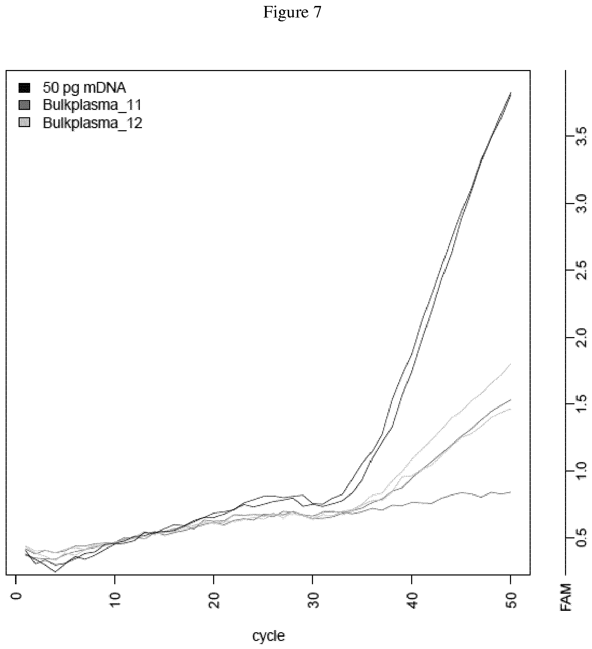

**Figure 7**: FOXF2 assay realtime PCR amplification curves for bulk plasma from healthy individuals in comparison to a sample with very low amount of methylated DNA (50 pg).

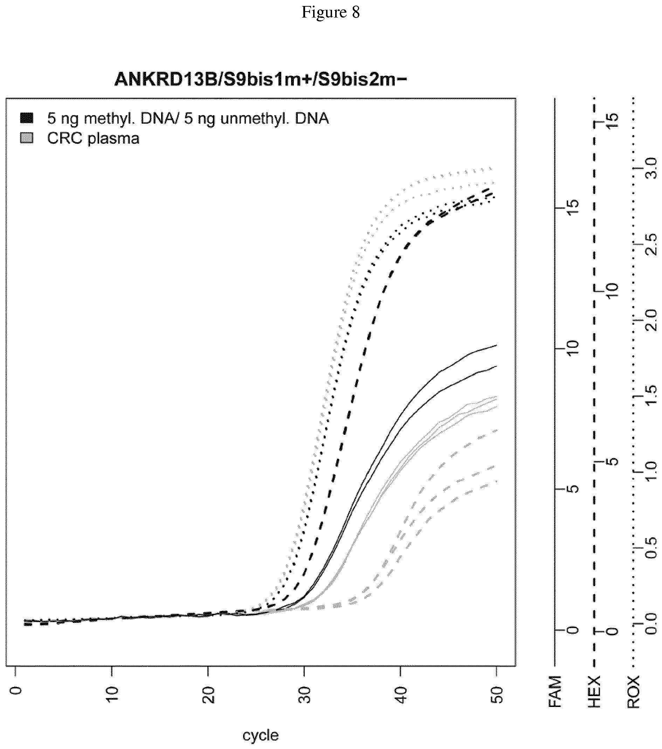

**Figure 8****:** ANKRD13B triplex assay amplification curves for CRC plasma in grey (and a calibrator in black). The dotted curves show the unmethylated background DNA as determined by unmethylated Septin9, the dashed curves show CRC tumor DNA as determined by methylated Septin9. The straight lines show methylation measured for ANKRD13B.

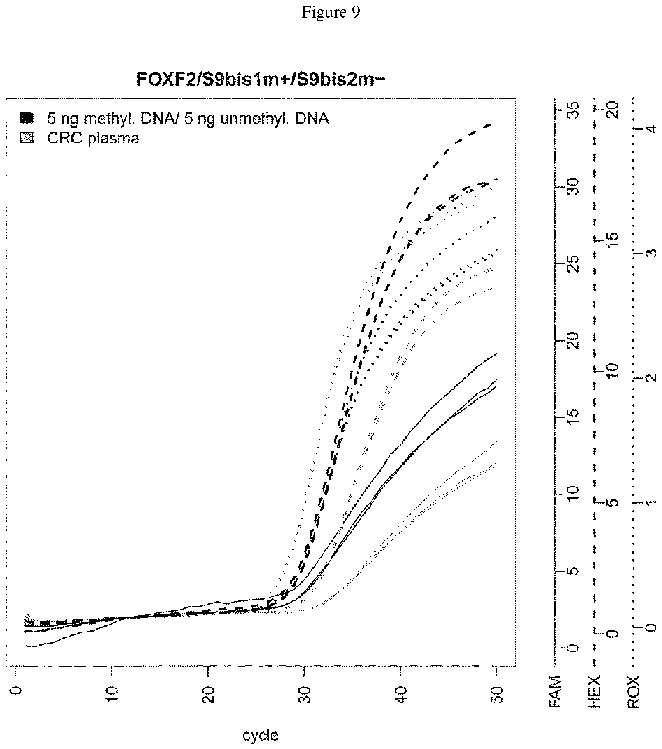

**Figure 9****:** FOXF2 triplex assay amplification curves for CRC plasma in grey (and a calibrator in black). The dotted curves show the unmethylated background DNA as determined by unmethylated Septin9, the dashed curves show CRC tumor DNA as determined by methylated Septin9. The straight lines show methylation measured for FOXF2.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. As used in this specification and the appended claims, the singular forms "a", "an", and "the" include plural referents, unless the content clearly dictates otherwise.

### Aspects of the invention and particular embodiments thereof

In a first aspect, the present invention relates to a method for detecting DNA methylation within genomic DNA having a sequence comprised in SEQ ID NO: 11 and/or 16 and/or within genomic DNA having a sequence comprised in SEQ ID NO: 36 from a sample comprising cell-free DNA from blood or a sample derived therefrom of a subject, wherein the subject has an increased risk for or is suspected of having a cancer selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

In a preferred embodiment, the cancer selected from the group consisting of stomach, liver, colon, ovary, esophagus, bladder and prostate cancer and/or from the group consisting of lung, colon, breast, stomach and esophagus cancer is as defined by the American Cancer Society. The cancer may be of any subtype and stage as defined below.

Specifically, it is preferred that when DNA methylation with respect to SEQ ID NO: 11 and/or 16 is detected for diagnosing or screening for cancer, one or more of the following risk factors can be attributed to the subject:
- Stomach cancer: One or more risk factors selected from the group consisting of male gender, age of 50 or older, ethnicity (in particular Hispanic American, African American or Asian/Pacific Islander), Helicobacter pylori infection, stomach lymphoma, diet rich of smoked foods, salted fish and meat and/or pickled vegetables, tobacco consumption (in particular smoking), being overweight or obese, previous stomach surgery, pernicious anemia, Menetrier disease (hypertrophic gastropathy), type A blood, inherited cancer syndromes or predispositions (including hereditary diffuse gastric cancer, hereditary non-polyposis colorectal cancer (HNPCC), familial adenomatous polyposis (FAP), BRCA1 and/or BRCA2 mutations, Li-Fraumeni syndrome, Peutz-Jeghers syndrome (PJS), family history of stomach cancer (first degree relative, i.e. parents, siblings or children), non-cancerous stomach polyps, Epstein-Barr virus infection, occupation (in particular workers in the coal, metal and rubber industries), and common variable immune deficiency (CVID).
- Liver cancer: One or more risk factors selected from the group consisting of age of 45 or older (in particular 50 or older), male gender, ethnicity (in particular Asian/Pacific Islander), chronic viral hepatitis, cirrhosis, heavy alcohol use (more than 3 or 4 alcohol units a day for men, or more than 2 or 3 alcohol units a day for women; an alcohol unit is defined as 10 ml (8 g) of pure alcohol), obesity, type 2 diabetes, inherited metabolic diseases (in particular those leading to cirrhosis, such as hereditary hemochromatosis, tyrosinemia, alpha1-antitrypsin deficiency, porphyria cutanea tarda, glycogen storage diseases, Wilson disease), exposure to a carcinogen (in particular aflatoxins, vinyl chloride and thorium dioxide (Thorotrast), anabolic steroids, arsenic, infection with parasites (in particular those causing schistosomiasis), and tobacco consumption (in particular smoking).
- Colon cancer: One or more risk factors selected from the group consisting of age of 50 or older, a personal history of colorectal polyps or colorectal cancer (wherein the cancer was completely removed), a personal history of inflammatory bowel disease, family history of colorectal cancer or adenomatous polyps (first degree relative), having an inherited syndrome (such as familial adenomatous polyposis, Lynch syndrome (hereditary non-polyposis colon cancer, or HNPCC), Turcot syndrome, Peutz-Jeghers syndrome, or MUTYH-associated polyposis), ethnicity (in particular African American or Jew of Eastern European descent), having type 2 diabetes, being overweight or obese, physical inactivity, tobacco consumption (in particular smoking), and heavy alcohol use (as defined above).
- Ovary cancer: One or more risk factors selected from the group consisting of age of 40 or older (in particular of 63 and older and more particular after menopause), female gender, obesity, first full-term pregnancy after age 35 or no pregancy, no use of birth control pills (in particular never or not in the last 3, 4 or 6 months), exposure to fertility drugs (in particular clomiphene citrate), exposure to androgens, estrogen therapy and/or hormone therapy, family history of ovarian cancer, breast cancer, or colorectal cancer (in particular in first degree relatives), having an inherited cancer syndrome or predisposition (such as hereditary breast and ovarian cancer syndrome, PTEN tumor hamartoma syndrome, hereditary nonpolyposis colon cancer, Peutz-Jeghers syndrome, or MUTYH-associated polyposis), personal history of breast cancer, use of talcum powder applied directly to the genital area, high fat diet, use of analgesics, and tobacco consumption (in particular smoking).
- Esophagus cancer: One or more risk factors selected from the group consisting of male gender, age of 50 or older (in particular 55 or older), gastroesophageal reflux disease, Barrett's esophagus, tobacco consumption (in particular smoking), heavy alcohol use (as defined above), being overweight or obese, diet rich in processed meats, drinking hot liquids regularly, achalasia, tylosis, Plummer-Vinson syndrome, occupation (in particular work exposure to chemical fumes, e.g. solvents used for dry cleaning), past injury to the esophagus, personal history of other cancers (such as lung cancer, mouth cancer, or throat cancer), and human papilloma virus (HPV) infection.
- Bladder cancer: One or more risk factors selected from the group consisting of tobacco consumption (in particular smoking), occupation (in particular work exposure to aromatic amines and/or workers in rubber, leather, textiles, paint and printing industries as well as machinists, hairdressers and truck drivers), ethnicity (in particular White), age of 50 or older (in particular 55 or older), male gender, chronic bladder irritation and infections, personal history of bladder or other urothelial cancer (completely removed and/or other subtype), bladder birth defects, family history of bladder cancer (in particular in first degree relatives), having an inherited syndrome or predisposition (such as a mutation of the retinoblastoma (RB1) gene, Cowden disease caused by mutations in the PTEN gene, or Lynch syndrome), past or current chemotherapy with cyclophosphamide, past or current radiation therapy with radiation to the pelvis, exposure to pioglitazone, food supplements containing aristolochic acid and/or arsenic in drinking water, and low fluid consumption.
- Prostate cancer: One or more risk factors selected from the group consisting of age of 50 or older (in particular 65 or older), male gender, ethnicity (in particular African American and Caribbean men of African ancestry), family history of prostate cancer (in particular in first degree relatives), having an inherited syndrome or predisposition (such as a mutation of the BRCA1 or BRCA2 gene, or Lynch syndrome), diet (in particular rich in red meat or high-fat dairy products), obesity, tobacco consumption (in particular smoking), occupation (in particular firefighters), inflammation of the prostate, sexually transmitted infections (in particular gonorrhea or chlamydia), and vasectomy.

Further, it is preferred that when DNA methylation with respect to SEQ ID NO: 36 is detected for diagnosing or screening for cancer, one or more of the following risk factors can be attributed to the subject:
- Lung cancer: One or more risk factors selected from the group consisting of tobacco consumption (in particular smoking), exposure to radon, exposure to asbestos, exposure to radioactive ores such as uranium, inhalation of chemicals or minerals such as arsenic, beryllium, cadmium, silica, vinyl chloride, nickel compounds, chromium compounds, coal products, mustard gas, or chloromethyl ethers, inhalation of diesel exhaust, increased exposure to air pollution (e.g. due to working and/or living in inner cities), radiation therapy to the lungs, arsenic in drinking water, personal history of lung cancer (completely removed and/or other subtype), and family history of lung cancer (in particular first degree relatives).
- Colon cancer: One or more risk factors selected from the group defined above with respect to SEQ ID NO: 1.
- Breast cancer: One or more risk factors selected from the group consisting of female gender, age of 45 or older (in particular 50 or 55 or older), having a risk-increasing inherited syndrome or predisposition (such as mutation of the BRCA1, BRCA2, ATM, TP53, CHEK2, PTEN, CDH1, STK11 and/or PALB2 gene), family history of breast cancer (in particular first degree relatives), personal history of breast cancer in another part of the breast or in the other breast, ethnicity (in particular White), dense breast tissue (in particular a dense breast on mammogram 1.2 to 2 times of the average breast density of women), benign breast conditions (such as (i) non-proliferative lesions including fibrosis and/or simple cysts, mild hyperplasia, adenosis (non-sclerosing), ductal ectasia, Phyllodes tumor (benign), single papilloma, fat necrosis, periductal fibrosis, squamous and apocrine metaplasia, and epithelial-related calcifications, or (ii) proliferative lesions without atypia including usual ductal hyperplasia (without atypia), fibroadenoma, sclerosing adenosis, papillomas (in particular papillomatosis), and radial scar, or (iii) proliferative lesions with atypia including atypical ductal hyperplasia (ADH) and atypical lobular hyperplasia (ALH)), lobular carcinoma in situ, above average number of menstrual periods (e.g. due to menstruation before age of 12 and/or menopause after age of 55), previous chest radiation, diethylstilbestrol exposure, not having children or having the first child after the age of 30 (in particular combined with the age risk factor defined above), use of oral contraceptives or depot-medroxyprogesterone acetate, hormone therapy after menopause, heavy alcohol use (as defined above), being overweight or obese, and physical inactivity.

- Stomach cancer: One or more risk factors selected from the group defined above with respect to SEQ ID NO: 11 and/or 16.
- Esophagus cancer: One or more risk factors selected from the group defined above with respect to SEQ ID NO: 11 and/or 16.

In a preferred embodiment, the genomic DNA comprises at least one CpG dinucleotide, preferably at least 2, 3, 4, 5, or 6 CpG dinucleotides. Generally, the methylation of at least one CpG dinucleotide comprised in the genomic DNA is detected, preferably of at least 2, 3, 4, 5, or 6 CpG dinucleotides.

Further, it is preferred that the genomic DNA having a sequence comprised in SEQ ID NO: 11 and/or 16 has a sequence comprised in SEQ ID NO: 21. Also, it is preferred that the genomic DNA having a sequence comprised in SEQ ID NO: 36 has a sequence comprised in SEQ ID NO: 41, more preferably in SEQ ID NO: 46 and most preferably in SEQ ID NO: 51.

Definitions and embodiments described below, in particular under the header 'Definitions and further embodiments of the invention' apply to the method of the first aspect.

In a second aspect, the present invention relates to a method for detecting the presence or absence of cancer or a risk thereof in a subject, comprising detecting DNA methylation within genomic DNA having a sequence comprised in SEQ ID NO: 11 and/or 16, and/or within genomic DNA having a sequence comprised in SEQ ID NO: 36 from a sample comprising cell-free DNA from blood or a sample derived therefrom of a subject, wherein the cancer is selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

Preferably, the cancer is selected from the group consisting of stomach, liver, colon, ovary, esophagus, bladder and prostate cancer (when detecting DNA methylation with respect to SEQ ID NO: 11 and/or 16) and/or from the group consisting of lung, colon, breast, stomach and esophagus cancer (when detecting DNA methylation with respect to SEQ ID NO: 36). The cancer may be of any subtype and stage as defined below, i.e. the presence or absence of any subtype and/or stage can be detected.

In a preferred embodiment,
(i) the presence of a significant amount of methylated genomic DNA having a sequence comprised in SEQ ID NO: 11 and/or 16 is indicative for the presence of stomach, liver, colon, ovary, esophagus, bladder or prostate cancer or a risk thereof, and the absence of a significant amount of methylated genomic DNA having a sequence comprised in SEQ ID NO: 11 and/or 16 is indicative for the absence of stomach, liver, colon, ovary, esophagus, bladder and prostate cancer or a risk thereof,
(ii) the presence of a significant amount of methylated genomic DNA having a sequence comprised in SEQ ID NO: 36 is indicative for the presence of lung, colon, breast, stomach or esophagus cancer or a risk thereof, and the absence of a significant amount of methylated genomic DNA having a sequence comprised in SEQ ID NO: 36 is indicative for the absence of lung, colon, breast, stomach and esophagus cancer or a risk thereof
(iii) the presence of a significant amount of methylated genomic DNA having a sequence comprised in SEQ ID NO: 11 and/or 16 and of a significant amount of methylated genomic DNA having a sequence comprised in SEQ ID NO: 36 is indicative for the presence of stomach, colon or esophagus cancer or a risk thereof, and
(iv) the presence of a significant amount of methylated genomic DNA having a sequence comprised in either SEQ ID NO: 11 and/or 16 or in SEQ ID NO: 36 and the absence of a significant amount of methylated genomic DNA having a sequence comprised in the other is indicative for the presence of cancer or a risk thereof according to the indications of (i) and (ii) combined.

Preferably, in (iv),
(iv.a) the presence of a significant amount of methylated genomic DNA having a sequence comprised in SEQ ID NO: 11 and/or 16 and the absence of a significant amount of methylated genomic DNA having a sequence comprised in SEQ ID NO: 36 is indicative for the presence of liver, ovary, bladder or prostate cancer or a risk thereof, and
(iv.b) the absence of a significant amount of methylated genomic DNA having a sequence comprised in SEQ ID NO: 11 and/or 16 and the presence of a significant amount of methylated genomic DNA having a sequence comprised in SEQ ID NO: 36 is indicative for the presence of lung or breast cancer or a risk thereof.

The term "a significant amount of methylated genomic DNA" as used herein refers to an amount of at least X molecules of the methylated target DNA per ml of the sample used, preferably per ml of blood, serum or plasma. X may be as low as 1 and is usually a value between and including 1 and 50, in particular 2, 3, 4, 5, 10, 15, 20, 25, 30 or 40. For determination whether there is such a significant amount, the methylated target DNA may be, but does not necessarily have to be quantified. The determination, if no quantification is performed, may also be made by comparison to a standard, for example a standard comprising genomic DNA and therein a certain amount of fully methylated DNA, e.g. the equivalence of X genomes, wherein X is as above. The term may also refer to an amount of at least Y% of methylated target DNA in the sample (wherein the sum of methylated and unmethylated target DNA is 100%), wherein Y may be as low as 0.05 and is usually a value between and including 0.05 and 5, preferably 0.05 and 1 and more preferably 0.05 and 0.5. For example, Y may be 0.05, 0.1, 0.2, 0.3, 0.5, 1.0, 1.5, 2.0, 2.5, 3.0, 4.0 or 5.0.

A preferred way of carrying out the method of the first or second aspect comprises the steps of
(a) converting cytosine unmethylated in the 5-position to uracil or another base that does not hybridize to guanine in the genomic DNA;
(b) amplifying methylation-specifically a region of the converted DNA;
(c) detecting the presence or absence of DNA amplified in step (b);
wherein the presence or absence of amplified DNA is indicative of the presence or absence, respectively, of methylated genomic DNA.

The amplification is preferably performed by methylation-specific PCR (i.e. an amplicon is produced depending on whether one or more CpG sites are converted or not) using primers which are methylation-specific (and specific to bisulfite-converted DNA) or more preferably primers which are methylation-unspecific, but specific to bisulfite-converted DNA (i.e. hybridize only to converted DNA by covering at least one converted C). In case of the latter, methylation-specificity is achieved by using methylation-specific blocker oligonucleotides, which hybridize specifically to converted or non-converted CpG sites and thereby terminate the PCR polymerization. In a most preferred embodiment, the step of amplifying comprises a PCR, preferably a real-time PCR, in particular HeavyMethyl™ or HeavyMethyl™-MethyLight™.

In other words, the region of the converted DNA is amplified methylation-specifically by using at least one methylation-specific oligonucleotide. Preferably, the at least one methylation-specific oligonucleotide is a primer or a blocker. In specific embodiments, the region of the converted DNA is amplified methylation-specifically by using one or two (i.e. a pair of) methylation-specific primers, or by using two (i.e. a pair of) methylation-unspecific primers and one or two (i.e. pair of) methylation specific blockers.

Further, the at least one methylation-specific oligonucleotide (e.g. a primer, blocker or a probe as referred to herein) preferably is substantially identical or complementary to a stretch of contiguous nucleotides of SEQ ID NOs 12-15 and/or 17-20 for amplifying converted DNA derived from genomic DNA having a sequence comprised in SEQ ID NO: 11 and/or 16, or of SEQ ID NOs 37-40 for amplifying converted DNA derived from genomic DNA having a sequence comprised in SEQ ID NO: 36.

The presence or absence of amplified DNA can be detected as described below, in particular by real-time PCR or by Next Generation Sequencing

In a preferred embodiment, detecting the presence of absence of DNA amplified in step (b) comprises determining the amount of DNA amplified in step (b). The amount can be determined with the detection means described below, in particular by real-time PCR or by Next Generation Sequencing.

Further, it is preferred that the converted DNA is amplified methylation-specifically by using at least one methylation-specific oligonucleotide which is substantially identical or complementary to a stretch of contiguous nucleotides of
SEQ ID NOs 22-25 for amplifying converted DNA derived from genomic DNA having a sequence comprised in SEQ ID NO: 21.

Also, it is preferred that the converted DNA is amplified methylation-specifically by using at least one methylation-specific oligonucleotide which is substantially complementary to a stretch of contiguous nucleotides of
- SEQ ID NOs 42-45 for amplifying converted DNA derived from genomic DNA having a sequence comprised in SEQ ID NO: 41,
- SEQ ID NOs 47-50 for amplifying converted DNA derived from genomic DNA having a sequence comprised in SEQ ID NO: 46, and
- SEQ ID NOs 52-55 for amplifying converted DNA derived from genomic DNA having a sequence comprised in SEQ ID NO: 51.

In a particular embodiment, the method of the second aspect further comprises confirming an indicated cancer and/or narrowing down the indicated cancers by using one or more further means for detecting an indicated cancer. The further means may be a cancer marker (or "biomarker") or a conventional (non-marker) detection means. The cancer marker can for example be a DNA methylation marker, a mutation marker (e.g. SNP), an antigen marker, a protein marker, a miRNA marker, a cancer specific metabolite, or an expression marker (e.g. RNA or protein expression). The conventional means can for example be a biopsy (e.g. visual biopsy examination with or without staining methods for example for protein or expression markers), an imaging technique (e.g. X-ray imaging, CT scan, nuclear imaging such as PET and SPECT, ultrasound, magnetic resonance imaging (MRI), thermography, endoscopy, digital mammography, colonoscopy or virtual colonoscopy, laparoscopy, angiogram, bone scan or sentinel node mapping for breast cancer) or a physical, e.g. tactile examination. If the further means is for confirmation of a single indicated cancer, it is preferred that it is a biopsy or other means that removes and examines a solid tissue sample of the subject from the tissue for which the cancer is indicated (i.e. no liquid tissue such as blood). If the further means is for narrowing down a set of indicated cancers, it is preferred that it is a non-invasive or a minimally invasive means. Such means can be a further marker or a conventional means such as an imaging technique or a physical examination. Generally, the further means is suitable for detecting a cancer the presence of which is indicated by the presence of DNA methylation of genomic DNA having a sequence comprised in SEQ ID NO: 11 and/or 16, and/or SEQ ID NO: 36. Specifically, it is preferred that
- for stomach cancer, the further means for cancer detection is selected from the group consisting of biopsy, endoscopy, ultrasound, X-ray, CT scan, MRI, PET scan and laparoscopy;
- for liver cancer, the further means for cancer detection is selected from the group consisting of physical examination, blood test for alphafetoprotein (AFP), ultrasound, CT scan, MRI, angiogram, biopsy, and laparoscopy;
- for colon cancer, the further means for cancer detection is selected from the group consisting of colonoscopy, blood test for anemia and/or carcinoembryonic antigen (CEA), CR scan, MRI, ultrasound, X-ray, and PET scan;
- for ovary cancer, the further means for cancer detection is selected from the group consisting of physical examination, ultrasound, CT scan, X-ray, MRI, PET scan, laparoscopy, biopsy, blood test for CA-125, human chorionic gonadotropin (HCG), alpha-fetoprotein (AFP), lactate dehydrogenase (LDH), inhibin, estrogen and/or testosterone, and colonoscopy;
- for esophagus cancer, the further means for cancer detection is selected from the group consisting of X-ray, upper endoscopy, endoscopic ultrasound, bronchoscopy, biopsy, CT scan, MRI and PET scan;
- for bladder cancer, the further means for cancer detection is selected from the group consisting of cytoscopy, urine test for tumor cells, biopsy (in particular transurethral resection of bladder tumor), CT scan, MRI and PET scan;
- for prostate cancer, the further means for cancer detection is selected from the group consisting of PSA test, DRE test, PCA3 test, ultrasound (in particular transrectal ultrasound), biopsy, MRI, MRI fusion biopsy, and CT scan;
- for lung cancer, the further means for cancer detection is selected from the group consisting of biopsy, testing for mutations in on one or more of the genes EGFR, ALK, KRAS, BRAF, HER2, ROS1, and/or RET (in particular EGFR and/or ALK), sputum cytology, bronchoscopy, needle aspiration or core biopsy, thoracentesis, thoracoscopy, mediastinoscopy, thoracotomy, endobronchial ultrasound, endoscopic esophageal ultrasound, CT scan, PET scan, and MRI scan;
- for breast cancer, the further means for cancer detection is selected from the group consisting of mammography, ultrasound, MRI, biopsy, complete blood count, serum chemistry, and physical examination.

In a preferred embodiment, the further means is suitable for detecting
(i) stomach, liver, colon, ovary, esophagus, bladder and/or, preferably or, prostate cancer or a risk thereof for confirming and/or narrowing down the determination of the presence of cancer using genomic DNA having a sequence comprised in SEQ ID NO: 1, preferably if DNA methylation is detected therein,
(ii) lung, colon, breast, stomach and/or, preferably or, esophagus cancer or a risk thereof for confirming and/or narrowing down the determination of the presence of cancer using genomic DNA having a sequence comprised in SEQ ID NO: 36, preferably if DNA methylation is detected therein,
(iii) - stomach, colon and/or, preferably or, esophagus cancer or a risk thereof using genomic DNA having a sequence comprised in SEQ ID NO: 1 and genomic DNA having a sequence comprised in SEQ ID NO: 36, preferably if DNA methylation is detected in both,
   - liver, ovary, bladder or prostate cancer or a risk thereof using genomic DNA having a sequence comprised in SEQ ID NO: 11 and/or 16 and genomic DNA having a sequence comprised in SEQ ID NO: 36, preferably if DNA methylation is detected in the former but not the latter,
   - lung or breast cancer or a risk thereof using genomic DNA having a sequence comprised in SEQ ID NO: 11 and/or 16 and genomic DNA having a sequence comprised in SEQ ID NO: 36, preferably if DNA methylation is not detected in the former but in the latter,
   for confirming and/or narrowing down the determination of the presence of cancer using genomic DNA having a sequence comprised in SEQ ID NO: 11 and/or 16 and genomic DNA having a sequence comprised in SEQ ID NO: 36.

Definitions given and embodiments described with respect to the first aspect apply also to the second aspect, in as far as they are applicable. Also, definitions and embodiments described below, in particular under the header 'Definitions and further embodiments of the invention' apply to the method of the second aspect.

In a third aspect, the present invention relates to the use of an oligonucleotide selected from the group consisting of a primer, blocker or probe, which has a sequence that is substantially identical or complementary to a stretch of contiguous nucleotides of SEQ ID NOs 12-15 or 17-20, or SEQ ID NOs 37-40, in a method for detecting DNA methylation for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer for SEQ ID NOs 12-15 and 17-20 and/or from the group consisting of lung, colon, breast, stomach and esophagus cancer for SEQ ID NOs 37-40.

Preferably, the oligonucleotide has a sequence that is substantially identical or complementary to a stretch of contiguous nucleotides of SEQ ID NOs 22-25, or from the group consisting of SEQ ID NOs 42-45, 47-50 and 52-55.

Generally, the oligonucleotide comprises at least 1, 2 or 3 dinucleotides derived from conversion of the C of a CpG dinucleotide in SEQ ID NO: 11 and/or 16 (alternatively SEQ ID NO: 36 or a sub-sequence of either as recited above) or its complement into a T. In this case, the oligonucleotide is a methylation-specific oligonucleotide. This methylation-specific oligonucleotide is also specific for bisulfite-converted DNA, since it comprises at least one nucleotide derived from conversion of a C not in a CpG context (e.g. of a CpC, CpA or CpT dinucleotide) in SEQ ID NO: 11 and/or 16 (alternatively SEQ ID NO: 36 or a sub-sequence of either as recited above) or its complement into a T.

Alternatively, the oligonucleotide may also not comprise a dinucleotide derived from conversion of the C of a CpG dinucleotide in SEQ ID NO: 111 and/or 16 (alternatively SEQ ID NO: 36 or a sub-sequence of either as recited above) or its complement into a T. In that case, it is a methylation-unspecific oligonucleotide, which is nonetheless specific for bisulfite-converted DNA, since it comprises at least one nucleotide derived from conversion of a C not in a CpG context (e.g. a C of a CpC, CpA or CpT dinucleotide) in SEQ ID NO: 111 and/or 16 (alternatively SEQ ID NO: 36 or a sub-sequence of either as recited above) or its complement into a T. A methylation-unspecific oligonucleotide can, however, cover one or more dinucleotides derived from conversion of the C of a CpG dinucleotide in SEQ ID NO: 11 and/or 16 (alternatively SEQ ID NO: 36 or a sub-sequence of either as recited above) or its complement into a T with a mismatch that does not distinguish between converted and not converted C or with a spacer as defined herein.

A probe preferably has one or more modifications selected from the group consisting of a detectable label and a quencher, and/or a length of 5-40 nucleotides. Further, a probe is preferably a methylation-specific oligonucleotide. A blocker preferably has one or more modifications selected from the group consisting of a non-extensible 3' end and a backbone resistant to the 5' nuclease activity of polymerase, and/or a length of 15-50 nucleotides. Further, a blocker is preferably a methylation-specific oligonucleotide. A primer preferably has a length of 10-40 nucleotides. In particular the primer may be either a methylation-specific oligonucleotide or a methylation-unspecific oligonucleotide.

In a specific embodiment, the blocker has a sequence according to SEQ ID NO: 58 with a 3' and/or 5' deletion or addition of up to 5 nucleotides and/or the probe has a sequence according to SEQ ID NO: 59 with a 3' and/or 5' deletion or addition of up to 5 nucleotides, wherein the additions preferably are according to SEQ ID NO: 11 and/or 16 (i.e. are extensions of the blocker in SEQ ID NO: 11 and/or 16). In another specific embodiment, the blocker has a sequence according to SEQ ID NO: 62 with a 3' and/or 5' deletion or addition of up to 5 nucleotides and/or the probe has a sequence according to SEQ ID NO: 63 with a 3' and/or 5' deletion or addition of up to 5 nucleotides, wherein the additions preferably are according to SEQ ID NO: 36 (i.e. are extensions of the blocker in SEQ ID NO: 36).

Definitions given and embodiments described with respect to the first and second aspect apply also to the third aspect, in as far as they are applicable. Also, definitions and embodiments described below, in particular under the header 'Definitions and further embodiments of the invention' apply to the oligonucleotide of the third aspect.

In a fourth aspect, the present invention relates to the use of a kit comprising at least a first and a second oligonucleotide, wherein the first and the second oligonucleotides are oligonucleotides of aspect 3, in a method for detecting DNA methylation for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer for SEQ ID NOs 12-15 or 17-20 and/or from the group consisting of lung, colon, breast, stomach and esophagus cancer for SEQ ID NOs 37-40.

In a kit wherein the oligonucleotides are suitable for a real-time PCR, it is preferred that the first oligonucleotide is a methylation-specific probe according to aspect 3 and the second oligonucleotide is a methylation-specific blocker or a methylation-specific primer, according to aspect 3. In a preferred embodiment, the second oligonucleotide is a methylation-specific blocker. In this embodiment, it is preferred that the kit further comprises a methylation-unspecific primer according to aspect 3.

In a kit wherein the oligonucleotides are suitable for a PCR follow by sequencing, the first oligonucleotide is a primer according to aspect 3 and the second oligonucleotide is also a primer according to aspect 3 or a methylation-specific blocker according to aspect 3. In one embodiment, the first oligonucleotide and the second oligonucleotide are methylation-unspecific primers. Preferably, these primers are a primer pair, i.e. a forward and a reverse primer. This kit is for the simultaneously amplification and sequencing of both methylated and unmethylated target DNA (the latter being for example non-tumor background DNA), wherein the amount of unmethylated target DNA is determined for quantification of the methylated target DNA as described herein. In another embodiment, the first oligonucleotide and the second oligonucleotide are methylation-specific primers. Preferably, these primers are a primer pair, i.e. a forward and a reverse primer. In yet another embodiment, the first oligonucleotide is a methylation-unspecific primer and the second oligonucleotide is a methylation-specific blocker. These kits are for the amplification and sequencing only of the methylated target DNA. Generally, it is preferred that if the kit comprises a primer and/or a blocker, the primer and/or blocker comes as a pair, i.e. as a forward with a reverse primer (or vice versa) and/or a forward with reverse blocker (or vice versa). A forward blocker is a blocker that blocks extension from a forward primer and a reverse blocker is a blocker that blocks extension from a reverse primer.

Definitions given and embodiments described with respect to the first, second and third aspect apply also to the fourth aspect, in as far as they are applicable. Also, definitions and embodiments described below, in particular under the header 'Definitions and further embodiments of the invention' apply to the kit of the fourth aspect.

In a fifth aspect, the present invention relates to the use of the method of the first or the second aspect, of the oligonucleotide of the third aspect or of the kit of the fourth aspect for the diagnosis of cancer, for the assessment of the response to treatment of cancer, for the monitoring of cancer, or for the screening of subjects to detect an increased likelihood of cancer.

Definitions given and embodiments described with respect to the first, second, third and fourth aspect apply also to the fifth aspect, in as far as they are applicable. Also, definitions and embodiments described below, in particular under the header 'Definitions and further embodiments of the invention' apply to the use of the fifth aspect.

Further disclosed is a method of treating cancer in a subject in which the presence of cancer has been detected according to the method of the second aspect, with a cancer treatment regimen suitable for treating the cancer. In other words, the present invention relates to the use of a cancer treatment suitable for treating a cancer in a subject in which the presence of cancer has been detected according to the method of the second aspect. The present invention also relates to a method comprising the method of the second aspect and subsequently referring a subject for such a cancer treatment. Definitions and further embodiments for the cancer treatment regimen are given below.

Definitions given and embodiments described with respect to the first, second, third, fourth and fifth aspect apply also to this method, in as far as they are applicable. Also, definitions and embodiments described below, in particular under the header 'Definitions and further embodiments of the invention' apply to this method.

### Definitions and further embodiments of the invention

The specification uses a variety of terms and phrases, which have certain meanings as defined below. Preferred meanings are to be construed as preferred embodiments of the aspects of the invention described herein. As such, they and also further embodiments described in the following can be combined with any embodiment of the aspects of the invention and in particular any preferred embodiment of the aspects of the invention described above.

The term "detecting DNA methylation" as used herein refers to at least qualitatively analysing for the presence or absence of methylated target DNA. Methylation is preferably determined at 1 or more, 2 or more, 3 or more, 4 or more, 5 or more, 6 or more, 10 or more, 15 or more or 30 or more CpG sites of the target DNA. Usually, the CpG sites analysed are co-methylated in cancer, such that also CpG sites of neighbouring DNA are methylated and can be analysed in addition or instead. "At least qualitatively" means that also a quantitative determination of methylated target DNA, if present, can be performed. Such a "determining the amount" can be performed as described herein.

The presence or absence of amplified DNA can be detected by any means known in the art, e.g. autoradiography, silver staining or ethidium bromide staining. Preferably, the presence or absence of DNA amplified in step (b) is detected by real-time PCR or by sequencing the amplified DNA. In particular if 3 or more (e.g. 4 or more or 5 or more) different target DNAs (i.e. markers) are examined in parallel, for example a target DNA within the DNA according to SEQ ID NOs 11 and/or 16, a target DNA within the DNA according to SEQ ID NOs 36, and one or more further target DNA, it is preferred that the presence or absence of amplified DNA is detected by sequencing.

In a real-time PCR, the presence of DNA amplified in step (b) is preferably detected by using a methylation-specific oligonucleotide which is a probe. The DNA is preferably amplified methylation-specifically using methylation-specific primers or a methylation-specific blocker with methylation-unspecific primers.

A detection by sequencing is preferably a detection by Next Generation Sequencing. Therein, the converted (e.g. bisulfite converted) methylated target DNA of the sample can be amplified methylation-specifically. Then, the amplified sequences are sequenced and the presence of methylated template is deduced from the presence of sequences or sequence reads derived from the amplified converted target DNA.

Generally, a quantification (e.g. determining the amount of methylated target DNA) may be absolute, e.g. in pg per mL or ng per mL sample, copies per mL sample, number of PCR cycles etc., or it may be relative, e.g. 10 fold higher than in a control sample or as percentage of methylation of a reference control. Determining the amount of methylated target DNA in the sample may comprise normalizing for the amount of total DNA in the sample. Normalizing for the amount of total DNA in the test sample preferably comprises calculating the ratio of the amount of methylated target DNA and (i) the amount of DNA of a reference site or (ii) the amount of total DNA of the target (e.g. the amount of methylated target DNA plus the amount of unmethylated target DNA, the latter preferably measured on the reverse strand. A reference site can be any genomic site and does not have to be a gene. It is preferred that the number of occurrences of the sequence of the reference site is stable or expected to be stable (i.e. constant) over a large population (e.g. is not in a repeat, i.e. in repetitive DNA). The reference site can, for instance be a housekeeping gene such as beta-Actin.

The amount of methylated target DNA in the sample may be expressed as the proportion of the amount of methylated target DNA relative to the amount of methylated target DNA in a reference sample comprising substantially fully methylated genomic DNA. Preferably, determining the proportion of methylated target DNA comprises determining the amount of methylated DNA of the same target in a reference sample, inter sample normalization of total methylated DNA, preferably by using the methylation unspecific measurement of a reference site, and dividing the ratio derived from the test sample by the corresponding ratio derived from the reference sample. The proportion can be expressed as a percentage or PMR as defined below by multiplying the result of the division by 100.

The term "PMR", "Percentage of Methylated Reference", or "Percentage of fully Methylated Reference" describes the degree of methylation and is usually calculated by dividing the gene to reference ratio by the gene to completely methylated reference ratio (obtained, e.g. by CpG methyltransferase, for example *SssI* treatment of the normally unmethylated reference) and multiplying by 100. The determination of the PMR is described in detail in Ogino et al. (JMD May 2006, Vol. 8, No. 2). The amount of methylated target DNA may also be calculated with the ΔΔ*Ct* method by using the real-time PCR cycle threshold (Ct) values for target and a reference site (ref) from samples of subjects and the calibrator (cal) sample (methylated at the target locus) as follows: ΔΔ*Ct* = ((Ct_{target} - Ct_{ref}) - (Ct_{targetcal} - Ct_{refcal})); PMR = 100 ^{∗} x^{-ΔΔ*Ct*}, wherein x is the assumed PCR efficiency. Generally, the PCR efficiency is assumed to be between 1-3, preferably it is 2 or about 2 (e.g. 1.9 to 2.1).

Furthermore, the amount of methylated target DNA may be determined by sequencing, preferably Next Generation Sequencing. Therein, the converted (e.g. bisulfite converted) target DNA can be amplified methylation-unspecifically, i.e. the target DNA is amplified whether or not it is methylated (in other words whether or not cytosines of the CpG sites are converted). This can be achieved by bisulfite-specific primers which are not methylation-specific. Then, the amplified sequences are sequenced and subsequently counted. The ratio of sequences derived from converted methylated DNA (identified in the sequences by CpG sites) and sequences derived from converted unmethylated DNA is calculated, resulting in a (relative) amount of methylated target DNA.

It is preferred that the amount of methylated target DNA is determined by sequencing rather than by real-time PCR in particular if the number of markers (i.e. target DNAs) examined in parallel is 3 or larger, preferably 4 or larger, and more preferably 5 or larger. Preferably, the amount of methylated DNA is the amount over at least 3, more preferably 4-8, most preferably 6 experimental repetitions or parallel experiments.

The term "Next Generation Sequencing" (NGS, also known as 2^{nd} or 3^{rd} generation sequencing) refers to a sequencing the bases of a small fragment of DNA are sequentially identified from signals emitted as each fragment is re-synthesized from a DNA template strand. NGS extends this process across millions of reactions in a massively parallel fashion, rather than being limited to a single or a few DNA fragments. This advance enables rapid sequencing of the amplified DNA, with the latest instruments capable of producing hundreds of gigabases of data in a single sequencing run. See, e.g., Shendure and Ji, Nature Biotechnology 26, 1135-1145 (2008) or Mardis, Annu Rev Genomics Hum Genet. 2008;9:387-402. Suitably NGS platforms are available commercially, e.g. the Roche 454 platform, the Roche 454 Junior platform, the Illumina HiSeq or MiSeq platforms, or the Life Technologies SOLiD 5500 or Ion Torrent platforms.

The term "calibrator sample" refers to a sample comprising control DNA with known DNA concentration and known target methylation state. The control DNA is preferably, but not necessarily, human DNA that is artificially methylated, preferably substantially fully methylated. In a preferred embodiment, the artificial methylation is achieved by using DNA-Methyltransferases. The DNA itself can be, for example, cell line DNA, plasmid DNA, artificial DNA, or combinations/mixtures thereof.

DNA methylated at the target locus is preferably cell line DNA from one or more cell lines, preferably of those that are well characterized and of which the genomic target methylation state is known and/or of which the target is known to be substantially fully methylated. Substantially fully methylated genomic DNA preferably is DNA, particularly genomic DNA, which has all or substantially all CpG sites methylated. "Substantially all" in this respect means at least 95%, 96%, 97%, 98%, 99%, 99.5% or 99.9%. In a preferred embodiment, the methylation of all or substantially all CpG sites is achieved by treating the DNA with a CpG methyltransferase in a manner that provides for the methylation of all or substantially all CpG sites.

The term "methylated" or "hypermethylated" as used herein refers to a biochemical process involving the addition of a methyl group to the cytosine or adenine DNA nucleotides. DNA methylation at the 5 position of cytosine, especially in promoter regions, can have the effect of reducing gene expression and has been found in every vertebrate examined. In adult non-gamete cells, DNA methylation typically occurs in a CpG site. The term "CpG site" or "CpG dinucleotide", as used herein, refers to regions of DNA where a cytosine nucleotide occurs next to a guanine nucleotide in the linear sequence of bases along its length. "CpG" is shorthand for "C-phosphate-G", that is cytosine and guanine separated by only one phosphate; phosphate links any two nucleosides together in DNA. The "CpG" notation is used to distinguish this linear sequence from the CG base-pairing of cytosine and guanine. Cytosines in CpG dinucleotides can be methylated to form 5-methylcytosine. The term "CpG site" or "CpG site of genomic DNA" is also used with respect to the site of a former (unmethylated) CpG site in DNA in which the unmethylated C of the CpG site was converted to another as described herein (e.g. by bisulfite to uracil). The application provides the genomic sequence of each relevant DNA region as well as the bisulfite converted sequences of each converted strand. CpG sites referred to are always the CpG sites of the genomic sequence, even if the converted sequence does no longer contain these CpG sites due to the conversion. Specifically, methylation in the context of the present invention means hypermethylation. The term "hypermethylation" refers to an aberrant methylation pattern or status (i.e. the presence or absence of methylation of one or more nucleotides), wherein one or more nucleotides, preferably C(s) of a CpG site(s), are methylated compared to the same genomic DNA from a non-cancer cell of the subject or a subject not suffering or having suffered from the cancer the subject is treated for, preferably any cancer (healthy control). In particular, it refers to an increased presence of 5-mCyt at one or a plurality of CpG dinucleotides within a DNA sequence of a test DNA sample, relative to the amount of 5-mCyt found at corresponding CpG dinucleotides within a healthy control DNA sample. Hypermethylation as a methylation status/pattern can be determined at one or more CpG site(s). If more than one CpG site is used, hypermethylation can be determined at each site separately or as an average of the CpG sites taken together. Alternatively, all assessed CpG sites must be methylated such that the requirement hypermethylation is fulfilled.

Methylation is detected in particular in a region of the DNA according to the SEQ ID NO referred to (the "target DNA"). The term "target DNA" as used herein refers to a genomic nucleotide sequence at a specific chromosomal location. In the context of the present invention, it is typically a genetic marker that is known to be methylated in the state of disease (for example in cancer cells vs. non-cancer cells). A genetic marker can be a coding or non-coding region of genomic DNA.

The term "region of the target DNA" or "region of the converted DNA" as used herein refers to a part of the target DNA which is to be analysed. Generally, the region is at least 40, 50, 60, 70, 80, 90, 100, 150, or 200 or 300 base pairs (bp) long and/or not longer than 500, 600, 700, 800, 900 or 1000 bp (e.g. 40-500 or 80-500 bp). In particular, it is a region comprising at least 3, 4, 5, 6, 7, 8, 9, 10, 11, 12, 13, 14 or 15 CpG sites of the genomic DNA. Preferably, these sites are not covered by a primer used for amplification of the region. Preferably, at least 3, 4, 5, 6, 7, 8, 9, 10, 11, 12, 13, 14 or 15 (but not necessarily all, in particular CpG sites covered by a spacer or methylation-unspecific mismatch, e.g. in a primer) of these CpG sites are methylated in the target DNA.

The target DNAs of the invention are given in Figure 1 and Table 1.

The term "sample" as used herein refers to biological material obtained from a subject and comprises genomic DNA from all chromosomes, preferably genomic DNA covering the whole genome. The sample comprises, if a subject has cancer, cell-free genomic DNA (including the target DNA) from cancer cells, preferably circulating genomic DNA from cancer cells.

The term "sample comprising cell-free DNA from blood" as used herein refers to a body fluid sample comprising cell-free DNA from blood. While in a preferred embodiment this sample is blood, the term also comprises other body fluids. For example, urine comprises cell-free DNA from blood.. The term "sample derived from a sample comprising cell-free DNA from blood" as used herein refers to any sample that is derived by *in vitro* processing. For example, if the sample is blood, it is preferred that the sample derived therefore is plasma or serum.

The term "cell-free DNA" as used herein or its synonyms "cfDNA", "extracellular DNA", "circulating DNA" and "free circulating DNA" refers to DNA that is not comprised within an intact cell in the respective body fluid which is the sample or from which the sample is derived, but which is freely circulating in the body liquid sample. Cell-free DNA usually is genomic DNA that is fragmented as described below.

Typically, in samples comprising the target DNA, especially extracellular target DNA, from cancer cells, there is also target DNA from non-cancer cells which is not methylated contrary to the target DNA from cancer cells. Usually, said target DNA from non-cancer cells exceeds the amount from diseased cells by at least 10-fold, at least 100-fold, at least 1,000-fold or at least 10,000-fold. Generally, the genomic DNA comprised in the sample is at least partially fragmented. "At least partially fragmented" means that at least the extracellular DNA, in particular at least the extracellular target DNA, from cancer cells, is fragmented. The term "fragmented genomic DNA" refers to pieces of DNA of the genome of a cell, in particular a cancer cell, that are the result of a partial physical, chemical and/or biological break-up of the lengthy DNA into discrete fragments of shorter length. Particularly, "fragmented" means fragmentation of at least some of the genomic DNA, preferably the target DNA, into fragments shorter than 1,500 bp, 1,300 bp, 1,100 bp, 1,000 bp, 900 bp, 800 bp, 700 bp, 600 bp, 500 bp, 400 bp, 300 bp, 200 bp or 100 bp. "At least some" in this respect means at least 5%, 10%, 20%, 30%, 40%, 50% or 75%.

The term "genomic DNA" as used herein refers to chromosomal DNA and is used to distinguish from coding DNA. As such, it includes exons, introns as well as regulatory sequences, in particular promoters, belonging to a gene.

The phrase "converting, in DNA, cytosine unmethylated in the 5-position to uracil or another base that does not hybridize to guanine" as used herein refers to a process of chemically treating the DNA in such a way that all or substantially all of the unmethylated cytosine bases are converted to uracil bases, or another base which is dissimilar to cytosine in terms of base pairing behaviour, while the 5-methylcytosine bases remain unchanged. The conversion of unmethylated, but not methylated, cytosine bases within the DNA sample is conducted with a converting agent. The term "converting agent" as used herein relates to a reagent capable of converting an unmethylated cytosine to uracil or to another base that is detectably dissimilar to cytosine in terms of hybridization properties. The converting agent is preferably a bisulfite such as disulfite, or hydrogen sulfite. The reaction is performed according to standard procedures (Frommer et al., 1992, Proc Natl Acad Sci USA 89:1827-31; Olek, 1996, Nucleic Acids Res 24:5064-6; EP 1394172). It is also possible to conduct the conversion enzymatically, e.g by use of methylation specific cytidine deaminases. Most preferably, the converting agent is sodium bisulfite or bisulfite.

The term "amplifying" or "generating an amplicon" as used herein refers to an increase in the number of copies of the target nucleic acid and its complementary sequence, or particularly a region thereof. The amplification may be performed by using any method known in the art. The amplification of nucleic acid includes methods that require multiple cycles during the amplification process or method that are performed at a single temperature. Cycling techniques are exemplified by methods requiring thermo-cycling. The methods requiring thermo-cycling include polymerase chain reaction (PCR), which is well known in the art. The PCR includes denaturing a double-stranded DNA into single stranded DNAs by thermal denaturation, annealing a primer to the single stranded DNAs; and synthesizing a complementary strand from the primer. Isothermal amplification is an amplification performed at a single temperature or where the major aspect of the amplification process is performed at a single temperature. In the PCR process, the product of the reaction is heated to separate the two strands such that another primer may bind to the template. Conversely, the isothermal techniques rely on a strand displacing polymerase in order to separate the two strands of a double strand and re-copy the template. Isothermal techniques may be classified into methods that rely on the replacement of a primer to initiate a reiterative template copying and those that rely on continued re-use or new synthesis of a single primer molecule. The methods that rely on the replacement of the primer include helicase dependant amplification (HDA), exonuclease dependent amplification, recombinase polymerase amplification (RPA), and loop mediated amplification (LAMP). The methods that rely on continued re-use or new synthesis of a single primer molecule include strand displacement amplification (SDA) or nucleic acid based amplification (NASBA and TMA).

The amplification is preferably performed by methylation-specific PCR (i.e. an amplicon is produced depending on whether one or more CpG sites are converted or not) using primers which are methylation-unspecific, but specific to bisulfite-converted DNA (i.e. hybridize only to converted DNA by covering at least one converted C). Methylation-specificity is achieved by using methylation-specific blocker oligonucleotides, which hybridize to specifically to converted or non-converted CpG sites and thereby terminate the PCR polymerization. In a most preferred embodiment, the step of amplifying comprises a real-time PCR, in particular HeavyMethyl™ or HeavyMethyl™-MethyLight™. Alternatively, a methylation-specific PCR may use methylation-specific primers and no blockers.

The term HeavyMethyl™ as used herein refers to an assay, wherein methylation specific blocking probes (also referred to herein as blockers) covering CpG positions between, or covered by the amplification primers enable methylation-specific selective amplification of the target DNA or a region thereof.

The term "MethyLight™" refers to a high-throughput quantitative methylation assay that utilizes fluorescence-based real-time PCR technology that requires no further manipulations after the PCR step (Eads et al., Cancer Res. 59:2302-2306, 1999). Briefly, the MethyLight™ process begins with a mixed sample of genomic DNA that is converted, e.g. in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process. It may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. The term "HeavyMethyl™ MethyLight™" assay refers to a HeavyMethyl™ MethyLight™ assay, which is a variation of the MethyLight™ assay, wherein the MethyLight™ assay is combined with methylation specific blocking probes covering CpG positions between the amplification primers.

The term "annealing", when used with respect to an oligonucleotide, is to be understood as a bond of an oligonucleotide to an at least substantially complementary sequence along the lines of the Watson-Crick base pairings in the sample DNA, forming a duplex structure, under moderate or stringent hybridization conditions. When it is used with respect to a single nucleotide or base, it refers to the binding according to Watson-Crick base pairings, e.g. C-G, A-T and A-U. Stringent hybridization conditions involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderate conditions involve washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

"Substantially complementary" means that an oligonucleotide does not need to reflect the exact sequence of the template and can comprise mismatches and/or spacers as defined herein. "Substantially identical" means that an oligonucleotide does not need to be 100% identical to a reference sequence but can comprise mismatches and/or spacers as defined herein. It is preferred that a substantially complementary or identical oligonucleotide comprises 1 to 3, i.e. 1, 2 or 3 mismatches and/or spacers, preferably one mismatch or spacer per oligonucleotide, such that the intended annealing does not not fail due to the mismatches and/or spacers.. To enable annealing despite mismatches and/or spacers, it is preferred that an oligonucleotide does not comprise more than 1 mismatch per 10 nucleotides (rounded up if the first decimal is 5 or higher, otherwise rounded down) of the oligonucleotide.

The term "oligonucleotide" as used herein refers to a linear oligomer of 5 to 50 ribonucleotides or preferably deoxyribonucleotides. Preferably, it has the structure of a single-stranded DNA fragment.

The term "primer oligonucleotide" as used herein refers to a single-stranded oligonucleotide sequence substantially complementary to a nucleic acid sequence sought to be copied (the template) and serves as a starting point for synthesis of a primer extension product. Preferably, a primer oligonucleotide is 10 to 40 nucleotides, more preferably 15-30 nucleotides and most preferably 19 to 25 nucleotides in length.

The term "blocker" as used herein refers to a molecule which binds in a methylation-specific manner to a single-strand of DNA (i.e. it is specific for either the converted methylated or preferably for the converted unmethylated DNA or the amplified DNA derived from it) and prevents amplification of the DNA by binding to it, for example by preventing a primer to bind or by preventing primer extension where it binds. Non-limiting examples for blockers are sequence and/or methylation specific antibodies (blocking e.g. primer binding or the polymerase) and in particular blocker oligonucleotides.

A "blocker oligonucleotide" may be a blocker that prevents the extension of the primer located upstream of the blocker oligonucleotide. It comprises nucleosides/nucleotides having a backbone resistant to the 5' nuclease activity of the polymerase. This may be achieved, for example, by comprising peptide nucleic acid (PNA), locked nucleic acid (LNA), Morpholino, glycol nucleic acid (GNA), threose nucleic acid (TNA), bridged nucleic acids (BNA), N3'-P5' phosphoramidate (NP) oligomers, minor groove binder-linked-oligonucleotides (MGB- linked oligonucleotides), phosphorothioate (PS) oligomers, CrC₄alkylphosphonate oligomers, phosphoramidates, β-phosphodiester oligonucleotides, a-phosphodiester oligonucleotides or a combination thereof. Alternatively, it may be a non-extendable oligonucleotide with a binding site on the DNA single-strand that overlaps with the binding site of a primer oligonucleotide. When the blocker is bound, the primer cannot bind and therefore the amplicon is not generated. When the blocker is not bound, the primer-binding site is accessible and the amplicon is generated. For such an overlapping blocker, it is preferable that the affinity of the blocker is higher than the affinity of the primer for the DNA. A blocker oligonucleotide is typically 15 to 50, preferably 20 to 40 and more preferably 25 to 35 nucleotides long. "At least one blocker" refers in particular to a number of 1, 2, 3, 4 or 5 blockers, more particularly to 1-2 or 1-3 blockers. Also, a blocker oligonucleotide cannot by itself act as a primer (i.e. cannot be extended by a polymerase) due to a non-extensible 3' end.

The term "probe oligonucleotide" or "probe" as used herein refers to an oligonucleotide that is used to detect an amplicon by annealing to one strand of the amplicon, usually not where any of the primer oligonucleotides binds (i.e. not to a sequence segment of the one strand which overlaps with a sequence segment a primer oligonucleotide anneals to). Preferably it anneals without a mismatch or spacer, in other words it is preferably complementary to one strand of the amplicon. A probe oligonucleotide is preferably 5-40 nucleotides, more preferably 10 to 25 and most preferably 25 to 20 nucleotides long. Usually, the probe is linked, preferably covalently linked, to at least one detectable label which allows detection of the amplicon and/or at least one quencher which allows quenching the signal of a (preferably the) detectable label. The term "detectable label" as used herein does not exhibit any particular limitation. The detectable label may be selected from the group consisting of radioactive labels, luminescent labels, fluorescent dyes, compounds having an enzymatic activity, magnetic labels, antigens, and compounds having a high binding affinity for a detectable label. For example, fluorescent dyes linked to a probe may serve as a detection label, e.g. in a real-time PCR. Suitable radioactive markers are P-32, S-35, 1-125, and H-3, suitable luminescent markers are chemiluminescent compounds, preferably luminol, and suitable fluorescent markers are preferably dansyl chloride, fluorcein-5-isothiocyanate, and 4-fluor-7-nitrobenz-2-aza-1,3 diazole, in particular 6-Carboxyfluorescein (FAM), 6-Hexachlorofluorescein (HEX), 5(6)-Carboxytetramethylrhodamine (TAMRA), 5(6)-Carboxy-X-Rhodamine (ROX), Cyanin-5-Fluorophor (Cy5) and derivates thereof; suitable enzyme markers are horseradish peroxidase, alkaline phosphatase, a-galactosidase, acetylcholinesterase, or biotin. A probe may also be linked to a quencher. The term "quencher" as used herein refers to a molecule that deactivates or modulates the signal of a corresponding detectable label, e.g. by energy transfer, electron transfer, or by a chemical mechanism as defined by IUPAC (see compendium of chemical terminology 2^{nd} ed. 1997). In particular, the quencher modulates the light emission of a detectable label that is a fluorescent dye. In some cases, a quencher may itself be a fluorescent molecule that emits fluorescence at a characteristic wavelength distinct from the label whose fluorescence it is quenching. In other cases, the quencher does not itself fluoresce (i.e., the quencher is a "dark acceptor"). Such quenchers include, for example, dabcyl, methyl red, the QSY diarylrhodamine dyes, and the like.

The term "covering a CpG site" as used herein with respect to an oligonucleotide refers to the oligonucleotide annealing to a region of DNA comprising this CpG site, before or after conversion of the C of the CpG site (i.e. the CpG site of the corresponding genomic DNA when it is referred to a bisulfite converted sequence). The annealing may, with respect to the CpG site (or former CpG site if the C was converted), be methylation-specific or methylationunspecific as described below.

The term "methylation-specific" as used herein refers generally to the dependency from the presence or absence of CpG methylation.

The term "methylation-specific" as used herein with respect to an oligonucleotide means that the oligonucleotide does or does not anneal to a single-strand of DNA (in which cytosine unmethylated in the 5-position has been converted to uracil or another base that does not hybridize to guanine, and where it comprises at least one CpG site before conversion) without a mismatch regarding the position of the C in the at least one CpG site, depending on whether the C of the at least one CpG sites was unmethylated or methylated prior to the conversion, i.e. on whether the C has been converted or not. The methylation-specificity can be either positive (the oligonucleotide anneals without said mismatch if the C was not converted) or negative (the oligonucleotide anneals without said mismatch if the C was converted). To prevent annealing of the oligonucleotide contrary to its specificity, it preferably covers at least 2, 3, 4, 5 or 6 and preferably 3 to 6 CpG sites before conversion.

The term "methylation-unspecific" as used herein refers generally to the independency from the presence or absence of CpG methylation.

The term "methylation-unspecific" as used herein with respect to an oligonucleotide means that the oligonucleotide does anneal to a single-strand of DNA (in which cytosine unmethylated in the 5-position has been converted to uracil or another base that does not hybridize to guanine, and where it may or may not comprise at least one CpG site before conversion) irrespective of whether the C of the at least one CpG site was unmethylated or methylated prior to the conversion, i.e. of whether the C has been converted or not. In one case, the region of the single-strand of DNA the oligonucleotide anneals to does not comprise any CpG sites (before and after conversion) and the oligonuclotide is methylation-unspecific solely for this reason. While a methylation-unspecific oligonucleotide may cover one or more CpG dinucleotides, it does so with mismatches and/ or spacers. The term "mismatch" as used herein refers to base-pair mismatch in DNA, more specifically a base-pair that is unable to form normal base-pairing interactions (i.e., other than "A" with "T" or "U", or "G" with "C").

An oligonucleotide according to the third aspect, i.e. a probe, blocker or primer, may also cover an SNP site with an SNP-unspecific mismatch or with a spacer.

The term "SNP site" as used herein refers to the site of an "SNP", i.e. a single nucleotide polymorphism at a particular position in the, preferably human, genome that varies among a population of individuals. SNPs of the genomic DNA the present application refers to are known in the art and can be found in online databases such as dbSNP of NCBI (http://www.ncbi.nlm.nih.gov/snp).

The term "SNP-unspecific mismatch" as used herein refers to a mismatch that is due to a nucleotide substitution that does not substitute the nucleotide with one that corresponds to a nucleotide that is found at the same position in the genome of another individual of the same population.

The term "spacer" as used herein refers to a non-nucleotide spacer molecule, which increases, when joining two nucleotides, the distance between the two nucleotides to about the distance of one nucleotide (i.e. the distance the two nucleotides would be apart if they were joined by a third nucleotide). Non-limiting examples for spacers are Inosine, d-Uracil, halogenated bases, Amino-dT, C3, C12, Spacer 9, Spacer 18, and dSpacer)

The term "reflects" as used herein is to be understood to mean "is a result of" or "shows".

The phrase "method for detecting the presence or absence of cancer in a subject" as used herein refers to a determination whether the subject has cancer or not. As will be understood by persons skilled in the art, such assessment normally may not be correct for 100% of the subjects, although it preferably is correct. The term, however, requires that a correct indication can be made for a statistically significant part of the subjects. Whether a part is statistically significant can be determined easily by the person skilled in the art using several well known statistical evaluation tools, for example, determination of confidence intervals, determination of p values, Student's t-test, Mann-Whitney test, etc. Details are provided in Dowdy and Wearden, Statistics for Research, John Wiley & Sons, New York 1983. The preferred confidence intervals are at least 50%, at least 60%, at least 70%, at least 80%, at least 90%, at least 95%. The p values are preferably 0.05, 0.01, or 0.005.

The term "risk thereof" with respect to the method for detecting the presence or absence of cancer in a subject refers to the detection of an increased risk of developing the cancer or an increased probability of having it. If the subject already has an increased risk in view of one or more risk factors that can be attributed to it (as defined herein), the 'risk therof refers to a risk that is increased further, i.e. that is in addition to the risk due to those risk factors.

The term "cancer" as used herein refers to a large family of diseases which involve abnormal cell growth with the potential to invade or spread to other parts of the body. The cells form a subset of neoplasms or tumors. A neoplasm or tumor is a group of cells that have undergone unregulated growth, and will often form a mass or lump, but may be distributed diffusely. Preferably, the term "cancer" is defined by one or more of the following characteristics:
- self-sufficiency in growth signalling,
- insensitivity to anti-growth signals,
- evasion of apoptosis,
- enabling of a limitless replicative potential,
- induction and sustainment of angiogenesis, and/or
- activation of metastasis and invasion of tissue.

The cancer with respect to the present invention is selected from the group consisting of liver cancer, esophagus cancer, stomach cancer, pancreas cancer, breast cancer, lung cancer, neck cancer, kidney cancer, thyroid cancer, prostate cancer, bladder cancer, colon cancer, and ovary cancer.

The term "stomach cancer" or "gastric cancer" is used in the broadest sense and refers to all cancers that start in the stomach. It includes the subtypes adenocarcinoma, lymphoma, gastrointestinal stromal tumor (GIST), carcinoid tumor and aquamous cell carcinoma, small cell carcinoma and leiomyosarcoma starting in the stomach. It also includes the following stages (as defined by the corresponding TNM classification(s) in brackets): stage 0 (Tis, N0, M0), stage IA (T1, N0, M0), stage IB (T1, N1, M0; or T2, N0, M0), stage IIA (T1, N2, M0; T2, N1, M0; or T2, N0, M0), stage IIB (T1, N3, M0; T2, N2, M0; T3, N1, M0; or T4a, N0, M0), stage IIIA (T1, N2, M0; T2, N1, M0; or T2, N0, M0), stage IIIB (T3, N3, M0; T4a, N2, M0; or T4b, N0 or N1, M0), stage IIIC (T4a, N3, M0; or T4b, N2 or N3, M0), and stage IV (any other T, N, and M).

The term "liver cancer" or "hepatic cancer" is used in the broadest sense and refers to all cancers that start in the liver. It includes the subtypes hepatocellular carcinoma (HCC), cholangiocarcinoma (bile duct cancer), angiosarcoma and hepatoblastoma. It also includes the following stages (as defined by the corresponding TNM classification(s) in brackets): stage I (T1, N0, M0), stage II (T2, N0, M0), stage IIIA (T3a, N0, M0), stage IIIb (T3b, N0, M0), stage IIIc (T4, N0, M0), stage IVA (any T, N1, M0), stage IVB (any T, any N and M1).

The term "esophagus cancer" or "oesophageal cancer" is used in the broadest sense and refers to all cancers that start in the esophagus. It includes the subtypes esophageal squamous-cell carcinoma and esophageal adenocarcinoma. It also includes the following stages (as defined by the corresponding TNM classification(s) in brackets): For squamous cell carcinoma: stage 0 (Tis, N0, M0, GX or G1), stage IA (T1, N0, M0, GX or G1), stage IB (T1, N0, M0, G2 or G3; or T2 or T3, N0, M0, GX or G1), stage IIA (T2 or T3, N0, M0, GX or G1, location upper or middle; or T2 or T3, N0, M0, G2 or G3, location lower), stage IIB (T2 or T3, N0, M0, G2 or G3, location upper or middle; or T1 or T2, N1, M0), stage IIIA (T1 or T2, N2, M0; T3, N1, M0; or T4a, N0, M0), stage IIIC (T4a, N1 or N2, M0; T4b, any N, M0; or any T, N3, M0) and stage IV (any T, any N, M1); For adenocarcinoma: stage 0 (Tis, N0, M0, GX or G1), stage IA (T1, N0, M0, GX, G1 or G2), stage IB (T1, N0, M0, G3; or T2, N0, M0, GX, G1, or G2), stage IIA (T2, N0, M0, G3), stage IIB (T3, N0, M0; or T1 or T2, N1, M0), stage IIIA (T1 or T2, N2, M0; T3, N1, M0; or T3, N2, M0), stage IIIC (T4a, N1 or N2, M0; T4b, any N, M0; or any T, N3, M0), and stage IV (any T, any N, M1).

The term "pancreas cancer" is used in the broadest sense and refers to all cancers that start in the pancreas. It includes the subtypes exocrine cancers, endocrine cancers, pancreatoblastoma, sarcomas of the pancreas, and lymphoma. Exocrine cancers include adenocarcinomas, in particular ductal adenocarcinomas, as well as cystic tumours and cancer of the acinar cells. Endocrine cancers include gastrinomas, insulinomas, somatostatinomas, VIPomas, and glucagonomas. It also includes the following stages (as defined by the corresponding TNM classification(s) in brackets): stage 0 (Tis, N0, M0), stage IA (T1, N0, M0), stage IB (T2, N0, M0), stage IIA (T3, N0, M0), stage IIB (T1-3, N1, M0), stage III (T4, any N, M0), and stage IV (any T, any N, M1).

The term "breast cancer" is used in the broadest sense and refers to all cancers that start in the breast. It includes the subtypes ductal carcinoma in situ, invasive ductal carcinoma (including tubular carcinoma of the breast, medullary carcinoma of the breast, mucinous carcinoma of the breast, papillary carcinoma of the breast, and cribriform carcinoma of the breast), invasive lobular carcinoma, inflammatory breast cancer, lobular carcinoma in situ, male breast cancer, Paget's disease of the nipple, and Phyllodes tumors of the breast. It also includes the following stages (as defined by the corresponding TNM classification(s) in brackets): stage 0: (Tis, N0, M0), stage IA (T1, N0, M0), stage IB (T0 or T1, N1mi, M0), stage IIA (T0 or T1, N1 (but not N1mi), M0; or T2, N0, M0), stage IIB (T2, N1, M0; or T3, N0, M0), stage IIIA (T0 to T2, N2, M0; or T3, N1 or N2, M0), stage IIIB (T4, N0 to N2, M0), stage IIIC (any T, N3, M0), and stage IV (any T, any N, M1).

The term "neck cancer" or "head and neck cancer" is used in the broadest sense and refers to all cancers that start in the neck or in the head. It includes the subtypes laryngeal cancer, hypopharyngeal cancer, nasal cavity and paranasal sinus cancer, nasopharyngeal cancer, salivary gland cancer and oral and oropharyngeal cancer. It also includes the following stages (as defined by the corresponding TNM classification(s) in brackets): stage 0 (Tis, N0, M0), stage I (T1, N0, M0), stage II (T2, N0, M0), stage III (T3, N0, M0; or T1 to T3, N1, M0), stage IVA (T4a, N0 or N1, M0; or T1 to T4a, N2, M0), stage IVB (T4b, any N, M0 or any T, N3, M0), and stage IVC (any T, any N, M1).

The term "kidney cancer" or "renal cancer" is used in the broadest sense and refers to all cancers that start in the kidney. It includes the subtypes renal cell carcinoma or RCC (including clear cell RCC, papillary RCC, chromophobe RCC, and collecting duct RCC), transitional cell carcinoma, Wilms tumor, and renal sarcoma. It also includes the following stages (as defined by the corresponding TNM classification(s) in brackets): stage I (T1, N0, M0), stage II (T2, N0, M0), stage III (T3, N0, M0; or T1 to T3, N1, M0), and stage IV (T4, any N, M0; or any T, any N, M1).

The term "thyroid cancer" is used in the broadest sense and refers to all cancers that start in the thyroid. It includes the subtypes differentiated thyroid cancers (including papillary carcinoma, follicular carcinoma, and Hürthle cell carcinoma), medullary thyroid carcinoma, anaplastic carcinoma, thyroid lymphoma, and thyroid sarcoma. It also includes the following stages (as defined by the corresponding TNM classification(s) in brackets): For papillary or follicular (differentiated) thyroid cancer in patients younger than 45: stage I (any T, any N, M0), and stage II (any T, any N, M1); for papillary or follicular (differentiated) thyroid cancer in patients 45 years and older: stage I (T1, N0, M0), stage II (T2, N0, M0), stage III (T3, N0, M0; or T1 to T3, N1a, M0), stage IVA (T4a, any N, M0; or T1 to T3, N1b, M0), stage IVB (T4b, any N, M0), and stage IVC (any T, any N, M1); for medullary thyroid cancer: stage I (T1, N0, M0), stage II (T2, N0, M0; or T3, N0, M0), stage III (T1 to T3, N1a, M0), stage IVA (T4a, any N, M0; or T1 to T3, N1b, M0), stage IVB (T4b, any N, M0), and stage IVC (any T, any N, M1); for anaplastic (undifferentiated) thyroid cancer: stage IVA (T4a, any N, M0), stage IVB (T4b, any N, M0), and stage IVC (any T, any N, M1).

The term "prostate cancer" is used in the broadest sense and refers to all cancers that start in the prostate. It includes the subtypes adenocarcinoma, sarcoma, small cell carcinoma, neuroendocrine tumor (other than small cell carcinoma), and transitional cell carcinomas. It also includes the following stages (as defined by the corresponding TNM classification(s) in brackets): stage I (T1, N0, M0, Gleason score 6 or less, PSA less than 10; or T2a, N0, M0, Gleason score 6 or less, PSA less than 10), stage IIA (T1, N0, M0, Gleason score of 7, PSA less than 20; T1, N0, M0, Gleason score of 6 or less, PSA at least 10 but less than 20; or T2a or T2b, N0, M0, Gleason score of 7 or less, PSA less than 20), stage IIB (T2c, N0, M0, any Gleason score, any PSA; T1 or T2, N0, M0, any Gleason score, PSA of 20 or more; or T1 or T2, N0, M0, Gleason score of 8 or higher, any PSA), stage III (T3, N0, M0, any Gleason score, any PSA) and stage IV (T4, N0, M0, any Gleason score, any PSA; any T, N1, M0, any Gleason score, any PSA; or any T, any N, M1, any Gleason score, any PSA).

The term "bladder cancer" is used in the broadest sense and refers to all cancers that start in the bladder. It includes the subtypes transitional cell (urothelial) carcinoma (including papillary and flat carcinomas), squamous cell carcinoma, adenocarcinoma, small cell carcinoma, and sarcoma. It also includes the following stages (as defined by the corresponding TNM classification(s) in brackets): stage 0a (Ta, N0, M0), stage 0is (Tis, N0, M0), stage I (T1, N0, M0), stage II (T2a or T2b, N0, M0), stage III (T3a, T3b, or T4a, N0, M0), and stage IV (T4b, N0, M0; any T, N1 to N3, M0; or any T, any N, M1).

The term "ovary cancer" or "ovarian cancer" is used in the broadest sense and refers to all cancers that start in the ovaries. It includes the subtypes benign epithelial ovarian tumors, tumors of low malignant potential, and malignant epithelial ovarian tumors. It also includes the following stages (as defined by the corresponding TNM classification(s) in brackets): stage IA (T1a, N0, M0), stage IB (T1b, N0, M0), stage IC (T1c, N0, M0), stage IIA (T2a, N0, M0), stage IIB (T2b, N0, M0), stage IIIA1 (T1 or T2, N1, M0), stage IIIA2 (T3a2, N0 or N1, M0), stage IIIB (T3b, N0 or N1, M0), stage IIIC (T3c, N0 or N1, M0), and stage IV (any T, any N, M1)

The term "colon cancer" or "colorectal cancer" is used in the broadest sense and refers to (1) all stages and all forms of cancer arising from epithelial cells of the large intestine and/or rectum and/or (2) all stages and all forms of cancer affecting the lining of the large intestine and/or rectum. It includes the subtypes adenocarcinoma, gastrointestinal carcinoid tumor, gastrointestinal stromal tumor, primary colorectal lymphoma, leiomyosarcoma, melanoma or squamous cell carcinoma, each originating from the colon (colon cancer) or the rectum (rectal cancer). In the staging systems used for classification of colorectal cancer, the colon and rectum are treated as one organ. It also includes the following stages (as defined by the corresponding TNM classification(s) in brackets): stage 0 (Tis, N0, M0), stage I (T1-T2, N0, M0), stage IIA (T3, N0, M0), stage IIB (T4a, N0, M0), stage IIC (T4b, N0, M0), stage IIIA (T1-T2, N1, M0; or T1, N2a, M0), stage IIIB (T3-T4a, N1, M0; T2-T3, N2a, M0; or T1-T2, N2b, M0), stage IIIC (T4a, N2a, M0; T3-T4a, N2b, M0; or T4b N1-N2 M0), and stage IVA (any T, any N, M1a), stage IVA (any T, any N, M1b).

The term "lung cancer" is used in the broadest sense and refers to all cancers that start in the lung. It includes the subtypes small cell lung cancer (SCLC) and non-small cell lung cancer (NSCLC). The term "SCLC" or "small cell lung cancer", as used herein, refers to an undifferentiated neoplasm, preferably composed of primitive- or embryonic-appearing cells. As the name implies, the cells in small-cell carcinomas are smaller than normal cells and barely have room for any cytoplasm. The term "NSCLC" or "non-small cell lung cancer", as used herein, refers to a group of heterogeneous diseases grouped together because their prognosis and management is roughly identical and includes, according to the histological classification of the World Health Organization/International Association for the Study of Lung Cancer (Travis WD et al. Histological typing of lung and pleural tumors. 3rd ed. Berlin: Springer-Verlag, 1999):
(i) Squamous cell carcinoma (SCC), accounting for 30% to 40% of NSCLC, starts in the larger breathing tubes but grows slower meaning that the size of these tumors varies on diagnosis.
(ii) Adenocarcinoma is the most common subtype of NSCLC, accounting for 50% to 60% of NSCLC, which starts near the gas-exchanging surface of the lung and which includes a subtype, the bronchioalveolar carcinoma, which may have different responses to treatment.
(iii) Large cell carcinoma is a fast-growing form that grows near the surface of the lung. It is primarily a diagnosis of exclusion, and when more investigation is done, it is usually reclassified to squamous cell carcinoma or adenocarcinoma.
(iv) Adenosquamous carcinoma is a type of cancer that contains two types of cells: squamous cells (thin, flat cells that line certain organs) and gland-like cells.
(v) Carcinomas with pleomorphic, sarcomatoid or sarcomatous elements. This is a group of rare tumors reflecting a continuum in histological heterogeneity as well as epithelial and mesenchymal differentiation.
(vi) Carcinoid tumor is a slow-growing neuroendocrine lung tumor and begins in cells that are capable of releasing a hormone in response to a stimulus provided by the nervous system.
(vii) Carcinomas of salivary gland type begin in salivary gland cells located inside the large airways of the lung.
(viii) Unclassified carcinomas include cancers that do not fit into any of the aforementioned lung cancer categories.

The NSCLC may be a squamous cell carcinoma, adenocarcinoma, large cell (undifferentiated) carcinoma, adenosquamous carcinoma and sarcomatoid carcinoma.

The lung cancer may be a stage 0, IA, IB, IIa, lib, IIIa, IIIb or IV lung cancer as defined by the corresponding TNM classification(s) in brackets: stage 0 (TisN0M0), stage IA (T1, N0, M0), stage IB (T2, M0, N0), stage IIA (T1, N1, M0; or T2, N1, M0), stage IIB (T3, N0, M0), stage IIIA (T1, N2 M0; T2, N2, M0; T3, N1, M0; T3, N2, M0; T4, N0, M0; or T4, N1, M0), stage IIIB (T1, N3, M0; T2, N3, M0; T3, N3, M0; T4, N2, M0; or T4, N3, M0), and stage IV (any T or any N with M1).

The TNM classification is a staging system for malignant cancer. As used herein the term "TNM classification" refers to the 6^{th} edition of the TNM stage grouping as defined in Sobin et al. (International Union Against Cancer (UICC), TNM Classification of Malignant tumors, 6th ed. New York; Springer, 2002, pp. 191-203).

The term "cancer cell" as used herein refers to a cell that acquires a characteristic set of functional capabilities during their development, particularly one or more of the following: the ability to evade apoptosis, self-sufficiency in growth signals, insensitivity to anti-growth signals, tissue invasion/metastasis, significant growth potential, and/or sustained angiogenesis. The term is meant to encompass both pre-malignant and malignant cancer cells.

The term "tumor DNA" or "tumor DNA of a cancer cell" as used herein refers simply to DNA of a cancer cell. It is used only to distinguish DNA of a cancer cell more clearly from other DNA referred to herein. Thus, unless ambiguities are introduced, the term "DNA of a cancer cell" may be used instead.

The term "subject" as used herein refers to an individual, such as a human, a non-human primate (e.g. chimpanzees and other apes and monkey species); farm animals, such as birds, fish, cattle, sheep, pigs, goats and horses; domestic mammals, such as dogs and cats; laboratory animals including rodents, such as mice, rats and guinea pigs. The term does not denote a particular age or sex. In a particular meaning, the subject is a mammal. In a preferred meaning, the subject is a human. In principle, the subject can be any subject of which the methylation status within genomic DNA having a sequence comprised in SEQ ID NO: 111 and/or 16 and/or within genomic DNA having a sequence comprised in SEQ ID NO: 36, in particular from a sample comprising cell-free DNA from blood or a sample derived therefrom of a subject, is not known. Depending on what the method of the first aspect is to be used for, the term "subject" may have different limitations. For example, if the method is to be used for detecting cancer or screening subjects for cancer, the subject is not known to have cancer, i.e. it may or may not have cancer. In this example, the subject preferably is at risk or increased risk or is suspected to have cancer. "At risk or increased risk" means that one or more risk factors can be attributed to the subject), preferably as defined by the American Cancer Society generally or for the respective cancer.

The term "is indicative for" or "indicates" as used herein refers to an act of identifying or specifying the thing to be indicated. As will be understood by persons skilled in the art, such assessment normally may not be correct for 100% of the subjects, although it preferably is correct. The term, however, requires that a correct indication can be made for a statistically significant part of the subjects. For a description of statistic significance and suitable confidence intervals and p values, see above.

The term "amplifying" or "generating an amplicon" as used herein refers to amplifying a defined region of a double-stranded or single-stranded DNA template, typically with a polymerase chain reaction (PCR). An "amplicon" is a double-stranded fragment of DNA according to said defined region.

The term "pair of primers" as used herein refers to two oligonucleotides, namely a forward and a reverse primer, that have, with respect to a double-stranded nucleic acid molecule, sequences that are (at least substantially) identical to one strand each such that they each anneal to the complementary strand of the strand they are (at least substantially) identical to. The term "forward primer" refers to the primer which is (at least substantially) identical to the forward strand (as defined by the direction of the genomic reference sequence) of the double-stranded nucleic acid molecule, and the term "reverse primer" refers to the primer which is (at least substantially) identical to the reverse complementary strand of the forward strand in the double-stranded nucleic acid molecule. The distance between the sites where forward and reverse primer anneal to their template depends on the length of the amplicon the primers are supposed to allow generating. Typically, with respect to the present invention it is between 40 and 1000 bp. Preferred amplicon sizes are specified herein. In case of single-stranded DNA template that is to be amplified using a pair of primers, only one of the primers anneals to the single strand in the first amplification cycle. The other primer then binds to the newly generated complementary strand such that the result of amplification is a double-stranded DNA fragment. The phrase "pair of primers suitable for generating an amplicon from a single strand of genomic DNA in which cytosine unmethylated in the 5-position has been converted to uracil or another base that does not hybridize to guanine" refers to a pair of primers which takes into account a base change from unmethylated cytosines to uracil, which basepairs with adenine and is therefore replaced with thymine in the amplicon.

The term "diagnosis" as used herein refers to a determination whether a subject does or does not have cancer, and preferably also which cancer. A diagnosis by methylation analysis of the target DNA as described herein may be supplemented with a further means as described herein to and/or narrow down the cancer detected with the methylation analysis. As will be understood by persons skilled in the art, the diagnosis normally may not be correct for 100% of the subjects, although it preferably is correct. The term, however, requires that a correct diagnosis can be made for a statistically significant part of the subjects. For a description of statistic significance and suitable confidence intervals and p values, see above.

The phrase "response to treatment", "response to therapy" as used herein refers to the response of a subject suffering from cancer to a therapy for treating said disease. Standard criteria (Miller, et al., Cancer, 1981; 47(1): 207-14) can be used herewith to evaluate the response to therapy include response, stabilization and progression. The term "response", as used herein, can be a complete response (or complete remission) which is the disappearance of all detectable malignant disease or a partial response which is defined as approximately >50% decrease in the sum of products of the largest perpendicular diameters of one or more lesions (tumor lesions), no new lesions and no progression of any lesion. Subjects achieving complete or partial response are considered "responders", and all other subjects are considered "non-responders". The term "stabilization", as used herein, is defined as a < 50% decrease or a < 25% increase in tumor size. The term "progression", as used herein, is defined as an increase in the size of tumor lesions by > 25% or appearance of new lesions.

The term "monitoring" as used herein refers to the accompaniment of a diagnosed cancer during a treatment procedure or during a certain period of time, typically during at least 1 week, 2 weeks, 3 weeks, 4 weeks, 1 month, 2 months, 3 months, 4 months, 5 months, 6 months, 1 year, 2 years, 3 years, 5 years, 10 years, or any other period of time. The term "accompaniment" means that states of and, in particular, changes of these states of a cancer may be detected based on the amount of methylated target DNA, particular based on changes in the amount in any type of periodical time segment, determined e.g., daily or 1, 2, 3, 4, 5, 6, 7, 8, 9, 10, 11, 12, 13, 14 or 15 times per month (no more than one determination per day) over the course of the treatment, which may be up to 1, 2, 3, 4, 5, 6, 7, 8, 9, 10, 11, 12, 15 or 24 months. Amounts or changes in the amounts can also be determined at treatment specific events, e.g. before and/or after every treatment cycle or drug/therapy administration. A cycle is the time between one round of treatment until the start of the next round. Cancer treatment is usually not a single treatment, but a course of treatments. A course usually takes between 3 to 6 months, but can be more or less than that. During a course of treatment, there are usually between 4 to 8 cycles of treatment. Usually a cycle of treatment includes a treatment break to allow the body to recover. As will be understood by persons skilled in the art, the result of the monitoring normally may not be correct for 100% of the subjects, although it preferably is correct. The term, however, requires that a correct result of the monitoring can be achieved for a statistically significant part of the subjects. For a description of statistic significance and suitable confidence intervals and p values, see above.

The phrase "screening of subjects to detect an increased likelihood of cancer" refers to the use of the method of the first aspect with samples of a population of subjects. Preferably, the subjects have an increased risk for or are suspected of having a cancer selected from the groups recited herein with respect to certain SEQ ID NOs. In particular, one or more of the following risk factors recited herein can be attributed to the subjects of the population. In a specific embodiment, the same one or more risk factors can be attributed to all subjects of the population. For example, the population may be characterized by a certain minimal age (e.g. 50 or older). It is to be understood that the term "screening" does not necessarily indicate a definite diagnosis, but is intended to indicate an increased possibility of the presence or of the absence of a certain cancer or of a set of cancers. An indicated increased possibility is preferably confirmed and/or narrowed down using a further means as described herein. As will be understood by persons skilled in the art, the screening result normally may not be correct for 100% of the subjects, although it preferably is correct. The term, however, requires that a correct screening result can be achieved for a statistically significant part of the subjects. For a description of statistic significance and suitable confidence intervals and p values, see above.

The term "treatment" or "treating" with respect to cancer as used herein refers to a therapeutic treatment, wherein the goal is to reduce progression of cancer. Beneficial or desired clinical results include, but are not limited to, release of symptoms, reduction of the length of the disease, stabilized pathological state (specifically not deteriorated), slowing down of the disease's progression, improving the pathological state and/or remission (both partial and total), preferably detectable. A successful treatment does not necessarily mean cure, but it can also mean a prolonged survival, compared to the expected survival if the treatment is not applied. In a preferred embodiment, the treatment is a first line treatment, i.e. the cancer was not treated previously. Cancer treatment involves a treatment regimen.

The term "treatment regimen" as used herein refers to how the subject is treated in view of the disease and available procedures and medication. Non-limiting examples of cancer treatment regimes are chemotherapy, surgery and/or irradiation or combinations thereof. The early detection of cancer the present invention enables allows in particular for a surgical treatment, especially for a curative resection. In particular, the term "treatment regimen" refers to administering one or more anti-cancer agents or therapies. The term "anti-cancer agent or therapy" as used herein refers to chemical, physical or biological agents or therapies, or surgery, including combinations thereof, with antiproliferative, antioncogenic and/or carcinostatic properties.

### SEQ IDs referred to in the application

The present application refers to SEQ ID NOs 1-63. An overview and explanation of these SED IDs is given in the following Table 1:

**Table 1: SEQ IDs of the specification. rc means reverse complement, C to T or G to A means converted by bisulfite conversion of cytosines outside of CpG context into uracil and replaced by thymidine in subsequent amplification. bis1 refers to the bisulfite converted forward strand (as recited in the SEQ ID of the respective genomic DNA) and bis2 to the bisulfite converted reverse complement strand of the forward strand (reverse complement of the SEQ ID of the respective genomic DNA), whereby the direction of the strand is defined by the direction of the genomic reference sequence as e.g. obtained from the genome build (HCGR38). For a mapping of the sequences, see Figure 1.**

| | | | |
|---|---|---|---|
| ANKRD13B gene associated region 17: 29584769-29619761 | | FOXF2 gene 6:1389834-1395597 | |
| SEQ ID NO: 1 | genomic reference | SEQ ID NO: 31 | genomic reference |
| SEQ ID NO: 2 | C to T (bisl) | SEQ ID NO: 32 | C to T (bisl) |
| SEQ ID NO: 3 | rc C to T (bis1) | SEQ ID NO: 33 | rc C to T (bis1) |
| SEQ ID NO: 4 | G to A (bis2 rc) | SEQ ID NO: 34 | G to A (bis2 rc) |
| SEQ ID NO: 5 | G to A (bis2 rc) rc | SEQ ID NO: 35 | G to A (bis2 rc) rc |
| ANKRD13B gene 17: 29589769-29614761 | | FOXF2 assay + CpG island1 6:1388905-1391158 | |
| SEQ ID NO: 6 | genomic reference | | |
| SEQ ID NO: 7 | C to T (bis1) | SEQ ID NO: 36 | genomic reference |
| SEQ ID NO: 8 | rc C to T (bis1) | SEQ ID NO: 37 | C to T (bis1) |
| SEQ ID NO: 9 | G to A (bis2 rc) | SEQ ID NO: 38 | rc C to T (bis1) |
| SEQ ID NO: 10 | G to A (bis2 rc) rc | SEQ ID NO: 39 | G to A (bis2 rc) |
| | | SEQ ID NO: 40 | G to A (bis2 rc) rc |
| ANKRD13B assay+CpG island 17: 29612281-29613752 | | FOXF2 extended ass ay 6:1389866-1390941 | |
| SEQ ID NO: 11 | genomic reference | SEQ ID NO: 41 | genomic reference |
| SEQ ID NO: 12 | C to T (bis1) | SEQ ID NO: 42 | C to T (bis1) |
| SEQ ID NO: 13 | rc C to T (bis1) | SEQ ID NO: 43 | rc C to T (bis1) |
| SEQ ID NO: 14 | G to A (bis2 rc) | SEQ ID NO: 44 | G to A (bis2 rc) |
| SEQ ID NO: 15 | G to A (bis2 rc) rc | SEQ ID NO: 45 | G to A (bis2 rc) rc |
| ANKRD13B extended assay 17: 29613051-29614143 | | FOXF2 assay + MicroRNA MIR6720 6: 1390314-1390441 | |
| SEQ ID NO: 16 | genomic reference | SEQ ID NO: 46 | genomic reference |
| SEQ ID NO: 17 | C to T (bis1) | SEQ ID NO: 47 | C to T (bis1) |
| SEQ ID NO: 18 | rc C to T (bis1) | SEQ ID NO: 48 | rc C to T (bis1) |
| SEQ ID NO: 19 | G to A (bis2 rc) | SEQ ID NO: 49 | G to A (bis2 rc) |
| SEQ ID NO: 20 | G to A (bis2 rc) rc | SEQ ID NO: 50 | G to A (bis2 rc) rc |
| ANKRD13B assay 17: 29613551-29613643 | | FOXF2 assay 6:1390366-1390441 | |
| SEQ ID NO: 21 | genomic reference | SEQ ID NO: 51 | genomic reference |
| SEQ ID NO: 22 | C to T (bis1) | SEQ ID NO: 52 | C to T (bis1) |
| SEQ ID NO: 23 | rc C to T (bis1) | SEQ ID NO: 53 | rc C to T (bis1) |
| SEQ ID NO: 24 | G to A (bis2 rc) | SEQ ID NO: 54 | G to A (bis2 rc) |
| SEQ ID NO: 25 | G to A (bis2 rc) rc | SEQ ID NO: 55 | G to A (bis2 rc) rc |
| FOXF2 gene associated region 6:1384834-1400597 | | SEQ ID NO: 56 ANKRD13B-F4 primer | |
| | | SEQ ID NO: 57 ANKRD13B-R4 primer | |
| SEQ ID NO: 26 | genomic reference | SEQ ID NO: 58 ANKRD13B-B7 blocker | |
| SEQ ID NO: 27 | C to T (bis1) | SEQ ID NO: 59 ANKRD13B-P4 probe | |
| SEQ ID NO: 28 | rc C to T (bis1) | SEQ ID NO: 60 FOXF2-F4 primer | |
| SEQ ID NO: 29 | G to A (bis2 rc) | SEQ ID NO: 61 FOXF2-R5 primer | |
| SEQ ID NO: 30 | G to A (bis2 rc) rc | SEQ ID NO: 62 FOXF2-B5 blocker | |
| | | SEQ ID NO: 63 FOXF2-P4 probe | |

The invention is described by way of the following examples which are to be construed as merely illustrative and not limitative of the scope of the invention.

### EXAMPLE 1

### Sample preparation:

Technical samples used for assessing the assay were prepared as mixtures of bisulfite treated PBL (peripheral blood) DNA known to be unmethylated in the assay region (Human Genomic DNA: Male, Promega GmbH) and methylated DNA (DNA treated with DNA-Methyltransferases - Universal Methylated DNA, Merck Chemicals GmbH) in 10 ng/ml total. As a second technical reference control for unmethylated DNA genome wide randomly amplified DNA (Phi) was used. DNA isolated from different cancer tissues was purchased commercially from Biocat GmbH. Control DNAs and tissue DNA were bisulfite treated with the EpiTect Bisulfite Kit (Qiagen GmbH) following the manufacturers protocol.

Blood plasma samples from colorectal cancer patients and healthy individuals were collected as defined in the instructions for use (IFU) of the Epi proColon 2.0 kit (Epigenomics AG). Briefly, EDTA plasma was prepared by two centrifugation steps. Bulk plasma (a pool of plasma samples from healthy individuals; Plasma, Normal EDTA, Cliniqa Co.) was centrifuged under the same conditions before usage. Until processing, plasma samples were stored at -70°C.

### Sample/DNA processing:

Technical samples/DNA mixtures were used directly. Plasma samples were processed analogous to the pre-analytic workflow as defined in the instructions for use (IFU) of the Epi proColon 2.0 kit (Epigenomics AG).

PCR reactions either used 10 ng total DNA in each reaction for technical samples or the processed equivalent of about 1 ml plasma for plasma samples. Except for the PCR mix containing the relevant oligomers as layed out for the assays in this application, the real-time PCR on a LightCycler 480 (Roche Applied Sciences) was done following the PCR protocols of the Epi proColon 2.0 PCR kit (Epigenomics AG).

Beside methylation status of the investigated markers ANKRD13B and FOXF2, level of methylated and unmethylated Septin9 were measured to determine the total DNA yield in the individual PCR reaction. Calculation of DNA amount was done using technical DNA as a calibrator (e.g. 50% methylated DNA: 5 ng unmethylated bisulfite PBL DNA plus 5 ng methylated DNA). Methylation degree was determined by the ratio of the DNA amount of the investigated markers to the total amount of DNA (sum of methylated and unmethylated Septin9 marker).

### Results:

Methylation of the investigated markers ANKRD13B and FOXF2 could be successfully detected using the heavy methyl PCR assays described herein (Figure 2). Technical samples with only 50 pg methylated DNA (in a total background of 10 ng unmethylated DNA) show a strong signal in real time PCR. In contrast, samples processed from bulk plasma (pool of healthy individuals) show no or very faint signal in the PCR (Figure 6 and Figure 7).

The methylation status of the investigated marker was next tested on DNA derived from different cancer tissue. As shown in Figure 3B, the methylation degree of ANKRD13B in DNA from stomach, liver, colon, ovary, esophagus, bladder and prostate cancer was more than 10% in contrast to normal colon tissue (only 0.5% methylated DNA). It is expected that this strong difference of methylation in tissue can also be measured in liquid biopsies. As proof-of-concept, plasma samples from healthy individuals and CRC patiens were examined. Although mean methylation degree was only 8% in CRC plasma, samples can be clearly distinguished from healthy individuals (no detection at all - 0% methylation in plasma from healthy donors). One example of a real time PCR amplification plot detecting methylated ANKRD13B in a plasma sample of an individual CRC patient is shown in Figure 8. Beside the detection of methylated ANKRD13, signals are observed for methylated Septin 9 as a known CRC marker and unmethylated Septin 9 for the detection of unmethylated background DNA.

Figure 4B shows an amount of more than 10 % methylated DNA for FOXF2 in lung, colon, breast, stomach and esophagus cancer. Normal colon tissue shows low signals for methylated FOXF2 (only 0.2% methylated DNA). Again, as proof-of-concept for the detection of methylation in liquid biopsies, plasma samples from healthy individuals and CRC patients were examined (Figure 4A). The mean amount of methylated DNA was 10% in CRC plasma and can clearly distinguished from healthy donors (detection in merely one single sample with only 0.03% methylated target DNA). One example of a real time PCR amplification plot detecting methylated FOXF2 in a plasma sample of an individual CRC patient is shown in Figure 9. Beside the detection of methylated ANKRD13, signals are observed for methylated Septin 9 as a known CRC marker and unmethylated Septin 9 for the detection of unmethylated background DNA.

The methylation degree of both investigated markers for the individual cancer tissues are compared in a matrix shown in Figure 5. In that way a direct comparison of the methylation degree in the same kind of cancer tissue by different markers becomes obvious. Different cluster can be observed a) cancer tissues than are not methylated for the investigated marker, e.g. pancreas or kidney cancer tissue, b) cancer tissues that only show a significant amount of methylation for one marker, e.g. methylated ANKRD13 in prostate cancer tissue or methylated FOXF2 in lung cancer tissue and c) cancer tissues that are detected by both investigated markers, e.g. colon or esophagus cancer tisse. If in a measurement a cluster refered to b) is observed, specific diagnosis can be given. For the other cluster (a) and (c) further marker or a conventional diagnosis needs to be added to allow to distinguish the different forms of cancers.

## Claims

1. A method for detecting DNA methylation within genomic DNA having a sequence comprised in SEQ ID NO: 11 and/or 16, and/or within genomic DNA having a sequence comprised in SEQ ID NO: 36, from a sample comprising cell-free DNA from blood or a sample derived therefrom of a subject, wherein the subject has an increased risk for or is suspected of having a cancer selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

2. The method of claim 1, wherein the genomic DNA having a sequence comprised in SEQ ID NO: 11 and/or 16 has a sequence comprised in SEQ ID NO: 21, and/or wherein the genomic DNA having a sequence comprised in SEQ ID NO: 36 has a sequence comprised in SEQ ID NO: 41, preferably in SEQ ID NO: 46 and more preferably in SEQ ID NO: 51.

3. The method of claim 1 or 2, wherein DNA methylation is detected in a target DNA that is methylated in the cancer.

4. The method of any one of claims 1 to 3, comprising the steps of
(a) converting cytosine unmethylated in the 5-position to uracil or another base that does not hybridize to guanine in the genomic DNA;
(b) amplifying methylation-specifically a region of the converted DNA;
(c) detecting the presence or absence of DNA amplified in step (b);
wherein the presence or absence of amplified DNA is indicative of the presence or absence, respectively, of methylated genomic DNA.

5. The method of claim 4, wherein the region of the converted DNA is amplified methylation-specifically by using at least one methylation-specific oligonucleotide which is substantially identical or complementary to a stretch of contiguous nucleotides of SEQ ID NOs 12-15 and/or 17-20 for amplifying converted DNA derived from genomic DNA having a sequence comprised in SEQ ID NO: 11 and/or 16, respectively, or of SEQ ID NOs 37-40 for amplifying converted DNA derived from genomic DNA having a sequence comprised in SEQ ID NO: 36.

6. A method for detecting the presence or absence of cancer or a risk thereof in a subject, comprising detecting DNA methylation within genomic DNA having a sequence comprised in SEQ ID NO: 11 and/or 16, and/or within genomic DNA having a sequence comprised in SEQ ID NO: 36 from a sample comprising cell-free DNA from blood or a sample derived therefrom of a subject, wherein the cancer is selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

7. The method of claim 6, wherein the genomic DNA having a sequence comprised in SEQ ID NO: 11 and/or 16 has a sequence comprised in SEQ ID NO: 21, and/or wherein the genomic DNA having a sequence comprised in SEQ ID NO: 36 has a sequence comprised in SEQ ID NO: 41, preferably in SEQ ID NO: 46 and more preferably in SEQ ID NO: 51.

8. The method of claim 6 or 7, wherein DNA methylation is detected in a target DNA that is methylated in the cancer.

9. The method of any one of claims 6 to 8, wherein
(i) the presence of a significant amount of methylated genomic DNA having a sequence comprised in SEQ ID NO: 11 and/or 16 is indicative for the presence of stomach, liver, colon, ovary, esophagus, bladder or prostate cancer or a risk thereof, and the absence of a significant amount of methylated genomic DNA having a sequence comprised in SEQ ID NO: 11 and/or 16 is indicative for the absence of stomach, liver, colon, ovary, esophagus, bladder and prostate cancer or a risk thereof,
(ii) the presence of a significant amount of methylated genomic DNA having a sequence comprised in SEQ ID NO: 36 is indicative for the presence of lung, colon, breast, stomach or esophagus cancer or a risk thereof, and the absence of a significant amount of methylated genomic DNA having a sequence comprised in SEQ ID NO: 36 is indicative for the absence of lung, colon, breast, stomach and esophagus cancer or a risk thereof
(iii) the presence of a significant amount of methylated genomic DNA having a sequence comprised in SEQ ID NO: 11 and/or 16 and of a significant amount of methylated genomic DNA having a sequence comprised in SEQ ID NO: 36 is indicative for the presence of stomach, colon or esophagus cancer or a risk thereof, and
(iv) the presence of a significant amount of methylated genomic DNA having a sequence comprised in either SEQ ID NO:11 and/or 16, or in SEQ ID NO: 36, and the absence of a significant amount of methylated genomic DNA having a sequence comprised in the other is indicative for the presence of cancer or a risk thereof according to the indications of (i) and (ii) combined.

10. The method of claim 9, further comprising confirming an indicated cancer and/or narrowing down the indicated cancers by using one or more further means for detecting an indicated cancer.

11. The method of claim 9, wherein the amount of methylated genomic DNA is determined by real-time PCR or by sequencing.

12. Use of an oligonucleotide selected from the group consisting of a primer, blocker or probe, which has a sequence that is substantially identical or complementary to a stretch of contiguous nucleotides of SEQ ID NOs 12-15 or 17-20, or which has a sequence that is substantially identical or complementary to a stretch of contiguous nucleotides of SEQ ID NOs 37-40, in a method for detecting DNA methylation for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer for SEQ ID NOs 12-15 and 17-20 and/or from the group consisting of lung, colon, breast, stomach and esophagus cancer for SEQ ID NOs 37-40.

13. Use of a kit comprising at least a first and a second oligonucleotide, wherein the first oligonucleotide and the second oligonucleotide are oligonucleotides as defined in claim 12, in a method for detecting DNA methylation for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer for SEQ ID NOs 12-15 or 17-20 and/or from the group consisting of lung, colon, breast, stomach and esophagus cancer for SEQ ID NOs 37-40.

14. The use of claim 13, wherein the first oligonucleotide is a probe as defined in claim 12 that is methylation-specific and the second oligonucleotide is a blocker as defined in claim 12 that is methylation-specific or a primer as defined in claim 12 that is methylation-specific.

15. The use of claim 13, wherein the first oligonucleotide is a primer as defined in claim 12 and the second oligonucleotide is also a primer as defined in claim 12 or a blocker as defined in claim 12 that is methylation-specific.

16. Use of the method of claims 1-5 or of claims 6-11 for the diagnosis of cancer, for the assessment of the response to treatment of cancer, for the monitoring of cancer, or for the screening of subjects to detect an increased likelihood of cancer, wherein the cancer is selected from the group consisting of stomach, liver, colon, ovary, esophagus, bladder and prostate cancer when detecting DNA methylation within genomic DNA having a sequence comprised in SEQ ID NO: 11 and/or 16, and/or from the group consisting of lung, colon, breast, stomach and esophagus cancer when detecting DNA methylation within genomic DNA having a sequence comprised in SEQ ID NO: 36.

## Patentansprüche

1. Ein Verfahren zum Nachweis von DNA-Methylierung in genomischer DNA mit einer Sequenz, die in SEQ ID NO: 11 und/oder 16 enthalten ist, und/oder in genomischer DNA mit einer Sequenz, die in SEQ ID NO: 36 enthalten ist, aus einer Probe, die zellfreie DNA aus Blut umfasst, oder aus einer davon abgeleiteten Probe eines Individuums, wobei das Individuum ein erhöhtes Risiko für einen Krebs hat oder vermutet wird, dass es an einem Krebs leidet, wobei der Krebs ausgewählt ist aus der Gruppe bestehend aus Magen-, Leber-, Dickdarm-, Eierstock-, Speiseröhren-, Blasen- und Prostatakrebs, wenn DNA-Methylierung in genomischer DNA mit einer Sequenz, die in SEQ ID NO: 11 und/oder 16 enthalten ist, nachgewiesen wird, und/oder aus der Gruppe bestehend aus Lungen-, Dickdarm-, Brust-, Magen- und Speiseröhrenkrebs, wenn DNA-Methylierung in genomischer DNA mit einer Sequenz, die in SEQ ID NO: 36 enthalten ist, nachgewiesen wird.

2. Das Verfahren nach Anspruch 1, wobei die genomische DNA mit einer Sequenz, die in SEQ ID NO: 11 und/oder 16 enthalten ist, eine Sequenz aufweist, die in SEQ ID NO: 21 enthalten ist, und/oder wobei die genomische DNA mit einer Sequenz, die in SEQ ID NO: 36 enthalten ist, eine Sequenz aufweist, die in SEQ ID NO: 41, vorzugsweise in SEQ ID NO: 46 und noch bevorzugter in SEQ ID NO: 51 enthalten ist.

3. Das Verfahren nach Anspruch 1 oder 2, wobei DNA-Methylierung in einer Ziel-DNA nachgewiesen wird, die in dem Krebs methyliert ist.

4. Das Verfahren nach einem der Ansprüche 1 bis 3, umfassend die folgenden Schritte
(a) Umwandlung von in der 5-Position unmethyliertem Cytosin in Uracil oder eine andere Base, die in der genomischen DNA nicht mit Guanin hybridisiert;
(b) methylierungsspezifische Amplifizierung einer Region der umgewandelten DNA;
(c) Nachweis des Vorhandenseins oder Nichtvorhandenseins der in Schritt (b) amplifizierten DNA;
wobei das Vorhandensein oder Nichtvorhandensein von amplifizierter DNA auf das Vorhandensein bzw. Nichtvorhandensein von methylierter genomischer DNA hinweist.

5. Das Verfahren nach Anspruch 4, wobei der Bereich der umgewandelten DNA unter Verwendung mindestens eines methylierungsspezifischen Oligonukleotids methylierungsspezifisch amplifiziert wird, das im Wesentlichen identisch oder komplementär zu einem Abschnitt zusammenhängender Nukleotide der SEQ ID NOs 12-15 und/oder 17-20 ist, zur Amplifikation umgewandelter DNA, die von genomischer DNA mit einer in SEQ ID NO: 11 und/oder 16 enthaltenen Sequenz stammt, oder der SEQ ID NOs 37-40, zur Amplifikation umgewandelter DNA, die von genomischer DNA mit einer in SEQ ID NO: 36 enthaltenen Sequenz stammt.

6. Ein Verfahren zum Nachweis des Vorhandenseins oder Nichtvorhandenseins von Krebs oder eines Risikos davon in einem Individuum, umfassend den Nachweis von DNA-Methylierung in genomischer DNA mit einer Sequenz, die in SEQ ID NO: 11 und/oder 16 enthalten ist, und/oder in genomischer DNA mit einer Sequenz, die in SEQ ID NO: 36 enthalten ist: 36, aus einer Probe, die zellfreie DNA aus Blut umfasst, oder aus einer davon abgeleiteten Probe eines Individuums, wobei der Krebs ausgewählt ist aus der Gruppe bestehend aus Magen-, Leber-, Dickdarm-, Eierstock-, Speiseröhren-, Blasen- und Prostatakrebs, wenn DNA-Methylierung in genomischer DNA mit einer Sequenz, die in SEQ ID NO: 11 und/oder 16 enthalten ist, nachgewiesen wird, und/oder aus der Gruppe bestehend aus Lungen-, Dickdarm-, Brust-, Magen- und Speiseröhrenkrebs, wenn DNA-Methylierung in genomischer DNA mit einer Sequenz, die in SEQ ID NO: 36 enthalten ist, nachgewiesen wird.

7. Das Verfahren nach Anspruch 6, wobei die genomische DNA mit einer Sequenz, die in SEQ ID NO: 11 und/oder 16 enthalten ist, eine Sequenz aufweist, die in SEQ ID NO: 21 enthalten ist, und/oder wobei die genomische DNA mit einer Sequenz, die in SEQ ID NO: 36 enthalten ist, eine Sequenz aufweist, die in SEQ ID NO: 41, vorzugsweise in SEQ ID NO: 46 und noch bevorzugter in SEQ ID NO: 51 enthalten ist.

8. Das Verfahren nach Anspruch 6 oder 7, wobei DNA-Methylierung in einer Ziel-DNA nachgewiesen wird, die in dem Krebs methyliert ist.

9. Das Verfahren nach einem der Ansprüche 6 bis 8, wobei
(i) das Vorhandensein einer signifikanten Menge an methylierter genomischer DNA mit einer Sequenz, die in SEQ ID NO: 11 und/oder 16 enthalten ist, auf das Vorhandensein von Magen-, Leber-, Dickdarm-, Eierstock-, Speiseröhren-, Blasen- oder Prostatakrebs oder eines Risikos dafür hinweist, und das Nichtvorhandensein einer signifikanten Menge an methylierter genomischer DNA mit einer Sequenz, die in SEQ ID NO: 11 und/oder 16 enthalten ist, auf das Nichtvorhandensein von Magen-, Leber-, Dickdarm-, Eierstock-, Speiseröhren-, Blasen- und Prostatakrebs oder eines Risikos dafür hinweist,
(ii) das Vorhandensein einer signifikanten Menge an methylierter genomischer DNA mit einer Sequenz, die in SEQ ID NO: 36 enthalten ist, auf das Vorhandensein von Lungen-, Dickdarm-, Brust-, Magen- oder Speiseröhrenkrebs oder eines Risikos dafür hinweist, und das Nichtvorhandensein einer signifikanten Menge an methylierter genomischer DNA mit einer Sequenz, die in SEQ ID NO: 36 enthalten ist, auf das Nichtvorhandensein von Lungen-, Dickdarm-, Brust-, Magen- oder Speiseröhrenkrebs oder eines Risikos dafür hinweist
(iii) das Vorhandensein einer signifikanten Menge an methylierter genomischer DNA mit einer Sequenz, die in SEQ ID NO: 11 und/oder 16 enthalten ist, und einer signifikanten Menge an methylierter genomischer DNA mit einer Sequenz, die in SEQ ID NO: 36 enthalten ist, auf das Vorhandensein von Magen-, Darm- oder Speiseröhrenkrebs oder ein Risiko dafür hinweist, und
(iv) das Vorhandensein einer signifikanten Menge an methylierter genomischer DNA mit einer Sequenz, die entweder in SEQ ID NO: 11 und/oder 16 oder in SEQ ID NO: 36 enthalten ist, und das Fehlen einer signifikanten Menge an methylierter genomischer DNA mit einer Sequenz, die in der anderen enthalten ist, auf das Vorhandensein von Krebs oder eines Risikos dafür gemäß den kombinierten Indikationen von (i) und (ii) hinweist.

10. Das Verfahren nach Anspruch 9, weiterhin umfassend die Bestätigung eines indizierten Krebses und/oder die Eingrenzung der indizierten Krebse durch Verwendung eines oder mehrerer weiterer Mittel zum Nachweis eines indizierten Krebses.

11. Das Verfahren nach Anspruch 9, wobei die Menge an methylierter genomischer DNA durch Realtime-PCR oder durch Sequenzierung bestimmt wird.

12. Verwendung eines Oligonukleotids, ausgewählt aus der Gruppe bestehend aus einem Primer, Blocker oder einer Sonde, die eine Sequenz aufweist, die im Wesentlichen identisch oder komplementär zu einem Abschnitt von zusammenhängenden Nukleotiden der SEQ ID NOs 12-15 oder 17-20 ist, oder die eine Sequenz aufweist, die im Wesentlichen identisch oder komplementär zu einem Abschnitt von zusammenhängenden Nukleotiden der SEQ ID NOs 37-40 ist, in einem Verfahren zum Nachweis von DNA-Methylierung für die Diagnose von Krebs, für die Beurteilung des Ansprechens auf die Behandlung von Krebs, für die Überwachung von Krebs oder für das Screening von Individuen, um eine erhöhte Wahrscheinlichkeit von Krebs zu erkennen, wobei der Krebs ausgewählt ist aus der Gruppe bestehend aus Magen-, Leber-, Dickdarm-, Eierstock-, Speiseröhren-, Blasen- und Prostatakrebs für SEQ ID NOs 12-15 und 17-20 und/oder aus der Gruppe bestehend aus Lungen-, Dickdarm-, Brust-, Magen- und Speiseröhrenkrebs für SEQ ID NOs 37-40.

13. Verwendung eines Kits, umfassend mindestens ein erstes und ein zweites Oligonukleotid, wobei das erste Oligonukleotid und das zweite Oligonukleotid Oligonukleotide wie in Anspruch 12 definiert sind, in einem Verfahren zum Nachweis von DNA-Methylierung für die Diagnose von Krebs, für die Beurteilung des Ansprechens auf die Behandlung von Krebs, für die Überwachung von Krebs, oder für das Screening von Individuen zum Nachweis einer erhöhten Wahrscheinlichkeit von Krebs, wobei der Krebs ausgewählt ist aus der Gruppe bestehend aus Magen-, Leber-, Dickdarm-, Eierstock-, Speiseröhren-, Blasen- und Prostatakrebs für die SEQ ID NOs 12-15 oder 17-20 und/oder aus der Gruppe bestehend aus Lungen-, Dickdarm-, Brust-, Magen- und Speiseröhrenkrebs für die SEQ ID NOs 37-40.

14. Die Verwendung nach Anspruch 13, wobei das erste Oligonukleotid eine Sonde nach Anspruch 12 ist, die methylierungsspezifisch ist, und das zweite Oligonukleotid ein Blocker nach Anspruch 12, der methylierungsspezifisch ist, oder ein Primer nach Anspruch 12, der methylierungsspezifisch ist, ist.

15. Die Verwendung nach Anspruch 13, wobei das erste Oligonukleotid ein Primer nach Anspruch 12 ist und das zweite Oligonukleotid ebenfalls ein Primer nach Anspruch 12 oder ein Blocker nach Anspruch 12, der methylierungsspezifisch ist, ist.

16. Verwendung des Verfahrens nach den Ansprüchen 1-5 oder 6-11 für die Diagnose von Krebs, für die Beurteilung des Ansprechens auf die Behandlung von Krebs, für die Überwachung von Krebs oder für das Screening von Individuen zum Nachweis einer erhöhten Wahrscheinlichkeit von Krebs, wobei der Krebs ausgewählt ist aus der Gruppe bestehend aus Magen-, Leber-, Dickdarm-, Eierstock-, Speiseröhren-, Blasen- und Prostatakrebs, wenn DNA-Methylierung in genomischer DNA mit einer Sequenz, die in SEQ ID NO: 11 und/oder 16 enthalten ist, und/oder aus der Gruppe bestehend aus Lungen-, Dickdarm-, Brust-, Magen- und Speiseröhrenkrebs, wenn DNA-Methylierung in genomischer DNA mit einer Sequenz, die in SEQ ID NO: 36 enthalten ist, nachgewiesen wird.

## Revendications

1. Procédé pour détecter une méthylation d'ADN au sein d'ADN génomique ayant une séquence comprise dans la/les séquence(s) SEQ ID NO: 11 et/ou 16, et/ou au sein d'ADN génomique ayant une séquence comprise dans la séquence SEQ ID NO: 36, à partir d'un échantillon comprenant de l'ADN acellulaire provenant de sang ou d'un échantillon dérivé de celui-ci d'un sujet, dans lequel le sujet a un risque accru d'un ou est suspecté d'être atteint d'un cancer choisi dans l'ensemble constitué par les cancers de l'estomac, du foie, du côlon, de l'ovaire, de l'œsophage, de la vessie et de la prostate lorsqu'on détecte une méthylation d'ADN au sein d'ADN génomique ayant une séquence comprise dans la/les séquence(s) SEQ ID NO: 11 et/ou 16, et/ou dans l'ensemble constitué par les cancers du poumon, du côlon, du sein, de l'estomac et de l'œsophage lorsqu'on détecte une méthylation d'ADN au sein d'ADN génomique ayant une séquence comprise dans la séquence SEQ ID NO: 36.

2. Procédé selon la revendication 1, dans lequel l'ADN génomique ayant une séquence comprise dans la/les séquence(s) SEQ ID NO: 11 et/ou 16 a une séquence comprise dans la séquence SEQ ID NO: 21, et/ou dans lequel l'ADN génomique ayant une séquence comprise dans la séquence SEQ ID NO: 36 a une séquence comprise dans la séquence SEQ ID NO: 41, de préférence dans la séquence SEQ ID NO: 46, et de façon plus particulièrement préférée dans la séquence SEQ ID NO: 51.

3. Procédé selon la revendication 1 ou 2, dans lequel la méthylation d'ADN est détectée dans un ADN cible qui est méthylé dans le cancer.

4. Procédé selon l'une quelconque des revendications 1 à 3, comprenant les étapes consistant à
(a) convertir une cytosine non méthylée en la position 5 en uracile ou une autre base qui ne s'hybride pas avec la guanine dans l'ADN génomique ;
(b) amplifier de façon spécifique de méthylation une région de l'ADN converti ;
(c) détecter la présence ou l'absence d'ADN amplifié dans l'étape (b) ;
dans lequel la présence ou l'absence d'ADN amplifié est indicatrice de la présence ou de l'absence, respectivement, d'ADN génomique méthylé.

5. Procédé selon la revendication 4, dans lequel la région de l'ADN converti est amplifiée de façon spécifique de méthylation par utilisation d'au moins un oligonucléotide spécifique de méthylation qui est sensiblement identique à ou complémentaire d'un segment de nucléotides contigus de séquences SEQ ID NO: 12-15 et/ou 17-20 pour amplifier de l'ADN converti dérivé d'ADN génomique ayant une séquence comprise dans la/les séquence(s) SEQ ID NO: 11 et/ou 16, respectivement, ou de séquences SEQ ID NO: 37-40 pour amplifier de l'ADN converti dérivé d'ADN génomique ayant une séquence comprise dans la séquence SEQ ID NO: 36.

6. Procédé pour détecter la présence ou l'absence d'un cancer ou d'un risque de cancer chez un sujet, comprenant le fait de détecter une méthylation d'ADN au sein d'ADN génomique ayant une séquence comprise dans la/les séquence(s) SEQ ID NO: 11 et/ou 16, et/ou au sein d'ADN génomique ayant une séquence comprise dans la séquence SEQ ID NO: 36 à partir d'un échantillon comprenant de l'ADN acellulaire provenant de sang ou d'un échantillon dérivé de celui-ci d'un sujet, dans lequel le cancer est choisi dans l'ensemble constitué par les cancers de l'estomac, du foie, du côlon, de l'ovaire, de l'œsophage, de la vessie et de la prostate lorsqu'on détecte une méthylation d'ADN au sein d'ADN génomique ayant une séquence comprise dans la/les séquence(s) SEQ ID NO: 11 et/ou 16, et/ou dans l'ensemble constitué par les cancers du poumon, du côlon, du sein, de l'estomac et de l'œsophage lorsqu'on détecte une méthylation d'ADN au sein d'ADN génomique ayant une séquence comprise dans la séquence SEQ ID NO: 36.

7. Procédé selon la revendication 6, dans lequel l'ADN génomique ayant une séquence comprise dans la/les séquence(s) SEQ ID NO: 11 et/ou 16 a une séquence comprise dans la séquence SEQ ID NO: 21, et/ou dans lequel l'ADN génomique ayant une séquence comprise dans la séquence SEQ ID NO: 36 a une séquence comprise dans la séquence SEQ ID NO: 41, de préférence dans la séquence SEQ ID NO: 46 et de façon plus particulièrement préférée dans la séquence SEQ ID NO: 51.

8. Procédé selon la revendication 6 ou 7, dans lequel la méthylation d'ADN est détectée dans un ADN cible qui est méthylé dans le cancer.

9. Procédé selon l'une quelconque des revendications 6 à 8, dans lequel
(i) la présence d'une quantité significative d'ADN génomique méthylé ayant une séquence comprise dans la/les séquence(s) SEQ ID NO: 11 et/ou 16 est indicatrice de la présence de cancer de l'estomac, du foie, du côlon, de l'ovaire, de l'œsophage, de la vessie ou de la prostate ou d'un risque d'un tel cancer, et l'absence d'une quantité significative d'ADN génomique méthylé ayant une séquence comprise dans la/les séquence(s) SEQ ID NO: 11 et/ou 16 est indicatrice de l'absence de cancer de l'estomac, du foie, du côlon, de l'ovaire, de l'œsophage, de la vessie et de la prostate ou d'un risque d'un tel cancer,
(ii) la présence d'une quantité significative d'ADN génomique méthylé ayant une séquence comprise dans la séquence SEQ ID NO: 36 est indicatrice de la présence de cancer du poumon, du côlon, du sein, de l'estomac ou de l'œsophage ou d'un risque d'un tel cancer, et l'absence d'une quantité significative d'ADN génomique méthylé ayant une séquence comprise dans la séquence SEQ ID NO: 36 est indicatrice de l'absence de cancer du poumon, du côlon, du sein, de l'estomac et de l'œsophage ou d'un risque d'un tel cancer,
(iii) la présence d'une quantité significative d'ADN génomique méthylé ayant une séquence comprise dans la/les séquence(s) SEQ ID NO: 11 et/ou 16 et d'une quantité significative d'ADN génomique méthylé ayant une séquence comprise dans la séquence SEQ ID NO: 36 est indicatrice de la présence de cancer de l'estomac, du côlon ou de l'œsophage ou d'un risque d'un tel cancer, et
(iv) la présence d'une quantité significative d'ADN génomique méthylé ayant une séquence comprise dans soit la/les séquence(s) SEQ ID NO: 11 et/ou 16, soit dans la séquence SEQ ID NO: 36, et l'absence d'une quantité significative d'ADN génomique méthylé ayant une séquence comprise dans l'autre est indicatrice de la présence de cancer ou d'un risque d'un tel cancer selon les indications de (i) et (ii) combinés.

10. Procédé selon la revendication 9, comprenant en outre le fait de confirmer un cancer indiqué et/ou de restreindre la diversité des cancers indiqués en utilisant un ou plusieurs moyen(s) supplémentaire(s) pour détecter un cancer indiqué.

11. Procédé selon la revendication 9, dans lequel la quantité d'ADN génomique méthylé est déterminée par PCR en temps réel ou par séquençage.

12. Utilisation d'un oligonucléotide choisi dans l'ensemble constitué par une amorce, un bloquant ou une sonde, qui a une séquence qui est sensiblement identique à ou complémentaire d'un segment de nucléotides contigus de séquences SEQ ID NO: 12-15 ou 17-20, ou qui a une séquence qui est sensiblement identique à ou complémentaire d'un segment de nucléotides contigus de séquences SEQ ID NO: 37-40, dans un procédé pour détecter une méthylation d'ADN pour le diagnostic d'un cancer, pour l'évaluation de la réponse à un traitement d'un cancer, pour la surveillance d'un cancer, ou pour le dépistage de sujets afin de détecter une probabilité accrue de cancer, le cancer étant choisi dans l'ensemble constitué par les cancers de l'estomac, du foie, du côlon, de l'ovaire, de l'œsophage, de la vessie et de la prostate pour les séquences SEQ ID NO: 12-15 et 17-20 et/ou dans l'ensemble constitué par les cancers du poumon, du côlon, du sein, de l'estomac et de l'œsophage pour les séquences SEQ ID NO: 37-40.

13. Utilisation d'un kit comprenant au moins un premier et un second oligonucléotide, dans laquelle le premier oligonucléotide et le second oligonucléotide sont des oligonucléotides tels que définis dans la revendication 12, dans un procédé pour détecter une méthylation d'ADN pour le diagnostic d'un cancer, pour l'évaluation de la réponse à un traitement d'un cancer, pour la surveillance d'un cancer, ou pour le dépistage de sujets afin de détecter une probabilité accrue de cancer, le cancer étant choisi dans l'ensemble constitué par les cancers de l'estomac, du foie, du côlon, de l'ovaire, de l'œsophage, de la vessie et de la prostate pour les séquences SEQ ID NO: 12-15 et 17-20 et/ou dans l'ensemble constitué par les cancers du poumon, du côlon, du sein, de l'estomac et de l'œsophage pour les séquences SEQ ID NO: 37-40.

14. Utilisation selon la revendication 13, dans laquelle le premier oligonucléotide est une sonde telle que définie dans la revendication 12, qui est spécifique de méthylation, et le second oligonucléotide est un bloquant tel que défini dans la revendication 12, qui est spécifique de méthylation, ou une amorce telle que définie dans la revendication 12, qui est spécifique de méthylation.

15. Utilisation selon la revendication 13, dans laquelle le premier oligonucléotide est une amorce telle que définie dans la revendication 12 et le second oligonucléotide est également une amorce telle que définie dans la revendication 12, ou un bloquant tel que défini dans la revendication 12, qui est spécifique de méthylation.

16. Utilisation du procédé selon les revendications 1 à 5 ou les revendications 6 à 11, pour le diagnostic d'un cancer, pour l'évaluation de la réponse à un traitement d'un cancer, pour la surveillance d'un cancer, ou pour le dépistage de sujets afin de détecter une probabilité accrue de cancer, le cancer étant choisi dans l'ensemble constitué par les cancers de l'estomac, du foie, du côlon, de l'ovaire, de l'œsophage, de la vessie et de la prostate lorsqu'on détecte une méthylation d'ADN au sein d'ADN génomique ayant une séquence comprise dans la/les séquence(s) SEQ ID NO: 11 et/ou 16, et/ou dans l'ensemble constitué par les cancers du poumon, du côlon, du sein, de l'estomac et de l'œsophage lorsqu'on détecte une méthylation d'ADN au sein d'ADN génomique ayant une séquence comprise dans la séquence SEQ ID NO: 36.


### SEQUENCE LISTING

<110> Epigenomics AG
<120> METHODS FOR DETECTING CpG METHYLATION OF TUMOUR-DERIVED DNA IN BLOOD SAMPLES
<130> 536-152 PCT
<150> EP 16 153 452.4
   <151> 2016-01-29
<160> 63
<170> PatentIn version 3.5
<210> 1
   <211> 34993
   <212> DNA
   <213> Homo sapiens
<400> 1
<210> 2
   <211> 34993
   <212> DNA
   <213> Artificial Sequence
<220>
   <223> C to T (bisl)
<400> 2
   ggataagaat tttttttaat tagaaggtaa tttttaggtt tttttttttt aaggattttt 60
   attaaagttt ttaggaaaat tgaggagatt ttgagttagg ggtttagttt gagttttgtt 120
   tatagtaagt gatttttatt gaggtatgaa aataggaagt aaaggagaga ggtttgtttt 180
   tgggtttagg tttttttttt tttttttttt ttgagacgga gtttcgtttt gtcgtttagg 240
   ttggagtgta gcgggtttag gttttttaag ggattttttt ttaattaggg taatagaggg 300
   atgtttaagg tgatcgttaa tatttttttg gggggttgat tttagggttt aggaggaagg 360
   gattagtttt tttcgttaag agggggagtt ttagtatgta tttttgggtt ggttttattt 420
   aaatagtgag gtattttagg tgtggagatt tttttttttt tattttaaat attattgggt 480
   tagggggttg ttagttagtt atttttagta gtttaggtag gaagtcggaa atgatgtagt 540
   ttaaattttt tttggtgggg ttttttgtaa gtggggagtc gtttagggta ggattataaa 600
   tgggttgatt tgaggtttgt ggttttatcg gagaagttag gtatggaagg ttgattatat 660
   atttacggtt aatggtttta gagttttaag ttatgggtag attagatgtg gtgagtgatt 720
   ttggttatgg tagtaattaa gattaggatg taggagtgtg gtttaagggt ttgtttggta 780
   ttttttttta gtttggggga ggggtttttt ttttttttta ggtgatttga gaagaatggt 840
   aggtaaagga tattgttttg gtttgggtag tcgttattgg tgttagttta tttttggatg 900
   atatagatgg gacgtttatt ttaattattt tgtttttttt ttttgtgtaa gtgtttttgg 960
   gattatttag attgtagttt aaggtgaagg gatttttgcg ttaagaagtt ttttggatta 1020
   aatttgggac ggaaaggata ggaattgtaa gggaattcgt tagttttttt tttgcgtttt 1080
   tggttttatc ggtagggtat gggtttgtat gtgtggagag gtagtaggcg tgattttggg 1140
   attacgttaa ttagtgttaa ttttatggag gtttttatta cggtgaattt ttgttattat 1200
   ttgaagttat tagtggtttt tagggttagt aggtttgtat atataggtta gttttaggtt 1260
   tttttttttt gagttaaggg ttagattttt attagattaa gggaaaagtt tatattatga 1320
   aggtaggcga gagtagtttt tggggtagag ttagaatggt ggttattttt ggggtgtacg 1380
   gattgggagg gggtatttgg aagtttttta aggtgatgga aatgttttat aatatgtttg 1440
   tttaatttgt agtttatatg cggtttaggt agttttgaat gtggtttaat ataaatttat 1500
   aaattttttt aaaatattat gagatttttt tgtgatttta ttttttattt ttatttttgt 1560
   ttgttttatt tagtttatta gttattatta gtgttagcgt attttaggtg tggtttaaga 1620
   aaattttttt tttaatgtgg tttagggaag taaaaagatt ggatattttt attttataat 1680
   atgattagag tagtagttat atgggtatat atatatgtaa aagtttatta ggatgaatat 1740
   ttaggtgtag gtattgatat atataaattt tatttttata agggaggaag aggagtttaa 1800
   tatttgtttt attttgtaat ttatattatg aagagataaa ttagtttgta gtttgtttcg 1860
   taatatgttt tcggttttgt ttattagaga tatttatagg ataggtattt tgaggttttt 1920
   atttttattt ttagggttgt taaagtggaa aaggttgaga tagaagttta tggggtgagg 1980
   agaggttttt gattgagatc gtgtggcgag gtgggttaag tttaaggaaa ggggaagatg 2040
   tatatttttt agggtttttt gttttttttt ttttttggat tttttaggta ggtaggaata 2100
   tatacggtgt ttgacgaggt ggtataagtt gttagagtag ttatgttatg tttagttgtt 2160
   tagtaatata tttatatttg ttttgtgtgt ttatgttcgg gagtagaggg aggatggggt 2220
   tttttgggga taggttgtat tttatttttg atttttatga ttttttaaat taattaagta 2280
   taaaaagttt ttggttttat ttttgtggtt aggattgtag ggtttttatt ataataaaag 2340
   aagtttgtga ttttggggtg gagcgatata ttatggggtt ggtattggat ttggatttcg 2400
   gattgttggg tatttttatg tttttttgtt tgagatgtta taggatttag attaaatatg 2460
   tcgttgtatg ttagggttag tattttaggg tttaggtgtg ggatagtttt aagtagttag 2520
   ttagggtttt aataagtagt taaatattta gggagtgttt gttatgtgtt ggatatttcg 2580
   ttgggtgtta ggtgggttga tgagtaagat tgtttatatt ttttttattt tattattttt 2640
   atttttttat tatttttttg atttttattg tgaattaggg gaaaggaggt atttggggta 2700
   taaatttttt tgatagatga ggatttggga ggaggagttg ggtaatttaa tatatttgat 2760
   gtttattatt tttttttgag tttattgggt atttagaggt tgtggtcgga tatttaacgt 2820
   aatatataaa ggataattgt tttgttggtg ttgtttttta tttttttatt ttagaattag 2880
   attatagatt cggagatggt agttttttaa aaattatttt tattttaatt tttttgtttt 2940
   tgaattgagg aagttgagga ttttatagtg aggaaagttt tgttgggatt atagagtaag 3000
   ttgggaataa aggtaatatt aagttttaga ttttttcgat tgggcgtttg ttaagatttt 3060
   tttttatatt ttatgttatg tttattttac ggagttgtgg aatagtgggg agatatttat 3120
   aaggtaaggt gtatttttag ttttgttatt tttagatttg tttgtagttc ggggaacgga 3180
   gttatagttt tttaggggta tggattttta attttagggt tttttaaatt atgataagtt 3240
   tttaggaggt tttggttata attatttatg gagtattttg tgtttaagtt ttgttaagtt 3300
   atagttttta aatattttag tttggtattt ttagaattat tagagtattt gtgaggttta 3360
   atcggtgggg ttttgggttt gtttaagggg tggttgaaag ttatgggttt ataaattagt 3420
   attttatatt tttattttta attatgttta tagtttcgtt agagtttacg gattttcgta 3480
   gtttgtttag gggtttatta gggagatagt ttttttgtta aagaaagaat ggatgtgggt 3540
   tagagttttt tttttaggtt agttgtagag tttcgttatt ggttgttttt ttttattaga 3600
   agggagtagt tttttggatt attatttttg atttttttag taggtttggg gggttttatt 3660
   ttagtttagt tttaattttg ttaggaaaag gaagggaatt attatgtaaa tatagttagg 3720
   agataggtta ggaagttagt aggatacggg gaatttcggt acggtttttt atttattttt 3780
   ggttagattg ggttagggga tagggagggg ttcgtattta ggatagtttg atgttaagtt 3840
   tattttttta agtaggtttt atcggtgaat aggaatagta gggtttagtc gagttgtttt 3900
   tagttattat tagtaggttt ggggatgttt ttcgagaaga tattcgggtt tagattggtt 3960
   ttgaacgtta ggttcggtaa ggagcgttag ttttaggaat tcgaggtagg gagttttggg 4020
   agtagttatc gtttatttgt ttttgaggag acggtattta ggttttattg gcgtatttgt 4080
   tcggtttttt gttttttgtt ttttattatt tcggtaggcg tatcgattac gcgagtagta 4140
   ggtgtaagac gcgggggtgg tattgagatg atataaaatt atattcgttt ttatttttta 4200
   agttttgacg gttttcgtta cggttttttt gttattttag gttggcgttt tcgtcggata 4260
   tagagatgga ggtggaggag tagttagttg ttcgtttttt ttagatcgag gagcgggagg 4320
   cggcgtcggg aacgtttttt ttcgttagtt tacggttttt agggagtatg ggtattttag 4380
   gtcggttttt tcgttaaggg cgtagtttcg tagtggtcga ggcgggggtt tagtttggag 4440
   gtcgtagttt ttcgtttcgt ttagtttttc ggtttcgtat agcgtttttg ttaggtttcg 4500
   gcgttcgttc ggcggcggtt tggttgtgcg gtttcgtttt cggtttcgtc gcgtttattc 4560
   ggggcgttgt agttcgtata tattttcgtt cgcggttttt ttcgggatat ttttagcggc 4620
   gacgcggtcg tagttttttg ggttagcgtg gggggcgcgg gcggcgggtt cgggcggcgg 4680
   cggcggtttt tgttgttcgg ttcggtttcg gcgaggtttc ggcgaggttt cgcgcgttcg 4740
   gttaatcgtt cgtttcgggg ttggttcgga attcgtcgtg ttcgtttgtt cgttcgtttt 4800
   cgggcgtttt tcgtttcgcg tcgtttcgtc gtcgttcgcg gttttttttt tcgttttttg 4860
   cgcggttttc ggtcgttttt gtaggaagcg gcgtaggttt tgtttatagg gggaggggtc 4920
   gtattttcgt ttcgtcgcgc gttgtcggtc gcgtttttta gagaaaggaa gttcgggcgc 4980
   gggtaggagg gggcgggggc gtttaaaggg gtcgcggtag atgttcggtt tcggcgggta 5040
   gcgtggtttg cggttttggt tttcgttgta gcgttcgttt ggttcggagg taatgtgagt 5100
   tatttttata gttttttttt ttttattttt cgcgtggggt ggatcggggg acgttgggtt 5160
   tacggacggc gagggtgggg attagggaag aaggatttgg aggtttaagt ttcgttttag 5220
   gttaaagggg ttgtgttttg gggtattcgt ttatttttgg ttggggtgag gtgtgcgttt 5280
   ttgtttcgta ggtgtttata tttgtgtgtt taggggtgtg tgtgtatgtg ttatattttt 5340
   gttgaatacg tttattgtta ataatatgga cgtttaggtg ttagaaaggt taggtagtgt 5400
   atttttttgt tttcggatta aaaatatatt tattttttta tgtatttttt tatttagaga 5460
   ggagagaatt tgggcggttt ttaaagattt ttcgaggagt gattattgtg tttgtgtgta 5520
   tttatattta tttgttattc gttttatata tatttacggt gtttttgggt tattagaggt 5580
   gtgtgtgttt agaaagggtg tatataatat agaggggtcg ttttaggtga cggttttttt 5640
   ttttggattt tatacgacga ggtaggtaaa ggtaaggtag gtgagggacg tgggtgggtt 5700
   ggggggtagg gagtttaggt agaggtcgta tatgagcgta gttttcgtat tgagtatgcg 5760
   tttttatacg cgtgtgtcgc gcgtgtattt aatttttaat ttttgtagta gtaaaaggtt 5820
   cgggtatttt gtttgtgggg agagggtaga ggtttttagg gttagatttg ggggttaggt 5880
   tagtggtgat aattgaggag gggtttttta agagttagag atttttgttt gttaagatgg 5940
   gggtagtttt ttggtagggg tgtttaagtt ggtcgattat tttaacggtg ttagagcggt 6000
   agtgatttgt gggtgggtag gttttttttt taggggagat aagtaatgtt tatgagttat 6060
   attattcgaa atatatgtaa agttgttttt atcgtttttt gttgcggttt agattgataa 6120
   gtttgtttga aatcgcgatt tatgggtttt atttataatt gtattttaaa aaaattttgt 6180
   aggttttttt tttttcgata gttagtcgtt tagttggtgg ggtttttgta agtaacgata 6240
   tttagttggt gtgtaggggg gagtattatt tgtcgtttcg agttgttttt ttattagagt 6300
   ttatgggttt ggtggtttta gcgggaaggg ggtaaggggc ggggggagtg agacgggagg 6360
   agtggttagg tagttcgtgt taggcgttta gtttacgagc ggtggtcgtt cgtttatttt 6420
   cgatcgattt ttagttatgg aattttattt attaaatgtc gaggaggttg ttggtagttt 6480
   tgtcgtttgg ttgtttttgt tttttcgttg tgtgggcgcg tttcgagtac gtggtagttg 6540
   agagcgcggg tcgaagcgta agcgatttgg gtttagtgtt tatcgtcgtt tggtaaaaaa 6600
   agaatacggt cgtttagggg atagtttttt ataatcgata attttttata atttattttg 6660
   aatttttttt gttttcggat ttttgggttt tgttggggaa gaaggatgat ttattttgtt 6720
   tattttttta ttttattttt tattttttgg ttggtttaga gattatttgg gagtggtttt 6780
   ggcgcggata tatcgtttaa attttgtttt ttttagtttc gttatagtta aatttttggg 6840
   tttgggattg gagataaaag ggtttttgga ttttttcgtg gagatgttat tattttagga 6900
   agtaggattg ttatagtgga attatatata aggttcgaaa aataatttaa taaggaaatg 6960
   tataggtatg ttttcggtta tttttagatg ttttttaatc gcgttagttt ttttttttgt 7020
   attggttgta gttacgatat tttgttgatt tttggtggtg taggcgggta gcggtttcgg 7080
   gttttcggag acgttagttg gtttttttta ggcgggagaa aggggcgagg tagttcgttt 7140
   tttttttatt ttttttcgtt ggggtttggt attatagggt gtggaggagg aaggagggaa 7200
   gattatataa agaaatttga gagagtttgt aagtttttgt tggtattttt tatataaata 7260
   ttttttggat tgttgtttta agtagggtcg gtgtggaatt ttttattttt gttttgtttt 7320
   gaggtgggtt taagaaggta tcgtgtttgg tttcgttatt tgtttcggtt cgtttggttg 7380
   ggggagggag gcgttgtttt tagggtagtt attattttta gtttatttta ggaggtagta 7440
   tttagatttt tattttgaag gggagtggat cgagaaagtc ggaaaaatta agtagtttgt 7500
   ttttttaagg ttaggtagtt ggagttattc gaattttggt ttttttgata ttatagtttt 7560
   ttttttttag ggattggttt aagttttttg gtttttttag gttttgtaat tggcgtttga 7620
   gaggttaatt taatttttat tatatttttt gtagggatta ttttggaagt atttttttat 7680
   tttggggtta tttaggaagg agatagtttt tggaattagt tatttttttt tttgaagggt 7740
   tagttgggtg gaggaacgga gttgttattt gttgtttttt ttgtttaatt ttattttggt 7800
   ttttgttggg gatagtgtgc ggggggaaag ggattatttt tttcgcgtta ttggatattt 7860
   tgtttttaat gatatgtgtg gaagatggag tttaattttt taatagggta gggaagattt 7920
   tttagaaatg ttttaggggt tagatttttg gttgttttaa tgatttttgg tattgatgtt 7980
   ttatattttt tttaggagtt tttaattaga cgtttttttt tgggaattcg gatttttttt 8040
   tatggttttt gggatttaag tttaattttg aagattatat ttttatttcg ttttttaaac 8100
   gttcgattta ggttcgtgtt tttttatttt tttagtaaaa ggtaaaatgt tagcggggga 8160
   ggtgcgtttt agtttatttg gcgttagggt ttcgttcgcg tttgggttag gtttgatttt 8220
   tcgtttattt ttttacgaga ttcggggata gtgtagattt ttttcggtta tttgttattt 8280
   ttagttttac gaggcggatt ataaatttta tgatgtcgcg gggttgagtt gataagtttt 8340
   attcggggcg tttggttttt tttttttttt tttattattt ttatattttc gtatttcgcg 8400
   tcggtcgttc gggttgaggg tcgggttatg tagttgtggg gattcggggg ttgcgggcgc 8460
   gcgtttttgc ggcggcgttt tcggggttcg cgtttcgtgc gttttcgcgt tcgttgcggg 8520
   cgtttgtttt tttcgtcgag cggcgttttt gtgtgcgggg gtgtgggagg cgagcgcgag 8580
   ttcgcgtagc gtcgtcgagt gttcgttttt ttttagggcg ggtgggggtt ttttcgcgtc 8640
   gttcgtcgtc gtcgtttcgc gaggacgttc gtcgttcgcg tcgttcgtat cgcgtcgggc 8700
   gcgtcgtttt cgttcgtcgt cgttcgtata tgttcgagtc gtagtttcgc gagtaggtag 8760
   cgtcggtttt tcgtttcgcg gtttcgggtt tcggtttcgg cgtcgttttt ttttttcggt 8820
   cgggcgtcgc ggtttcggta tgaggagcgg gcgatgattt tcgttaacgt tttcgttagg 8880
   aaggggttcg agggtaagta ttcgttgtat tatttcgtgt ggtataatcg ttatcgcgag 8940
   ttggagaagg aggttcgcgc gggttaggta ggagcgtttt cggggcgtcg cggggatttc 9000
   gcggtcgggt acgttcgttc ggtttgggga gggggtgcgg cgcgggttgt ttcgtttttt 9060
   tggcgagttt gcggcgcgtt tttgtttgcg gtcgcggttt ttgtgtttgt tgatcgggaa 9120
   agggtgattt ttttcggtcg ggacgggcgg gattatacgt gtttcggatt tatttagatg 9180
   agagtagagg tttttgtgtt tttttttcgt cggggtagtg agggttgtag ggaggggttg 9240
   cgggttgtgt cggtgtttta tgtggtggga gagttcgttt tagttagttg agcggcgata 9300
   tttttcgagt gagggtgcgg aggtgggaaa ggtttgcgtg gggagggggt tataggttaa 9360
   gaatggggaa gaggttgtgt tatattttta ggtattttag ttttatttag cgtttttttt 9420
   ttaacggtga gcgttttttt ttttagtttt taaattgggt agatttttgg attttttaga 9480
   gtttagtttt agtttaggaa tagggagtgg agaaagattt ttttgggagg ttttaagggt 9540
   tttgtttagg tgttgaaaag cgtttttttg ttttttggtg ttggggtttg gtttttaggc 9600
   gtagaggaag ttgaggggtt ttggttgttg gaaggatttg aggttttgcg tattaggtgg 9660
   gttttttggt ttttgttggt tagtaaggtt acgtgggtta ttagttttgg gttgggtagt 9720
   ggtttttaga aaatagggga aggggggatg ttttttggtt atgttttagg atgttttttg 9780
   gttatgtttt agaataaagg taggtatagg ttatagattg ttatttttgt ttttggtatt 9840
   taggttatag gtaggtattt atggtagaga tgatttttcg tttttagggt gggttgtaag 9900
   agttagagtt gggttcgggt tttatggttt gttgtaggtt gagaatttta gttttggatt 9960
   gggtttgaga ttttggggtt atgggttggg ggtggttagc gtagttgttc gttgtgggaa 10020
   tttatttttt gagggttgtt tattgagtta tgagaagttt agtttgagga gttgtgtaat 10080
   cgttaaggga agtatggagg tggagggtag gttatcggat tgagtaggtt gggggttagg 10140
   atgtgttttt gaagttttac gttgagttag tttttaggtt tgtatggtat ttagttttgt 10200
   atggtagttt ttgtaaatat gcgagtgatt tgtgttcgta ggtttttggg tcggtttggg 10260
   tggttatttt ttgttatttg tgtttatgtt agtatttttc gagttttttt ttagattgaa 10320
   ttttttagat tttgtttttg tttatttatt ataaaggtag tgttttgtag ttacgagaag 10380
   tttgttaaag agagtgggtt atagtgtttt taggaaatag atgaataaat agggtattaa 10440
   tttttgtttt taaggagttt ttgttttggt gaagtaagtg tgaataggaa attttagggt 10500
   gatgtgtaaa gcgttaagat atggttagtt agtttttgtt ggtttttttt tgtttttttt 10560
   ttttgaaagt aaaagaagat aagttttatg tgggaagaat tttttagggg tttaaggatt 10620
   attaggtaga aggttgaatt tttagaatgt tttagtttta tttttttgtt tgagggtggg 10680
   tggttttttt attgtagaga ttggagagag gtagaatgtt ttgggagaaa agattttttt 10740
   agtaggtttt tttgttgata attttgaagt gggtggttat tttgttgggg taaggttggg 10800
   gtggggggta tcggagtcgt tgtaggtaga ggtgttgggg gttgtttatg tgtttttttt 10860
   ttttattttt ttggttgttt tagaaataag gttttagatt tatttttatt tttgttttag 10920
   tgagttaatg tttttttgaa atggatattg tttgttttgg atttaggtta gggggaatat 10980
   agggtattgg tggaggttaa tttttgttta attatagagg agttaggtta gttttttttt 11040
   ttttttaggc gtgttaaaaa gaggaatgtt atttcgtgta ggtgggagag ttttttagtt 11100
   gttttttttt tcgaggattg gtttagagtg aggtttgtat ttagatgtat attgtttatt 11160
   ttttagggta ttaggttttg ttttttgtaa gggggatttg gggatagtta tttaatatta 11220
   tgagaaaagt attgtggaat tttggtattt tagttagggg tttttggttt ttagtttttt 11280
   tggtattagt cgttatagaa atagttcggt tttttttaag tatttaggag ttaggttttt 11340
   ttggttttgg tttggtatag ttgttttttt tttattttaa gaaagtttcg tagaggtttt 11400
   tcgtgggtag gtttggtagt gtagttggtt gaggaggggg ttagtatggt tatttttaga 11460
   gtaggttaat gggttttgtt tagttttaaa ttgttttgtt ggaggttgga agtagggagt 11520
   gatgggtaga taggaggtgt ttggtgaagg gaaggggtat ttagtagtgt tgtgatgtta 11580
   gttcggtgtt ttgtttgtcg ttatgtatgt tttttgttag gtcggggggg atagggttgt 11640
   aagttaggga atatgttagt ttttttttgt ttggtatggt tagttgggta tggttttggt 11700
   tttggagtcg tgagtttttg gtgttttttg gggtgggaat agggggaagg gttagtttgt 11760
   gtagttttcg tgggagggat atgtgtgggt tgggtttggg gagtaggatt ttgtttttgg 11820
   taattagaat tttgaagtgg gtagattgtg gttttagaga attatttttt ttttttttgg 11880
   ggcgtttttt atcgtgggtt tgttttttgt tttttttgtt tatttttttg atttagaggt 11940
   aggggagatg ggttgtgggg tttaggatgt gcgaggagta ttttagattt ttttttttgt 12000
   ttatattggg tagttttgga tgtggggtat tattatagtt ggtaagttag gaagttcggg 12060
   agagggagtt ataaatttta gaaattaaaa attaatgtgt tttgggatga gggggagggg 12120
   ttgtaagggt aggatgggag gggtgatatg gagggagaag tttagggtgc ggtttttata 12180
   gttcgtggtt ttttacgtag tttgaggatg tattttgttt tttttttttt gaatgagttt 12240
   aggtagattt gtttttgtat tttacgttgg gtaggagagg ttaagtaacg attatattag 12300
   atttttgttt tttttttcgt gggttgggga aggggttgag taggtattat tttgagtata 12360
   ggttttgcgt tttttatttt tttcgtaagt attgtgttta gtttatatat gagatgtatg 12420
   ttttttatat tttttttaat aaaaggattt tgtttatgtt aaaggagttt ggaaagtatt 12480
   ttgttagtgt atatgggtgt gaggggtgtt tttgtgtaaa tacgtatata tacgttatat 12540
   aggagtggat ttttgtaggg ttagaaggta gttgggtatg tatgtgtttg tatatttaag 12600
   ggtatacgtg agtatttatg agcgtacgag tgtgtatttg tatggtatta gtgtgtatta 12660
   gagtgtggtg tgtgtgtttg agtggaggta gggaggtttt ttgtttaggg atttttagtt 12720
   ataaaatttt aaagatttta ttttttgatt ttagttgagg tttgttggga taaggaattt 12780
   tcgttttttt ggtggggagg gttgggtgtt gggttttatg ggaaattttt ttattttagt 12840
   aattaaatag ttagaggttt ttgttgagga gaagttggag gtttttgttg aggagattaa 12900
   tgaatttttt tatttgggtt tagggtaagg tgttatttat attgtaggcg gtattttggg 12960
   tttgtgggga gagttgtttt ggagtttggt tgagtgtatg gggttttgta tggggtgtat 13020
   ggggtgttga gttatttagg tttttttggg gagtgttttt gaggtgtggg gatggggagg 13080
   agtattttgt ttgtaaggaa gtgttagtcg ttttttggag tttggagttg aggtatttga 13140
   tggggaggtg gaggtagtat ttggagtttt gaggttaggt agcggtttgt tagtttttta 13200
   gggttttatt agttgtagcg gttgcggttg tagtatttgg ggtagggttg ggaggtttgg 13260
   attgtttagt atttttttag ttagttcggg ttttgtggtt tttttttatt ttcgttgtcg 13320
   ggtttaggat tttatttagg ttgtaagttt cggagtttag tttaattagg attggataag 13380
   agtaaataat attttttttt tttgtgaggg gttatagttt agaggttaat aggggttagg 13440
   ttcgttggtt tttttgaggt ttttagtata ttgaaaaatg aagaaatttt aggataaggt 13500
   tataaaaatt atataaataa ttttaaatta tttaaaatgt tttatttttg ataatggagt 13560
   ttatggttat gttttaaaat atttaattgt tgttagaaga tgcgtaatga aaagtagtgt 13620
   tttttgtttc gtttttttta tttttttagt attttggatt ttttttgtta tttatttttt 13680
   taattataaa taatatattg tttatattgt tttttttttt tttttttttt tttttttttt 13740
   tttttttttg tttttttttt tttttttttt tttttttttt tttttttttt ttttttatga 13800
   gatagcgttt tattttgtta tttaggttgg agtgtagtag tgtgattata gtttagtgta 13860
   gtttttgttt tttaggttta ggtgattttt ttattttagt tttttaagga gttaggatta 13920
   taggtggtgt tattatgttt ggttaatttt tgtatttttt tttgtagaga tggggttttg 13980
   ttatgttgtt taggttggtt tcgaattttt gagtttaagt gatttattta ttttggtttt 14040
   ttaaagtgtt gggattatag acgtgagttt ttcggtataa ataatatatt tttgtaatga 14100
   ttttttggtt aattattttt ttttttttgt gtttttttag tttagtattt tatgtatttg 14160
   tttagtaaat atttatgtgt taggtgttgt ttggggagtg gggaattagt agtgaataaa 14220
   gtaggtaggg atttggttga tggggttttt gttcgaggtg taggaggtgt ttaataggta 14280
   tttggacggg tagtggatag agttatagta gttgttattt tagatgggtt gttagggtgg 14340
   gtttttttaa ggaggtgata tttgagtagt agtttgagtg aagggaggga gaaagttttg 14400
   gggttttttg gaggattttg gagaacgttt cggggaaggt tattggcgaa gggtggagta 14460
   aatgagttta aagagaagtt tggggttatt ggattttttg ttatttacga tggaaagtta 14520
   ttggaagatt ttgaattaaa gagtgttagg atttgagtaa tattttatag ggtgatttta 14580
   gttgttatag gtgaggtgga taatagagtt tggtaatcgg ggagtagtaa agaggtagta 14640
   gggggttgtt agtgagagat gggggggtaa tagttgtaag gtagtggaaa ttgggcggtt 14700
   tttaggtttg ttttggaggt agagtttata agtttttttg atgggttaga tgtggagggt 14760
   gcgggaaaaa gaagagatgt agatagtttt aaagttggtt ttttttttta tttgaaaatg 14820
   tagtaaattt aggtgatgtt tattgtttat gggggtaggt gtagttgttt aggaatgtag 14880
   aaaataagga aaagttaaga aagttatttt tagaatgaag aaggtaagag aaagggtagg 14940
   agacgttatg gaaagttagg ttgttttatt ttgttttatg aatttaatga tttttttata 15000
   ttttttttga ggatgtaaat tattgttttt ttttaaagag ttttttttta ttttttgtat 15060
   taagagaatt ttttttgggt ttttagtatt taatttgttt tttttttttt tttttttttt 15120
   ttttttgata cggagtttcg ttttgttttt cgcgttagag tgtagtggcg taatttcggt 15180
   ttattgtaag tttcgttttt cgggtttata ttattttttt gttttagttt ttcgagtagt 15240
   tgtgattata ggcgttcgtt attacgttta gttaattttt tgtattttta gtagagacgg 15300
   ggttttatcg tgttagttag gatggtttcg attttttgat ttcgtgattc gtttatttcg 15360
   gttttttaaa gagttgggat tataggcgtg agttattgta tttagtcgtt ttagtattta 15420
   atttgtatgt tttggtttat tttatatggg aagtttttat taagtgttta gttttttttg 15480
   gttgttgggt tatatttaag aatggggttg gggagtttag ggatgtgtgg gtgttattgt 15540
   aaggtgatta ggtggtaggt tggtttgttt attgtagggt tattagatgt ttgtattttg 15600
   aggtttttgg gataattttt tattcgggta gatagggtgg tggtggtgtg atttggttgt 15660
   tagtggttta ggatttaggt agggaagggg ttggggtttt acggattatt ggtttttagt 15720
   attatatttt attttttgtt gtggttaatg tttttgagtt tagagtattt tatgtttaat 15780
   ttttataaag aataaatttt tggtgttttg tggtgaggaa ggggggtatt ttttagttta 15840
   atgaggttgg gaaggggatt aggggatttt gaattgtaat tagttttttt gtatttagtt 15900
   ttagttttat ttttattttt gttattttta ttgtttgtag gttttattta tattttttag 15960
   gggggttggg ggttatttgg tttgtttttt atgtggacgt ttttttttgt ggttgtttta 16020
   gttttagttt tttttgtttt tttaagttag ttgtgaggtt ttagttattt tttatgtttt 16080
   aaagttgtgt tgaagttttt tatttgttga tatttttttt ttttattttt gtgggtttta 16140
   aatattttat tgattttttt tttttttttt ttttgagata gggtttggtt ttgttgttta 16200
   ggttggagtg tagtgggatg atttttgttt attgtaattt ttggtttttt ggtttaaggg 16260
   atttttttat tttagttttt tagtagttgg gacgataggt atgtattatt acgtttggtt 16320
   aattttagta tttttttgta gtatagagtt ttattatatt gtttacgttg gttttaaatt 16380
   tttgggttta agtgatttgt ttatttcggt tttttaaagt ttataggtat gagttattgc 16440
   gtttagtttg atattttttt tttttttttt tgagatagag tttttttttg ttatttaggt 16500
   tggagtgtag tggtgcgatt tttgtttatt gtaattttta ttttttaggt ttaagcgatt 16560
   tttgtgtttt agttttttta gtagttggga ttataggaat tttttattac gtttagttaa 16620
   tttttgtatt tttagtagag atggggtttt attatgttgg ttaggttggt tttaaatttt 16680
   tgatttttgg tgatttgttc gtttcggttt tttaaagtgt tgggattata ggtatgagtt 16740
   attgtgtttg gttttttgat atttttatat atttaatatt ttttggtatt ttttaatttt 16800
   ttgtatagat ttaagttttt atttgggatt attttttttt tatttgaaga atttttttta 16860
   atattttttg tatatttggt aataaatttt tttagaattt gtttgtttgt taatatattt 16920
   attttttttt tagttttgaa gaatattttt atcgggtatg gaattttagg ttaatattag 16980
   ttatattgga ttagagaaaa aaaaaaaaag aattttaggt tgaaagtttt tgtttttgtt 17040
   tttttttttt agtattttaa agattttatt atattgtttt ttggtttata tagtttttga 17100
   tgagaagaat atagtatttt ttgtatttgt ttttttatat ataatggatt ttattttttt 17160
   gaatgttttt aggatttttt ttttattatt agtttttagt atttgattat gatggatttt 17220
   ggtgtagttt atttcgtgtt tgttttattt ggggtttatt gaattttttg gtttttggtt 17280
   ttattttggt attagttttt ataataaatt attataaatt aggtgggtta aaataataaa 17340
   tatttggtcg ggtgtggtgg tttacgtttg taattttagt attttgggag gtcgaggcgg 17400
   gcggattacg aggttaggag atcgagatta tgttggttaa tacggtgaaa tttcgttttt 17460
   attaaaaata taaaaaatta gtcgggcgtg gtggtgggtg tttgtaattt tagttatttg 17520
   ggaggttgag gtaggagaat ggcgtgaatc ggggaagcgg agtttatagt gagttgagat 17580
   tacgttattg tattttagtt tgggcgatag agtgagattt tattttaaaa aaaaaaaaaa 17640
   aaaatttatt ttgttatagt tcgggagata taaatataaa tttgagatgt tggtagggtg 17700
   gtagtttttt taaaggtttt agggaagagt tttgttttgt tttttttaga ttttggtgat 17760
   tgggttataa tttttttggt ttgtggttgt agaattttag tttttgtttt cgtttttata 17820
   tggttttttt atttttttga gtgttttaga aagatatttg ttattggatt tagggttttt 17880
   tgggtgattt aggattattt tattttgaga tttttaattt aattatgttt gtaaagattt 17940
   ttttttgaaa taaggtttta tttataggtt gtgagggtta gaatgtgatg tggatatgtt 18000
   ttaggagggg tattattttt taatttatta tagtagtttt tattaagttt ggaaaaattt 18060
   tggacgtttt tttttttttt tattatttat ttatttattt tgaaatggag tttttttttg 18120
   tcgtttaagt tggagtgtag tgatacgatt tcggtttatt gtaatttttt tttttcgggt 18180
   ttaagcgatt tttttgtttt agtttttaag tagttgggat tataggtgtt tattattacg 18240
   tttggttaat ttttgtattt ttagtaaaaa tggggtttta ttatattggt taggttggtt 18300
   ttaaattttt gatgttgtga tttattcgtt ttggtttttt aaagtattgg gattataggc 18360
   gtgagttatc gtattcggtt tattttttta aatttgtttt atttgtttta tttttttatt 18420
   ttgttttttg ggattttaat agatatatgt tataatgttt gataatgttt tatagattat 18480
   gaggttttat agagataggg ttttgttttg ttgtttaggt tggagtgtag tggcgtgatt 18540
   atagtttatt gtagtttcga atttttgggt ttaagggatt tttttgtttt agttttagag 18600
   gagttgggat tatagttgtt tgttattatg tttagttaat gtaaaagttg ttttgttttg 18660
   ttttgttttg tttgtagaga tagagttttg ttgtattgtt tgggttggtt ttaaattttt 18720
   gaatttaagt taatttttcg ttttggtttt taaaagtgtt gggattatag aagtttagtt 18780
   aattttattt tttagtattg tttttttttt ttgtgtttta gtttggaaag ttttattgtt 18840
   gtttttaaat ttattgattt ttaaattgtg tagtgtttaa tttgttgtta tttttattta 18900
   gtgaattttt tattttagtt attgtattta ttatttttag atggtttatt gggttttttt 18960
   ttatattttt tatttttttt tttatgaata ttttttttat gttttttttt aaatttttga 19020
   gtatattgag ttatttatta tttttattat ttttgagttt tgtttttatg aaattatttt 19080
   ttttttgatt ataggttata tttttttttt tttttttttt tttttgagat ggagtttttt 19140
   tttttgttgt ttaggttgga gtgtaggggc gttatttcgg tttattgtaa gttcgatttt 19200
   ttgatttcgt gatttattcg ttttagtttt ttaaaatgtt gggattatag gtttacgtga 19260
   gttatcgtat tttgtttata agttttattt ttttattttt tggtatattt agtaattttt 19320
   aattgaattt tggatattgt aaatatgatg ttaagtattt ggatttgtta ttgtttttta 19380
   aagagtgttg ggttttgttt aggtagatta ggtaaattat ttgtaaatta gtttgatttt 19440
   tttgaggttt ttttttttga gatagagttt tgttttgtta tttaggtttg agtgtagtgg 19500
   ggtaatttta gtttattgta gttttaattt tttgggttta agggattttt ttgttttagt 19560
   tattgagtag ttgaggttat aggtatatgt tattatattt ggttaatttt ttgtattttt 19620
   tgtagatatg gggttttggt atgttgttta ggttggtttt gaatttttag gtttaagtga 19680
   tttgtttgtt ttagtttttt aaagtgttgg gattagaggt gtgagttatc gtgtttggtt 19740
   tttgatgtta attttttttt tttttttttt tttttttttt tttttttttt tgagatggag 19800
   ttttgttttg ttatttaggt tagagtgtag tggtgtagtt ttggtttatt gtaattttcg 19860
   ttttttgggt ttaagtgatt tttgtgtttt agttttttga gtagttggga ttataggtat 19920
   ttattattaa gttcggttaa ttttttgtat ttttagtaga gatgtggttt cgttattttg 19980
   gttaggttgg ttttgaattt ttgattttag gtgatttgtt cgttttcgtt ttttaaagtg 20040
   ttgggattat aggtatgagt tattgtattc ggtttgaggt taatttttaa tttgttaggg 20100
   tgggtttagg tgagtttttt ttttagggat agtttagttt tattgttaag gtataatttt 20160
   tttgaggttt gattaaatat gtatttttat tttaggaggt tggagtttga ttattttttt 20220
   gtttttggtg agttttgaga atttattatt ttattgtttt tttttgtgtg gttttattga 20280
   gttttattat atatatgtga agtttagtat ttaggaagat ttgaagagat ttttatgtag 20340
   atttttggag ttttttgagt agtttatttt tttttagaat tgtgttttgt tgtaattttt 20400
   aatattttgg tttttttgaa ttttgttttt atttttttaa tttaggaaga gtattatgtt 20460
   ttgttcgggt tttttttttt tgaattatag tttggaatgt ttttttaggt agaaatttag 20520
   ggatgttata gagtttattt tgtttatttt ttttatttta gggattatag ttttggattt 20580
   ttaaaaatag ttattttttt ttatatatat tttttatata gagaaatata tatatatata 20640
   tatataaata tatatatata tatatatata tatatatata tatatatata tatatacgta 20700
   tatgtatata tatatttgag atagcgtttt attttgttgt ttagtttgga gtgtagaggt 20760
   gtgatgatat tttattgtag ttttaatttt ttaggtttaa gcgatttttt tattttagtt 20820
   tttgagtagt tgggattata ggtataggtt attatattcg gttaattttt ttgtattttt 20880
   tgtagagatg gggttttgtt atattgttta ggttgatttt taatttttgg gtttaagtaa 20940
   ttcgttaatt tttgtttttt aaattgttgg gattataggg gtgagttttt gtgtttagtt 21000
   tttatatatt ttgtagtaat aattttttta ttgtttatgg tggtagagta attttattta 21060
   ttatggtagg agtagaagtt tttttattgg cgttttaatt aagtttagag aagaggaggg 21120
   agttatatgt atgtggttag tttattgttt tttttttttt tgagacggag ttttgttttt 21180
   gtcgtttagg ttggagtgta gtggtatgat ttcggtttat tgtaattttc gtttttcggg 21240
   tttaagcggt ttttttgttt tagttttttg attagttggg attataggta tgggttatta 21300
   cgtttggtta attttgtatt tttagtagag atggggtttt tttatgttgt taggttggtt 21360
   tcgaattttt aattttaggt gatttattcg tttcggtttt ttaaagtgtt gggattatag 21420
   gcgtgagtta tcgcgtttgg tttgttagtt tattgttttt aattagaagt tttagatttt 21480
   cgaaatatat ttaaatattt atgtttaaga tcgggcgtgg tggtttacgt ttgtaatttt 21540
   aatattttgg gaggttaagg taggcgaatt atttgaggtt aggagttaga gattagtttg 21600
   ggtaatatat taaaatttcg tttttattaa aatataaaaa ttagtttggt atagtggtgt 21660
   tcgtttgtaa ttttagttat ttagaaggtt gaggtataag aatcgtttga atttgggagg 21720
   cggaagttgt aatgagttaa gatggtatta ttgtatttta gtttgggtga tatagtgaga 21780
   ttttgtttta aaaaataaat aaataaataa ataaataaaa tttacgttta ataaatatgt 21840
   tttttaagtt aggtgtaggg gtttgtaatt ttagttgttt gggaggttga aataggagga 21900
   ttgtttaagt ttaggagttt gagattagtt tgggtgatat agtgagattt tgttttaagt 21960
   aaaaaaaaaa aaaaaaaaaa aaaaaaaaaa aattttttgt ttgggtattg cggtttatat 22020
   ttgtaatttt agtattttgg gagttcgagt taggtggatt atttgaggtt aggagtttaa 22080
   gattagtttg gttaatatgg tgaaatttta tttttattaa aaatataaaa aattagttag 22140
   acgtgttggc gaatgtttgt aattttagtt atttgggagg ttgaggtagg agaatcgttt 22200
   gaatttggga gacggaggtt atagcgagtt gatattacgt tattgtattt tagtttagtt 22260
   aatagagtga gatttcgttt ttttaaaaaa aatataaaaa aaaattttaa aaaaagtttt 22320
   tattattttt attatataat agaaatttta tgtaatgtaa ttatgttatt tataaaatta 22380
   taggatttgt ataaatttta atgtatagtg ttttataagt ataggatatg gttgtgaaat 22440
   tgtattaata ttatgaattt gttattttat ttttgtttgt gtattattgt aatcgtttgt 22500
   taaattttat ttagtgataa tgttagtgat ttaaattggt agtttttttt gaaggtggtt 22560
   tgggattttt agggtaggtt tggttttagg tgagtttttt ggttaatagt ttttttgtta 22620
   ttttatttta gttttaggtt tatttttggt aggtaggtta ttgggttttg gaggagagga 22680
   tttagttagg ggtttttggt ggataggaag ggaggaagtt tttttttttt tagagttatt 22740
   ttttgttttg ttttttaagt ttttttttat ttacgtgttt gttttttcgt tgtttatagt 22800
   gtttgttttt tcgttgttta tagtgtttgt tttttcgttg ttttgtgagg ttggtgaggt 22860
   agagtagttt tagtaggggg tgggggttgt tgtttgtttt agggttgttt ggtttcggag 22920
   ggttgttttc gacgtgattg gggttttatt ttttattttt tttttttttt taggtggata 22980
   tcgagtagtt ggattttcgc ggtcggattt ttttgtattt ggttattacg ttggggtatt 23040
   ttgagtgtgt tcgtgtgttt ttggcgtacg gcgtagacgt gggtagggag aatcgtagcg 23100
   gttggatagg tgggtagttt tgtttatttt agttttatag tcggggtttt tttagtatta 23160
   tagatttatg gttggttgaa gggttttgtt ttgtgatttt gtttcgtttt tttttagtgt 23220
   tttaggaggt tgtgagtatt cgggatttgg agttggtgta gttggtgttt cggtatcggg 23280
   attattagcg ggtggtgaag cggttggcgg gtattttcgt gtttttggag aagttgcgta 23340
   aggtgaggtt tagtttttta gtttttacgg gagtttttga gttttttttt agtgtagatt 23400
   tagttttttt ttttttcgtt ttttaggttt aggattttta cgtggagatg aaatgggagt 23460
   ttattagttg gggtaagcgg gttgtagtag gtcgtttttg ggttttttta ttttaggttt 23520
   tgcgtttgtt tttttgtttg gaatgtgttt ttatagtttt ttcggcggtt ttttttatgt 23580
   tttagtttag tttagggtta tcgttttagt taggtttttt tagattgttt tagatattgt 23640
   tttgatttta ttattatttt ttttattttg ttatgatttg tttattgtat ttatgatttg 23700
   tcgttttttg ttagaaggta agttttgaga gggtaggagt tttgtttttt ttattgttgt 23760
   atttttagtg gttggaatat ttagtaggtg ttttataaat atttgttgaa agaatggatg 23820
   aaagagtgag tgagtgaatt aataaatgat ggatattagg gatttagtgg tgatagaaat 23880
   atgttcgttt cgattttagg ttgttttttt gtgtgagtat ggtttatatt ggttagggag 23940
   ggggttttgg aggagaggtt gttttttttt tagttttttt ttttgttttt ggttatacgg 24000
   attttaaatt ttatgttttg agttggttta ggtcgtgtag gttagattag ttaaagttat 24060
   ttttaatttt cgtttttatt agtgtttttg gtgtttaaga tttgttttag tgatatttat 24120
   aaagtgtgga agagcggtta gaatttgagg gtagatatta tatttttggg ttttgattat 24180
   atgatttggt agcgagggaa tcgtagtttt gtttttaggg gttaaggtta ggtaggtagg 24240
   aagtggggta gggtggggac gatgggaggt agttttaggt tatttttgat ttttttgtgt 24300
   tgtttcgtgt ttggtagata taagcgtcgt ggttatggag attgattacg atcgtcgggt 24360
   ggtgtatata gagattttgg tattggttgg gtaggatcgg gagttgttgt tggttgttgt 24420
   ttagtttatt gaggaatagg tgttgagtcg gtttatcgcg ttcgtcgtta ttatttagtt 24480
   tgatattaag aatatttttt ttgagaggtg ggtggatatg gttttggtta tttttgagtt 24540
   agttcggtgg ggtgtttgtt ttatttggat tattttgttt ttttaggaat aagattggta 24600
   ttttgggttg gcgtagtgaa aagacggaga tggtgaatgg gtatgaagtt aaggtgaggt 24660
   tgtagttttt agttgttagt ttagttgtat ttttgtttat agtaagttgt ataggggatt 24720
   ttgatttttt ttttttagtt ttggaggtat agaagtaatg tagcgtggtt aagtatttat 24780
   atttgggttt aaggtatttt tttggtgttt tatatggatg tatggatgta tatatagggt 24840
   acgtgtatac gtatatatat atatatattt attttttata gtaatttttt ttggtaggtg 24900
   ttgttttgtt agtattttta ttttacgtat gtttattgag tttttttttt ggtattttat 24960
   ttatttttta taataatttt aggaagtagg gatgatcggt ttattttatg atgaggaaat 25020
   agaggtttag agtggttttg tgatttgtat aaggttatat agttgttaag tggtagagtt 25080
   ggggtttaga ttttggttgg tttgatttta gagttaggtt tttattttta aaaatgattg 25140
   tttggttggg tatggtggtt tatgtttgta attttagcgt tttgggaggt cgaggtgagc 25200
   ggattatttg aggttagttc gagattagtt tggttaatat ggtgaaattt cgtttttatt 25260
   aataatataa aaaattagtc gggtgtggta gtaggcgttt gtaattttag ttattcggga 25320
   ggttgaggta ggagaattgt ttgaatttgg gaggcggagt ttgtagtgtg tagcgatttc 25380
   gttatagtat tttagtttgg gtaatagagc gagattttat ttaaaaaaaa aaaaaaaaaa 25440
   aggttttgtt tgttattttg ggatttttat gttgtttacg atatgaaagt aggaattaat 25500
   aaatgttatg ggttttgtgt gtggtatttt ttttgaaagt tttttttgtt tttgtagttt 25560
   gggaaggttt ttgtgttatt tttattagtg tatggattat gttgttataa ttttatttta 25620
   ttgttttttt atagagattg taagttttag gagggtaggg attatgtttg gtttgtttat 25680
   tgtatttttg atgtttattt taatgtttgg tatataaagg ttttttaaaa aatgtttgtt 25740
   gaatgaataa gtgaatgaat gaatgaatga atgattttat atatttattt atttaaaagt 25800
   ttgaagtaga gtttttttag gattttttgt tatagggagt gtttttagtg tttttagggg 25860
   taagatatag ggtaagatta gaattgttta tgagtttttt ttttatttgt ttttaggtgt 25920
   atggggtatt taacgtggag tttattattc gtatacggat agaatatttt ttagaatagt 25980
   ataagggtaa ggttaaaggt aatgaggtag agttggatgg ggagaggtgt tgtaggattg 26040
   cgggaattag tttttatttt agtgttttta ttagtatttt tattggtatt ttagtaagag 26100
   gttggatgtt ttatttaggt cgatagattt taatttaggt gttaagtttt aaggatgggt 26160
   agtggttgtt tagagtatta agaagttttt aaggatttat ttgggtttag tggagagtta 26220
   tgttgtaagt gattattaat gttgttgttg aatttgggag aggagagaaa tgttatgtat 26280
   gttatttatt ggttatttta gtttagtggg atatatttta ggtagaaagt taggggattt 26340
   tgttatagat tttgtatgtt tattgatgtt atatttgatt ttttgggtat tagatatcgt 26400
   gttcgaggtg aaaaagtata atttttattt tataggggaa taatatgaaa ttatgtttat 26460
   ggaagtgttt gaagtaaata tatatacgta tatgtatatt ttatagtgtt tgtcggggta 26520
   ggcgttttat tgtttagggt tatcgagttg gagaggagta tttttggaag ttgtttttga 26580
   tttttaggtt ttttttttag tgttttaagt tgtgtttata gaggttttgg tttttggttt 26640
   ttgaagtatt tagttttttt taggtgaagg tgtttgagta tgtggttggg tgagtaggtt 26700
   ttattttagg aatcggtttt ttttttaggt tttgggtgtt gttatttttg atgaggtgtt 26760
   taggtgtgtg tggagttgcg gtgttttttg agatgttata ttttttgtag gttgtaagat 26820
   atttttgtag ttttttttgg gaatcgttga gtagtacggg ggtttttaaa atggggtgag 26880
   tgtgtgtagg ggtattcgta agtggaggga tgtggatgtg gtttaggagg aggtttggga 26940
   agcgttttgt ttagtatggt ttatgtggat ttttttttta taatgtttaa ggttatgtta 27000
   gcgttatatt ggtagagggg atagtttggg ggttttgtga taataaatga gttggtttgg 27060
   tattaggaat tgaggggagt ttttgggttt ttttttatgt ggtatagatt ttgattattt 27120
   agattttgag ttaagttaat tttattgtta ttattgtaga agaatatttt aattttaatt 27180
   ttgagttggg taatcgtgat atgggtcgtt ttatggaatt gattattaag atatagaagt 27240
   gaggtttttg tcggtgttgg gaaggtgggg ggtcggggtt ttaggagatg ttgggaggtt 27300
   atggtttttt gtagtgtttt ttattttttg ggatttggtg ggggtaggtt taaggttaag 27360
   ttgtggttgt gtgaggagta ttttttgttt ttgtgtgagt aggtggtttt tattattgat 27420
   tttatggtcg ttagtaatgc gttttttgtt aagtttcggg attttattat tttgcgtttg 27480
   ttttttggtt ttttagttaa gattggtgag atcgtacggt tatttttttt gagttcgtgg 27540
   cgggtcgatc ggggtttaga tgaggtcggg gtggggttga ggttgaggtg tgaggggttg 27600
   agtggtggcg tttaaaggtt tttttatttt tagaaatttc gattttttat atttttaacg 27660
   ttcgtattat tttcgggaat tttaacggtt gcgacgaatc ggtgttatcg gtgcgaggta 27720
   gttttagtag cgagacgttt tttttaggta gcgatttttt tagcgttagt agttttagtt 27780
   ttacgagtga ggtttttcgc gagaacgttt gtttttcggt ttttttcggg gtgggtggga 27840
   ggggcgcgtt cggtcgtgtt tgatttagtt ttcgcgtttt tagttttttg tcgcggttgc 27900
   gagatttttt tagcgttgtt cgaggtttcg cgcggttata gtatgatggg cggttagcgg 27960
   gaggcggcga ttcgggacga cgacgacgat ttgttgtaat tcgttattta gtagagtttg 28020
   tttgaggcgg gtagtgagta tgattaggtg cgttttcgcg ggcgcgcggg gttacgggat 28080
   ttcgcgtcgt tacggttaac gtttacgttt tttcgtaggt tattatttgg gaggcgttaa 28140
   ttaatagtaa gttaggtatt tattttatgt tttacgaggg tcgtcgatag gataggttag 28200
   tgttcgttgg gtcggagaga atttttcgga gaggattttg ttttttttaa gttaggatat 28260
   agtcggagaa ttcgcggggt tttcggtagg gatttttttg gtatcgggat ggtttttgtt 28320
   attatttttg ttttcgaggc ggtaggtagg ggttagggat ttaggtttta tcgcggagtt 28380
   tagatttttt ttaggtattg acggggagaa tttggtaggg tttgggtttc gttacgatta 28440
   ggtgggggtt aggacgtcgc gggattgatc gtttttatta gagggtggtg gtcgcgggtc 28500
   gttttggagt ttttacgtcg tgtttcggtt tcggtcggtg ggtgggtgcg ttcgggagtt 28560
   cgcgatattt tttttttatt tttaggagcg tttcgtttac gtcgtagcgt tagtttgcgt 28620
   tttcggcgtt agtgtttagt tttcggttta gtttagggtt aggtttcggc ggttacgtgt 28680
   ttcggagtta cgacgagtag ttgcggttgg cgatggaatt gtcggcgtag gagtaggagg 28740
   agaggcggcg gcgcgcgcgt taggaggagg aggagttgga gcgtattttg aggtttttat 28800
   tgatcgagta gtagcgtttt ttgtcgggat tttcgttagc gttacgcgcg ttacgtttag 28860
   ggttaggagt tagataaatt tcggtttgcg cgtttgtaga gcggcggttg gagattggag 28920
   ttatcgtttc gcgggtgtag tagtatagta ggtacggttc ggggagggat tcggtatggt 28980
   cgcggggtat ttttttaggt tagggttttg gaggtaattg gtacggtttg gtttttttgt 29040
   tttgttgtat tttgattttt taattcgttt ttttgggttt agatttgttt tgttttaggg 29100
   cggagttagg cggttttgag ggggagatga atttttagag gagcgttgtt tttatttttt 29160
   gttttttttg gtcggttcgt atttttgttt attaatttta ttttaggata ttgtttttga 29220
   agtttttgta tttttgtttt ttatttttgg ggggtagttg agtttttcgc gtgttgtgtt 29280
   attgttttgt tttagatata aattagtacg tttagggttt agtttttttt ttatttcggt 29340
   attttagcgt taagtgtatt tagcggggta ttcggttttt ttagttttta tattcgtttt 29400
   gggttttagg gtggttttag tttttttttg ggtagttaga agttggagtt ttatttttta 29460
   aggtatattt tttttttttt ttttttgtga ttagggagtg tgtttaacgt agttttttgg 29520
   tttgtgtaag ttttgatttt tttatggtgt ttcggagagt ggggagtata ttgggttggg 29580
   gtaagtatta gatttaagta gattggatat aaagggttcg tttagggttt tggcgttatt 29640
   tttatttttt tttattagtt gttgttagtt tttgtggttg tatattttat ttgtttttat 29700
   acggagattg attttaaaat tttttattta atggtgttaa ttttcggttt ttttttgttt 29760
   tattttattt atttagagaa gtatagattt cgttaggggt aggggtttat cgtatatttt 29820
   tgtttcgggt ttgtttggga ttggtttttt cggtttagtt agtgaggttt agaagggata 29880
   taaagaggga tggaagaaaa gaataaagag aaattgtttt ttttattttt ttttttgatg 29940
   ttaggggtat tagattgatt ttgaggtata aataaaagag gttttatatc ggaggttttt 30000
   gttatttaat tttatttggg gggaacgttt acggtgtggg ttggggtttt tttgaatttt 30060
   agagtggaga gagttttagg gtaagaggtt gtttgatgga ttttgagttt aggagtgtat 30120
   cgttaggttt tttaggagtt tgggggtagt atttgtagag gttgggggtt tcgttttttt 30180
   tatttttatt tatgtaggag gtatagattg aacgtttagt aaaatatttt atttagtacg 30240
   ttttgttttt ttgagagagg taacggtaga gggttggggg tagtgtgtgt gtatttgtga 30300
   tgaatttaat tttttatgtt agttttttcg agattttttc gcgggggttg tcgcgaggaa 30360
   gtagttttcg tttttgtcgg tggaaagaag gaagagagtt ttttattata gatcgttgtg 30420
   ggtgtagtta ttgggttcgg ttttgtagta aacgggttgt gtatgtgttt gtagaattgg 30480
   ggtgacgttt atttggttta tcgtttaggg tgatggaatt tagtgagttg attaggagaa 30540
   gcgttgttat tttttttagt ttgggggtat ttattaaatc gttattcgtt tgaggttaag 30600
   tttaggtttt aataatttta agtattacgt taatattaag tatttttttt gttttgggcg 30660
   gaggataaag ggggatatta tttcgtcgtg tagtattatt gttgagtttt ttaggttatt 30720
   ttaggttttt ttaggttagt tttagtcgag ttttagacga ggttcgtagt ttagttttcg 30780
   ttggagcgac gtgggttttt tttaagtttt aagagttttg gtttttcgcg aggggcggag 30840
   tttttttttt agtttcgttt tcgggtttag tttaagaagg cggggttcgg agttcgggat 30900
   taaaagtcgg ggcggggtcg agtttgtgcg tttcgtttcg tttcgtttgt ttggcgcgcg 30960
   gggttagttc gtgtcgttta ttagttcgta tagtatgttt tttagagtcg tgatgcgttg 31020
   tttttgggtt tgtattcgtt cgcggagggt tttgattttt tttagtagcg tttttagggt 31080
   gtgttgttgt tggggcggag gatagagagg tcgtgtcgta taggggcggt tgggggagat 31140
   gtagattggg ttagagtgta gtagggtcga ttttatcgat aagggggcgt cgttggtcga 31200
   ttggttgcgg cgggggtcgg agggcgggcg tacgtttagg atgttgcgtt tcgtgattcg 31260
   gagttcgcgg tgtttggggg gtatatagtc gttttttagc gaaatgagta cgggttcggc 31320
   gttttggtcg gatagttatt cgttcgtttt tagggtcggt ttcgggtttg gcgtattcgg 31380
   gtatagatcg ttttggaaga ggtttgattg cgggggtggg tggataggag gatttgcgtg 31440
   agagggtgcg gggtttgttt cgtttttttt cgtttcgtag ttttgtttaa tttttttttc 31500
   ggttttttat tttgcggggt atagttatga tgataggttt atattttttt tcgtgtagtt 31560
   tgtagaattt ataagggagt agggtttagt attttcgtag atttttacgt ttttaatttt 31620
   ttttgtttaa gattaagggg gttggaggtt aatatttgtt tagcgtttat tatgtatatt 31680
   gtatattata tatattattg gttttgtaat aaaatatttt gatggtgtgt ttattattat 31740
   tttatagatg aggaaattga agttgagaat gtaaggttag tgagcggtgg gtttggtatt 31800
   gaaatagagt tgttttattt tttcgtttcg tttttttaaa gtattgtatt attggggaag 31860
   tttcgtggtt aggattcgat atgagtgggg gatagaggcg ggtaggtgtt taggaggggt 31920
   taaggttgtt gatcgggcga ttttatattt gttgatattt agtttttttt tgggtatgaa 31980
   atttatgttt cgttgcggtt ttttgttgtt gaacgtgttt aggtagtgta cgaaaggcgg 32040
   ttcgtcggta attttaaagt atcgaatgtt gttgtcgttt tgtaaaatta gtcggtttag 32100
   gggcgcgttc ggataaggtt ttttatattt ttatttagtg ttttgttttt tttgttcggt 32160
   cgtgagtatt ttgttatata ggtagacgat gttggagtcg ggatcgtaaa agggtaatag 32220
   gatttcgttg tttgtgttta ttttttgtag tgttattggt ttttcgaagt tgttttgttc 32280
   gagtatagga ggcgtgatta gttttgtttt attttttcgt gttatttcgg gatttttaga 32340
   agttttttta cgttttttgg tttttttaaa ttttatgttt taaattttta gtgattagag 32400
   ttgtaggttt tttttttggg tatatttttt tttttttttt gcgtatatag tttttttttt 32460
   tttgtatata tattcgttgc gcgtcgtatg tagttatagt aaattatttt tagggattcg 32520
   gtattattag gtgggggcgt tagcgtagga ttttttacgc gacgtagtaa aattgggtag 32580
   aggggtgggt gtgagaatgg taggcgttgt agaggtggta gtagttttta gttttgttta 32640
   ggtgggtatg ttttgcgggt ggggggcgtt tggttatttt ttcgtgatgt tagttttcga 32700
   ggtatatatt ttaagttttt agttgcgtta tcgggtttta taggtttagt tttcgttggt 32760
   ttatgcgggt gaagttcgtg gtgaagatat ggttttggcg cgtgaagacg gttcgtatgg 32820
   gttttatttt ttcgtgggtc gtaaattttt tttgggggga gggggagata gggagggata 32880
   ttatttagtt gtttgttttg aggagtatgg agggatgagt aattagtttg ttgatttggg 32940
   tgttcggggt ggaggagcgg atgcggggaa gagataaatg gggtttaagt atttagtttt 33000
   ttgcggggtt acgtttggtt tttgatttgg agagtattcg ggtttggaga gagtaggagg 33060
   tacgcggttt ttagtcgatt tcgaattttt aagcgcgttt aaaggtcgat ttttagagta 33120
   ggggtgtttg ttggtttttc ggagtcgtag cgaggttttt ttcgaatggg agtttttcgt 33180
   ttcgttttgg ttttttgggc gttagttata gttcgggaag gcgttttcgg tagatttaga 33240
   gaaggagcga gttagcgcgt tcggcgcggt ttggtttatc gggttttaga gcgcgagttg 33300
   tcgtttattt attttgttga agtcggtgtt gagtagtttt tcgtttgcgg tgaagatagt 33360
   tcgtagcggg cgggcgtttt cgtgaggtcg ggtttgtttt tgttcgtcgg gttagtgggt 33420
   tagagcgttt cggcgttagt ttcgatcgtt ttcggcgcgt ttgttttacg tagatatgta 33480
   cgcggtttac gtattcgtat tttaggttag atttgaggtt ggcgaggtta gggtcgtcgt 33540
   ttttttaggg tattcggttg ggataagtaa cgcgcgaggg ttgttttatt tttcgtttag 33600
   cgggtttata aggagaaagg gttagttttc gagttagtgt taggttttag attttggaag 33660
   atgcgtttgg gtgtcggttg agaggagagg ggtttaggag tttaggtttt atgtaggttt 33720
   tggtgggagg atggagtttt tagttttttt tttttttaat tttaaagcga ggagtttttt 33780
   tttgagtttt tgagatgtaa gggaggggag ggaataggag tcgggtagag aattgtaggt 33840
   tgggggttta tttagggtag agatttgtgg ttgtagagac gggggaaggg ggttagtttt 33900
   tggagtaggg atgcgatagg tgaaagtcgg gattaggggt tttgataatg gtgggtataa 33960
   gggtgttgat agttggttat tggttaaggg gttttgggga gtggttaggt taggtattta 34020
   tcgttattat ttggtttttt ttggggttaa tgatgcgtaa ggttttgttt ttgtaggtgg 34080
   tggttagtag gttatcgttg ttgttttagt atatattgtg gatgacgttt gggtgtatat 34140
   tatttaggtt tagtagtatt ttttcggtgt ttatatttta gatgatgatt atattgttat 34200
   tatttgttta gagtggttag gtatggttat ttgggtttta tttttgttat tatttattta 34260
   tttatggggg attttttttt agtttttaga tttgtattga gtaggatatt tttggtagta 34320
   gggtgttagg agaggatgtt tatacgtttg gagtggtttt taagtgtgat gataggtttc 34380
   gtaatgttgc gtatgggggt atagtttgga atttgttata tttgggtagg aagaaaaggt 34440
   atatggtggg tggaatgagg agttattttg tttttttatt ttttttttat tttttttttt 34500
   aattttattg gtttttttga gtggtaaggg ttaaatatag ggtaagatgt ggtggtgtat 34560
   gtttagaata gaagtagttg ggagttagga gggggtattg ttagtttttt agagattttt 34620
   tatttatttt agtagggaga ttttagagta ttgttttgtt gttttttagg tattaagttg 34680
   gttatggtta taattagttt taagtagtgt attttgggag ggggtgggta tgtagttaaa 34740
   aatagtttag gagttgggta gggtagggtt ttgggattta gtattttttt tattattatt 34800
   tttagtattt tttttagtat aggtgaggta gttttttttg tttttttgtt taattgttta 34860
   gtagtagtta tttggttttt attatgatgg tggtgtcgtt tgaggtattg gcgataacgt 34920
   tgttattgtg tggatattag ttaatattta gtataggggt agtgtgttta gtgattagtg 34980
   ggtagttttt att 34993
<210> 3
   <211> 34993
   <212> DNA
   <213> Artificial Sequence
<220>
   <223> rc C to T (bis1)
<400> 3
   aataaaaact acccactaat cactaaacac actaccccta tactaaatat taactaatat 60
   ccacacaata acaacgttat cgccaatacc tcaaacgaca ccaccatcat aataaaaacc 120
   aaataactac tactaaacaa ttaaacaaaa aaacaaaaaa aactacctca cctatactaa 180
   aaaaaatact aaaaataata ataaaaaaaa tactaaatcc caaaacccta ccctacccaa 240
   ctcctaaact atttttaact acatacccac cccctcccaa aatacactac ttaaaactaa 300
   ttataaccat aaccaactta atacctaaaa aacaacaaaa caatactcta aaatctccct 360
   actaaaataa ataaaaaatc tctaaaaaac taacaatacc ccctcctaac tcccaactac 420
   ttctattcta aacatacacc accacatctt accctatatt taacccttac cactcaaaaa 480
   aaccaataaa attaaaaaaa aaaataaaaa aaaaataaaa aaacaaaata actcctcatt      540
   ccacccacca tatacctttt cttcctaccc aaatataaca aattccaaac tataccccca      600
   tacgcaacat tacgaaacct atcatcacac ttaaaaacca ctccaaacgt ataaacatcc      660
   tctcctaaca ccctactacc aaaaatatcc tactcaatac aaatctaaaa actaaaaaaa      720
   aatcccccat aaataaataa ataataacaa aaataaaacc caaataacca tacctaacca      780
   ctctaaacaa ataataacaa tataatcatc atctaaaata taaacaccga aaaaatacta      840
   ctaaacctaa ataatataca cccaaacgtc atccacaata tatactaaaa caacaacgat      900
   aacctactaa ccaccaccta caaaaacaaa accttacgca tcattaaccc caaaaaaaac     960
   caaataataa cgataaatac ctaacctaac cactccccaa aaccccttaa ccaataacca    1020
   actatcaaca cccttatacc caccattatc aaaaccccta atcccgactt tcacctatcg    1080
   catccctact ccaaaaacta acccccttcc cccgtctcta caaccacaaa tctctaccct     1140
   aaataaaccc ccaacctaca attctctacc cgactcctat tccctcccct cccttacatc     1200
   tcaaaaactc aaaaaaaaac tcctcgcttt aaaattaaaa aaaaaaaaaa ctaaaaactc     1260
   catcctccca ccaaaaccta cataaaacct aaactcctaa acccctctcc tctcaaccga     1320
   cacccaaacg catcttccaa aatctaaaac ctaacactaa ctcgaaaact aaccctttct     1380
   ccttataaac ccgctaaacg aaaaataaaa caaccctcgc gcgttactta tcccaaccga     1440
   ataccctaaa aaaacgacga ccctaacctc gccaacctca aatctaacct aaaatacgaa     1500
   tacgtaaacc gcgtacatat ctacgtaaaa caaacgcgcc gaaaacgatc gaaactaacg     1560
   ccgaaacgct ctaacccact aacccgacga acaaaaacaa acccgacctc acgaaaacgc     1620
   ccgcccgcta cgaactatct tcaccgcaaa cgaaaaacta ctcaacaccg acttcaacaa     1680
   aataaataaa cgacaactcg cgctctaaaa cccgataaac caaaccgcgc cgaacgcgct     1740
   aactcgctcc ttctctaaat ctaccgaaaa cgccttcccg aactataact aacgcccaaa     1800
   aaaccaaaac gaaacgaaaa actcccattc gaaaaaaacc tcgctacgac tccgaaaaac     1860
   caacaaacac ccctactcta aaaatcgacc tttaaacgcg cttaaaaatt cgaaatcgac     1920
   taaaaaccgc gtacctccta ctctctccaa acccgaatac tctccaaatc aaaaaccaaa     1980
   cgtaaccccg caaaaaacta aatacttaaa ccccatttat ctcttccccg catccgctcc     2040
   tccaccccga acacccaaat caacaaacta attactcatc cctccatact cctcaaaaca     2100
   aacaactaaa taatatccct ccctatctcc ccctcccccc aaaaaaaatt tacgacccac     2160
   gaaaaaataa aacccatacg aaccgtcttc acgcgccaaa accatatctt caccacgaac     2220
   ttcacccgca taaaccaacg aaaactaaac ctataaaacc cgataacgca actaaaaact     2280
   taaaatatat acctcgaaaa ctaacatcac gaaaaaataa ccaaacgccc cccacccgca     2340
   aaacataccc acctaaacaa aactaaaaac tactaccacc tctacaacgc ctaccattct     2400
   cacacccacc cctctaccca attttactac gtcgcgtaaa aaatcctacg ctaacgcccc 2460
   cacctaataa taccgaatcc ctaaaaataa tttactataa ctacatacga cgcgcaacga 2520
   atatatatac aaaaaaaaaa aaactatata cgcaaaaaaa aaaaaaaaat atacccaaaa 2580
   aaaaaaccta caactctaat cactaaaaat ttaaaacata aaatttaaaa aaaccaaaaa 2640
   acgtaaaaaa acttctaaaa atcccgaaat aacacgaaaa aataaaacaa aactaatcac 2700
   gcctcctata ctcgaacaaa acaacttcga aaaaccaata acactacaaa aaataaacac 2760
   aaacaacgaa atcctattac ccttttacga tcccgactcc aacatcgtct acctatataa 2820
   caaaatactc acgaccgaac aaaaaaaaca aaacactaaa taaaaatata aaaaacctta 2880
   tccgaacgcg cccctaaacc gactaatttt acaaaacgac aacaacattc gatactttaa 2940
   aattaccgac gaaccgcctt tcgtacacta cctaaacacg ttcaacaaca aaaaaccgca 3000
   acgaaacata aatttcatac ccaaaaaaaa actaaatatc aacaaatata aaatcgcccg 3060
   atcaacaacc ttaacccctc ctaaacacct acccgcctct atcccccact catatcgaat 3120
   cctaaccacg aaacttcccc aataatacaa tactttaaaa aaacgaaacg aaaaaataaa 3180
   acaactctat ttcaatacca aacccaccgc tcactaacct tacattctca acttcaattt 3240
   cctcatctat aaaataataa taaacacacc atcaaaatat tttattacaa aaccaataat 3300
   atatataata tacaatatac ataataaacg ctaaacaaat attaacctcc aaccccctta 3360
   atcttaaaca aaaaaaatta aaaacgtaaa aatctacgaa aatactaaac cctactccct 3420
   tataaattct acaaactaca cgaaaaaaaa tataaaccta tcatcataac tataccccgc 3480
   aaaataaaaa accgaaaaaa aaattaaaca aaactacgaa acgaaaaaaa acgaaacaaa 3540
   ccccgcaccc tctcacgcaa atcctcctat ccacccaccc ccgcaatcaa acctcttcca 3600
   aaacgatcta tacccgaata cgccaaaccc gaaaccgacc ctaaaaacga acgaataact 3660
   atccgaccaa aacgccgaac ccgtactcat ttcgctaaaa aacgactata taccccccaa 3720
   acaccgcgaa ctccgaatca cgaaacgcaa catcctaaac gtacgcccgc cctccgaccc 3780
   ccgccgcaac caatcgacca acgacgcccc cttatcgata aaatcgaccc tactacactc 3840
   taacccaatc tacatctccc ccaaccgccc ctatacgaca cgacctctct atcctccgcc 3900
   ccaacaacaa cacaccctaa aaacgctact aaaaaaaatc aaaaccctcc gcgaacgaat 3960
   acaaacccaa aaacaacgca tcacgactct aaaaaacata ctatacgaac taataaacga 4020
   cacgaactaa ccccgcgcgc caaacaaacg aaacgaaacg aaacgcacaa actcgacccc 4080
   gccccgactt ttaatcccga actccgaacc ccgccttctt aaactaaacc cgaaaacgaa 4140
   actaaaaaaa aaactccgcc cctcgcgaaa aaccaaaact cttaaaactt aaaaaaaacc 4200
   cacgtcgctc caacgaaaac taaactacga acctcgtcta aaactcgact aaaactaacc 4260
   taaaaaaacc taaaataacc taaaaaactc aacaataata ctacacgacg aaataatatc 4320
   cccctttatc ctccgcccaa aacaaaaaaa atacttaata ttaacgtaat acttaaaatt 4380
   attaaaacct aaacttaacc tcaaacgaat aacgatttaa taaatacccc caaactaaaa 4440
   aaaataacaa cgcttctcct aatcaactca ctaaattcca tcaccctaaa cgataaacca 4500
   aataaacgtc accccaattc tacaaacaca tacacaaccc gtttactaca aaaccgaacc 4560
   caataactac acccacaacg atctataata aaaaactctc ttccttcttt ccaccgacaa 4620
   aaacgaaaac tacttcctcg cgacaacccc cgcgaaaaaa tctcgaaaaa actaacataa 4680
   aaaattaaat tcatcacaaa tacacacaca ctacccccaa ccctctaccg ttacctctct 4740
   caaaaaaaca aaacgtacta aataaaatat tttactaaac gttcaatcta tacctcctac 4800
   ataaataaaa ataaaaaaaa cgaaaccccc aacctctaca aatactaccc ccaaactcct 4860
   aaaaaaccta acgatacact cctaaactca aaatccatca aacaacctct taccctaaaa 4920
   ctctctccac tctaaaattc aaaaaaaccc caacccacac cgtaaacgtt ccccccaaat 4980
   aaaattaaat aacaaaaacc tccgatataa aacctctttt atttatacct caaaatcaat 5040
   ctaatacccc taacatcaaa aaaaaaaata aaaaaaacaa tttctcttta ttcttttctt 5100
   ccatccctct ttatatccct tctaaacctc actaactaaa ccgaaaaaac caatcccaaa 5160
   caaacccgaa acaaaaatat acgataaacc cctaccccta acgaaatcta tacttctcta 5220
   aataaataaa ataaaacaaa aaaaaaccga aaattaacac cattaaataa aaaattttaa 5280
   aatcaatctc cgtataaaaa caaataaaat atacaaccac aaaaactaac aacaactaat 5340
   aaaaaaaaat aaaaataacg ccaaaaccct aaacgaaccc tttatatcca atctacttaa 5400
   atctaatact taccccaacc caatatactc cccactctcc gaaacaccat aaaaaaatca 5460
   aaacttacac aaaccaaaaa actacgttaa acacactccc taatcacaaa aaaaaaaaaa 5520
   aaaaaatata ccttaaaaaa taaaactcca acttctaact acccaaaaaa aaactaaaac 5580
   caccctaaaa cccaaaacga atataaaaac taaaaaaacc gaataccccg ctaaatacac 5640
   ttaacgctaa aataccgaaa taaaaaaaaa actaaaccct aaacgtacta atttatatct 5700
   aaaacaaaac aataacacaa cacgcgaaaa actcaactac cccccaaaaa taaaaaacaa 5760
   aaatacaaaa acttcaaaaa caatatccta aaataaaatt aataaacaaa aatacgaacc 5820
   gaccaaaaaa aacaaaaaat aaaaacaacg ctcctctaaa aattcatctc cccctcaaaa 5880
   ccgcctaact ccgccctaaa acaaaacaaa tctaaaccca aaaaaacgaa ttaaaaaatc 5940
   aaaatacaac aaaacaaaaa aaccaaaccg taccaattac ctccaaaacc ctaacctaaa 6000
   aaaatacccc gcgaccatac cgaatccctc cccgaaccgt acctactata ctactacacc 6060
   cgcgaaacga taactccaat ctccaaccgc cgctctacaa acgcgcaaac cgaaatttat 6120
   ctaactccta accctaaacg taacgcgcgt aacgctaacg aaaatcccga caaaaaacgc 6180
   tactactcga tcaataaaaa cctcaaaata cgctccaact cctcctcctc ctaacgcgcg 6240
   cgccgccgcc tctcctccta ctcctacgcc gacaattcca tcgccaaccg caactactcg 6300
   tcgtaactcc gaaacacgta accgccgaaa cctaacccta aactaaaccg aaaactaaac 6360
   actaacgccg aaaacgcaaa ctaacgctac gacgtaaacg aaacgctcct aaaaataaaa 6420
   aaaaaatatc gcgaactccc gaacgcaccc acccaccgac cgaaaccgaa acacgacgta 6480
   aaaactccaa aacgacccgc gaccaccacc ctctaataaa aacgatcaat cccgcgacgt 6540
   cctaaccccc acctaatcgt aacgaaaccc aaaccctacc aaattctccc cgtcaatacc 6600
   taaaaaaaat ctaaactccg cgataaaacc taaatcccta acccctacct accgcctcga 6660
   aaacaaaaat aataacaaaa accatcccga taccaaaaaa atccctaccg aaaaccccgc 6720
   gaattctccg actatatcct aacttaaaaa aaacaaaatc ctctccgaaa aattctctcc 6780
   gacccaacga acactaacct atcctatcga cgaccctcgt aaaacataaa ataaatacct 6840
   aacttactat taattaacgc ctcccaaata ataacctacg aaaaaacgta aacgttaacc 6900
   gtaacgacgc gaaatcccgt aaccccgcgc gcccgcgaaa acgcacctaa tcatactcac 6960
   tacccgcctc aaacaaactc tactaaataa cgaattacaa caaatcgtcg tcgtcgtccc 7020
   gaatcgccgc ctcccgctaa ccgcccatca tactataacc gcgcgaaacc tcgaacaacg 7080
   ctaaaaaaat ctcgcaaccg cgacaaaaaa ctaaaaacgc gaaaactaaa tcaaacacga 7140
   ccgaacgcgc ccctcccacc caccccgaaa aaaaccgaaa aacaaacgtt ctcgcgaaaa 7200
   acctcactcg taaaactaaa actactaacg ctaaaaaaat cgctacctaa aaaaaacgtc 7260
   tcgctactaa aactacctcg caccgataac accgattcgt cgcaaccgtt aaaattcccg 7320
   aaaataatac gaacgttaaa aatataaaaa atcgaaattt ctaaaaataa aaaaaccttt 7380
   aaacgccacc actcaacccc tcacacctca acctcaaccc caccccgacc tcatctaaac 7440
   cccgatcgac ccgccacgaa ctcaaaaaaa ataaccgtac gatctcacca atcttaacta 7500
   aaaaaccaaa aaacaaacgc aaaataataa aatcccgaaa cttaacaaaa aacgcattac 7560
   taacgaccat aaaatcaata ataaaaacca cctactcaca caaaaacaaa aaatactcct 7620
   cacacaacca caacttaacc ttaaacctac ccccaccaaa tcccaaaaaa taaaaaacac 7680
   tacaaaaaac cataacctcc caacatctcc taaaaccccg accccccacc ttcccaacac 7740
   cgacaaaaac ctcacttcta tatcttaata atcaattcca taaaacgacc catatcacga 7800
   ttacccaact caaaattaaa attaaaatat tcttctacaa taataacaat aaaattaact 7860
   taactcaaaa tctaaataat caaaatctat accacataaa aaaaaaccca aaaactcccc 7920
   tcaattccta ataccaaacc aactcattta ttatcacaaa acccccaaac tatcccctct 7980
   accaatataa cgctaacata accttaaaca ttataaaaaa aaaatccaca taaaccatac 8040
   taaacaaaac gcttcccaaa cctcctccta aaccacatcc acatccctcc acttacgaat 8100
   acccctacac acactcaccc cattttaaaa acccccgtac tactcaacga ttcccaaaaa 8160
   aaactacaaa aatatcttac aacctacaaa aaatataaca tctcaaaaaa caccgcaact 8220
   ccacacacac ctaaacacct catcaaaaat aacaacaccc aaaacctaaa aaaaaaaccg 8280
   attcctaaaa taaaacctac tcacccaacc acatactcaa acaccttcac ctaaaaaaaa 8340
   ctaaatactt caaaaaccaa aaaccaaaac ctctataaac acaacttaaa acactaaaaa 8400
   aaaaacctaa aaatcaaaaa caacttccaa aaatactcct ctccaactcg ataaccctaa 8460
   acaataaaac gcctaccccg acaaacacta taaaatatac atatacgtat atatatttac 8520
   ttcaaacact tccataaaca taatttcata ttattcccct ataaaataaa aattatactt 8580
   tttcacctcg aacacgatat ctaataccca aaaaatcaaa tataacatca ataaacatac 8640
   aaaatctata acaaaatccc ctaactttct acctaaaata tatcccacta aactaaaata 8700
   accaataaat aacatacata acatttctct cctctcccaa attcaacaac aacattaata 8760
   atcacttaca acataactct ccactaaacc caaataaatc cttaaaaact tcttaatact 8820
   ctaaacaacc actacccatc cttaaaactt aacacctaaa ttaaaatcta tcgacctaaa 8880
   taaaacatcc aacctcttac taaaatacca ataaaaatac taataaaaac actaaaataa 8940
   aaactaattc ccgcaatcct acaacacctc tccccatcca actctacctc attaccttta 9000
   accttaccct tatactattc taaaaaatat tctatccgta tacgaataat aaactccacg 9060
   ttaaataccc catacaccta aaaacaaata aaaaaaaaac tcataaacaa ttctaatctt 9120
   accctatatc ttacccctaa aaacactaaa aacactccct ataacaaaaa atcctaaaaa 9180
   aactctactt caaactttta aataaataaa tatataaaat cattcattca ttcattcatt 9240
   cacttattca ttcaacaaac atttttaaaa aaacctttat ataccaaaca ttaaaataaa 9300
   catcaaaaat acaataaaca aaccaaacat aatccctacc ctcctaaaac ttacaatctc 9360
   tataaaaaaa caataaaata aaattataac aacataatcc atacactaat aaaaataaca 9420
   caaaaacctt cccaaactac aaaaacaaaa aaaactttca aaaaaaatac cacacacaaa 9480
   acccataaca tttattaatt cctactttca tatcgtaaac aacataaaaa tcccaaaata 9540
   acaaacaaaa cctttttttt tttttttttt taaataaaat ctcgctctat tacccaaact 9600
   aaaatactat aacgaaatcg ctacacacta caaactccgc ctcccaaatt caaacaattc 9660
   tcctacctca acctcccgaa taactaaaat tacaaacgcc tactaccaca cccgactaat 9720
   tttttatatt attaataaaa acgaaatttc accatattaa ccaaactaat ctcgaactaa 9780
   cctcaaataa tccgctcacc tcgacctccc aaaacgctaa aattacaaac ataaaccacc 9840
   atacccaacc aaacaatcat ttttaaaaat aaaaacctaa ctctaaaatc aaaccaacca 9900
   aaatctaaac cccaactcta ccacttaaca actatataac cttatacaaa tcacaaaacc 9960
   actctaaacc tctatttcct catcataaaa taaaccgatc atccctactt cctaaaatta 10020
   ttataaaaaa taaataaaat accaaaaaaa aaactcaata aacatacgta aaataaaaat 10080
   actaacaaaa caacacctac caaaaaaaat tactataaaa aataaatata tatatatata 10140
   tacgtataca cgtaccctat atatacatcc atacatccat ataaaacacc aaaaaaatac 10200
   cttaaaccca aatataaata cttaaccacg ctacattact tctatacctc caaaactaaa 10260
   aaaaaaaaat caaaatcccc tatacaactt actataaaca aaaatacaac taaactaaca 10320
   actaaaaact acaacctcac cttaacttca tacccattca ccatctccgt cttttcacta 10380
   cgccaaccca aaataccaat cttattccta aaaaaacaaa ataatccaaa taaaacaaac 10440
   accccaccga actaactcaa aaataaccaa aaccatatcc acccacctct caaaaaaaat 10500
   attcttaata tcaaactaaa taataacgac gaacgcgata aaccgactca acacctattc 10560
   ctcaataaac taaacaacaa ccaacaacaa ctcccgatcc tacccaacca ataccaaaat 10620
   ctctatatac accacccgac gatcgtaatc aatctccata accacgacgc ttatatctac 10680
   caaacacgaa acaacacaaa aaaatcaaaa ataacctaaa actacctccc atcgtcccca 10740
   ccctacccca cttcctacct acctaacctt aacccctaaa aacaaaacta cgattccctc 10800
   gctaccaaat catataatca aaacccaaaa atataatatc taccctcaaa ttctaaccgc 10860
   tcttccacac tttataaata tcactaaaac aaatcttaaa caccaaaaac actaataaaa 10920
   acgaaaatta aaaataactt taactaatct aacctacacg acctaaacca actcaaaaca 10980
   taaaatttaa aatccgtata accaaaaaca aaaaaaaaaa ctaaaaaaaa aacaacctct 11040
   cctccaaaac cccctcccta accaatataa accatactca cacaaaaaaa caacctaaaa 11100
   tcgaaacgaa catatttcta tcaccactaa atccctaata tccatcattt attaattcac 11160
   tcactcactc tttcatccat tctttcaaca aatatttata aaacacctac taaatattcc 11220
   aaccactaaa aatacaacaa taaaaaaaac aaaactccta ccctctcaaa acttaccttc 11280
   taacaaaaaa cgacaaatca taaatacaat aaacaaatca taacaaaata aaaaaaataa 11340
   taataaaatc aaaacaatat ctaaaacaat ctaaaaaaac ctaactaaaa cgataaccct 11400
   aaactaaact aaaacataaa aaaaaccgcc gaaaaaacta taaaaacaca ttccaaacaa 11460
   aaaaacaaac gcaaaaccta aaataaaaaa acccaaaaac gacctactac aacccgctta 11520
   ccccaactaa taaactccca tttcatctcc acgtaaaaat cctaaaccta aaaaacgaaa 11580
   aaaaaaaaaa ctaaatctac actaaaaaaa aactcaaaaa ctcccgtaaa aactaaaaaa 11640
   ctaaacctca ccttacgcaa cttctccaaa aacacgaaaa tacccgccaa ccgcttcacc 11700
   acccgctaat aatcccgata ccgaaacacc aactacacca actccaaatc ccgaatactc 11760
   acaacctcct aaaacactaa aaaaaaacga aacaaaatca caaaacaaaa cccttcaacc 11820
   aaccataaat ctataatact aaaaaaaccc cgactataaa actaaaataa acaaaactac 11880
   ccacctatcc aaccgctacg attctcccta cccacgtcta cgccgtacgc caaaaacaca 11940
   cgaacacact caaaataccc caacgtaata accaaataca aaaaaatccg accgcgaaaa 12000
   tccaactact cgatatccac ctaaaaaaaa aaaaaaaata aaaaataaaa ccccaatcac 12060
   gtcgaaaaca accctccgaa accaaacaac cctaaaacaa acaacaaccc ccacccccta    12120
   ctaaaactac tctacctcac caacctcaca aaacaacgaa aaaacaaaca ctataaacaa 12180
   cgaaaaaaca aacactataa acaacgaaaa aacaaacacg taaataaaaa aaaacttaaa 12240
   aaacaaaaca aaaaataact ctaaaaaaaa aaaaacttcc tcccttccta tccaccaaaa 12300
   acccctaact aaatcctctc ctccaaaacc caataaccta cctaccaaaa ataaacctaa 12360
   aactaaaata aaataacaaa aaaactatta accaaaaaac tcacctaaaa ccaaacctac 12420
   cctaaaaatc ccaaaccacc ttcaaaaaaa actaccaatt taaatcacta acattatcac 12480
   taaataaaat ttaacaaacg attacaataa tacacaaaca aaaataaaat aacaaattca 12540
   taatattaat acaatttcac aaccatatcc tatacttata aaacactata cattaaaatt 12600
   tatacaaatc ctataatttt ataaataaca taattacatt acataaaatt tctattatat 12660
   aataaaaata ataaaaactt tttttaaaat ttttttttat atttttttta aaaaaacgaa 12720
   atctcactct attaactaaa ctaaaataca ataacgtaat atcaactcgc tataacctcc 12780
   gtctcccaaa ttcaaacgat tctcctacct caacctccca aataactaaa attacaaaca 12840
   ttcgccaaca cgtctaacta attttttata tttttaataa aaataaattt tcaccatatt 12900
   aaccaaacta atcttaaact cctaacctca aataatccac ctaactcgaa ctcccaaaat 12960
   actaaaatta caaatataaa ccgcaatacc caaacaaaaa attttttttt tttttttttt 13020
   tttttttttt tttacttaaa acaaaatctc actatatcac ccaaactaat ctcaaactcc 13080
   taaacttaaa caatcctcct atttcaacct cccaaacaac taaaattaca aacccctaca 13140
   cctaacttaa aaaacatatt tattaaacgt aaattttatt tatttattta tttatttatt 13200
   ttttaaaaca aaatctcact atatcaccca aactaaaata caataatacc atcttaactc 13260
   attacaactt ccgcctccca aattcaaacg attcttatac ctcaaccttc taaataacta 13320
   aaattacaaa cgaacaccac tataccaaac taatttttat attttaataa aaacgaaatt 13380
   ttaatatatt acccaaacta atctctaact cctaacctca aataattcgc ctaccttaac 13440
   ctcccaaaat attaaaatta caaacgtaaa ccaccacgcc cgatcttaaa cataaatatt 13500
   taaatatatt tcgaaaatct aaaacttcta attaaaaaca ataaactaac aaaccaaacg 13560
   cgataactca cgcctataat cccaacactt taaaaaaccg aaacgaataa atcacctaaa 13620
   attaaaaatt cgaaaccaac ctaacaacat aaaaaaaccc catctctact aaaaatacaa 13680
   aattaaccaa acgtaataac ccatacctat aatcccaact aatcaaaaaa ctaaaacaaa 13740
   aaaaccgctt aaacccgaaa aacgaaaatt acaataaacc gaaatcatac cactacactc 13800
   caacctaaac gacaaaaaca aaactccgtc tcaaaaaaaa aaaaaacaat aaactaacca 13860
   catacatata actccctcct cttctctaaa cttaattaaa acgccaataa aaaaacttct 13920
   actcctacca taataaataa aattactcta ccaccataaa caataaaaaa attattacta 13980
   caaaatatat aaaaactaaa cacaaaaact cacccctata atcccaacaa tttaaaaaac 14040
   aaaaattaac gaattactta aacccaaaaa ttaaaaatca acctaaacaa tataacaaaa 14100
   ccccatctct acaaaaaata caaaaaaatt aaccgaatat aataacctat acctataatc 14160
   ccaactactc aaaaactaaa ataaaaaaat cgcttaaacc taaaaaatta aaactacaat 14220
   aaaatatcat cacacctcta cactccaaac taaacaacaa aataaaacgc tatctcaaat 14280
   atatatatac atatacgtat atatatatat atatatatat atatatatat atatatatat 14340
   atatatttat atatatatat atatatattt ctctatataa aaaatatata taaaaaaaaa 14400
   taactatttt taaaaatcca aaactataat ccctaaaata aaaaaaataa acaaaataaa 14460
   ctctataaca tccctaaatt tctacctaaa aaaacattcc aaactataat tcaaaaaaaa 14520
   aaaacccgaa caaaacataa tactcttcct aaattaaaaa aataaaaaca aaattcaaaa 14580
   aaaccaaaat attaaaaatt acaacaaaac acaattctaa aaaaaaataa actactcaaa 14640
   aaactccaaa aatctacata aaaatctctt caaatcttcc taaatactaa acttcacata 14700
   tatataataa aactcaataa aaccacacaa aaaaaaacaa taaaataata aattctcaaa 14760
   actcaccaaa aacaaaaaaa taatcaaact ccaacctcct aaaataaaaa tacatattta 14820
   atcaaacctc aaaaaaatta taccttaaca ataaaactaa actatcccta aaaaaaaaac 14880
   tcacctaaac ccaccctaac aaattaaaaa ttaacctcaa accgaataca ataactcata 14940
   cctataatcc caacacttta aaaaacgaaa acgaacaaat cacctaaaat caaaaattca 15000
   aaaccaacct aaccaaaata acgaaaccac atctctacta aaaatacaaa aaattaaccg 15060
   aacttaataa taaataccta taatcccaac tactcaaaaa actaaaacac aaaaatcact 15120
   taaacccaaa aaacgaaaat tacaataaac caaaactaca ccactacact ctaacctaaa 15180
   taacaaaaca aaactccatc tcaaaaaaaa aaaaaaaaaa aaaaaaaaaa aaaaaaaaaa 15240
   aattaacatc aaaaaccaaa cacgataact cacacctcta atcccaacac tttaaaaaac 15300
   taaaacaaac aaatcactta aacctaaaaa ttcaaaacca acctaaacaa cataccaaaa 15360
   ccccatatct acaaaaaata caaaaaatta accaaatata ataacatata cctataacct 15420
   caactactca ataactaaaa caaaaaaatc ccttaaaccc aaaaaattaa aactacaata 15480
   aactaaaatt accccactac actcaaacct aaataacaaa acaaaactct atctcaaaaa 15540
   aaaaaacctc aaaaaaatca aactaattta caaataattt acctaatcta cctaaacaaa 15600
   acccaacact ctttaaaaaa caataacaaa tccaaatact taacatcata tttacaatat 15660
   ccaaaattca attaaaaatt actaaatata ccaaaaaata aaaaaataaa acttataaac 15720
   aaaatacgat aactcacgta aacctataat cccaacattt taaaaaacta aaacgaataa 15780
   atcacgaaat caaaaaatcg aacttacaat aaaccgaaat aacgccccta cactccaacc 15840
   taaacaacaa aaaaaaaaac tccatctcaa aaaaaaaaaa aaaaaaaaaa aaatataacc 15900
   tataatcaaa aaaaaaataa tttcataaaa acaaaactca aaaataataa aaataataaa 15960
   taactcaata tactcaaaaa tttaaaaaaa aacataaaaa aaatattcat aaaaaaaaaa 16020
   ataaaaaata taaaaaaaaa cccaataaac catctaaaaa taataaatac aataactaaa 16080
   ataaaaaatt cactaaataa aaataacaac aaattaaaca ctacacaatt taaaaatcaa 16140
   taaatttaaa aacaacaata aaactttcca aactaaaaca caaaaaaaaa aaacaatact 16200
   aaaaaataaa attaactaaa cttctataat cccaacactt ttaaaaacca aaacgaaaaa 16260
   ttaacttaaa ttcaaaaatt taaaaccaac ccaaacaata caacaaaact ctatctctac 16320
   aaacaaaaca aaacaaaaca aaacaacttt tacattaact aaacataata acaaacaact 16380
   ataatcccaa ctcctctaaa actaaaacaa aaaaatccct taaacccaaa aattcgaaac 16440
   tacaataaac tataatcacg ccactacact ccaacctaaa caacaaaaca aaaccctatc 16500
   tctataaaac ctcataatct ataaaacatt atcaaacatt ataacatata tctattaaaa 16560
   tcccaaaaaa caaaataaaa aaataaaaca aataaaacaa atttaaaaaa ataaaccgaa 16620
   tacgataact cacgcctata atcccaatac tttaaaaaac caaaacgaat aaatcacaac 16680
   atcaaaaatt taaaaccaac ctaaccaata taataaaacc ccatttttac taaaaataca 16740
   aaaattaacc aaacgtaata ataaacacct ataatcccaa ctacttaaaa actaaaacaa 16800
   aaaaatcgct taaacccgaa aaaaaaaaat tacaataaac cgaaatcgta tcactacact 16860
   ccaacttaaa cgacaaaaaa aaactccatt tcaaaataaa taaataaata ataaaaaaaa 16920
   aaaaaaacgt ccaaaatttt tccaaactta ataaaaacta ctataataaa ttaaaaaata    16980
   atacccctcc taaaacatat ccacatcaca ttctaaccct cacaacctat aaataaaacc    17040
   ttatttcaaa aaaaaatctt tacaaacata attaaattaa aaatctcaaa ataaaataat 17100
   cctaaatcac ccaaaaaacc ctaaatccaa taacaaatat ctttctaaaa cactcaaaaa 17160
   aataaaaaaa ccatataaaa acgaaaacaa aaactaaaat tctacaacca caaaccaaaa 17220
   aaattataac ccaatcacca aaatctaaaa aaaacaaaac aaaactcttc cctaaaacct 17280
   ttaaaaaaac taccacccta ccaacatctc aaatttatat ttatatctcc cgaactataa 17340
   caaaataaat tttttttttt tttttttaaa ataaaatctc actctatcgc ccaaactaaa 17400
   atacaataac gtaatctcaa ctcactataa actccgcttc cccgattcac gccattctcc 17460
   tacctcaacc tcccaaataa ctaaaattac aaacacccac caccacgccc gactaatttt 17520
   ttatattttt aataaaaacg aaatttcacc gtattaacca acataatctc gatctcctaa 17580
   cctcgtaatc cgcccgcctc gacctcccaa aatactaaaa ttacaaacgt aaaccaccac 17640
   acccgaccaa atatttatta ttttaaccca cctaatttat aataatttat tataaaaact 17700
   aataccaaaa taaaaccaaa aaccaaaaaa ttcaataaac cccaaataaa acaaacacga 17760
   aataaactac accaaaatcc atcataatca aatactaaaa actaataata aaaaaaaaat 17820
   cctaaaaaca ttcaaaaaaa taaaatccat tatatataaa aaaacaaata caaaaaatac 17880
   tatattcttc tcatcaaaaa ctatataaac caaaaaacaa tataataaaa tctttaaaat 17940
   actaaaaaaa aaaaacaaaa acaaaaactt tcaacctaaa attctttttt tttttttctc 18000
   taatccaata taactaatat taacctaaaa ttccataccc gataaaaata ttcttcaaaa 18060
   ctaaaaaaaa aataaatata ttaacaaaca aacaaattct aaaaaaattt attaccaaat 18120
   atacaaaaaa tattaaaaaa aattcttcaa ataaaaaaaa aataatccca aataaaaact 18180
   taaatctata caaaaaatta aaaaatacca aaaaatatta aatatataaa aatatcaaaa 18240
   aaccaaacac aataactcat acctataatc ccaacacttt aaaaaaccga aacgaacaaa 18300
   tcaccaaaaa tcaaaaattt aaaaccaacc taaccaacat aataaaaccc catctctact 18360
   aaaaatacaa aaattaacta aacgtaataa aaaattccta taatcccaac tactaaaaaa 18420
   actaaaacac aaaaatcgct taaacctaaa aaataaaaat tacaataaac aaaaatcgca 18480
   ccactacact ccaacctaaa taacaaaaaa aaactctatc tcaaaaaaaa aaaaaaaaaa 18540
   tatcaaacta aacgcaataa ctcataccta taaactttaa aaaaccgaaa taaacaaatc 18600
   acttaaaccc aaaaatttaa aaccaacgta aacaatataa taaaactcta tactacaaaa 18660
   aaatactaaa attaaccaaa cgtaataata catacctatc gtcccaacta ctaaaaaact 18720
   aaaataaaaa aatcccttaa accaaaaaac caaaaattac aataaacaaa aatcatccca 18780
   ctacactcca acctaaacaa caaaaccaaa ccctatctca aaaaaaaaaa aaaaaaaaaa 18840
   tcaataaaat atttaaaacc cacaaaaata aaaaaaaaaa atatcaacaa ataaaaaact 18900
   tcaacacaac tttaaaacat aaaaaataac taaaacctca caactaactt aaaaaaacaa 18960
   aaaaaactaa aactaaaaca accacaaaaa aaaacgtcca cataaaaaac aaaccaaata 19020
   acccccaacc cccctaaaaa atataaataa aacctacaaa caataaaaat aacaaaaata 19080
   aaaataaaac taaaactaaa tacaaaaaaa ctaattacaa ttcaaaatcc cctaatcccc 19140
   ttcccaacct cattaaacta aaaaataccc cccttcctca ccacaaaaca ccaaaaattt 19200
   attctttata aaaattaaac ataaaatact ctaaactcaa aaacattaac cacaacaaaa 19260
   aataaaatat aatactaaaa accaataatc cgtaaaaccc caaccccttc cctacctaaa 19320
   tcctaaacca ctaacaacca aatcacacca ccaccaccct atctacccga ataaaaaatt 19380
   atcccaaaaa cctcaaaata caaacatcta ataaccctac aataaacaaa ccaacctacc 19440
   acctaatcac cttacaataa cacccacaca tccctaaact ccccaacccc attcttaaat 19500
   ataacccaac aaccaaaaaa aactaaacac ttaataaaaa cttcccatat aaaataaacc 19560
   aaaacataca aattaaatac taaaacgact aaatacaata actcacgcct ataatcccaa 19620
   ctctttaaaa aaccgaaata aacgaatcac gaaatcaaaa aatcgaaacc atcctaacta 19680
   acacgataaa accccgtctc tactaaaaat acaaaaaatt aactaaacgt aataacgaac 19740
   gcctataatc acaactactc gaaaaactaa aacaaaaaaa taatataaac ccgaaaaacg 19800
   aaacttacaa taaaccgaaa ttacgccact acactctaac gcgaaaaaca aaacgaaact 19860
   ccgtatcaaa aaaaaaaaaa aaaaaaaaaa aaaaaacaaa ttaaatacta aaaacccaaa 19920
   aaaaattctc ttaatacaaa aaataaaaaa aaactcttta aaaaaaaaca ataatttaca 19980
   tcctcaaaaa aaatataaaa aaatcattaa attcataaaa caaaataaaa caacctaact 20040
   ttccataacg tctcctaccc tttctcttac cttcttcatt ctaaaaataa ctttcttaac 20100
   ttttccttat tttctacatt cctaaacaac tacacctacc cccataaaca ataaacatca 20160
   cctaaattta ctacattttc aaataaaaaa aaaaaccaac tttaaaacta tctacatctc 20220
   ttctttttcc cgcaccctcc acatctaacc catcaaaaaa acttataaac tctacctcca 20280
   aaacaaacct aaaaaccgcc caatttccac taccttacaa ctattacccc cccatctctc 20340
   actaacaacc ccctactacc tctttactac tccccgatta ccaaactcta ttatccacct 20400
   cacctataac aactaaaatc accctataaa atattactca aatcctaaca ctctttaatt 20460
   caaaatcttc caataacttt ccatcgtaaa taacaaaaaa tccaataacc ccaaacttct 20520
   ctttaaactc atttactcca cccttcgcca ataaccttcc ccgaaacgtt ctccaaaatc 20580
   ctccaaaaaa ccccaaaact ttctccctcc cttcactcaa actactactc aaatatcacc 20640
   tccttaaaaa aacccaccct aacaacccat ctaaaataac aactactata actctatcca 20700
   ctacccgtcc aaatacctat taaacacctc ctacacctcg aacaaaaacc ccatcaacca 20760
   aatccctacc tactttattc actactaatt ccccactccc caaacaacac ctaacacata 20820
   aatatttact aaacaaatac ataaaatact aaactaaaaa aacacaaaaa aaaaaaaata 20880
   attaaccaaa aaatcattac aaaaatatat tatttatacc gaaaaactca cgtctataat 20940
   cccaacactt taaaaaacca aaataaataa atcacttaaa ctcaaaaatt cgaaaccaac 21000
   ctaaacaaca taacaaaacc ccatctctac aaaaaaaaat acaaaaatta accaaacata 21060
   ataacaccac ctataatcct aactccttaa aaaactaaaa taaaaaaatc acctaaacct 21120
   aaaaaacaaa aactacacta aactataatc acactactac actccaacct aaataacaaa 21180
   ataaaacgct atctcataaa aaaaaaaaaa aaaaaaaaaa aaaaaaaaaa aaaaaaaaaa 21240
   aaacaaaaaa aaaaaaaaaa aaaaaaaaaa aaaaaaaaaa aaaacaatat aaacaatata 21300
   ttatttataa ttaaaaaaat aaataacaaa aaaaatccaa aatactaaaa aaataaaaaa 21360
   aacgaaacaa aaaacactac ttttcattac gcatcttcta acaacaatta aatattttaa 21420
   aacataacca taaactccat tatcaaaaat aaaacatttt aaataattta aaattattta 21480
   tataattttt ataaccttat cctaaaattt cttcattttt caatatacta aaaacctcaa 21540
   aaaaaccaac gaacctaacc cctattaacc tctaaactat aacccctcac aaaaaaaaaa 21600
   aatattattt actcttatcc aatcctaatt aaactaaact ccgaaactta caacctaaat 21660
   aaaatcctaa acccgacaac gaaaataaaa aaaaaccaca aaacccgaac taactaaaaa 21720
   aatactaaac aatccaaacc tcccaaccct accccaaata ctacaaccgc aaccgctaca 21780
   actaataaaa ccctaaaaaa ctaacaaacc gctacctaac ctcaaaactc caaatactac 21840
   ctccacctcc ccatcaaata cctcaactcc aaactccaaa aaacgactaa cacttcctta 21900
   caaacaaaat actcctcccc atccccacac ctcaaaaaca ctccccaaaa aaacctaaat 21960
   aactcaacac cccatacacc ccatacaaaa ccccatacac tcaaccaaac tccaaaacaa 22020
   ctctccccac aaacccaaaa taccgcctac aatataaata acaccttacc ctaaacccaa 22080
   ataaaaaaat tcattaatct cctcaacaaa aacctccaac ttctcctcaa caaaaacctc 22140
   taactattta attactaaaa taaaaaaatt tcccataaaa cccaacaccc aaccctcccc 22200
   accaaaaaaa cgaaaattcc ttatcccaac aaacctcaac taaaatcaaa aaataaaatc 22260
   tttaaaattt tataactaaa aatccctaaa caaaaaacct ccctacctcc actcaaacac 22320
   acacaccaca ctctaataca cactaatacc atacaaatac acactcgtac gctcataaat 22380
   actcacgtat acccttaaat atacaaacac atacataccc aactaccttc taaccctaca 22440
   aaaatccact cctatataac gtatatatac gtatttacac aaaaacaccc ctcacaccca 22500
   tatacactaa caaaatactt tccaaactcc tttaacataa acaaaatcct tttattaaaa 22560
   aaaatataaa aaacatacat ctcatatata aactaaacac aatacttacg aaaaaaataa 22620
   aaaacgcaaa acctatactc aaaataatac ctactcaacc ccttccccaa cccacgaaaa 22680
   aaaaaacaaa aatctaatat aatcgttact taacctctcc tacccaacgt aaaatacaaa 22740
   aacaaatcta cctaaactca ttcaaaaaaa aaaaaacaaa atacatcctc aaactacgta 22800
   aaaaaccacg aactataaaa accgcaccct aaacttctcc ctccatatca cccctcccat 22860
   cctaccctta caacccctcc ccctcatccc aaaacacatt aatttttaat ttctaaaatt 22920
   tataactccc tctcccgaac ttcctaactt accaactata ataatacccc acatccaaaa 22980
   ctacccaata taaacaaaaa aaaaaatcta aaatactcct cgcacatcct aaaccccaca 23040
   acccatctcc cctacctcta aatcaaaaaa ataaacaaaa aaaacaaaaa acaaacccac 23100
   gataaaaaac gccccaaaaa aaaaaaaaat aattctctaa aaccacaatc tacccacttc 23160
   aaaattctaa ttaccaaaaa caaaatccta ctccccaaac ccaacccaca catatccctc 23220
   ccacgaaaac tacacaaact aacccttccc cctattccca ccccaaaaaa caccaaaaac 23280
   tcacgactcc aaaaccaaaa ccatacccaa ctaaccatac caaacaaaaa aaaactaaca 23340
   tattccctaa cttacaaccc tatccccccc gacctaacaa aaaacataca taacgacaaa 23400
   caaaacaccg aactaacatc acaacactac taaatacccc ttcccttcac caaacacctc 23460
   ctatctaccc atcactccct acttccaacc tccaacaaaa caatttaaaa ctaaacaaaa 23520
   cccattaacc tactctaaaa ataaccatac taaccccctc ctcaaccaac tacactacca 23580
   aacctaccca cgaaaaacct ctacgaaact ttcttaaaat aaaaaaaaaa caactatacc 23640
   aaaccaaaac caaaaaaacc taactcctaa atacttaaaa aaaaccgaac tatttctata 23700
   acgactaata ccaaaaaaac taaaaaccaa aaacccctaa ctaaaatacc aaaattccac 23760
   aatacttttc tcataatatt aaataactat ccccaaatcc cccttacaaa aaacaaaacc    23820
   taatacccta aaaaataaac aatatacatc taaatacaaa cctcactcta aaccaatcct    23880
   cgaaaaaaaa aacaactaaa aaactctccc acctacacga aataacattc ctctttttaa 23940
   cacgcctaaa aaaaaaaaaa aactaaccta actcctctat aattaaacaa aaattaacct 24000
   ccaccaatac cctatattcc ccctaaccta aatccaaaac aaacaatatc catttcaaaa 24060
   aaacattaac tcactaaaac aaaaataaaa ataaatctaa aaccttattt ctaaaacaac 24120
   caaaaaaata aaaaaaaaaa acacataaac aacccccaac acctctacct acaacgactc 24180
   cgataccccc caccccaacc ttaccccaac aaaataacca cccacttcaa aattatcaac 24240
   aaaaaaacct actaaaaaaa tcttttctcc caaaacattc tacctctctc caatctctac 24300
   aataaaaaaa ccacccaccc tcaaacaaaa aaataaaact aaaacattct aaaaattcaa 24360
   ccttctacct aataatcctt aaacccctaa aaaattcttc ccacataaaa cttatcttct 24420
   tttactttca aaaaaaaaaa acaaaaaaaa accaacaaaa actaactaac catatcttaa 24480
   cgctttacac atcaccctaa aatttcctat tcacacttac ttcaccaaaa caaaaactcc 24540
   ttaaaaacaa aaattaatac cctatttatt catctatttc ctaaaaacac tataacccac 24600
   tctctttaac aaacttctcg taactacaaa acactacctt tataataaat aaacaaaaac 24660
   aaaatctaaa aaattcaatc taaaaaaaaa ctcgaaaaat actaacataa acacaaataa 24720
   caaaaaataa ccacccaaac cgacccaaaa acctacgaac acaaatcact cgcatattta 24780
   caaaaactac catacaaaac taaataccat acaaacctaa aaactaactc aacgtaaaac 24840
   ttcaaaaaca catcctaacc cccaacctac tcaatccgat aacctaccct ccacctccat 24900
   acttccctta acgattacac aactcctcaa actaaacttc tcataactca ataaacaacc 24960
   ctcaaaaaat aaattcccac aacgaacaac tacgctaacc acccccaacc cataacccca 25020
   aaatctcaaa cccaatccaa aactaaaatt ctcaacctac aacaaaccat aaaacccgaa 25080
   cccaactcta actcttacaa cccaccctaa aaacgaaaaa tcatctctac cataaatacc 25140
   tacctataac ctaaatacca aaaacaaaaa taacaatcta taacctatac ctacctttat 25200
   tctaaaacat aaccaaaaaa catcctaaaa cataaccaaa aaacatcccc ccttccccta 25260
   ttttctaaaa accactaccc aacccaaaac taataaccca cgtaacctta ctaaccaaca 25320
   aaaaccaaaa aacccaccta atacgcaaaa cctcaaatcc ttccaacaac caaaacccct 25380
   caacttcctc tacgcctaaa aaccaaaccc caacaccaaa aaacaaaaaa acgcttttca 25440
   acacctaaac aaaaccctta aaacctccca aaaaaatctt tctccactcc ctattcctaa 25500
   actaaaacta aactctaaaa aatccaaaaa tctacccaat ttaaaaacta aaaaaaaaaa 25560
   cgctcaccgt taaaaaaaaa acgctaaata aaactaaaat acctaaaaat ataacacaac 25620
   ctcttcccca ttcttaacct ataaccccct ccccacgcaa acctttccca cctccgcacc 25680
   ctcactcgaa aaatatcgcc gctcaactaa ctaaaacgaa ctctcccacc acataaaaca 25740
   ccgacacaac ccgcaacccc tccctacaac cctcactacc ccgacgaaaa aaaaacacaa 25800
   aaacctctac tctcatctaa ataaatccga aacacgtata atcccgcccg tcccgaccga 25860
   aaaaaatcac cctttcccga tcaacaaaca caaaaaccgc gaccgcaaac aaaaacgcgc 25920
   cgcaaactcg ccaaaaaaac gaaacaaccc gcgccgcacc ccctccccaa accgaacgaa 25980
   cgtacccgac cgcgaaatcc ccgcgacgcc ccgaaaacgc tcctacctaa cccgcgcgaa 26040
   cctccttctc caactcgcga taacgattat accacacgaa ataatacaac gaatacttac 26100
   cctcgaaccc cttcctaacg aaaacgttaa cgaaaatcat cgcccgctcc tcataccgaa 26160
   accgcgacgc ccgaccgaaa aaaaaaaacg acgccgaaac cgaaacccga aaccgcgaaa 26220
   cgaaaaaccg acgctaccta ctcgcgaaac tacgactcga acatatacga acgacgacga 26280
   acgaaaacga cgcgcccgac gcgatacgaa cgacgcgaac gacgaacgtc ctcgcgaaac 26340
   gacgacgacg aacgacgcga aaaaaccccc acccgcccta aaaaaaaacg aacactcgac 26400
   gacgctacgc gaactcgcgc tcgcctccca cacccccgca cacaaaaacg ccgctcgacg 26460
   aaaaaaacaa acgcccgcaa cgaacgcgaa aacgcacgaa acgcgaaccc cgaaaacgcc    26520
   gccgcaaaaa cgcgcgcccg caacccccga atccccacaa ctacataacc cgaccctcaa    26580
   cccgaacgac cgacgcgaaa tacgaaaata taaaaataat aaaaaaaaaa aaaaaaaacc 26640
   aaacgccccg aataaaactt atcaactcaa ccccgcgaca tcataaaatt tataatccgc 26700
   ctcgtaaaac taaaaataac aaataaccga aaaaaatcta cactatcccc gaatctcgta 26760
   aaaaaataaa cgaaaaatca aacctaaccc aaacgcgaac gaaaccctaa cgccaaataa 26820
   actaaaacgc acctcccccg ctaacatttt accttttact aaaaaaataa aaaaacacga 26880
   acctaaatcg aacgtttaaa aaacgaaata aaaatataat cttcaaaatt aaacttaaat 26940
   cccaaaaacc ataaaaaaaa atccgaattc ccaaaaaaaa acgtctaatt aaaaactcct 27000
   aaaaaaaata taaaacatca ataccaaaaa tcattaaaac aaccaaaaat ctaaccccta 27060
   aaacatttct aaaaaatctt ccctacccta ttaaaaaatt aaactccatc ttccacacat 27120
   atcattaaaa acaaaatatc caataacgcg aaaaaaataa tccctttccc cccgcacact 27180
   atccccaaca aaaaccaaaa taaaattaaa caaaaaaaac aacaaataac aactccgttc 27240
   ctccacccaa ctaacccttc aaaaaaaaaa ataactaatt ccaaaaacta tctccttcct 27300
   aaataacccc aaaataaaaa aatacttcca aaataatccc tacaaaaaat ataataaaaa 27360
   ttaaattaac ctctcaaacg ccaattacaa aacctaaaaa aaccaaaaaa cttaaaccaa 27420
   tccctaaaaa aaaaaaacta taatatcaaa aaaaccaaaa ttcgaataac tccaactacc 27480
   taaccttaaa aaaacaaact acttaatttt tccgactttc tcgatccact ccccttcaaa 27540
   ataaaaatct aaatactacc tcctaaaata aactaaaaat aataactacc ctaaaaacaa 27600
   cgcctccctc ccccaaccaa acgaaccgaa acaaataacg aaaccaaaca cgataccttc 27660
   ttaaacccac ctcaaaacaa aacaaaaata aaaaattcca caccgaccct acttaaaaca 27720
   acaatccaaa aaatatttat ataaaaaata ccaacaaaaa cttacaaact ctctcaaatt 27780
   tctttatata atcttccctc cttcctcctc cacaccctat aataccaaac cccaacgaaa 27840
   aaaaataaaa aaaaaacgaa ctacctcgcc cctttctccc gcctaaaaaa aaccaactaa 27900
   cgtctccgaa aacccgaaac cgctacccgc ctacaccacc aaaaatcaac aaaatatcgt 27960
   aactacaacc aatacaaaaa aaaaaactaa cgcgattaaa aaacatctaa aaataaccga 28020
   aaacatacct atacatttcc ttattaaatt atttttcgaa ccttatatat aattccacta 28080
   taacaatcct acttcctaaa ataataacat ctccacgaaa aaatccaaaa acccttttat 28140
   ctccaatccc aaacccaaaa atttaactat aacgaaacta aaaaaaacaa aatttaaacg 28200
   atatatccgc gccaaaacca ctcccaaata atctctaaac caaccaaaaa ataaaaaata 28260
   aaataaaaaa ataaacaaaa taaatcatcc ttcttcccca acaaaaccca aaaatccgaa 28320
   aacaaaaaaa attcaaaata aattataaaa aattatcgat tataaaaaac tatcccctaa 28380
   acgaccgtat tcttttttta ccaaacgacg ataaacacta aacccaaatc gcttacgctt 28440
   cgacccgcgc tctcaactac cacgtactcg aaacgcgccc acacaacgaa aaaacaaaaa 28500
   caaccaaacg acaaaactac caacaacctc ctcgacattt aataaataaa attccataac 28560
   taaaaatcga tcgaaaataa acgaacgacc accgctcgta aactaaacgc ctaacacgaa 28620
   ctacctaacc actcctcccg tctcactccc cccgcccctt acccccttcc cgctaaaacc 28680
   accaaaccca taaactctaa taaaaaaaca actcgaaacg acaaataata ctccccccta 28740
   cacaccaact aaatatcgtt acttacaaaa accccaccaa ctaaacgact aactatcgaa 28800
   aaaaaaaaaa cctacaaaat ttttttaaaa tacaattata aataaaaccc ataaatcgcg 28860
   atttcaaaca aacttatcaa tctaaaccgc aacaaaaaac gataaaaaca actttacata 28920
   tatttcgaat aatataactc ataaacatta cttatctccc ctaaaaaaaa aacctaccca 28980
   cccacaaatc actaccgctc taacaccgtt aaaataatcg accaacttaa acacccctac 29040
   caaaaaacta cccccatctt aacaaacaaa aatctctaac tcttaaaaaa cccctcctca 29100
   attatcacca ctaacctaac ccccaaatct aaccctaaaa acctctaccc tctccccaca 29160
   aacaaaatac ccgaaccttt tactactaca aaaattaaaa attaaataca cgcgcgacac 29220
   acgcgtataa aaacgcatac tcaatacgaa aactacgctc atatacgacc tctacctaaa 29280
   ctccctaccc cccaacccac ccacgtccct cacctacctt acctttacct acctcgtcgt 29340
   ataaaatcca aaaaaaaaaa ccgtcaccta aaacgacccc tctatattat atacaccctt 29400
   tctaaacaca cacacctcta ataacccaaa aacaccgtaa atatatataa aacgaataac 29460
   aaataaatat aaatacacac aaacacaata atcactcctc gaaaaatctt taaaaaccgc 29520
   ccaaattctc tcctctctaa ataaaaaaat acataaaaaa ataaatatat ttttaatccg 29580
   aaaacaaaaa aatacactac ctaacctttc taacacctaa acgtccatat tattaacaat 29640
   aaacgtattc aacaaaaata taacacatac acacacaccc ctaaacacac aaatataaac 29700
   acctacgaaa caaaaacgca cacctcaccc caaccaaaaa taaacgaata ccccaaaaca 29760
   caaccccttt aacctaaaac gaaacttaaa cctccaaatc cttcttccct aatccccacc 29820
   ctcgccgtcc gtaaacccaa cgtcccccga tccaccccac gcgaaaaata aaaaaaaaaa 29880
   aactataaaa ataactcaca ttacctccga accaaacgaa cgctacaacg aaaaccaaaa 29940
   ccgcaaacca cgctacccgc cgaaaccgaa catctaccgc gaccccttta aacgcccccg 30000
   ccccctccta cccgcgcccg aacttccttt ctctaaaaaa cgcgaccgac aacgcgcgac 30060
   gaaacgaaaa tacgacccct ccccctataa acaaaaccta cgccgcttcc tacaaaaacg 30120
   accgaaaacc gcgcaaaaaa cgaaaaaaaa aaccgcgaac gacgacgaaa cgacgcgaaa 30180
   cgaaaaacgc ccgaaaacga acgaacaaac gaacacgacg aattccgaac caaccccgaa 30240
   acgaacgatt aaccgaacgc gcgaaacctc gccgaaacct cgccgaaacc gaaccgaaca 30300
   acaaaaaccg ccgccgccgc ccgaacccgc cgcccgcgcc ccccacgcta acccaaaaaa 30360
   ctacgaccgc gtcgccgcta aaaatatccc gaaaaaaacc gcgaacgaaa atatatacga 30420
   actacaacgc cccgaataaa cgcgacgaaa ccgaaaacga aaccgcacaa ccaaaccgcc 30480
   gccgaacgaa cgccgaaacc taacaaaaac gctatacgaa accgaaaaac taaacgaaac 30540
   gaaaaactac gacctccaaa ctaaaccccc gcctcgacca ctacgaaact acgcccttaa 30600
   cgaaaaaacc gacctaaaat acccatactc cctaaaaacc gtaaactaac gaaaaaaaac 30660
   gttcccgacg ccgcctcccg ctcctcgatc taaaaaaaac gaacaactaa ctactcctcc 30720
   acctccatct ctatatccga cgaaaacgcc aacctaaaat aacaaaaaaa ccgtaacgaa 30780
   aaccgtcaaa acttaaaaaa taaaaacgaa tataatttta tatcatctca ataccacccc 30840
   cgcgtcttac acctactact cgcgtaatcg atacgcctac cgaaataata aaaaacaaaa 30900
   aacaaaaaac cgaacaaata cgccaataaa acctaaatac cgtctcctca aaaacaaata 30960
   aacgataact actcccaaaa ctccctacct cgaattccta aaactaacgc tccttaccga 31020
   acctaacgtt caaaaccaat ctaaacccga atatcttctc gaaaaacatc cccaaaccta 31080
   ctaataataa ctaaaaacaa ctcgactaaa ccctactatt cctattcacc gataaaacct 31140
   acttaaaaaa ataaacttaa catcaaacta tcctaaatac gaacccctcc ctatccccta 31200
   acccaatcta accaaaaata aataaaaaac cgtaccgaaa ttccccgtat cctactaact 31260
   tcctaaccta tctcctaact atatttacat aataattccc ttccttttcc taacaaaatt 31320
   aaaactaaac taaaataaaa ccccccaaac ctactaaaaa aatcaaaaat aataatccaa 31380
   aaaactactc ccttctaata aaaaaaaaca accaataacg aaactctaca actaacctaa 31440
   aaaaaaaact ctaacccaca tccattcttt ctttaacaaa aaaactatct ccctaataaa 31500
   cccctaaaca aactacgaaa atccgtaaac tctaacgaaa ctataaacat aattaaaaat 31560
   aaaaatataa aatactaatt tataaaccca taactttcaa ccacccctta aacaaaccca 31620
   aaaccccacc gattaaacct cacaaatact ctaataattc taaaaatacc aaactaaaat 31680
   atttaaaaac tataacttaa caaaacttaa acacaaaata ctccataaat aattataacc 31740
   aaaacctcct aaaaacttat cataatttaa aaaaccctaa aattaaaaat ccatacccct 31800
   aaaaaactat aactccgttc cccgaactac aaacaaatct aaaaataaca aaactaaaaa 31860
   tacaccttac cttataaata tctccccact attccacaac tccgtaaaat aaacataaca 31920
   taaaatataa aaaaaaatct taacaaacgc ccaatcgaaa aaatctaaaa cttaatatta 31980
   cctttattcc caacttactc tataatccca acaaaacttt cctcactata aaatcctcaa 32040
   cttcctcaat tcaaaaacaa aaaaattaaa ataaaaataa tttttaaaaa actaccatct 32100
   ccgaatctat aatctaattc taaaataaaa aaataaaaaa caacaccaac aaaacaatta 32160
   tcctttatat attacgttaa atatccgacc acaacctcta aatacccaat aaactcaaaa 32220
   aaaaataata aacatcaaat atattaaatt acccaactcc tcctcccaaa tcctcatcta 32280
   tcaaaaaaat ttatacccca aatacctcct ttcccctaat tcacaataaa aatcaaaaaa 32340
   ataataaaaa aataaaaata ataaaataaa aaaaatataa acaatcttac tcatcaaccc 32400
   acctaacacc caacgaaata tccaacacat aacaaacact ccctaaatat ttaactactt 32460
   attaaaaccc taactaacta cttaaaacta tcccacacct aaaccctaaa atactaaccc 32520
   taacatacaa cgacatattt aatctaaatc ctataacatc tcaaacaaaa aaacataaaa 32580
   atacccaaca atccgaaatc caaatccaat accaacccca taatatatcg ctccacccca 32640
   aaatcacaaa cttcttttat tataataaaa accctacaat cctaaccaca aaaataaaac 32700
   caaaaacttt ttatacttaa ttaatttaaa aaatcataaa aatcaaaaat aaaatacaac 32760
   ctatccccaa aaaaccccat cctccctcta ctcccgaaca taaacacaca aaacaaatat 32820
   aaatatatta ctaaacaact aaacataaca taactactct aacaacttat accacctcgt 32880
   caaacaccgt atatattcct acctacctaa aaaatccaaa aaaaaaaaaa aacaaaaaac 32940
   cctaaaaaat atacatcttc ccctttcctt aaacttaacc cacctcgcca cacgatctca 33000
   atcaaaaacc tctcctcacc ccataaactt ctatctcaac cttttccact ttaacaaccc 33060
   taaaaataaa aataaaaacc tcaaaatacc tatcctataa atatctctaa taaacaaaac 33120
   cgaaaacata ttacgaaaca aactacaaac taatttatct cttcataata taaattacaa 33180
   aataaaacaa atattaaact cctcttcctc ccttataaaa ataaaattta tatatatcaa 33240
   tacctacacc taaatattca tcctaataaa cttttacata tatatatacc catataacta 33300
   ctactctaat catattataa aataaaaata tccaatcttt ttacttccct aaaccacatt 33360
   aaaaaaaaaa ttttcttaaa ccacacctaa aatacgctaa cactaataat aactaataaa 33420
   ctaaataaaa caaacaaaaa taaaaataaa aaataaaatc acaaaaaaat ctcataatat 33480
   tttaaaaaaa tttataaatt tatattaaac cacattcaaa actacctaaa ccgcatataa 33540
   actacaaatt aaacaaacat attataaaac atttccatca ccttaaaaaa cttccaaata 33600
   ccccctccca atccgtacac cccaaaaata accaccattc taactctacc ccaaaaacta 33660
   ctctcgccta ccttcataat ataaactttt cccttaatct aataaaaatc taacccttaa 33720
   ctcaaaaaaa aaaaacctaa aactaaccta tatatacaaa cctactaacc ctaaaaacca 33780
   ctaataactt caaataataa caaaaattca ccgtaataaa aacctccata aaattaacac 33840
   taattaacgt aatcccaaaa tcacgcctac tacctctcca cacatacaaa cccataccct 33900
   accgataaaa ccaaaaacgc aaaaaaaaaa ctaacgaatt cccttacaat tcctatcctt 33960
   tccgtcccaa atttaatcca aaaaacttct taacgcaaaa atcccttcac cttaaactac 34020
   aatctaaata atcccaaaaa cacttacaca aaaaaaaaaa acaaaataat taaaataaac 34080
   gtcccatcta tatcatccaa aaataaacta acaccaataa cgactaccca aaccaaaaca 34140
   atatccttta cctaccattc ttctcaaatc acctaaaaaa aaaaaaaaac ccctccccca 34200
   aactaaaaaa aaataccaaa caaaccctta aaccacactc ctacatccta atcttaatta 34260
   ctaccataac caaaatcact caccacatct aatctaccca taacttaaaa ctctaaaacc 34320
   attaaccgta aatatataat caaccttcca tacctaactt ctccgataaa accacaaacc 34380
   tcaaatcaac ccatttataa tcctacccta aacgactccc cacttacaaa aaaccccacc 34440
   aaaaaaaatt taaactacat catttccgac ttcctaccta aactactaaa aataactaac 34500
   taacaacccc ctaacccaat aatatttaaa ataaaaaaaa aaaaatctcc acacctaaaa 34560
   tacctcacta tttaaataaa accaacccaa aaatacatac taaaactccc cctcttaacg 34620
   aaaaaaacta atcccttcct cctaaaccct aaaatcaacc ccccaaaaaa atattaacga 34680
   tcaccttaaa catccctcta ttaccctaat taaaaaaaaa tcccttaaaa aacctaaacc 34740
   cgctacactc caacctaaac gacaaaacga aactccgtct caaaaaaaaa aaaaaaaaaa 34800
   aaacctaaac ccaaaaacaa acctctctcc tttacttcct attttcatac ctcaataaaa 34860
   atcacttact ataaacaaaa ctcaaactaa acccctaact caaaatctcc tcaattttcc 34920
   taaaaacttt aataaaaatc cttaaaaaaa aaaaacctaa aaattacctt ctaattaaaa 34980
   aaaattctta tcc 34993
<210> 4
   <211> 34993
   <212> DNA
   <213> Artificial Sequence
<220>
   <223> G to A (bis2 rc)
<400> 4
   aaataaaaat ctctcccaac taaaaaacaa ttctcaaacc tttcccctct aaaaacttcc      60
   accaaaattc ccaaaaaaat taaaaaaact ctaaatcaaa aacccaacct aaactctacc     120
   cacaacaaat aacccccact aaaacataaa aacaaaaaat aaaaaaaaaa aacctatctt     180
   taaatttaaa cttttttttt tttttttttt ttaaaacgaa atcccgctct atcgcccaaa     240
   ctaaaataca acgaatttaa actttttaaa aaaccccctc ccaatcaaaa caacaaaaaa     300
   atacccaaaa taaccgccaa caccttccta aaaaactaac tccaaaactc aaaaaaaaaa     360
   aaccaacctt cttcgccaaa aaaaaaaacc ctaacataca cttctaaact aactttaccc     420
   aaataataaa acaccccaaa tataaaaacc tccctctccc cactccaaac accactaaat     480
   caaaaaacta ccaaccaacc acctccaaca acccaaacaa aaaaccgaaa ataatacaac     540
   ccaaattcct cttaataaaa tttcctataa ataaaaaacc gcccaaaaca aaatcataaa     600
   taaactaatc taaaatctat aatcctaccg aaaaaaccaa acataaaaaa ctaactatat     660
   atccacgacc aataactcca aaaccctaaa ccataaacaa accaaatata ataaataacc     720
   ctaactataa caataaccaa aaccaaaata caaaaatata atccaaaaat ttacctaaca     780
   ctttccccca acctaaaaaa aaaattctcc ttctcctcca aataacctaa aaaaaataat     840
   aaacaaaaaa cactatccta atctaaacaa ccgctattaa tatcaactca tttctaaata       900
   acacaaataa aacgcccacc ctaaccattc tatctcctcc ctctatacaa atacccttaa       960
   aactacccaa attataattt aaaataaaaa aatccttacg tcaaaaaact ccttaaacca    1020
   aacctaaaac gaaaaaaaca aaaactataa aaaaactcgt caaccctccc cctacgcttc    1080
   taactccacc gacaaaatat aaatctatat atataaaaaa ataacaaacg taatcctaaa 1140
   atcacgttaa ccaataccaa ccccataaaa acctctacta cgataaattc ctaccaccac 1200
   ctaaaaccat caataacctc caaaaccaat aaatttacac atacaaacta acctcaaacc 1260
   tccctcccct aaactaaaaa ccaaatctcc actaaactaa aaaaaaaacc cacatcataa 1320
   aaacaaacga aaataatcct taaaataaaa tcaaaataat aattacctct aaaatacacg 1380
   aactaaaaaa aaatacttaa aaacccccta aaataataaa aatattctat aacatactta 1440
   tctaacctac aatccatata cgacccaaac aactttaaat ataacccaac acaaattcat 1500
   aaactttctt aaaacattat aaaacttttt tataacttta tttttatttt ttatttttac 1560
   ttattttatt taactcacca actatcatta atattaacgt attttaaata taacccaaaa 1620
   aaattcttct tccaatataa cccaaaaaaa caaaaaaatt aaataccctt attctataac 1680
   ataatcaaaa caataattac ataaatatac acacatataa aaacccatca aaataaacac 1740
   tcaaatacaa acattaacac acacaaattc tacctccata aaaaaaaaaa aaaaacccaa 1800
   catttatccc actctacaat ctacactata aaaaaacaaa tcaacctata acttatcccg 1860
   taacatatcc ccgaccttac tcaccaaaaa tacctacaaa acaaacatct taaaaccttc 1920
   actctcaccc ccaaaattac caaaataaaa aaaactaaaa taaaaaccca taaaataaaa 1980
   aaaaaccctt aactaaaatc gtataacgaa ataaaccaaa cctaaaaaaa aaaaaaaata 2040
   cacattctcc aaaactccct acccccttcc tctcttaaac tccccaaaca aacaaaaaca 2100
   catacgatac ctaacgaaat aacacaaact accaaaacaa ccatatcata tccaactacc 2160
   taacaacaca ctcacactta tcctatatac ccatacccga aaacaaaaaa aaaataaaac 2220
   tctctaaaaa caaactacat ctcacctcta acctccataa ccccccaaat taaccaaaca 2280
   caaaaaatcc ctaaccccac tcctataatt aaaactacaa aactcctatc ataataaaaa 2340
   aaacctataa ccctaaaata aaacgataca ccataaaact aacactaaat ctaaactccg 2400
   aactactaaa taccctcata cctctctacc taaaatacca caaaacccaa accaaacata 2460
   ccgctacata ccaaaaccaa catcctaaaa tccaaatata aaataatccc aaacaaccaa     2520
   ccaaaatcct aacaaacaac caaacactca aaaaatacct accatatact aaacacctcg     2580
   ctaaatacta aataaactaa taaataaaac tatccacatc ctccccaccc caccatcctc 2640
   accttctcac caccttccta acctctatta taaatcaaaa aaaaaaaaac atctaaaaca 2700
   caaattcctc taacaaataa aaacctaaaa aaaaaaacta aacaatccaa cacatctaat 2760
   acccaccatt cctccctaaa cccactaaac atccaaaaac tataatcgaa cattcaacgc 2820
   aacatacaaa aaacaactac cttactaata ctacccttca cccctccacc ccaaaatcaa 2880
   accacaaatt cgaaaataac aaccccccaa aaaccatttc tactccaact tctttacctc 2940
   taaattaaaa aaactaaaaa ccccacaata aaaaaaatct tattaaaatt acaaaataaa 3000
   ctaaaaacaa aaacaacact aaaccccaaa ctcccccgat taaacgccta ctaaaactct 3060
   cctccacatt ccataccata cttaccccac gaaattataa aacaataaaa aaacatttat 3120
   aaaacaaaat acatcctcaa ccctaccatc cccaaaccta cctacaactc gaaaaacgaa 3180
   atcacaattt cttaaaaata taaaccctca accccaaaac cctctaaacc ataataaacc 3240
   cctaaaaaac cttaatcata accacctata aaatatttta tacccaaacc ctactaaatc 3300
   ataattccca aatactctaa cttaacaccc ccaaaaccac caaaacatct ataaaactca 3360
   accgataaaa tcctaaacct actcaaaaaa taactaaaaa ttataaaccc acaaaccaac 3420
   accccatact ctcactctta atcataccca caaccccgct aaaattcacg aacctccgca 3480
   acctacccaa aaacctacca aaaaaataac tcctttacta aaaaaaaaat aaatataaac 3540
   taaaacttcc ttcccaaacc aactacaaaa ccccgtcact aactatcctc cctcatcaaa 3600
   aaaaaacaac ctcttaaatc attaccccta acttcctcaa caaacctaaa aaatttcact 3660
   ccaacccaat cctaactcta ttaaaaaaaa aaaaaaaacc accatataaa tacaaccaaa 3720
   aaacaaacca aaaaaccaac aaaatacgaa aaatctcgac acgacctccc acccatttct 3780
   aaccaaacta aactaaaaaa caaaaaaaaa cccgcaccca aaataactta ataccaaact 3840
   cactttccca aacaaacctt accgataaac aaaaacaaca aaatccaacc gaactattcc 3900
   caatcaccac caacaaacct aaaaatacct cccgaaaaaa cacccgaatc taaactaacc 3960
   ctaaacgcca aacccgacaa aaaacgctaa ccttaaaaac ccgaaacaaa aaatcctaaa 4020
   aataaccacc gctcatctac tcctaaaaaa acgatattca aaccccacta acgcatctac 4080
   ccgaccctct actccttacc ctccaccacc ccgacaaacg taccgaccac gcgaacaaca 4140
   aatacaaaac gcgaaaataa cactaaaata acataaaacc acactcgccc ccacctccca 4200
   aaccctaacg acctccgcca cgaccctcct accaccccaa actaacgctt ccgtcgaata 4260
   caaaaataaa aataaaaaaa caaccaacta cccgcctccc tcaaaccgaa aaacgaaaaa 4320
   cgacgccgaa aacgtccttc ctcgctaatc cacgacctcc aaaaaacata aacaccccaa 4380
   accgaccctc ccgccaaaaa cgcaactccg caataaccga aacgaaaacc caacctaaaa 4440
   accgcaaccc cccgccccgc ccaaccctcc gaccccgcac aacgctctta ccaaaccccg 4500
   acgcccgccc gacgacgacc taactatacg atcccgcccc cgaccccgcc gcgcttaccc 4560
   gaaacgctac aatccgcaca cacctccgct cgcgacccct ttcgaaacat cctcaacgac 4620
   gacgcgaccg caaccctcta aaccaacgta aaaaacgcga acgacgaacc cgaacgacga 4680
   cgacgacccc tactacccga cccgactccg acgaaacccc gacgaaactc cgcgcgcccg 4740
   accaaccgtc cgccccgaaa ctaacccgaa acccgccgta cccgcctacc cgctcgccct 4800
   cgaacgccct ccgccccgcg ccgccccgcc gccgctcgcg actcctctct ccgcccccta 4860
   cgcgactccc gaccgcttct acaaaaaacg acgcaaaccc tacccacaaa aaaaaaaacc 4920
   gcacccccgc cccgccgcgc gctaccgacc gcgcccccta aaaaaaaaaa acccgaacgc 4980
   gaacaaaaaa aaacgaaaac gcttaaaaaa accgcgacaa atacccgacc ccgacgaaca 5040
   acgtaaccta cgaccctaac ctccgctaca acgctcgcct aacccgaaaa caatataaat 5100
   caccctcaca accccctccc cttcactttc cgcgtaaaat aaaccgaaaa acgctaaacc 5160
   cacgaacgac gaaaataaaa attaaaaaaa aaaaacttaa aaacccaaat cccgctccaa    5220
   atcaaaaaaa ctatacctta aaatacccgt ctatctctaa ttaaaataaa atatacgtcc    5280
   ctatcccgta aatatccaca tctatatatc caaaaatata tatatatata tcacactctt 5340
   actaaatacg cccactacta acaatataaa cgcccaaata ccaaaaaaat caaacaatat 5400
   attcccctac ctccgaacta aaaatatatc tatctctcca tacatccttc catccaaaaa 5460
   aaaaaaaatc taaacgattt ttaaaaacct cccgaaaaat aaccactata cctatataca 5520
   ctcacatcca cttatcatcc gccttacaca cacccacgat atttctaaac caccaaaaat 5580
   atatatatcc aaaaaaaata catataacat aaaaaaaccg tttcaaataa cgacctttct 5640
   tcctaaactt cacacgacga aacaaacaaa aacaaaacaa ataaaaaacg taaataaact    5700
   aaaaaacaaa aaacctaaat aaaaaccgca cataaacgca acccccgcac taaacatacg    5760
   cccccatacg cgtataccgc gcgtataccc aacccccaat ccctacaaca acaaaaaacc 5820
   cgaacactct acttataaaa aaaaaataaa aatccccaaa accaaaccta aaaaccaaat 5880
   caataataac aattaaaaaa aaattctcca aaaatcaaaa actcctactt attaaaataa 5940
   aaacaatcct ttaacaaaaa tactcaaatt aatcgattat cccaacgata ccaaaacgac 6000
   aataatttat aaataaacaa actccttccc taaaaaaaac aaataatact cataaatcac 6060
   accacccgaa atacatacaa aactacctcc atcgcctttt actacgaccc aaattaacaa 6120
   atttatttaa aatcgcgatc cataaacttt atttataatt acaccttaaa aaaatcttac 6180
   aaacctcccc tccctcgaca accaaccgct caactaataa aacttttaca aacaacgata 6240
   cctaactaat atataaaaaa aaacaccacc taccgccccg aactactctc tcaccaaaat 6300
   ccataaactt aataacctca acgaaaaaaa aataaaaaac gaaaaaaata aaacgaaaaa 6360
   aataaccaaa caacccgtac caaacgctca atccacgaac gataaccgcc cgttcacccc 6420
   cgatcgaccc ccaactataa aatttcattt attaaatatc gaaaaaacta ctaacaaccc 6480
   taccgcttaa ctatctttat ttctccgcta tataaacgcg tctcgaacac gtaacaacta 6540
   aaaacgcgaa ccgaaacgca aacgacctaa attcaatatc taccgccgcc taacaaaaaa 6600
   aaaacacgac cgcccaaaaa acaattcccc acaatcgaca actccccaca atccatccta 6660
   aactccccct atcctcgaac tcctaaactt tactaaaaaa aaaaaataat tcactctact 6720
   tacccctcca ccccactccc catctcctaa ttaactcaaa aactacctaa aaataaccct 6780
   aacgcgaaca catcgtccaa actttatttc ccccaacccc gtcacaacca aacttctaaa 6840
   cctaaaacta aaaataaaaa aatttctaaa ttcccccgta aaaatatcac caccccaaaa 6900
   aacaaaacta ctacaataaa atcacacaca aaattcgaaa aacaatttaa taaaaaaata 6960
   cacaaacata ctcccgacca ctcctaaata cccctcaacc gcgccaaccc cctcccctac 7020
   actaactaca accacgacac tctactaacc cctaataata caaacgaaca acgatcccga 7080
   acccccgaaa acgccaacta actcctccca aacgaaaaaa aaaaacgaaa caacccgctc 7140
   ctccctcatc tcttcccgct aaaacctaac accacaaaat ataaaaaaaa aaaaaaaaaa 7200
   aaccacacaa aaaaacctaa aaaaatttac aaacttttac taacacctcc cacacaaaca 7260
   cttcttaaat tattatttta aataaaaccg atataaaatc ccttattcct accttattct 7320
   aaaataaact caaaaaaaca ccgtacctaa tcccgccacc tatctcgacc cgcctaatta 7380
   aaaaaaaaaa acgctatttc caaaacaatc attatcctta actcacccca aaaaataata 7440
   tctaaattct cattttaaaa aaaaataaac cgaaaaaatc gaaaaaatta aacaatttac 7500
   tcttttaaaa tcaaacaatt aaaactattc gaactctaat ctctctaaca tcacaatccc 7560
   ctccctccaa aaactaatcc aaacctctta atccttccaa accttacaat taacgcttaa 7620
   aaaaccaatt caacttttac cacaccctct acaaaaacca ttctaaaaat acctcttcac 7680
   cctaaaatca ttcaaaaaaa aaacaatctc taaaatcaat cacttccctt cctaaaaaac 7740
   caactaaata aaaaaacgaa actaccacct actacttttt ctatccaacc tcactctaat 7800
   tcttactaaa aacaatatac gaaaaaaaaa aaaccatctt tcccgcgtta ctaaacattc 7860
   tatttccaat aacatatata aaaaataaaa tttaattttc taacaaaaca aaaaaaactc 7920
   ctcaaaaata ttccaaaaac caaaccccta actactccaa taacccttaa tactaatacc 7980
   ccacaccttt ctcaaaaatt cccaatcaaa cgctctcttc taaaaatccg aatccttctt 8040
   cataacccct aaaacccaaa ctcaattcta aaaaccacac ctccaccccg tctcccaaac 8100
   gctcgaccta aacccgtacc cccccacttc cccaacaaaa aacaaaatac caacgaaaaa 8160
   aatacgtccc aacccaccta acgtcaaaac cccgcccgcg tctaaaccaa acctaactcc 8220
   ccgcccactc tcccacgaaa tccgaaaaca atacaaaccc tcttcgacca cctaccaccc 8280
   ccaatttcac gaaacgaact acaaacccca taataccgcg aaactaaact aacaaactct 8340
   acccgaaacg cctaatcccc ttcctctctc cccatcatcc ccacatcccc gtatcccgcg 8400
   ccgaccgctc gaactaaaaa ccgaactata caactataaa aacccgaaaa ctacgaacgc 8460
   gcgtccctac gacgacgtcc ccgaaacccg cgtcccgtac gcccccgcgc ccgctacgaa 8520
   cgcctactcc ctccgccgaa cgacgtcttt atatacgaaa atataaaaaa cgaacgcgaa 8580
   tccgcgcaac gccgccgaat acccgctccc tcccaaaacg aataaaaacc tctccgcgcc 8640
   gcccgccgcc gccgcctcgc gaaaacgccc gtcgcccgcg ccgcccgcac cgcgccgaac 8700
   gcgccgcccc cgcccgccgc cgctcgcaca tacccgaacc gcaaccccgc gaacaaacaa 8760
   cgccgacccc ccgccccgcg accccgaacc ccgactccga cgccgtccct cctccccgac 8820
   cgaacgccgc gaccccgaca taaaaaacga acgataatcc ccgccaacgc ctccgccaaa 8880
   aaaaaacccg aaaacaaata tccgctacac tacctcgtat aacacaaccg ccaccgcgaa 8940
   ctaaaaaaaa aaatccgcgc gaaccaaata aaaacgcctt cgaaacgccg cgaaaacccc 9000
   gcgaccgaac acgcccgtcc gacctaaaaa aaaaatacga cgcgaactat cccgcctccc 9060
   taacgaattt acgacgcgtc tttatttacg accgcgactt ttatacttat taaccgaaaa 9120
   aaaataatcc cctccgaccg aaacgaacga aaccacacgt atttcgaact cacctaaata 9180
   aaaataaaaa cctctatacc cctcctccgc cgaaacaata aaaactacaa aaaaaaacta 9240
   cgaactatat cgatattcca tataataaaa aaatccgccc caaccaatta aacgacgaca 9300
   cctcccgaat aaaaatacga aaataaaaaa aacttacgta aaaaaaaaac cataaatcaa 9360
   aaataaaaaa aaaactatat cacactccca aacaccccaa ccccacccaa cgccctctcc 9420
   ccaacgataa acgctttctc ttttaactct caaactaaac aaatccttaa atctctcaaa 9480
   acccaacccc aactcaaaaa caaaaaataa aaaaaaaccc tcttaaaaaa ccccaaaaac 9540
   cttatccaaa tactaaaaaa cgctctccta ccctctaata ctaaaaccta atttctaaac 9600
   gcaaaaaaaa ctaaaaaacc ctaactacta aaaaaactta aaattctacg caccaaataa 9660
   actccctaat ttctactaat caataaaacc acgtaaatca ccaactttaa actaaacaat 9720
   aatctccaaa aaacaaaaaa aaaaaaaata tcttctaacc atacttcaaa atatcttcta 9780
   accatacttc aaaacaaaaa caaacacaaa tcacaaatta ccacttctac ccttaacacc 9840
   caaaccacaa ataaacactc ataacaaaaa taaccccccg cctccaaaat aaactacaaa 9900
   aaccaaaact aaacccgaat cccataactt actacaaact aaaaacccca attttaaact 9960
   aaatctaaaa ccttaaaacc ataaactaaa aataattaac gcaattaccc gctataaaaa 10020
   tttaccttct aaaaactact tattaaatca taaaaaattc aatctaaaaa actatacaat 10080
   cgccaaaaaa aacataaaaa taaaaaacaa accatcgaac taaacaaact aaaaatcaaa 10140
   atatatctct aaaatcccac gctaaaccaa cccccaaacc tacataacat ccaactctac 10200
   ataacaatcc ctataaatat acgaataacc tatacccgca aacctctaaa ccgatctaaa 10260
   taaccacttc ctattaccta tacctatacc aacactctcc gaatttctcc tcaaactaaa 10320
   ctcctcaaac cctatcttta tttatctatc ataaaaacaa tactctacaa ccacgaaaaa 10380
   cctattaaaa aaaataaatc acaatacccc caaaaaacaa ataaacaaac aaaacatcaa 10440
   cccctaccct caaaaaatct ctattctaat aaaacaaata taaacaaaaa attccaaaat 10500
   aatatacaaa acgctaaaac ataaccaacc aacctctact aactcttctc tactttcctt 10560
   ccctaaaaac aaaaaaaaac aaacctcata taaaaaaaac ttcccaaaaa tccaaaaatc 10620
   actaaacaaa aaactaaatc tccaaaatac ctcaatccca ctcccttacc taaaaataaa 10680
   taatcctttt actacaaaaa ctaaaaaaaa acaaaatacc ctaaaaaaaa aaacttctcc 10740
   aacaaacctc tctactaata atcctaaaat aaataaccat cttactaaaa taaaattaaa 10800
   ataaaaaaca ccgaaaccgc tacaaataaa aatattaaaa actactcata tatttctctc 10860
   tcccacccct ctaactacct caaaaacaaa atcccaaatt cacccctacc cctaccccaa 10920
   taaattaata tctccttaaa ataaacacta cctatcctaa atccaaacca aaaaaaatat 10980
   aaaacactaa taaaaattaa cccttattca accacaaaaa aaccaaacca atcctctcct 11040
   tccctcaaac gtaccaaaaa aaaaaatacc actccgtata aataaaaaaa ttccctaact 11100
   atctcccctc tcgaaaacta acccaaaata aaacttatat ccaaatatac actacctact 11160
   ccccaaaaca ccaaactcta ttttctacaa aaaaaaccta aaaacaacca cctaacatca 11220
   taaaaaaaac actataaaac cctaacatcc caaccaaaaa tccctaacct ctaatccccc 11280
   taataccaac cgccacaaaa acaactcgac tttcctcaaa cactcaaaaa ccaaatttcc 11340
   ctaatcttaa cttaacataa ctacctcttc ttcactccaa aaaaaccccg caaaaaccct 11400
   ccgtaaacaa acttaacaat acaactaact aaaaaaaaaa ccaacataac caccttcaaa 11460
   acaaattaat aaactttact caaccccaaa ttactctact aaaaactaaa aacaaaaaat 11520
   aataaacaaa taaaaaatac ctaataaaaa aaaaaaacac ccaacaatac tataatacca 11580
   acccgatacc ctacttaccg ctatacatat tctctaccaa atcgaaaaaa acaaaattat 11640
   aaaccaaaaa atataccaat tcccccttac ctaacataac caactaaaca taatcctaat 11700
   tctaaaatcg taaattccta atattcccta aaataaaaac aaaaaaaaaa attaacctat 11760
   acaactcccg taaaaaaaac atatataaac taaacctaaa aaacaaaact ctacccctaa 11820
   caaccaaaac cctaaaataa acaaattata atcccaaaaa atcattttcc ttctccctaa 11880
   aacgttctcc accgtaaact tacttcctac tttccctatt cacccttcta acctaaaaac 11940
   aaaaaaaata aattataaaa cccaaaatat acgaaaaaca cctcaaaccc ctccctttac 12000
   ctacactaaa caatcctaaa tataaaacat caccacaact aataaactaa aaaactcgaa 12060
   aaaaaaaacc acaaacccca aaaaccaaaa atcaatatat tctaaaataa aaaaaaaaaa 12120
   ctacaaaaac aaaataaaaa aaataacata aaaaaaaaaa tccaaaatac gatccccaca 12180
   acccgtaacc tcctacgcaa cttaaaaata catcctacct ccctcccctt aaataaactc 12240
   aaacaaacct acccctacac cccacgctaa acaaaaaaaa tcaaacaacg actacaccaa 12300
   actcctacct tcctcctcgt aaactaaaaa aaaaactaaa caaacaccac cttaaacaca 12360
   aactctacgt ccctcacctt cctcgcaaac actatattta atccatacat aaaatatata 12420
   cttttcatac ttcccttaac aaaaaaattc tactcatact aaaaaaattt aaaaaatacc 12480
   ctactaatac acataaatat aaaaaatatt cctatacaaa tacgcacaca cacgttacac 12540
   aaaaataaac tcctacaaaa ccaaaaaaca actaaacata catatactta tacacttaaa 12600
   aacacacgta aatacctata aacgtacgaa tatatatcta tataatacca atatatacta 12660
   aaatataata tatataccta aataaaaata aaaaaactct ctacccaaaa acccccaact 12720
   acaaaactcc aaaaactcca ccttttaact ccaactaaaa cctactaaaa taaaaaatct 12780
   ccgttctctt aataaaaaaa actaaatatt aaatttcata aaaaaccttt tcattccaac 12840
   aattaaataa ctaaaaacct ctactaaaaa aaaactaaaa acctctacta aaaaaattaa 12900
   taaattctcc tacctaaacc caaaacaaaa taccacttac actacaaacg acactctaaa 12960
   cttataaaaa aaactactct aaaacttaac taaatatata aaattctaca taaaatatat 13020
   aaaatactaa accatccaaa cctccctaaa aaatactcct aaaatataaa aataaaaaaa 13080
   aacaccctac ttacaaaaaa ataccaaccg ctctctaaaa cctaaaacta aaacacctaa 13140
   taaaaaaata aaaacaacat ctaaaaccct aaaaccaaac aacgacctac caactcccta 13200
   aaatcttatt aactacaacg actacgacta caacatctaa aacaaaacta aaaaacctaa 13260
   attactcaac acttccccaa tcaactcgaa ccctataacc ctctcccact cccgctaccg 13320
   aactcaaaac cccacccaaa ttacaaaccc cgaaatccaa cccaaccaaa attaaacaaa 13380
   aataaacaac actccccttc tctataaaaa accacaactc aaaaaccaac aaaaaccaaa 13440
   cccgctaatt tttttaaaac tcccaatata ttaaaaaata aaaaaattcc aaaacaaaac 13500
   tataaaaatt atataaataa tcttaaatta tttaaaatat ttcattttta acaataaaat 13560
   tcataatcat actttaaaat atttaactat tactaaaaaa tacgtaataa aaaacaatac 13620
   tccttactcc gcctctccca cctccccaac actctaaact ccttttatca tttacctccc 13680
   taattataaa caatatatta tttatactac cctctcccct ccccttctcc ctccctccct 13740
   cccttcccta cctccctccc tttctctttc tttcttcttc ttttttttct tttttcataa 13800
   aacaacgtct cactctatca cccaaactaa aatacaataa tataatcaca actcaataca 13860
   acctctacct cccaaactca aataatcctc ccatctcaac ctcccaaaaa actaaaacta 13920
   caaataatac caccatacct aactaatttt tatatttttt tttataaaaa taaaatctta 13980
   ccatattact caaactaatc tcgaactcct aaactcaaat aatccaccta ctttaacctc 14040
   ccaaaatact aaaattacaa acgtaaaccc cccgacataa acaatatact tctacaataa 14100
   tttcttaatt aattaccttc ccctccttat accccctcaa cctaatactc tatacattta 14160
   tctaacaaat atttatatac caaatactac ctaaaaaata aaaaaccaac aataaacaaa 14220
   acaaacaaaa acctaactaa taaaacttct attcgaaata caaaaaatac tcaacaaaca 14280
   cttaaacgaa caataaataa aaccataaca actattacct caaataaact atcaaaataa 14340
   acttttctaa aaaaataaca tttaaacaac aacctaaata aaaaaaaaaa aaaaacttta 14400
   aaactctcta aaaaatccta aaaaacgttc cgaaaaaaac cattaacgaa aaataaaaca 14460
   aataaattca aaaaaaaatc taaaaccatt aaattcccta ctacctacga taaaaaacca 14520
   ctaaaaaatt ttaaactaaa aaatatcaaa acctaaataa cacttcacaa aataacttta 14580
   actaccataa ataaaataaa caataaaacc taacaaccga aaaacaacaa aaaaacaata 14640
   aaaaactacc aataaaaaat aaaaaaacaa caactacaaa ataataaaaa ctaaacgact 14700
   tctaaatcta ttttaaaaac aaaacctaca aatttttcta ataaattaaa tataaaaaat 14760
   acgaaaaaaa aaaaaaatat aaacaatttt aaaattaatt tttttttcca cctaaaaata 14820
   caataaatcc aaataatatt tactatttat aaaaacaaat acaactatcc aaaaatacaa 14880
   aaaacaaaaa aaaattaaaa aaatcacttc caaaataaaa aaaacaaaaa aaaaaacaaa 14940
   aaacgtcata aaaaaccaaa ctatcccatt ttatttcata aattcaataa tctttttata 15000
   tttttcctaa aaatataaat tactattttc ccccaaaaaa ttttctttta ttccctacat 15060
   caaaaaaatt tcctctaaat tcttaacatc taatttattt tttttttttt tttttttttt 15120
   ttttttaata cgaaatctcg ctctatcccc cgcgctaaaa tacaataacg caatctcgac 15180
   tcactacaaa ctccgcctcc cgaattcaca ccattctcct acctcaacct cccgaataac 15240
   tataactaca aacgcccgcc accacgccca actaattttt tatattttta ataaaaacga 15300
   aatttcaccg tattaaccaa aataatctcg atctcctaac ctcgtaatcc gcccacctcg 15360
   acctcccaaa aaactaaaat tacaaacgta aaccactaca cccaaccgtc ttaacatcta 15420
   atttatatat tttaatttat ttcacataaa aaactttcat caaatatcca atttctccta 15480
   actattaaat catatttaaa aataaaacta aaaaactcaa aaatatataa atactactat 15540
   aaaataatca aataacaaac taacctattc attataaaat caccaaatat ctatattcta 15600
   aaatctttaa aacaacttcc tactcgaata aataaaataa taataatata acctaactac 15660
   caataattca aaacccaaat aaaaaaaaaa ctaaaatttc acgaatcact aactttcaac 15720
   accatacctc atcctctact ataaccaata ctcctaaatc caaaacatct catacccaat 15780
   ttctacaaaa aataaacccc taatatccta taataaaaaa aaaaaacatc tcccaactta 15840
   ataaaactaa aaaaaaaacc aaaaaatcct aaattataac caacctccct acactcaact 15900
   ctaacttcac ctccacctct accatcccca ttacctacaa atcctaccta cacctttcaa 15960
   aaaaactaaa aatcacctaa cttactcctt atataaacgt ttccctctat aactacttta 16020
   atttcaactt cctctactcc cctaaatcaa ttataaaacc ccaaccactt tccatattct 16080
   aaaattatat taaaatttct catctactaa catctcttct ctttattctt ataaatttta 16140
   aatattttac taactttttt tttttttttt ttttaaaaca aaatctaact ttattaccta 16200
   aactaaaata caataaaata atctctactc actacaacct ctaactccct aactcaaaaa 16260
   atcctcccac ctcaacctcc caataactaa aacgataaac atacaccacc acgcctaact 16320
   aattttaata tttttttata acacaaaatt tcaccacatt acccacgcta atttcaaact 16380
   cctaaactca aataatctac ccacctcgac ctcccaaaac ttacaaacat aaaccactac 16440
   gcccaaccta acattctttt tttttttttt taaaacaaaa tctctctcta tcacccaaac 16500
   taaaatacaa taatacgatc tctattcact acaacctcca cctcccaaat tcaaacgatt 16560
   cttatacctc aacctcccta ataactaaaa ttacaaaaat cccccaccac gcccaactaa 16620
   tttttatatt tttaataaaa ataaaatttc accatattaa ccaaactaat ctcaaactcc 16680
   taacctctaa taacctaccc gcctcgacct cccaaaatac taaaattaca aacataaacc 16740
   actataccta acctcctaac attcttatac atttaacatt ttttaacact cttcaatcct 16800
   ttatacaaat tcaaatttct atctaaaatc attttctttc cacctaaaaa actcccttta 16860
   acatttccta cacatctaac aataaattcc cttaaaattt atttatctac caacatattt 16920
   atttcttctt caattttaaa aaatattttc accgaatata aaattctaaa tcaataccaa 16980
   tcatactaaa ttaaaaaaaa aaaaaaaaaa aattctaaat taaaaatttt tatttttatt 17040
   tttttccccc aacactttaa aaatttcatt atattatttc ctaacttaca taatttctaa 17100
   taaaaaaaat ataatatttc ctatatctat tcttccatac ataataaacc tcatttcttt 17160
   aaatactttt aaaattttct ttttaccact aattttcaac atttaattat aataaacctt 17220
   aatataattt acttcgtatt tatcttactt aaaattcatt aaatttctta attcttaact 17280
   ttatcttaat attaatttcc acaacaaatt actacaaact aaataaatta aaacaacaaa 17340
   tatttaaccg aatataataa ctcacgccta taatcccaac attttaaaaa accgaaacga 17400
   acgaatcacg aaatcaaaaa atcgaaacca tactaactaa cacgataaaa ccccgtctct 17460
   actaaaaata caaaaaatta accgaacgta ataataaata cctataatcc caactactta 17520
   aaaaactaaa acaaaaaaat aacgtaaacc gaaaaaacga aacttacaat aaactaaaat 17580
   cacgccacta cactccaacc taaacgacaa aataaaattc catctcaaaa aaaaaaaaaa 17640
   aaaatttatt ctatcacaat tcgaaaaaca taaatacaaa tttaaaatat taacaaaata 17700
   acaatccttc caaaaactct aaaaaaaaat cctaccttat ctctttcaaa ttctaataac 17760
   taaaccacaa ccctcttaac ttataactac aaaactctaa tctctacctc cgtcttcaca 17820
   taaccttccc acctctctaa atatcttaaa aaaacaccta tcattaaatt taaaaccccc 17880
   taaataatcc aaaattattt catcttaaaa tccttaactt aattatatct acaaaaaccc 17940
   ttttttaaaa taaaatctca ttcataaact ataaaaatca aaatataata taaacatatc 18000
   ttaaaaaaaa caccattctt caacccacca taataatttt tatcaaattt aaaaaaattt 18060
   taaacgttct ttctttcttt tattatttat ttacttattt taaaataaaa tctccctcta 18120
   tcgcccaaac taaaatacaa taacacgatc tcgactcatt acaacctccc cttcccgaat 18180
   tcaaacgatt ctcctacctc aacccccaaa taactaaaac tacaaatacc caccaccacg 18240
   cctaactaat ttttatattt ttaataaaaa taaaatttca ccatattaac caaactaatc 18300
   tcaaactcct aatattataa tccacccgcc ttaacctccc aaaatactaa aattacaaac 18360
   gtaaaccacc gcacccgacc tatttctcta aatttatctt atttatccta cttctttatt 18420
   ctatccttta aaattccaat aaatatatat tacaatactt aataatatct tacaaattat 18480
   aaaactccat aaaaacaaaa tcttactcta ttacccaaac taaaatacaa taacgtaatc 18540
   ataactcact acaacctcga actcctaaac tcaaaaaatc ctcctacctc aacttcaaaa 18600
   aaactaaaac tacaactact taccaccata cccaactaat ataaaaatta ttttatttta 18660
   ttttatttta tttataaaaa caaaatctta ctatattacc taaactaatc ttaaactcct 18720
   aaactcaaac caacctcccg ccttaacctc caaaaatact aaaactataa aaacccaacc 18780
   aactttattt ttcaatacta tttttctcct ctatacttca atttaaaaaa ttctattact 18840
   atcttcaaat tcactaattt ttaaattata caatatctaa tctactatta tttccattca 18900
   ataaattttt catttcaatt actatactta ctatctttaa ataatccact aaattctttt 18960
   ttatacctct cattcctctc ctcataaaca tctttttcat atttttcttt aaactcttaa 19020
   atatattaaa ccatctatca tctttatcat ttctaaattt tatttctata aaattatttt 19080
   tctcctaatt ataaattata ttttctttct tttttttttt tttttaaaat aaaatctctc 19140
   tctctattac ccaaactaaa atacaaaaac gccatctcga ctcactacaa actcgatctc 19200
   ctaacctcgt aatccacccg cctcaacctc ccaaaatact aaaattacaa actcacgtaa 19260
   accaccgcac cctacctata aattttattt tcctactttt taacatatct aataattttt 19320
   aactaaattt taaacattat aaatataata ttaaacatct aaatttatta ttattcttta 19380
   aaaaatatta aactttattc aaacaaacta aataaattac ttacaaatca atttaacctt 19440
   tttaaaactt ttttttttaa aacaaaatct tactctatca cccaaactta aatacaataa 19500
   aacaatctca actcattaca acctcaacct cctaaactta aaaaatcctc ctacctcaat 19560
   cactaaataa ctaaaactac aaacacatac taccatacct aactaatttt ttatattttt 19620
   tataaacata aaattttaat atattaccca aactaatctt aaactcctaa actcaaataa 19680
   tctacctacc tcaacctccc aaaatactaa aattaaaaat ataaaccacc gtacctaacc 19740
   tttaatacta atttttcttt ttctttttct tttcttttct tttttttttt taaaataaaa 19800
   tcttactcta tcacccaaac caaaatataa taatacaatc ttaactcact acaacctccg 19860
   cctcctaaat tcaaataatt cttatacctc aacctcctaa ataactaaaa ttacaaacac 19920
   ccatcaccaa acccgactaa ttttttatat ttttaataaa aatataattt cgccatttta 19980
   accaaactaa tcttaaactc ctaacctcaa ataatctacc cgcctccgcc tcccaaaata 20040
   ctaaaattac aaacataaac cactacaccc gacctaaaac taatttttaa tctattaaaa 20100
   taaatctaaa taaacctttt ctctaaaaat aatttaactc tactactaaa acataaccct 20160
   tctaaaatct aattaaatat acatctccac tctaaaaaac taaaacttaa ttatctcctt 20220
   acttttaata aactctaaaa atttaccatt ttattactcc tttttatata atcttactaa 20280
   atttcactat acacatataa aatttaatat tcaaaaaaac ttaaaaaaac ccctatacaa 20340
   atttctaaaa ttttttaaat aatttactct tttccaaaac tatactctac tacaaccttt 20400
   aataccttaa cttccctaaa tcctatctcc atcttctcaa ctcaaaaaaa acaccatact 20460
   ctactcgaac ttcccctcct taaattacaa tctaaaatat tcctccaaac aaaaatccaa 20520
   aaatattaca aaactcacct tattcatttt ccttacttca aaaatcacaa ttctaaactt 20580
   ctaaaaacaa ctatttctct ttacatatat tttctatata aaaaaatata tatatataca 20640
   cacacaaaca cacacacaca cacacataca cacacacata tatatataca cacacacgca 20700
   tatatatata tatatttaaa acaacgtctc actctattac ccaacctaaa atacaaaaat 20760
   ataataatac ctcactacaa cctcaacctc ccaaacttaa acgatcctcc cacctcaacc 20820
   tctaaataac taaaactaca aacacaaacc atcatacccg actaattctt ttatattttt 20880
   tataaaaata aaattttacc atattaccca aactaatctc taactcctaa actcaaacaa 20940
   tccgccaacc tttacctccc aaattactaa aattacaaaa ataaacctct atacccaacc 21000
   ttcatatatt ttacaacaat aattttctca ttatttataa taacaaaata atttcattta 21060
   ttataataaa aataaaaacc tttttactaa cgttttaatc aaattcaaaa aaaaaaaaaa 21120
   aattacatac atataatcaa ttcactattc cttttttttt taaaacgaaa ttttactctt 21180
   atcgcccaaa ctaaaataca ataacataat ctcgactcac tacaacctcc gcctcccgaa 21240
   ttcaaacgat tctcttacct caacctccta attaactaaa attacaaaca taaaccacca 21300
   cgcctaacta attttatatt tttaataaaa ataaaatttc tccatattat caaactaatc 21360
   tcgaactccc aacctcaaat aatctacccg cctcgacctc ccaaaatact aaaattacaa 21420
   acgtaaacta ccgcgcctaa cctatcaatt cactattctt aaccaaaaat cctaaactct 21480
   cgaaatatat ttaaatactt atacttaaaa ccgaacgtaa taactcacgc ctataatccc 21540
   aacactttaa aaaaccaaaa caaacgaatc acttaaaatc aaaaattaaa aaccaaccta 21600
   aacaacatat caaaaccccg tctctactaa aatacaaaaa ttaacctaac ataataatac 21660
   tcgcctataa tcccaactac tcaaaaaact aaaacacaaa aatcgcttaa acctaaaaaa 21720
   cgaaaattac aataaaccaa aataacacca ctacactcta acctaaataa cacaataaaa 21780
   ctctatctca aaaaacaaac aaacaaacaa acaaacaaaa cttacgttta acaaacatac 21840
   tttctaaacc aaatacaaaa acttataatc ccaactactt aaaaaactaa aacaaaaaaa 21900
   ttactcaaac ctaaaaattt aaaaccaacc taaataacat aataaaaccc tatctcaaat 21960
   aaaaaaaaaa aaaaaaaaaa aaaaaaaaaa aatcttttat ctaaacacta cgactcacac 22020
   ctataatccc aacactttaa aaacccgaac caaataaatc acctaaaatc aaaaattcaa 22080
   aactaaccta accaacataa taaaaaccca tctctactaa aaatacaaaa aattaaccaa 22140
   acgtattaac gaatatctat aatcccaact acttaaaaaa ctaaaacaaa aaaatcgctt 22200
   aaacctaaaa aacgaaaatt acaacgaact aatatcacgt cattatactc caacctaacc 22260
   aacaaaataa aactccgtcc ccccaaaaaa aacacaaaaa aaaaccccaa aaaaaacttt 22320
   tatcactttt atcacacaac aaaaatctca tacaatataa tcatattatt cacaaaatta 22380
   taaaatttat acaaatttta atacataata tcccacaaac acaaaacata attataaaat 22440
   tatattaata ccataaactt attatttcat tcctatctat atattactat aaccgtttat 22500
   taaacctcac tcaataataa tatcaataat ctaaattaat aactcttcct aaaaataacc 22560
   taaaaccctt aaaataaatc taatcccaaa taaactcctt aaccaacaac ctctttacca 22620
   ccccacttca atcccaaact tattcttaac aaacaaacca ctaaacccta aaaaaaaaaa 22680
   cccaaccaaa aacccttaat aaacaaaaaa aaaaaaaacc ctccctcttc caaaattact 22740
   ttctatccta tcttctaaac ccctttccat ccacgtatct atctttccgt tactcataat 22800
   atctatcttt ccgttaccca taatatctat ctttccgtta ccttataaaa ttaataaaac 22860
   aaaacaaccc caacaaaaaa taaaaattac tacctactcc aaaactatct aactccgaaa 22920
   aactacctcc gacgtaacta aaactttatc ttccactcct cctcctcctc caaataaaca 22980
   tcgaacaact aaatccccgc gaccgaactc ccctacacct aaccaccacg ctaaaacacc 23040
   ttaaatatac ccgtatactc ctaacgcacg acgcaaacgt aaacaaaaaa aatcgcaacg 23100
   actaaacaaa taaacaaccc tactcacccc aaccccacaa ccgaaacctc cccaacatca 23160
   taaacctata attaactaaa aaatcctacc ctataacctt acctcgccct cccccaatac 23220
   tccaaaaaac tataaatacc cgaaacctaa aactaataca actaatactt cgataccgaa 23280
   actaccaacg aataataaaa cgactaacga acatccccgt actcctaaaa aaactacgca 23340
   aaataaaacc caacctctca acctccacga aaacccttaa acccttctcc aatacaaact 23400
   taacctctct ccccttcgtc ctccaaaccc aaaacttcta cgtaaaaata aaataaaaat 23460
   tcactaacta aaataaacga actacaacaa atcgcttcta aactctccca ctttaaatcc 23520
   tacgcttact cccctaccta aaatatattc tcataattct tccgacgatt ccctctatac 23580
   cttaactcaa ctcaaaacca ccgcctcaac taaacctttc caaattaccc caaatactat 23640
   cttaactcca ttattattct cttcactcta tcataattta cttactatat tcataaccta 23700
   ccgctcctta ttaaaaaata aactctaaaa aaacaaaaat cctatctttt tcactattat 23760
   attcccaata actaaaacac tcaataaata tttcataaat atttattaaa aaaataaata 23820
   aaaaaataaa taaataaatt aacaaataat aaacactaaa aacttaataa taacaaaaac 23880
   atacccgtcc cgacctcaaa ttactcttct atataaatat aatctacact aaccaaaaaa 23940
   aaaattttaa aaaaaaaact actctctctt caattccctc ccctattcct aaccacacga 24000
   atcccaaacc ccatacccta aactaatcca aaccgtataa accaaaccaa tcaaaaccac 24060
   ctccaacctc cgcttccacc aataccccta atatccaaaa tctaccctaa taacacctac 24120
   aaaatataaa aaaacgacca aaacctaaaa ataaacacca cactcctaaa ctttaaccac 24180
   ataacctaac aacgaaaaaa ccgcaacttt atcttcaaaa accaaaatca aacaaacaaa 24240
   aaataaaaca aaataaaaac gataaaaaac aaccccaaac cacccctaat ccccctatac 24300
   tattccgtat ctaacaaaca caaacgccgt aatcataaaa attaaccacg accgccgaat 24360
   aatatacaca aaaactctaa cactaactaa acaaaaccga aaactactac taactactac 24420
   tcaacccact aaaaaacaaa tactaaaccg acttaccgcg cccgtcgtca ccactcaact 24480
   taacaccaaa aatatctcct ttaaaaaata aataaacata accttaatca cccttaaacc 24540
   aacccgataa aatacctacc tcacctaaac cactctactt ccccaaaaac aaaactaaca 24600
   tcctaaacta acgcaataaa aaaacgaaaa taataaataa atataaaact aaaataaaac 24660
   tacaactccc aactaccaac ccaactacac tcttacctac aacaaactat acaaaaaatt 24720
   ctaacctccc ttccccaacc ctaaaaatac aaaaacaata caacgtaatc aaacacttat 24780
   atttaaattc aaaacacttc tctaatattt tatataaata tataaatata tacacaaaac 24840
   acgtacacac gcacacacac acacacattt attcctcaca acaacccttt ctaataaata 24900
   ctattctatt aatattccca ttttacgcat atttattaaa tcctttcctt aacatcccat 24960
   ttactcttca caacaacccc aaaaaataaa aataatcgat ccattttata ataaaaaaat 25020
   aaaaacttaa aataattcta taacctatac aaaatcacac aactattaaa taacaaaatt 25080
   aaaattcaaa tcctaactaa cctaacccta aaaccaaacc tttatcccta aaaataacta 25140
   cctaactaaa cataataact catacctata atcccaacgc tttaaaaaac cgaaataaac 25200
   gaatcaccta aaatcaattc gaaaccaacc taactaacat aataaaaccc cgtctctact 25260
   aacaatacaa aaaattaacc gaatataata acaaacgcct ataatcccaa ctactcgaaa 25320
   aactaaaaca aaaaaattac ttaaacctaa aaaacgaaac ttacaatata caacgatccc 25380
   gccacaacac tccaacctaa acaacaaaac gaaactccat ctaaaaaaaa aaaaaaaaaa 25440
   aaactctacc tattactcta aaacccctat attacccacg acataaaaac aaaaactaac 25500
   aaataccata aattttatat ataacatctc ctctaaaaac ctcccctact tctataatct 25560
   aaaaaaacct ctatactacc cccaccaata cataaaccat actattataa ctccatttta 25620
   ttatctcctt ataaaaatta taaatctcaa aaaaacaaaa atcatattta acttatttac 25680
   tatatcccta atacctacct caatacctaa cacataaaaa ctcctcaaaa aatatttatt 25740
   aaataaataa ataaataaat aaataaataa ataacctcat atattcactt acccaaaaac 25800
   ctaaaacaaa atctctccaa aaccctttac tacaaaaaat attcccaata cccttaaaaa 25860
   taaaacacaa aataaaacca aaactactta taaacccttt cttcatctat ccccaaatat 25920
   ataaaacatc taacgtaaaa ctcatcaccc gcacacgaac aaaacatctt tcaaaacaac 25980
   acaaaaacaa aatcaaaaat aataaaacaa aactaaataa aaaaaaatat tacaaaacta 26040
   cgaaaaccaa ctcccacttc aatacttcca tcaatattcc cactaacatc ctaacaaaaa 26100
   actaaatact ttattcaaac cgataaattt caattcaaat accaaaccct aaaaataaat 26160
   aataactacc taaaatacta aaaaatttct aaaaactcat ctaaattcaa taaaaaacca 26220
   tactacaaat aattactaat attactatta aatttaaaaa aaaaaaaaaa taccatatat 26280
   accatttatt aaccattcta atctaataaa acatatccta aataaaaaat caaaaaacct 26340
   taccacaaat tctatatacc cattaatacc acacctaatt ctctaaatac caaataccgt 26400
   atccgaaata aaaaaacata atttctacct cacaaaaaaa caacataaaa tcatacctat 26460
   aaaaatacct aaaacaaaca cacacacgca catacacacc ccacaatacc taccgaaaca 26520
   aacgttttac tatccaaaat caccgaacta aaaaaaaaca cttttaaaaa ttacttctaa 26580
   cctctaaacc ttccttttaa tattttaaac tatactcaca aaaaccctaa cccctaatcc 26640
   ttaaaacact caatcccctt caaataaaaa tacctaaata tataattaaa taaacaaacc 26700
   ccaccccaaa aaccgacctc ctccctaaat cttaaatact atcacctcta ataaaatacc 26760
   caaatatata taaaattacg atatcctcta aaataccaca tttcttataa actataaaac 26820
   acctttacaa tccttcctaa aaatcgctaa acaacacgaa aacccccaaa ataaaataaa 26880
   tatatacaaa aatacccgta aataaaaaaa tataaatata actcaaaaaa aaacttaaaa 26940
   aacgtcctac ttaacataac ctatataaac ctcctttcca caatacccaa aaccatatca 27000
   acgccacact aacaaaaaaa acaacttaaa aaccttataa caacaaataa attaacctaa 27060
   cattaaaaac taaaaaaaac tcctaaaccc ctccccatat aatacaaacc ctaatcactc 27120
   aaactctaaa ccaaaccaac cccactacca tcactacaaa aaaatacttc aaccccaact 27180
   ttaaactaaa caaccgtaat ataaaccgcc ccataaaact aaccaccaaa acacaaaaat 27240
   aaaaccccta ccgatactaa aaaaataaaa aaccgaaact ccaaaaaata ctaaaaaacc 27300
   ataacttcct acaatatccc ttacccctta aaatctaata aaaacaaatt caaaaccaaa 27360
   ctataactat ataaaaaaca tcccctatcc ctatataaac aaataacccc catcattaac 27420
   ctcataaccg tcaacaatac gctttttacc aaactccgaa acttcatcac cctacgtcta 27480
   cctcctaact tcccaattaa aattaataaa accgcacgac catttctcct aaacccgtaa 27540
   cgaaccgacc gaaatttaaa taaaatcgaa ataaaactaa aactaaaata taaaaaacta 27600
   aataataacg cctaaaaatt tcctcatcct caaaaatccc gatcttccac atcctcaacg 27660
   cccgcatcac cttcgaaaac ctcaacgact acgacgaacc gataccatcg atacgaaaca 27720
   accccaacaa cgaaacgcct tccccaaaca acgactcctc caacgtcaac aactccaact 27780
   ccacgaataa aaccccccgc gaaaacgcct acccctcgac tctccccgaa ataaataaaa 27840
   aaaacgcgcc cgaccgtacc taacccaacc cccgcgcccc caacctccta ccgcgactac 27900
   gaaatctccc caacgttatt cgaaaccccg cgcgactaca acataataaa cgaccaacga 27960
   aaaacgacga cccgaaacga cgacgacgac ctactacaat tcgccatcca acaaaaccta 28020
   cttaaaacga acaataaata taaccaaata cgtctccgcg aacgcgcgaa accacgaaac 28080
   tccgcgccgc cacgactaac gcccacgcct ccccgcaaat caccatctaa aaaacgctaa 28140
   ccaacaacaa accaaacacc caccccatat cctacgaaaa tcgccgacaa aacaaatcaa 28200
   tacccgctaa accgaaaaaa atcctccgaa aaaaatccta tctcccctaa accaaaacac 28260
   aaccgaaaaa cccgcgaaac cctcgacaaa aaccctccta atatcgaaat aacttctacc 28320
   accactccta cctccgaaac gacaaacaaa aatcaaaaat ccaaacttca ccgcgaaatt 28380
   caaactctcc ccaaacatta acgaaaaaaa cttaataaaa tctaaactcc gccacgacca 28440
   aataaaaacc aaaacgccgc gaaactaacc gcctccacta aaaaataata accgcgaacc 28500
   gccctaaaac ctccacgccg tatcccgacc ccgaccgata aataaatacg cccgaaaacc 28560
   cgcgacattc cttccccacc cccaaaaacg ccccgcccac gccgcaacgc caacctacgc 28620
   ccccgacgtc aatacccaac cctcgaccca actcaaaacc aaattccgac gaccacgtat 28680
   tccgaaacta cgacgaacaa ctacgactaa cgataaaact atcgacgcaa aaacaaaaaa 28740
   aaaaacgacg acgcgcgcgc caaaaaaaaa aaaaactaaa acgcatccta aaactctcac 28800
   taaccgaaca ataacgcccc ctaccgaaac cctcgccaac gccacgcgcg ccacgcccaa 28860
   aaccaaaaac caaacaaacc ccgacctacg cgcctacaaa acgacgacta aaaactaaaa 28920
   ccaccgcctc gcgaatacaa caacacaaca aacacgattc gaaaaaaaat tcgacataac 28980
   cgcgaaatac cttcccaaac caaaacccta aaaacaacta acacgaccta atccccctac 29040
   tttactatat tctaattccc caacccgctc ccctaaactc aaatctatcc tatcctaaaa 29100
   cgaaaccaaa cgatcctaaa aaaaaaataa atccttaaaa aaacgctatc cctatccctt 29160
   actccttcta accgatccgc atttctattc actaacccca ctccaaaaca ctacccctaa 29220
   aacctttaca tctctactct tcactcctaa aaaacaacta aactccccgc gtactatatc 29280
   actactttat tccaaacaca aaccaacacg tctaaaaccc aacccctccc ccaccccgac 29340
   atttcaacgt caaatacact taacgaaata cccgactccc ccaaccccca cacccgtcct 29400
   aaatctcaaa ataattccaa cttctcctta aacaaccaaa aattaaaatc ccatccccca 29460
   aaacacattt tttttttttt ttttttataa tcaaaaaata tatccaacgt aaccccctaa 29520
   cctatacaaa ccctaactcc ctcataatac ctcgaaaaat aaaaaacata ttaaactaaa 29580
   ataaacacta aacccaaata aactaaacac aaaaaactcg cccaaaaccc taacgccacc 29640
   cccacccctt cccaccaact actactaacc tctataatta tacatcccac ttacccccac 29700
   acgaaaacta actctaaaac ccttcatcca ataatactaa cccccgactc tcccctaccc 29760
   cacctcaccc acccaaaaaa acacaaaccc cgccaaaaac aaaaacccac cgcacaccct 29820
   tatcccgaac ctatctaaaa ctaaccttcc cgactcaacc aataaaactc aaaaaaaaca 29880
   caaaaaaaaa taaaaaaaaa aaacaaaaaa aaactattcc tcccaccccc ttccctaata 29940
   ccaaaaacac caaactaatt ctaaaacaca aataaaaaaa acttcatacc gaaaactttt 30000
   actacctaac tttacttaaa aaaaacgcct acgatataaa ttaaaatcct tctaaacctc 30060
   aaaataaaaa aaactctaaa ataaaaaact acctaataaa ccctaaaccc aaaaatacat 30120
   cgccaaatct cccaaaaacc taaaaataac atttacaaaa actaaaaatc tcgttcccct 30180
   cactcccacc catacaaaaa acacaaacta aacgcttaat aaaatatttc attcaatacg 30240
   tcttattttt ctaaaaaaaa caacgacaaa aaattaaaaa caatatatat atatttataa 30300
   taaacctaac tcctcatacc aattctctcg aaatttcttc gcgaaaacta ccgcgaaaaa 30360
   acaaccctcg cccctatcga taaaaaaaaa aaaaaaaatt ctctaccaca aaccgctata 30420
   aatataacca ctaaatccga ctctacaaca aacgaattat atatatatct acaaaactaa 30480
   aataacgccc atctaattta ccgctcaaaa taataaaatc caataaacta attaaaaaaa 30540
   acgctatcat tttccccaac ctaaaaatac ccatcaaatc gccacccgtt taaaatcaaa 30600
   ctcaaactcc aataacccca aacatcacgc taatactaaa cactttccct accctaaacg 30660
   aaaaacaaaa aaaaacacca cctcgccgta caacaccatt actaaacccc ccaaattacc 30720
   ccaaacctcc ctaaaccaac tccaaccgaa tcccaaacga aactcgcaat ccaacctccg 30780
   ctaaaacgac gtaaatctcc cctaaactcc aaaaattcta atctcccgcg aaaaacgaaa 30840
   ttccctcccc aatcccgccc ccgaacccaa cccaaaaaaa cgaaatccga aattcgaaac 30900
   taaaaaccga aacgaaaccg aacttatacg ccccgccccg ctccgcctac ctaacgcgcg 30960
   aaactaatcc gtaccgtcca ccaactcgca caacatattc tccaaaaccg taatacgcta 31020
   ctcctaaacc tacacccgct cgcgaaaaac cttaatctct tccaacaacg tctccaaaat 31080
   atactactac taaaacgaaa aacaaaaaaa ccgtatcgta taaaaacgat taaaaaaaat 31140
   ataaattaaa ccaaaataca acaaaaccga tcttaccgac aaaaaaacgt cgctaaccga 31200
   ctaactacga cgaaaaccga aaaacgaacg cacgtccaaa atattacgct tcgtaacccg 31260
   aaactcgcga tacttaaaaa acacataacc gtccctcaac gaaataaaca cgaattcgac 31320
   gtcctaaccg aataaccatt cgtccgcttc taaaaccgac tccgaaccta acgtatccga 31380
   atacaaatcg tcctaaaaaa aatctaacta cgaaaataaa taaacaaaaa aacttacgta 31440
   aaaaaatacg aaacttactc cgctcccttc cgcctcgtaa ccctacccaa cccttctccc 31500
   gacccctcac cttacgaaac acaatcataa taataaattc acactttctt tcgtataact 31560
   tataaaacct ataaaaaaac aaaattcaac accctcgcaa acttccacgt ccttaacttc 31620
   tcctatctaa aaccaaaaaa attaaaaacc aacacttact caacgcctac catacatatt 31680
   acacattaca cacattatta attctacaat aaaacaccct aataatatat tcaccatcat 31740
   cttataaata aaaaaactaa aattaaaaat ataaaactaa taaacgataa atctaacact 31800
   aaaacaaaac tatcttatcc ccccgccccg cttttccaaa acactacatt actaaaaaaa 31860
   ccccgtaact aaaacccgac ataaataaaa aacaaaaacg aataaatatt caaaaaaaac 31920
   caaaactact aaccgaacga tctcacactt actaacatcc aatccccttt taaacataaa 31980
   acccataccc cgctacgact ctttactact aaacgtattc aaataataca cgaaaaacga 32040
   ctcgtcgata atctcaaaat accgaatact actatcgccc tacaaaatca atcgattcaa 32100
   aaacgcgccc gaacaaaacc ctccacatct ccatccaata ccctattctc cctacccgac 32160
   cgtaaacacc ttaccacaca aataaacgat actaaaatcg aaatcgtaaa aaaacaataa 32220
   aaccccgtta cttatatcca tctcctacaa taccactaac tcctcgaaat tattctaccc 32280
   gaacacaaaa aacgtaacca accctacccc accctcccgt actacttcga aacccccaaa    32340
   aaccccttta cgcttcctaa ccttcccaaa ccctataccc caaacctcta ataaccaaaa 32400
   ttacaaaccc ctcccctaaa cacatctctc ctcctcccct acgcacataa ctcctcctcc 32460
   cctacacata tacccgctac gcgccgcata caatcacaac aaaccacccc caaaaactcg 32520
   acatcaccaa ataaaaacgc caacgcaaaa tccttcacgc gacgcaacaa aactaaacaa 32580
   aaaaataaat ataaaaataa caaacgctac aaaaataaca acaaccttca atcctatcca 32640
   aataaacata ccctacgaat aaaaaacgcc taaccactct cccgtaatac caatccccga 32700
   aacacacacc ccaaacctcc aactacgtta ccgaatccca caaacccaac tctcgctaac 32760
   tcatacgaat aaaacccgta ataaaaatat aaccctaacg cgtaaaaacg acccgcataa 32820
   acctcatccc ctcgtaaacc gcaaacctct cctaaaaaaa aaaaaaaaca aaaaaaaaca 32880
   tcacccaact acctacccta aaaaacataa aaaaataaac aaccaactta ctaacttaaa 32940
   tatccgaaat aaaaaaacga atacgaaaaa aaaacaaata aaacctaaac actcaacttc 33000
   ctacgaaacc acgtctaacc cctaacctaa aaaacatccg aacctaaaaa aaacaaaaaa 33060
   cacgcgactc tcaaccgatc ccgaaccctt aaacgcgctc aaaaaccgat ccctaaaaca 33120
   aaaatatcta ctaactcccc gaaatcgcaa cgaaactttt cccgaataaa aactccccgc 33180
   tccgctctaa cctcttaaac gccaaccaca acccgaaaaa acgctcccga caaacccaaa 33240
   aaaaaaacga actaacgcgc ccgacgcgac ctaacctacc gaatcccaaa acgcgaatta 33300
   ccgctcactc atcctactaa aaccgatact aaacaacttc ccgtctacga taaaaacaac 33360
   ccgcaacgaa cgaacgccct cgtaaaaccg aacttactcc tactcgccga attaataaat 33420
   taaaacgccc cgacgccaac cccgaccgct ctcgacgcgc ttactccacg caaacataca 33480
   cgcgacccac gcacccgcac cccaaattaa acttaaaact aacgaaacca aaaccgccgc 33540
   cctcccaaaa catccgacta aaataaacaa cgcgcgaaaa ttaccccatc ccccgcccaa 33600
   cgaatccaca aaaaaaaaaa accaaccctc gaaccaatat caaaccccaa accctaaaaa 33660
   atacgtctaa ataccgacta aaaaaaaaaa aatccaaaaa ctcaaatttc atacaaattc 33720
   taataaaaaa ataaaatttc caacttctcc cctcctcaat cccaaaacga aaaacccttc 33780
   tctaaacttc taaaatacaa aaaaaaaaaa aaaacaaaaa ccgaacaaaa aattacaaac 33840
   taaaaactta cttaaaacaa aaacctataa ttacaaaaac gaaaaaaaaa aactaatctc 33900
   taaaacaaaa atacgacaaa taaaaaccga aaccaaaaac tctaataata ataaatacaa 33960
   aaatactaac aactaaccac taatcaaaaa attctaaaaa ataaccaaac caaacactca 34020
   ccgccaccac ttaacctttt ctaaaatcaa taatacgcaa aatcttatcc ttacaaataa 34080
   taactaacaa actaccgtta ctattccaac acacactata aataacgtct aaatacatat 34140
   catccaaact caacaacacc tccccgatac ccacattcca aataataatc acattatcac 34200
   cacctaccca aaataaccaa acataatcac ctaaatccca cctttaccac cacccaccca 34260
   tctataaaaa acccctcctt aacccccaaa cctacactaa acaaaacatt cctaacaata 34320
   aaataccaaa aaaaaatacc cacacgctta aaataaccct caaatataat aataaattcc 34380
   gtaatattac gcataaaaat ataatctaaa atctaccaca cctaaataaa aaaaaaaaat 34440
   atataataaa taaaataaaa aaccacccta tccctccact ccttccctac cttttttttt 34500
   aaccctacta actctcttaa ataataaaaa tcaaatacaa aacaaaatat aataatacat 34560
   acctaaaaca aaaacaacta aaaaccaaaa aaaaacacta ccaactctcc aaaaacctcc 34620
   cattcactcc aacaaaaaaa cttcaaaaca ctaccctact actctccaaa caccaaatta 34680
   accataacta taattaatcc taaacaatac atcctaaaaa aaaataaaca tataactaaa 34740
   aataatccaa aaactaaaca aaacaaaacc ctaaaaccca acacctccct tattaccacc 34800
   cttaacacct tccccaacac aaataaaaca accttcccta ctcccctact caactaccca 34860
   acaataacca cctaactcct accataataa taatatcgtc taaaacacta acgataacgt 34920
   tatcattata taaacaccaa tcaatatcca acacaaaaac aatataccca ataaccaata 34980
   aataattctt atc 34993
<210> 5
   <211> 34993
   <212> DNA
   <213> Artificial Sequence
<220>
   <223> G to A (bis2 rc) rc
<400> 5
   gataagaatt atttattggt tattgggtat attgtttttg tgttggatat tgattggtgt 60
   ttatataatg ataacgttat cgttagtgtt ttagacgata ttattattat ggtaggagtt 120
   aggtggttat tgttgggtag ttgagtaggg gagtagggaa ggttgtttta tttgtgttgg 180
   ggaaggtgtt aagggtggta ataagggagg tgttgggttt tagggttttg ttttgtttag 240
   tttttggatt atttttagtt atatgtttat ttttttttag gatgtattgt ttaggattaa 300
   ttatagttat ggttaatttg gtgtttggag agtagtaggg tagtgttttg aagttttttt 360
   gttggagtga atgggaggtt tttggagagt tggtagtgtt tttttttggt ttttagttgt 420
   ttttgtttta ggtatgtatt attatatttt gttttgtatt tgatttttat tatttaagag 480
   agttagtagg gttaaaaaaa aaggtaggga aggagtggag ggatagggtg gttttttatt 540
   ttatttatta tatatttttt ttttttattt aggtgtggta gattttagat tatattttta 600
   tgcgtaatat tacggaattt attattatat ttgagggtta ttttaagcgt gtgggtattt 660
   ttttttggta ttttattgtt aggaatgttt tgtttagtgt aggtttgggg gttaaggagg 720
   ggttttttat agatgggtgg gtggtggtaa aggtgggatt taggtgatta tgtttggtta 780
   ttttgggtag gtggtgataa tgtgattatt atttggaatg tgggtatcgg ggaggtgttg 840
   ttgagtttgg atgatatgta tttagacgtt atttatagtg tgtgttggaa tagtaacggt 900
   agtttgttag ttattatttg taaggataag attttgcgta ttattgattt tagaaaaggt 960
   taagtggtgg cggtgagtgt ttggtttggt tattttttag aattttttga ttagtggtta 1020
   gttgttagta tttttgtatt tattattatt agagtttttg gtttcggttt ttatttgtcg 1080
   tatttttgtt ttagagatta gttttttttt ttcgtttttg taattatagg tttttgtttt 1140
   aagtaagttt ttagtttgta attttttgtt cggtttttgt tttttttttt tttttgtatt 1200
   ttagaagttt agagaagggt ttttcgtttt gggattgagg aggggagaag ttggaaattt 1260
   tattttttta ttagaatttg tatgaaattt gagtttttgg attttttttt ttttagtcgg 1320
   tatttagacg tattttttag ggtttggggt ttgatattgg ttcgagggtt ggtttttttt 1380
   ttttgtggat tcgttgggcg ggggatgggg taattttcgc gcgttgttta ttttagtcgg 1440
   atgttttggg agggcggcgg ttttggtttc gttagtttta agtttaattt ggggtgcggg 1500
   tgcgtgggtc gcgtgtatgt ttgcgtggag taagcgcgtc gagagcggtc ggggttggcg 1560
   tcggggcgtt ttaatttatt aattcggcga gtaggagtaa gttcggtttt acgagggcgt 1620
   tcgttcgttg cgggttgttt ttatcgtaga cgggaagttg tttagtatcg gttttagtag 1680
   gatgagtgag cggtaattcg cgttttggga ttcggtaggt taggtcgcgt cgggcgcgtt 1740
   agttcgtttt ttttttgggt ttgtcgggag cgttttttcg ggttgtggtt ggcgtttaag 1800
   aggttagagc ggagcgggga gtttttattc gggaaaagtt tcgttgcgat ttcggggagt 1860
   tagtagatat ttttgtttta gggatcggtt tttgagcgcg tttaagggtt cgggatcggt 1920
   tgagagtcgc gtgttttttg ttttttttag gttcggatgt tttttaggtt aggggttaga 1980
   cgtggtttcg taggaagttg agtgtttagg ttttatttgt ttttttttcg tattcgtttt 2040
   tttatttcgg atatttaagt tagtaagttg gttgtttatt tttttatgtt ttttagggta 2100
   ggtagttggg tgatgttttt ttttgttttt tttttttttt aggagaggtt tgcggtttac 2160
   gaggggatga ggtttatgcg ggtcgttttt acgcgttagg gttatatttt tattacgggt 2220
   tttattcgta tgagttagcg agagttgggt ttgtgggatt cggtaacgta gttggaggtt 2280
   tggggtgtgt gtttcgggga ttggtattac gggagagtgg ttaggcgttt tttattcgta 2340
   gggtatgttt atttggatag gattgaaggt tgttgttatt tttgtagcgt ttgttatttt 2400
   tatatttatt tttttgttta gttttgttgc gtcgcgtgaa ggattttgcg ttggcgtttt 2460
   tatttggtga tgtcgagttt ttgggggtgg tttgttgtga ttgtatgcgg cgcgtagcgg 2520
   gtatatgtgt aggggaggag gagttatgtg cgtaggggag gaggagagat gtgtttaggg 2580
   gaggggtttg taattttggt tattagaggt ttggggtata gggtttggga aggttaggaa 2640
   gcgtaaaggg gtttttgggg gtttcgaagt agtacgggag ggtggggtag ggttggttac 2700
   gttttttgtg ttcgggtaga ataatttcga ggagttagtg gtattgtagg agatggatat 2760
   aagtaacggg gttttattgt ttttttacga tttcgatttt agtatcgttt atttgtgtgg 2820
   taaggtgttt acggtcgggt agggagaata gggtattgga tggagatgtg gagggttttg 2880
   ttcgggcgcg tttttgaatc gattgatttt gtagggcgat agtagtattc ggtattttga 2940
   gattatcgac gagtcgtttt tcgtgtatta tttgaatacg tttagtagta aagagtcgta 3000
   gcggggtatg ggttttatgt ttaaaagggg attggatgtt agtaagtgtg agatcgttcg 3060
   gttagtagtt ttggtttttt ttgaatattt attcgttttt gttttttatt tatgtcgggt 3120
   tttagttacg gggttttttt agtaatgtag tgttttggaa aagcggggcg gggggataag 3180
   atagttttgt tttagtgtta gatttatcgt ttattagttt tatattttta attttagttt 3240
   ttttatttat aagatgatgg tgaatatatt attagggtgt tttattgtag aattaataat 3300
   gtgtgtaatg tgtaatatgt atggtaggcg ttgagtaagt gttggttttt aatttttttg 3360
   gttttagata ggagaagtta aggacgtgga agtttgcgag ggtgttgaat tttgtttttt 3420
   tataggtttt ataagttata cgaaagaaag tgtgaattta ttattatgat tgtgtttcgt 3480
   aaggtgaggg gtcgggagaa gggttgggta gggttacgag gcggaaggga gcggagtaag 3540
   tttcgtattt ttttacgtaa gtttttttgt ttatttattt tcgtagttag atttttttta 3600
   ggacgatttg tattcggata cgttaggttc ggagtcggtt ttagaagcgg acgaatggtt 3660
   attcggttag gacgtcgaat tcgtgtttat ttcgttgagg gacggttatg tgttttttaa 3720
   gtatcgcgag tttcgggtta cgaagcgtaa tattttggac gtgcgttcgt ttttcggttt 3780
   tcgtcgtagt tagtcggtta gcgacgtttt tttgtcggta agatcggttt tgttgtattt 3840
   tggtttaatt tatatttttt ttaatcgttt ttatacgata cggttttttt gtttttcgtt 3900
   ttagtagtag tatattttgg agacgttgtt ggaagagatt aaggtttttc gcgagcgggt 3960
   gtaggtttag gagtagcgta ttacggtttt ggagaatatg ttgtgcgagt tggtggacgg 4020
   tacggattag tttcgcgcgt taggtaggcg gagcggggcg gggcgtataa gttcggtttc 4080
   gtttcggttt ttagtttcga atttcggatt tcgttttttt gggttgggtt cgggggcggg 4140
   attggggagg gaatttcgtt tttcgcggga gattagaatt tttggagttt aggggagatt 4200
   tacgtcgttt tagcggaggt tggattgcga gtttcgtttg ggattcggtt ggagttggtt 4260
   tagggaggtt tggggtaatt tggggggttt agtaatggtg ttgtacggcg aggtggtgtt 4320
   tttttttgtt tttcgtttag ggtagggaaa gtgtttagta ttagcgtgat gtttggggtt 4380
   attggagttt gagtttgatt ttaaacgggt ggcgatttga tgggtatttt taggttgggg 4440
   aaaatgatag cgtttttttt aattagttta ttggatttta ttattttgag cggtaaatta 4500
   gatgggcgtt attttagttt tgtagatata tatataattc gtttgttgta gagtcggatt 4560
   tagtggttat atttatagcg gtttgtggta gagaattttt tttttttttt ttatcgatag 4620
   gggcgagggt tgttttttcg cggtagtttt cgcgaagaaa tttcgagaga attggtatga 4680
   ggagttaggt ttattataaa tatatatata ttgtttttaa ttttttgtcg ttgttttttt 4740
   tagaaaaata agacgtattg aatgaaatat tttattaagc gtttagtttg tgttttttgt 4800
   atgggtggga gtgaggggaa cgagattttt agtttttgta aatgttattt ttaggttttt 4860
   gggagatttg gcgatgtatt tttgggttta gggtttatta ggtagttttt tattttagag 4920
   ttttttttat tttgaggttt agaaggattt taatttatat cgtaggcgtt ttttttaagt 4980
   aaagttaggt agtaaaagtt ttcggtatga agtttttttt atttgtgttt tagaattagt 5040
   ttggtgtttt tggtattagg gaagggggtg ggaggaatag tttttttttg tttttttttt 5100
   ttattttttt ttgtgttttt tttgagtttt attggttgag tcgggaaggt tagttttaga 5160
   taggttcggg ataagggtgt gcggtgggtt tttgtttttg gcggggtttg tgtttttttg 5220
   ggtgggtgag gtggggtagg ggagagtcgg gggttagtat tattggatga agggttttag 5280
   agttagtttt cgtgtggggg taagtgggat gtataattat agaggttagt agtagttggt 5340
   gggaaggggt gggggtggcg ttagggtttt gggcgagttt tttgtgttta gtttatttgg 5400
   gtttagtgtt tattttagtt taatatgttt tttatttttc gaggtattat gagggagtta 5460
   gggtttgtat aggttagggg gttacgttgg atatattttt tgattataaa aaaaaaaaaa     5520
   aaaaaatgtg ttttggggga tgggatttta atttttggtt gtttaaggag aagttggaat     5580
   tattttgaga tttaggacgg gtgtgggggt tgggggagtc gggtatttcg ttaagtgtat 5640
   ttgacgttga aatgtcgggg tgggggaggg gttgggtttt agacgtgttg gtttgtgttt 5700
   ggaataaagt agtgatatag tacgcgggga gtttagttgt tttttaggag tgaagagtag 5760
   agatgtaaag gttttagggg tagtgttttg gagtggggtt agtgaataga aatgcggatc 5820
   ggttagaagg agtaagggat agggatagcg tttttttaag gatttatttt ttttttagga 5880
   tcgtttggtt tcgttttagg ataggataga tttgagttta ggggagcggg ttggggaatt 5940
   agaatatagt aaagtagggg gattaggtcg tgttagttgt ttttagggtt ttggtttggg 6000
   aaggtatttc gcggttatgt cgaatttttt ttcgaatcgt gtttgttgtg ttgttgtatt 6060
   cgcgaggcgg tggttttagt ttttagtcgt cgttttgtag gcgcgtaggt cggggtttgt 6120
   ttggtttttg gttttgggcg tggcgcgcgt ggcgttggcg agggtttcgg tagggggcgt 6180
   tattgttcgg ttagtgagag ttttaggatg cgttttagtt tttttttttt ttggcgcgcg 6240
   cgtcgtcgtt tttttttttg tttttgcgtc gatagtttta tcgttagtcg tagttgttcg 6300
   tcgtagtttc ggaatacgtg gtcgtcggaa tttggttttg agttgggtcg agggttgggt 6360
   attgacgtcg ggggcgtagg ttggcgttgc ggcgtgggcg gggcgttttt gggggtgggg 6420
   aaggaatgtc gcgggttttc gggcgtattt atttatcggt cggggtcggg atacggcgtg 6480
   gaggttttag ggcggttcgc ggttattatt ttttagtgga ggcggttagt ttcgcggcgt 6540
   tttggttttt atttggtcgt ggcggagttt agattttatt aagttttttt cgttaatgtt 6600
   tggggagagt ttgaatttcg cggtgaagtt tggatttttg atttttgttt gtcgtttcgg 6660
   aggtaggagt ggtggtagaa gttatttcga tattaggagg gtttttgtcg agggtttcgc 6720
   gggtttttcg gttgtgtttt ggtttagggg agataggatt tttttcggag gatttttttc 6780
   ggtttagcgg gtattgattt gttttgtcgg cgattttcgt aggatatggg gtgggtgttt 6840
   ggtttgttgt tggttagcgt tttttagatg gtgatttgcg gggaggcgtg ggcgttagtc 6900
   gtggcggcgc ggagtttcgt ggtttcgcgc gttcgcggag acgtatttgg ttatatttat 6960
   tgttcgtttt aagtaggttt tgttggatgg cgaattgtag taggtcgtcg tcgtcgtttc 7020
   gggtcgtcgt ttttcgttgg tcgtttatta tgttgtagtc gcgcggggtt tcgaataacg 7080
   ttggggagat ttcgtagtcg cggtaggagg ttgggggcgc gggggttggg ttaggtacgg 7140
   tcgggcgcgt tttttttatt tatttcgggg agagtcgagg ggtaggcgtt ttcgcggggg 7200
   gttttattcg tggagttgga gttgttgacg ttggaggagt cgttgtttgg ggaaggcgtt 7260
   tcgttgttgg ggttgtttcg tatcgatggt atcggttcgt cgtagtcgtt gaggttttcg 7320
   aaggtgatgc gggcgttgag gatgtggaag atcgggattt ttgaggatga ggaaattttt 7380
   aggcgttatt atttagtttt ttatatttta gttttagttt tatttcgatt ttatttaaat 7440
   ttcggtcggt tcgttacggg tttaggagaa atggtcgtgc ggttttatta attttaattg 7500
   ggaagttagg aggtagacgt agggtgatga agtttcggag tttggtaaaa agcgtattgt 7560
   tgacggttat gaggttaatg atgggggtta tttgtttata tagggatagg ggatgttttt 7620
   tatatagtta tagtttggtt ttgaatttgt ttttattaga ttttaagggg taagggatat 7680
   tgtaggaagt tatggttttt tagtattttt tggagtttcg gttttttatt tttttagtat 7740
   cggtaggggt tttatttttg tgttttggtg gttagtttta tggggcggtt tatattacgg 7800
   ttgtttagtt taaagttggg gttgaagtat ttttttgtag tgatggtagt ggggttggtt 7860
   tggtttagag tttgagtgat tagggtttgt attatatggg gaggggttta ggagtttttt 7920
   ttagttttta atgttaggtt aatttatttg ttgttataag gtttttaagt tgtttttttt 7980
   gttagtgtgg cgttgatatg gttttgggta ttgtggaaag gaggtttata taggttatgt 8040
   taagtaggac gttttttaag tttttttttg agttatattt atattttttt atttacgggt 8100
   atttttgtat atatttattt tattttgggg gttttcgtgt tgtttagcga tttttaggaa 8160
   ggattgtaaa ggtgttttat agtttataag aaatgtggta ttttagagga tatcgtaatt 8220
   ttatatatat ttgggtattt tattagaggt gatagtattt aagatttagg gaggaggtcg 8280
   gtttttgggg tggggtttgt ttatttaatt atatatttag gtatttttat ttgaagggga 8340
   ttgagtgttt taaggattag gggttagggt ttttgtgagt atagtttaaa atattaaaag 8400
   gaaggtttag aggttagaag taatttttaa aagtgttttt ttttagttcg gtgattttgg 8460
   atagtaaaac gtttgtttcg gtaggtattg tggggtgtgt atgtgcgtgt gtgtgtttgt 8520
   tttaggtatt tttataggta tgattttatg ttgttttttt gtgaggtaga aattatgttt 8580
   ttttatttcg gatacggtat ttggtattta gagaattagg tgtggtatta atgggtatat 8640
   agaatttgtg gtaaggtttt ttgatttttt atttaggata tgttttatta gattagaatg 8700
   gttaataaat ggtatatatg gtattttttt ttttttttaa atttaatagt aatattagta 8760
   attatttgta gtatggtttt ttattgaatt tagatgagtt tttagaaatt ttttagtatt 8820
   ttaggtagtt attatttatt tttagggttt ggtatttgaa ttgaaattta tcggtttgaa 8880
   taaagtattt agttttttgt taggatgtta gtgggaatat tgatggaagt attgaagtgg 8940
   gagttggttt tcgtagtttt gtaatatttt tttttattta gttttgtttt attatttttg 9000
   attttgtttt tgtgttgttt tgaaagatgt tttgttcgtg tgcgggtgat gagttttacg 9060
   ttagatgttt tatatatttg gggatagatg aagaaagggt ttataagtag ttttggtttt 9120
   attttgtgtt ttatttttaa gggtattggg aatatttttt gtagtaaagg gttttggaga 9180
   gattttgttt taggtttttg ggtaagtgaa tatatgaggt tatttattta tttatttatt 9240
   tatttattta tttaataaat attttttgag gagtttttat gtgttaggta ttgaggtagg 9300
   tattagggat atagtaaata agttaaatat gatttttgtt tttttgagat ttataatttt 9360
   tataaggaga taataaaatg gagttataat agtatggttt atgtattggt gggggtagta 9420
   tagaggtttt tttagattat agaagtaggg gaggttttta gaggagatgt tatatataaa 9480
   atttatggta tttgttagtt tttgttttta tgtcgtgggt aatatagggg ttttagagta 9540
   ataggtagag tttttttttt tttttttttt tagatggagt ttcgttttgt tgtttaggtt 9600
   ggagtgttgt ggcgggatcg ttgtatattg taagtttcgt tttttaggtt taagtaattt 9660
   ttttgtttta gtttttcgag tagttgggat tataggcgtt tgttattata ttcggttaat 9720
   tttttgtatt gttagtagag acggggtttt attatgttag ttaggttggt ttcgaattga 9780
   ttttaggtga ttcgtttatt tcggtttttt aaagcgttgg gattataggt atgagttatt 9840
   atgtttagtt aggtagttat ttttagggat aaaggtttgg ttttagggtt aggttagtta 9900
   ggatttgaat tttaattttg ttatttaata gttgtgtgat tttgtatagg ttatagaatt 9960
   attttaagtt tttatttttt tattataaaa tggatcgatt atttttattt tttggggttg 10020
   ttgtgaagag taaatgggat gttaaggaaa ggatttaata aatatgcgta aaatgggaat 10080
   attaatagaa tagtatttat tagaaagggt tgttgtgagg aataaatgtg tgtgtgtgtg 10140
   tgcgtgtgta cgtgttttgt gtatatattt atatatttat ataaaatatt agagaagtgt 10200
   tttgaattta aatataagtg tttgattacg ttgtattgtt tttgtatttt tagggttggg 10260
   gaagggaggt tagaattttt tgtatagttt gttgtaggta agagtgtagt tgggttggta 10320
   gttgggagtt gtagttttat tttagtttta tatttattta ttattttcgt ttttttattg 10380
   cgttagttta ggatgttagt tttgtttttg gggaagtaga gtggtttagg tgaggtaggt 10440
   attttatcgg gttggtttaa gggtgattaa ggttatgttt atttattttt taaaggagat 10500
   atttttggtg ttaagttgag tggtgacgac gggcgcggta agtcggttta gtatttgttt 10560
   tttagtgggt tgagtagtag ttagtagtag ttttcggttt tgtttagtta gtgttagagt 10620
   ttttgtgtat attattcggc ggtcgtggtt aatttttatg attacggcgt ttgtgtttgt 10680
   tagatacgga atagtatagg gggattaggg gtggtttggg gttgtttttt atcgttttta 10740
   ttttgtttta ttttttgttt gtttgatttt ggtttttgaa gataaagttg cggttttttc 10800
   gttgttaggt tatgtggtta aagtttagga gtgtggtgtt tatttttagg ttttggtcgt 10860
   ttttttatat tttgtaggtg ttattagggt agattttgga tattaggggt attggtggaa 10920
   gcggaggttg gaggtggttt tgattggttt ggtttatacg gtttggatta gtttagggta 10980
   tggggtttgg gattcgtgtg gttaggaata ggggagggaa ttgaagagag agtagttttt 11040
   tttttaaaat tttttttttg gttagtgtag attatattta tatagaagag taatttgagg 11100
   tcgggacggg tatgtttttg ttattattaa gtttttagtg tttattattt gttaatttat 11160
   ttatttattt ttttatttat ttttttaata aatatttatg aaatatttat tgagtgtttt 11220
   agttattggg aatataatag tgaaaaagat aggatttttg tttttttaga gtttattttt 11280
   taataaggag cggtaggtta tgaatatagt aagtaaatta tgatagagtg aagagaataa 11340
   taatggagtt aagatagtat ttggggtaat ttggaaaggt ttagttgagg cggtggtttt 11400
   gagttgagtt aaggtataga gggaatcgtc ggaagaatta tgagaatata ttttaggtag 11460
   gggagtaagc gtaggattta aagtgggaga gtttagaagc gatttgttgt agttcgttta 11520
   ttttagttag tgaattttta ttttattttt acgtagaagt tttgggtttg gaggacgaag 11580
   gggagagagg ttaagtttgt attggagaag ggtttaaggg ttttcgtgga ggttgagagg 11640
   ttgggtttta ttttgcgtag tttttttagg agtacgggga tgttcgttag tcgttttatt 11700
   attcgttggt agtttcggta tcgaagtatt agttgtatta gttttaggtt tcgggtattt 11760
   atagtttttt ggagtattgg gggagggcga ggtaaggtta tagggtagga ttttttagtt 11820
   aattataggt ttatgatgtt ggggaggttt cggttgtggg gttggggtga gtagggttgt 11880
   ttatttgttt agtcgttgcg attttttttg tttacgtttg cgtcgtgcgt taggagtata 11940
   cgggtatatt taaggtgttt tagcgtggtg gttaggtgta ggggagttcg gtcgcgggga 12000
   tttagttgtt cgatgtttat ttggaggagg aggaggagtg gaagataaag ttttagttac 12060
   gtcggaggta gtttttcgga gttagatagt tttggagtag gtagtaattt ttattttttg 12120
   ttggggttgt tttgttttat taattttata aggtaacgga aagatagata ttatgggtaa 12180
   cggaaagata gatattatga gtaacggaaa gatagatacg tggatggaaa ggggtttaga 12240
   agataggata gaaagtaatt ttggaagagg gagggttttt tttttttttg tttattaagg 12300
   gtttttggtt gggttttttt ttttagggtt tagtggtttg tttgttaaga ataagtttgg 12360
   gattgaagtg gggtggtaaa gaggttgttg gttaaggagt ttatttggga ttagatttat 12420
   tttaagggtt ttaggttatt tttaggaaga gttattaatt tagattattg atattattat 12480
   tgagtgaggt ttaataaacg gttatagtaa tatatagata ggaatgaaat aataagttta 12540
   tggtattaat ataattttat aattatgttt tgtgtttgtg ggatattatg tattaaaatt 12600
   tgtataaatt ttataatttt gtgaataata tgattatatt gtatgagatt tttgttgtgt 12660
   gataaaagtg ataaaagttt tttttggggt ttttttttgt gttttttttg gggggacgga 12720
   gttttatttt gttggttagg ttggagtata atgacgtgat attagttcgt tgtaattttc 12780
   gttttttagg tttaagcgat ttttttgttt tagtttttta agtagttggg attatagata 12840
   ttcgttaata cgtttggtta attttttgta tttttagtag agatgggttt ttattatgtt 12900
   ggttaggtta gttttgaatt tttgatttta ggtgatttat ttggttcggg tttttaaagt 12960
   gttgggatta taggtgtgag tcgtagtgtt tagataaaag attttttttt tttttttttt 13020
   tttttttttt tttatttgag atagggtttt attatgttat ttaggttggt tttaaatttt 13080
   taggtttgag taattttttt gttttagttt tttaagtagt tgggattata agtttttgta 13140
   tttggtttag aaagtatgtt tgttaaacgt aagttttgtt tgtttgtttg tttgtttgtt 13200
   ttttgagata gagttttatt gtgttattta ggttagagtg tagtggtgtt attttggttt 13260
   attgtaattt tcgtttttta ggtttaagcg atttttgtgt tttagttttt tgagtagttg 13320
   ggattatagg cgagtattat tatgttaggt taatttttgt attttagtag agacggggtt 13380
   ttgatatgtt gtttaggttg gtttttaatt tttgatttta agtgattcgt ttgttttggt 13440
   tttttaaagt gttgggatta taggcgtgag ttattacgtt cggttttaag tataagtatt 13500
   taaatatatt tcgagagttt aggatttttg gttaagaata gtgaattgat aggttaggcg 13560
   cggtagttta cgtttgtaat tttagtattt tgggaggtcg aggcgggtag attatttgag 13620
   gttgggagtt cgagattagt ttgataatat ggagaaattt tatttttatt aaaaatataa 13680
   aattagttag gcgtggtggt ttatgtttgt aattttagtt aattaggagg ttgaggtaag 13740
   agaatcgttt gaattcggga ggcggaggtt gtagtgagtc gagattatgt tattgtattt 13800
   tagtttgggc gataagagta aaatttcgtt ttaaaaaaaa aaggaatagt gaattgatta 13860
   tatgtatgta attttttttt tttttttgaa tttgattaaa acgttagtaa aaaggttttt 13920
   atttttatta taataaatga aattattttg ttattataaa taatgagaaa attattgttg 13980
   taaaatatat gaaggttggg tatagaggtt tatttttgta attttagtaa tttgggaggt 14040
   aaaggttggc ggattgtttg agtttaggag ttagagatta gtttgggtaa tatggtaaaa 14100
   ttttattttt ataaaaaata taaaagaatt agtcgggtat gatggtttgt gtttgtagtt 14160
   ttagttattt agaggttgag gtgggaggat cgtttaagtt tgggaggttg aggttgtagt 14220
   gaggtattat tatatttttg tattttaggt tgggtaatag agtgagacgt tgttttaaat 14280
   atatatatat atatgcgtgt gtgtgtatat atatatgtgt gtgtgtatgt gtgtgtgtgt 14340
   gtgtgtttgt gtgtgtatat atatatattt ttttatatag aaaatatatg taaagagaaa 14400
   tagttgtttt tagaagttta gaattgtgat ttttgaagta aggaaaatga ataaggtgag 14460
   ttttgtaata tttttggatt tttgtttgga ggaatatttt agattgtaat ttaaggaggg 14520
   gaagttcgag tagagtatgg tgtttttttt gagttgagaa gatggagata ggatttaggg 14580
   aagttaaggt attaaaggtt gtagtagagt atagttttgg aaaagagtaa attatttaaa 14640
   aaattttaga aatttgtata ggggtttttt taagtttttt tgaatattaa attttatatg 14700
   tgtatagtga aatttagtaa gattatataa aaaggagtaa taaaatggta aatttttaga 14760
   gtttattaaa agtaaggaga taattaagtt ttagtttttt agagtggaga tgtatattta 14820
   attagatttt agaagggtta tgttttagta gtagagttaa attattttta gagaaaaggt 14880
   ttatttagat ttattttaat agattaaaaa ttagttttag gtcgggtgta gtggtttatg 14940
   tttgtaattt tagtattttg ggaggcggag gcgggtagat tatttgaggt taggagttta 15000
   agattagttt ggttaaaatg gcgaaattat atttttatta aaaatataaa aaattagtcg 15060
   ggtttggtga tgggtgtttg taattttagt tatttaggag gttgaggtat aagaattatt 15120
   tgaatttagg aggcggaggt tgtagtgagt taagattgta ttattatatt ttggtttggg 15180
   tgatagagta agattttatt ttaaaaaaaa aaaagaaaag aaaagaaaaa gaaaaagaaa 15240
   aattagtatt aaaggttagg tacggtggtt tatattttta attttagtat tttgggaggt 15300
   tgaggtaggt agattatttg agtttaggag tttaagatta gtttgggtaa tatattaaaa 15360
   ttttatgttt ataaaaaata taaaaaatta gttaggtatg gtagtatgtg tttgtagttt 15420
   tagttattta gtgattgagg taggaggatt ttttaagttt aggaggttga ggttgtaatg 15480
   agttgagatt gttttattgt atttaagttt gggtgataga gtaagatttt gttttaaaaa 15540
   aaaaagtttt aaaaaggtta aattgatttg taagtaattt atttagtttg tttgaataaa 15600
   gtttaatatt ttttaaagaa taataataaa tttagatgtt taatattata tttataatgt 15660
   ttaaaattta gttaaaaatt attagatatg ttaaaaagta ggaaaataaa atttataggt 15720
   agggtgcggt ggtttacgtg agtttgtaat tttagtattt tgggaggttg aggcgggtgg 15780
   attacgaggt taggagatcg agtttgtagt gagtcgagat ggcgtttttg tattttagtt 15840
   tgggtaatag agagagagat tttattttaa aaaaaaaaaa aaaagaaaga aaatataatt 15900
   tataattagg agaaaaataa ttttatagaa ataaaattta gaaatgataa agatgataga 15960
   tggtttaata tatttaagag tttaaagaaa aatatgaaaa agatgtttat gaggagagga 16020
   atgagaggta taaaaaagaa tttagtggat tatttaaaga tagtaagtat agtaattgaa 16080
   atgaaaaatt tattgaatgg aaataatagt agattagata ttgtataatt taaaaattag 16140
   tgaatttgaa gatagtaata gaatttttta aattgaagta tagaggagaa aaatagtatt 16200
   gaaaaataaa gttggttggg tttttatagt tttagtattt ttggaggtta aggcgggagg 16260
   ttggtttgag tttaggagtt taagattagt ttaggtaata tagtaagatt ttgtttttat 16320
   aaataaaata aaataaaata aaataatttt tatattagtt gggtatggtg gtaagtagtt 16380
   gtagttttag tttttttgaa gttgaggtag gaggattttt tgagtttagg agttcgaggt 16440
   tgtagtgagt tatgattacg ttattgtatt ttagtttggg taatagagta agattttgtt 16500
   tttatggagt tttataattt gtaagatatt attaagtatt gtaatatata tttattggaa 16560
   ttttaaagga tagaataaag aagtaggata aataagataa atttagagaa ataggtcggg 16620
   tgcggtggtt tacgtttgta attttagtat tttgggaggt taaggcgggt ggattataat 16680
   attaggagtt tgagattagt ttggttaata tggtgaaatt ttatttttat taaaaatata 16740
   aaaattagtt aggcgtggtg gtgggtattt gtagttttag ttatttgggg gttgaggtag 16800
   gagaatcgtt tgaattcggg aaggggaggt tgtaatgagt cgagatcgtg ttattgtatt 16860
   ttagtttggg cgatagaggg agattttatt ttaaaataag taaataaata ataaaagaaa 16920
   gaaagaacgt ttaaaatttt tttaaatttg ataaaaatta ttatggtggg ttgaagaatg 16980
   gtgttttttt taagatatgt ttatattata ttttgatttt tatagtttat gaatgagatt 17040
   ttattttaaa aaagggtttt tgtagatata attaagttaa ggattttaag atgaaataat 17100
   tttggattat ttagggggtt ttaaatttaa tgataggtgt ttttttaaga tatttagaga 17160
   ggtgggaagg ttatgtgaag acggaggtag agattagagt tttgtagtta taagttaaga 17220
   gggttgtggt ttagttatta gaatttgaaa gagataaggt aggatttttt tttagagttt 17280
   ttggaaggat tgttattttg ttaatatttt aaatttgtat ttatgttttt cgaattgtga 17340
   tagaataaat tttttttttt tttttttgag atggaatttt attttgtcgt ttaggttgga 17400
   gtgtagtggc gtgattttag tttattgtaa gtttcgtttt ttcggtttac gttatttttt 17460
   tgttttagtt ttttaagtag ttgggattat aggtatttat tattacgttc ggttaatttt 17520
   ttgtattttt agtagagacg gggttttatc gtgttagtta gtatggtttc gattttttga 17580
   tttcgtgatt cgttcgtttc ggttttttaa aatgttggga ttataggcgt gagttattat 17640
   attcggttaa atatttgttg ttttaattta tttagtttgt agtaatttgt tgtggaaatt 17700
   aatattaaga taaagttaag aattaagaaa tttaatgaat tttaagtaag ataaatacga 17760
   agtaaattat attaaggttt attataatta aatgttgaaa attagtggta aaaagaaaat 17820
   tttaaaagta tttaaagaaa tgaggtttat tatgtatgga agaatagata taggaaatat 17880
   tatatttttt ttattagaaa ttatgtaagt taggaaataa tataatgaaa tttttaaagt 17940
   gttgggggaa aaaaataaaa ataaaaattt ttaatttaga attttttttt tttttttttt 18000
   taatttagta tgattggtat tgatttagaa ttttatattc ggtgaaaata ttttttaaaa 18060
   ttgaagaaga aataaatatg ttggtagata aataaatttt aagggaattt attgttagat 18120
   gtgtaggaaa tgttaaaggg agttttttag gtggaaagaa aatgatttta gatagaaatt 18180
   tgaatttgta taaaggattg aagagtgtta aaaaatgtta aatgtataag aatgttagga 18240
   ggttaggtat agtggtttat gtttgtaatt ttagtatttt gggaggtcga ggcgggtagg 18300
   ttattagagg ttaggagttt gagattagtt tggttaatat ggtgaaattt tatttttatt 18360
   aaaaatataa aaattagttg ggcgtggtgg gggatttttg taattttagt tattagggag 18420
   gttgaggtat aagaatcgtt tgaatttggg aggtggaggt tgtagtgaat agagatcgta 18480
   ttattgtatt ttagtttggg tgatagagag agattttgtt ttaaaaaaaa aaaaaaagaa 18540
   tgttaggttg ggcgtagtgg tttatgtttg taagttttgg gaggtcgagg tgggtagatt 18600
   atttgagttt aggagtttga aattagcgtg ggtaatgtgg tgaaattttg tgttataaaa 18660
   aaatattaaa attagttagg cgtggtggtg tatgtttatc gttttagtta ttgggaggtt 18720
   gaggtgggag gattttttga gttagggagt tagaggttgt agtgagtaga gattatttta 18780
   ttgtatttta gtttaggtaa taaagttaga ttttgtttta aaaaaaaaaa aaaaaaaaag 18840
   ttagtaaaat atttaaaatt tataagaata aagagaagag atgttagtag atgagaaatt 18900
   ttaatataat tttagaatat ggaaagtggt tggggtttta taattgattt aggggagtag 18960
   aggaagttga aattaaagta gttatagagg gaaacgttta tataaggagt aagttaggtg 19020
   atttttagtt tttttgaaag gtgtaggtag gatttgtagg taatggggat ggtagaggtg 19080
   gaggtgaagt tagagttgag tgtagggagg ttggttataa tttaggattt tttggttttt 19140
   tttttagttt tattaagttg ggagatgttt ttttttttta ttataggata ttaggggttt 19200
   attttttgta gaaattgggt atgagatgtt ttggatttag gagtattggt tatagtagag 19260
   gatgaggtat ggtgttgaaa gttagtgatt cgtgaaattt tagttttttt tttatttggg 19320
   ttttgaatta ttggtagtta ggttatatta ttattatttt atttattcga gtaggaagtt 19380
   gttttaaaga ttttagaata tagatatttg gtgattttat aatgaatagg ttagtttgtt 19440
   atttgattat tttatagtag tatttatata tttttgagtt ttttagtttt atttttaaat 19500
   atgatttaat agttaggaga aattggatat ttgatgaaag ttttttatgt gaaataaatt 19560
   aaaatatata aattagatgt taagacggtt gggtgtagtg gtttacgttt gtaattttag 19620
   ttttttggga ggtcgaggtg ggcggattac gaggttagga gatcgagatt attttggtta 19680
   atacggtgaa atttcgtttt tattaaaaat ataaaaaatt agttgggcgt ggtggcgggc 19740
   gtttgtagtt atagttattc gggaggttga ggtaggagaa tggtgtgaat tcgggaggcg 19800
   gagtttgtag tgagtcgaga ttgcgttatt gtattttagc gcgggggata gagcgagatt 19860
   tcgtattaaa aaaaaaaaaa aaaaaaaaaa aaaaaataaa ttagatgtta agaatttaga 19920
   ggaaattttt ttgatgtagg gaataaaaga aaattttttg ggggaaaata gtaatttata 19980
   tttttaggaa aaatataaaa agattattga atttatgaaa taaaatggga tagtttggtt 20040
   ttttatgacg ttttttgttt ttttttttgt tttttttatt ttggaagtga tttttttaat 20100
   ttttttttgt tttttgtatt tttggatagt tgtatttgtt tttataaata gtaaatatta 20160
   tttggattta ttgtattttt aggtggaaaa aaaaattaat tttaaaattg tttatatttt 20220
   tttttttttt cgtatttttt atatttaatt tattagaaaa atttgtaggt tttgttttta 20280
   aaatagattt agaagtcgtt tagtttttat tattttgtag ttgttgtttt tttatttttt 20340
   attggtagtt ttttattgtt tttttgttgt ttttcggttg ttaggtttta ttgtttattt 20400
   tatttatggt agttaaagtt attttgtgaa gtgttattta ggttttgata ttttttagtt 20460
   taaaattttt tagtggtttt ttatcgtagg tagtagggaa tttaatggtt ttagattttt 20520
   ttttgaattt atttgtttta tttttcgtta atggtttttt tcggaacgtt ttttaggatt 20580
   ttttagagag ttttaaagtt tttttttttt ttttatttag gttgttgttt aaatgttatt 20640
   tttttagaaa agtttatttt gatagtttat ttgaggtaat agttgttatg gttttattta 20700
   ttgttcgttt aagtgtttgt tgagtatttt ttgtatttcg aatagaagtt ttattagtta 20760
   ggtttttgtt tgttttgttt attgttggtt ttttattttt taggtagtat ttggtatata 20820
   aatatttgtt agataaatgt atagagtatt aggttgaggg ggtataagga ggggaaggta 20880
   attaattaag aaattattgt agaagtatat tgtttatgtc gggggtttta cgtttgtaat 20940
   tttagtattt tgggaggtta aagtaggtgg attatttgag tttaggagtt cgagattagt 21000
   ttgagtaata tggtaagatt ttatttttat aaaaaaaaat ataaaaatta gttaggtatg 21060
   gtggtattat ttgtagtttt agttttttgg gaggttgaga tgggaggatt atttgagttt 21120
   gggaggtaga ggttgtattg agttgtgatt atattattgt attttagttt gggtgataga 21180
   gtgagacgtt gttttatgaa aaaagaaaaa aaagaagaag aaagaaagag aaagggaggg 21240
   aggtagggaa gggagggagg gagggagaag gggaggggag agggtagtat aaataatata 21300
   ttgtttataa ttagggaggt aaatgataaa aggagtttag agtgttgggg aggtgggaga 21360
   ggcggagtaa ggagtattgt tttttattac gtatttttta gtaatagtta aatattttaa 21420
   agtatgatta tgaattttat tgttaaaaat gaaatatttt aaataattta agattattta 21480
   tataattttt atagttttgt tttggaattt ttttattttt taatatattg ggagttttaa 21540
   aaaaattagc gggtttggtt tttgttggtt tttgagttgt ggttttttat agagaagggg 21600
   agtgttgttt atttttgttt aattttggtt gggttggatt tcggggtttg taatttgggt 21660
   ggggttttga gttcggtagc gggagtggga gagggttata gggttcgagt tgattgggga 21720
   agtgttgagt aatttaggtt ttttagtttt gttttagatg ttgtagtcgt agtcgttgta 21780
   gttaataaga ttttagggag ttggtaggtc gttgtttggt tttagggttt tagatgttgt 21840
   ttttattttt ttattaggtg ttttagtttt aggttttaga gagcggttgg tatttttttg 21900
   taagtagggt gttttttttt atttttatat tttaggagta ttttttaggg aggtttggat 21960
   ggtttagtat tttatatatt ttatgtagaa ttttatatat ttagttaagt tttagagtag 22020
   ttttttttat aagtttagag tgtcgtttgt agtgtaagtg gtattttgtt ttgggtttag 22080
   gtaggagaat ttattaattt ttttagtaga ggtttttagt ttttttttag tagaggtttt 22140
   tagttattta attgttggaa tgaaaaggtt ttttatgaaa tttaatattt agtttttttt 22200
   attaagagaa cggagatttt ttattttagt aggttttagt tggagttaaa aggtggagtt 22260
   tttggagttt tgtagttggg ggtttttggg tagagagttt ttttattttt atttaggtat 22320
   atatattata ttttagtata tattggtatt atatagatat atattcgtac gtttataggt 22380
   atttacgtgt gtttttaagt gtataagtat atgtatgttt agttgttttt tggttttgta 22440
   ggagtttatt tttgtgtaac gtgtgtgtgc gtatttgtat aggaatattt tttatattta 22500
   tgtgtattag tagggtattt tttaaatttt tttagtatga gtagaatttt tttgttaagg 22560
   gaagtatgaa aagtatatat tttatgtatg gattaaatat agtgtttgcg aggaaggtga 22620
   gggacgtaga gtttgtgttt aaggtggtgt ttgtttagtt ttttttttag tttacgagga 22680
   ggaaggtagg agtttggtgt agtcgttgtt tgattttttt tgtttagcgt ggggtgtagg 22740
   ggtaggtttg tttgagttta tttaagggga gggaggtagg atgtattttt aagttgcgta 22800
   ggaggttacg ggttgtgggg atcgtatttt ggattttttt ttttatgtta ttttttttat 22860
   tttgtttttg tagttttttt tttttatttt agaatatatt gatttttggt ttttggggtt 22920
   tgtggttttt tttttcgagt tttttagttt attagttgtg gtgatgtttt atatttagga 22980
   ttgtttagtg taggtaaagg gaggggtttg aggtgttttt cgtatatttt gggttttata 23040
   atttattttt tttgttttta ggttagaagg gtgaataggg aaagtaggaa gtaagtttac 23100
   ggtggagaac gttttaggga gaaggaaaat gattttttgg gattataatt tgtttatttt 23160
   agggttttgg ttgttagggg tagagttttg ttttttaggt ttagtttata tatgtttttt 23220
   ttacgggagt tgtataggtt aatttttttt tttgttttta ttttagggaa tattaggaat 23280
   ttacgatttt agaattagga ttatgtttag ttggttatgt taggtaaggg ggaattggta 23340
   tattttttgg tttataattt tgtttttttc gatttggtag agaatatgta tagcggtaag 23400
   tagggtatcg ggttggtatt atagtattgt tgggtgtttt ttttttttat taggtatttt 23460
   ttatttgttt attatttttt gtttttagtt tttagtagag taatttgggg ttgagtaaag 23520
   tttattaatt tgttttgaag gtggttatgt tggttttttt tttagttagt tgtattgtta 23580
   agtttgttta cggagggttt ttgcggggtt tttttggagt gaagaagagg tagttatgtt 23640
   aagttaagat tagggaaatt tggtttttga gtgtttgagg aaagtcgagt tgtttttgtg 23700
   gcggttggta ttagggggat tagaggttag ggatttttgg ttgggatgtt agggttttat 23760
   agtgtttttt ttatgatgtt aggtggttgt ttttaggttt tttttgtaga aaatagagtt 23820
   tggtgttttg gggagtaggt agtgtatatt tggatataag ttttattttg ggttagtttt 23880
   cgagagggga gatagttagg gaattttttt atttatacgg agtggtattt ttttttttgg 23940
   tacgtttgag ggaaggagag gattggtttg gtttttttgt ggttgaataa gggttaattt 24000
   ttattagtgt tttatatttt ttttggtttg gatttaggat aggtagtgtt tattttaagg 24060
   agatattaat ttattggggt aggggtaggg gtgaatttgg gattttgttt ttgaggtagt 24120
   tagaggggtg ggagagagaa atatatgagt agtttttaat atttttattt gtagcggttt 24180
   cggtgttttt tattttaatt ttattttagt aagatggtta tttattttag gattattagt 24240
   agagaggttt gttggagaag tttttttttt tagggtattt tgtttttttt tagtttttgt 24300
   agtaaaagga ttatttattt ttaggtaagg gagtgggatt gaggtatttt ggagatttag 24360
   ttttttgttt agtgattttt ggatttttgg gaagtttttt ttatatgagg tttgtttttt 24420
   tttgttttta gggaaggaaa gtagagaaga gttagtagag gttggttggt tatgttttag 24480
   cgttttgtat attattttgg aattttttgt ttatatttgt tttattagaa tagagatttt 24540
   ttgagggtag gggttgatgt tttgtttgtt tatttgtttt ttgggggtat tgtgatttat 24600
   tttttttaat aggtttttcg tggttgtaga gtattgtttt tatgatagat aaataaagat 24660
   agggtttgag gagtttagtt tgaggagaaa ttcggagagt gttggtatag gtataggtaa 24720
   taggaagtgg ttatttagat cggtttagag gtttgcgggt ataggttatt cgtatattta 24780
   tagggattgt tatgtagagt tggatgttat gtaggtttgg gggttggttt agcgtgggat 24840
   tttagagata tattttgatt tttagtttgt ttagttcgat ggtttgtttt ttatttttat 24900
   gttttttttg gcgattgtat agttttttag attgaatttt ttatgattta ataagtagtt 24960
   tttagaaggt aaatttttat agcgggtaat tgcgttaatt atttttagtt tatggtttta 25020
   aggttttaga tttagtttaa aattggggtt tttagtttgt agtaagttat gggattcggg 25080
   tttagttttg gtttttgtag tttattttgg aggcgggggg ttatttttgt tatgagtgtt 25140
   tatttgtggt ttgggtgtta agggtagaag tggtaatttg tgatttgtgt ttgtttttgt 25200
   tttgaagtat ggttagaaga tattttgaag tatggttaga agatattttt tttttttttg 25260
   ttttttggag attattgttt agtttaaagt tggtgattta cgtggtttta ttgattagta 25320
   gaaattaggg agtttatttg gtgcgtagaa ttttaagttt ttttagtagt tagggttttt 25380
   tagttttttt tgcgtttaga aattaggttt tagtattaga gggtaggaga gcgtttttta 25440
   gtatttggat aaggtttttg gggtttttta agagggtttt tttttatttt ttgtttttga 25500
   gttggggttg ggttttgaga gatttaagga tttgtttagt ttgagagtta aaagagaaag 25560
   cgtttatcgt tggggagagg gcgttgggtg gggttggggt gtttgggagt gtgatatagt 25620
   ttttttttta tttttgattt atggtttttt ttttacgtaa gtttttttta ttttcgtatt 25680
   tttattcggg aggtgtcgtc gtttaattgg ttggggcgga tttttttatt atatggaata 25740
   tcgatatagt tcgtagtttt tttttgtagt ttttattgtt tcggcggagg aggggtatag 25800
   aggtttttat ttttatttag gtgagttcga aatacgtgtg gtttcgttcg tttcggtcgg 25860
   aggggattat tttttttcgg ttaataagta taaaagtcgc ggtcgtaaat aaagacgcgt 25920
   cgtaaattcg ttagggaggc gggatagttc gcgtcgtatt ttttttttag gtcggacggg 25980
   cgtgttcggt cgcggggttt tcgcggcgtt tcgaaggcgt ttttatttgg ttcgcgcgga 26040
   tttttttttt tagttcgcgg tggcggttgt gttatacgag gtagtgtagc ggatatttgt 26100
   tttcgggttt ttttttggcg gaggcgttgg cggggattat cgttcgtttt ttatgtcggg 26160
   gtcgcggcgt tcggtcgggg aggagggacg gcgtcggagt cggggttcgg ggtcgcgggg 26220
   cggggggtcg gcgttgtttg ttcgcggggt tgcggttcgg gtatgtgcga gcggcggcgg 26280
   gcgggggcgg cgcgttcggc gcggtgcggg cggcgcgggc gacgggcgtt ttcgcgaggc 26340
   ggcggcggcg ggcggcgcgg agaggttttt attcgttttg ggagggagcg ggtattcggc 26400
   ggcgttgcgc ggattcgcgt tcgtttttta tattttcgta tataaagacg tcgttcggcg 26460
   gagggagtag gcgttcgtag cgggcgcggg ggcgtacggg acgcgggttt cggggacgtc 26520
   gtcgtaggga cgcgcgttcg tagttttcgg gtttttatag ttgtatagtt cggtttttag 26580
   ttcgagcggt cggcgcggga tacggggatg tggggatgat ggggagagag gaaggggatt 26640
   aggcgtttcg ggtagagttt gttagtttag tttcgcggta ttatggggtt tgtagttcgt 26700
   ttcgtgaaat tgggggtggt aggtggtcga agagggtttg tattgttttc ggatttcgtg 26760
   ggagagtggg cggggagtta ggtttggttt agacgcgggc ggggttttga cgttaggtgg 26820
   gttgggacgt atttttttcg ttggtatttt gttttttgtt ggggaagtgg gggggtacgg 26880
   gtttaggtcg agcgtttggg agacggggtg gaggtgtggt ttttagaatt gagtttgggt 26940
   tttaggggtt atgaagaagg attcggattt ttagaagaga gcgtttgatt gggaattttt 27000
   gagaaaggtg tggggtatta gtattaaggg ttattggagt agttaggggt ttggtttttg 27060
   gaatattttt gaggagtttt ttttgttttg ttagaaaatt aaattttatt ttttatatat 27120
   gttattggaa atagaatgtt tagtaacgcg ggaaagatgg tttttttttt ttcgtatatt 27180
   gtttttagta agaattagag tgaggttgga tagaaaaagt agtaggtggt agtttcgttt 27240
   ttttatttag ttggtttttt aggaagggaa gtgattgatt ttagagattg tttttttttt 27300
   gaatgatttt agggtgaaga ggtattttta gaatggtttt tgtagagggt gtggtaaaag 27360
   ttgaattggt tttttaagcg ttaattgtaa ggtttggaag gattaagagg tttggattag 27420
   tttttggagg gaggggattg tgatgttaga gagattagag ttcgaatagt tttaattgtt 27480
   tgattttaaa agagtaaatt gtttaatttt ttcgattttt tcggtttatt tttttttaaa 27540
   atgagaattt agatattatt ttttggggtg agttaaggat aatgattgtt ttggaaatag 27600
   cgtttttttt ttttaattag gcgggtcgag ataggtggcg ggattaggta cggtgttttt 27660
   ttgagtttat tttagaataa ggtaggaata agggatttta tatcggtttt atttaaaata 27720
   ataatttaag aagtgtttgt gtgggaggtg ttagtaaaag tttgtaaatt tttttaggtt 27780
   tttttgtgtg gttttttttt tttttttttt tatattttgt ggtgttaggt tttagcggga 27840
   agagatgagg gaggagcggg ttgtttcgtt tttttttttc gtttgggagg agttagttgg 27900
   cgttttcggg ggttcgggat cgttgttcgt ttgtattatt aggggttagt agagtgtcgt 27960
   ggttgtagtt agtgtagggg agggggttgg cgcggttgag gggtatttag gagtggtcgg 28020
   gagtatgttt gtgtattttt ttattaaatt gtttttcgaa ttttgtgtgt gattttattg 28080
   tagtagtttt gttttttggg gtggtgatat ttttacgggg gaatttagaa atttttttat 28140
   ttttagtttt aggtttagaa gtttggttgt gacggggttg ggggaaataa agtttggacg 28200
   atgtgttcgc gttagggtta tttttaggta gtttttgagt taattaggag atggggagtg 28260
   gggtggaggg gtaagtagag tgaattattt ttttttttta gtaaagttta ggagttcgag 28320
   gataggggga gtttaggatg gattgtgggg agttgtcgat tgtggggaat tgttttttgg 28380
   gcggtcgtgt tttttttttg ttaggcggcg gtagatattg aatttaggtc gtttgcgttt 28440
   cggttcgcgt ttttagttgt tacgtgttcg agacgcgttt atatagcgga gaaataaaga 28500
   tagttaagcg gtagggttgt tagtagtttt ttcgatattt aataaatgaa attttatagt 28560
   tgggggtcga tcgggggtga acgggcggtt atcgttcgtg gattgagcgt ttggtacggg 28620
   ttgtttggtt atttttttcg ttttattttt ttcgtttttt attttttttt cgttgaggtt 28680
   attaagttta tggattttgg tgagagagta gttcggggcg gtaggtggtg ttttttttta 28740
   tatattagtt aggtatcgtt gtttgtaaaa gttttattag ttgagcggtt ggttgtcgag 28800
   ggaggggagg tttgtaagat ttttttaagg tgtaattata aataaagttt atggatcgcg 28860
   attttaaata aatttgttaa tttgggtcgt agtaaaaggc gatggaggta gttttgtatg 28920
   tatttcgggt ggtgtgattt atgagtatta tttgtttttt ttagggaagg agtttgttta 28980
   tttataaatt attgtcgttt tggtatcgtt gggataatcg attaatttga gtatttttgt 29040
   taaaggattg tttttatttt aataagtagg agtttttgat ttttggagaa ttttttttta 29100
   attgttatta ttgatttggt ttttaggttt ggttttgggg atttttattt tttttttata 29160
   agtagagtgt tcgggttttt tgttgttgta gggattgggg gttgggtata cgcgcggtat 29220
   acgcgtatgg gggcgtatgt ttagtgcggg ggttgcgttt atgtgcggtt tttatttagg 29280
   ttttttgttt tttagtttat ttacgttttt tatttgtttt gtttttgttt gtttcgtcgt 29340
   gtgaagttta ggaagaaagg tcgttatttg aaacggtttt tttatgttat atgtattttt 29400
   tttggatata tatatttttg gtggtttaga aatatcgtgg gtgtgtgtaa ggcggatgat 29460
   aagtggatgt gagtgtatat aggtatagtg gttatttttc gggaggtttt taaaaatcgt 29520
   ttagattttt ttttttttgg atggaaggat gtatggagag atagatatat ttttagttcg 29580
   gaggtagggg aatatattgt ttgatttttt tggtatttgg gcgtttatat tgttagtagt 29640
   gggcgtattt agtaagagtg tgatatatat atatatattt ttggatatat agatgtggat 29700
   atttacggga tagggacgta tattttattt taattagaga tagacgggta ttttaaggta 29760
   tagttttttt gatttggagc gggatttggg tttttaagtt tttttttttt aatttttatt 29820
   ttcgtcgttc gtgggtttag cgtttttcgg tttattttac gcggaaagtg aaggggaggg 29880
   ggttgtgagg gtgatttata ttgttttcgg gttaggcgag cgttgtagcg gaggttaggg 29940
   tcgtaggtta cgttgttcgt cggggtcggg tatttgtcgc ggttttttta agcgttttcg 30000
   tttttttttg ttcgcgttcg ggtttttttt ttttaggggg cgcggtcggt agcgcgcggc 30060
   ggggcggggg tgcggttttt ttttttgtgg gtagggtttg cgtcgttttt tgtagaagcg 30120
   gtcgggagtc gcgtaggggg cggagagagg agtcgcgagc ggcggcgggg cggcgcgggg 30180
   cggagggcgt tcgagggcga gcgggtaggc gggtacggcg ggtttcgggt tagtttcggg 30240
   gcggacggtt ggtcgggcgc gcggagtttc gtcggggttt cgtcggagtc gggtcgggta 30300
   gtaggggtcg tcgtcgtcgt tcgggttcgt cgttcgcgtt ttttacgttg gtttagaggg 30360
   ttgcggtcgc gtcgtcgttg aggatgtttc gaaaggggtc gcgagcggag gtgtgtgcgg 30420
   attgtagcgt ttcgggtaag cgcggcgggg tcgggggcgg gatcgtatag ttaggtcgtc 30480
   gtcgggcggg cgtcggggtt tggtaagagc gttgtgcggg gtcggagggt tgggcggggc 30540
   ggggggttgc ggtttttagg ttgggttttc gtttcggtta ttgcggagtt gcgtttttgg 30600
   cgggagggtc ggtttggggt gtttatgttt tttggaggtc gtggattagc gaggaaggac 30660
   gttttcggcg tcgtttttcg tttttcggtt tgagggaggc gggtagttgg ttgttttttt 30720
   atttttattt ttgtattcga cggaagcgtt agtttggggt ggtaggaggg tcgtggcgga 30780
   ggtcgttagg gtttgggagg tgggggcgag tgtggtttta tgttatttta gtgttatttt 30840
   cgcgttttgt atttgttgtt cgcgtggtcg gtacgtttgt cggggtggtg gagggtaagg 30900
   agtagagggt cgggtagatg cgttagtggg gtttgaatat cgttttttta ggagtagatg 30960
   agcggtggtt atttttagga ttttttgttt cgggttttta aggttagcgt tttttgtcgg 31020
   gtttggcgtt tagggttagt ttagattcgg gtgttttttc gggaggtatt tttaggtttg 31080
   ttggtggtga ttgggaatag ttcggttgga ttttgttgtt tttgtttatc ggtaaggttt 31140
   gtttgggaaa gtgagtttgg tattaagtta ttttgggtgc gggttttttt ttgtttttta 31200
   gtttagtttg gttagaaatg ggtgggaggt cgtgtcgaga tttttcgtat tttgttggtt 31260
   ttttggtttg ttttttggtt gtatttatat ggtggttttt tttttttttt taatagagtt 31320
   aggattgggt tggagtgaaa ttttttaggt ttgttgagga agttaggggt aatgatttaa 31380
   gaggttgttt ttttttgatg agggaggata gttagtgacg gggttttgta gttggtttgg 31440
   gaaggaagtt ttagtttata tttatttttt ttttagtaaa ggagttattt ttttggtagg 31500
   tttttgggta ggttgcggag gttcgtgaat tttagcgggg ttgtgggtat gattaagagt 31560
   gagagtatgg ggtgttggtt tgtgggttta taatttttag ttattttttg agtaggttta 31620
   ggattttatc ggttgagttt tatagatgtt ttggtggttt tgggggtgtt aagttagagt 31680
   atttgggaat tatgatttag tagggtttgg gtataaaata ttttataggt ggttatgatt 31740
   aaggtttttt aggggtttat tatggtttag agggttttgg ggttgagggt ttatattttt 31800
   aagaaattgt gatttcgttt ttcgagttgt aggtaggttt ggggatggta gggttgagga 31860
   tgtattttgt tttataaatg tttttttatt gttttataat ttcgtggggt aagtatggta 31920
   tggaatgtgg aggagagttt tagtaggcgt ttaatcgggg gagtttgggg tttagtgttg 31980
   tttttgtttt tagtttattt tgtaatttta ataagatttt ttttattgtg gggtttttag 32040
   tttttttaat ttagaggtaa agaagttgga gtagaaatgg tttttggggg gttgttattt 32100
   tcgaatttgt ggtttgattt tggggtggag gggtgaaggg tagtattagt aaggtagttg 32160
   ttttttgtat gttgcgttga atgttcgatt atagtttttg gatgtttagt gggtttaggg 32220
   aggaatggtg ggtattagat gtgttggatt gtttagtttt tttttttagg tttttatttg 32280
   ttagaggaat ttgtgtttta gatgtttttt tttttttgat ttataataga ggttaggaag 32340
   gtggtgagaa ggtgaggatg gtggggtggg gaggatgtgg atagttttat ttattagttt 32400
   atttagtatt tagcgaggtg tttagtatat ggtaggtatt ttttgagtgt ttggttgttt 32460
   gttaggattt tggttggttg tttgggatta ttttatattt ggattttagg atgttggttt 32520
   tggtatgtag cggtatgttt ggtttgggtt ttgtggtatt ttaggtagag aggtatgagg 32580
   gtatttagta gttcggagtt tagatttagt gttagtttta tggtgtatcg ttttatttta 32640
   gggttatagg ttttttttat tatgatagga gttttgtagt tttaattata ggagtggggt 32700
   tagggatttt ttgtgtttgg ttaatttggg gggttatgga ggttagaggt gagatgtagt 32760
   ttgtttttag agagttttat tttttttttg ttttcgggta tgggtatata ggataagtgt 32820
   gagtgtgttg ttaggtagtt ggatatgata tggttgtttt ggtagtttgt gttatttcgt 32880
   taggtatcgt atgtgttttt gtttgtttgg ggagtttaag agaggaaggg ggtagggagt 32940
   tttggagaat gtgtattttt tttttttttt aggtttggtt tatttcgtta tacgatttta 33000
   gttaagggtt tttttttatt ttatgggttt ttattttagt tttttttatt ttggtaattt 33060
   tgggggtgag agtgaaggtt ttaagatgtt tgttttgtag gtatttttgg tgagtaaggt 33120
   cggggatatg ttacgggata agttataggt tgatttgttt ttttatagtg tagattgtag 33180
   agtgggataa atgttgggtt tttttttttt ttttatggag gtagaatttg tgtgtgttaa 33240
   tgtttgtatt tgagtgttta ttttgatggg tttttatatg tgtgtatatt tatgtaatta 33300
   ttgttttgat tatgttatag aataagggta tttaattttt ttgttttttt gggttatatt 33360
   ggaagaagaa tttttttggg ttatatttaa aatacgttaa tattaatgat agttggtgag 33420
   ttaaataaaa taagtaaaaa taaaaataaa aaataaagtt ataaaaaagt tttataatgt 33480
   tttaagaaag tttatgaatt tgtgttgggt tatatttaaa gttgtttggg tcgtatatgg 33540
   attgtaggtt agataagtat gttatagaat atttttatta ttttaggggg tttttaagta 33600
   ttttttttta gttcgtgtat tttagaggta attattattt tgattttatt ttaaggatta 33660
   ttttcgtttg tttttatgat gtgggttttt tttttagttt agtggagatt tggtttttag 33720
   tttaggggag ggaggtttga ggttagtttg tatgtgtaaa tttattggtt ttggaggtta 33780
   ttgatggttt taggtggtgg taggaattta tcgtagtaga ggtttttatg gggttggtat 33840
   tggttaacgt gattttagga ttacgtttgt tattttttta tatatataga tttatatttt 33900
   gtcggtggag ttagaagcgt agggggaggg ttgacgagtt tttttatagt ttttgttttt 33960
   ttcgttttag gtttggttta aggagttttt tgacgtaagg atttttttat tttaaattat 34020
   aatttgggta gttttaaggg tatttgtata gagggaggag atagaatggt tagggtgggc 34080
   gttttatttg tgttatttag aaatgagttg atattaatag cggttgttta gattaggata 34140
   gtgttttttg tttattattt tttttaggtt atttggagga gaaggagaat ttttttttta 34200
   ggttggggga aagtgttagg taaatttttg gattatattt ttgtattttg gttttggtta 34260
   ttgttatagt tagggttatt tattatattt ggtttgttta tggtttaggg ttttggagtt 34320
   attggtcgtg gatatatagt tagtttttta tgtttggttt tttcggtagg attatagatt 34380
   ttagattagt ttatttatga ttttgttttg ggcggttttt tatttatagg aaattttatt 34440
   aagaggaatt tgggttgtat tattttcggt tttttgtttg ggttgttgga ggtggttggt 34500
   tggtagtttt ttgatttagt ggtgtttgga gtggggagag ggaggttttt atatttgggg 34560
   tgttttatta tttgggtaaa gttagtttag aagtgtatgt tagggttttt ttttttggcg 34620
   aagaaggttg gttttttttt tttgagtttt ggagttagtt ttttaggaag gtgttggcgg 34680
   ttattttggg tatttttttg ttgttttgat tgggaggggg ttttttaaaa agtttaaatt 34740
   cgttgtattt tagtttgggc gatagagcgg gatttcgttt taaaaaaaaa aaaaaaaaaa 34800
   aagtttaaat ttaaagatag gttttttttt tttatttttt gtttttatgt tttagtgggg 34860
   gttatttgtt gtgggtagag tttaggttgg gtttttgatt tagagttttt ttaatttttt 34920
   tgggaatttt ggtggaagtt tttagagggg aaaggtttga gaattgtttt ttagttggga 34980
   gagattttta ttt 34993
<210> 6
   <211> 24993
   <212> DNA
   <213> Homo sapiens
<400> 6
   gcttaaaggg gccgcggcag atgcccggcc ccggcgggca gcgtggcctg cggccctggc      60
   ctccgctgca gcgctcgcct ggcccggagg caatgtgagt caccctcaca gccccctccc     120
   cttcactttc cgcgtggggt ggaccggggg acgctgggcc cacggacggc gagggtgggg     180
   attagggaag aaggacttgg aggcccaagt cccgctccag gtcaaagggg ctgtgccttg     240
   gggtacccgt ctatctctgg ttggggtgag gtgtgcgtcc ctgtcccgta ggtgtccaca     300
   tctgtgtgtc caggggtgtg tgtgtatgtg tcacactctt gctgaatacg cccactgcta     360
   acaatatgga cgcccaggtg ccagaaaggt caggcagtgt attcccctgc ctccggacta     420
   aaaatatatc tatctctcca tgcatccttc catccagaga ggagagaatc tgggcggttt 480
   ttaaagacct cccgaggagt gaccactgtg cctgtgtgca ctcacatcca cttgtcatcc 540
   gccttacaca cacccacggt gtttctgggc caccagaggt gtgtgtgtcc agaaagggtg 600
   catataacat agaggggccg tttcaggtga cggcctttct tcctggactt cacacgacga 660
   ggcaggcaaa ggcaaggcag gtgagggacg tgggtgggct ggggggcagg gagcctaggt 720
   agaggccgca catgagcgca gcccccgcac tgagcatgcg cccccatacg cgtgtgccgc 780
   gcgtgtaccc aacccccaat ccctgcagca gcaaaaggcc cgggcactct gcttgtgggg 840
   agagggtaga ggtccccagg gccagacctg ggggccaggt cagtggtgac aattgaggag 900
   gggttctcca agagtcagag actcctgctt gttaagatgg gggcagtcct ttggcagggg 960
   tgctcaagtt ggtcgattat cccaacggtg ccagagcggc agtgatttgt gggtgggcag 1020
   gctccttccc taggggagac aagtaatgct catgagtcac accacccgaa atacatgcaa 1080
   agctgcctcc atcgcctttt gctgcggccc agattgacaa gtttgtttga aatcgcgatc 1140
   catgggcttt atttataatt gcaccttaaa aaaatcttgc aggcctcccc tccctcgaca 1200
   gccagccgct cagctggtgg ggcttttgca agcaacgata cctagctggt gtgtaggggg 1260
   gagcaccacc tgccgccccg agctgctctc tcaccagagt ccatgggctt ggtggcctca 1320
   gcgggaaggg ggtaaggggc ggggggagtg agacgggagg agtggccagg cagcccgtgc 1380
   caggcgctca gtccacgagc ggtggccgcc cgttcacccc cgatcgaccc ccagctatgg 1440
   aatttcattt attaaatgtc gaggaggctg ctggcagccc tgccgcttgg ctgtctttgt 1500
   ttctccgctg tgtgggcgcg tctcgagcac gtggcagctg agagcgcggg ccgaagcgca 1560
   agcgacctgg gttcagtgtc taccgccgcc tggcaaaaaa agaacacggc cgcccagggg 1620
   acagttcccc acaatcgaca actccccaca atccatcctg aactccccct gtcctcggac 1680
   tcctgggctt tgctggggaa gaaggatgat tcactctgct tacccctcca ccccactccc 1740
   catctcctgg ttggctcaga gactacctgg gagtggccct ggcgcggaca catcgtccaa 1800
   actttgtttc ccccagcccc gtcacagcca aacttctggg cctgggactg gagataaaag 1860
   ggtttctgga ttcccccgtg gagatgtcac caccccagga agcaggactg ctacagtgga 1920
   atcacacaca aggttcgaaa aacaatttaa taaggaaatg cacaggcatg ctcccggcca 1980
   ctcctagatg cccctcaacc gcgccagccc cctcccctgc actggctgca gccacgacac 2040
   tctgctgacc cctggtggtg caggcgggca gcggtcccgg gcccccggag acgccagctg 2100
   gctcctccca ggcgggagaa aggggcgagg cagcccgctc ctccctcatc tcttcccgct 2160
   ggggcctggc accacagggt gtggaggagg aaggagggaa gaccacacaa agaaacctga 2220
   gagagtttgc aagcttttgc tggcacctcc cacacaaaca cttcttggat tgttgtttta 2280
   agtagggccg gtgtggaatc ccttattcct gccttgttct gaggtgggct caagaaggca 2340
   ccgtgcctgg tcccgccacc tgtctcggcc cgcctggttg ggggagggag gcgctgtttc 2400
   cagggcagtc attatcctta gctcacccca ggaggtagta tctagattct cattttgaag 2460
   gggagtggac cgagaaagtc ggaaaaatta agcagtttgc tcttttaagg tcaggcagtt 2520
   ggagctattc gaactctggt ctctctgaca tcacagtccc ctccctccag ggactggtcc 2580
   aagcctcttg gtccttccag gccttgcaat tggcgcttga gaggccaatt caacttttac 2640
   cacaccctct gcagggacca ttctggaagt acctcttcac cctggggtca ttcaggaagg 2700
   agacagtctc tggaatcagt cacttccctt cctgaagggc cagctgggtg gaggaacgga 2760
   gctgccacct gctgcttttt ctgtccaacc tcactctggt tcttgctggg gacagtgtgc 2820
   ggggggaaag ggaccatctt tcccgcgtta ctggacattc tgtttccaat gacatgtgtg 2880
   gaagatggag tttaattttc taacagggca gggaagactc ctcagaaatg ttccaggggc 2940
   cagacccctg gctgctccaa tgacccttgg tactgatgcc ccacaccttt ctcaggagtt 3000
   cccaatcaga cgctctcttc tgggaatccg gatccttctt catggcccct gggacccaag 3060
   ctcaattctg aagaccacac ctccaccccg tctcccaaac gctcgaccta ggcccgtgcc 3120
   cccccacttc cccagcaaaa ggcaaaatgc cagcggggga ggtgcgtccc agcccacctg 3180
   gcgtcagggc cccgcccgcg tctgggccag gcctgactcc ccgcccactc tcccacgaga 3240
   tccggggaca gtgcagaccc tcttcggcca cctgccaccc ccagtttcac gaggcggact 3300
   acaaacccca tgatgccgcg gggctgagct gacaagctct acccggggcg cctggtcccc 3360
   ttcctctctc cccatcatcc ccacatcccc gtatcccgcg ccggccgctc gggctgaggg 3420
   ccgggctatg cagctgtggg gacccggggg ctgcgggcgc gcgtccctgc ggcggcgtcc 3480
   ccggggcccg cgtcccgtgc gcccccgcgc ccgctgcggg cgcctgctcc ctccgccgag 3540
   cggcgtcttt gtgtgcgggg gtgtgggagg cgagcgcgag tccgcgcagc gccgccgagt 3600
   gcccgctccc tcccagggcg ggtgggggcc tctccgcgcc gcccgccgcc gccgcctcgc 3660
   gaggacgccc gtcgcccgcg ccgcccgcac cgcgccgggc gcgccgcccc cgcccgccgc 3720
   cgctcgcaca tgcccgagcc gcagccccgc gagcaggcag cgccggcccc ccgccccgcg 3780
   gccccgggcc ccggctccgg cgccgtccct cctccccggc cgggcgccgc ggccccggca 3840
   tgaggagcgg gcgatgatcc ccgccaacgc ctccgccagg aaggggcccg agggcaagta 3900
   tccgctgcac tacctcgtgt ggcacaaccg ccaccgcgag ctggagaagg aggtccgcgc 3960
   gggccaggta ggagcgcctt cggggcgccg cggggacccc gcggccgggc acgcccgtcc 4020
   ggcctgggga gggggtgcgg cgcgggctgt cccgcctccc tggcgagttt gcggcgcgtc 4080
   tttgtttgcg gccgcggctt ttgtgcttgt tgaccgggaa agggtgatcc cctccggccg 4140
   ggacgggcgg gaccacacgt gtttcggact cacctagatg agagtagagg cctctgtgcc 4200
   cctcctccgc cggggcagtg agggctgcag ggaggggctg cgggctgtgt cggtgttcca 4260
   tgtggtggga gagtccgccc cagccagttg agcggcgaca cctcccgagt gagggtgcgg 4320
   aggtgggaaa ggcttgcgtg gggagggggc cataggtcaa gaatggggaa gaggctgtgt 4380
   cacactccca ggcaccccag ccccacccag cgccctctcc ccaacggtga gcgctttctc 4440
   ttttagctct caaactgggc agatccttgg atctctcaga gcccagcccc agctcaggaa 4500
   cagggagtgg agaaagaccc tcttgggagg ccccaagggc cttgtccagg tgctgaaaag 4560
   cgctctcctg ccctctggtg ctggggcctg gtttctaggc gcagaggaag ctgaggggcc 4620
   ctggctgctg gaaggacttg aggttctgcg caccaggtgg gctccctggt ttctgctggt 4680
   cagtaaggcc acgtgggtca ccagctttgg gctgggcagt ggtctccaga aaacagggga 4740
   aggggggatg tcttctggcc atgcttcagg atgtcttctg gccatgcttc agaacaaagg 4800
   caggcacagg tcacagattg ccacttctgc ccttggcacc caggccacag gtaggcactc 4860
   atggcagaga tgaccccccg cctccagggt gggctgcaag agccagagct gggcccgggt 4920
   cccatggctt gctgcaggct gagaacccca gttttggact gggtctgaga ccttggggcc 4980
   atgggctggg ggtggttagc gcagttgccc gctgtgggaa tttaccttct gagggctgct 5040
   tattgagtca tgagaagttc agtctgagga gctgtgcaat cgccaaggga agcatggagg 5100
   tggagggcag gccatcggac tgagcaggct gggggtcagg atgtgtctct gaagtcccac 5160
   gctgagccag cccccaggcc tgcatggcat ccagctctgc atggcagtcc ctgtaaatat 5220
   gcgagtgacc tgtgcccgca ggcctctggg ccggtctggg tggccacttc ctgttacctg 5280
   tgcctatgcc agcactctcc gagtttctcc tcagactgaa ctcctcagac cctgtctttg 5340
   tttatctatc ataaaggcag tgctctgcag ccacgagaag cctgttaaag agagtgggtc 5400
   acagtgcccc caggaaacag atgaacaaac agggcatcaa cccctgccct caaggagtct 5460
   ctgttctggt gaagcaagtg tgaacaggaa attccagggt gatgtgcaaa gcgctaagac 5520
   atggccagcc agcctctgct ggctcttctc tgctttcctt ccctgaaagc aaaagaagac 5580
   aagcctcatg tgggaagaac ttcccagggg tccaaggatc actaggcaga aggctgaatc 5640
   tccagaatgc ctcagtccca ctcccttgcc tgagggtggg tggtcctttt actgcagaga 5700
   ctggagagag gcagaatgcc ctgggagaaa agacttctcc agcaggcctc tctgctgata 5760
   atcctgaagt gggtggccat cttgctgggg taaggttggg gtggggggca ccggagccgc 5820
   tgcaggtaga ggtgttgggg gctgctcatg tgtttctctc tcccacccct ctggctgcct 5880
   cagaaacaag gtcccagatt cacccctacc cctgccccag tgagttaatg tctccttgaa 5940
   atggacactg cctgtcctgg atccaggcca gggggaatat agggcactgg tggaggttaa 6000
   cccttgttca accacagagg agccaggcca gtcctctcct tccctcaggc gtgccaaaaa 6060
   gaggaatgcc actccgtgta ggtgggagag ttccctagct gtctcccctc tcgaggactg 6120
   gcccagagtg aggcttgtat ccagatgtac actgcctact ccccagggca ccaggctctg 6180
   ttttctgcaa gggggacctg gggacagcca cctaacatca tgagaaaagc actgtggaac 6240
   cctggcatcc cagccagggg tccctggcct ctagtccccc tggtaccagc cgccacagaa 6300
   acagctcggc tttcctcaag cactcaggag ccaggtttcc ctggtcttgg cttggcatag 6360
   ctgcctcttc ttcactccaa gaaagccccg cagaggccct ccgtgggcag gcttggcagt 6420
   gcagctggct gaggaggggg ccagcatggc caccttcaga gcaggttaat gggctttgct 6480
   cagccccaaa ttgctctgct ggaggctgga agcagggagt gatgggcaga taggaggtgc 6540
   ctggtgaagg gaaggggcac ccagcagtgc tgtgatgcca gcccggtgcc ctgcttgccg 6600
   ctatgcatgt tctctgccag gtcggggggg acagggttgt aagccaggga atatgccagt 6660
   tcccccttgc ctggcatggc cagctgggca tggtcctggt tctggagtcg tgagttcctg 6720
   gtgttccctg gggtgggaac agggggaagg gttagcctgt gcagctcccg tgggagggac 6780
   atgtgtgggc tgggcctggg gagcaggact ctgcccctgg caaccagaac cctgaagtgg 6840
   gcagattgtg gtcccagaga atcattttcc ttctccctgg ggcgttctcc accgtgggct 6900
   tgcttcctgc tttccctgtt cacccttctg acctagaggc aggggagatg ggttgtgggg 6960
   cccaggatgt gcgaggagca cctcagaccc ctccctttgc ctacactggg cagtcctgga 7020
   tgtggggcat caccacagct ggtaagctag gaagctcggg agagggagcc acaaacccca 7080
   gaaaccaaaa atcaatgtgt tctgggatga gggggagggg ctgcaagggc aggatgggag 7140
   gggtgacatg gagggagaag tccagggtgc ggtccccaca gcccgtggcc tcctacgcag 7200
   cttgaggatg catcctgcct ccctcccctt gaatgagctc aggcagacct gcccctgcac 7260
   cccacgctgg gcaggagagg tcaagcaacg actacaccag actcctgcct tcctcctcgt 7320
   gggctgggga aggggctgag caggcaccac cttgagcaca ggctctgcgt ccctcacctt 7380
   cctcgcaagc actgtgttta gtccatacat gagatgtatg cttttcatac ttcccttaac 7440
   aaaaggattc tgctcatgct aaaggagttt ggaaagtacc ctgctagtgc acatgggtgt 7500
   gaggggtgtt cctgtgcaaa tacgcacaca cacgttacac aggagtggac tcctgcaggg 7560
   ccagaaggca gctgggcatg catgtgcttg tacacttaag ggcacacgtg agtacctatg 7620
   agcgtacgag tgtgtatctg tatggtacca gtgtgtacta gagtgtggtg tgtgtgcctg 7680
   agtggaggta gggaggctct ctgcccaggg acccccagct acaaaactcc aaagactcca 7740
   ccttttgact ccagctgagg cctgctggga taaggaatct ccgttctctt ggtggggagg 7800
   gctgggtgtt gggtttcatg ggaaaccttt tcattccagc aattaaatag ctagaggcct 7860
   ctgctgagga gaagctggag gcctctgctg aggagattaa tgaattctcc tacctgggcc 7920
   cagggcaagg tgccacttac actgcaggcg gcactctggg cttgtgggga gagctgctct 7980
   ggagcttggc tgagtgtatg gggttctgca tggggtgtat ggggtgctga gccatccagg 8040
   cctccctggg gagtgctcct gaggtgtggg gatggggagg agcaccctgc ttgcaaggaa 8100
   gtgccagccg ctctctggag cctggagctg aggcacctga tggggaggtg gaggcagcat 8160
   ctggagccct gaggccaggc agcggcctgc cagctcccta gggtcttatt agctgcagcg 8220
   gctgcggctg cagcatctgg ggcagggctg ggaggcctgg attgctcagc acttccccag 8280
   tcagctcggg ccctgtggcc ctctcccact cccgctgccg ggctcaggac cccacccagg 8340
   ttgcaagccc cggagtccag cccaaccagg attggacaag agtaaacaac actccccttc 8400
   tctgtgaggg gccacagctc agaggccaac aggggccagg cccgctggtt tttttgaggc 8460
   tcccagtata ttgaaaaatg aagaaattcc aggacaaggc tataaaaatt atataaataa 8520
   tcttaaatta tttaaaatgt ttcatttttg acaatggagt tcatggtcat gctttaaaat 8580
   atttaactgt tgctagaaga tgcgtaatga aaagcagtgc tccttgctcc gcctctccca 8640
   cctccccagc actctggact ccttttgtca tttacctccc taattataaa caatatattg 8700
   tttatactgc cctctcccct ccccttctcc ctccctccct cccttccctg cctccctccc 8760
   tttctctttc tttcttcttc ttttttttct tttttcatga gacagcgtct cactctgtca 8820
   cccaggctgg agtgcagtag tgtgatcaca gctcagtgca gcctctgcct cccaggctca 8880
   ggtgatcctc ccatctcagc ctcccaagga gctaggacta caggtggtgc caccatgcct 8940
   ggctaatttt tgtatttttt tttgtagaga tggggtcttg ccatgttgct caggctggtc 9000
   tcgaactcct gagctcaagt gatccaccta ctttggcctc ccaaagtgct gggattacag 9060
   acgtgagccc cccggcataa acaatatact tctgcaatga tttcttggtt aattaccttc 9120
   ccctccttgt gccccctcag cctagtactc tatgcatttg tctagcaaat atttatgtgc 9180
   caggtgctgc ctggggagtg gggaaccagc agtgaacaaa gcaggcaggg acctggctga 9240
   tggggcttct gttcgaggtg caggaggtgc tcaacaggca cttggacggg cagtggatag 9300
   agccatagca gctgttacct cagatgggct gtcagggtgg gcttttctaa ggaggtgaca 9360
   tttgagcagc agcctgagtg aagggaggga gaaagctttg gggctctctg gaggatcctg 9420
   gagaacgttc cggggaaggc cattggcgaa gggtggagca aatgagttca aagagaagtc 9480
   tggggccatt ggattccctg ctacctacga tggaaagcca ctggaagatt ttgaactaaa 9540
   gagtgtcagg acctgagtaa cacttcacag ggtgacttta gctgccatag gtgaggtgga 9600
   caatagagcc tggcaaccgg ggagcagcaa agaggcagta gggggctgcc agtgagagat 9660
   gggggggcaa cagctgcaag gtagtggaaa ctgggcggct tctaggtctg ttttggaggc 9720
   agagcctaca agtttttctg atgggttaga tgtggagggt gcgggaaaaa gaagagatgt 9780
   agacagtttt aaagttggtt tttttttcca cctgaaaatg cagtaaatcc aggtgatgtt 9840
   tactgtttat gggggcaggt gcagctgtcc aggaatgcag aaaacaagga aaagttaaga 9900
   aagtcacttc cagaatgaag aaggcaagag aaagggcagg agacgtcatg gaaagccagg 9960
   ctgtcccatt ttgtttcatg aattcaatga tctttttata tttttcctga ggatgtaaat 10020
   tactgttttc ccccaaagag ttttctttta ttccctgcat caagagaatt tcctctgggt 10080
   tcttagcatc taatttgttt tttttttttt tttttttttt ttttttgata cggagtctcg 10140
   ctctgtcccc cgcgctagag tgcagtggcg caatctcggc tcactgcaag ctccgcctcc 10200
   cgggttcaca ccattctcct gcctcagcct cccgagtagc tgtgactaca ggcgcccgcc 10260
   accacgccca gctaattttt tgtattttta gtagagacgg ggtttcaccg tgttagccag 10320
   gatggtctcg atctcctgac ctcgtgatcc gcccacctcg gcctcccaaa gagctgggat 10380
   tacaggcgtg agccactgca cccagccgtc ttagcatcta atttgtatgt tttggtttat 10440
   ttcacatggg aagctttcat caagtgtcca gtttctcctg gctgttgggt catatttaag 10500
   aatggggctg gggagctcag ggatgtgtgg gtgctactgt aaggtgatca ggtggcaggc 10560
   tggcctgttc attgtagggt caccagatgt ctgtattctg aggtctttgg gacaacttcc 10620
   tactcgggta gatagggtgg tggtggtgtg acctggctgc cagtggttca ggacccaggt 10680
   agggaagggg ctggggtttc acggatcact ggctttcagc accatacctc atcctctgct 10740
   gtggccaatg ctcctgagtc cagagcatct catgcccaat ttctacaaag aataaacccc 10800
   tggtgtcctg tggtgaggaa ggggggcatc tcccagctta atgaggctgg gaaggggacc 10860
   aggggatcct gaattgtaac cagcctccct gcactcagct ctagcttcac ctccacctct 10920
   gccatcccca ttgcctgcag gtcctaccta cacctttcag gggggctggg ggtcacctgg 10980
   cttgctcctt atgtggacgt ttccctctgt ggctgcttta gtttcagctt cctctgctcc 11040
   cctaagtcag ttgtgaggcc ccagccactt tccatgttct aaagttgtgt tgaagtttct 11100
   catctgctga catctcttct ctttattctt gtgggtttta aatattttac tgactttttt 11160
   tttttttttt ttttgagaca gggtctggct ttgttgccta ggctggagtg cagtgggatg 11220
   atctctgctc actgcaacct ctggctccct ggctcaaggg atcctcccac ctcagcctcc 11280
   cagtagctgg gacgataggc atgcaccacc acgcctggct aattttagta tttttttgta 11340
   gcacagagtt tcaccacatt gcccacgctg gtttcaaact cctgggctca agtgatctgc 11400
   ccacctcggc ctcccaaagc ttacaggcat gagccactgc gcccagcctg acattctttt 11460
   tttttttttt tgagacagag tctctctctg tcacccaggc tggagtgcag tggtgcgatc 11520
   tctgttcact gcaacctcca cctcccaggt tcaagcgatt cttgtgcctc agcctcccta 11580
   gtagctggga ttacaggaat cccccaccac gcccagctaa tttttgtatt tttagtagag 11640
   atggggtttc accatgttgg ccaggctggt ctcaaactcc tgacctctgg tgacctgccc 11700
   gcctcggcct cccaaagtgc tgggattaca ggcatgagcc actgtgcctg gcctcctgac 11760
   attcttatac atttaacatt ttttggcact cttcaatcct ttgtacagat tcaagtttct 11820
   atctgggatc attttctttc cacctgaaga actcccttta acatttcctg cacatctggc 11880
   aataaattcc cttagaattt gtttgtctgc caacatattt atttcttctt cagttttgaa 11940
   gaatattttc accgggtatg gaattctagg tcaataccag tcatactgga ttagagaaaa 12000
   aaaaaaaaag aattctaggt tgaaagtttt tgtttttgtt tttttccccc agcactttaa 12060
   agatttcatt atattgtttc ctggcttaca tagtttctga tgagaagaat atagtatttc 12120
   ctgtatctgt tcttccatac ataatggacc tcatttcttt gaatgctttt aggattttct 12180
   ttttaccact agttttcagc atttgattat gatggacctt ggtgtagttt acttcgtgtt 12240
   tgtcttactt ggggttcatt gaatttcttg gttcttggct ttatcttggt attagtttcc 12300
   acaacaaatt actacaaact aggtgggtta aaacaacaaa tatttggccg ggtgtggtgg 12360
   ctcacgcctg taatcccagc attttgggag gccgaggcgg gcggatcacg aggtcaggag 12420
   atcgagacca tgctggctaa cacggtgaaa ccccgtctct actaaaaata caaaaaatta 12480
   gccgggcgtg gtggtgggtg cctgtaatcc cagctacttg ggaggctgag gcaggagaat 12540
   ggcgtgaacc ggggaagcgg agcttacagt gagctgagat cacgccactg cactccagcc 12600
   tgggcgacag agtgagattc catctcaaaa aaaaaaaaaa aaaatttatt ctgtcacagt 12660
   tcgggagaca taaatacaaa tttgagatgt tggcagggtg gcagtccttc caaaggctct 12720
   agggaagagt cctgccttgt ctctttcaga ttctggtgac tgggccacaa ccctcttggc 12780
   ttgtggctgc agaactctag tctctgcctc cgtcttcaca tggccttccc acctctctga 12840
   gtgtcttaga aagacacctg tcattggatt tagggccccc tgggtgatcc aggattattt 12900
   catcttgaga tccttaactt aattatgtct gcaaagaccc ttttttgaaa taaggtctca 12960
   ttcataggct gtgagggtca gaatgtgatg tggacatgtc ttaggagggg caccattctt 13020
   caacccacca tagtagtttt tatcaagttt ggaaaaattt tggacgttct ttctttcttt 13080
   tattatttat ttacttattt tgaaatggag tctccctctg tcgcccaagc tggagtgcag 13140
   tgacacgatc tcggctcatt gcaacctccc cttcccgggt tcaagcgatt ctcctgcctc 13200
   agcccccaag tagctgggac tacaggtgcc caccaccacg cctggctaat tttgtatttt 13260
   ttagtaaaaa tggggtttca ccatattggc caggctggtc tcaaactcct gatgttgtga 13320
   tccacccgcc ttggcctccc aaagtactgg gattacaggc gtgagccacc gcacccggcc 13380
   tatttctcta aatttgtctt atttgtccta cttctttatt ctgtcctttg ggattccaat 13440
   agatatatgt tacaatgctt gataatgtct tacagattat gaggctccat agagacaggg 13500
   tcttgctctg ttgcccaggc tggagtgcag tggcgtgatc atagctcact gcagcctcga 13560
   actcctgggc tcaagggatc ctcctgcctc agcttcagag gagctgggac tacagctgct 13620
   tgccaccatg cccagctaat gtaaaagttg ttttgttttg ttttgttttg tttgtagaga 13680
   cagagtcttg ctgtattgcc tgggctggtc ttaaactcct gaactcaagc caacctcccg 13740
   ccttggcctc caaaagtgct gggactatag aagcccagcc aactttattt ttcagtactg 13800
   tttttctcct ctgtgcttca gtttggaaag ttctattgct gtcttcaaat tcactgattt 13860
   ttaaattgtg cagtgtctaa tctgctgtta tttccattca gtgaattttt catttcagtt 13920
   actgtactta ctatctttag atggtccact gggttctttt ttatacctct cattcctctc 13980
   ctcatgaaca tctttttcat gtttttcttt aaactcttga gtatattgag ccatctatca 14040
   tctttatcat ttctgagttt tgtttctatg aaattatttt tctcctgatt ataggttata 14100
   ttttctttct tttttttttt tttttgagat ggagtctctc tctctgttgc ccaggctgga 14160
   gtgcaggggc gccatctcgg ctcactgcaa gctcgatctc ctgacctcgt gatccacccg 14220
   cctcagcctc ccaaaatgct gggattacag gctcacgtga gccaccgcac cctgcctata 14280
   agttttattt tcctactttt tggcatatct agtaattttt aactgaattt tggacattgt 14340
   aaatatgatg ttaagcatct ggatttgtta ttgttcttta aagagtgttg ggctttgttc 14400
   aggcagacta ggtaaattac ttgcaaatca gtttgacctt tttgaggctt ttttttttga 14460
   gacagagtct tgctctgtca cccaggcttg agtgcagtgg ggcaatctca gctcattgca 14520
   gcctcaacct cctgggctta agggatcctc ctgcctcagt cactgagtag ctgaggctac 14580
   aggcacatgc taccatacct ggctaatttt ttgtattttt tgtagacatg gggttttggt 14640
   atgttgccca ggctggtctt gaactcctag gctcaagtga tctgcctgcc tcagcctccc 14700
   aaagtgctgg gattagaggt gtgagccacc gtgcctggcc tttgatgcta atttttcttt 14760
   ttctttttct tttcttttct tttttttttt tgagatggag tcttgctctg tcacccaggc 14820
   cagagtgtag tggtgcagtc ttggctcact gcaacctccg cctcctgggt tcaagtgatt 14880
   cttgtgcctc agcctcctga gtagctggga ttacaggcac ccatcaccaa gcccggctaa 14940
   ttttttgtat ttttagtaga gatgtggttt cgccattttg gccaggctgg tcttgaactc 15000
   ctgacctcag gtgatctgcc cgcctccgcc tcccaaagtg ctgggattac aggcatgagc 15060
   cactgcaccc ggcctgaggc taatttttaa tctgttaggg tgggtctagg tgagcctttt 15120
   ctctagggat agtttagctc tactgctaag gcataaccct tctgaggtct gattaaatat 15180
   gcatctccac tctaggaggc tggagcttga ttatctcctt gcttttggtg agctctgaga 15240
   atttaccatt ttattgctcc tttttgtgtg gtcttactga gtttcactat acacatgtga 15300
   agtttagtat tcaggaagac ttgaagagac ccctatgcag atttctggag ttttttgagt 15360
   agtttactct tttccagaac tgtgctctgc tgcaaccttt aataccttgg cttccctgaa 15420
   tcctgtctcc atcttctcaa ctcaggaaga gcaccatgct ctgctcgggc ttcccctcct 15480
   tgaattacag tctggaatgt tcctccaggc agaaatccag ggatgttaca gagctcacct 15540
   tgttcatttt ccttacttca gggatcacag ttctggactt ctaaaaacag ctatttctct 15600
   ttacatatat tttctatata gagaaatata tatatataca cacacaaaca cacacacaca 15660
   cacacataca cacacacata tatatataca cacacacgca tatgtatata tatatttgag 15720
   acagcgtctc actctgttgc ccagcctgga gtgcagaggt gtgatgatac ctcactgcag 15780
   cctcaacctc ccaggcttaa gcgatcctcc cacctcagcc tctgagtagc tgggactaca 15840
   ggcacaggcc atcatacccg gctaattctt ttgtattttt tgtagagatg gggttttgcc 15900
   atattgccca ggctgatctc taactcctgg gctcaagcaa tccgccaacc tttgcctccc 15960
   aaattgctgg gattacaggg gtgagcctct gtgcccagcc ttcatatatt ttgcagcaat 16020
   aattttctca ttgtttatgg tggcagagta atttcattta ttatggtagg agtagaagcc 16080
   tttttactgg cgttttaatc aagttcagag aagaggaggg agttacatgc atgtggtcag 16140
   ttcactgttc cttttttttt tgagacggag ttttgctctt gtcgcccagg ctggagtgca 16200
   gtggcatgat ctcggctcac tgcaacctcc gcctcccggg ttcaagcggt tctcttgcct 16260
   cagcctcctg attagctggg attacaggca tgggccacca cgcctggcta attttgtatt 16320
   tttagtagag atggggtttc tccatgttgt caggctggtc tcgaactccc aacctcaggt 16380
   gatctacccg cctcggcctc ccaaagtgct gggattacag gcgtgagcta ccgcgcctgg 16440
   cctgtcagtt cactgttctt aaccagaagt cctagactct cgaaatatat ttaaatactt 16500
   atgcttaaga ccgggcgtgg tggctcacgc ctgtaatccc aacactttgg gaggccaagg 16560
   caggcgaatc acttgaggtc aggagttaga gaccagcctg ggcaacatat caaaaccccg 16620
   tctctactaa aatacaaaaa ttagcctggc atagtggtgc tcgcctgtaa tcccagctac 16680
   tcagaaggct gaggcacaag aatcgcttga acctgggagg cggaagttgc aatgagccaa 16740
   gatggcacca ctgcactcta gcctgggtga cacagtgaga ctctgtctca aaaaacaaac 16800
   aaacaaacaa acaaacaaaa cttacgttta acaaacatgc tttctaagcc aggtgcaggg 16860
   gcttgtaatc ccagctgctt gggaggctga aacaggagga ttgctcaagc ctaggagttt 16920
   gagaccagcc tgggtgacat agtgagaccc tgtctcaagt aaaaaaaaaa aaaaaaaaaa 16980
   aaaaaaaaaa aatcttttgt ctgggcactg cggctcacac ctgtaatccc agcactttgg 17040
   gagcccgagc caggtggatc acctgaggtc aggagttcaa gactagcctg gccaacatgg 17100
   tgaaaaccca tctctactaa aaatacaaaa aattagccag acgtgttggc gaatgtctgt 17160
   aatcccagct acttgggagg ctgaggcagg agaatcgctt gaacctggga gacggaggtt 17220
   acagcgagct gatatcacgt cattgtactc cagcctagcc aacagagtga gactccgtcc 17280
   ccccaaaaaa aacacaaaaa aaaaccccaa aaaaagcttt tatcactttt atcacacaac 17340
   agaaatctca tgcaatgtaa tcatgttatt cacaaaatta taggatttgt acaaatttta 17400
   atgcatagtg tcccacaagc acaggacatg gttgtgaaat tgtattaata ccatgaactt 17460
   gttatttcat tcctgtctgt gtattactgt aaccgtttgt taaacctcac tcagtgataa 17520
   tgtcagtgat ctaaattggt agctcttcct gaaggtggcc tgggaccctt agggtaggtc 17580
   tggtcccagg tgagctcctt ggccaacagc ctctttgcca ccccacttca gtcccaggct 17640
   tattcttggc aggcaggcca ctgggccctg gaggagagga cccagccagg ggcccttggt 17700
   ggacaggaag ggaggaagcc ctccctcttc cagagttact ttctgtcctg tcttctaagc 17760
   ccctttccat ccacgtgtct gtctttccgt tgctcatagt gtctgtcttt ccgttgccca 17820
   tagtgtctgt ctttccgttg ccttgtgagg ttggtgaggc agagcagccc cagcaggggg 17880
   tgggggttgc tgcctgctcc agggctgtct ggctccggag ggctgcctcc gacgtgactg 17940
   gggctttatc ttccactcct cctcctcctc caggtggaca tcgagcagct ggatccccgc 18000
   ggccggactc ccctgcacct ggccaccacg ctggggcacc ttgagtgtgc ccgtgtgctc 18060
   ctggcgcacg gcgcagacgt gggcagggag aatcgcagcg gctggacagg tgggcagccc 18120
   tgctcacccc agccccacag ccggggcctc cccagcatca tagacctatg gttggctgaa 18180
   gggtcctgcc ctgtgacctt gcctcgccct cccccagtgc tccaggaggc tgtgagtacc 18240
   cgggacctgg agctggtgca gctggtgctt cggtaccggg actaccagcg ggtggtgaag 18300
   cggctggcgg gcatccccgt gctcctggag aagctgcgca aggtgaggcc cagcctctca 18360
   gcctccacgg gagcccttga gcccttctcc agtgcagact tagcctctct ccccttcgtc 18420
   ctccaggccc aggacttcta cgtggagatg aaatgggagt tcactagctg gggtaagcgg 18480
   gctgcagcag gtcgcttctg ggctctccca ctttaggtcc tgcgcttgct cccctgcctg 18540
   gaatgtgttc tcatagttct tccggcggtt ccctctatgc cttagctcag ctcagggcca 18600
   ccgcctcagc taggcctttc cagattgccc cagatactgt cttgactcca ttattattct 18660
   cttcactctg tcatgatttg cttactgtat tcatgacctg ccgctccttg ttagaaggta 18720
   agctctgaga gggcaggagt cctgtctttt tcactgttgt attcccagtg gctggaacac 18780
   tcagtaggtg tttcataaat atttgttgaa agaatggatg aaagagtgag tgagtgaatt 18840
   aacaaatgat ggacactagg gacttagtgg tgacagaaac atgcccgtcc cgacctcagg 18900
   ttgctcttct gtgtgagtat ggtctacact ggccagggag ggggttttgg aggagaggct 18960
   gctctctctt cagttccctc ccctgttcct ggccacacgg atcccaaacc ccatgccctg 19020
   agctggtcca ggccgtgtag gccagaccag tcaaagccac ctccaacctc cgcttccacc 19080
   agtgcccctg gtgtccaaga tctgccctag tgacacctac aaagtgtgga agagcggcca 19140
   gaacctgagg gtagacacca cactcctggg ctttgaccac atgacctggc agcgagggaa 19200
   ccgcagcttt gtcttcaggg gccaaggtca ggcaggcagg aagtggggca gggtggggac 19260
   gatgggaggc agccccaggc cacccctgat ccccctgtgc tgttccgtgt ctggcagaca 19320
   caagcgccgt ggtcatggag attgaccacg accgccgggt ggtgtacaca gagactctgg 19380
   cactggctgg gcaggaccgg gagctgctgc tggctgctgc tcagcccact gaggaacagg 19440
   tgctgagccg gcttaccgcg cccgtcgtca ccactcagct tgacaccaag aatatctcct 19500
   ttgagaggtg ggtggacatg gccttggtca cccttgagcc agcccggtgg ggtgcctgcc 19560
   tcacctggac cactctgctt ccccaggaac aagactggca tcctgggctg gcgcagtgaa 19620
   aagacggaga tggtgaatgg gtatgaagct aaggtgaggc tgcagctccc agctgccagc 19680
   ccagctgcac tcttgcctac agcaagctgt acaggggatt ctgacctccc ttccccagcc 19740
   ctggaggtac agaagcaatg cagcgtggtc aagcacttat atttgggttc aaggcacttc 19800
   tctggtgttt tatatggatg tatggatgta tacacagggc acgtgcacac gcacacacac 19860
   acacacattt attcctcaca gcaacccttt ctggtaggtg ctgttctgtt agtattccca 19920
   ttttacgcat gtttattgag tcctttcctt ggcatcccat ttactcttca caacaacccc 19980
   aggaagtagg gatgatcggt ccattttatg atgaggaaat agaggcttag agtggttctg 20040
   tgacctgtac aaggtcacac agctgttaag tggcagagtt ggggttcaga tcctggctgg 20100
   cctgacccta gagccaggcc tttatcccta aaaatgactg cctggctggg catggtggct 20160
   catgcctgta atcccagcgc tttgggaggc cgaggtgagc ggatcacctg aggtcagttc 20220
   gagaccagcc tggctaacat ggtgaaaccc cgtctctact aacaatacaa aaaattagcc 20280
   gggtgtggta gcaggcgcct gtaatcccag ctactcggga ggctgaggca ggagaattgc 20340
   ttgaacctgg gaggcggagc ttgcagtgtg cagcgatccc gccacagcac tccagcctgg 20400
   gcaacagagc gagactccat ctaaaaaaaa aaaaaaaaaa aggctctgcc tgttactctg 20460
   ggacccctat gttgcccacg acatgaaagc aggaactaac aaatgccatg ggttttgtgt 20520
   gtggcatctc ctctgaaagc ctcccctgct tctgtagtct gggaaggcct ctgtgctacc 20580
   cccaccagtg catggaccat gctgttataa ctccatttta ttgtctcctt atagagattg 20640
   taagtctcag gagggcaggg atcatgtttg gcttgtttac tgtatccctg atgcctacct 20700
   caatgcctgg cacataaagg ctcctcaaaa aatgtttgtt gaatgaataa gtgaatgaat 20760
   gaatgaatga atgacctcat atattcactt acccaaaagc ctgaagcaga gtctctccag 20820
   gaccctttgc tacagggagt gttcccagtg cccttagggg taagacacag ggtaagacca 20880
   gaactgctta tgagcccttt cttcatctgt ccccaggtgt atggggcatc taacgtggag 20940
   ctcatcaccc gcacacggac agaacatctt tcagaacagc acaagggcaa ggtcaaaggt 21000
   aatgaggcag agctggatgg ggagaggtgt tgcaggactg cgggaaccag ctcccacttc 21060
   agtgcttcca tcagtattcc cactggcatc ctagcaagag gctggatgct ttattcaggc 21120
   cgatagattt caattcaggt gccaagccct aaggatgggt agtggctgcc tagagtacta 21180
   agaagtttct aaggactcat ctgggttcag tggagagcca tgctgcaagt gattactaat 21240
   gttgctgttg aatttgggag aggagagaaa tgccatgtat gccatttatt ggccattcta 21300
   gtctagtggg acatatccta ggtagaaagt caggggacct tgccacagat tctgtatgcc 21360
   cattgatgcc acacctgatt ctctgggtac cagataccgt gtccgaggtg aaaaagcata 21420
   atttctacct cacaggggaa caacatgaaa tcatgcctat ggaagtgcct gaagcaaaca 21480
   cacacacgca catgcacacc ccacagtgcc tgccggggca ggcgttttac tgtccagggt 21540
   caccgagctg gagaggagca cttttggaag ttgcttctga cctctaggcc ttccttttag 21600
   tgttttaagc tgtgctcaca gaggccctgg cccctggtcc ttgaagcact cagtcccctt 21660
   caggtgaagg tgcctgagta tgtggttggg tgagcaggcc ccaccccagg aaccggcctc 21720
   ctccctaggt cttgggtgct gtcacctctg atgaggtgcc caggtgtgtg tggagttgcg 21780
   gtgtcctctg agatgccaca tttcttgtag gctgtaagac acctttgcag tccttcctgg 21840
   gaatcgctga gcagcacggg ggcccccaaa atggggtgag tgtgtgcagg ggtacccgta 21900
   agtggaggga tgtggatgtg gctcaggagg aggcttggga agcgtcctgc ttagcatggc 21960
   ctatgtggac ctcctttcca caatgcccaa ggccatgtca gcgccacact ggcagagggg 22020
   acagcttggg ggccttgtga caacaaatga gttggcctgg cattaggaac tgaggggagc 22080
   tcctgggccc ctccccatgt ggtacagacc ctgatcactc agactctgag ccaagccaac 22140
   cccactgcca tcactgcaga agaatacttc aaccccaact ttgagctggg caaccgtgat 22200
   atgggccgcc ccatggaact gaccaccaag acacagaagt gaggcccctg ccggtgctgg 22260
   gaaggtgggg ggccggggct ccaggagatg ctgggaggcc atggcttcct gcagtgtccc 22320
   ttaccccttg ggatctggtg ggggcaggtt caaggccaag ctgtggctgt gtgaggagca 22380
   tcccctgtcc ctgtgtgagc aggtggcccc catcattgac ctcatggccg tcagcaatgc 22440
   gctttttgcc aagctccggg acttcatcac cctgcgtctg cctcctggct tcccagttaa 22500
   gattggtgag accgcacggc catttctcct gagcccgtgg cgggccgacc ggggtttaga 22560
   tgaggtcggg gtggggctga ggctgaggtg tgaggggctg agtggtggcg cctaaaggtt 22620
   tcctcatcct cagaaatccc gatcttccac atcctcaacg cccgcatcac cttcgggaac 22680
   ctcaacggct gcgacgaacc ggtgccatcg gtgcgaggca gccccagcag cgagacgcct 22740
   tccccaggca gcgactcctc cagcgtcagc agctccagct ccacgagtga ggccccccgc 22800
   gagaacgcct gcccctcggc tctccccggg gtgggtggga ggggcgcgcc cggccgtgcc 22860
   tgacccagcc cccgcgcccc cagcctcctg ccgcggctgc gagatctccc cagcgttgtt 22920
   cgaggccccg cgcggctaca gcatgatggg cggccagcgg gaggcggcga cccgggacga 22980
   cgacgacgac ctgctgcaat tcgccatcca gcagagcctg cttgaggcgg gcagtgagta 23040
   tgaccaggtg cgtctccgcg ggcgcgcggg gccacgggac tccgcgccgc cacggctaac 23100
   gcccacgcct ccccgcaggt caccatctgg gaggcgctaa ccaacagcaa gccaggcacc 23160
   caccccatgt cctacgaggg tcgccgacag gacaggtcag tgcccgctgg gccggagaga 23220
   atcctccgga gaggatcctg tctcccctaa gccaggacac agccggagaa cccgcggggc 23280
   cctcggcagg gaccctcctg gtatcgggat ggcttctgcc accactcctg cctccgaggc 23340
   ggcaggcagg ggtcagggat ccaggcttca ccgcggagtt cagactctcc ccaggcattg 23400
   acggggagaa cttggtaggg tctgggctcc gccacgacca ggtgggggcc aggacgccgc 23460
   gggactgacc gcctccacta gagggtggtg gccgcgggcc gccctggagc ctccacgccg 23520
   tgtcccggcc ccggccggtg ggtgggtgcg cccgggagcc cgcgacattc cttccccacc 23580
   cccaggagcg ccccgcccac gccgcagcgc cagcctgcgc ccccggcgtc agtgcccagc 23640
   cctcggccca gctcagggcc aggttccggc ggccacgtgt tccggagcta cgacgagcag 23700
   ctgcggctgg cgatggaact gtcggcgcag gagcaggagg agaggcggcg gcgcgcgcgc 23760
   caggaggagg aggagctgga gcgcatcctg aggctctcac tgaccgagca gtagcgcccc 23820
   ctgccgggac cctcgccagc gccacgcgcg ccacgcccag ggccaggagc cagacaaacc 23880
   ccggcctgcg cgcctgcaga gcggcggctg gagactggag ccaccgcctc gcgggtgcag 23940
   cagcacagca ggcacggttc ggggagggat tcggcatggc cgcggggtac cttcccaggc 24000
   cagggccctg gaggcaactg gcacggcctg gtccccctgc tttgctgtat tctgattccc 24060
   caacccgctc ccctgggctc agatctgtcc tgtcctaggg cggagccagg cggtcctgag 24120
   ggggagatga atccttagag gagcgctgtc cctatccctt gctccttctg gccggtccgc 24180
   atttctgttc actaacccca ctccaggaca ctgcccctga agcctttgca tctctgctct 24240
   tcactcctgg ggggcagctg agctccccgc gtgctgtgtc actgctttgt tccagacaca 24300
   aaccagcacg tctagggccc agcccctccc ccaccccggc atttcagcgt caagtgcact 24360
   tagcggggta cccggctccc ccagccccca cacccgtcct gggtctcagg gtggttccag 24420
   cttctccttg ggcagccaga agttggagtc ccatccccca aggcacattt tttttttttt 24480
   ttttttgtga tcagggagtg tgtccaacgt agccccctgg cctgtgcaag ccctgactcc 24540
   ctcatggtgc ctcggagagt ggggagcata ttgggctggg gtaagcacta gacccaagta 24600
   gactggacac aaagggctcg cccagggccc tggcgccacc cccacccctt cccaccagct 24660
   gctgctagcc tctgtggttg tacatcccac ttgcccccac acggagactg actctaaaac 24720
   ccttcatcca atggtgctaa cccccggctc tcccctgccc cacctcaccc acccagagaa 24780
   gcacagaccc cgccaggggc aggggcccac cgcacaccct tgtcccgggc ctgtctggga 24840
   ctggccttcc cggctcagcc agtgaggctc agaagggaca caaagaggga tggaagaaaa 24900
   gaacaaagag aaactgttcc tcccaccccc ttccctgatg ccaggggcac cagactgatt 24960
   ctgaggcaca aataaaagag gcttcatacc gga 24993
<210> 7
   <211> 24993
   <212> DNA
   <213> Artificial Sequence
<220>
   <223> C to T (bisl)
<400> 7
   gtttaaaggg gtcgcggtag atgttcggtt tcggcgggta gcgtggtttg cggttttggt      60
   tttcgttgta gcgttcgttt ggttcggagg taatgtgagt tatttttata gttttttttt     120
   ttttattttt cgcgtggggt ggatcggggg acgttgggtt tacggacggc gagggtgggg     180
   attagggaag aaggatttgg aggtttaagt ttcgttttag gttaaagggg ttgtgttttg     240
   gggtattcgt ttatttttgg ttggggtgag gtgtgcgttt ttgtttcgta ggtgtttata     300
   tttgtgtgtt taggggtgtg tgtgtatgtg ttatattttt gttgaatacg tttattgtta     360
   ataatatgga cgtttaggtg ttagaaaggt taggtagtgt atttttttgt tttcggatta     420
   aaaatatatt tattttttta tgtatttttt tatttagaga ggagagaatt tgggcggttt     480
   ttaaagattt ttcgaggagt gattattgtg tttgtgtgta tttatattta tttgttattc     540
   gttttatata tatttacggt gtttttgggt tattagaggt gtgtgtgttt agaaagggtg     600
   tatataatat agaggggtcg ttttaggtga cggttttttt ttttggattt tatacgacga     660
   ggtaggtaaa ggtaaggtag gtgagggacg tgggtgggtt ggggggtagg gagtttaggt     720
   agaggtcgta tatgagcgta gttttcgtat tgagtatgcg tttttatacg cgtgtgtcgc     780
   gcgtgtattt aatttttaat ttttgtagta gtaaaaggtt cgggtatttt gtttgtgggg     840
   agagggtaga ggtttttagg gttagatttg ggggttaggt tagtggtgat aattgaggag     900
   gggtttttta agagttagag atttttgttt gttaagatgg gggtagtttt ttggtagggg     960
   tgtttaagtt ggtcgattat tttaacggtg ttagagcggt agtgatttgt gggtgggtag    1020
   gttttttttt taggggagat aagtaatgtt tatgagttat attattcgaa atatatgtaa    1080
   agttgttttt atcgtttttt gttgcggttt agattgataa gtttgtttga aatcgcgatt    1140
   tatgggtttt atttataatt gtattttaaa aaaattttgt aggttttttt tttttcgata    1200
   gttagtcgtt tagttggtgg ggtttttgta agtaacgata tttagttggt gtgtaggggg    1260
   gagtattatt tgtcgtttcg agttgttttt ttattagagt ttatgggttt ggtggtttta    1320
   gcgggaaggg ggtaaggggc ggggggagtg agacgggagg agtggttagg tagttcgtgt    1380
   taggcgttta gtttacgagc ggtggtcgtt cgtttatttt cgatcgattt ttagttatgg    1440
   aattttattt attaaatgtc gaggaggttg ttggtagttt tgtcgtttgg ttgtttttgt    1500
   tttttcgttg tgtgggcgcg tttcgagtac gtggtagttg agagcgcggg tcgaagcgta    1560
   agcgatttgg gtttagtgtt tatcgtcgtt tggtaaaaaa agaatacggt cgtttagggg 1620
   atagtttttt ataatcgata attttttata atttattttg aatttttttt gttttcggat 1680
   ttttgggttt tgttggggaa gaaggatgat ttattttgtt tattttttta ttttattttt 1740
   tattttttgg ttggtttaga gattatttgg gagtggtttt ggcgcggata tatcgtttaa 1800
   attttgtttt ttttagtttc gttatagtta aatttttggg tttgggattg gagataaaag 1860
   ggtttttgga ttttttcgtg gagatgttat tattttagga agtaggattg ttatagtgga 1920
   attatatata aggttcgaaa aataatttaa taaggaaatg tataggtatg ttttcggtta 1980
   tttttagatg ttttttaatc gcgttagttt ttttttttgt attggttgta gttacgatat 2040
   tttgttgatt tttggtggtg taggcgggta gcggtttcgg gttttcggag acgttagttg 2100
   gtttttttta ggcgggagaa aggggcgagg tagttcgttt tttttttatt ttttttcgtt 2160
   ggggtttggt attatagggt gtggaggagg aaggagggaa gattatataa agaaatttga 2220
   gagagtttgt aagtttttgt tggtattttt tatataaata ttttttggat tgttgtttta 2280
   agtagggtcg gtgtggaatt ttttattttt gttttgtttt gaggtgggtt taagaaggta 2340
   tcgtgtttgg tttcgttatt tgtttcggtt cgtttggttg ggggagggag gcgttgtttt 2400
   tagggtagtt attattttta gtttatttta ggaggtagta tttagatttt tattttgaag 2460
   gggagtggat cgagaaagtc ggaaaaatta agtagtttgt ttttttaagg ttaggtagtt 2520
   ggagttattc gaattttggt ttttttgata ttatagtttt ttttttttag ggattggttt 2580
   aagttttttg gtttttttag gttttgtaat tggcgtttga gaggttaatt taatttttat 2640
   tatatttttt gtagggatta ttttggaagt atttttttat tttggggtta tttaggaagg 2700
   agatagtttt tggaattagt tatttttttt tttgaagggt tagttgggtg gaggaacgga 2760
   gttgttattt gttgtttttt ttgtttaatt ttattttggt ttttgttggg gatagtgtgc 2820
   ggggggaaag ggattatttt tttcgcgtta ttggatattt tgtttttaat gatatgtgtg 2880
   gaagatggag tttaattttt taatagggta gggaagattt tttagaaatg ttttaggggt 2940
   tagatttttg gttgttttaa tgatttttgg tattgatgtt ttatattttt tttaggagtt 3000
   tttaattaga cgtttttttt tgggaattcg gatttttttt tatggttttt gggatttaag 3060
   tttaattttg aagattatat ttttatttcg ttttttaaac gttcgattta ggttcgtgtt 3120
   tttttatttt tttagtaaaa ggtaaaatgt tagcggggga ggtgcgtttt agtttatttg 3180
   gcgttagggt ttcgttcgcg tttgggttag gtttgatttt tcgtttattt ttttacgaga 3240
   ttcggggata gtgtagattt ttttcggtta tttgttattt ttagttttac gaggcggatt 3300
   ataaatttta tgatgtcgcg gggttgagtt gataagtttt attcggggcg tttggttttt 3360
   tttttttttt tttattattt ttatattttc gtatttcgcg tcggtcgttc gggttgaggg 3420
   tcgggttatg tagttgtggg gattcggggg ttgcgggcgc gcgtttttgc ggcggcgttt 3480
   tcggggttcg cgtttcgtgc gttttcgcgt tcgttgcggg cgtttgtttt tttcgtcgag 3540
   cggcgttttt gtgtgcgggg gtgtgggagg cgagcgcgag ttcgcgtagc gtcgtcgagt 3600
   gttcgttttt ttttagggcg ggtgggggtt ttttcgcgtc gttcgtcgtc gtcgtttcgc 3660
   gaggacgttc gtcgttcgcg tcgttcgtat cgcgtcgggc gcgtcgtttt cgttcgtcgt 3720
   cgttcgtata tgttcgagtc gtagtttcgc gagtaggtag cgtcggtttt tcgtttcgcg 3780
   gtttcgggtt tcggtttcgg cgtcgttttt ttttttcggt cgggcgtcgc ggtttcggta 3840
   tgaggagcgg gcgatgattt tcgttaacgt tttcgttagg aaggggttcg agggtaagta 3900
   ttcgttgtat tatttcgtgt ggtataatcg ttatcgcgag ttggagaagg aggttcgcgc 3960
   gggttaggta ggagcgtttt cggggcgtcg cggggatttc gcggtcgggt acgttcgttc 4020
   ggtttgggga gggggtgcgg cgcgggttgt ttcgtttttt tggcgagttt gcggcgcgtt 4080
   tttgtttgcg gtcgcggttt ttgtgtttgt tgatcgggaa agggtgattt ttttcggtcg 4140
   ggacgggcgg gattatacgt gtttcggatt tatttagatg agagtagagg tttttgtgtt 4200
   tttttttcgt cggggtagtg agggttgtag ggaggggttg cgggttgtgt cggtgtttta 4260
   tgtggtggga gagttcgttt tagttagttg agcggcgata tttttcgagt gagggtgcgg 4320
   aggtgggaaa ggtttgcgtg gggagggggt tataggttaa gaatggggaa gaggttgtgt 4380
   tatattttta ggtattttag ttttatttag cgtttttttt ttaacggtga gcgttttttt 4440
   ttttagtttt taaattgggt agatttttgg attttttaga gtttagtttt agtttaggaa 4500
   tagggagtgg agaaagattt ttttgggagg ttttaagggt tttgtttagg tgttgaaaag 4560
   cgtttttttg ttttttggtg ttggggtttg gtttttaggc gtagaggaag ttgaggggtt 4620
   ttggttgttg gaaggatttg aggttttgcg tattaggtgg gttttttggt ttttgttggt 4680
   tagtaaggtt acgtgggtta ttagttttgg gttgggtagt ggtttttaga aaatagggga 4740
   aggggggatg ttttttggtt atgttttagg atgttttttg gttatgtttt agaataaagg 4800
   taggtatagg ttatagattg ttatttttgt ttttggtatt taggttatag gtaggtattt 4860
   atggtagaga tgatttttcg tttttagggt gggttgtaag agttagagtt gggttcgggt 4920
   tttatggttt gttgtaggtt gagaatttta gttttggatt gggtttgaga ttttggggtt 4980
   atgggttggg ggtggttagc gtagttgttc gttgtgggaa tttatttttt gagggttgtt 5040
   tattgagtta tgagaagttt agtttgagga gttgtgtaat cgttaaggga agtatggagg 5100
   tggagggtag gttatcggat tgagtaggtt gggggttagg atgtgttttt gaagttttac 5160
   gttgagttag tttttaggtt tgtatggtat ttagttttgt atggtagttt ttgtaaatat 5220
   gcgagtgatt tgtgttcgta ggtttttggg tcggtttggg tggttatttt ttgttatttg 5280
   tgtttatgtt agtatttttc gagttttttt ttagattgaa ttttttagat tttgtttttg 5340
   tttatttatt ataaaggtag tgttttgtag ttacgagaag tttgttaaag agagtgggtt 5400
   atagtgtttt taggaaatag atgaataaat agggtattaa tttttgtttt taaggagttt 5460
   ttgttttggt gaagtaagtg tgaataggaa attttagggt gatgtgtaaa gcgttaagat 5520
   atggttagtt agtttttgtt ggtttttttt tgtttttttt ttttgaaagt aaaagaagat 5580
   aagttttatg tgggaagaat tttttagggg tttaaggatt attaggtaga aggttgaatt 5640
   tttagaatgt tttagtttta tttttttgtt tgagggtggg tggttttttt attgtagaga 5700
   ttggagagag gtagaatgtt ttgggagaaa agattttttt agtaggtttt tttgttgata 5760
   attttgaagt gggtggttat tttgttgggg taaggttggg gtggggggta tcggagtcgt 5820
   tgtaggtaga ggtgttgggg gttgtttatg tgtttttttt ttttattttt ttggttgttt 5880
   tagaaataag gttttagatt tatttttatt tttgttttag tgagttaatg tttttttgaa 5940
   atggatattg tttgttttgg atttaggtta gggggaatat agggtattgg tggaggttaa 6000
   tttttgttta attatagagg agttaggtta gttttttttt ttttttaggc gtgttaaaaa 6060
   gaggaatgtt atttcgtgta ggtgggagag ttttttagtt gttttttttt tcgaggattg 6120
   gtttagagtg aggtttgtat ttagatgtat attgtttatt ttttagggta ttaggttttg 6180
   ttttttgtaa gggggatttg gggatagtta tttaatatta tgagaaaagt attgtggaat 6240
   tttggtattt tagttagggg tttttggttt ttagtttttt tggtattagt cgttatagaa 6300
   atagttcggt tttttttaag tatttaggag ttaggttttt ttggttttgg tttggtatag 6360
   ttgttttttt tttattttaa gaaagtttcg tagaggtttt tcgtgggtag gtttggtagt 6420
   gtagttggtt gaggaggggg ttagtatggt tatttttaga gtaggttaat gggttttgtt 6480
   tagttttaaa ttgttttgtt ggaggttgga agtagggagt gatgggtaga taggaggtgt 6540
   ttggtgaagg gaaggggtat ttagtagtgt tgtgatgtta gttcggtgtt ttgtttgtcg 6600
   ttatgtatgt tttttgttag gtcggggggg atagggttgt aagttaggga atatgttagt 6660
   ttttttttgt ttggtatggt tagttgggta tggttttggt tttggagtcg tgagtttttg 6720
   gtgttttttg gggtgggaat agggggaagg gttagtttgt gtagttttcg tgggagggat 6780
   atgtgtgggt tgggtttggg gagtaggatt ttgtttttgg taattagaat tttgaagtgg 6840
   gtagattgtg gttttagaga attatttttt ttttttttgg ggcgtttttt atcgtgggtt 6900
   tgttttttgt tttttttgtt tatttttttg atttagaggt aggggagatg ggttgtgggg 6960
   tttaggatgt gcgaggagta ttttagattt ttttttttgt ttatattggg tagttttgga 7020
   tgtggggtat tattatagtt ggtaagttag gaagttcggg agagggagtt ataaatttta 7080
   gaaattaaaa attaatgtgt tttgggatga gggggagggg ttgtaagggt aggatgggag 7140
   gggtgatatg gagggagaag tttagggtgc ggtttttata gttcgtggtt ttttacgtag 7200
   tttgaggatg tattttgttt tttttttttt gaatgagttt aggtagattt gtttttgtat 7260
   tttacgttgg gtaggagagg ttaagtaacg attatattag atttttgttt tttttttcgt 7320
   gggttgggga aggggttgag taggtattat tttgagtata ggttttgcgt tttttatttt 7380
   tttcgtaagt attgtgttta gtttatatat gagatgtatg ttttttatat tttttttaat 7440
   aaaaggattt tgtttatgtt aaaggagttt ggaaagtatt ttgttagtgt atatgggtgt 7500
   gaggggtgtt tttgtgtaaa tacgtatata tacgttatat aggagtggat ttttgtaggg 7560
   ttagaaggta gttgggtatg tatgtgtttg tatatttaag ggtatacgtg agtatttatg 7620
   agcgtacgag tgtgtatttg tatggtatta gtgtgtatta gagtgtggtg tgtgtgtttg 7680
   agtggaggta gggaggtttt ttgtttaggg atttttagtt ataaaatttt aaagatttta 7740
   ttttttgatt ttagttgagg tttgttggga taaggaattt tcgttttttt ggtggggagg 7800
   gttgggtgtt gggttttatg ggaaattttt ttattttagt aattaaatag ttagaggttt 7860
   ttgttgagga gaagttggag gtttttgttg aggagattaa tgaatttttt tatttgggtt 7920
   tagggtaagg tgttatttat attgtaggcg gtattttggg tttgtgggga gagttgtttt 7980
   ggagtttggt tgagtgtatg gggttttgta tggggtgtat ggggtgttga gttatttagg 8040
   tttttttggg gagtgttttt gaggtgtggg gatggggagg agtattttgt ttgtaaggaa 8100
   gtgttagtcg ttttttggag tttggagttg aggtatttga tggggaggtg gaggtagtat 8160
   ttggagtttt gaggttaggt agcggtttgt tagtttttta gggttttatt agttgtagcg 8220
   gttgcggttg tagtatttgg ggtagggttg ggaggtttgg attgtttagt atttttttag 8280
   ttagttcggg ttttgtggtt tttttttatt ttcgttgtcg ggtttaggat tttatttagg 8340
   ttgtaagttt cggagtttag tttaattagg attggataag agtaaataat attttttttt 8400
   tttgtgaggg gttatagttt agaggttaat aggggttagg ttcgttggtt tttttgaggt 8460
   ttttagtata ttgaaaaatg aagaaatttt aggataaggt tataaaaatt atataaataa 8520
   ttttaaatta tttaaaatgt tttatttttg ataatggagt ttatggttat gttttaaaat 8580
   atttaattgt tgttagaaga tgcgtaatga aaagtagtgt tttttgtttc gtttttttta 8640
   tttttttagt attttggatt ttttttgtta tttatttttt taattataaa taatatattg 8700
   tttatattgt tttttttttt tttttttttt tttttttttt tttttttttg tttttttttt 8760
   tttttttttt tttttttttt tttttttttt ttttttatga gatagcgttt tattttgtta 8820
   tttaggttgg agtgtagtag tgtgattata gtttagtgta gtttttgttt tttaggttta 8880
   ggtgattttt ttattttagt tttttaagga gttaggatta taggtggtgt tattatgttt 8940
   ggttaatttt tgtatttttt tttgtagaga tggggttttg ttatgttgtt taggttggtt 9000
   tcgaattttt gagtttaagt gatttattta ttttggtttt ttaaagtgtt gggattatag 9060
   acgtgagttt ttcggtataa ataatatatt tttgtaatga ttttttggtt aattattttt 9120
   ttttttttgt gtttttttag tttagtattt tatgtatttg tttagtaaat atttatgtgt 9180
   taggtgttgt ttggggagtg gggaattagt agtgaataaa gtaggtaggg atttggttga 9240
   tggggttttt gttcgaggtg taggaggtgt ttaataggta tttggacggg tagtggatag 9300
   agttatagta gttgttattt tagatgggtt gttagggtgg gtttttttaa ggaggtgata 9360
   tttgagtagt agtttgagtg aagggaggga gaaagttttg gggttttttg gaggattttg 9420
   gagaacgttt cggggaaggt tattggcgaa gggtggagta aatgagttta aagagaagtt 9480
   tggggttatt ggattttttg ttatttacga tggaaagtta ttggaagatt ttgaattaaa 9540
   gagtgttagg atttgagtaa tattttatag ggtgatttta gttgttatag gtgaggtgga 9600
   taatagagtt tggtaatcgg ggagtagtaa agaggtagta gggggttgtt agtgagagat 9660
   gggggggtaa tagttgtaag gtagtggaaa ttgggcggtt tttaggtttg ttttggaggt 9720
   agagtttata agtttttttg atgggttaga tgtggagggt gcgggaaaaa gaagagatgt 9780
   agatagtttt aaagttggtt ttttttttta tttgaaaatg tagtaaattt aggtgatgtt 9840
   tattgtttat gggggtaggt gtagttgttt aggaatgtag aaaataagga aaagttaaga 9900
   aagttatttt tagaatgaag aaggtaagag aaagggtagg agacgttatg gaaagttagg 9960
   ttgttttatt ttgttttatg aatttaatga tttttttata ttttttttga ggatgtaaat 10020
   tattgttttt ttttaaagag ttttttttta ttttttgtat taagagaatt ttttttgggt 10080
   ttttagtatt taatttgttt tttttttttt tttttttttt ttttttgata cggagtttcg 10140
   ttttgttttt cgcgttagag tgtagtggcg taatttcggt ttattgtaag tttcgttttt 10200
   cgggtttata ttattttttt gttttagttt ttcgagtagt tgtgattata ggcgttcgtt 10260
   attacgttta gttaattttt tgtattttta gtagagacgg ggttttatcg tgttagttag 10320
   gatggtttcg attttttgat ttcgtgattc gtttatttcg gttttttaaa gagttgggat 10380
   tataggcgtg agttattgta tttagtcgtt ttagtattta atttgtatgt tttggtttat 10440
   tttatatggg aagtttttat taagtgttta gttttttttg gttgttgggt tatatttaag 10500
   aatggggttg gggagtttag ggatgtgtgg gtgttattgt aaggtgatta ggtggtaggt 10560
   tggtttgttt attgtagggt tattagatgt ttgtattttg aggtttttgg gataattttt 10620
   tattcgggta gatagggtgg tggtggtgtg atttggttgt tagtggttta ggatttaggt 10680
   agggaagggg ttggggtttt acggattatt ggtttttagt attatatttt attttttgtt 10740
   gtggttaatg tttttgagtt tagagtattt tatgtttaat ttttataaag aataaatttt 10800
   tggtgttttg tggtgaggaa ggggggtatt ttttagttta atgaggttgg gaaggggatt 10860
   aggggatttt gaattgtaat tagttttttt gtatttagtt ttagttttat ttttattttt 10920
   gttattttta ttgtttgtag gttttattta tattttttag gggggttggg ggttatttgg 10980
   tttgtttttt atgtggacgt ttttttttgt ggttgtttta gttttagttt tttttgtttt 11040
   tttaagttag ttgtgaggtt ttagttattt tttatgtttt aaagttgtgt tgaagttttt 11100
   tatttgttga tatttttttt ttttattttt gtgggtttta aatattttat tgattttttt 11160
   tttttttttt ttttgagata gggtttggtt ttgttgttta ggttggagtg tagtgggatg 11220
   atttttgttt attgtaattt ttggtttttt ggtttaaggg atttttttat tttagttttt 11280
   tagtagttgg gacgataggt atgtattatt acgtttggtt aattttagta tttttttgta 11340
   gtatagagtt ttattatatt gtttacgttg gttttaaatt tttgggttta agtgatttgt 11400
   ttatttcggt tttttaaagt ttataggtat gagttattgc gtttagtttg atattttttt 11460
   tttttttttt tgagatagag tttttttttg ttatttaggt tggagtgtag tggtgcgatt 11520
   tttgtttatt gtaattttta ttttttaggt ttaagcgatt tttgtgtttt agttttttta 11580
   gtagttggga ttataggaat tttttattac gtttagttaa tttttgtatt tttagtagag 11640
   atggggtttt attatgttgg ttaggttggt tttaaatttt tgatttttgg tgatttgttc 11700
   gtttcggttt tttaaagtgt tgggattata ggtatgagtt attgtgtttg gttttttgat 11760
   atttttatat atttaatatt ttttggtatt ttttaatttt ttgtatagat ttaagttttt 11820
   atttgggatt attttttttt tatttgaaga atttttttta atattttttg tatatttggt 11880
   aataaatttt tttagaattt gtttgtttgt taatatattt attttttttt tagttttgaa 11940
   gaatattttt atcgggtatg gaattttagg ttaatattag ttatattgga ttagagaaaa 12000
   aaaaaaaaag aattttaggt tgaaagtttt tgtttttgtt tttttttttt agtattttaa 12060
   agattttatt atattgtttt ttggtttata tagtttttga tgagaagaat atagtatttt 12120
   ttgtatttgt ttttttatat ataatggatt ttattttttt gaatgttttt aggatttttt 12180
   ttttattatt agtttttagt atttgattat gatggatttt ggtgtagttt atttcgtgtt 12240
   tgttttattt ggggtttatt gaattttttg gtttttggtt ttattttggt attagttttt 12300
   ataataaatt attataaatt aggtgggtta aaataataaa tatttggtcg ggtgtggtgg 12360
   tttacgtttg taattttagt attttgggag gtcgaggcgg gcggattacg aggttaggag 12420
   atcgagatta tgttggttaa tacggtgaaa tttcgttttt attaaaaata taaaaaatta 12480
   gtcgggcgtg gtggtgggtg tttgtaattt tagttatttg ggaggttgag gtaggagaat 12540
   ggcgtgaatc ggggaagcgg agtttatagt gagttgagat tacgttattg tattttagtt 12600
   tgggcgatag agtgagattt tattttaaaa aaaaaaaaaa aaaatttatt ttgttatagt 12660
   tcgggagata taaatataaa tttgagatgt tggtagggtg gtagtttttt taaaggtttt 12720
   agggaagagt tttgttttgt tttttttaga ttttggtgat tgggttataa tttttttggt 12780
   ttgtggttgt agaattttag tttttgtttt cgtttttata tggttttttt atttttttga 12840
   gtgttttaga aagatatttg ttattggatt tagggttttt tgggtgattt aggattattt 12900
   tattttgaga tttttaattt aattatgttt gtaaagattt ttttttgaaa taaggtttta 12960
   tttataggtt gtgagggtta gaatgtgatg tggatatgtt ttaggagggg tattattttt 13020
   taatttatta tagtagtttt tattaagttt ggaaaaattt tggacgtttt tttttttttt 13080
   tattatttat ttatttattt tgaaatggag tttttttttg tcgtttaagt tggagtgtag 13140
   tgatacgatt tcggtttatt gtaatttttt tttttcgggt ttaagcgatt tttttgtttt 13200
   agtttttaag tagttgggat tataggtgtt tattattacg tttggttaat ttttgtattt 13260
   ttagtaaaaa tggggtttta ttatattggt taggttggtt ttaaattttt gatgttgtga 13320
   tttattcgtt ttggtttttt aaagtattgg gattataggc gtgagttatc gtattcggtt 13380
   tattttttta aatttgtttt atttgtttta tttttttatt ttgttttttg ggattttaat 13440
   agatatatgt tataatgttt gataatgttt tatagattat gaggttttat agagataggg 13500
   ttttgttttg ttgtttaggt tggagtgtag tggcgtgatt atagtttatt gtagtttcga 13560
   atttttgggt ttaagggatt tttttgtttt agttttagag gagttgggat tatagttgtt 13620
   tgttattatg tttagttaat gtaaaagttg ttttgttttg ttttgttttg tttgtagaga 13680
   tagagttttg ttgtattgtt tgggttggtt ttaaattttt gaatttaagt taatttttcg 13740
   ttttggtttt taaaagtgtt gggattatag aagtttagtt aattttattt tttagtattg 13800
   tttttttttt ttgtgtttta gtttggaaag ttttattgtt gtttttaaat ttattgattt 13860
   ttaaattgtg tagtgtttaa tttgttgtta tttttattta gtgaattttt tattttagtt 13920
   attgtattta ttatttttag atggtttatt gggttttttt ttatattttt tatttttttt 13980
   tttatgaata ttttttttat gttttttttt aaatttttga gtatattgag ttatttatta 14040
   tttttattat ttttgagttt tgtttttatg aaattatttt ttttttgatt ataggttata 14100
   tttttttttt tttttttttt tttttgagat ggagtttttt tttttgttgt ttaggttgga 14160
   gtgtaggggc gttatttcgg tttattgtaa gttcgatttt ttgatttcgt gatttattcg 14220
   ttttagtttt ttaaaatgtt gggattatag gtttacgtga gttatcgtat tttgtttata 14280
   agttttattt ttttattttt tggtatattt agtaattttt aattgaattt tggatattgt 14340
   aaatatgatg ttaagtattt ggatttgtta ttgtttttta aagagtgttg ggttttgttt 14400
   aggtagatta ggtaaattat ttgtaaatta gtttgatttt tttgaggttt ttttttttga 14460
   gatagagttt tgttttgtta tttaggtttg agtgtagtgg ggtaatttta gtttattgta 14520
   gttttaattt tttgggttta agggattttt ttgttttagt tattgagtag ttgaggttat 14580
   aggtatatgt tattatattt ggttaatttt ttgtattttt tgtagatatg gggttttggt 14640
   atgttgttta ggttggtttt gaatttttag gtttaagtga tttgtttgtt ttagtttttt 14700
   aaagtgttgg gattagaggt gtgagttatc gtgtttggtt tttgatgtta attttttttt 14760
   tttttttttt tttttttttt tttttttttt tgagatggag ttttgttttg ttatttaggt 14820
   tagagtgtag tggtgtagtt ttggtttatt gtaattttcg ttttttgggt ttaagtgatt 14880
   tttgtgtttt agttttttga gtagttggga ttataggtat ttattattaa gttcggttaa 14940
   ttttttgtat ttttagtaga gatgtggttt cgttattttg gttaggttgg ttttgaattt 15000
   ttgattttag gtgatttgtt cgttttcgtt ttttaaagtg ttgggattat aggtatgagt 15060
   tattgtattc ggtttgaggt taatttttaa tttgttaggg tgggtttagg tgagtttttt 15120
   ttttagggat agtttagttt tattgttaag gtataatttt tttgaggttt gattaaatat 15180
   gtatttttat tttaggaggt tggagtttga ttattttttt gtttttggtg agttttgaga 15240
   atttattatt ttattgtttt tttttgtgtg gttttattga gttttattat atatatgtga 15300
   agtttagtat ttaggaagat ttgaagagat ttttatgtag atttttggag ttttttgagt 15360
   agtttatttt tttttagaat tgtgttttgt tgtaattttt aatattttgg tttttttgaa 15420
   ttttgttttt atttttttaa tttaggaaga gtattatgtt ttgttcgggt tttttttttt 15480
   tgaattatag tttggaatgt ttttttaggt agaaatttag ggatgttata gagtttattt 15540
   tgtttatttt ttttatttta gggattatag ttttggattt ttaaaaatag ttattttttt 15600
   ttatatatat tttttatata gagaaatata tatatatata tatataaata tatatatata 15660
   tatatatata tatatatata tatatatata tatatacgta tatgtatata tatatttgag 15720
   atagcgtttt attttgttgt ttagtttgga gtgtagaggt gtgatgatat tttattgtag 15780
   ttttaatttt ttaggtttaa gcgatttttt tattttagtt tttgagtagt tgggattata 15840
   ggtataggtt attatattcg gttaattttt ttgtattttt tgtagagatg gggttttgtt 15900
   atattgttta ggttgatttt taatttttgg gtttaagtaa ttcgttaatt tttgtttttt 15960
   aaattgttgg gattataggg gtgagttttt gtgtttagtt tttatatatt ttgtagtaat 16020
   aattttttta ttgtttatgg tggtagagta attttattta ttatggtagg agtagaagtt 16080
   tttttattgg cgttttaatt aagtttagag aagaggaggg agttatatgt atgtggttag 16140
   tttattgttt tttttttttt tgagacggag ttttgttttt gtcgtttagg ttggagtgta 16200
   gtggtatgat ttcggtttat tgtaattttc gtttttcggg tttaagcggt ttttttgttt 16260
   tagttttttg attagttggg attataggta tgggttatta cgtttggtta attttgtatt 16320
   tttagtagag atggggtttt tttatgttgt taggttggtt tcgaattttt aattttaggt 16380
   gatttattcg tttcggtttt ttaaagtgtt gggattatag gcgtgagtta tcgcgtttgg 16440
   tttgttagtt tattgttttt aattagaagt tttagatttt cgaaatatat ttaaatattt 16500
   atgtttaaga tcgggcgtgg tggtttacgt ttgtaatttt aatattttgg gaggttaagg 16560
   taggcgaatt atttgaggtt aggagttaga gattagtttg ggtaatatat taaaatttcg 16620
   tttttattaa aatataaaaa ttagtttggt atagtggtgt tcgtttgtaa ttttagttat 16680
   ttagaaggtt gaggtataag aatcgtttga atttgggagg cggaagttgt aatgagttaa 16740
   gatggtatta ttgtatttta gtttgggtga tatagtgaga ttttgtttta aaaaataaat 16800
   aaataaataa ataaataaaa tttacgttta ataaatatgt tttttaagtt aggtgtaggg 16860
   gtttgtaatt ttagttgttt gggaggttga aataggagga ttgtttaagt ttaggagttt 16920
   gagattagtt tgggtgatat agtgagattt tgttttaagt aaaaaaaaaa aaaaaaaaaa 16980
   aaaaaaaaaa aattttttgt ttgggtattg cggtttatat ttgtaatttt agtattttgg 17040
   gagttcgagt taggtggatt atttgaggtt aggagtttaa gattagtttg gttaatatgg 17100
   tgaaaattta tttttattaa aaatataaaa aattagttag acgtgttggc gaatgtttgt 17160
   aattttagtt atttgggagg ttgaggtagg agaatcgttt gaatttggga gacggaggtt 17220
   atagcgagtt gatattacgt tattgtattt tagtttagtt aatagagtga gatttcgttt 17280
   ttttaaaaaa aatataaaaa aaaattttaa aaaaagtttt tattattttt attatataat 17340
   agaaatttta tgtaatgtaa ttatgttatt tataaaatta taggatttgt ataaatttta 17400
   atgtatagtg ttttataagt ataggatatg gttgtgaaat tgtattaata ttatgaattt 17460
   gttattttat ttttgtttgt gtattattgt aatcgtttgt taaattttat ttagtgataa 17520
   tgttagtgat ttaaattggt agtttttttt gaaggtggtt tgggattttt agggtaggtt 17580
   tggttttagg tgagtttttt ggttaatagt ttttttgtta ttttatttta gttttaggtt 17640
   tatttttggt aggtaggtta ttgggttttg gaggagagga tttagttagg ggtttttggt 17700
   ggataggaag ggaggaagtt tttttttttt tagagttatt ttttgttttg ttttttaagt 17760
   ttttttttat ttacgtgttt gttttttcgt tgtttatagt gtttgttttt tcgttgttta 17820
   tagtgtttgt tttttcgttg ttttgtgagg ttggtgaggt agagtagttt tagtaggggg 17880
   tgggggttgt tgtttgtttt agggttgttt ggtttcggag ggttgttttc gacgtgattg 17940
   gggttttatt ttttattttt tttttttttt taggtggata tcgagtagtt ggattttcgc 18000
   ggtcggattt ttttgtattt ggttattacg ttggggtatt ttgagtgtgt tcgtgtgttt 18060
   ttggcgtacg gcgtagacgt gggtagggag aatcgtagcg gttggatagg tgggtagttt 18120
   tgtttatttt agttttatag tcggggtttt tttagtatta tagatttatg gttggttgaa 18180
   gggttttgtt ttgtgatttt gtttcgtttt tttttagtgt tttaggaggt tgtgagtatt 18240
   cgggatttgg agttggtgta gttggtgttt cggtatcggg attattagcg ggtggtgaag 18300
   cggttggcgg gtattttcgt gtttttggag aagttgcgta aggtgaggtt tagtttttta 18360
   gtttttacgg gagtttttga gttttttttt agtgtagatt tagttttttt ttttttcgtt 18420
   ttttaggttt aggattttta cgtggagatg aaatgggagt ttattagttg gggtaagcgg 18480
   gttgtagtag gtcgtttttg ggttttttta ttttaggttt tgcgtttgtt tttttgtttg 18540
   gaatgtgttt ttatagtttt ttcggcggtt ttttttatgt tttagtttag tttagggtta 18600
   tcgttttagt taggtttttt tagattgttt tagatattgt tttgatttta ttattatttt 18660
   ttttattttg ttatgatttg tttattgtat ttatgatttg tcgttttttg ttagaaggta 18720
   agttttgaga gggtaggagt tttgtttttt ttattgttgt atttttagtg gttggaatat 18780
   ttagtaggtg ttttataaat atttgttgaa agaatggatg aaagagtgag tgagtgaatt 18840
   aataaatgat ggatattagg gatttagtgg tgatagaaat atgttcgttt cgattttagg 18900
   ttgttttttt gtgtgagtat ggtttatatt ggttagggag ggggttttgg aggagaggtt 18960
   gttttttttt tagttttttt ttttgttttt ggttatacgg attttaaatt ttatgttttg 19020
   agttggttta ggtcgtgtag gttagattag ttaaagttat ttttaatttt cgtttttatt 19080
   agtgtttttg gtgtttaaga tttgttttag tgatatttat aaagtgtgga agagcggtta 19140
   gaatttgagg gtagatatta tatttttggg ttttgattat atgatttggt agcgagggaa 19200
   tcgtagtttt gtttttaggg gttaaggtta ggtaggtagg aagtggggta gggtggggac 19260
   gatgggaggt agttttaggt tatttttgat ttttttgtgt tgtttcgtgt ttggtagata 19320
   taagcgtcgt ggttatggag attgattacg atcgtcgggt ggtgtatata gagattttgg 19380
   tattggttgg gtaggatcgg gagttgttgt tggttgttgt ttagtttatt gaggaatagg 19440
   tgttgagtcg gtttatcgcg ttcgtcgtta ttatttagtt tgatattaag aatatttttt 19500
   ttgagaggtg ggtggatatg gttttggtta tttttgagtt agttcggtgg ggtgtttgtt 19560
   ttatttggat tattttgttt ttttaggaat aagattggta ttttgggttg gcgtagtgaa 19620
   aagacggaga tggtgaatgg gtatgaagtt aaggtgaggt tgtagttttt agttgttagt 19680
   ttagttgtat ttttgtttat agtaagttgt ataggggatt ttgatttttt ttttttagtt 19740
   ttggaggtat agaagtaatg tagcgtggtt aagtatttat atttgggttt aaggtatttt 19800
   tttggtgttt tatatggatg tatggatgta tatatagggt acgtgtatac gtatatatat 19860
   atatatattt attttttata gtaatttttt ttggtaggtg ttgttttgtt agtattttta 19920
   ttttacgtat gtttattgag tttttttttt ggtattttat ttatttttta taataatttt 19980
   aggaagtagg gatgatcggt ttattttatg atgaggaaat agaggtttag agtggttttg 20040
   tgatttgtat aaggttatat agttgttaag tggtagagtt ggggtttaga ttttggttgg 20100
   tttgatttta gagttaggtt tttattttta aaaatgattg tttggttggg tatggtggtt 20160
   tatgtttgta attttagcgt tttgggaggt cgaggtgagc ggattatttg aggttagttc 20220
   gagattagtt tggttaatat ggtgaaattt cgtttttatt aataatataa aaaattagtc 20280
   gggtgtggta gtaggcgttt gtaattttag ttattcggga ggttgaggta ggagaattgt 20340
   ttgaatttgg gaggcggagt ttgtagtgtg tagcgatttc gttatagtat tttagtttgg 20400
   gtaatagagc gagattttat ttaaaaaaaa aaaaaaaaaa aggttttgtt tgttattttg 20460
   ggatttttat gttgtttacg atatgaaagt aggaattaat aaatgttatg ggttttgtgt 20520
   gtggtatttt ttttgaaagt tttttttgtt tttgtagttt gggaaggttt ttgtgttatt 20580
   tttattagtg tatggattat gttgttataa ttttatttta ttgttttttt atagagattg 20640
   taagttttag gagggtaggg attatgtttg gtttgtttat tgtatttttg atgtttattt 20700
   taatgtttgg tatataaagg ttttttaaaa aatgtttgtt gaatgaataa gtgaatgaat 20760
   gaatgaatga atgattttat atatttattt atttaaaagt ttgaagtaga gtttttttag 20820
   gattttttgt tatagggagt gtttttagtg tttttagggg taagatatag ggtaagatta 20880
   gaattgttta tgagtttttt ttttatttgt ttttaggtgt atggggtatt taacgtggag 20940
   tttattattc gtatacggat agaatatttt ttagaatagt ataagggtaa ggttaaaggt 21000
   aatgaggtag agttggatgg ggagaggtgt tgtaggattg cgggaattag tttttatttt 21060
   agtgttttta ttagtatttt tattggtatt ttagtaagag gttggatgtt ttatttaggt 21120
   cgatagattt taatttaggt gttaagtttt aaggatgggt agtggttgtt tagagtatta 21180
   agaagttttt aaggatttat ttgggtttag tggagagtta tgttgtaagt gattattaat 21240
   gttgttgttg aatttgggag aggagagaaa tgttatgtat gttatttatt ggttatttta 21300
   gtttagtggg atatatttta ggtagaaagt taggggattt tgttatagat tttgtatgtt 21360
   tattgatgtt atatttgatt ttttgggtat tagatatcgt gttcgaggtg aaaaagtata 21420
   atttttattt tataggggaa taatatgaaa ttatgtttat ggaagtgttt gaagtaaata 21480
   tatatacgta tatgtatatt ttatagtgtt tgtcggggta ggcgttttat tgtttagggt 21540
   tatcgagttg gagaggagta tttttggaag ttgtttttga tttttaggtt ttttttttag 21600
   tgttttaagt tgtgtttata gaggttttgg tttttggttt ttgaagtatt tagttttttt 21660
   taggtgaagg tgtttgagta tgtggttggg tgagtaggtt ttattttagg aatcggtttt 21720
   ttttttaggt tttgggtgtt gttatttttg atgaggtgtt taggtgtgtg tggagttgcg 21780
   gtgttttttg agatgttata ttttttgtag gttgtaagat atttttgtag ttttttttgg 21840
   gaatcgttga gtagtacggg ggtttttaaa atggggtgag tgtgtgtagg ggtattcgta 21900
   agtggaggga tgtggatgtg gtttaggagg aggtttggga agcgttttgt ttagtatggt 21960
   ttatgtggat ttttttttta taatgtttaa ggttatgtta gcgttatatt ggtagagggg 22020
   atagtttggg ggttttgtga taataaatga gttggtttgg tattaggaat tgaggggagt 22080
   ttttgggttt ttttttatgt ggtatagatt ttgattattt agattttgag ttaagttaat 22140
   tttattgtta ttattgtaga agaatatttt aattttaatt ttgagttggg taatcgtgat 22200
   atgggtcgtt ttatggaatt gattattaag atatagaagt gaggtttttg tcggtgttgg 22260
   gaaggtgggg ggtcggggtt ttaggagatg ttgggaggtt atggtttttt gtagtgtttt 22320
   ttattttttg ggatttggtg ggggtaggtt taaggttaag ttgtggttgt gtgaggagta 22380
   ttttttgttt ttgtgtgagt aggtggtttt tattattgat tttatggtcg ttagtaatgc 22440
   gttttttgtt aagtttcggg attttattat tttgcgtttg ttttttggtt ttttagttaa 22500
   gattggtgag atcgtacggt tatttttttt gagttcgtgg cgggtcgatc ggggtttaga 22560
   tgaggtcggg gtggggttga ggttgaggtg tgaggggttg agtggtggcg tttaaaggtt 22620
   tttttatttt tagaaatttc gattttttat atttttaacg ttcgtattat tttcgggaat 22680
   tttaacggtt gcgacgaatc ggtgttatcg gtgcgaggta gttttagtag cgagacgttt 22740
   tttttaggta gcgatttttt tagcgttagt agttttagtt ttacgagtga ggtttttcgc 22800
   gagaacgttt gtttttcggt ttttttcggg gtgggtggga ggggcgcgtt cggtcgtgtt 22860
   tgatttagtt ttcgcgtttt tagttttttg tcgcggttgc gagatttttt tagcgttgtt 22920
   cgaggtttcg cgcggttata gtatgatggg cggttagcgg gaggcggcga ttcgggacga 22980
   cgacgacgat ttgttgtaat tcgttattta gtagagtttg tttgaggcgg gtagtgagta 23040
   tgattaggtg cgttttcgcg ggcgcgcggg gttacgggat ttcgcgtcgt tacggttaac 23100
   gtttacgttt tttcgtaggt tattatttgg gaggcgttaa ttaatagtaa gttaggtatt 23160
   tattttatgt tttacgaggg tcgtcgatag gataggttag tgttcgttgg gtcggagaga 23220
   atttttcgga gaggattttg ttttttttaa gttaggatat agtcggagaa ttcgcggggt 23280
   tttcggtagg gatttttttg gtatcgggat ggtttttgtt attatttttg ttttcgaggc 23340
   ggtaggtagg ggttagggat ttaggtttta tcgcggagtt tagatttttt ttaggtattg 23400
   acggggagaa tttggtaggg tttgggtttc gttacgatta ggtgggggtt aggacgtcgc 23460
   gggattgatc gtttttatta gagggtggtg gtcgcgggtc gttttggagt ttttacgtcg 23520
   tgtttcggtt tcggtcggtg ggtgggtgcg ttcgggagtt cgcgatattt tttttttatt 23580
   tttaggagcg tttcgtttac gtcgtagcgt tagtttgcgt tttcggcgtt agtgtttagt 23640
   tttcggttta gtttagggtt aggtttcggc ggttacgtgt ttcggagtta cgacgagtag 23700
   ttgcggttgg cgatggaatt gtcggcgtag gagtaggagg agaggcggcg gcgcgcgcgt 23760
   taggaggagg aggagttgga gcgtattttg aggtttttat tgatcgagta gtagcgtttt 23820
   ttgtcgggat tttcgttagc gttacgcgcg ttacgtttag ggttaggagt tagataaatt 23880
   tcggtttgcg cgtttgtaga gcggcggttg gagattggag ttatcgtttc gcgggtgtag 23940
   tagtatagta ggtacggttc ggggagggat tcggtatggt cgcggggtat ttttttaggt 24000
   tagggttttg gaggtaattg gtacggtttg gtttttttgt tttgttgtat tttgattttt 24060
   taattcgttt ttttgggttt agatttgttt tgttttaggg cggagttagg cggttttgag 24120
   ggggagatga atttttagag gagcgttgtt tttatttttt gttttttttg gtcggttcgt 24180
   atttttgttt attaatttta ttttaggata ttgtttttga agtttttgta tttttgtttt 24240
   ttatttttgg ggggtagttg agtttttcgc gtgttgtgtt attgttttgt tttagatata 24300
   aattagtacg tttagggttt agtttttttt ttatttcggt attttagcgt taagtgtatt 24360
   tagcggggta ttcggttttt ttagttttta tattcgtttt gggttttagg gtggttttag 24420
   tttttttttg ggtagttaga agttggagtt ttatttttta aggtatattt tttttttttt 24480
   ttttttgtga ttagggagtg tgtttaacgt agttttttgg tttgtgtaag ttttgatttt 24540
   tttatggtgt ttcggagagt ggggagtata ttgggttggg gtaagtatta gatttaagta 24600
   gattggatat aaagggttcg tttagggttt tggcgttatt tttatttttt tttattagtt 24660
   gttgttagtt tttgtggttg tatattttat ttgtttttat acggagattg attttaaaat 24720
   tttttattta atggtgttaa ttttcggttt ttttttgttt tattttattt atttagagaa 24780
   gtatagattt cgttaggggt aggggtttat cgtatatttt tgtttcgggt ttgtttggga 24840
   ttggtttttt cggtttagtt agtgaggttt agaagggata taaagaggga tggaagaaaa 24900
   gaataaagag aaattgtttt ttttattttt ttttttgatg ttaggggtat tagattgatt 24960
   ttgaggtata aataaaagag gttttatatc gga 24993
<210> 8
   <211> 24993
   <212> DNA
   <213> Artificial Sequence
<220>
   <223> rc C to T (bisl)
<400> 8
   tccgatataa aacctctttt atttatacct caaaatcaat ctaatacccc taacatcaaa       60
   aaaaaaaata aaaaaaacaa tttctcttta ttcttttctt ccatccctct ttatatccct      120
   tctaaacctc actaactaaa ccgaaaaaac caatcccaaa caaacccgaa acaaaaatat      180
   acgataaacc cctaccccta acgaaatcta tacttctcta aataaataaa ataaaacaaa      240
   aaaaaaccga aaattaacac cattaaataa aaaattttaa aatcaatctc cgtataaaaa      300
   caaataaaat atacaaccac aaaaactaac aacaactaat aaaaaaaaat aaaaataacg      360
   ccaaaaccct aaacgaaccc tttatatcca atctacttaa atctaatact taccccaacc      420
   caatatactc cccactctcc gaaacaccat aaaaaaatca aaacttacac aaaccaaaaa      480
   actacgttaa acacactccc taatcacaaa aaaaaaaaaa aaaaaatata ccttaaaaaa      540
   taaaactcca acttctaact acccaaaaaa aaactaaaac caccctaaaa cccaaaacga      600
   atataaaaac taaaaaaacc gaataccccg ctaaatacac ttaacgctaa aataccgaaa      660
   taaaaaaaaa actaaaccct aaacgtacta atttatatct aaaacaaaac aataacacaa      720
   cacgcgaaaa actcaactac cccccaaaaa taaaaaacaa aaatacaaaa acttcaaaaa      780
   caatatccta aaataaaatt aataaacaaa aatacgaacc gaccaaaaaa aacaaaaaat 840
   aaaaacaacg ctcctctaaa aattcatctc cccctcaaaa ccgcctaact ccgccctaaa 900
   acaaaacaaa tctaaaccca aaaaaacgaa ttaaaaaatc aaaatacaac aaaacaaaaa 960
   aaccaaaccg taccaattac ctccaaaacc ctaacctaaa aaaatacccc gcgaccatac 1020
   cgaatccctc cccgaaccgt acctactata ctactacacc cgcgaaacga taactccaat 1080
   ctccaaccgc cgctctacaa acgcgcaaac cgaaatttat ctaactccta accctaaacg 1140
   taacgcgcgt aacgctaacg aaaatcccga caaaaaacgc tactactcga tcaataaaaa 1200
   cctcaaaata cgctccaact cctcctcctc ctaacgcgcg cgccgccgcc tctcctccta 1260
   ctcctacgcc gacaattcca tcgccaaccg caactactcg tcgtaactcc gaaacacgta 1320
   accgccgaaa cctaacccta aactaaaccg aaaactaaac actaacgccg aaaacgcaaa 1380
   ctaacgctac gacgtaaacg aaacgctcct aaaaataaaa aaaaaatatc gcgaactccc 1440
   gaacgcaccc acccaccgac cgaaaccgaa acacgacgta aaaactccaa aacgacccgc 1500
   gaccaccacc ctctaataaa aacgatcaat cccgcgacgt cctaaccccc acctaatcgt 1560
   aacgaaaccc aaaccctacc aaattctccc cgtcaatacc taaaaaaaat ctaaactccg 1620
   cgataaaacc taaatcccta acccctacct accgcctcga aaacaaaaat aataacaaaa 1680
   accatcccga taccaaaaaa atccctaccg aaaaccccgc gaattctccg actatatcct 1740
   aacttaaaaa aaacaaaatc ctctccgaaa aattctctcc gacccaacga acactaacct 1800
   atcctatcga cgaccctcgt aaaacataaa ataaatacct aacttactat taattaacgc 1860
   ctcccaaata ataacctacg aaaaaacgta aacgttaacc gtaacgacgc gaaatcccgt 1920
   aaccccgcgc gcccgcgaaa acgcacctaa tcatactcac tacccgcctc aaacaaactc 1980
   tactaaataa cgaattacaa caaatcgtcg tcgtcgtccc gaatcgccgc ctcccgctaa 2040
   ccgcccatca tactataacc gcgcgaaacc tcgaacaacg ctaaaaaaat ctcgcaaccg 2100
   cgacaaaaaa ctaaaaacgc gaaaactaaa tcaaacacga ccgaacgcgc ccctcccacc 2160
   caccccgaaa aaaaccgaaa aacaaacgtt ctcgcgaaaa acctcactcg taaaactaaa 2220
   actactaacg ctaaaaaaat cgctacctaa aaaaaacgtc tcgctactaa aactacctcg 2280
   caccgataac accgattcgt cgcaaccgtt aaaattcccg aaaataatac gaacgttaaa 2340
   aatataaaaa atcgaaattt ctaaaaataa aaaaaccttt aaacgccacc actcaacccc 2400
   tcacacctca acctcaaccc caccccgacc tcatctaaac cccgatcgac ccgccacgaa 2460
   ctcaaaaaaa ataaccgtac gatctcacca atcttaacta aaaaaccaaa aaacaaacgc 2520
   aaaataataa aatcccgaaa cttaacaaaa aacgcattac taacgaccat aaaatcaata 2580
   ataaaaacca cctactcaca caaaaacaaa aaatactcct cacacaacca caacttaacc 2640
   ttaaacctac ccccaccaaa tcccaaaaaa taaaaaacac tacaaaaaac cataacctcc 2700
   caacatctcc taaaaccccg accccccacc ttcccaacac cgacaaaaac ctcacttcta 2760
   tatcttaata atcaattcca taaaacgacc catatcacga ttacccaact caaaattaaa 2820
   attaaaatat tcttctacaa taataacaat aaaattaact taactcaaaa tctaaataat 2880
   caaaatctat accacataaa aaaaaaccca aaaactcccc tcaattccta ataccaaacc 2940
   aactcattta ttatcacaaa acccccaaac tatcccctct accaatataa cgctaacata 3000
   accttaaaca ttataaaaaa aaaatccaca taaaccatac taaacaaaac gcttcccaaa 3060
   cctcctccta aaccacatcc acatccctcc acttacgaat acccctacac acactcaccc 3120
   cattttaaaa acccccgtac tactcaacga ttcccaaaaa aaactacaaa aatatcttac 3180
   aacctacaaa aaatataaca tctcaaaaaa caccgcaact ccacacacac ctaaacacct 3240
   catcaaaaat aacaacaccc aaaacctaaa aaaaaaaccg attcctaaaa taaaacctac 3300
   tcacccaacc acatactcaa acaccttcac ctaaaaaaaa ctaaatactt caaaaaccaa 3360
   aaaccaaaac ctctataaac acaacttaaa acactaaaaa aaaaacctaa aaatcaaaaa 3420
   caacttccaa aaatactcct ctccaactcg ataaccctaa acaataaaac gcctaccccg 3480
   acaaacacta taaaatatac atatacgtat atatatttac ttcaaacact tccataaaca 3540
   taatttcata ttattcccct ataaaataaa aattatactt tttcacctcg aacacgatat 3600
   ctaataccca aaaaatcaaa tataacatca ataaacatac aaaatctata acaaaatccc 3660
   ctaactttct acctaaaata tatcccacta aactaaaata accaataaat aacatacata 3720
   acatttctct cctctcccaa attcaacaac aacattaata atcacttaca acataactct 3780
   ccactaaacc caaataaatc cttaaaaact tcttaatact ctaaacaacc actacccatc 3840
   cttaaaactt aacacctaaa ttaaaatcta tcgacctaaa taaaacatcc aacctcttac 3900
   taaaatacca ataaaaatac taataaaaac actaaaataa aaactaattc ccgcaatcct 3960
   acaacacctc tccccatcca actctacctc attaccttta accttaccct tatactattc 4020
   taaaaaatat tctatccgta tacgaataat aaactccacg ttaaataccc catacaccta 4080
   aaaacaaata aaaaaaaaac tcataaacaa ttctaatctt accctatatc ttacccctaa 4140
   aaacactaaa aacactccct ataacaaaaa atcctaaaaa aactctactt caaactttta 4200
   aataaataaa tatataaaat cattcattca ttcattcatt cacttattca ttcaacaaac 4260
   atttttaaaa aaacctttat ataccaaaca ttaaaataaa catcaaaaat acaataaaca 4320
   aaccaaacat aatccctacc ctcctaaaac ttacaatctc tataaaaaaa caataaaata 4380
   aaattataac aacataatcc atacactaat aaaaataaca caaaaacctt cccaaactac 4440
   aaaaacaaaa aaaactttca aaaaaaatac cacacacaaa acccataaca tttattaatt 4500
   cctactttca tatcgtaaac aacataaaaa tcccaaaata acaaacaaaa cctttttttt 4560
   tttttttttt taaataaaat ctcgctctat tacccaaact aaaatactat aacgaaatcg 4620
   ctacacacta caaactccgc ctcccaaatt caaacaattc tcctacctca acctcccgaa 4680
   taactaaaat tacaaacgcc tactaccaca cccgactaat tttttatatt attaataaaa 4740
   acgaaatttc accatattaa ccaaactaat ctcgaactaa cctcaaataa tccgctcacc 4800
   tcgacctccc aaaacgctaa aattacaaac ataaaccacc atacccaacc aaacaatcat 4860
   ttttaaaaat aaaaacctaa ctctaaaatc aaaccaacca aaatctaaac cccaactcta 4920
   ccacttaaca actatataac cttatacaaa tcacaaaacc actctaaacc tctatttcct 4980
   catcataaaa taaaccgatc atccctactt cctaaaatta ttataaaaaa taaataaaat 5040
   accaaaaaaa aaactcaata aacatacgta aaataaaaat actaacaaaa caacacctac 5100
   caaaaaaaat tactataaaa aataaatata tatatatata tacgtataca cgtaccctat 5160
   atatacatcc atacatccat ataaaacacc aaaaaaatac cttaaaccca aatataaata 5220
   cttaaccacg ctacattact tctatacctc caaaactaaa aaaaaaaaat caaaatcccc 5280
   tatacaactt actataaaca aaaatacaac taaactaaca actaaaaact acaacctcac 5340
   cttaacttca tacccattca ccatctccgt cttttcacta cgccaaccca aaataccaat 5400
   cttattccta aaaaaacaaa ataatccaaa taaaacaaac accccaccga actaactcaa 5460
   aaataaccaa aaccatatcc acccacctct caaaaaaaat attcttaata tcaaactaaa 5520
   taataacgac gaacgcgata aaccgactca acacctattc ctcaataaac taaacaacaa 5580
   ccaacaacaa ctcccgatcc tacccaacca ataccaaaat ctctatatac accacccgac 5640
   gatcgtaatc aatctccata accacgacgc ttatatctac caaacacgaa acaacacaaa 5700
   aaaatcaaaa ataacctaaa actacctccc atcgtcccca ccctacccca cttcctacct 5760
   acctaacctt aacccctaaa aacaaaacta cgattccctc gctaccaaat catataatca 5820
   aaacccaaaa atataatatc taccctcaaa ttctaaccgc tcttccacac tttataaata 5880
   tcactaaaac aaatcttaaa caccaaaaac actaataaaa acgaaaatta aaaataactt 5940
   taactaatct aacctacacg acctaaacca actcaaaaca taaaatttaa aatccgtata 6000
   accaaaaaca aaaaaaaaaa ctaaaaaaaa aacaacctct cctccaaaac cccctcccta 6060
   accaatataa accatactca cacaaaaaaa caacctaaaa tcgaaacgaa catatttcta 6120
   tcaccactaa atccctaata tccatcattt attaattcac tcactcactc tttcatccat 6180
   tctttcaaca aatatttata aaacacctac taaatattcc aaccactaaa aatacaacaa 6240
   taaaaaaaac aaaactccta ccctctcaaa acttaccttc taacaaaaaa cgacaaatca 6300
   taaatacaat aaacaaatca taacaaaata aaaaaaataa taataaaatc aaaacaatat 6360
   ctaaaacaat ctaaaaaaac ctaactaaaa cgataaccct aaactaaact aaaacataaa 6420
   aaaaaccgcc gaaaaaacta taaaaacaca ttccaaacaa aaaaacaaac gcaaaaccta 6480
   aaataaaaaa acccaaaaac gacctactac aacccgctta ccccaactaa taaactccca 6540
   tttcatctcc acgtaaaaat cctaaaccta aaaaacgaaa aaaaaaaaaa ctaaatctac 6600
   actaaaaaaa aactcaaaaa ctcccgtaaa aactaaaaaa ctaaacctca ccttacgcaa 6660
   cttctccaaa aacacgaaaa tacccgccaa ccgcttcacc acccgctaat aatcccgata 6720
   ccgaaacacc aactacacca actccaaatc ccgaatactc acaacctcct aaaacactaa 6780
   aaaaaaacga aacaaaatca caaaacaaaa cccttcaacc aaccataaat ctataatact 6840
   aaaaaaaccc cgactataaa actaaaataa acaaaactac ccacctatcc aaccgctacg 6900
   attctcccta cccacgtcta cgccgtacgc caaaaacaca cgaacacact caaaataccc 6960
   caacgtaata accaaataca aaaaaatccg accgcgaaaa tccaactact cgatatccac 7020
   ctaaaaaaaa aaaaaaaata aaaaataaaa ccccaatcac gtcgaaaaca accctccgaa 7080
   accaaacaac cctaaaacaa acaacaaccc ccacccccta ctaaaactac tctacctcac 7140
   caacctcaca aaacaacgaa aaaacaaaca ctataaacaa cgaaaaaaca aacactataa 7200
   acaacgaaaa aacaaacacg taaataaaaa aaaacttaaa aaacaaaaca aaaaataact 7260
   ctaaaaaaaa aaaaacttcc tcccttccta tccaccaaaa acccctaact aaatcctctc 7320
   ctccaaaacc caataaccta cctaccaaaa ataaacctaa aactaaaata aaataacaaa 7380
   aaaactatta accaaaaaac tcacctaaaa ccaaacctac cctaaaaatc ccaaaccacc 7440
   ttcaaaaaaa actaccaatt taaatcacta acattatcac taaataaaat ttaacaaacg 7500
   attacaataa tacacaaaca aaaataaaat aacaaattca taatattaat acaatttcac 7560
   aaccatatcc tatacttata aaacactata cattaaaatt tatacaaatc ctataatttt 7620
   ataaataaca taattacatt acataaaatt tctattatat aataaaaata ataaaaactt 7680
   tttttaaaat ttttttttat atttttttta aaaaaacgaa atctcactct attaactaaa 7740
   ctaaaataca ataacgtaat atcaactcgc tataacctcc gtctcccaaa ttcaaacgat 7800
   tctcctacct caacctccca aataactaaa attacaaaca ttcgccaaca cgtctaacta 7860
   attttttata tttttaataa aaataaattt tcaccatatt aaccaaacta atcttaaact 7920
   cctaacctca aataatccac ctaactcgaa ctcccaaaat actaaaatta caaatataaa 7980
   ccgcaatacc caaacaaaaa attttttttt tttttttttt tttttttttt tttacttaaa 8040
   acaaaatctc actatatcac ccaaactaat ctcaaactcc taaacttaaa caatcctcct 8100
   atttcaacct cccaaacaac taaaattaca aacccctaca cctaacttaa aaaacatatt 8160
   tattaaacgt aaattttatt tatttattta tttatttatt ttttaaaaca aaatctcact 8220
   atatcaccca aactaaaata caataatacc atcttaactc attacaactt ccgcctccca 8280
   aattcaaacg attcttatac ctcaaccttc taaataacta aaattacaaa cgaacaccac 8340
   tataccaaac taatttttat attttaataa aaacgaaatt ttaatatatt acccaaacta 8400
   atctctaact cctaacctca aataattcgc ctaccttaac ctcccaaaat attaaaatta 8460
   caaacgtaaa ccaccacgcc cgatcttaaa cataaatatt taaatatatt tcgaaaatct 8520
   aaaacttcta attaaaaaca ataaactaac aaaccaaacg cgataactca cgcctataat 8580
   cccaacactt taaaaaaccg aaacgaataa atcacctaaa attaaaaatt cgaaaccaac 8640
   ctaacaacat aaaaaaaccc catctctact aaaaatacaa aattaaccaa acgtaataac 8700
   ccatacctat aatcccaact aatcaaaaaa ctaaaacaaa aaaaccgctt aaacccgaaa 8760
   aacgaaaatt acaataaacc gaaatcatac cactacactc caacctaaac gacaaaaaca 8820
   aaactccgtc tcaaaaaaaa aaaaaacaat aaactaacca catacatata actccctcct 8880
   cttctctaaa cttaattaaa acgccaataa aaaaacttct actcctacca taataaataa 8940
   aattactcta ccaccataaa caataaaaaa attattacta caaaatatat aaaaactaaa 9000
   cacaaaaact cacccctata atcccaacaa tttaaaaaac aaaaattaac gaattactta 9060
   aacccaaaaa ttaaaaatca acctaaacaa tataacaaaa ccccatctct acaaaaaata 9120
   caaaaaaatt aaccgaatat aataacctat acctataatc ccaactactc aaaaactaaa 9180
   ataaaaaaat cgcttaaacc taaaaaatta aaactacaat aaaatatcat cacacctcta 9240
   cactccaaac taaacaacaa aataaaacgc tatctcaaat atatatatac atatacgtat 9300
   atatatatat atatatatat atatatatat atatatatat atatatttat atatatatat 9360
   atatatattt ctctatataa aaaatatata taaaaaaaaa taactatttt taaaaatcca 9420
   aaactataat ccctaaaata aaaaaaataa acaaaataaa ctctataaca tccctaaatt 9480
   tctacctaaa aaaacattcc aaactataat tcaaaaaaaa aaaacccgaa caaaacataa 9540
   tactcttcct aaattaaaaa aataaaaaca aaattcaaaa aaaccaaaat attaaaaatt 9600
   acaacaaaac acaattctaa aaaaaaataa actactcaaa aaactccaaa aatctacata 9660
   aaaatctctt caaatcttcc taaatactaa acttcacata tatataataa aactcaataa 9720
   aaccacacaa aaaaaaacaa taaaataata aattctcaaa actcaccaaa aacaaaaaaa 9780
   taatcaaact ccaacctcct aaaataaaaa tacatattta atcaaacctc aaaaaaatta 9840
   taccttaaca ataaaactaa actatcccta aaaaaaaaac tcacctaaac ccaccctaac 9900
   aaattaaaaa ttaacctcaa accgaataca ataactcata cctataatcc caacacttta 9960
   aaaaacgaaa acgaacaaat cacctaaaat caaaaattca aaaccaacct aaccaaaata 10020
   acgaaaccac atctctacta aaaatacaaa aaattaaccg aacttaataa taaataccta 10080
   taatcccaac tactcaaaaa actaaaacac aaaaatcact taaacccaaa aaacgaaaat 10140
   tacaataaac caaaactaca ccactacact ctaacctaaa taacaaaaca aaactccatc 10200
   tcaaaaaaaa aaaaaaaaaa aaaaaaaaaa aaaaaaaaaa aattaacatc aaaaaccaaa 10260
   cacgataact cacacctcta atcccaacac tttaaaaaac taaaacaaac aaatcactta 10320
   aacctaaaaa ttcaaaacca acctaaacaa cataccaaaa ccccatatct acaaaaaata 10380
   caaaaaatta accaaatata ataacatata cctataacct caactactca ataactaaaa 10440
   caaaaaaatc ccttaaaccc aaaaaattaa aactacaata aactaaaatt accccactac 10500
   actcaaacct aaataacaaa acaaaactct atctcaaaaa aaaaaacctc aaaaaaatca 10560
   aactaattta caaataattt acctaatcta cctaaacaaa acccaacact ctttaaaaaa 10620
   caataacaaa tccaaatact taacatcata tttacaatat ccaaaattca attaaaaatt 10680
   actaaatata ccaaaaaata aaaaaataaa acttataaac aaaatacgat aactcacgta 10740
   aacctataat cccaacattt taaaaaacta aaacgaataa atcacgaaat caaaaaatcg 10800
   aacttacaat aaaccgaaat aacgccccta cactccaacc taaacaacaa aaaaaaaaac 10860
   tccatctcaa aaaaaaaaaa aaaaaaaaaa aaatataacc tataatcaaa aaaaaaataa 10920
   tttcataaaa acaaaactca aaaataataa aaataataaa taactcaata tactcaaaaa 10980
   tttaaaaaaa aacataaaaa aaatattcat aaaaaaaaaa ataaaaaata taaaaaaaaa 11040
   cccaataaac catctaaaaa taataaatac aataactaaa ataaaaaatt cactaaataa 11100
   aaataacaac aaattaaaca ctacacaatt taaaaatcaa taaatttaaa aacaacaata 11160
   aaactttcca aactaaaaca caaaaaaaaa aaacaatact aaaaaataaa attaactaaa 11220
   cttctataat cccaacactt ttaaaaacca aaacgaaaaa ttaacttaaa ttcaaaaatt 11280
   taaaaccaac ccaaacaata caacaaaact ctatctctac aaacaaaaca aaacaaaaca 11340
   aaacaacttt tacattaact aaacataata acaaacaact ataatcccaa ctcctctaaa 11400
   actaaaacaa aaaaatccct taaacccaaa aattcgaaac tacaataaac tataatcacg 11460
   ccactacact ccaacctaaa caacaaaaca aaaccctatc tctataaaac ctcataatct 11520
   ataaaacatt atcaaacatt ataacatata tctattaaaa tcccaaaaaa caaaataaaa 11580
   aaataaaaca aataaaacaa atttaaaaaa ataaaccgaa tacgataact cacgcctata 11640
   atcccaatac tttaaaaaac caaaacgaat aaatcacaac atcaaaaatt taaaaccaac 11700
   ctaaccaata taataaaacc ccatttttac taaaaataca aaaattaacc aaacgtaata 11760
   ataaacacct ataatcccaa ctacttaaaa actaaaacaa aaaaatcgct taaacccgaa 11820
   aaaaaaaaat tacaataaac cgaaatcgta tcactacact ccaacttaaa cgacaaaaaa 11880
   aaactccatt tcaaaataaa taaataaata ataaaaaaaa aaaaaaacgt ccaaaatttt 11940
   tccaaactta ataaaaacta ctataataaa ttaaaaaata atacccctcc taaaacatat 12000
   ccacatcaca ttctaaccct cacaacctat aaataaaacc ttatttcaaa aaaaaatctt 12060
   tacaaacata attaaattaa aaatctcaaa ataaaataat cctaaatcac ccaaaaaacc 12120
   ctaaatccaa taacaaatat ctttctaaaa cactcaaaaa aataaaaaaa ccatataaaa 12180
   acgaaaacaa aaactaaaat tctacaacca caaaccaaaa aaattataac ccaatcacca 12240
   aaatctaaaa aaaacaaaac aaaactcttc cctaaaacct ttaaaaaaac taccacccta 12300
   ccaacatctc aaatttatat ttatatctcc cgaactataa caaaataaat tttttttttt 12360
   tttttttaaa ataaaatctc actctatcgc ccaaactaaa atacaataac gtaatctcaa 12420
   ctcactataa actccgcttc cccgattcac gccattctcc tacctcaacc tcccaaataa 12480
   ctaaaattac aaacacccac caccacgccc gactaatttt ttatattttt aataaaaacg 12540
   aaatttcacc gtattaacca acataatctc gatctcctaa cctcgtaatc cgcccgcctc 12600
   gacctcccaa aatactaaaa ttacaaacgt aaaccaccac acccgaccaa atatttatta 12660
   ttttaaccca cctaatttat aataatttat tataaaaact aataccaaaa taaaaccaaa 12720
   aaccaaaaaa ttcaataaac cccaaataaa acaaacacga aataaactac accaaaatcc 12780
   atcataatca aatactaaaa actaataata aaaaaaaaat cctaaaaaca ttcaaaaaaa 12840
   taaaatccat tatatataaa aaaacaaata caaaaaatac tatattcttc tcatcaaaaa 12900
   ctatataaac caaaaaacaa tataataaaa tctttaaaat actaaaaaaa aaaaacaaaa 12960
   acaaaaactt tcaacctaaa attctttttt tttttttctc taatccaata taactaatat 13020
   taacctaaaa ttccataccc gataaaaata ttcttcaaaa ctaaaaaaaa aataaatata 13080
   ttaacaaaca aacaaattct aaaaaaattt attaccaaat atacaaaaaa tattaaaaaa 13140
   aattcttcaa ataaaaaaaa aataatccca aataaaaact taaatctata caaaaaatta 13200
   aaaaatacca aaaaatatta aatatataaa aatatcaaaa aaccaaacac aataactcat 13260
   acctataatc ccaacacttt aaaaaaccga aacgaacaaa tcaccaaaaa tcaaaaattt 13320
   aaaaccaacc taaccaacat aataaaaccc catctctact aaaaatacaa aaattaacta 13380
   aacgtaataa aaaattccta taatcccaac tactaaaaaa actaaaacac aaaaatcgct 13440
   taaacctaaa aaataaaaat tacaataaac aaaaatcgca ccactacact ccaacctaaa 13500
   taacaaaaaa aaactctatc tcaaaaaaaa aaaaaaaaaa tatcaaacta aacgcaataa 13560
   ctcataccta taaactttaa aaaaccgaaa taaacaaatc acttaaaccc aaaaatttaa 13620
   aaccaacgta aacaatataa taaaactcta tactacaaaa aaatactaaa attaaccaaa 13680
   cgtaataata catacctatc gtcccaacta ctaaaaaact aaaataaaaa aatcccttaa 13740
   accaaaaaac caaaaattac aataaacaaa aatcatccca ctacactcca acctaaacaa 13800
   caaaaccaaa ccctatctca aaaaaaaaaa aaaaaaaaaa tcaataaaat atttaaaacc 13860
   cacaaaaata aaaaaaaaaa atatcaacaa ataaaaaact tcaacacaac tttaaaacat 13920
   aaaaaataac taaaacctca caactaactt aaaaaaacaa aaaaaactaa aactaaaaca 13980
   accacaaaaa aaaacgtcca cataaaaaac aaaccaaata acccccaacc cccctaaaaa 14040
   atataaataa aacctacaaa caataaaaat aacaaaaata aaaataaaac taaaactaaa 14100
   tacaaaaaaa ctaattacaa ttcaaaatcc cctaatcccc ttcccaacct cattaaacta 14160
   aaaaataccc cccttcctca ccacaaaaca ccaaaaattt attctttata aaaattaaac 14220
   ataaaatact ctaaactcaa aaacattaac cacaacaaaa aataaaatat aatactaaaa 14280
   accaataatc cgtaaaaccc caaccccttc cctacctaaa tcctaaacca ctaacaacca 14340
   aatcacacca ccaccaccct atctacccga ataaaaaatt atcccaaaaa cctcaaaata 14400
   caaacatcta ataaccctac aataaacaaa ccaacctacc acctaatcac cttacaataa 14460
   cacccacaca tccctaaact ccccaacccc attcttaaat ataacccaac aaccaaaaaa 14520
   aactaaacac ttaataaaaa cttcccatat aaaataaacc aaaacataca aattaaatac 14580
   taaaacgact aaatacaata actcacgcct ataatcccaa ctctttaaaa aaccgaaata 14640
   aacgaatcac gaaatcaaaa aatcgaaacc atcctaacta acacgataaa accccgtctc 14700
   tactaaaaat acaaaaaatt aactaaacgt aataacgaac gcctataatc acaactactc 14760
   gaaaaactaa aacaaaaaaa taatataaac ccgaaaaacg aaacttacaa taaaccgaaa 14820
   ttacgccact acactctaac gcgaaaaaca aaacgaaact ccgtatcaaa aaaaaaaaaa 14880
   aaaaaaaaaa aaaaaacaaa ttaaatacta aaaacccaaa aaaaattctc ttaatacaaa 14940
   aaataaaaaa aaactcttta aaaaaaaaca ataatttaca tcctcaaaaa aaatataaaa 15000
   aaatcattaa attcataaaa caaaataaaa caacctaact ttccataacg tctcctaccc 15060
   tttctcttac cttcttcatt ctaaaaataa ctttcttaac ttttccttat tttctacatt 15120
   cctaaacaac tacacctacc cccataaaca ataaacatca cctaaattta ctacattttc 15180
   aaataaaaaa aaaaaccaac tttaaaacta tctacatctc ttctttttcc cgcaccctcc 15240
   acatctaacc catcaaaaaa acttataaac tctacctcca aaacaaacct aaaaaccgcc 15300
   caatttccac taccttacaa ctattacccc cccatctctc actaacaacc ccctactacc 15360
   tctttactac tccccgatta ccaaactcta ttatccacct cacctataac aactaaaatc 15420
   accctataaa atattactca aatcctaaca ctctttaatt caaaatcttc caataacttt 15480
   ccatcgtaaa taacaaaaaa tccaataacc ccaaacttct ctttaaactc atttactcca 15540
   cccttcgcca ataaccttcc ccgaaacgtt ctccaaaatc ctccaaaaaa ccccaaaact 15600
   ttctccctcc cttcactcaa actactactc aaatatcacc tccttaaaaa aacccaccct 15660
   aacaacccat ctaaaataac aactactata actctatcca ctacccgtcc aaatacctat 15720
   taaacacctc ctacacctcg aacaaaaacc ccatcaacca aatccctacc tactttattc 15780
   actactaatt ccccactccc caaacaacac ctaacacata aatatttact aaacaaatac 15840
   ataaaatact aaactaaaaa aacacaaaaa aaaaaaaata attaaccaaa aaatcattac 15900
   aaaaatatat tatttatacc gaaaaactca cgtctataat cccaacactt taaaaaacca 15960
   aaataaataa atcacttaaa ctcaaaaatt cgaaaccaac ctaaacaaca taacaaaacc 16020
   ccatctctac aaaaaaaaat acaaaaatta accaaacata ataacaccac ctataatcct 16080
   aactccttaa aaaactaaaa taaaaaaatc acctaaacct aaaaaacaaa aactacacta 16140
   aactataatc acactactac actccaacct aaataacaaa ataaaacgct atctcataaa 16200
   aaaaaaaaaa aaaaaaaaaa aaaaaaaaaa aaaaaaaaaa aaacaaaaaa aaaaaaaaaa 16260
   aaaaaaaaaa aaaaaaaaaa aaaacaatat aaacaatata ttatttataa ttaaaaaaat 16320
   aaataacaaa aaaaatccaa aatactaaaa aaataaaaaa aacgaaacaa aaaacactac 16380
   ttttcattac gcatcttcta acaacaatta aatattttaa aacataacca taaactccat 16440
   tatcaaaaat aaaacatttt aaataattta aaattattta tataattttt ataaccttat 16500
   cctaaaattt cttcattttt caatatacta aaaacctcaa aaaaaccaac gaacctaacc 16560
   cctattaacc tctaaactat aacccctcac aaaaaaaaaa aatattattt actcttatcc 16620
   aatcctaatt aaactaaact ccgaaactta caacctaaat aaaatcctaa acccgacaac 16680
   gaaaataaaa aaaaaccaca aaacccgaac taactaaaaa aatactaaac aatccaaacc 16740
   tcccaaccct accccaaata ctacaaccgc aaccgctaca actaataaaa ccctaaaaaa 16800
   ctaacaaacc gctacctaac ctcaaaactc caaatactac ctccacctcc ccatcaaata 16860
   cctcaactcc aaactccaaa aaacgactaa cacttcctta caaacaaaat actcctcccc 16920
   atccccacac ctcaaaaaca ctccccaaaa aaacctaaat aactcaacac cccatacacc 16980
   ccatacaaaa ccccatacac tcaaccaaac tccaaaacaa ctctccccac aaacccaaaa 17040
   taccgcctac aatataaata acaccttacc ctaaacccaa ataaaaaaat tcattaatct 17100
   cctcaacaaa aacctccaac ttctcctcaa caaaaacctc taactattta attactaaaa 17160
   taaaaaaatt tcccataaaa cccaacaccc aaccctcccc accaaaaaaa cgaaaattcc 17220
   ttatcccaac aaacctcaac taaaatcaaa aaataaaatc tttaaaattt tataactaaa 17280
   aatccctaaa caaaaaacct ccctacctcc actcaaacac acacaccaca ctctaataca 17340
   cactaatacc atacaaatac acactcgtac gctcataaat actcacgtat acccttaaat 17400
   atacaaacac atacataccc aactaccttc taaccctaca aaaatccact cctatataac 17460
   gtatatatac gtatttacac aaaaacaccc ctcacaccca tatacactaa caaaatactt 17520
   tccaaactcc tttaacataa acaaaatcct tttattaaaa aaaatataaa aaacatacat 17580
   ctcatatata aactaaacac aatacttacg aaaaaaataa aaaacgcaaa acctatactc 17640
   aaaataatac ctactcaacc ccttccccaa cccacgaaaa aaaaaacaaa aatctaatat 17700
   aatcgttact taacctctcc tacccaacgt aaaatacaaa aacaaatcta cctaaactca 17760
   ttcaaaaaaa aaaaaacaaa atacatcctc aaactacgta aaaaaccacg aactataaaa 17820
   accgcaccct aaacttctcc ctccatatca cccctcccat cctaccctta caacccctcc 17880
   ccctcatccc aaaacacatt aatttttaat ttctaaaatt tataactccc tctcccgaac 17940
   ttcctaactt accaactata ataatacccc acatccaaaa ctacccaata taaacaaaaa 18000
   aaaaaatcta aaatactcct cgcacatcct aaaccccaca acccatctcc cctacctcta 18060
   aatcaaaaaa ataaacaaaa aaaacaaaaa acaaacccac gataaaaaac gccccaaaaa 18120
   aaaaaaaaat aattctctaa aaccacaatc tacccacttc aaaattctaa ttaccaaaaa 18180
   caaaatccta ctccccaaac ccaacccaca catatccctc ccacgaaaac tacacaaact 18240
   aacccttccc cctattccca ccccaaaaaa caccaaaaac tcacgactcc aaaaccaaaa 18300
   ccatacccaa ctaaccatac caaacaaaaa aaaactaaca tattccctaa cttacaaccc 18360
   tatccccccc gacctaacaa aaaacataca taacgacaaa caaaacaccg aactaacatc 18420
   acaacactac taaatacccc ttcccttcac caaacacctc ctatctaccc atcactccct 18480
   acttccaacc tccaacaaaa caatttaaaa ctaaacaaaa cccattaacc tactctaaaa 18540
   ataaccatac taaccccctc ctcaaccaac tacactacca aacctaccca cgaaaaacct 18600
   ctacgaaact ttcttaaaat aaaaaaaaaa caactatacc aaaccaaaac caaaaaaacc 18660
   taactcctaa atacttaaaa aaaaccgaac tatttctata acgactaata ccaaaaaaac 18720
   taaaaaccaa aaacccctaa ctaaaatacc aaaattccac aatacttttc tcataatatt    18780
   aaataactat ccccaaatcc cccttacaaa aaacaaaacc taatacccta aaaaataaac    18840
   aatatacatc taaatacaaa cctcactcta aaccaatcct cgaaaaaaaa aacaactaaa 18900
   aaactctccc acctacacga aataacattc ctctttttaa cacgcctaaa aaaaaaaaaa 18960
   aactaaccta actcctctat aattaaacaa aaattaacct ccaccaatac cctatattcc 19020
   ccctaaccta aatccaaaac aaacaatatc catttcaaaa aaacattaac tcactaaaac 19080
   aaaaataaaa ataaatctaa aaccttattt ctaaaacaac caaaaaaata aaaaaaaaaa 19140
   acacataaac aacccccaac acctctacct acaacgactc cgataccccc caccccaacc 19200
   ttaccccaac aaaataacca cccacttcaa aattatcaac aaaaaaacct actaaaaaaa 19260
   tcttttctcc caaaacattc tacctctctc caatctctac aataaaaaaa ccacccaccc 19320
   tcaaacaaaa aaataaaact aaaacattct aaaaattcaa ccttctacct aataatcctt 19380
   aaacccctaa aaaattcttc ccacataaaa cttatcttct tttactttca aaaaaaaaaa 19440
   acaaaaaaaa accaacaaaa actaactaac catatcttaa cgctttacac atcaccctaa 19500
   aatttcctat tcacacttac ttcaccaaaa caaaaactcc ttaaaaacaa aaattaatac 19560
   cctatttatt catctatttc ctaaaaacac tataacccac tctctttaac aaacttctcg 19620
   taactacaaa acactacctt tataataaat aaacaaaaac aaaatctaaa aaattcaatc 19680
   taaaaaaaaa ctcgaaaaat actaacataa acacaaataa caaaaaataa ccacccaaac 19740
   cgacccaaaa acctacgaac acaaatcact cgcatattta caaaaactac catacaaaac 19800
   taaataccat acaaacctaa aaactaactc aacgtaaaac ttcaaaaaca catcctaacc 19860
   cccaacctac tcaatccgat aacctaccct ccacctccat acttccctta acgattacac 19920
   aactcctcaa actaaacttc tcataactca ataaacaacc ctcaaaaaat aaattcccac 19980
   aacgaacaac tacgctaacc acccccaacc cataacccca aaatctcaaa cccaatccaa 20040
   aactaaaatt ctcaacctac aacaaaccat aaaacccgaa cccaactcta actcttacaa 20100
   cccaccctaa aaacgaaaaa tcatctctac cataaatacc tacctataac ctaaatacca 20160
   aaaacaaaaa taacaatcta taacctatac ctacctttat tctaaaacat aaccaaaaaa 20220
   catcctaaaa cataaccaaa aaacatcccc ccttccccta ttttctaaaa accactaccc 20280
   aacccaaaac taataaccca cgtaacctta ctaaccaaca aaaaccaaaa aacccaccta 20340
   atacgcaaaa cctcaaatcc ttccaacaac caaaacccct caacttcctc tacgcctaaa 20400
   aaccaaaccc caacaccaaa aaacaaaaaa acgcttttca acacctaaac aaaaccctta 20460
   aaacctccca aaaaaatctt tctccactcc ctattcctaa actaaaacta aactctaaaa 20520
   aatccaaaaa tctacccaat ttaaaaacta aaaaaaaaaa cgctcaccgt taaaaaaaaa 20580
   acgctaaata aaactaaaat acctaaaaat ataacacaac ctcttcccca ttcttaacct 20640
   ataaccccct ccccacgcaa acctttccca cctccgcacc ctcactcgaa aaatatcgcc 20700
   gctcaactaa ctaaaacgaa ctctcccacc acataaaaca ccgacacaac ccgcaacccc 20760
   tccctacaac cctcactacc ccgacgaaaa aaaaacacaa aaacctctac tctcatctaa 20820
   ataaatccga aacacgtata atcccgcccg tcccgaccga aaaaaatcac cctttcccga 20880
   tcaacaaaca caaaaaccgc gaccgcaaac aaaaacgcgc cgcaaactcg ccaaaaaaac 20940
   gaaacaaccc gcgccgcacc ccctccccaa accgaacgaa cgtacccgac cgcgaaatcc 21000
   ccgcgacgcc ccgaaaacgc tcctacctaa cccgcgcgaa cctccttctc caactcgcga 21060
   taacgattat accacacgaa ataatacaac gaatacttac cctcgaaccc cttcctaacg 21120
   aaaacgttaa cgaaaatcat cgcccgctcc tcataccgaa accgcgacgc ccgaccgaaa 21180
   aaaaaaaacg acgccgaaac cgaaacccga aaccgcgaaa cgaaaaaccg acgctaccta 21240
   ctcgcgaaac tacgactcga acatatacga acgacgacga acgaaaacga cgcgcccgac 21300
   gcgatacgaa cgacgcgaac gacgaacgtc ctcgcgaaac gacgacgacg aacgacgcga 21360
   aaaaaccccc acccgcccta aaaaaaaacg aacactcgac gacgctacgc gaactcgcgc 21420
   tcgcctccca cacccccgca cacaaaaacg ccgctcgacg aaaaaaacaa acgcccgcaa 21480
   cgaacgcgaa aacgcacgaa acgcgaaccc cgaaaacgcc gccgcaaaaa cgcgcgcccg 21540
   caacccccga atccccacaa ctacataacc cgaccctcaa cccgaacgac cgacgcgaaa 21600
   tacgaaaata taaaaataat aaaaaaaaaa aaaaaaaacc aaacgccccg aataaaactt 21660
   atcaactcaa ccccgcgaca tcataaaatt tataatccgc ctcgtaaaac taaaaataac 21720
   aaataaccga aaaaaatcta cactatcccc gaatctcgta aaaaaataaa cgaaaaatca 21780
   aacctaaccc aaacgcgaac gaaaccctaa cgccaaataa actaaaacgc acctcccccg 21840
   ctaacatttt accttttact aaaaaaataa aaaaacacga acctaaatcg aacgtttaaa 21900
   aaacgaaata aaaatataat cttcaaaatt aaacttaaat cccaaaaacc ataaaaaaaa 21960
   atccgaattc ccaaaaaaaa acgtctaatt aaaaactcct aaaaaaaata taaaacatca 22020
   ataccaaaaa tcattaaaac aaccaaaaat ctaaccccta aaacatttct aaaaaatctt 22080
   ccctacccta ttaaaaaatt aaactccatc ttccacacat atcattaaaa acaaaatatc 22140
   caataacgcg aaaaaaataa tccctttccc cccgcacact atccccaaca aaaaccaaaa 22200
   taaaattaaa caaaaaaaac aacaaataac aactccgttc ctccacccaa ctaacccttc 22260
   aaaaaaaaaa ataactaatt ccaaaaacta tctccttcct aaataacccc aaaataaaaa 22320
   aatacttcca aaataatccc tacaaaaaat ataataaaaa ttaaattaac ctctcaaacg 22380
   ccaattacaa aacctaaaaa aaccaaaaaa cttaaaccaa tccctaaaaa aaaaaaacta 22440
   taatatcaaa aaaaccaaaa ttcgaataac tccaactacc taaccttaaa aaaacaaact 22500
   acttaatttt tccgactttc tcgatccact ccccttcaaa ataaaaatct aaatactacc 22560
   tcctaaaata aactaaaaat aataactacc ctaaaaacaa cgcctccctc ccccaaccaa 22620
   acgaaccgaa acaaataacg aaaccaaaca cgataccttc ttaaacccac ctcaaaacaa 22680
   aacaaaaata aaaaattcca caccgaccct acttaaaaca acaatccaaa aaatatttat 22740
   ataaaaaata ccaacaaaaa cttacaaact ctctcaaatt tctttatata atcttccctc 22800
   cttcctcctc cacaccctat aataccaaac cccaacgaaa aaaaataaaa aaaaaacgaa 22860
   ctacctcgcc cctttctccc gcctaaaaaa aaccaactaa cgtctccgaa aacccgaaac 22920
   cgctacccgc ctacaccacc aaaaatcaac aaaatatcgt aactacaacc aatacaaaaa 22980
   aaaaaactaa cgcgattaaa aaacatctaa aaataaccga aaacatacct atacatttcc 23040
   ttattaaatt atttttcgaa ccttatatat aattccacta taacaatcct acttcctaaa 23100
   ataataacat ctccacgaaa aaatccaaaa acccttttat ctccaatccc aaacccaaaa 23160
   atttaactat aacgaaacta aaaaaaacaa aatttaaacg atatatccgc gccaaaacca 23220
   ctcccaaata atctctaaac caaccaaaaa ataaaaaata aaataaaaaa ataaacaaaa 23280
   taaatcatcc ttcttcccca acaaaaccca aaaatccgaa aacaaaaaaa attcaaaata 23340
   aattataaaa aattatcgat tataaaaaac tatcccctaa acgaccgtat tcttttttta 23400
   ccaaacgacg ataaacacta aacccaaatc gcttacgctt cgacccgcgc tctcaactac 23460
   cacgtactcg aaacgcgccc acacaacgaa aaaacaaaaa caaccaaacg acaaaactac 23520
   caacaacctc ctcgacattt aataaataaa attccataac taaaaatcga tcgaaaataa 23580
   acgaacgacc accgctcgta aactaaacgc ctaacacgaa ctacctaacc actcctcccg 23640
   tctcactccc cccgcccctt acccccttcc cgctaaaacc accaaaccca taaactctaa 23700
   taaaaaaaca actcgaaacg acaaataata ctccccccta cacaccaact aaatatcgtt 23760
   acttacaaaa accccaccaa ctaaacgact aactatcgaa aaaaaaaaaa cctacaaaat 23820
   ttttttaaaa tacaattata aataaaaccc ataaatcgcg atttcaaaca aacttatcaa 23880
   tctaaaccgc aacaaaaaac gataaaaaca actttacata tatttcgaat aatataactc 23940
   ataaacatta cttatctccc ctaaaaaaaa aacctaccca cccacaaatc actaccgctc 24000
   taacaccgtt aaaataatcg accaacttaa acacccctac caaaaaacta cccccatctt 24060
   aacaaacaaa aatctctaac tcttaaaaaa cccctcctca attatcacca ctaacctaac 24120
   ccccaaatct aaccctaaaa acctctaccc tctccccaca aacaaaatac ccgaaccttt 24180
   tactactaca aaaattaaaa attaaataca cgcgcgacac acgcgtataa aaacgcatac 24240
   tcaatacgaa aactacgctc atatacgacc tctacctaaa ctccctaccc cccaacccac 24300
   ccacgtccct cacctacctt acctttacct acctcgtcgt ataaaatcca aaaaaaaaaa 24360
   ccgtcaccta aaacgacccc tctatattat atacaccctt tctaaacaca cacacctcta 24420
   ataacccaaa aacaccgtaa atatatataa aacgaataac aaataaatat aaatacacac 24480
   aaacacaata atcactcctc gaaaaatctt taaaaaccgc ccaaattctc tcctctctaa 24540
   ataaaaaaat acataaaaaa ataaatatat ttttaatccg aaaacaaaaa aatacactac 24600
   ctaacctttc taacacctaa acgtccatat tattaacaat aaacgtattc aacaaaaata 24660
   taacacatac acacacaccc ctaaacacac aaatataaac acctacgaaa caaaaacgca    24720
   cacctcaccc caaccaaaaa taaacgaata ccccaaaaca caaccccttt aacctaaaac    24780
   gaaacttaaa cctccaaatc cttcttccct aatccccacc ctcgccgtcc gtaaacccaa 24840
   cgtcccccga tccaccccac gcgaaaaata aaaaaaaaaa aactataaaa ataactcaca 24900
   ttacctccga accaaacgaa cgctacaacg aaaaccaaaa ccgcaaacca cgctacccgc 24960
   cgaaaccgaa catctaccgc gaccccttta aac 24993
<210> 9
   <211> 24993
   <212> DNA
   <213> Artificial Sequence
<220>
   <223> G to A (bis2 rc)
<400> 9
   acttaaaaaa accgcgacaa atacccgacc ccgacgaaca acgtaaccta cgaccctaac 60
   ctccgctaca acgctcgcct aacccgaaaa caatataaat caccctcaca accccctccc 120
   cttcactttc cgcgtaaaat aaaccgaaaa acgctaaacc cacgaacgac gaaaataaaa 180
   attaaaaaaa aaaaacttaa aaacccaaat cccgctccaa atcaaaaaaa ctatacctta 240
   aaatacccgt ctatctctaa ttaaaataaa atatacgtcc ctatcccgta aatatccaca 300
   tctatatatc caaaaatata tatatatata tcacactctt actaaatacg cccactacta 360
   acaatataaa cgcccaaata ccaaaaaaat caaacaatat attcccctac ctccgaacta 420
   aaaatatatc tatctctcca tacatccttc catccaaaaa aaaaaaaatc taaacgattt 480
   ttaaaaacct cccgaaaaat aaccactata cctatataca ctcacatcca cttatcatcc 540
   gccttacaca cacccacgat atttctaaac caccaaaaat atatatatcc aaaaaaaata 600
   catataacat aaaaaaaccg tttcaaataa cgacctttct tcctaaactt cacacgacga 660
   aacaaacaaa aacaaaacaa ataaaaaacg taaataaact aaaaaacaaa aaacctaaat 720
   aaaaaccgca cataaacgca acccccgcac taaacatacg cccccatacg cgtataccgc 780
   gcgtataccc aacccccaat ccctacaaca acaaaaaacc cgaacactct acttataaaa 840
   aaaaaataaa aatccccaaa accaaaccta aaaaccaaat caataataac aattaaaaaa 900
   aaattctcca aaaatcaaaa actcctactt attaaaataa aaacaatcct ttaacaaaaa 960
   tactcaaatt aatcgattat cccaacgata ccaaaacgac aataatttat aaataaacaa 1020
   actccttccc taaaaaaaac aaataatact cataaatcac accacccgaa atacatacaa 1080
   aactacctcc atcgcctttt actacgaccc aaattaacaa atttatttaa aatcgcgatc 1140
   cataaacttt atttataatt acaccttaaa aaaatcttac aaacctcccc tccctcgaca 1200
   accaaccgct caactaataa aacttttaca aacaacgata cctaactaat atataaaaaa 1260
   aaacaccacc taccgccccg aactactctc tcaccaaaat ccataaactt aataacctca 1320
   acgaaaaaaa aataaaaaac gaaaaaaata aaacgaaaaa aataaccaaa caacccgtac 1380
   caaacgctca atccacgaac gataaccgcc cgttcacccc cgatcgaccc ccaactataa 1440
   aatttcattt attaaatatc gaaaaaacta ctaacaaccc taccgcttaa ctatctttat 1500
   ttctccgcta tataaacgcg tctcgaacac gtaacaacta aaaacgcgaa ccgaaacgca 1560
   aacgacctaa attcaatatc taccgccgcc taacaaaaaa aaaacacgac cgcccaaaaa 1620
   acaattcccc acaatcgaca actccccaca atccatccta aactccccct atcctcgaac 1680
   tcctaaactt tactaaaaaa aaaaaataat tcactctact tacccctcca ccccactccc 1740
   catctcctaa ttaactcaaa aactacctaa aaataaccct aacgcgaaca catcgtccaa 1800
   actttatttc ccccaacccc gtcacaacca aacttctaaa cctaaaacta aaaataaaaa 1860
   aatttctaaa ttcccccgta aaaatatcac caccccaaaa aacaaaacta ctacaataaa 1920
   atcacacaca aaattcgaaa aacaatttaa taaaaaaata cacaaacata ctcccgacca 1980
   ctcctaaata cccctcaacc gcgccaaccc cctcccctac actaactaca accacgacac 2040
   tctactaacc cctaataata caaacgaaca acgatcccga acccccgaaa acgccaacta 2100
   actcctccca aacgaaaaaa aaaaacgaaa caacccgctc ctccctcatc tcttcccgct 2160
   aaaacctaac accacaaaat ataaaaaaaa aaaaaaaaaa aaccacacaa aaaaacctaa 2220
   aaaaatttac aaacttttac taacacctcc cacacaaaca cttcttaaat tattatttta 2280
   aataaaaccg atataaaatc ccttattcct accttattct aaaataaact caaaaaaaca 2340
   ccgtacctaa tcccgccacc tatctcgacc cgcctaatta aaaaaaaaaa acgctatttc 2400
   caaaacaatc attatcctta actcacccca aaaaataata tctaaattct cattttaaaa 2460
   aaaaataaac cgaaaaaatc gaaaaaatta aacaatttac tcttttaaaa tcaaacaatt 2520
   aaaactattc gaactctaat ctctctaaca tcacaatccc ctccctccaa aaactaatcc 2580
   aaacctctta atccttccaa accttacaat taacgcttaa aaaaccaatt caacttttac 2640
   cacaccctct acaaaaacca ttctaaaaat acctcttcac cctaaaatca ttcaaaaaaa 2700
   aaacaatctc taaaatcaat cacttccctt cctaaaaaac caactaaata aaaaaacgaa 2760
   actaccacct actacttttt ctatccaacc tcactctaat tcttactaaa aacaatatac 2820
   gaaaaaaaaa aaaccatctt tcccgcgtta ctaaacattc tatttccaat aacatatata 2880
   aaaaataaaa tttaattttc taacaaaaca aaaaaaactc ctcaaaaata ttccaaaaac 2940
   caaaccccta actactccaa taacccttaa tactaatacc ccacaccttt ctcaaaaatt 3000
   cccaatcaaa cgctctcttc taaaaatccg aatccttctt cataacccct aaaacccaaa 3060
   ctcaattcta aaaaccacac ctccaccccg tctcccaaac gctcgaccta aacccgtacc 3120
   cccccacttc cccaacaaaa aacaaaatac caacgaaaaa aatacgtccc aacccaccta 3180
   acgtcaaaac cccgcccgcg tctaaaccaa acctaactcc ccgcccactc tcccacgaaa 3240
   tccgaaaaca atacaaaccc tcttcgacca cctaccaccc ccaatttcac gaaacgaact 3300
   acaaacccca taataccgcg aaactaaact aacaaactct acccgaaacg cctaatcccc 3360
   ttcctctctc cccatcatcc ccacatcccc gtatcccgcg ccgaccgctc gaactaaaaa 3420
   ccgaactata caactataaa aacccgaaaa ctacgaacgc gcgtccctac gacgacgtcc 3480
   ccgaaacccg cgtcccgtac gcccccgcgc ccgctacgaa cgcctactcc ctccgccgaa 3540
   cgacgtcttt atatacgaaa atataaaaaa cgaacgcgaa tccgcgcaac gccgccgaat 3600
   acccgctccc tcccaaaacg aataaaaacc tctccgcgcc gcccgccgcc gccgcctcgc 3660
   gaaaacgccc gtcgcccgcg ccgcccgcac cgcgccgaac gcgccgcccc cgcccgccgc 3720
   cgctcgcaca tacccgaacc gcaaccccgc gaacaaacaa cgccgacccc ccgccccgcg 3780
   accccgaacc ccgactccga cgccgtccct cctccccgac cgaacgccgc gaccccgaca 3840
   taaaaaacga acgataatcc ccgccaacgc ctccgccaaa aaaaaacccg aaaacaaata 3900
   tccgctacac tacctcgtat aacacaaccg ccaccgcgaa ctaaaaaaaa aaatccgcgc 3960
   gaaccaaata aaaacgcctt cgaaacgccg cgaaaacccc gcgaccgaac acgcccgtcc 4020
   gacctaaaaa aaaaatacga cgcgaactat cccgcctccc taacgaattt acgacgcgtc 4080
   tttatttacg accgcgactt ttatacttat taaccgaaaa aaaataatcc cctccgaccg 4140
   aaacgaacga aaccacacgt atttcgaact cacctaaata aaaataaaaa cctctatacc 4200
   cctcctccgc cgaaacaata aaaactacaa aaaaaaacta cgaactatat cgatattcca 4260
   tataataaaa aaatccgccc caaccaatta aacgacgaca cctcccgaat aaaaatacga 4320
   aaataaaaaa aacttacgta aaaaaaaaac cataaatcaa aaataaaaaa aaaactatat 4380
   cacactccca aacaccccaa ccccacccaa cgccctctcc ccaacgataa acgctttctc 4440
   ttttaactct caaactaaac aaatccttaa atctctcaaa acccaacccc aactcaaaaa 4500
   caaaaaataa aaaaaaaccc tcttaaaaaa ccccaaaaac cttatccaaa tactaaaaaa 4560
   cgctctccta ccctctaata ctaaaaccta atttctaaac gcaaaaaaaa ctaaaaaacc 4620
   ctaactacta aaaaaactta aaattctacg caccaaataa actccctaat ttctactaat 4680
   caataaaacc acgtaaatca ccaactttaa actaaacaat aatctccaaa aaacaaaaaa 4740
   aaaaaaaata tcttctaacc atacttcaaa atatcttcta accatacttc aaaacaaaaa 4800
   caaacacaaa tcacaaatta ccacttctac ccttaacacc caaaccacaa ataaacactc 4860
   ataacaaaaa taaccccccg cctccaaaat aaactacaaa aaccaaaact aaacccgaat 4920
   cccataactt actacaaact aaaaacccca attttaaact aaatctaaaa ccttaaaacc 4980
   ataaactaaa aataattaac gcaattaccc gctataaaaa tttaccttct aaaaactact 5040
   tattaaatca taaaaaattc aatctaaaaa actatacaat cgccaaaaaa aacataaaaa 5100
   taaaaaacaa accatcgaac taaacaaact aaaaatcaaa atatatctct aaaatcccac 5160
   gctaaaccaa cccccaaacc tacataacat ccaactctac ataacaatcc ctataaatat 5220
   acgaataacc tatacccgca aacctctaaa ccgatctaaa taaccacttc ctattaccta 5280
   tacctatacc aacactctcc gaatttctcc tcaaactaaa ctcctcaaac cctatcttta 5340
   tttatctatc ataaaaacaa tactctacaa ccacgaaaaa cctattaaaa aaaataaatc 5400
   acaatacccc caaaaaacaa ataaacaaac aaaacatcaa cccctaccct caaaaaatct 5460
   ctattctaat aaaacaaata taaacaaaaa attccaaaat aatatacaaa acgctaaaac 5520
   ataaccaacc aacctctact aactcttctc tactttcctt ccctaaaaac aaaaaaaaac 5580
   aaacctcata taaaaaaaac ttcccaaaaa tccaaaaatc actaaacaaa aaactaaatc 5640
   tccaaaatac ctcaatccca ctcccttacc taaaaataaa taatcctttt actacaaaaa 5700
   ctaaaaaaaa acaaaatacc ctaaaaaaaa aaacttctcc aacaaacctc tctactaata 5760
   atcctaaaat aaataaccat cttactaaaa taaaattaaa ataaaaaaca ccgaaaccgc 5820
   tacaaataaa aatattaaaa actactcata tatttctctc tcccacccct ctaactacct 5880
   caaaaacaaa atcccaaatt cacccctacc cctaccccaa taaattaata tctccttaaa 5940
   ataaacacta cctatcctaa atccaaacca aaaaaaatat aaaacactaa taaaaattaa 6000
   cccttattca accacaaaaa aaccaaacca atcctctcct tccctcaaac gtaccaaaaa 6060
   aaaaaatacc actccgtata aataaaaaaa ttccctaact atctcccctc tcgaaaacta 6120
   acccaaaata aaacttatat ccaaatatac actacctact ccccaaaaca ccaaactcta 6180
   ttttctacaa aaaaaaccta aaaacaacca cctaacatca taaaaaaaac actataaaac 6240
   cctaacatcc caaccaaaaa tccctaacct ctaatccccc taataccaac cgccacaaaa 6300
   acaactcgac tttcctcaaa cactcaaaaa ccaaatttcc ctaatcttaa cttaacataa 6360
   ctacctcttc ttcactccaa aaaaaccccg caaaaaccct ccgtaaacaa acttaacaat 6420
   acaactaact aaaaaaaaaa ccaacataac caccttcaaa acaaattaat aaactttact 6480
   caaccccaaa ttactctact aaaaactaaa aacaaaaaat aataaacaaa taaaaaatac 6540
   ctaataaaaa aaaaaaacac ccaacaatac tataatacca acccgatacc ctacttaccg 6600
   ctatacatat tctctaccaa atcgaaaaaa acaaaattat aaaccaaaaa atataccaat 6660
   tcccccttac ctaacataac caactaaaca taatcctaat tctaaaatcg taaattccta 6720
   atattcccta aaataaaaac aaaaaaaaaa attaacctat acaactcccg taaaaaaaac 6780
   atatataaac taaacctaaa aaacaaaact ctacccctaa caaccaaaac cctaaaataa 6840
   acaaattata atcccaaaaa atcattttcc ttctccctaa aacgttctcc accgtaaact 6900
   tacttcctac tttccctatt cacccttcta acctaaaaac aaaaaaaata aattataaaa 6960
   cccaaaatat acgaaaaaca cctcaaaccc ctccctttac ctacactaaa caatcctaaa 7020
   tataaaacat caccacaact aataaactaa aaaactcgaa aaaaaaaacc acaaacccca 7080
   aaaaccaaaa atcaatatat tctaaaataa aaaaaaaaaa ctacaaaaac aaaataaaaa 7140
   aaataacata aaaaaaaaaa tccaaaatac gatccccaca acccgtaacc tcctacgcaa 7200
   cttaaaaata catcctacct ccctcccctt aaataaactc aaacaaacct acccctacac 7260
   cccacgctaa acaaaaaaaa tcaaacaacg actacaccaa actcctacct tcctcctcgt 7320
   aaactaaaaa aaaaactaaa caaacaccac cttaaacaca aactctacgt ccctcacctt 7380
   cctcgcaaac actatattta atccatacat aaaatatata cttttcatac ttcccttaac 7440
   aaaaaaattc tactcatact aaaaaaattt aaaaaatacc ctactaatac acataaatat 7500
   aaaaaatatt cctatacaaa tacgcacaca cacgttacac aaaaataaac tcctacaaaa 7560
   ccaaaaaaca actaaacata catatactta tacacttaaa aacacacgta aatacctata 7620
   aacgtacgaa tatatatcta tataatacca atatatacta aaatataata tatataccta 7680
   aataaaaata aaaaaactct ctacccaaaa acccccaact acaaaactcc aaaaactcca 7740
   ccttttaact ccaactaaaa cctactaaaa taaaaaatct ccgttctctt aataaaaaaa 7800
   actaaatatt aaatttcata aaaaaccttt tcattccaac aattaaataa ctaaaaacct 7860
   ctactaaaaa aaaactaaaa acctctacta aaaaaattaa taaattctcc tacctaaacc 7920
   caaaacaaaa taccacttac actacaaacg acactctaaa cttataaaaa aaactactct 7980
   aaaacttaac taaatatata aaattctaca taaaatatat aaaatactaa accatccaaa 8040
   cctccctaaa aaatactcct aaaatataaa aataaaaaaa aacaccctac ttacaaaaaa 8100
   ataccaaccg ctctctaaaa cctaaaacta aaacacctaa taaaaaaata aaaacaacat 8160
   ctaaaaccct aaaaccaaac aacgacctac caactcccta aaatcttatt aactacaacg 8220
   actacgacta caacatctaa aacaaaacta aaaaacctaa attactcaac acttccccaa 8280
   tcaactcgaa ccctataacc ctctcccact cccgctaccg aactcaaaac cccacccaaa 8340
   ttacaaaccc cgaaatccaa cccaaccaaa attaaacaaa aataaacaac actccccttc 8400
   tctataaaaa accacaactc aaaaaccaac aaaaaccaaa cccgctaatt tttttaaaac 8460
   tcccaatata ttaaaaaata aaaaaattcc aaaacaaaac tataaaaatt atataaataa 8520
   tcttaaatta tttaaaatat ttcattttta acaataaaat tcataatcat actttaaaat 8580
   atttaactat tactaaaaaa tacgtaataa aaaacaatac tccttactcc gcctctccca 8640
   cctccccaac actctaaact ccttttatca tttacctccc taattataaa caatatatta 8700
   tttatactac cctctcccct ccccttctcc ctccctccct cccttcccta cctccctccc 8760
   tttctctttc tttcttcttc ttttttttct tttttcataa aacaacgtct cactctatca 8820
   cccaaactaa aatacaataa tataatcaca actcaataca acctctacct cccaaactca 8880
   aataatcctc ccatctcaac ctcccaaaaa actaaaacta caaataatac caccatacct 8940
   aactaatttt tatatttttt tttataaaaa taaaatctta ccatattact caaactaatc 9000
   tcgaactcct aaactcaaat aatccaccta ctttaacctc ccaaaatact aaaattacaa 9060
   acgtaaaccc cccgacataa acaatatact tctacaataa tttcttaatt aattaccttc 9120
   ccctccttat accccctcaa cctaatactc tatacattta tctaacaaat atttatatac 9180
   caaatactac ctaaaaaata aaaaaccaac aataaacaaa acaaacaaaa acctaactaa 9240
   taaaacttct attcgaaata caaaaaatac tcaacaaaca cttaaacgaa caataaataa 9300
   aaccataaca actattacct caaataaact atcaaaataa acttttctaa aaaaataaca 9360
   tttaaacaac aacctaaata aaaaaaaaaa aaaaacttta aaactctcta aaaaatccta 9420
   aaaaacgttc cgaaaaaaac cattaacgaa aaataaaaca aataaattca aaaaaaaatc 9480
   taaaaccatt aaattcccta ctacctacga taaaaaacca ctaaaaaatt ttaaactaaa 9540
   aaatatcaaa acctaaataa cacttcacaa aataacttta actaccataa ataaaataaa 9600
   caataaaacc taacaaccga aaaacaacaa aaaaacaata aaaaactacc aataaaaaat 9660
   aaaaaaacaa caactacaaa ataataaaaa ctaaacgact tctaaatcta ttttaaaaac 9720
   aaaacctaca aatttttcta ataaattaaa tataaaaaat acgaaaaaaa aaaaaaatat 9780
   aaacaatttt aaaattaatt tttttttcca cctaaaaata caataaatcc aaataatatt 9840
   tactatttat aaaaacaaat acaactatcc aaaaatacaa aaaacaaaaa aaaattaaaa 9900
   aaatcacttc caaaataaaa aaaacaaaaa aaaaaacaaa aaacgtcata aaaaaccaaa 9960
   ctatcccatt ttatttcata aattcaataa tctttttata tttttcctaa aaatataaat 10020
   tactattttc ccccaaaaaa ttttctttta ttccctacat caaaaaaatt tcctctaaat 10080
   tcttaacatc taatttattt tttttttttt tttttttttt ttttttaata cgaaatctcg 10140
   ctctatcccc cgcgctaaaa tacaataacg caatctcgac tcactacaaa ctccgcctcc 10200
   cgaattcaca ccattctcct acctcaacct cccgaataac tataactaca aacgcccgcc 10260
   accacgccca actaattttt tatattttta ataaaaacga aatttcaccg tattaaccaa 10320
   aataatctcg atctcctaac ctcgtaatcc gcccacctcg acctcccaaa aaactaaaat 10380
   tacaaacgta aaccactaca cccaaccgtc ttaacatcta atttatatat tttaatttat 10440
   ttcacataaa aaactttcat caaatatcca atttctccta actattaaat catatttaaa 10500
   aataaaacta aaaaactcaa aaatatataa atactactat aaaataatca aataacaaac 10560
   taacctattc attataaaat caccaaatat ctatattcta aaatctttaa aacaacttcc 10620
   tactcgaata aataaaataa taataatata acctaactac caataattca aaacccaaat 10680
   aaaaaaaaaa ctaaaatttc acgaatcact aactttcaac accatacctc atcctctact 10740
   ataaccaata ctcctaaatc caaaacatct catacccaat ttctacaaaa aataaacccc 10800
   taatatccta taataaaaaa aaaaaacatc tcccaactta ataaaactaa aaaaaaaacc 10860
   aaaaaatcct aaattataac caacctccct acactcaact ctaacttcac ctccacctct 10920
   accatcccca ttacctacaa atcctaccta cacctttcaa aaaaactaaa aatcacctaa 10980
   cttactcctt atataaacgt ttccctctat aactacttta atttcaactt cctctactcc 11040
   cctaaatcaa ttataaaacc ccaaccactt tccatattct aaaattatat taaaatttct 11100
   catctactaa catctcttct ctttattctt ataaatttta aatattttac taactttttt 11160
   tttttttttt ttttaaaaca aaatctaact ttattaccta aactaaaata caataaaata 11220
   atctctactc actacaacct ctaactccct aactcaaaaa atcctcccac ctcaacctcc 11280
   caataactaa aacgataaac atacaccacc acgcctaact aattttaata tttttttata 11340
   acacaaaatt tcaccacatt acccacgcta atttcaaact cctaaactca aataatctac 11400
   ccacctcgac ctcccaaaac ttacaaacat aaaccactac gcccaaccta acattctttt 11460
   tttttttttt taaaacaaaa tctctctcta tcacccaaac taaaatacaa taatacgatc 11520
   tctattcact acaacctcca cctcccaaat tcaaacgatt cttatacctc aacctcccta 11580
   ataactaaaa ttacaaaaat cccccaccac gcccaactaa tttttatatt tttaataaaa 11640
   ataaaatttc accatattaa ccaaactaat ctcaaactcc taacctctaa taacctaccc 11700
   gcctcgacct cccaaaatac taaaattaca aacataaacc actataccta acctcctaac 11760
   attcttatac atttaacatt ttttaacact cttcaatcct ttatacaaat tcaaatttct 11820
   atctaaaatc attttctttc cacctaaaaa actcccttta acatttccta cacatctaac 11880
   aataaattcc cttaaaattt atttatctac caacatattt atttcttctt caattttaaa 11940
   aaatattttc accgaatata aaattctaaa tcaataccaa tcatactaaa ttaaaaaaaa 12000
   aaaaaaaaaa aattctaaat taaaaatttt tatttttatt tttttccccc aacactttaa 12060
   aaatttcatt atattatttc ctaacttaca taatttctaa taaaaaaaat ataatatttc 12120
   ctatatctat tcttccatac ataataaacc tcatttcttt aaatactttt aaaattttct 12180
   ttttaccact aattttcaac atttaattat aataaacctt aatataattt acttcgtatt 12240
   tatcttactt aaaattcatt aaatttctta attcttaact ttatcttaat attaatttcc 12300
   acaacaaatt actacaaact aaataaatta aaacaacaaa tatttaaccg aatataataa 12360
   ctcacgccta taatcccaac attttaaaaa accgaaacga acgaatcacg aaatcaaaaa 12420
   atcgaaacca tactaactaa cacgataaaa ccccgtctct actaaaaata caaaaaatta 12480
   accgaacgta ataataaata cctataatcc caactactta aaaaactaaa acaaaaaaat 12540
   aacgtaaacc gaaaaaacga aacttacaat aaactaaaat cacgccacta cactccaacc 12600
   taaacgacaa aataaaattc catctcaaaa aaaaaaaaaa aaaatttatt ctatcacaat 12660
   tcgaaaaaca taaatacaaa tttaaaatat taacaaaata acaatccttc caaaaactct 12720
   aaaaaaaaat cctaccttat ctctttcaaa ttctaataac taaaccacaa ccctcttaac 12780
   ttataactac aaaactctaa tctctacctc cgtcttcaca taaccttccc acctctctaa 12840
   atatcttaaa aaaacaccta tcattaaatt taaaaccccc taaataatcc aaaattattt 12900
   catcttaaaa tccttaactt aattatatct acaaaaaccc ttttttaaaa taaaatctca 12960
   ttcataaact ataaaaatca aaatataata taaacatatc ttaaaaaaaa caccattctt 13020
   caacccacca taataatttt tatcaaattt aaaaaaattt taaacgttct ttctttcttt 13080
   tattatttat ttacttattt taaaataaaa tctccctcta tcgcccaaac taaaatacaa 13140
   taacacgatc tcgactcatt acaacctccc cttcccgaat tcaaacgatt ctcctacctc 13200
   aacccccaaa taactaaaac tacaaatacc caccaccacg cctaactaat ttttatattt 13260
   ttaataaaaa taaaatttca ccatattaac caaactaatc tcaaactcct aatattataa 13320
   tccacccgcc ttaacctccc aaaatactaa aattacaaac gtaaaccacc gcacccgacc 13380
   tatttctcta aatttatctt atttatccta cttctttatt ctatccttta aaattccaat 13440
   aaatatatat tacaatactt aataatatct tacaaattat aaaactccat aaaaacaaaa 13500
   tcttactcta ttacccaaac taaaatacaa taacgtaatc ataactcact acaacctcga 13560
   actcctaaac tcaaaaaatc ctcctacctc aacttcaaaa aaactaaaac tacaactact 13620
   taccaccata cccaactaat ataaaaatta ttttatttta ttttatttta tttataaaaa 13680
   caaaatctta ctatattacc taaactaatc ttaaactcct aaactcaaac caacctcccg 13740
   ccttaacctc caaaaatact aaaactataa aaacccaacc aactttattt ttcaatacta 13800
   tttttctcct ctatacttca atttaaaaaa ttctattact atcttcaaat tcactaattt 13860
   ttaaattata caatatctaa tctactatta tttccattca ataaattttt catttcaatt 13920
   actatactta ctatctttaa ataatccact aaattctttt ttatacctct cattcctctc 13980
   ctcataaaca tctttttcat atttttcttt aaactcttaa atatattaaa ccatctatca 14040
   tctttatcat ttctaaattt tatttctata aaattatttt tctcctaatt ataaattata 14100
   ttttctttct tttttttttt tttttaaaat aaaatctctc tctctattac ccaaactaaa 14160
   atacaaaaac gccatctcga ctcactacaa actcgatctc ctaacctcgt aatccacccg 14220
   cctcaacctc ccaaaatact aaaattacaa actcacgtaa accaccgcac cctacctata 14280
   aattttattt tcctactttt taacatatct aataattttt aactaaattt taaacattat 14340
   aaatataata ttaaacatct aaatttatta ttattcttta aaaaatatta aactttattc 14400
   aaacaaacta aataaattac ttacaaatca atttaacctt tttaaaactt ttttttttaa 14460
   aacaaaatct tactctatca cccaaactta aatacaataa aacaatctca actcattaca 14520
   acctcaacct cctaaactta aaaaatcctc ctacctcaat cactaaataa ctaaaactac 14580
   aaacacatac taccatacct aactaatttt ttatattttt tataaacata aaattttaat 14640
   atattaccca aactaatctt aaactcctaa actcaaataa tctacctacc tcaacctccc 14700
   aaaatactaa aattaaaaat ataaaccacc gtacctaacc tttaatacta atttttcttt 14760
   ttctttttct tttcttttct tttttttttt taaaataaaa tcttactcta tcacccaaac 14820
   caaaatataa taatacaatc ttaactcact acaacctccg cctcctaaat tcaaataatt 14880
   cttatacctc aacctcctaa ataactaaaa ttacaaacac ccatcaccaa acccgactaa 14940
   ttttttatat ttttaataaa aatataattt cgccatttta accaaactaa tcttaaactc 15000
   ctaacctcaa ataatctacc cgcctccgcc tcccaaaata ctaaaattac aaacataaac 15060
   cactacaccc gacctaaaac taatttttaa tctattaaaa taaatctaaa taaacctttt 15120
   ctctaaaaat aatttaactc tactactaaa acataaccct tctaaaatct aattaaatat 15180
   acatctccac tctaaaaaac taaaacttaa ttatctcctt acttttaata aactctaaaa 15240
   atttaccatt ttattactcc tttttatata atcttactaa atttcactat acacatataa 15300
   aatttaatat tcaaaaaaac ttaaaaaaac ccctatacaa atttctaaaa ttttttaaat 15360
   aatttactct tttccaaaac tatactctac tacaaccttt aataccttaa cttccctaaa 15420
   tcctatctcc atcttctcaa ctcaaaaaaa acaccatact ctactcgaac ttcccctcct 15480
   taaattacaa tctaaaatat tcctccaaac aaaaatccaa aaatattaca aaactcacct 15540
   tattcatttt ccttacttca aaaatcacaa ttctaaactt ctaaaaacaa ctatttctct 15600
   ttacatatat tttctatata aaaaaatata tatatataca cacacaaaca cacacacaca 15660
   cacacataca cacacacata tatatataca cacacacgca tatatatata tatatttaaa 15720
   acaacgtctc actctattac ccaacctaaa atacaaaaat ataataatac ctcactacaa 15780
   cctcaacctc ccaaacttaa acgatcctcc cacctcaacc tctaaataac taaaactaca 15840
   aacacaaacc atcatacccg actaattctt ttatattttt tataaaaata aaattttacc 15900
   atattaccca aactaatctc taactcctaa actcaaacaa tccgccaacc tttacctccc 15960
   aaattactaa aattacaaaa ataaacctct atacccaacc ttcatatatt ttacaacaat 16020
   aattttctca ttatttataa taacaaaata atttcattta ttataataaa aataaaaacc 16080
   tttttactaa cgttttaatc aaattcaaaa aaaaaaaaaa aattacatac atataatcaa 16140
   ttcactattc cttttttttt taaaacgaaa ttttactctt atcgcccaaa ctaaaataca 16200
   ataacataat ctcgactcac tacaacctcc gcctcccgaa ttcaaacgat tctcttacct 16260
   caacctccta attaactaaa attacaaaca taaaccacca cgcctaacta attttatatt 16320
   tttaataaaa ataaaatttc tccatattat caaactaatc tcgaactccc aacctcaaat 16380
   aatctacccg cctcgacctc ccaaaatact aaaattacaa acgtaaacta ccgcgcctaa 16440
   cctatcaatt cactattctt aaccaaaaat cctaaactct cgaaatatat ttaaatactt 16500
   atacttaaaa ccgaacgtaa taactcacgc ctataatccc aacactttaa aaaaccaaaa 16560
   caaacgaatc acttaaaatc aaaaattaaa aaccaaccta aacaacatat caaaaccccg 16620
   tctctactaa aatacaaaaa ttaacctaac ataataatac tcgcctataa tcccaactac 16680
   tcaaaaaact aaaacacaaa aatcgcttaa acctaaaaaa cgaaaattac aataaaccaa 16740
   aataacacca ctacactcta acctaaataa cacaataaaa ctctatctca aaaaacaaac 16800
   aaacaaacaa acaaacaaaa cttacgttta acaaacatac tttctaaacc aaatacaaaa 16860
   acttataatc ccaactactt aaaaaactaa aacaaaaaaa ttactcaaac ctaaaaattt 16920
   aaaaccaacc taaataacat aataaaaccc tatctcaaat aaaaaaaaaa aaaaaaaaaa 16980
   aaaaaaaaaa aatcttttat ctaaacacta cgactcacac ctataatccc aacactttaa 17040
   aaacccgaac caaataaatc acctaaaatc aaaaattcaa aactaaccta accaacataa 17100
   taaaaaccca tctctactaa aaatacaaaa aattaaccaa acgtattaac gaatatctat 17160
   aatcccaact acttaaaaaa ctaaaacaaa aaaatcgctt aaacctaaaa aacgaaaatt 17220
   acaacgaact aatatcacgt cattatactc caacctaacc aacaaaataa aactccgtcc 17280
   ccccaaaaaa aacacaaaaa aaaaccccaa aaaaaacttt tatcactttt atcacacaac 17340
   aaaaatctca tacaatataa tcatattatt cacaaaatta taaaatttat acaaatttta 17400
   atacataata tcccacaaac acaaaacata attataaaat tatattaata ccataaactt 17460
   attatttcat tcctatctat atattactat aaccgtttat taaacctcac tcaataataa 17520
   tatcaataat ctaaattaat aactcttcct aaaaataacc taaaaccctt aaaataaatc 17580
   taatcccaaa taaactcctt aaccaacaac ctctttacca ccccacttca atcccaaact 17640
   tattcttaac aaacaaacca ctaaacccta aaaaaaaaaa cccaaccaaa aacccttaat 17700
   aaacaaaaaa aaaaaaaacc ctccctcttc caaaattact ttctatccta tcttctaaac 17760
   ccctttccat ccacgtatct atctttccgt tactcataat atctatcttt ccgttaccca 17820
   taatatctat ctttccgtta ccttataaaa ttaataaaac aaaacaaccc caacaaaaaa 17880
   taaaaattac tacctactcc aaaactatct aactccgaaa aactacctcc gacgtaacta 17940
   aaactttatc ttccactcct cctcctcctc caaataaaca tcgaacaact aaatccccgc 18000
   gaccgaactc ccctacacct aaccaccacg ctaaaacacc ttaaatatac ccgtatactc 18060
   ctaacgcacg acgcaaacgt aaacaaaaaa aatcgcaacg actaaacaaa taaacaaccc 18120
   tactcacccc aaccccacaa ccgaaacctc cccaacatca taaacctata attaactaaa 18180
   aaatcctacc ctataacctt acctcgccct cccccaatac tccaaaaaac tataaatacc 18240
   cgaaacctaa aactaataca actaatactt cgataccgaa actaccaacg aataataaaa 18300
   cgactaacga acatccccgt actcctaaaa aaactacgca aaataaaacc caacctctca 18360
   acctccacga aaacccttaa acccttctcc aatacaaact taacctctct ccccttcgtc 18420
   ctccaaaccc aaaacttcta cgtaaaaata aaataaaaat tcactaacta aaataaacga 18480
   actacaacaa atcgcttcta aactctccca ctttaaatcc tacgcttact cccctaccta 18540
   aaatatattc tcataattct tccgacgatt ccctctatac cttaactcaa ctcaaaacca 18600
   ccgcctcaac taaacctttc caaattaccc caaatactat cttaactcca ttattattct 18660
   cttcactcta tcataattta cttactatat tcataaccta ccgctcctta ttaaaaaata 18720
   aactctaaaa aaacaaaaat cctatctttt tcactattat attcccaata actaaaacac 18780
   tcaataaata tttcataaat atttattaaa aaaataaata aaaaaataaa taaataaatt 18840
   aacaaataat aaacactaaa aacttaataa taacaaaaac atacccgtcc cgacctcaaa 18900
   ttactcttct atataaatat aatctacact aaccaaaaaa aaaattttaa aaaaaaaact    18960
   actctctctt caattccctc ccctattcct aaccacacga atcccaaacc ccatacccta    19020
   aactaatcca aaccgtataa accaaaccaa tcaaaaccac ctccaacctc cgcttccacc    19080
   aataccccta atatccaaaa tctaccctaa taacacctac aaaatataaa aaaacgacca    19140
   aaacctaaaa ataaacacca cactcctaaa ctttaaccac ataacctaac aacgaaaaaa 19200
   ccgcaacttt atcttcaaaa accaaaatca aacaaacaaa aaataaaaca aaataaaaac    19260
   gataaaaaac aaccccaaac cacccctaat ccccctatac tattccgtat ctaacaaaca    19320
   caaacgccgt aatcataaaa attaaccacg accgccgaat aatatacaca aaaactctaa 19380
   cactaactaa acaaaaccga aaactactac taactactac tcaacccact aaaaaacaaa 19440
   tactaaaccg acttaccgcg cccgtcgtca ccactcaact taacaccaaa aatatctcct 19500
   ttaaaaaata aataaacata accttaatca cccttaaacc aacccgataa aatacctacc 19560
   tcacctaaac cactctactt ccccaaaaac aaaactaaca tcctaaacta acgcaataaa 19620
   aaaacgaaaa taataaataa atataaaact aaaataaaac tacaactccc aactaccaac 19680
   ccaactacac tcttacctac aacaaactat acaaaaaatt ctaacctccc ttccccaacc 19740
   ctaaaaatac aaaaacaata caacgtaatc aaacacttat atttaaattc aaaacacttc 19800
   tctaatattt tatataaata tataaatata tacacaaaac acgtacacac gcacacacac 19860
   acacacattt attcctcaca acaacccttt ctaataaata ctattctatt aatattccca 19920
   ttttacgcat atttattaaa tcctttcctt aacatcccat ttactcttca caacaacccc 19980
   aaaaaataaa aataatcgat ccattttata ataaaaaaat aaaaacttaa aataattcta 20040
   taacctatac aaaatcacac aactattaaa taacaaaatt aaaattcaaa tcctaactaa 20100
   cctaacccta aaaccaaacc tttatcccta aaaataacta cctaactaaa cataataact 20160
   catacctata atcccaacgc tttaaaaaac cgaaataaac gaatcaccta aaatcaattc 20220
   gaaaccaacc taactaacat aataaaaccc cgtctctact aacaatacaa aaaattaacc 20280
   gaatataata acaaacgcct ataatcccaa ctactcgaaa aactaaaaca aaaaaattac 20340
   ttaaacctaa aaaacgaaac ttacaatata caacgatccc gccacaacac tccaacctaa 20400
   acaacaaaac gaaactccat ctaaaaaaaa aaaaaaaaaa aaactctacc tattactcta    20460
   aaacccctat attacccacg acataaaaac aaaaactaac aaataccata aattttatat    20520
   ataacatctc ctctaaaaac ctcccctact tctataatct aaaaaaacct ctatactacc 20580
   cccaccaata cataaaccat actattataa ctccatttta ttatctcctt ataaaaatta 20640
   taaatctcaa aaaaacaaaa atcatattta acttatttac tatatcccta atacctacct 20700
   caatacctaa cacataaaaa ctcctcaaaa aatatttatt aaataaataa ataaataaat 20760
   aaataaataa ataacctcat atattcactt acccaaaaac ctaaaacaaa atctctccaa 20820
   aaccctttac tacaaaaaat attcccaata cccttaaaaa taaaacacaa aataaaacca 20880
   aaactactta taaacccttt cttcatctat ccccaaatat ataaaacatc taacgtaaaa 20940
   ctcatcaccc gcacacgaac aaaacatctt tcaaaacaac acaaaaacaa aatcaaaaat 21000
   aataaaacaa aactaaataa aaaaaaatat tacaaaacta cgaaaaccaa ctcccacttc 21060
   aatacttcca tcaatattcc cactaacatc ctaacaaaaa actaaatact ttattcaaac 21120
   cgataaattt caattcaaat accaaaccct aaaaataaat aataactacc taaaatacta 21180
   aaaaatttct aaaaactcat ctaaattcaa taaaaaacca tactacaaat aattactaat 21240
   attactatta aatttaaaaa aaaaaaaaaa taccatatat accatttatt aaccattcta 21300
   atctaataaa acatatccta aataaaaaat caaaaaacct taccacaaat tctatatacc 21360
   cattaatacc acacctaatt ctctaaatac caaataccgt atccgaaata aaaaaacata 21420
   atttctacct cacaaaaaaa caacataaaa tcatacctat aaaaatacct aaaacaaaca 21480
   cacacacgca catacacacc ccacaatacc taccgaaaca aacgttttac tatccaaaat 21540
   caccgaacta aaaaaaaaca cttttaaaaa ttacttctaa cctctaaacc ttccttttaa 21600
   tattttaaac tatactcaca aaaaccctaa cccctaatcc ttaaaacact caatcccctt 21660
   caaataaaaa tacctaaata tataattaaa taaacaaacc ccaccccaaa aaccgacctc 21720
   ctccctaaat cttaaatact atcacctcta ataaaatacc caaatatata taaaattacg 21780
   atatcctcta aaataccaca tttcttataa actataaaac acctttacaa tccttcctaa 21840
   aaatcgctaa acaacacgaa aacccccaaa ataaaataaa tatatacaaa aatacccgta 21900
   aataaaaaaa tataaatata actcaaaaaa aaacttaaaa aacgtcctac ttaacataac 21960
   ctatataaac ctcctttcca caatacccaa aaccatatca acgccacact aacaaaaaaa 22020
   acaacttaaa aaccttataa caacaaataa attaacctaa cattaaaaac taaaaaaaac 22080
   tcctaaaccc ctccccatat aatacaaacc ctaatcactc aaactctaaa ccaaaccaac 22140
   cccactacca tcactacaaa aaaatacttc aaccccaact ttaaactaaa caaccgtaat 22200
   ataaaccgcc ccataaaact aaccaccaaa acacaaaaat aaaaccccta ccgatactaa 22260
   aaaaataaaa aaccgaaact ccaaaaaata ctaaaaaacc ataacttcct acaatatccc 22320
   ttacccctta aaatctaata aaaacaaatt caaaaccaaa ctataactat ataaaaaaca 22380
   tcccctatcc ctatataaac aaataacccc catcattaac ctcataaccg tcaacaatac 22440
   gctttttacc aaactccgaa acttcatcac cctacgtcta cctcctaact tcccaattaa 22500
   aattaataaa accgcacgac catttctcct aaacccgtaa cgaaccgacc gaaatttaaa 22560
   taaaatcgaa ataaaactaa aactaaaata taaaaaacta aataataacg cctaaaaatt 22620
   tcctcatcct caaaaatccc gatcttccac atcctcaacg cccgcatcac cttcgaaaac 22680
   ctcaacgact acgacgaacc gataccatcg atacgaaaca accccaacaa cgaaacgcct 22740
   tccccaaaca acgactcctc caacgtcaac aactccaact ccacgaataa aaccccccgc 22800
   gaaaacgcct acccctcgac tctccccgaa ataaataaaa aaaacgcgcc cgaccgtacc 22860
   taacccaacc cccgcgcccc caacctccta ccgcgactac gaaatctccc caacgttatt 22920
   cgaaaccccg cgcgactaca acataataaa cgaccaacga aaaacgacga cccgaaacga 22980
   cgacgacgac ctactacaat tcgccatcca acaaaaccta cttaaaacga acaataaata 23040
   taaccaaata cgtctccgcg aacgcgcgaa accacgaaac tccgcgccgc cacgactaac 23100
   gcccacgcct ccccgcaaat caccatctaa aaaacgctaa ccaacaacaa accaaacacc 23160
   caccccatat cctacgaaaa tcgccgacaa aacaaatcaa tacccgctaa accgaaaaaa 23220
   atcctccgaa aaaaatccta tctcccctaa accaaaacac aaccgaaaaa cccgcgaaac 23280
   cctcgacaaa aaccctccta atatcgaaat aacttctacc accactccta cctccgaaac 23340
   gacaaacaaa aatcaaaaat ccaaacttca ccgcgaaatt caaactctcc ccaaacatta 23400
   acgaaaaaaa cttaataaaa tctaaactcc gccacgacca aataaaaacc aaaacgccgc 23460
   gaaactaacc gcctccacta aaaaataata accgcgaacc gccctaaaac ctccacgccg 23520
   tatcccgacc ccgaccgata aataaatacg cccgaaaacc cgcgacattc cttccccacc 23580
   cccaaaaacg ccccgcccac gccgcaacgc caacctacgc ccccgacgtc aatacccaac 23640
   cctcgaccca actcaaaacc aaattccgac gaccacgtat tccgaaacta cgacgaacaa 23700
   ctacgactaa cgataaaact atcgacgcaa aaacaaaaaa aaaaacgacg acgcgcgcgc 23760
   caaaaaaaaa aaaaactaaa acgcatccta aaactctcac taaccgaaca ataacgcccc 23820
   ctaccgaaac cctcgccaac gccacgcgcg ccacgcccaa aaccaaaaac caaacaaacc 23880
   ccgacctacg cgcctacaaa acgacgacta aaaactaaaa ccaccgcctc gcgaatacaa 23940
   caacacaaca aacacgattc gaaaaaaaat tcgacataac cgcgaaatac cttcccaaac 24000
   caaaacccta aaaacaacta acacgaccta atccccctac tttactatat tctaattccc 24060
   caacccgctc ccctaaactc aaatctatcc tatcctaaaa cgaaaccaaa cgatcctaaa 24120
   aaaaaaataa atccttaaaa aaacgctatc cctatccctt actccttcta accgatccgc 24180
   atttctattc actaacccca ctccaaaaca ctacccctaa aacctttaca tctctactct 24240
   tcactcctaa aaaacaacta aactccccgc gtactatatc actactttat tccaaacaca 24300
   aaccaacacg tctaaaaccc aacccctccc ccaccccgac atttcaacgt caaatacact    24360
   taacgaaata cccgactccc ccaaccccca cacccgtcct aaatctcaaa ataattccaa    24420
   cttctcctta aacaaccaaa aattaaaatc ccatccccca aaacacattt tttttttttt 24480
   ttttttataa tcaaaaaata tatccaacgt aaccccctaa cctatacaaa ccctaactcc 24540
   ctcataatac ctcgaaaaat aaaaaacata ttaaactaaa ataaacacta aacccaaata 24600
   aactaaacac aaaaaactcg cccaaaaccc taacgccacc cccacccctt cccaccaact    24660
   actactaacc tctataatta tacatcccac ttacccccac acgaaaacta actctaaaac    24720
   ccttcatcca ataatactaa cccccgactc tcccctaccc cacctcaccc acccaaaaaa    24780
   acacaaaccc cgccaaaaac aaaaacccac cgcacaccct tatcccgaac ctatctaaaa    24840
   ctaaccttcc cgactcaacc aataaaactc aaaaaaaaca caaaaaaaaa taaaaaaaaa    24900
   aaacaaaaaa aaactattcc tcccaccccc ttccctaata ccaaaaacac caaactaatt    24960
   ctaaaacaca aataaaaaaa acttcatacc gaa                                  24993
<210> 10
   <211> 24993
   <212> DNA
   <213> Artificial Sequence
<220>
   <223> G to A (bis2 rc) rc
<400> 10
   ttcggtatga agtttttttt atttgtgttt tagaattagt ttggtgtttt tggtattagg 60
   gaagggggtg ggaggaatag tttttttttg tttttttttt ttattttttt ttgtgttttt 120
   tttgagtttt attggttgag tcgggaaggt tagttttaga taggttcggg ataagggtgt 180
   gcggtgggtt tttgtttttg gcggggtttg tgtttttttg ggtgggtgag gtggggtagg 240
   ggagagtcgg gggttagtat tattggatga agggttttag agttagtttt cgtgtggggg 300
   taagtgggat gtataattat agaggttagt agtagttggt gggaaggggt gggggtggcg 360
   ttagggtttt gggcgagttt tttgtgttta gtttatttgg gtttagtgtt tattttagtt 420
   taatatgttt tttatttttc gaggtattat gagggagtta gggtttgtat aggttagggg 480
   gttacgttgg atatattttt tgattataaa aaaaaaaaaa aaaaaatgtg ttttggggga 540
   tgggatttta atttttggtt gtttaaggag aagttggaat tattttgaga tttaggacgg 600
   gtgtgggggt tgggggagtc gggtatttcg ttaagtgtat ttgacgttga aatgtcgggg 660
   tgggggaggg gttgggtttt agacgtgttg gtttgtgttt ggaataaagt agtgatatag 720
   tacgcgggga gtttagttgt tttttaggag tgaagagtag agatgtaaag gttttagggg 780
   tagtgttttg gagtggggtt agtgaataga aatgcggatc ggttagaagg agtaagggat 840
   agggatagcg tttttttaag gatttatttt ttttttagga tcgtttggtt tcgttttagg 900
   ataggataga tttgagttta ggggagcggg ttggggaatt agaatatagt aaagtagggg 960
   gattaggtcg tgttagttgt ttttagggtt ttggtttggg aaggtatttc gcggttatgt 1020
   cgaatttttt ttcgaatcgt gtttgttgtg ttgttgtatt cgcgaggcgg tggttttagt 1080
   ttttagtcgt cgttttgtag gcgcgtaggt cggggtttgt ttggtttttg gttttgggcg 1140
   tggcgcgcgt ggcgttggcg agggtttcgg tagggggcgt tattgttcgg ttagtgagag 1200
   ttttaggatg cgttttagtt tttttttttt ttggcgcgcg cgtcgtcgtt tttttttttg 1260
   tttttgcgtc gatagtttta tcgttagtcg tagttgttcg tcgtagtttc ggaatacgtg 1320
   gtcgtcggaa tttggttttg agttgggtcg agggttgggt attgacgtcg ggggcgtagg 1380
   ttggcgttgc ggcgtgggcg gggcgttttt gggggtgggg aaggaatgtc gcgggttttc 1440
   gggcgtattt atttatcggt cggggtcggg atacggcgtg gaggttttag ggcggttcgc 1500
   ggttattatt ttttagtgga ggcggttagt ttcgcggcgt tttggttttt atttggtcgt 1560
   ggcggagttt agattttatt aagttttttt cgttaatgtt tggggagagt ttgaatttcg 1620
   cggtgaagtt tggatttttg atttttgttt gtcgtttcgg aggtaggagt ggtggtagaa 1680
   gttatttcga tattaggagg gtttttgtcg agggtttcgc gggtttttcg gttgtgtttt 1740
   ggtttagggg agataggatt tttttcggag gatttttttc ggtttagcgg gtattgattt 1800
   gttttgtcgg cgattttcgt aggatatggg gtgggtgttt ggtttgttgt tggttagcgt 1860
   tttttagatg gtgatttgcg gggaggcgtg ggcgttagtc gtggcggcgc ggagtttcgt 1920
   ggtttcgcgc gttcgcggag acgtatttgg ttatatttat tgttcgtttt aagtaggttt 1980
   tgttggatgg cgaattgtag taggtcgtcg tcgtcgtttc gggtcgtcgt ttttcgttgg 2040
   tcgtttatta tgttgtagtc gcgcggggtt tcgaataacg ttggggagat ttcgtagtcg 2100
   cggtaggagg ttgggggcgc gggggttggg ttaggtacgg tcgggcgcgt tttttttatt 2160
   tatttcgggg agagtcgagg ggtaggcgtt ttcgcggggg gttttattcg tggagttgga 2220
   gttgttgacg ttggaggagt cgttgtttgg ggaaggcgtt tcgttgttgg ggttgtttcg 2280
   tatcgatggt atcggttcgt cgtagtcgtt gaggttttcg aaggtgatgc gggcgttgag 2340
   gatgtggaag atcgggattt ttgaggatga ggaaattttt aggcgttatt atttagtttt 2400
   ttatatttta gttttagttt tatttcgatt ttatttaaat ttcggtcggt tcgttacggg 2460
   tttaggagaa atggtcgtgc ggttttatta attttaattg ggaagttagg aggtagacgt 2520
   agggtgatga agtttcggag tttggtaaaa agcgtattgt tgacggttat gaggttaatg 2580
   atgggggtta tttgtttata tagggatagg ggatgttttt tatatagtta tagtttggtt 2640
   ttgaatttgt ttttattaga ttttaagggg taagggatat tgtaggaagt tatggttttt 2700
   tagtattttt tggagtttcg gttttttatt tttttagtat cggtaggggt tttatttttg 2760
   tgttttggtg gttagtttta tggggcggtt tatattacgg ttgtttagtt taaagttggg 2820
   gttgaagtat ttttttgtag tgatggtagt ggggttggtt tggtttagag tttgagtgat 2880
   tagggtttgt attatatggg gaggggttta ggagtttttt ttagttttta atgttaggtt 2940
   aatttatttg ttgttataag gtttttaagt tgtttttttt gttagtgtgg cgttgatatg 3000
   gttttgggta ttgtggaaag gaggtttata taggttatgt taagtaggac gttttttaag 3060
   tttttttttg agttatattt atattttttt atttacgggt atttttgtat atatttattt 3120
   tattttgggg gttttcgtgt tgtttagcga tttttaggaa ggattgtaaa ggtgttttat 3180
   agtttataag aaatgtggta ttttagagga tatcgtaatt ttatatatat ttgggtattt 3240
   tattagaggt gatagtattt aagatttagg gaggaggtcg gtttttgggg tggggtttgt 3300
   ttatttaatt atatatttag gtatttttat ttgaagggga ttgagtgttt taaggattag 3360
   gggttagggt ttttgtgagt atagtttaaa atattaaaag gaaggtttag aggttagaag 3420
   taatttttaa aagtgttttt ttttagttcg gtgattttgg atagtaaaac gtttgtttcg 3480
   gtaggtattg tggggtgtgt atgtgcgtgt gtgtgtttgt tttaggtatt tttataggta 3540
   tgattttatg ttgttttttt gtgaggtaga aattatgttt ttttatttcg gatacggtat 3600
   ttggtattta gagaattagg tgtggtatta atgggtatat agaatttgtg gtaaggtttt 3660
   ttgatttttt atttaggata tgttttatta gattagaatg gttaataaat ggtatatatg 3720
   gtattttttt ttttttttaa atttaatagt aatattagta attatttgta gtatggtttt 3780
   ttattgaatt tagatgagtt tttagaaatt ttttagtatt ttaggtagtt attatttatt 3840
   tttagggttt ggtatttgaa ttgaaattta tcggtttgaa taaagtattt agttttttgt 3900
   taggatgtta gtgggaatat tgatggaagt attgaagtgg gagttggttt tcgtagtttt 3960
   gtaatatttt tttttattta gttttgtttt attatttttg attttgtttt tgtgttgttt 4020
   tgaaagatgt tttgttcgtg tgcgggtgat gagttttacg ttagatgttt tatatatttg 4080
   gggatagatg aagaaagggt ttataagtag ttttggtttt attttgtgtt ttatttttaa 4140
   gggtattggg aatatttttt gtagtaaagg gttttggaga gattttgttt taggtttttg 4200
   ggtaagtgaa tatatgaggt tatttattta tttatttatt tatttattta tttaataaat 4260
   attttttgag gagtttttat gtgttaggta ttgaggtagg tattagggat atagtaaata 4320
   agttaaatat gatttttgtt tttttgagat ttataatttt tataaggaga taataaaatg 4380
   gagttataat agtatggttt atgtattggt gggggtagta tagaggtttt tttagattat 4440
   agaagtaggg gaggttttta gaggagatgt tatatataaa atttatggta tttgttagtt 4500
   tttgttttta tgtcgtgggt aatatagggg ttttagagta ataggtagag tttttttttt 4560
   tttttttttt tagatggagt ttcgttttgt tgtttaggtt ggagtgttgt ggcgggatcg 4620
   ttgtatattg taagtttcgt tttttaggtt taagtaattt ttttgtttta gtttttcgag 4680
   tagttgggat tataggcgtt tgttattata ttcggttaat tttttgtatt gttagtagag 4740
   acggggtttt attatgttag ttaggttggt ttcgaattga ttttaggtga ttcgtttatt 4800
   tcggtttttt aaagcgttgg gattataggt atgagttatt atgtttagtt aggtagttat 4860
   ttttagggat aaaggtttgg ttttagggtt aggttagtta ggatttgaat tttaattttg 4920
   ttatttaata gttgtgtgat tttgtatagg ttatagaatt attttaagtt tttatttttt 4980
   tattataaaa tggatcgatt atttttattt tttggggttg ttgtgaagag taaatgggat 5040
   gttaaggaaa ggatttaata aatatgcgta aaatgggaat attaatagaa tagtatttat 5100
   tagaaagggt tgttgtgagg aataaatgtg tgtgtgtgtg tgcgtgtgta cgtgttttgt 5160
   gtatatattt atatatttat ataaaatatt agagaagtgt tttgaattta aatataagtg 5220
   tttgattacg ttgtattgtt tttgtatttt tagggttggg gaagggaggt tagaattttt 5280
   tgtatagttt gttgtaggta agagtgtagt tgggttggta gttgggagtt gtagttttat 5340
   tttagtttta tatttattta ttattttcgt ttttttattg cgttagttta ggatgttagt 5400
   tttgtttttg gggaagtaga gtggtttagg tgaggtaggt attttatcgg gttggtttaa 5460
   gggtgattaa ggttatgttt atttattttt taaaggagat atttttggtg ttaagttgag 5520
   tggtgacgac gggcgcggta agtcggttta gtatttgttt tttagtgggt tgagtagtag 5580
   ttagtagtag ttttcggttt tgtttagtta gtgttagagt ttttgtgtat attattcggc 5640
   ggtcgtggtt aatttttatg attacggcgt ttgtgtttgt tagatacgga atagtatagg 5700
   gggattaggg gtggtttggg gttgtttttt atcgttttta ttttgtttta ttttttgttt 5760
   gtttgatttt ggtttttgaa gataaagttg cggttttttc gttgttaggt tatgtggtta 5820
   aagtttagga gtgtggtgtt tatttttagg ttttggtcgt ttttttatat tttgtaggtg 5880
   ttattagggt agattttgga tattaggggt attggtggaa gcggaggttg gaggtggttt 5940
   tgattggttt ggtttatacg gtttggatta gtttagggta tggggtttgg gattcgtgtg 6000
   gttaggaata ggggagggaa ttgaagagag agtagttttt tttttaaaat tttttttttg 6060
   gttagtgtag attatattta tatagaagag taatttgagg tcgggacggg tatgtttttg 6120
   ttattattaa gtttttagtg tttattattt gttaatttat ttatttattt ttttatttat 6180
   ttttttaata aatatttatg aaatatttat tgagtgtttt agttattggg aatataatag 6240
   tgaaaaagat aggatttttg tttttttaga gtttattttt taataaggag cggtaggtta 6300
   tgaatatagt aagtaaatta tgatagagtg aagagaataa taatggagtt aagatagtat 6360
   ttggggtaat ttggaaaggt ttagttgagg cggtggtttt gagttgagtt aaggtataga 6420
   gggaatcgtc ggaagaatta tgagaatata ttttaggtag gggagtaagc gtaggattta 6480
   aagtgggaga gtttagaagc gatttgttgt agttcgttta ttttagttag tgaattttta 6540
   ttttattttt acgtagaagt tttgggtttg gaggacgaag gggagagagg ttaagtttgt 6600
   attggagaag ggtttaaggg ttttcgtgga ggttgagagg ttgggtttta ttttgcgtag 6660
   tttttttagg agtacgggga tgttcgttag tcgttttatt attcgttggt agtttcggta 6720
   tcgaagtatt agttgtatta gttttaggtt tcgggtattt atagtttttt ggagtattgg 6780
   gggagggcga ggtaaggtta tagggtagga ttttttagtt aattataggt ttatgatgtt 6840
   ggggaggttt cggttgtggg gttggggtga gtagggttgt ttatttgttt agtcgttgcg 6900
   attttttttg tttacgtttg cgtcgtgcgt taggagtata cgggtatatt taaggtgttt 6960
   tagcgtggtg gttaggtgta ggggagttcg gtcgcgggga tttagttgtt cgatgtttat 7020
   ttggaggagg aggaggagtg gaagataaag ttttagttac gtcggaggta gtttttcgga 7080
   gttagatagt tttggagtag gtagtaattt ttattttttg ttggggttgt tttgttttat 7140
   taattttata aggtaacgga aagatagata ttatgggtaa cggaaagata gatattatga 7200
   gtaacggaaa gatagatacg tggatggaaa ggggtttaga agataggata gaaagtaatt 7260
   ttggaagagg gagggttttt tttttttttg tttattaagg gtttttggtt gggttttttt 7320
   ttttagggtt tagtggtttg tttgttaaga ataagtttgg gattgaagtg gggtggtaaa 7380
   gaggttgttg gttaaggagt ttatttggga ttagatttat tttaagggtt ttaggttatt 7440
   tttaggaaga gttattaatt tagattattg atattattat tgagtgaggt ttaataaacg 7500
   gttatagtaa tatatagata ggaatgaaat aataagttta tggtattaat ataattttat 7560
   aattatgttt tgtgtttgtg ggatattatg tattaaaatt tgtataaatt ttataatttt 7620
   gtgaataata tgattatatt gtatgagatt tttgttgtgt gataaaagtg ataaaagttt 7680
   tttttggggt ttttttttgt gttttttttg gggggacgga gttttatttt gttggttagg 7740
   ttggagtata atgacgtgat attagttcgt tgtaattttc gttttttagg tttaagcgat 7800
   ttttttgttt tagtttttta agtagttggg attatagata ttcgttaata cgtttggtta 7860
   attttttgta tttttagtag agatgggttt ttattatgtt ggttaggtta gttttgaatt 7920
   tttgatttta ggtgatttat ttggttcggg tttttaaagt gttgggatta taggtgtgag 7980
   tcgtagtgtt tagataaaag attttttttt tttttttttt tttttttttt tttatttgag 8040
   atagggtttt attatgttat ttaggttggt tttaaatttt taggtttgag taattttttt 8100
   gttttagttt tttaagtagt tgggattata agtttttgta tttggtttag aaagtatgtt 8160
   tgttaaacgt aagttttgtt tgtttgtttg tttgtttgtt ttttgagata gagttttatt 8220
   gtgttattta ggttagagtg tagtggtgtt attttggttt attgtaattt tcgtttttta 8280
   ggtttaagcg atttttgtgt tttagttttt tgagtagttg ggattatagg cgagtattat 8340
   tatgttaggt taatttttgt attttagtag agacggggtt ttgatatgtt gtttaggttg 8400
   gtttttaatt tttgatttta agtgattcgt ttgttttggt tttttaaagt gttgggatta 8460
   taggcgtgag ttattacgtt cggttttaag tataagtatt taaatatatt tcgagagttt 8520
   aggatttttg gttaagaata gtgaattgat aggttaggcg cggtagttta cgtttgtaat 8580
   tttagtattt tgggaggtcg aggcgggtag attatttgag gttgggagtt cgagattagt 8640
   ttgataatat ggagaaattt tatttttatt aaaaatataa aattagttag gcgtggtggt 8700
   ttatgtttgt aattttagtt aattaggagg ttgaggtaag agaatcgttt gaattcggga 8760
   ggcggaggtt gtagtgagtc gagattatgt tattgtattt tagtttgggc gataagagta 8820
   aaatttcgtt ttaaaaaaaa aaggaatagt gaattgatta tatgtatgta attttttttt 8880
   tttttttgaa tttgattaaa acgttagtaa aaaggttttt atttttatta taataaatga 8940
   aattattttg ttattataaa taatgagaaa attattgttg taaaatatat gaaggttggg 9000
   tatagaggtt tatttttgta attttagtaa tttgggaggt aaaggttggc ggattgtttg 9060
   agtttaggag ttagagatta gtttgggtaa tatggtaaaa ttttattttt ataaaaaata 9120
   taaaagaatt agtcgggtat gatggtttgt gtttgtagtt ttagttattt agaggttgag 9180
   gtgggaggat cgtttaagtt tgggaggttg aggttgtagt gaggtattat tatatttttg 9240
   tattttaggt tgggtaatag agtgagacgt tgttttaaat atatatatat atatgcgtgt 9300
   gtgtgtatat atatatgtgt gtgtgtatgt gtgtgtgtgt gtgtgtttgt gtgtgtatat 9360
   atatatattt ttttatatag aaaatatatg taaagagaaa tagttgtttt tagaagttta 9420
   gaattgtgat ttttgaagta aggaaaatga ataaggtgag ttttgtaata tttttggatt 9480
   tttgtttgga ggaatatttt agattgtaat ttaaggaggg gaagttcgag tagagtatgg 9540
   tgtttttttt gagttgagaa gatggagata ggatttaggg aagttaaggt attaaaggtt 9600
   gtagtagagt atagttttgg aaaagagtaa attatttaaa aaattttaga aatttgtata 9660
   ggggtttttt taagtttttt tgaatattaa attttatatg tgtatagtga aatttagtaa 9720
   gattatataa aaaggagtaa taaaatggta aatttttaga gtttattaaa agtaaggaga 9780
   taattaagtt ttagtttttt agagtggaga tgtatattta attagatttt agaagggtta 9840
   tgttttagta gtagagttaa attattttta gagaaaaggt ttatttagat ttattttaat 9900
   agattaaaaa ttagttttag gtcgggtgta gtggtttatg tttgtaattt tagtattttg 9960
   ggaggcggag gcgggtagat tatttgaggt taggagttta agattagttt ggttaaaatg 10020
   gcgaaattat atttttatta aaaatataaa aaattagtcg ggtttggtga tgggtgtttg 10080
   taattttagt tatttaggag gttgaggtat aagaattatt tgaatttagg aggcggaggt 10140
   tgtagtgagt taagattgta ttattatatt ttggtttggg tgatagagta agattttatt 10200
   ttaaaaaaaa aaaagaaaag aaaagaaaaa gaaaaagaaa aattagtatt aaaggttagg 10260
   tacggtggtt tatattttta attttagtat tttgggaggt tgaggtaggt agattatttg 10320
   agtttaggag tttaagatta gtttgggtaa tatattaaaa ttttatgttt ataaaaaata 10380
   taaaaaatta gttaggtatg gtagtatgtg tttgtagttt tagttattta gtgattgagg 10440
   taggaggatt ttttaagttt aggaggttga ggttgtaatg agttgagatt gttttattgt 10500
   atttaagttt gggtgataga gtaagatttt gttttaaaaa aaaaagtttt aaaaaggtta 10560
   aattgatttg taagtaattt atttagtttg tttgaataaa gtttaatatt ttttaaagaa 10620
   taataataaa tttagatgtt taatattata tttataatgt ttaaaattta gttaaaaatt 10680
   attagatatg ttaaaaagta ggaaaataaa atttataggt agggtgcggt ggtttacgtg 10740
   agtttgtaat tttagtattt tgggaggttg aggcgggtgg attacgaggt taggagatcg 10800
   agtttgtagt gagtcgagat ggcgtttttg tattttagtt tgggtaatag agagagagat 10860
   tttattttaa aaaaaaaaaa aaaagaaaga aaatataatt tataattagg agaaaaataa 10920
   ttttatagaa ataaaattta gaaatgataa agatgataga tggtttaata tatttaagag 10980
   tttaaagaaa aatatgaaaa agatgtttat gaggagagga atgagaggta taaaaaagaa 11040
   tttagtggat tatttaaaga tagtaagtat agtaattgaa atgaaaaatt tattgaatgg 11100
   aaataatagt agattagata ttgtataatt taaaaattag tgaatttgaa gatagtaata 11160
   gaatttttta aattgaagta tagaggagaa aaatagtatt gaaaaataaa gttggttggg 11220
   tttttatagt tttagtattt ttggaggtta aggcgggagg ttggtttgag tttaggagtt 11280
   taagattagt ttaggtaata tagtaagatt ttgtttttat aaataaaata aaataaaata 11340
   aaataatttt tatattagtt gggtatggtg gtaagtagtt gtagttttag tttttttgaa 11400
   gttgaggtag gaggattttt tgagtttagg agttcgaggt tgtagtgagt tatgattacg 11460
   ttattgtatt ttagtttggg taatagagta agattttgtt tttatggagt tttataattt 11520
   gtaagatatt attaagtatt gtaatatata tttattggaa ttttaaagga tagaataaag 11580
   aagtaggata aataagataa atttagagaa ataggtcggg tgcggtggtt tacgtttgta 11640
   attttagtat tttgggaggt taaggcgggt ggattataat attaggagtt tgagattagt 11700
   ttggttaata tggtgaaatt ttatttttat taaaaatata aaaattagtt aggcgtggtg 11760
   gtgggtattt gtagttttag ttatttgggg gttgaggtag gagaatcgtt tgaattcggg 11820
   aaggggaggt tgtaatgagt cgagatcgtg ttattgtatt ttagtttggg cgatagaggg 11880
   agattttatt ttaaaataag taaataaata ataaaagaaa gaaagaacgt ttaaaatttt 11940
   tttaaatttg ataaaaatta ttatggtggg ttgaagaatg gtgttttttt taagatatgt 12000
   ttatattata ttttgatttt tatagtttat gaatgagatt ttattttaaa aaagggtttt 12060
   tgtagatata attaagttaa ggattttaag atgaaataat tttggattat ttagggggtt 12120
   ttaaatttaa tgataggtgt ttttttaaga tatttagaga ggtgggaagg ttatgtgaag 12180
   acggaggtag agattagagt tttgtagtta taagttaaga gggttgtggt ttagttatta 12240
   gaatttgaaa gagataaggt aggatttttt tttagagttt ttggaaggat tgttattttg 12300
   ttaatatttt aaatttgtat ttatgttttt cgaattgtga tagaataaat tttttttttt 12360
   tttttttgag atggaatttt attttgtcgt ttaggttgga gtgtagtggc gtgattttag 12420
   tttattgtaa gtttcgtttt ttcggtttac gttatttttt tgttttagtt ttttaagtag 12480
   ttgggattat aggtatttat tattacgttc ggttaatttt ttgtattttt agtagagacg 12540
   gggttttatc gtgttagtta gtatggtttc gattttttga tttcgtgatt cgttcgtttc 12600
   ggttttttaa aatgttggga ttataggcgt gagttattat attcggttaa atatttgttg 12660
   ttttaattta tttagtttgt agtaatttgt tgtggaaatt aatattaaga taaagttaag 12720
   aattaagaaa tttaatgaat tttaagtaag ataaatacga agtaaattat attaaggttt 12780
   attataatta aatgttgaaa attagtggta aaaagaaaat tttaaaagta tttaaagaaa 12840
   tgaggtttat tatgtatgga agaatagata taggaaatat tatatttttt ttattagaaa 12900
   ttatgtaagt taggaaataa tataatgaaa tttttaaagt gttgggggaa aaaaataaaa 12960
   ataaaaattt ttaatttaga attttttttt tttttttttt taatttagta tgattggtat 13020
   tgatttagaa ttttatattc ggtgaaaata ttttttaaaa ttgaagaaga aataaatatg 13080
   ttggtagata aataaatttt aagggaattt attgttagat gtgtaggaaa tgttaaaggg 13140
   agttttttag gtggaaagaa aatgatttta gatagaaatt tgaatttgta taaaggattg 13200
   aagagtgtta aaaaatgtta aatgtataag aatgttagga ggttaggtat agtggtttat 13260
   gtttgtaatt ttagtatttt gggaggtcga ggcgggtagg ttattagagg ttaggagttt 13320
   gagattagtt tggttaatat ggtgaaattt tatttttatt aaaaatataa aaattagttg 13380
   ggcgtggtgg gggatttttg taattttagt tattagggag gttgaggtat aagaatcgtt 13440
   tgaatttggg aggtggaggt tgtagtgaat agagatcgta ttattgtatt ttagtttggg 13500
   tgatagagag agattttgtt ttaaaaaaaa aaaaaaagaa tgttaggttg ggcgtagtgg 13560
   tttatgtttg taagttttgg gaggtcgagg tgggtagatt atttgagttt aggagtttga 13620
   aattagcgtg ggtaatgtgg tgaaattttg tgttataaaa aaatattaaa attagttagg 13680
   cgtggtggtg tatgtttatc gttttagtta ttgggaggtt gaggtgggag gattttttga 13740
   gttagggagt tagaggttgt agtgagtaga gattatttta ttgtatttta gtttaggtaa 13800
   taaagttaga ttttgtttta aaaaaaaaaa aaaaaaaaag ttagtaaaat atttaaaatt 13860
   tataagaata aagagaagag atgttagtag atgagaaatt ttaatataat tttagaatat 13920
   ggaaagtggt tggggtttta taattgattt aggggagtag aggaagttga aattaaagta 13980
   gttatagagg gaaacgttta tataaggagt aagttaggtg atttttagtt tttttgaaag 14040
   gtgtaggtag gatttgtagg taatggggat ggtagaggtg gaggtgaagt tagagttgag 14100
   tgtagggagg ttggttataa tttaggattt tttggttttt tttttagttt tattaagttg 14160
   ggagatgttt ttttttttta ttataggata ttaggggttt attttttgta gaaattgggt 14220
   atgagatgtt ttggatttag gagtattggt tatagtagag gatgaggtat ggtgttgaaa 14280
   gttagtgatt cgtgaaattt tagttttttt tttatttggg ttttgaatta ttggtagtta 14340
   ggttatatta ttattatttt atttattcga gtaggaagtt gttttaaaga ttttagaata 14400
   tagatatttg gtgattttat aatgaatagg ttagtttgtt atttgattat tttatagtag 14460
   tatttatata tttttgagtt ttttagtttt atttttaaat atgatttaat agttaggaga 14520
   aattggatat ttgatgaaag ttttttatgt gaaataaatt aaaatatata aattagatgt 14580
   taagacggtt gggtgtagtg gtttacgttt gtaattttag ttttttggga ggtcgaggtg 14640
   ggcggattac gaggttagga gatcgagatt attttggtta atacggtgaa atttcgtttt 14700
   tattaaaaat ataaaaaatt agttgggcgt ggtggcgggc gtttgtagtt atagttattc 14760
   gggaggttga ggtaggagaa tggtgtgaat tcgggaggcg gagtttgtag tgagtcgaga 14820
   ttgcgttatt gtattttagc gcgggggata gagcgagatt tcgtattaaa aaaaaaaaaa 14880
   aaaaaaaaaa aaaaaataaa ttagatgtta agaatttaga ggaaattttt ttgatgtagg 14940
   gaataaaaga aaattttttg ggggaaaata gtaatttata tttttaggaa aaatataaaa 15000
   agattattga atttatgaaa taaaatggga tagtttggtt ttttatgacg ttttttgttt 15060
   ttttttttgt tttttttatt ttggaagtga tttttttaat ttttttttgt tttttgtatt 15120
   tttggatagt tgtatttgtt tttataaata gtaaatatta tttggattta ttgtattttt 15180
   aggtggaaaa aaaaattaat tttaaaattg tttatatttt tttttttttt cgtatttttt 15240
   atatttaatt tattagaaaa atttgtaggt tttgttttta aaatagattt agaagtcgtt 15300
   tagtttttat tattttgtag ttgttgtttt tttatttttt attggtagtt ttttattgtt 15360
   tttttgttgt ttttcggttg ttaggtttta ttgtttattt tatttatggt agttaaagtt 15420
   attttgtgaa gtgttattta ggttttgata ttttttagtt taaaattttt tagtggtttt 15480
   ttatcgtagg tagtagggaa tttaatggtt ttagattttt ttttgaattt atttgtttta 15540
   tttttcgtta atggtttttt tcggaacgtt ttttaggatt ttttagagag ttttaaagtt 15600
   tttttttttt ttttatttag gttgttgttt aaatgttatt tttttagaaa agtttatttt 15660
   gatagtttat ttgaggtaat agttgttatg gttttattta ttgttcgttt aagtgtttgt 15720
   tgagtatttt ttgtatttcg aatagaagtt ttattagtta ggtttttgtt tgttttgttt 15780
   attgttggtt ttttattttt taggtagtat ttggtatata aatatttgtt agataaatgt 15840
   atagagtatt aggttgaggg ggtataagga ggggaaggta attaattaag aaattattgt 15900
   agaagtatat tgtttatgtc ggggggttta cgtttgtaat tttagtattt tgggaggtta 15960
   aagtaggtgg attatttgag tttaggagtt cgagattagt ttgagtaata tggtaagatt 16020
   ttatttttat aaaaaaaaat ataaaaatta gttaggtatg gtggtattat ttgtagtttt 16080
   agttttttgg gaggttgaga tgggaggatt atttgagttt gggaggtaga ggttgtattg 16140
   agttgtgatt atattattgt attttagttt gggtgataga gtgagacgtt gttttatgaa 16200
   aaaagaaaaa aaagaagaag aaagaaagag aaagggaggg aggtagggaa gggagggagg 16260
   gagggagaag gggaggggag agggtagtat aaataatata ttgtttataa ttagggaggt 16320
   aaatgataaa aggagtttag agtgttgggg aggtgggaga ggcggagtaa ggagtattgt 16380
   tttttattac gtatttttta gtaatagtta aatattttaa agtatgatta tgaattttat 16440
   tgttaaaaat gaaatatttt aaataattta agattattta tataattttt atagttttgt 16500
   tttggaattt ttttattttt taatatattg ggagttttaa aaaaattagc gggtttggtt 16560
   tttgttggtt tttgagttgt ggttttttat agagaagggg agtgttgttt atttttgttt 16620
   aattttggtt gggttggatt tcggggtttg taatttgggt ggggttttga gttcggtagc 16680
   gggagtggga gagggttata gggttcgagt tgattgggga agtgttgagt aatttaggtt 16740
   ttttagtttt gttttagatg ttgtagtcgt agtcgttgta gttaataaga ttttagggag 16800
   ttggtaggtc gttgtttggt tttagggttt tagatgttgt ttttattttt ttattaggtg 16860
   ttttagtttt aggttttaga gagcggttgg tatttttttg taagtagggt gttttttttt 16920
   atttttatat tttaggagta ttttttaggg aggtttggat ggtttagtat tttatatatt 16980
   ttatgtagaa ttttatatat ttagttaagt tttagagtag ttttttttat aagtttagag 17040
   tgtcgtttgt agtgtaagtg gtattttgtt ttgggtttag gtaggagaat ttattaattt 17100
   ttttagtaga ggtttttagt ttttttttag tagaggtttt tagttattta attgttggaa 17160
   tgaaaaggtt ttttatgaaa tttaatattt agtttttttt attaagagaa cggagatttt 17220
   ttattttagt aggttttagt tggagttaaa aggtggagtt tttggagttt tgtagttggg 17280
   ggtttttggg tagagagttt ttttattttt atttaggtat atatattata ttttagtata 17340
   tattggtatt atatagatat atattcgtac gtttataggt atttacgtgt gtttttaagt 17400
   gtataagtat atgtatgttt agttgttttt tggttttgta ggagtttatt tttgtgtaac 17460
   gtgtgtgtgc gtatttgtat aggaatattt tttatattta tgtgtattag tagggtattt 17520
   tttaaatttt tttagtatga gtagaatttt tttgttaagg gaagtatgaa aagtatatat 17580
   tttatgtatg gattaaatat agtgtttgcg aggaaggtga gggacgtaga gtttgtgttt 17640
   aaggtggtgt ttgtttagtt ttttttttag tttacgagga ggaaggtagg agtttggtgt 17700
   agtcgttgtt tgattttttt tgtttagcgt ggggtgtagg ggtaggtttg tttgagttta 17760
   tttaagggga gggaggtagg atgtattttt aagttgcgta ggaggttacg ggttgtgggg 17820
   atcgtatttt ggattttttt ttttatgtta ttttttttat tttgtttttg tagttttttt 17880
   tttttatttt agaatatatt gatttttggt ttttggggtt tgtggttttt tttttcgagt 17940
   tttttagttt attagttgtg gtgatgtttt atatttagga ttgtttagtg taggtaaagg 18000
   gaggggtttg aggtgttttt cgtatatttt gggttttata atttattttt tttgttttta 18060
   ggttagaagg gtgaataggg aaagtaggaa gtaagtttac ggtggagaac gttttaggga 18120
   gaaggaaaat gattttttgg gattataatt tgtttatttt agggttttgg ttgttagggg 18180
   tagagttttg ttttttaggt ttagtttata tatgtttttt ttacgggagt tgtataggtt 18240
   aatttttttt tttgttttta ttttagggaa tattaggaat ttacgatttt agaattagga 18300
   ttatgtttag ttggttatgt taggtaaggg ggaattggta tattttttgg tttataattt 18360
   tgtttttttc gatttggtag agaatatgta tagcggtaag tagggtatcg ggttggtatt 18420
   atagtattgt tgggtgtttt ttttttttat taggtatttt ttatttgttt attatttttt 18480
   gtttttagtt tttagtagag taatttgggg ttgagtaaag tttattaatt tgttttgaag 18540
   gtggttatgt tggttttttt tttagttagt tgtattgtta agtttgttta cggagggttt 18600
   ttgcggggtt tttttggagt gaagaagagg tagttatgtt aagttaagat tagggaaatt 18660
   tggtttttga gtgtttgagg aaagtcgagt tgtttttgtg gcggttggta ttagggggat 18720
   tagaggttag ggatttttgg ttgggatgtt agggttttat agtgtttttt ttatgatgtt 18780
   aggtggttgt ttttaggttt tttttgtaga aaatagagtt tggtgttttg gggagtaggt 18840
   agtgtatatt tggatataag ttttattttg ggttagtttt cgagagggga gatagttagg 18900
   gaattttttt atttatacgg agtggtattt ttttttttgg tacgtttgag ggaaggagag 18960
   gattggtttg gtttttttgt ggttgaataa gggttaattt ttattagtgt tttatatttt 19020
   ttttggtttg gatttaggat aggtagtgtt tattttaagg agatattaat ttattggggt 19080
   aggggtaggg gtgaatttgg gattttgttt ttgaggtagt tagaggggtg ggagagagaa 19140
   atatatgagt agtttttaat atttttattt gtagcggttt cggtgttttt tattttaatt 19200
   ttattttagt aagatggtta tttattttag gattattagt agagaggttt gttggagaag 19260
   tttttttttt tagggtattt tgtttttttt tagtttttgt agtaaaagga ttatttattt 19320
   ttaggtaagg gagtgggatt gaggtatttt ggagatttag ttttttgttt agtgattttt 19380
   ggatttttgg gaagtttttt ttatatgagg tttgtttttt tttgttttta gggaaggaaa 19440
   gtagagaaga gttagtagag gttggttggt tatgttttag cgttttgtat attattttgg 19500
   aattttttgt ttatatttgt tttattagaa tagagatttt ttgagggtag gggttgatgt 19560
   tttgtttgtt tatttgtttt ttgggggtat tgtgatttat tttttttaat aggtttttcg 19620
   tggttgtaga gtattgtttt tatgatagat aaataaagat agggtttgag gagtttagtt 19680
   tgaggagaaa ttcggagagt gttggtatag gtataggtaa taggaagtgg ttatttagat 19740
   cggtttagag gtttgcgggt ataggttatt cgtatattta tagggattgt tatgtagagt 19800
   tggatgttat gtaggtttgg gggttggttt agcgtgggat tttagagata tattttgatt 19860
   tttagtttgt ttagttcgat ggtttgtttt ttatttttat gttttttttg gcgattgtat 19920
   agttttttag attgaatttt ttatgattta ataagtagtt tttagaaggt aaatttttat 19980
   agcgggtaat tgcgttaatt atttttagtt tatggtttta aggttttaga tttagtttaa 20040
   aattggggtt tttagtttgt agtaagttat gggattcggg tttagttttg gtttttgtag 20100
   tttattttgg aggcgggggg ttatttttgt tatgagtgtt tatttgtggt ttgggtgtta 20160
   agggtagaag tggtaatttg tgatttgtgt ttgtttttgt tttgaagtat ggttagaaga 20220
   tattttgaag tatggttaga agatattttt tttttttttg ttttttggag attattgttt 20280
   agtttaaagt tggtgattta cgtggtttta ttgattagta gaaattaggg agtttatttg 20340
   gtgcgtagaa ttttaagttt ttttagtagt tagggttttt tagttttttt tgcgtttaga 20400
   aattaggttt tagtattaga gggtaggaga gcgtttttta gtatttggat aaggtttttg 20460
   gggtttttta agagggtttt tttttatttt ttgtttttga gttggggttg ggttttgaga 20520
   gatttaagga tttgtttagt ttgagagtta aaagagaaag cgtttatcgt tggggagagg 20580
   gcgttgggtg gggttggggt gtttgggagt gtgatatagt ttttttttta tttttgattt 20640
   atggtttttt ttttacgtaa gtttttttta ttttcgtatt tttattcggg aggtgtcgtc 20700
   gtttaattgg ttggggcgga tttttttatt atatggaata tcgatatagt tcgtagtttt 20760
   tttttgtagt ttttattgtt tcggcggagg aggggtatag aggtttttat ttttatttag 20820
   gtgagttcga aatacgtgtg gtttcgttcg tttcggtcgg aggggattat tttttttcgg 20880
   ttaataagta taaaagtcgc ggtcgtaaat aaagacgcgt cgtaaattcg ttagggaggc 20940
   gggatagttc gcgtcgtatt ttttttttag gtcggacggg cgtgttcggt cgcggggttt 21000
   tcgcggcgtt tcgaaggcgt ttttatttgg ttcgcgcgga tttttttttt tagttcgcgg 21060
   tggcggttgt gttatacgag gtagtgtagc ggatatttgt tttcgggttt ttttttggcg 21120
   gaggcgttgg cggggattat cgttcgtttt ttatgtcggg gtcgcggcgt tcggtcgggg 21180
   aggagggacg gcgtcggagt cggggttcgg ggtcgcgggg cggggggtcg gcgttgtttg 21240
   ttcgcggggt tgcggttcgg gtatgtgcga gcggcggcgg gcgggggcgg cgcgttcggc 21300
   gcggtgcggg cggcgcgggc gacgggcgtt ttcgcgaggc ggcggcggcg ggcggcgcgg 21360
   agaggttttt attcgttttg ggagggagcg ggtattcggc ggcgttgcgc ggattcgcgt 21420
   tcgtttttta tattttcgta tataaagacg tcgttcggcg gagggagtag gcgttcgtag 21480
   cgggcgcggg ggcgtacggg acgcgggttt cggggacgtc gtcgtaggga cgcgcgttcg 21540
   tagttttcgg gtttttatag ttgtatagtt cggtttttag ttcgagcggt cggcgcggga 21600
   tacggggatg tggggatgat ggggagagag gaaggggatt aggcgtttcg ggtagagttt 21660
   gttagtttag tttcgcggta ttatggggtt tgtagttcgt ttcgtgaaat tgggggtggt 21720
   aggtggtcga agagggtttg tattgttttc ggatttcgtg ggagagtggg cggggagtta 21780
   ggtttggttt agacgcgggc ggggttttga cgttaggtgg gttgggacgt atttttttcg 21840
   ttggtatttt gttttttgtt ggggaagtgg gggggtacgg gtttaggtcg agcgtttggg 21900
   agacggggtg gaggtgtggt ttttagaatt gagtttgggt tttaggggtt atgaagaagg 21960
   attcggattt ttagaagaga gcgtttgatt gggaattttt gagaaaggtg tggggtatta 22020
   gtattaaggg ttattggagt agttaggggt ttggtttttg gaatattttt gaggagtttt 22080
   ttttgttttg ttagaaaatt aaattttatt ttttatatat gttattggaa atagaatgtt 22140
   tagtaacgcg ggaaagatgg tttttttttt ttcgtatatt gtttttagta agaattagag 22200
   tgaggttgga tagaaaaagt agtaggtggt agtttcgttt ttttatttag ttggtttttt 22260
   aggaagggaa gtgattgatt ttagagattg tttttttttt gaatgatttt agggtgaaga 22320
   ggtattttta gaatggtttt tgtagagggt gtggtaaaag ttgaattggt tttttaagcg 22380
   ttaattgtaa ggtttggaag gattaagagg tttggattag tttttggagg gaggggattg 22440
   tgatgttaga gagattagag ttcgaatagt tttaattgtt tgattttaaa agagtaaatt 22500
   gtttaatttt ttcgattttt tcggtttatt tttttttaaa atgagaattt agatattatt 22560
   ttttggggtg agttaaggat aatgattgtt ttggaaatag cgtttttttt ttttaattag 22620
   gcgggtcgag ataggtggcg ggattaggta cggtgttttt ttgagtttat tttagaataa 22680
   ggtaggaata agggatttta tatcggtttt atttaaaata ataatttaag aagtgtttgt 22740
   gtgggaggtg ttagtaaaag tttgtaaatt tttttaggtt tttttgtgtg gttttttttt 22800
   tttttttttt tatattttgt ggtgttaggt tttagcggga agagatgagg gaggagcggg 22860
   ttgtttcgtt tttttttttc gtttgggagg agttagttgg cgttttcggg ggttcgggat 22920
   cgttgttcgt ttgtattatt aggggttagt agagtgtcgt ggttgtagtt agtgtagggg 22980
   agggggttgg cgcggttgag gggtatttag gagtggtcgg gagtatgttt gtgtattttt 23040
   ttattaaatt gtttttcgaa ttttgtgtgt gattttattg tagtagtttt gttttttggg 23100
   gtggtgatat ttttacgggg gaatttagaa atttttttat ttttagtttt aggtttagaa 23160
   gtttggttgt gacggggttg ggggaaataa agtttggacg atgtgttcgc gttagggtta 23220
   tttttaggta gtttttgagt taattaggag atggggagtg gggtggaggg gtaagtagag 23280
   tgaattattt ttttttttta gtaaagttta ggagttcgag gataggggga gtttaggatg 23340
   gattgtgggg agttgtcgat tgtggggaat tgttttttgg gcggtcgtgt tttttttttg 23400
   ttaggcggcg gtagatattg aatttaggtc gtttgcgttt cggttcgcgt ttttagttgt 23460
   tacgtgttcg agacgcgttt atatagcgga gaaataaaga tagttaagcg gtagggttgt 23520
   tagtagtttt ttcgatattt aataaatgaa attttatagt tgggggtcga tcgggggtga 23580
   acgggcggtt atcgttcgtg gattgagcgt ttggtacggg ttgtttggtt atttttttcg 23640
   ttttattttt ttcgtttttt attttttttt cgttgaggtt attaagttta tggattttgg 23700
   tgagagagta gttcggggcg gtaggtggtg ttttttttta tatattagtt aggtatcgtt 23760
   gtttgtaaaa gttttattag ttgagcggtt ggttgtcgag ggaggggagg tttgtaagat 23820
   ttttttaagg tgtaattata aataaagttt atggatcgcg attttaaata aatttgttaa 23880
   tttgggtcgt agtaaaaggc gatggaggta gttttgtatg tatttcgggt ggtgtgattt 23940
   atgagtatta tttgtttttt ttagggaagg agtttgttta tttataaatt attgtcgttt 24000
   tggtatcgtt gggataatcg attaatttga gtatttttgt taaaggattg tttttatttt 24060
   aataagtagg agtttttgat ttttggagaa ttttttttta attgttatta ttgatttggt 24120
   ttttaggttt ggttttgggg atttttattt tttttttata agtagagtgt tcgggttttt 24180
   tgttgttgta gggattgggg gttgggtata cgcgcggtat acgcgtatgg gggcgtatgt 24240
   ttagtgcggg ggttgcgttt atgtgcggtt tttatttagg ttttttgttt tttagtttat 24300
   ttacgttttt tatttgtttt gtttttgttt gtttcgtcgt gtgaagttta ggaagaaagg 24360
   tcgttatttg aaacggtttt tttatgttat atgtattttt tttggatata tatatttttg 24420
   gtggtttaga aatatcgtgg gtgtgtgtaa ggcggatgat aagtggatgt gagtgtatat 24480
   aggtatagtg gttatttttc gggaggtttt taaaaatcgt ttagattttt ttttttttgg 24540
   atggaaggat gtatggagag atagatatat ttttagttcg gaggtagggg aatatattgt 24600
   ttgatttttt tggtatttgg gcgtttatat tgttagtagt gggcgtattt agtaagagtg 24660
   tgatatatat atatatattt ttggatatat agatgtggat atttacggga tagggacgta 24720
   tattttattt taattagaga tagacgggta ttttaaggta tagttttttt gatttggagc 24780
   gggatttggg tttttaagtt tttttttttt aatttttatt ttcgtcgttc gtgggtttag 24840
   cgtttttcgg tttattttac gcggaaagtg aaggggaggg ggttgtgagg gtgatttata 24900
   ttgttttcgg gttaggcgag cgttgtagcg gaggttaggg tcgtaggtta cgttgttcgt 24960
   cggggtcggg tatttgtcgc ggttttttta agt 24993
<210> 11
   <211> 1472
   <212> DNA
   <213> Homo sapiens
<400> 11
   cgcacggcca tttctcctga gcccgtggcg ggccgaccgg ggtttagatg aggtcggggt 60
   ggggctgagg ctgaggtgtg aggggctgag tggtggcgcc taaaggtttc ctcatcctca 120
   gaaatcccga tcttccacat cctcaacgcc cgcatcacct tcgggaacct caacggctgc 180
   gacgaaccgg tgccatcggt gcgaggcagc cccagcagcg agacgccttc cccaggcagc 240
   gactcctcca gcgtcagcag ctccagctcc acgagtgagg ccccccgcga gaacgcctgc 300
   ccctcggctc tccccggggt gggtgggagg ggcgcgcccg gccgtgcctg acccagcccc 360
   cgcgccccca gcctcctgcc gcggctgcga gatctcccca gcgttgttcg aggccccgcg 420
   cggctacagc atgatgggcg gccagcggga ggcggcgacc cgggacgacg acgacgacct 480
   gctgcaattc gccatccagc agagcctgct tgaggcgggc agtgagtatg accaggtgcg 540
   tctccgcggg cgcgcggggc cacgggactc cgcgccgcca cggctaacgc ccacgcctcc 600
   ccgcaggtca ccatctggga ggcgctaacc aacagcaagc caggcaccca ccccatgtcc 660
   tacgagggtc gccgacagga caggtcagtg cccgctgggc cggagagaat cctccggaga 720
   ggatcctgtc tcccctaagc caggacacag ccggagaacc cgcggggccc tcggcaggga 780
   ccctcctggt atcgggatgg cttctgccac cactcctgcc tccgaggcgg caggcagggg 840
   tcagggatcc aggcttcacc gcggagttca gactctcccc aggcattgac ggggagaact 900
   tggtagggtc tgggctccgc cacgaccagg tgggggccag gacgccgcgg gactgaccgc 960
   ctccactaga gggtggtggc cgcgggccgc cctggagcct ccacgccgtg tcccggcccc 1020
   ggccggtggg tgggtgcgcc cgggagcccg cgacattcct tccccacccc caggagcgcc 1080
   ccgcccacgc cgcagcgcca gcctgcgccc ccggcgtcag tgcccagccc tcggcccagc 1140
   tcagggccag gttccggcgg ccacgtgttc cggagctacg acgagcagct gcggctggcg 1200
   atggaactgt cggcgcagga gcaggaggag aggcggcggc gcgcgcgcca ggaggaggag 1260
   gagctggagc gcatcctgag gctctcactg accgagcagt agcgccccct gccgggaccc 1320
   tcgccagcgc cacgcgcgcc acgcccaggg ccaggagcca gacaaacccc ggcctgcgcg 1380
   cctgcagagc ggcggctgga gactggagcc accgcctcgc gggtgcagca gcacagcagg 1440
   cacggttcgg ggagggattc ggcatggccg cg 1472
<210> 12
   <211> 1472
   <212> DNA
   <213> Artificial Sequence
<220>
   <223> C to T (bisl)
<400> 12
   cgtacggtta ttttttttga gttcgtggcg ggtcgatcgg ggtttagatg aggtcggggt 60
   ggggttgagg ttgaggtgtg aggggttgag tggtggcgtt taaaggtttt tttattttta 120
   gaaatttcga ttttttatat ttttaacgtt cgtattattt tcgggaattt taacggttgc 180
   gacgaatcgg tgttatcggt gcgaggtagt tttagtagcg agacgttttt tttaggtagc 240
   gattttttta gcgttagtag ttttagtttt acgagtgagg tttttcgcga gaacgtttgt 300
   ttttcggttt ttttcggggt gggtgggagg ggcgcgttcg gtcgtgtttg atttagtttt 360
   cgcgttttta gttttttgtc gcggttgcga gattttttta gcgttgttcg aggtttcgcg 420
   cggttatagt atgatgggcg gttagcggga ggcggcgatt cgggacgacg acgacgattt 480
   gttgtaattc gttatttagt agagtttgtt tgaggcgggt agtgagtatg attaggtgcg 540
   ttttcgcggg cgcgcggggt tacgggattt cgcgtcgtta cggttaacgt ttacgttttt 600
   tcgtaggtta ttatttggga ggcgttaatt aatagtaagt taggtattta ttttatgttt 660
   tacgagggtc gtcgatagga taggttagtg ttcgttgggt cggagagaat ttttcggaga 720
   ggattttgtt ttttttaagt taggatatag tcggagaatt cgcggggttt tcggtaggga 780
   tttttttggt atcgggatgg tttttgttat tatttttgtt ttcgaggcgg taggtagggg 840
   ttagggattt aggttttatc gcggagttta gatttttttt aggtattgac ggggagaatt 900
   tggtagggtt tgggtttcgt tacgattagg tgggggttag gacgtcgcgg gattgatcgt 960
   ttttattaga gggtggtggt cgcgggtcgt tttggagttt ttacgtcgtg tttcggtttc 1020
   ggtcggtggg tgggtgcgtt cgggagttcg cgatattttt tttttatttt taggagcgtt 1080
   tcgtttacgt cgtagcgtta gtttgcgttt tcggcgttag tgtttagttt tcggtttagt 1140
   ttagggttag gtttcggcgg ttacgtgttt cggagttacg acgagtagtt gcggttggcg 1200
   atggaattgt cggcgtagga gtaggaggag aggcggcggc gcgcgcgtta ggaggaggag 1260
   gagttggagc gtattttgag gtttttattg atcgagtagt agcgtttttt gtcgggattt 1320
   tcgttagcgt tacgcgcgtt acgtttaggg ttaggagtta gataaatttc ggtttgcgcg 1380
   tttgtagagc ggcggttgga gattggagtt atcgtttcgc gggtgtagta gtatagtagg 1440
   tacggttcgg ggagggattc ggtatggtcg cg 1472
<210> 13
   <211> 1472
   <212> DNA
   <213> Artificial Sequence
<220>
   <223> rc C to T (bisl)
<400> 13
   cgcgaccata ccgaatccct ccccgaaccg tacctactat actactacac ccgcgaaacg 60
   ataactccaa tctccaaccg ccgctctaca aacgcgcaaa ccgaaattta tctaactcct 120
   aaccctaaac gtaacgcgcg taacgctaac gaaaatcccg acaaaaaacg ctactactcg 180
   atcaataaaa acctcaaaat acgctccaac tcctcctcct cctaacgcgc gcgccgccgc 240
   ctctcctcct actcctacgc cgacaattcc atcgccaacc gcaactactc gtcgtaactc 300
   cgaaacacgt aaccgccgaa acctaaccct aaactaaacc gaaaactaaa cactaacgcc 360
   gaaaacgcaa actaacgcta cgacgtaaac gaaacgctcc taaaaataaa aaaaaaatat 420
   cgcgaactcc cgaacgcacc cacccaccga ccgaaaccga aacacgacgt aaaaactcca 480
   aaacgacccg cgaccaccac cctctaataa aaacgatcaa tcccgcgacg tcctaacccc 540
   cacctaatcg taacgaaacc caaaccctac caaattctcc ccgtcaatac ctaaaaaaaa 600
   tctaaactcc gcgataaaac ctaaatccct aacccctacc taccgcctcg aaaacaaaaa 660
   taataacaaa aaccatcccg ataccaaaaa aatccctacc gaaaaccccg cgaattctcc 720
   gactatatcc taacttaaaa aaaacaaaat cctctccgaa aaattctctc cgacccaacg 780
   aacactaacc tatcctatcg acgaccctcg taaaacataa aataaatacc taacttacta 840
   ttaattaacg cctcccaaat aataacctac gaaaaaacgt aaacgttaac cgtaacgacg 900
   cgaaatcccg taaccccgcg cgcccgcgaa aacgcaccta atcatactca ctacccgcct 960
   caaacaaact ctactaaata acgaattaca acaaatcgtc gtcgtcgtcc cgaatcgccg 1020
   cctcccgcta accgcccatc atactataac cgcgcgaaac ctcgaacaac gctaaaaaaa 1080
   tctcgcaacc gcgacaaaaa actaaaaacg cgaaaactaa atcaaacacg accgaacgcg 1140
   cccctcccac ccaccccgaa aaaaaccgaa aaacaaacgt tctcgcgaaa aacctcactc 1200
   gtaaaactaa aactactaac gctaaaaaaa tcgctaccta aaaaaaacgt ctcgctacta 1260
   aaactacctc gcaccgataa caccgattcg tcgcaaccgt taaaattccc gaaaataata 1320
   cgaacgttaa aaatataaaa aatcgaaatt tctaaaaata aaaaaacctt taaacgccac 1380
   cactcaaccc ctcacacctc aacctcaacc ccaccccgac ctcatctaaa ccccgatcga 1440
   cccgccacga actcaaaaaa aataaccgta cg 1472
<210> 14
   <211> 1472
   <212> DNA
   <213> Artificial Sequence
<220>
   <223> G to A (bis2 rc)
<400> 14
   cgcacgacca tttctcctaa acccgtaacg aaccgaccga aatttaaata aaatcgaaat      60
   aaaactaaaa ctaaaatata aaaaactaaa taataacgcc taaaaatttc ctcatcctca     120
   aaaatcccga tcttccacat cctcaacgcc cgcatcacct tcgaaaacct caacgactac     180
   gacgaaccga taccatcgat acgaaacaac cccaacaacg aaacgccttc cccaaacaac     240
   gactcctcca acgtcaacaa ctccaactcc acgaataaaa ccccccgcga aaacgcctac     300
   ccctcgactc tccccgaaat aaataaaaaa aacgcgcccg accgtaccta acccaacccc     360
   cgcgccccca acctcctacc gcgactacga aatctcccca acgttattcg aaaccccgcg    420
   cgactacaac ataataaacg accaacgaaa aacgacgacc cgaaacgacg acgacgacct     480
   actacaattc gccatccaac aaaacctact taaaacgaac aataaatata accaaatacg     540
   tctccgcgaa cgcgcgaaac cacgaaactc cgcgccgcca cgactaacgc ccacgcctcc     600
   ccgcaaatca ccatctaaaa aacgctaacc aacaacaaac caaacaccca ccccatatcc     660
   tacgaaaatc gccgacaaaa caaatcaata cccgctaaac cgaaaaaaat cctccgaaaa     720
   aaatcctatc tcccctaaac caaaacacaa ccgaaaaacc cgcgaaaccc tcgacaaaaa     780
   ccctcctaat atcgaaataa cttctaccac cactcctacc tccgaaacga caaacaaaaa     840
   tcaaaaatcc aaacttcacc gcgaaattca aactctcccc aaacattaac gaaaaaaact     900
   taataaaatc taaactccgc cacgaccaaa taaaaaccaa aacgccgcga aactaaccgc     960
   ctccactaaa aaataataac cgcgaaccgc cctaaaacct ccacgccgta tcccgacccc    1020
   gaccgataaa taaatacgcc cgaaaacccg cgacattcct tccccacccc caaaaacgcc    1080
   ccgcccacgc cgcaacgcca acctacgccc ccgacgtcaa tacccaaccc tcgacccaac    1140
   tcaaaaccaa attccgacga ccacgtattc cgaaactacg acgaacaact acgactaacg    1200
   ataaaactat cgacgcaaaa acaaaaaaaa aaacgacgac gcgcgcgcca aaaaaaaaaa    1260
   aaactaaaac gcatcctaaa actctcacta accgaacaat aacgccccct accgaaaccc   1320
   tcgccaacgc cacgcgcgcc acgcccaaaa ccaaaaacca aacaaacccc gacctacgcg    1380
   cctacaaaac gacgactaaa aactaaaacc accgcctcgc gaatacaaca acacaacaaa    1440
   cacgattcga aaaaaaattc gacataaccg cg                                  1472
<210> 15
   <211> 1472
   <212> DNA
   <213> Artificial Sequence
<220>
   <223> G to A (bis2 rc) rc
<400> 15
   cgcggttatg tcgaattttt tttcgaatcg tgtttgttgt gttgttgtat tcgcgaggcg 60
   gtggttttag tttttagtcg tcgttttgta ggcgcgtagg tcggggtttg tttggttttt 120
   ggttttgggc gtggcgcgcg tggcgttggc gagggtttcg gtagggggcg ttattgttcg 180
   gttagtgaga gttttaggat gcgttttagt tttttttttt tttggcgcgc gcgtcgtcgt 240
   tttttttttt gtttttgcgt cgatagtttt atcgttagtc gtagttgttc gtcgtagttt 300
   cggaatacgt ggtcgtcgga atttggtttt gagttgggtc gagggttggg tattgacgtc 360
   gggggcgtag gttggcgttg cggcgtgggc ggggcgtttt tgggggtggg gaaggaatgt 420
   cgcgggtttt cgggcgtatt tatttatcgg tcggggtcgg gatacggcgt ggaggtttta 480
   gggcggttcg cggttattat tttttagtgg aggcggttag tttcgcggcg ttttggtttt 540
   tatttggtcg tggcggagtt tagattttat taagtttttt tcgttaatgt ttggggagag 600
   tttgaatttc gcggtgaagt ttggattttt gatttttgtt tgtcgtttcg gaggtaggag 660
   tggtggtaga agttatttcg atattaggag ggtttttgtc gagggtttcg cgggtttttc 720
   ggttgtgttt tggtttaggg gagataggat ttttttcgga ggattttttt cggtttagcg 780
   ggtattgatt tgttttgtcg gcgattttcg taggatatgg ggtgggtgtt tggtttgttg 840
   ttggttagcg ttttttagat ggtgatttgc ggggaggcgt gggcgttagt cgtggcggcg 900
   cggagtttcg tggtttcgcg cgttcgcgga gacgtatttg gttatattta ttgttcgttt 960
   taagtaggtt ttgttggatg gcgaattgta gtaggtcgtc gtcgtcgttt cgggtcgtcg 1020
   tttttcgttg gtcgtttatt atgttgtagt cgcgcggggt ttcgaataac gttggggaga 1080
   tttcgtagtc gcggtaggag gttgggggcg cgggggttgg gttaggtacg gtcgggcgcg 1140
   ttttttttat ttatttcggg gagagtcgag gggtaggcgt tttcgcgggg ggttttattc 1200
   gtggagttgg agttgttgac gttggaggag tcgttgtttg gggaaggcgt ttcgttgttg 1260
   gggttgtttc gtatcgatgg tatcggttcg tcgtagtcgt tgaggttttc gaaggtgatg 1320
   cgggcgttga ggatgtggaa gatcgggatt tttgaggatg aggaaatttt taggcgttat 1380
   tatttagttt tttatatttt agttttagtt ttatttcgat tttatttaaa tttcggtcgg 1440
   ttcgttacgg gtttaggaga aatggtcgtg cg 1472
<210> 16
   <211> 1093
   <212> DNA
   <213> Homo sapiens
<400> 16
   tcggcaggga ccctcctggt atcgggatgg cttctgccac cactcctgcc tccgaggcgg      60
   caggcagggg tcagggatcc aggcttcacc gcggagttca gactctcccc aggcattgac     120
   ggggagaact tggtagggtc tgggctccgc cacgaccagg tgggggccag gacgccgcgg     180
   gactgaccgc ctccactaga gggtggtggc cgcgggccgc cctggagcct ccacgccgtg     240
   tcccggcccc ggccggtggg tgggtgcgcc cgggagcccg cgacattcct tccccacccc     300
   caggagcgcc ccgcccacgc cgcagcgcca gcctgcgccc ccggcgtcag tgcccagccc     360
   tcggcccagc tcagggccag gttccggcgg ccacgtgttc cggagctacg acgagcagct     420
   gcggctggcg atggaactgt cggcgcagga gcaggaggag aggcggcggc gcgcgcgcca     480
   ggaggaggag gagctggagc gcatcctgag gctctcactg accgagcagt agcgccccct    540
   gccgggaccc tcgccagcgc cacgcgcgcc acgcccaggg ccaggagcca gacaaacccc     600
   ggcctgcgcg cctgcagagc ggcggctgga gactggagcc accgcctcgc gggtgcagca     660
   gcacagcagg cacggttcgg ggagggattc ggcatggccg cggggtacct tcccaggcca     720
   gggccctgga ggcaactggc acggcctggt ccccctgctt tgctgtattc tgattcccca     780
   acccgctccc ctgggctcag atctgtcctg tcctagggcg gagccaggcg gtcctgaggg     840
   ggagatgaat ccttagagga gcgctgtccc tatcccttgc tccttctggc cggtccgcat     900
   ttctgttcac taaccccact ccaggacact gcccctgaag cctttgcatc tctgctcttc     960
   actcctgggg ggcagctgag ctccccgcgt gctgtgtcac tgctttgttc cagacacaaa    1020
   ccagcacgtc tagggcccag cccctccccc accccggcat ttcagcgtca agtgcactta    1080
   gcggggtacc cgg                                                       1093
<210> 17
   <211> 1093
   <212> DNA
   <213> Artificial Sequence
<220>
   <223> C to T (bisl)
<400> 17
   tcggtaggga tttttttggt atcgggatgg tttttgttat tatttttgtt ttcgaggcgg 60
   taggtagggg ttagggattt aggttttatc gcggagttta gatttttttt aggtattgac 120
   ggggagaatt tggtagggtt tgggtttcgt tacgattagg tgggggttag gacgtcgcgg 180
   gattgatcgt ttttattaga gggtggtggt cgcgggtcgt tttggagttt ttacgtcgtg 240
   tttcggtttc ggtcggtggg tgggtgcgtt cgggagttcg cgatattttt tttttatttt 300
   taggagcgtt tcgtttacgt cgtagcgtta gtttgcgttt tcggcgttag tgtttagttt 360
   tcggtttagt ttagggttag gtttcggcgg ttacgtgttt cggagttacg acgagtagtt 420
   gcggttggcg atggaattgt cggcgtagga gtaggaggag aggcggcggc gcgcgcgtta 480
   ggaggaggag gagttggagc gtattttgag gtttttattg atcgagtagt agcgtttttt 540
   gtcgggattt tcgttagcgt tacgcgcgtt acgtttaggg ttaggagtta gataaatttc 600
   ggtttgcgcg tttgtagagc ggcggttgga gattggagtt atcgtttcgc gggtgtagta 660
   gtatagtagg tacggttcgg ggagggattc ggtatggtcg cggggtattt ttttaggtta 720
   gggttttgga ggtaattggt acggtttggt ttttttgttt tgttgtattt tgatttttta 780
   attcgttttt ttgggtttag atttgttttg ttttagggcg gagttaggcg gttttgaggg 840
   ggagatgaat ttttagagga gcgttgtttt tattttttgt tttttttggt cggttcgtat 900
   ttttgtttat taattttatt ttaggatatt gtttttgaag tttttgtatt tttgtttttt 960
   atttttgggg ggtagttgag tttttcgcgt gttgtgttat tgttttgttt tagatataaa 1020
   ttagtacgtt tagggtttag tttttttttt atttcggtat tttagcgtta agtgtattta 1080
   gcggggtatt cgg 1093
<210> 18
   <211> 1093
   <212> DNA
   <213> Artificial Sequence
<220>
   <223> rc C to T (bisl)
<400> 18
   ccgaataccc cgctaaatac acttaacgct aaaataccga aataaaaaaa aaactaaacc 60
   ctaaacgtac taatttatat ctaaaacaaa acaataacac aacacgcgaa aaactcaact      120
   accccccaaa aataaaaaac aaaaatacaa aaacttcaaa aacaatatcc taaaataaaa      180
   ttaataaaca aaaatacgaa ccgaccaaaa aaaacaaaaa ataaaaacaa cgctcctcta      240
   aaaattcatc tccccctcaa aaccgcctaa ctccgcccta aaacaaaaca aatctaaacc      300
   caaaaaaacg aattaaaaaa tcaaaataca acaaaacaaa aaaaccaaac cgtaccaatt      360
   acctccaaaa ccctaaccta aaaaaatacc ccgcgaccat accgaatccc tccccgaacc      420
   gtacctacta tactactaca cccgcgaaac gataactcca atctccaacc gccgctctac      480
   aaacgcgcaa accgaaattt atctaactcc taaccctaaa cgtaacgcgc gtaacgctaa      540
   cgaaaatccc gacaaaaaac gctactactc gatcaataaa aacctcaaaa tacgctccaa      600
   ctcctcctcc tcctaacgcg cgcgccgccg cctctcctcc tactcctacg ccgacaattc      660
   catcgccaac cgcaactact cgtcgtaact ccgaaacacg taaccgccga aacctaaccc      720
   taaactaaac cgaaaactaa acactaacgc cgaaaacgca aactaacgct acgacgtaaa      780
   cgaaacgctc ctaaaaataa aaaaaaaata tcgcgaactc ccgaacgcac ccacccaccg      840
   accgaaaccg aaacacgacg taaaaactcc aaaacgaccc gcgaccacca ccctctaata      900
   aaaacgatca atcccgcgac gtcctaaccc ccacctaatc gtaacgaaac ccaaacccta      960
   ccaaattctc cccgtcaata cctaaaaaaa atctaaactc cgcgataaaa cctaaatccc     1020
   taacccctac ctaccgcctc gaaaacaaaa ataataacaa aaaccatccc gataccaaaa     1080
   aaatccctac cga                                                        1093
<210> 19
   <211> 1093
   <212> DNA
   <213> Artificial Sequence
<220>
   <223> G to A (bis2 rc)
<400> 19
   tcgacaaaaa ccctcctaat atcgaaataa cttctaccac cactcctacc tccgaaacga 60
   caaacaaaaa tcaaaaatcc aaacttcacc gcgaaattca aactctcccc aaacattaac 120
   gaaaaaaact taataaaatc taaactccgc cacgaccaaa taaaaaccaa aacgccgcga 180
   aactaaccgc ctccactaaa aaataataac cgcgaaccgc cctaaaacct ccacgccgta 240
   tcccgacccc gaccgataaa taaatacgcc cgaaaacccg cgacattcct tccccacccc 300
   caaaaacgcc ccgcccacgc cgcaacgcca acctacgccc ccgacgtcaa tacccaaccc 360
   tcgacccaac tcaaaaccaa attccgacga ccacgtattc cgaaactacg acgaacaact 420
   acgactaacg ataaaactat cgacgcaaaa acaaaaaaaa aaacgacgac gcgcgcgcca 480
   aaaaaaaaaa aaactaaaac gcatcctaaa actctcacta accgaacaat aacgccccct 540
   accgaaaccc tcgccaacgc cacgcgcgcc acgcccaaaa ccaaaaacca aacaaacccc 600
   gacctacgcg cctacaaaac gacgactaaa aactaaaacc accgcctcgc gaatacaaca 660
   acacaacaaa cacgattcga aaaaaaattc gacataaccg cgaaatacct tcccaaacca 720
   aaaccctaaa aacaactaac acgacctaat ccccctactt tactatattc taattcccca 780
   acccgctccc ctaaactcaa atctatccta tcctaaaacg aaaccaaacg atcctaaaaa 840
   aaaaataaat ccttaaaaaa acgctatccc tatcccttac tccttctaac cgatccgcat 900
   ttctattcac taaccccact ccaaaacact acccctaaaa cctttacatc tctactcttc 960
   actcctaaaa aacaactaaa ctccccgcgt actatatcac tactttattc caaacacaaa 1020
   ccaacacgtc taaaacccaa cccctccccc accccgacat ttcaacgtca aatacactta 1080
   acgaaatacc cga 1093
<210> 20
   <211> 1093
   <212> DNA
   <213> Artificial Sequence
<220>
   <223> G to A (bis2 rc) rc
<400> 20
   tcgggtattt cgttaagtgt atttgacgtt gaaatgtcgg ggtgggggag gggttgggtt 60
   ttagacgtgt tggtttgtgt ttggaataaa gtagtgatat agtacgcggg gagtttagtt 120
   gttttttagg agtgaagagt agagatgtaa aggttttagg ggtagtgttt tggagtgggg 180
   ttagtgaata gaaatgcgga tcggttagaa ggagtaaggg atagggatag cgttttttta 240
   aggatttatt ttttttttag gatcgtttgg tttcgtttta ggataggata gatttgagtt 300
   taggggagcg ggttggggaa ttagaatata gtaaagtagg gggattaggt cgtgttagtt 360
   gtttttaggg ttttggtttg ggaaggtatt tcgcggttat gtcgaatttt ttttcgaatc 420
   gtgtttgttg tgttgttgta ttcgcgaggc ggtggtttta gtttttagtc gtcgttttgt 480
   aggcgcgtag gtcggggttt gtttggtttt tggttttggg cgtggcgcgc gtggcgttgg 540
   cgagggtttc ggtagggggc gttattgttc ggttagtgag agttttagga tgcgttttag 600
   tttttttttt ttttggcgcg cgcgtcgtcg tttttttttt tgtttttgcg tcgatagttt 660
   tatcgttagt cgtagttgtt cgtcgtagtt tcggaatacg tggtcgtcgg aatttggttt 720
   tgagttgggt cgagggttgg gtattgacgt cgggggcgta ggttggcgtt gcggcgtggg 780
   cggggcgttt ttgggggtgg ggaaggaatg tcgcgggttt tcgggcgtat ttatttatcg 840
   gtcggggtcg ggatacggcg tggaggtttt agggcggttc gcggttatta ttttttagtg 900
   gaggcggtta gtttcgcggc gttttggttt ttatttggtc gtggcggagt ttagatttta 960
   ttaagttttt ttcgttaatg tttggggaga gtttgaattt cgcggtgaag tttggatttt 1020
   tgatttttgt ttgtcgtttc ggaggtagga gtggtggtag aagttatttc gatattagga 1080
   gggtttttgt cga 1093
<210> 21
   <211> 93
   <212> DNA
   <213> Homo sapiens
<400> 21
   gcatcctgag gctctcactg accgagcagt agcgccccct gccgggaccc tcgccagcgc 60
   cacgcgcgcc acgcccaggg ccaggagcca gac 93
<210> 22
   <211> 93
   <212> DNA
   <213> Artificial Sequence
<220>
   <223> C to T (bisl)
<400> 22
   gtattttgag gtttttattg atcgagtagt agcgtttttt gtcgggattt tcgttagcgt 60
   tacgcgcgtt acgtttaggg ttaggagtta gat 93
<210> 23
   <211> 93
   <212> DNA
   <213> Artificial Sequence
<220>
   <223> rc C to T (bisl)
<400> 23
   atctaactcc taaccctaaa cgtaacgcgc gtaacgctaa cgaaaatccc gacaaaaaac 60
   gctactactc gatcaataaa aacctcaaaa tac 93
<210> 24
   <211> 93
   <212> DNA
   <213> Artificial Sequence
<220>
   <223> G to A (bis2 rc)
<400> 24
   acatcctaaa actctcacta accgaacaat aacgccccct accgaaaccc tcgccaacgc 60
   cacgcgcgcc acgcccaaaa ccaaaaacca aac 93
<210> 25
   <211> 93
   <212> DNA
   <213> Artificial Sequence
<220>
   <223> G to A (bis2 rc) rc
<400> 25
   gtttggtttt tggttttggg cgtggcgcgc gtggcgttgg cgagggtttc ggtagggggc 60
   gttattgttc ggttagtgag agttttagga tgt 93
<210> 26
   <211> 15764
   <212> DNA
   <213> Homo sapiens
<400> 26
   cgcactggga tcggggcctg caggccgctg ggctggctcc tgggatcatc ccaaggtttt 60
   ggtctcgcaa ccgaggggac ccgcgcccgg caatttggga agtgcggaaa tggggtgagg 120
   ccggtcttcg cttgagggtc tcgtagattg gacccctttt tcccccttca agcaccctca 180
   gacctggcca gtgggtcatc gagtcgtgcc caccgcccag gccaggagat ctggggctac 240
   tgggagatct ctcatgaagg tggctataaa aagagccctg accgggacac aagcctttgg 300
   agagcggaag actctatcca aataaacact gggaactggg tggctggtgt tcaggcccgg 360
   aggtctgggg gtttctggac ctaggaagga gagcagactc caggagaggt gtgaaattcc 420
   cactcccggc caccttgctc tctccaaccc ccgcctccgc gcgtcctcct attgctgttc 480
   ccgtccgcat cttctgctgt tatgtgaagt ttctaaagag gcctctgcag cgtgcaggct 540
   ggtgctcttc cagcatttcc tcaatcacgc tgacaccccc aggccccagc actgagttct 600
   cgccgtccac atttagaaag gcctcgcagg agttcgagaa tcctctacac gacttaactt 660
   tgcgcctaaa tacagagcga aaacaggggt cagtctcatt ttgaattggg aagaggcttc 720
   cctcctcccg ggcccaggcg gaaggcaggg aggggtgcgt cttagatggc cgcaaacagt 780
   gcggtgcgga tgcgtctctc ctgtcggtga ctggtttagt ctaagccatt tcatgtgaaa 840
   cccacgaagc agaggaagca tgctaagaac acagaattta aaccgcaata atgaggagat 900
   ttcctagaat aaaacgccga ttacagaaca aaatcggaga aacccaagct tgttgaagtt 960
   tacattccct tgcgctgggg caggggtggg acctaaaggg ttggagggga aaggaacccg 1020
   cagcccagac gggacagtca accaaagcgg gatggagaga ggacccatcg cggcggaggc 1080
   tgcatagttt tggttaacca aggactgcct gggctgatct agactcattt atatgaacca 1140
   taccttccct tcccgtaaga aaaaaaatgt caagattcat gttatttata gggagtgaaa 1200
   gtgagtgaag agctcacccc cataagaagg aaccccagta ggggggtggg ggagccatcc 1260
   taagcaaggc tctgtttcag agacaaacac agctttcctt ttaaaccctg tctgctaacc 1320
   agaaattaag gtattcctcc cagggataca gaaattgaca taatttgcta tcacatacat 1380
   tcttactcaa gagatttgta tatatcagtg attaaaacaa agctcagaga ggctttgctg 1440
   taatgcagtg gaaggaacac tgactttgga gttccaggcc caggctgctg gccagctttg 1500
   tcacttgccc cagcgtgact tgtagccagt cacccagtct ctgagcctta gtttcctcac 1560
   ctcaaaagga ggatgatgat acctgggcca cttggtgggt gacaagatgg tacctgcgag 1620
   gtctgtaaca aacagatgca gggtttagtc actgtagacg tggtggtggt ctatgccatg 1680
   agaatagttt ccagaagcag aagtggggta tccaagaaag agttttggag aggagccttc 1740
   ctgctgatat aacttaagaa ggcccccaag ctgcttatct ttctggagtt cagggtctac 1800
   aggggcagta tgcagacccc tacagagagg gaaggcttcc agtcagctga ccgcctcctg 1860
   gccctaactg ctctccaatg tccactgcct tcccacgacc tggccggccc tcataacact 1920
   caccacactc taagtgggat ttcttgcaag tttagagttt aggtaaaatg gatactgctg 1980
   ttgcttggat ggagaaagga gccttttaaa aaaatccctc ataatttaac cagctgaatt 2040
   ggtagaagct aaaagaatct ggggtttttt gcttgtttcc ttgtttgttt taatgagtac 2100
   ctgtggactt cctaaaatct caggcaagtt ggaagtattt ttctggaaac tggctccata 2160
   gcttccatcg tattctcaga ggatgacccc ataaccccaa aactgggagc tactagtata 2220
   gaaggttatc actggtcctg cctggatttt aaggttagcg ccttttattc ttcagtctcc 2280
   gggtcctaaa accttttaag gcctgctctt aacaagttgg atctgtattc cctgagatcc 2340
   aaatacatat aattttcagg cacagagaat cagagagagt ccatggctaa gtgacaatga 2400
   gtactgaatg tttccaaagc ccgttgccca ccgcccccac ctacaaatga gataattgtc 2460
   cttttccccc acaagcttct cccctgggct ctttccattt aaagcacaca cacacacaca 2520
   cacacacaca cacacacact gaactctatg tcagtagagc tcctttaaga tcctatctgt 2580
   cctccccact ccagcttcta ttttatgaaa ataattatat tttaggcact tagagcattg 2640
   gtttagtgaa cacattatat ttactttcat atttagttat gtatttttaa gttattccac 2700
   acaaaatgac aaatgagggc aagagaaaaa aataccatga aatatatggg gacgcacccc 2760
   ccaaaaacgc tcagcagtca gtcagaaatt cttagaaagt ggaaacaact gcgaagccct 2820
   accccaaccc aatcaaaaca tcccacccgg aaaagagcga cttgtttctt ttttctattg 2880
   gaaatgagtg gtaaactgag ttcggtttgg cggtgttttc ttagctgact ctcggctaca 2940
   atcaagccat tttcggacac attcccatgt aggtaccagg ggtgctctgg aggaagctgt 3000
   cgacgcctgt tcagctaatt taggttttct ttgtctaatt actgtggagc ttagggagaa 3060
   ggagccggct cagggcagta aactgacact cggtctctgt gtgtggtatc caggcagggt 3120
   ttgatggaaa aagcctgctt ccactcaacc tcaggtcact gtctggggcc agccccggcc 3180
   cacccccctc ccacataccc ctcccctccc cacccgccct ctctgcatct acagccccac 3240
   cagcacactc agccaggagc agtcccccgg cactccaggg gcacacaggg ctcctgccgc 3300
   cccagggggc ctgcctaggc tgtgcggctg tgaaggctct ttgcctggtg tcttcctcct 3360
   gattactgca caacctccct ttctcctgcc tcctgcaact ccccaggctc cccagcaggc 3420
   acacccgtgc acctcggtcc tttcagccta gacgcagcgg ggtgcccagc tctacctcct 3480
   gtttggtccg ctggtctgca gcaccttctt tctgggcctc ccccttcagc ccttgggagg 3540
   ccccgccagc tccggcctcc tgctctcctc ctccctctcc cagctcctat ccctggtcgg 3600
   actacatttc cctgcacgtg taatccaata actaactgtc gggtctcatt ggaagtgatt 3660
   gctcccgagg gcacatctct ctcagagcct gactcttact tagggaggaa ttagccacaa 3720
   ctggggagaa gtgttctcgg acaggagtta gctgagacct ggcacagttt tgggtgggca 3780
   atgttaagac ggaggatgta aacctagtga cagctgcaca ccccttcagg actgactata 3840
   aatatagcca tctcgcttat ctcagggtct ttctttttct ttctctttct ttcttttctt 3900
   ccttccttcc tttttctttt gcactttttt cttacagaac actaactggg gtgcgggggt 3960
   ggggggaaga agtggaagca aatgcaaccc taagtcccat gagatccagg tttccagagt 4020
   ctttgaaact tggcctaggt ttctcagtgt aaatccctct gctcaaagtt ccgaaaacgt 4080
   ccacgatcgc cagcggcccg caaccctgcg gcggccccca gtcgccctaa caattgtcgg 4140
   ggagcctgca ggccctgcca gaaagagaga ggattggcac gttacctttg ggcgtctggc 4200
   accccggcgt gtggctgagg gcggcaaagt ctaaacgaag ttgacagatg gagtcagtat 4260
   cggccctggg gagggcggcc agggaggctc cgagcagccg cacccgcagc agcaccactc 4320
   ggcccggcgg gccaggagga ggaagggggc gcggaggggc ggggctgggt cctgggatgg 4380
   gacgcggggc ttgctgcggg gggcaaacag aaagaatatt tcatgaagtc agaggtggtc 4440
   gagttttgcg aggggtgggg gtcgtccccc tggcagaacc ggagtgagtc tgtgatccct 4500
   caattggaaa gagggtgagt gagggaaagg ggaggaggcc gcgagaggaa tgaagagcga 4560
   ggagaccctg gagagcgggg agcgagataa gagagggagg cggccgccag gtcgggaggg 4620
   ggaggagtga gcgcggcggc gcggagcgag gagggggcag ggcggggtgg ggggggaggg 4680
   agggagggag ggagcgagga gggcggccgg ggagggcgag ggagggagga agtccgagag 4740
   acagagcgag gagcgctcct gaagggagac gtcgggctcg tccccggggt ccaggccgcg 4800
   gtcccactcg cgggcgcagt cgcccggcgc cggccgctcg gccccgcggc ctgggaggcc 4860
   gttgcgcaag gcagggcccg cggctcgggg agggatgcgc cgggcgttgc ctcccgcccg 4920
   ccgccgccgc cgccgccaga agccccagag gagctgaggg aggcgacgcc gaagcgctgg 4980
   cccggagtgg ccacggctgc agcccgggcg gcgggctagg gcgctcgcag ggcttctggg 5040
   ccgaccccgc tccggcgcct ccgcttcccg cccggggccc gccctcgcgg cccggcctcg 5100
   ctcccgggtc ccagatgacc accgagggcg ggccgccgcc ggccccgctc cgccgcgcgt 5160
   gcagcccggt ccccggcgcg ctccaggccg ccctgatgag cccgccgccc gccgccgccg 5220
   ccgccgccgc cgccgccccg gagaccacct cctcctcctc gtcgtcgtcc tccgcctcct 5280
   gcgcctcgtc ctcgtcctcc tccaattcgg ccagcgcccc ctcggctgcc tgcaagagcg 5340
   cgggcggcgg cggcgcgggc gccgggagcg ggggcgccaa gaaggcgagc tcggggctgc 5400
   ggcggcccga gaagccgccc tactcgtaca tcgcgctcat cgtcatggcc atccagagct 5460
   cgcccagcaa gcgcctgacg ctcagcgaga tctaccagtt cctgcaggcg cgcttcccct 5520
   tcttccgcgg cgcctaccag ggctggaaga actcggtgcg ccacaatctc tcgctcaacg 5580
   agtgcttcat caagctgcct aagggcctcg ggcggcccgg caagggccac tactggacca 5640
   tcgacccggc cagcgagttc atgttcgagg agggctcgtt ccgccgccgg ccgcgcggct 5700
   tcaggcggaa gtgccaggcg ctcaagccca tgtaccaccg cgtggtgagc ggcttgggct 5760
   tcggggcgtc gctgctgccc cagggcttcg acttccaggc gcccccgtcg gcgccgctcg 5820
   gctgccacag ccagggcggc tacggcggcc tcgacatgat gcccgcgggc tacgacgccg 5880
   gcgcgggcgc ccccagccac gcgcaccctc accaccacca ccaccaccac gtcccgcaca 5940
   tgtcgcccaa cccgggttcc acctacatgg ccagctgccc ggtgcccgcg ggacccgggg 6000
   gcgtcggtgc ggccgggggc ggcggcggcg gcgactacgg gccggacagc agcagcagcc 6060
   cggtaccctc gtccccggcc atggcgagcg ccatcgaatg ccactcgccc tacacgagcc 6120
   ctgcggcgca ctggagctcg cctggcgcct cgccttacct caagcagccg cctgccctga 6180
   cgcccagcag caaccccgcc gcctcggcag gcctgcactc cagcatgtcc tcctactcgc 6240
   tggagcagag ctacttgcac cagaacgctc gcgaggacct ctcaggtaac gcagcacgct 6300
   ccagcccagc cagctgggcg caccgcttct aggcctccag ggctggcggc cactgccact 6360
   cccacaaaca gggaccccga agctaggagg tctgagggca ctgggaaaag cagatgctgg 6420
   ggaaagccgg catcttattc gtgggagcag ggatcagggt cttactgtcc tcccagtatc 6480
   cctggccagc agccgaagct tcagaattgt ctggctgctg tgagcgtggt gtaaagtccc 6540
   ctgccttcct gggctctttg gggatccctg tgcaggagac tgcaggtctc ccgttcagag 6600
   tctcacttag cttggagaga aggcatctgg cctttgggga acttggtcag gaagctgtgt 6660
   ctagttcctt ttgtcccttt ttttccttgc gttcattttg ctgtacttct gtcccgaagt 6720
   aaatgtcaga atcagatatg ttctgtatgt tggggtgtct gcagcgagat gtttgcagct 6780
   gggcaagtgt taagaggagg gggtgggagg gacagagctg gatcccagga tctttcacta 6840
   aagggacatt tgccagtgga gggggtcacc aaactgttgg agactcctcc ccttccctgc 6900
   ctcccccccc gcccaacaat ggacccacat tgatgataca ggtcctgggt ggcaggacct 6960
   gaaggaccct gctaggactc ccctgagaaa tgcgcctgcc ctgtggatgc cacttgtagt 7020
   gtggctggtt tacattgttc tggttgcaga ttatgaaaaa ttattgactt gctgaaggcg 7080
   gtgaaaggtg ggaggggacg gtagggatgg aagagggaat ttgaatattt gaccaggtta 7140
   atgtgaagaa cagagataaa cccttacatt aacccctcgc tcggagcacc tagggctgca 7200
   gattaaaatc ccatgggcct gccagtcaaa ggcaaggccc caagctactc tctcacactg 7260
   gcctcccaca gcaatgagac agtggcttgg agggtgatgc atgctttatg tgttcctgag 7320
   ccggtgtgca gcgggagcac ccggggagaa ggaaagccca gaaatgggca aaagatgttg 7380
   gttccctgga cggtgaaatt tcccaaatag gccttttaaa tgtgggtctc agaggttaac 7440
   caaacaaaga cagtctcggc aggaccacac cggagaggat tttagcctga cttaacccac 7500
   tgagaacaga gtcttgttca attttatggg aatttcaaca agtaaccaat acaggcggcc 7560
   ttcccctctt atttttgtat tcccttggac cggctgtcaa tcacacacac acacacacac 7620
   acacacacac acacacacac acacccctcg gtgcactggc cccttccctt ccccgcctgc 7680
   ctgctcctct cccagcctgc cagctgccct ccctgctccc cttcttctcc tctcccggca 7740
   gatccactca acttgaaccc ggactctgag gcttagcagc cttggagaaa ccgtactcta 7800
   ggaagaaaaa aaagcacaca ggaaagagac tcctgaccct tcctcaaggc tgctctcgcc 7860
   ccacctcata acactgggcg caggcacaag ctcccaaaac ttcggaatct ggatacatta 7920
   ctgtcttgga gggggtgaaa actgcccagc aaagcaagcc ggcctctgtg cccctaccca 7980
   gcggcctccc gcggccggcc ttccctggga gcctcgcaga acccccgacc gagtctggga 8040
   acgcccgggg gagacaatag ctcgggcctg acgcccgaca gggaccctct gcccttcctt 8100
   cctcgctgtc ttgctcccgt ccctatagga tacccacccg gccctgttgc catctccctg 8160
   gagcgccagc ggtggaggct tcggaggagc cggccggatg caccgggacc ccgcggggcc 8220
   tagttgggag cgcgccgccc actgaggcct gggggcccgg cgaggcgcgg gggtggccaa 8280
   ggcagtctcc ccggcacaca gatcagcaca tgtctacgcg cacacaccca gcacgaccct 8340
   ccctccccca gcacacccca agggccgcgc cctccgctcc tgggcctggt ggccatggtc 8400
   catgtgcccc tgccggcgca ggggctgccc tggagttctc cattaagccc tgcgagcact 8460
   tcaggaagga gctcgggctt ctgtcatccc gctcagcaca tcaaagcgcc gacggccgcc 8520
   ttcgtgggca ggtgtgagcg ctcgccctct ctcctcccga gcctgctatt ttgtggtcca 8580
   ggacttgcca tcagaacttg ctttaacgcg acgagggaac tcacctgtgt gcagagcgcc 8640
   gcgcacacat cggggctcgc gggacagggc agctgcgtta gcgcagcgcc gggtgtgttc 8700
   gggtaggtgt tgcgggcaag gaagtaggca gcggcccctg agcagccgcc tcgctccggc 8760
   attgcgggga cacggcgggg ctgaggccac gagagcaggg cccgagcccg gcgggccgtg 8820
   gttacggttt tcttgcactg aaaaactgaa tccggcccga agcgacgtgc actttatggt 8880
   ccccacacca ctcggttaac taagaaaaga cccgggcgaa tggacctaac gcagcccggt 8940
   gccgagaggg cccggtccag cagcctctgg ggcccagtgc gcagggcact gcgggccgat 9000
   tgcgccccgg ggccaggagg cgccgagaaa gcaaaagcaa aagccggcgg cgggtggagg 9060
   tcaagcggcc cggtgcaggc cggggaccgg ggagccgccc cgggagagca gggtccaggc 9120
   cttctctgtc cccctccagc ctggggctac ctcagggact cctcgcgctg ccagatgctt 9180
   cggaccaaag ggcatttctc tgcgccctga agtcccggct tctctagagg aagtctaaga 9240
   cgttcgggac ccacgaagat gcctgcggag gggcaggaga agaggcgtgc ttttctgccc 9300
   catcccttca tcttgcgatt gggttgtttt ctatcgtccc ctttggcaca gggagtcctc 9360
   tgcgctcgct gctgtgaccg gcccagttag ttgggaaaca aggctcagaa agaaggccgc 9420
   agtggtgttt ctggggtagg gtgcttggcc ctccctgaag ccagggctca gggcagcaca 9480
   ggccaggagc tctgccggga gagacccgga accagagtct gggtctagtt gtccggtacc 9540
   tgtgctcatt ctgagattaa agtgcacgcc ccagcctcag agaataaaat tggaattcca 9600
   ggacataaga atccccctcg agggggaact tgctctgtgt ttgtgcgagc atcaaaacaa 9660
   aacataatga ttccaaagca attctatgac gtgcagttgg tgtgtttgag ctacttctaa 9720
   agctgggctt tctctaccag ctgtctagca atttactaaa aggcacagca gcaccttttg 9780
   tactctgagg caaaataaga caccctgcca tccactggat gactttgttt ctgaagaggt 9840
   ttttttttct ttcttctttc agtgggactg ccccgttacc agcatcactc tactccagtg 9900
   tgtgacagaa aagatttcgt cctcaacttc aatgggattt cttctttcca tccctcagct 9960
   agcgggtcgt attatcacca tcaccaccag agcgtctgtc aggatattaa gccctgcgtc 10020
   atgtgaacgg aaagaggcca agcgatggcc gctctctcct ctcccctcct cagagggggc 10080
   agatagaaac tgggacggat tcaagtcaca tgcacgcgga tagcagtaag ccacacacct 10140
   gccacttagc cagaatgccc aggatcgcgt tggtcactgt tatttgccta ctgctggaag 10200
   aaggacaacc gctggcaagg tagcgttccc caatctgaat acctgcaggc tcccacatga 10260
   gggagagggc agactcaggt gggaagatgt gccatgcgta aggcatcaac gtgtatctgt 10320
   gggatcttcg ttgccttcag taatcagggt gtgaaaaaag cagacaagtt gtgtgtgtgt 10380
   gtgtgtgtct aagaaaactt gtgtgctttt caaaaaggca gtgctaagca caagatttca 10440
   agaaagcctc ttcttgttgc ctagctgagt gggagagtca ttttccccag acactacatt 10500
   tggatacagg tgccaaagaa cattattaag gaattattta gaaacaatgt gtctagttta 10560
   agaaagtggt tttcagtatt gtgacaatac aacgttttta caaggttgtt ttctaccacc 10620
   atattttaaa gatattttta tgaccgtgta tactcacact ttgcttgtat tttaaaagga 10680
   ggatatattt gcacttatgt atacttttac agtttgccaa aatattttgt tgtaaaattt 10740
   tttttcaata aaatgtatat aacaaatagt tgttttaggg gctgattttc tcttcgggca 10800
   ccctgagagc aatggatgcc agtttctcct ccccggcagg cagctgtgca actctaccca 10860
   caccccctcc agtggtgtaa ctgacatggt gtggggtctg tgccttgagt aacacaggcc 10920
   aggttctgat tgtgagtctt ctcaggagaa aaacagtgga agaaacgttt tctgttattt 10980
   ctttctttac ttcaccccag ctttttctgg caaaacaaaa accagaaggt gaaaggaact 11040
   agaaagaaaa ccaggcaaga taccgaccag ggtttgattt tgaggaaacc aattttgtgc 11100
   cccttctgtt ttctttcctg tgcccgggga gtgttctgcc ctttgccctc tctccctccc 11160
   gactggcatg ggttgacaga tgtggggcac agtggtaagc ccccctccag ggttggatgc 11220
   ccaaagaaca gcaccctgct tcttcccttt gcacgttgca attttgttat gggtggcggt 11280
   cacatgctgt cagaaatggg gttcagatct gaagaatgca gggggatccc tgagctgcag 11340
   ggggcaggct cccagcaggg ggtttaactt ccttggggag tttaatacag aagctggact 11400
   ccccacacgt tgctctgtgg ctgcggcctg tcccaccctc caattcagag cctggctggc 11460
   cctgctcagc atcggcacat agcaagccgg ttcagccctt ccttcctccc ggcctcctct 11520
   ccccactcct cccttttaac cagggattta acaagggaac ccctcagccc ctttttctgt 11580
   tcctgctaga tttcccagtc accagtgtta caggcagagc tggtgacgct cccgatggaa 11640
   gagcaaaatg ggccaactag gcaggaggac tgaccttctg tcccagcagc ccttcaccat 11700
   cacgagccat gttggggaca atgggtctga gagaagcaga gaaattatct gctccaatca 11760
   tgctgtgcat tttcctctcc aattcagggc tggagttttg tttttgtttt ttgctttttc 11820
   tttcttgatt ggaaaactca cctttgtttc ttatccctta aaaggtcttt ctcccagtca 11880
   gtcaccagac tcttggtatt cgtagggaac tgtttcacac cccagggttt ttgtattcta 11940
   agtgaatttc actgcaaaga gcgtctcctg agtgaaacat cttgtttccg aaagtcttaa 12000
   ccatctttct aatctttagg tttccttcct atatcttcta aaattaagaa gtgatttaaa 12060
   cattttttgt ctctccatta tcatatttgc agttaaataa ggccttttta ttttctttta 12120
   tagtgagcta ctcgggggcc tgctataaat tatgaagtca gtttcagagt ttgctccgac 12180
   cacagtgctg tgtttacatt ccttccgagg cagccggcat ggtcgtaatt tatatcctaa 12240
   acacttgaac gtaacttgtt tatacagaga atgacaggac ctcaaagacg aaaactgcgt 12300
   ctgcctgcct ggccgcattg ggaggcccag cgcgccccct ggtggcgcga ggagatccgc 12360
   ccaaggcgac tcaatgattc cttcaagctg cgcaagaact tgacaaaatg acccgttttg 12420
   tggtgccttc caatagagac atttttaagt atctcacttt cataagttat ggtttccagt 12480
   tttaggaaat actttgcttt cttctttctt cttcttcctt tttttttttt tttttgagac 12540
   agggtcgtgg tctgttgccc aggctggagt gcaatggcac gatctcagct cactgcagcc 12600
   tccaccgcct ggactcaagt gatcctcccg cctcagcctc ctgagtagct gggaccacag 12660
   gcacgcacca ccatgcctgg ctaatttttt aatttttgta gagacggggt cttgcctcgt 12720
   tgcccaggcg tgggcttttt tctattttac tcttttcgcc tactgtctca tcctgaaacc 12780
   agatcacaat tcagtaagaa caggcctgac catgctcagc agatgctaaa gccctgactt 12840
   gctacgtata ttcttcattt ctacatttct ccccaaataa ggctgttgtt ttgtttcctt 12900
   ttgagtcaaa attgtttctc tcctaactgt ccctcaaaga cacacacgta cacatagact 12960
   catttatatt cacccttttt tttttttttt tggagaaaca tgcagtgctt ggctttaaaa 13020
   gttgctcaaa tgttagtgat taaattgaaa aaaagtgcta cacctttata agtgaaaata 13080
   aatgaactaa ctcaactagg gataaatacc tcaactgttt tttcgtcaaa tggattttga 13140
   acttaggttt tctgctcatt ggcggaataa agtggtatat taaaagattt tattgttttc 13200
   tataataatt attgtttttt gttggtttca ttggagctgt atacataaag acaagaccag 13260
   ggaactacta agcatcatgc atcaagtgct gggcaccgtg gctgtcttac caaatctcac 13320
   acggctatct tacttaattc tgtcaatatc cctctgaggt gctgatattg accttattta 13380
   cagatctgta ctagctcctg actaggtcat gactttgggg ggtttgaccc atgtcttctg 13440
   atttcagttt atttccctcg tttctccaac actgcctcta agaattttgc aactgacact 13500
   gcagatcttt gagtggcagg aatattccct ctgaagcagc agttctcaca cttgagtgca 13560
   catcagaatc acctggaggg cttgttcaac acaggctgtg ggtccacccc accctccatt 13620
   tctgattcag tgggtccaag gttgtgttca aagatgtgta ttcctaataa actaataaaa 13680
   gaaggtgatg ctgataatgt tggtcctggg accacactct gaaaactgca gctcttgaaa 13740
   ggcagtttcg attacagatc tttttatagg ttccaatgga taggtattaa ggatagccta 13800
   ttaattggtt aagcaaagat ctggaaaggt ttttcaaata gtttgtccat ctaaatgcct 13860
   ctgtgattga ttatgggccc ctaggaaaat gcaaggctta attttgaacc ctaggccaga 13920
   aagtaggatc tccccatttt tattcaaagc aatgaggcct cttgttttat tgtagtaaac 13980
   attgcagaga ggagtcagat ggaatcattg aagtctacct gcctcagtgg aacaataagt 14040
   ctaggtactt gcacacagca gccagtcaga attctacaaa cagaaggagg cagtaatggg 14100
   agcagacagg aaaagaaact ccattattgg ttataaaacc ttttgagggt agaacagaga 14160
   aaaggcaaag aaggggggct gtgatctcag aggatagcaa tagtgatccc tagatatgaa 14220
   tgggtaggca gaaccgtggg tgaagaaggg agagagacag agagaggaaa agggagagaa 14280
   aggaagagaa agagagagat ggagacagag aagaaggagg ggaaagagga agatagtcaa 14340
   aaagagggaa agaggaagag aaaggaagag agagggagag aagagaggaa gagagacaca 14400
   tagagaagga aagggaaaag aaacggagaa gaaagagagg gagggaggga gagagggaga 14460
   taggaaggga gtgagatggg gtaagagaga gacaaagaaa gaagagagac tgggagagaa 14520
   agagagatac acagagaaag agagaggaag ggcaaagatg ggtggagggt gtgggaagaa 14580
   aggaggagga ggaaggaggg agagagacag agggagagaa agggtggagg aggaggagga 14640
   gagggaagca gggggaggga ggtaaggagg gaaagaggtc tggagaggag gagtctgcag 14700
   acatttcctg agctcctgtc cctggagctg agaggcagcc ctggaagctg gattcctgtc 14760
   ctctgaggcc accattctct ctccaggtcc ccagtgccaa cccacgagct gaaagaccca 14820
   tggacttctg ttttacttgg cttggcctga attctgggga agtaagtgaa ttctctagtt 14880
   gtgcatgatc aaattttctg aactgccaca gcgacttata cagaattagg gagaacaatc 14940
   ttgacttaga ttttagaaag tggaagtccg atttctgggg aaaatcttca ggtccctagg 15000
   ataggccacg gtatggcatt aaagggaatt agcagctcct tcccaaaaga aacactctct 15060
   ttgtagattc gtattatacc taattactct gcatcatggt gattaattca aaacaggcac 15120
   tgacttactt agaatggaaa tatattttaa tatattttat ccgaacggaa atacatttta 15180
   ccaaacctgc ctctatgaaa cactatctta gcaagaaaaa acaccacaaa tgtagacatg 15240
   gccagggagg gccagggcac acagacgtca aggtcattgg ggaaaaaatg aaagtcctgg 15300
   aagttctttt tgagttcaga gtgccccaag tccacagcaa tccaactgat aaatattcat 15360
   ctctccatgt cctgggggac gtgtgtgaga tacggagcct tggccatccc acggctgcat 15420
   cgccaggcct gtgggcttaa ctgtattcac gagagcacag acggagcgga cagcgggaca 15480
   gctttccctc cgaagggccc attccaccct ccagcctggc aacggaggct cggtcacagc 15540
   cgggcagtga ctcgtctgcc gggtggcacg gcctcacctc cccaaactgg cagagcacct 15600
   ccccctcaga tttggaaacc gctgtgagtt aggtggagtc caagcctgga gtgtggggag 15660
   aggaagacct gcctcctcgc tcctccttca gcagctccac gcagcacaga gcggaagtgt 15720
   cggtagaccg tgttgattga ctgactgggc cccagcgcgc cgtg 15764
<210> 27
   <211> 15764
   <212> DNA
   <213> Artificial Sequence
<220>
   <223> C to T (bisl)
<400> 27
   cgtattggga tcggggtttg taggtcgttg ggttggtttt tgggattatt ttaaggtttt      60
   ggtttcgtaa tcgaggggat tcgcgttcgg taatttggga agtgcggaaa tggggtgagg     120
   tcggttttcg tttgagggtt tcgtagattg gatttttttt ttttttttta agtattttta     180
   gatttggtta gtgggttatc gagtcgtgtt tatcgtttag gttaggagat ttggggttat     240
   tgggagattt tttatgaagg tggttataaa aagagttttg atcgggatat aagtttttgg     300
   agagcggaag attttattta aataaatatt gggaattggg tggttggtgt ttaggttcgg     360
   aggtttgggg gtttttggat ttaggaagga gagtagattt taggagaggt gtgaaatttt     420
   tattttcggt tattttgttt tttttaattt tcgttttcgc gcgttttttt attgttgttt 480
   tcgttcgtat tttttgttgt tatgtgaagt ttttaaagag gtttttgtag cgtgtaggtt 540
   ggtgtttttt tagtattttt ttaattacgt tgatattttt aggttttagt attgagtttt 600
   cgtcgtttat atttagaaag gtttcgtagg agttcgagaa ttttttatac gatttaattt 660
   tgcgtttaaa tatagagcga aaataggggt tagttttatt ttgaattggg aagaggtttt 720
   ttttttttcg ggtttaggcg gaaggtaggg aggggtgcgt tttagatggt cgtaaatagt 780
   gcggtgcgga tgcgtttttt ttgtcggtga ttggtttagt ttaagttatt ttatgtgaaa 840
   tttacgaagt agaggaagta tgttaagaat atagaattta aatcgtaata atgaggagat 900
   tttttagaat aaaacgtcga ttatagaata aaatcggaga aatttaagtt tgttgaagtt 960
   tatatttttt tgcgttgggg taggggtggg atttaaaggg ttggagggga aaggaattcg 1020
   tagtttagac gggatagtta attaaagcgg gatggagaga ggatttatcg cggcggaggt 1080
   tgtatagttt tggttaatta aggattgttt gggttgattt agatttattt atatgaatta 1140
   tatttttttt tttcgtaaga aaaaaaatgt taagatttat gttatttata gggagtgaaa 1200
   gtgagtgaag agtttatttt tataagaagg aattttagta ggggggtggg ggagttattt 1260
   taagtaaggt tttgttttag agataaatat agtttttttt ttaaattttg tttgttaatt 1320
   agaaattaag gtattttttt tagggatata gaaattgata taatttgtta ttatatatat 1380
   ttttatttaa gagatttgta tatattagtg attaaaataa agtttagaga ggttttgttg 1440
   taatgtagtg gaaggaatat tgattttgga gttttaggtt taggttgttg gttagttttg 1500
   ttatttgttt tagcgtgatt tgtagttagt tatttagttt ttgagtttta gtttttttat 1560
   tttaaaagga ggatgatgat atttgggtta tttggtgggt gataagatgg tatttgcgag 1620
   gtttgtaata aatagatgta gggtttagtt attgtagacg tggtggtggt ttatgttatg 1680
   agaatagttt ttagaagtag aagtggggta tttaagaaag agttttggag aggagttttt 1740
   ttgttgatat aatttaagaa ggtttttaag ttgtttattt ttttggagtt tagggtttat 1800
   aggggtagta tgtagatttt tatagagagg gaaggttttt agttagttga tcgttttttg 1860
   gttttaattg ttttttaatg tttattgttt ttttacgatt tggtcggttt ttataatatt 1920
   tattatattt taagtgggat tttttgtaag tttagagttt aggtaaaatg gatattgttg 1980
   ttgtttggat ggagaaagga gttttttaaa aaaatttttt ataatttaat tagttgaatt 2040
   ggtagaagtt aaaagaattt ggggtttttt gtttgttttt ttgtttgttt taatgagtat 2100
   ttgtggattt tttaaaattt taggtaagtt ggaagtattt ttttggaaat tggttttata 2160
   gtttttatcg tatttttaga ggatgatttt ataattttaa aattgggagt tattagtata 2220
   gaaggttatt attggttttg tttggatttt aaggttagcg ttttttattt tttagttttc 2280
   gggttttaaa attttttaag gtttgttttt aataagttgg atttgtattt tttgagattt 2340
   aaatatatat aatttttagg tatagagaat tagagagagt ttatggttaa gtgataatga 2400
   gtattgaatg tttttaaagt tcgttgttta tcgtttttat ttataaatga gataattgtt 2460
   tttttttttt ataagttttt tttttgggtt ttttttattt aaagtatata tatatatata 2520
   tatatatata tatatatatt gaattttatg ttagtagagt ttttttaaga ttttatttgt 2580
   tttttttatt ttagttttta ttttatgaaa ataattatat tttaggtatt tagagtattg 2640
   gtttagtgaa tatattatat ttatttttat atttagttat gtatttttaa gttattttat 2700
   ataaaatgat aaatgagggt aagagaaaaa aatattatga aatatatggg gacgtatttt 2760
   ttaaaaacgt ttagtagtta gttagaaatt tttagaaagt ggaaataatt gcgaagtttt 2820
   attttaattt aattaaaata ttttattcgg aaaagagcga tttgtttttt ttttttattg 2880
   gaaatgagtg gtaaattgag ttcggtttgg cggtgttttt ttagttgatt ttcggttata 2940
   attaagttat tttcggatat atttttatgt aggtattagg ggtgttttgg aggaagttgt 3000
   cgacgtttgt ttagttaatt taggtttttt ttgtttaatt attgtggagt ttagggagaa 3060
   ggagtcggtt tagggtagta aattgatatt cggtttttgt gtgtggtatt taggtagggt 3120
   ttgatggaaa aagtttgttt ttatttaatt ttaggttatt gtttggggtt agtttcggtt 3180
   tatttttttt ttatatattt tttttttttt tattcgtttt ttttgtattt atagttttat 3240
   tagtatattt agttaggagt agtttttcgg tattttaggg gtatataggg tttttgtcgt 3300
   tttagggggt ttgtttaggt tgtgcggttg tgaaggtttt ttgtttggtg tttttttttt 3360
   gattattgta taattttttt tttttttgtt ttttgtaatt ttttaggttt tttagtaggt 3420
   atattcgtgt atttcggttt ttttagttta gacgtagcgg ggtgtttagt tttatttttt 3480
   gtttggttcg ttggtttgta gtattttttt tttgggtttt tttttttagt ttttgggagg 3540
   tttcgttagt ttcggttttt tgtttttttt tttttttttt tagtttttat ttttggtcgg 3600
   attatatttt tttgtacgtg taatttaata attaattgtc gggttttatt ggaagtgatt 3660
   gttttcgagg gtatattttt tttagagttt gatttttatt tagggaggaa ttagttataa 3720
   ttggggagaa gtgttttcgg ataggagtta gttgagattt ggtatagttt tgggtgggta 3780
   atgttaagac ggaggatgta aatttagtga tagttgtata tttttttagg attgattata 3840
   aatatagtta tttcgtttat tttagggttt tttttttttt tttttttttt tttttttttt 3900
   tttttttttt tttttttttt gtattttttt tttatagaat attaattggg gtgcgggggt 3960
   ggggggaaga agtggaagta aatgtaattt taagttttat gagatttagg tttttagagt 4020
   ttttgaaatt tggtttaggt tttttagtgt aaattttttt gtttaaagtt tcgaaaacgt 4080
   ttacgatcgt tagcggttcg taattttgcg gcggttttta gtcgttttaa taattgtcgg 4140
   ggagtttgta ggttttgtta gaaagagaga ggattggtac gttatttttg ggcgtttggt 4200
   atttcggcgt gtggttgagg gcggtaaagt ttaaacgaag ttgatagatg gagttagtat 4260
   cggttttggg gagggcggtt agggaggttt cgagtagtcg tattcgtagt agtattattc 4320
   ggttcggcgg gttaggagga ggaagggggc gcggaggggc ggggttgggt tttgggatgg 4380
   gacgcggggt ttgttgcggg gggtaaatag aaagaatatt ttatgaagtt agaggtggtc 4440
   gagttttgcg aggggtgggg gtcgtttttt tggtagaatc ggagtgagtt tgtgattttt 4500
   taattggaaa gagggtgagt gagggaaagg ggaggaggtc gcgagaggaa tgaagagcga 4560
   ggagattttg gagagcgggg agcgagataa gagagggagg cggtcgttag gtcgggaggg 4620
   ggaggagtga gcgcggcggc gcggagcgag gagggggtag ggcggggtgg gggggagggg 4680
   agggagggag ggagcgagga gggcggtcgg ggagggcgag ggagggagga agttcgagag 4740
   atagagcgag gagcgttttt gaagggagac gtcgggttcg ttttcggggt ttaggtcgcg 4800
   gttttattcg cgggcgtagt cgttcggcgt cggtcgttcg gtttcgcggt ttgggaggtc 4860
   gttgcgtaag gtagggttcg cggttcgggg agggatgcgt cgggcgttgt ttttcgttcg 4920
   tcgtcgtcgt cgtcgttaga agttttagag gagttgaggg aggcgacgtc gaagcgttgg 4980
   ttcggagtgg ttacggttgt agttcgggcg gcgggttagg gcgttcgtag ggtttttggg 5040
   tcgatttcgt ttcggcgttt tcgtttttcg ttcggggttc gttttcgcgg ttcggtttcg 5100
   ttttcgggtt ttagatgatt atcgagggcg ggtcgtcgtc ggtttcgttt cgtcgcgcgt 5160
   gtagttcggt tttcggcgcg ttttaggtcg ttttgatgag ttcgtcgttc gtcgtcgtcg 5220
   tcgtcgtcgt cgtcgtttcg gagattattt tttttttttc gtcgtcgttt ttcgtttttt 5280
   gcgtttcgtt ttcgtttttt tttaattcgg ttagcgtttt ttcggttgtt tgtaagagcg 5340
   cgggcggcgg cggcgcgggc gtcgggagcg gggcgttaag gaaggcgagt tcggggttgc 5400
   ggcggttcga gaagtcgttt tattcgtata tcgcgtttat cgttatggtt atttagagtt 5460
   cgtttagtaa gcgtttgacg tttagcgaga tttattagtt tttgtaggcg cgtttttttt 5520
   tttttcgcgg cgtttattag ggttggaaga attcggtgcg ttataatttt tcgtttaacg 5580
   agtgttttat taagttgttt aagggtttcg ggcggttcgg taagggttat tattggatta 5640
   tcgattcggt tagcgagttt atgttcgagg agggttcgtt tcgtcgtcgg tcgcgcggtt 5700
   ttaggcggaa gtgttaggcg tttaagttta tgtattatcg cgtggtgagc ggtttgggtt 5760
   tcggggcgtc gttgttgttt tagggtttcg atttttaggc gttttcgtcg gcgtcgttcg 5820
   gttgttatag ttagggcggt tacggcggtt tcgatatgat gttcgcgggt tacgacgtcg 5880
   gcgcgggcgt ttttagttac gcgtattttt attattatta ttattattac gtttcgtata 5940
   tgtcgtttaa ttcgggtttt atttatatgg ttagttgttc ggtgttcgcg ggattcgggg 6000
   gcgtcggtgc ggtcgggggc ggcggcggcg gcgattacgg gtcggatagt agtagtagtt 6060
   cggtattttc gttttcggtt atggcgagcg ttatcgaatg ttattcgttt tatacgagtt 6120
   ttgcggcgta ttggagttcg tttggcgttt cgttttattt taagtagtcg tttgttttga 6180
   cgtttagtag taatttcgtc gtttcggtag gtttgtattt tagtatgttt ttttattcgt 6240
   tggagtagag ttatttgtat tagaacgttc gcgaggattt tttaggtaac gtagtacgtt 6300
   ttagtttagt tagttgggcg tatcgttttt aggtttttag ggttggcggt tattgttatt 6360
   tttataaata gggatttcga agttaggagg tttgagggta ttgggaaaag tagatgttgg 6420
   ggaaagtcgg tattttattc gtgggagtag ggattagggt tttattgttt ttttagtatt 6480
   tttggttagt agtcgaagtt ttagaattgt ttggttgttg tgagcgtggt gtaaagtttt 6540
   ttgttttttt gggttttttg gggatttttg tgtaggagat tgtaggtttt tcgtttagag 6600
   ttttatttag tttggagaga aggtatttgg tttttgggga atttggttag gaagttgtgt 6660
   ttagtttttt ttgttttttt ttttttttgc gtttattttg ttgtattttt gtttcgaagt 6720
   aaatgttaga attagatatg ttttgtatgt tggggtgttt gtagcgagat gtttgtagtt 6780
   gggtaagtgt taagaggagg gggtgggagg gatagagttg gattttagga ttttttatta 6840
   aagggatatt tgttagtgga gggggttatt aaattgttgg agattttttt ttttttttgt 6900
   tttttttttc gtttaataat ggatttatat tgatgatata ggttttgggt ggtaggattt 6960
   gaaggatttt gttaggattt ttttgagaaa tgcgtttgtt ttgtggatgt tatttgtagt 7020
   gtggttggtt tatattgttt tggttgtaga ttatgaaaaa ttattgattt gttgaaggcg 7080
   gtgaaaggtg ggaggggacg gtagggatgg aagagggaat ttgaatattt gattaggtta 7140
   atgtgaagaa tagagataaa tttttatatt aatttttcgt tcggagtatt tagggttgta 7200
   gattaaaatt ttatgggttt gttagttaaa ggtaaggttt taagttattt ttttatattg 7260
   gttttttata gtaatgagat agtggtttgg agggtgatgt atgttttatg tgtttttgag 7320
   tcggtgtgta gcgggagtat tcggggagaa ggaaagttta gaaatgggta aaagatgttg 7380
   gttttttgga cggtgaaatt ttttaaatag gttttttaaa tgtgggtttt agaggttaat 7440
   taaataaaga tagtttcggt aggattatat cggagaggat tttagtttga tttaatttat 7500
   tgagaataga gttttgttta attttatggg aattttaata agtaattaat ataggcggtt 7560
   tttttttttt atttttgtat ttttttggat cggttgttaa ttatatatat atatatatat 7620
   atatatatat atatatatat atatttttcg gtgtattggt tttttttttt tttcgtttgt 7680
   ttgttttttt tttagtttgt tagttgtttt ttttgttttt tttttttttt tttttcggta 7740
   gatttattta atttgaattc ggattttgag gtttagtagt tttggagaaa tcgtatttta 7800
   ggaagaaaaa aaagtatata ggaaagagat ttttgatttt tttttaaggt tgttttcgtt 7860
   ttattttata atattgggcg taggtataag tttttaaaat ttcggaattt ggatatatta 7920
   ttgttttgga gggggtgaaa attgtttagt aaagtaagtc ggtttttgtg tttttattta 7980
   gcggtttttc gcggtcggtt ttttttggga gtttcgtaga attttcgatc gagtttggga 8040
   acgttcgggg gagataatag ttcgggtttg acgttcgata gggatttttt gttttttttt 8100
   tttcgttgtt ttgttttcgt ttttatagga tatttattcg gttttgttgt tatttttttg 8160
   gagcgttagc ggtggaggtt tcggaggagt cggtcggatg tatcgggatt tcgcggggtt 8220
   tagttgggag cgcgtcgttt attgaggttt gggggttcgg cgaggcgcgg gggtggttaa 8280
   ggtagttttt tcggtatata gattagtata tgtttacgcg tatatattta gtacgatttt 8340
   ttttttttta gtatatttta agggtcgcgt ttttcgtttt tgggtttggt ggttatggtt 8400
   tatgtgtttt tgtcggcgta ggggttgttt tggagttttt tattaagttt tgcgagtatt 8460
   ttaggaagga gttcgggttt ttgttatttc gtttagtata ttaaagcgtc gacggtcgtt 8520
   ttcgtgggta ggtgtgagcg ttcgtttttt tttttttcga gtttgttatt ttgtggttta 8580
   ggatttgtta ttagaatttg ttttaacgcg acgagggaat ttatttgtgt gtagagcgtc 8640
   gcgtatatat cggggttcgc gggatagggt agttgcgtta gcgtagcgtc gggtgtgttc 8700
   gggtaggtgt tgcgggtaag gaagtaggta gcggtttttg agtagtcgtt tcgtttcggt 8760
   attgcgggga tacggcgggg ttgaggttac gagagtaggg ttcgagttcg gcgggtcgtg 8820
   gttacggttt ttttgtattg aaaaattgaa ttcggttcga agcgacgtgt attttatggt 8880
   ttttatatta ttcggttaat taagaaaaga ttcgggcgaa tggatttaac gtagttcggt 8940
   gtcgagaggg ttcggtttag tagtttttgg ggtttagtgc gtagggtatt gcgggtcgat 9000
   tgcgtttcgg ggttaggagg cgtcgagaaa gtaaaagtaa aagtcggcgg cgggtggagg 9060
   ttaagcggtt cggtgtaggt cggggatcgg ggagtcgttt cgggagagta gggtttaggt 9120
   tttttttgtt tttttttagt ttggggttat tttagggatt tttcgcgttg ttagatgttt 9180
   cggattaaag ggtatttttt tgcgttttga agtttcggtt tttttagagg aagtttaaga 9240
   cgttcgggat ttacgaagat gtttgcggag gggtaggaga agaggcgtgt ttttttgttt 9300
   tattttttta ttttgcgatt gggttgtttt ttatcgtttt ttttggtata gggagttttt 9360
   tgcgttcgtt gttgtgatcg gtttagttag ttgggaaata aggtttagaa agaaggtcgt 9420
   agtggtgttt ttggggtagg gtgtttggtt ttttttgaag ttagggttta gggtagtata 9480
   ggttaggagt tttgtcggga gagattcgga attagagttt gggtttagtt gttcggtatt 9540
   tgtgtttatt ttgagattaa agtgtacgtt ttagttttag agaataaaat tggaatttta 9600
   ggatataaga atttttttcg agggggaatt tgttttgtgt ttgtgcgagt attaaaataa 9660
   aatataatga ttttaaagta attttatgac gtgtagttgg tgtgtttgag ttatttttaa 9720
   agttgggttt tttttattag ttgtttagta atttattaaa aggtatagta gtattttttg 9780
   tattttgagg taaaataaga tattttgtta tttattggat gattttgttt ttgaagaggt 9840
   tttttttttt tttttttttt agtgggattg tttcgttatt agtattattt tattttagtg 9900
   tgtgatagaa aagatttcgt ttttaatttt aatgggattt ttttttttta ttttttagtt 9960
   agcgggtcgt attattatta ttattattag agcgtttgtt aggatattaa gttttgcgtt 10020
   atgtgaacgg aaagaggtta agcgatggtc gttttttttt tttttttttt tagagggggt 10080
   agatagaaat tgggacggat ttaagttata tgtacgcgga tagtagtaag ttatatattt 10140
   gttatttagt tagaatgttt aggatcgcgt tggttattgt tatttgttta ttgttggaag 10200
   aaggataatc gttggtaagg tagcgttttt taatttgaat atttgtaggt ttttatatga 10260
   gggagagggt agatttaggt gggaagatgt gttatgcgta aggtattaac gtgtatttgt 10320
   gggattttcg ttgtttttag taattagggt gtgaaaaaag tagataagtt gtgtgtgtgt 10380
   gtgtgtgttt aagaaaattt gtgtgttttt taaaaaggta gtgttaagta taagatttta 10440
   agaaagtttt tttttgttgt ttagttgagt gggagagtta ttttttttag atattatatt 10500
   tggatatagg tgttaaagaa tattattaag gaattattta gaaataatgt gtttagttta 10560
   agaaagtggt ttttagtatt gtgataatat aacgttttta taaggttgtt ttttattatt 10620
   atattttaaa gatattttta tgatcgtgta tatttatatt ttgtttgtat tttaaaagga 10680
   ggatatattt gtatttatgt atatttttat agtttgttaa aatattttgt tgtaaaattt 10740
   ttttttaata aaatgtatat aataaatagt tgttttaggg gttgattttt ttttcgggta 10800
   ttttgagagt aatggatgtt agtttttttt tttcggtagg tagttgtgta attttattta 10860
   tatttttttt agtggtgtaa ttgatatggt gtggggtttg tgttttgagt aatataggtt 10920
   aggttttgat tgtgagtttt tttaggagaa aaatagtgga agaaacgttt tttgttattt 10980
   ttttttttat tttattttag ttttttttgg taaaataaaa attagaaggt gaaaggaatt 11040
   agaaagaaaa ttaggtaaga tatcgattag ggtttgattt tgaggaaatt aattttgtgt 11100
   tttttttgtt tttttttttg tgttcgggga gtgttttgtt ttttgttttt tttttttttc 11160
   gattggtatg ggttgataga tgtggggtat agtggtaagt ttttttttag ggttggatgt 11220
   ttaaagaata gtattttgtt tttttttttt gtacgttgta attttgttat gggtggcggt 11280
   tatatgttgt tagaaatggg gtttagattt gaagaatgta gggggatttt tgagttgtag 11340
   ggggtaggtt tttagtaggg ggtttaattt ttttggggag tttaatatag aagttggatt 11400
   ttttatacgt tgttttgtgg ttgcggtttg ttttattttt taatttagag tttggttggt 11460
   tttgtttagt atcggtatat agtaagtcgg tttagttttt tttttttttc ggtttttttt 11520
   ttttattttt tttttttaat tagggattta ataagggaat tttttagttt ttttttttgt 11580
   ttttgttaga ttttttagtt attagtgtta taggtagagt tggtgacgtt ttcgatggaa 11640
   gagtaaaatg ggttaattag gtaggaggat tgattttttg ttttagtagt tttttattat 11700
   tacgagttat gttggggata atgggtttga gagaagtaga gaaattattt gttttaatta 11760
   tgttgtgtat tttttttttt aatttagggt tggagttttg tttttgtttt ttgttttttt 11820
   ttttttgatt ggaaaattta tttttgtttt ttatttttta aaaggttttt tttttagtta 11880
   gttattagat ttttggtatt cgtagggaat tgttttatat tttagggttt ttgtatttta 11940
   agtgaatttt attgtaaaga gcgttttttg agtgaaatat tttgttttcg aaagttttaa 12000
   ttattttttt aatttttagg tttttttttt atatttttta aaattaagaa gtgatttaaa 12060
   tattttttgt ttttttatta ttatatttgt agttaaataa ggttttttta ttttttttta 12120
   tagtgagtta ttcgggggtt tgttataaat tatgaagtta gttttagagt ttgtttcgat 12180
   tatagtgttg tgtttatatt tttttcgagg tagtcggtat ggtcgtaatt tatattttaa 12240
   atatttgaac gtaatttgtt tatatagaga atgataggat tttaaagacg aaaattgcgt 12300
   ttgtttgttt ggtcgtattg ggaggtttag cgcgtttttt ggtggcgcga ggagattcgt 12360
   ttaaggcgat ttaatgattt ttttaagttg cgtaagaatt tgataaaatg attcgttttg 12420
   tggtgttttt taatagagat atttttaagt attttatttt tataagttat ggtttttagt 12480
   tttaggaaat attttgtttt tttttttttt tttttttttt tttttttttt tttttgagat 12540
   agggtcgtgg tttgttgttt aggttggagt gtaatggtac gattttagtt tattgtagtt 12600
   tttatcgttt ggatttaagt gattttttcg ttttagtttt ttgagtagtt gggattatag 12660
   gtacgtatta ttatgtttgg ttaatttttt aatttttgta gagacggggt tttgtttcgt 12720
   tgtttaggcg tgggtttttt tttattttat ttttttcgtt tattgtttta ttttgaaatt 12780
   agattataat ttagtaagaa taggtttgat tatgtttagt agatgttaaa gttttgattt 12840
   gttacgtata ttttttattt ttatattttt ttttaaataa ggttgttgtt ttgttttttt 12900
   ttgagttaaa attgtttttt ttttaattgt tttttaaaga tatatacgta tatatagatt 12960
   tatttatatt tatttttttt tttttttttt tggagaaata tgtagtgttt ggttttaaaa 13020
   gttgtttaaa tgttagtgat taaattgaaa aaaagtgtta tatttttata agtgaaaata 13080
   aatgaattaa tttaattagg gataaatatt ttaattgttt tttcgttaaa tggattttga 13140
   atttaggttt tttgtttatt ggcggaataa agtggtatat taaaagattt tattgttttt 13200
   tataataatt attgtttttt gttggtttta ttggagttgt atatataaag ataagattag 13260
   ggaattatta agtattatgt attaagtgtt gggtatcgtg gttgttttat taaattttat 13320
   acggttattt tatttaattt tgttaatatt tttttgaggt gttgatattg attttattta 13380
   tagatttgta ttagtttttg attaggttat gattttgggg ggtttgattt atgttttttg 13440
   attttagttt atttttttcg tttttttaat attgttttta agaattttgt aattgatatt 13500
   gtagattttt gagtggtagg aatatttttt ttgaagtagt agtttttata tttgagtgta 13560
   tattagaatt atttggaggg tttgtttaat ataggttgtg ggtttatttt attttttatt 13620
   tttgatttag tgggtttaag gttgtgttta aagatgtgta tttttaataa attaataaaa 13680
   gaaggtgatg ttgataatgt tggttttggg attatatttt gaaaattgta gtttttgaaa 13740
   ggtagtttcg attatagatt tttttatagg ttttaatgga taggtattaa ggatagttta 13800
   ttaattggtt aagtaaagat ttggaaaggt tttttaaata gtttgtttat ttaaatgttt 13860
   ttgtgattga ttatgggttt ttaggaaaat gtaaggttta attttgaatt ttaggttaga 13920
   aagtaggatt tttttatttt tatttaaagt aatgaggttt tttgttttat tgtagtaaat 13980
   attgtagaga ggagttagat ggaattattg aagtttattt gttttagtgg aataataagt 14040
   ttaggtattt gtatatagta gttagttaga attttataaa tagaaggagg tagtaatggg 14100
   agtagatagg aaaagaaatt ttattattgg ttataaaatt ttttgagggt agaatagaga 14160
   aaaggtaaag aaggggggtt gtgattttag aggatagtaa tagtgatttt tagatatgaa 14220
   tgggtaggta gaatcgtggg tgaagaaggg agagagatag agagaggaaa agggagagaa 14280
   aggaagagaa agagagagat ggagatagag aagaaggagg ggaaagagga agatagttaa 14340
   aaagagggaa agaggaagag aaaggaagag agagggagag aagagaggaa gagagatata 14400
   tagagaagga aagggaaaag aaacggagaa gaaagagagg gagggaggga gagagggaga 14460
   taggaaggga gtgagatggg gtaagagaga gataaagaaa gaagagagat tgggagagaa 14520
   agagagatat atagagaaag agagaggaag ggtaaagatg ggtggagggt gtgggaagaa 14580
   aggaggagga ggaaggaggg agagagatag agggagagaa agggtggagg aggaggagga 14640
   gagggaagta gggggaggga ggtaaggagg gaaagaggtt tggagaggag gagtttgtag 14700
   atattttttg agtttttgtt tttggagttg agaggtagtt ttggaagttg gatttttgtt 14760
   ttttgaggtt attatttttt ttttaggttt ttagtgttaa tttacgagtt gaaagattta 14820
   tggatttttg ttttatttgg tttggtttga attttgggga agtaagtgaa ttttttagtt 14880
   gtgtatgatt aaattttttg aattgttata gcgatttata tagaattagg gagaataatt 14940
   ttgatttaga ttttagaaag tggaagttcg atttttgggg aaaattttta ggtttttagg 15000
   ataggttacg gtatggtatt aaagggaatt agtagttttt ttttaaaaga aatatttttt 15060
   ttgtagattc gtattatatt taattatttt gtattatggt gattaattta aaataggtat 15120
   tgatttattt agaatggaaa tatattttaa tatattttat tcgaacggaa atatatttta 15180
   ttaaatttgt ttttatgaaa tattatttta gtaagaaaaa atattataaa tgtagatatg 15240
   gttagggagg gttagggtat atagacgtta aggttattgg ggaaaaaatg aaagttttgg 15300
   aagttttttt tgagtttaga gtgttttaag tttatagtaa tttaattgat aaatatttat 15360
   ttttttatgt tttgggggac gtgtgtgaga tacggagttt tggttatttt acggttgtat 15420
   cgttaggttt gtgggtttaa ttgtatttac gagagtatag acggagcgga tagcgggata 15480
   gttttttttt cgaagggttt attttatttt ttagtttggt aacggaggtt cggttatagt 15540
   cgggtagtga ttcgtttgtc gggtggtacg gttttatttt tttaaattgg tagagtattt    15600
   tttttttaga tttggaaatc gttgtgagtt aggtggagtt taagtttgga gtgtggggag    15660
   aggaagattt gttttttcgt ttttttttta gtagttttac gtagtataga gcggaagtgt    15720
   cggtagatcg tgttgattga ttgattgggt tttagcgcgt cgtg                     15764
<210> 28
   <211> 15764
   <212> DNA
   <213> Artificial Sequence
<220>
   <223> rc C to T (bisl)
<400> 28
<210> 29
   <211> 15764
   <212> DNA
   <213> Artificial Sequence
<220>
   <223> G to A (bis2 rc)
<400> 29
   cgcactaaaa tcgaaaccta caaaccgcta aactaactcc taaaatcatc ccaaaatttt      60
   aatctcgcaa ccgaaaaaac ccgcgcccga caatttaaaa aatacgaaaa taaaataaaa     120
   ccgatcttcg cttaaaaatc tcgtaaatta aacccctttt tcccccttca aacaccctca     180
   aacctaacca ataaatcatc gaatcgtacc caccgcccaa accaaaaaat ctaaaactac     240
   taaaaaatct ctcataaaaa taactataaa aaaaacccta accgaaacac aaacctttaa     300
   aaaacgaaaa actctatcca aataaacact aaaaactaaa taactaatat tcaaacccga     360
   aaatctaaaa atttctaaac ctaaaaaaaa aaacaaactc caaaaaaaat ataaaattcc     420
   cactcccgac caccttactc tctccaaccc ccgcctccgc gcgtcctcct attactattc 480
   ccgtccgcat cttctactat tatataaaat ttctaaaaaa acctctacaa cgtacaaact 540
   aatactcttc caacatttcc tcaatcacgc taacaccccc aaaccccaac actaaattct 600
   cgccgtccac atttaaaaaa acctcgcaaa aattcgaaaa tcctctacac gacttaactt 660
   tacgcctaaa tacaaaacga aaacaaaaat caatctcatt ttaaattaaa aaaaaacttc 720
   cctcctcccg aacccaaacg aaaaacaaaa aaaaatacgt cttaaataac cgcaaacaat 780
   acgatacgaa tacgtctctc ctatcgataa ctaatttaat ctaaaccatt tcatataaaa 840
   cccacgaaac aaaaaaaaca tactaaaaac acaaaattta aaccgcaata ataaaaaaat 900
   ttcctaaaat aaaacgccga ttacaaaaca aaatcgaaaa aacccaaact tattaaaatt 960
   tacattccct tacgctaaaa caaaaataaa acctaaaaaa ttaaaaaaaa aaaaaacccg 1020
   caacccaaac gaaacaatca accaaaacga aataaaaaaa aaacccatcg cgacgaaaac 1080
   tacataattt taattaacca aaaactacct aaactaatct aaactcattt atataaacca 1140
   taccttccct tcccgtaaaa aaaaaaatat caaaattcat attatttata aaaaataaaa 1200
   ataaataaaa aactcacccc cataaaaaaa aaccccaata aaaaaataaa aaaaccatcc 1260
   taaacaaaac tctatttcaa aaacaaacac aactttcctt ttaaacccta tctactaacc 1320
   aaaaattaaa atattcctcc caaaaataca aaaattaaca taatttacta tcacatacat 1380
   tcttactcaa aaaatttata tatatcaata attaaaacaa aactcaaaaa aactttacta 1440
   taatacaata aaaaaaacac taactttaaa attccaaacc caaactacta accaacttta 1500
   tcacttaccc caacgtaact tataaccaat cacccaatct ctaaacctta atttcctcac 1560
   ctcaaaaaaa aaataataat acctaaacca cttaataaat aacaaaataa tacctacgaa 1620
   atctataaca aacaaataca aaatttaatc actataaacg taataataat ctataccata 1680
   aaaataattt ccaaaaacaa aaataaaata tccaaaaaaa aattttaaaa aaaaaccttc 1740
   ctactaatat aacttaaaaa aacccccaaa ctacttatct ttctaaaatt caaaatctac 1800
   aaaaacaata tacaaacccc tacaaaaaaa aaaaacttcc aatcaactaa ccgcctccta 1860
   accctaacta ctctccaata tccactacct tcccacgacc taaccgaccc tcataacact 1920
   caccacactc taaataaaat ttcttacaaa tttaaaattt aaataaaata aatactacta 1980
   ttacttaaat aaaaaaaaaa accttttaaa aaaatccctc ataatttaac caactaaatt 2040
   aataaaaact aaaaaaatct aaaatttttt acttatttcc ttatttattt taataaatac 2100
   ctataaactt cctaaaatct caaacaaatt aaaaatattt ttctaaaaac taactccata 2160
   acttccatcg tattctcaaa aaataacccc ataaccccaa aactaaaaac tactaatata 2220
   aaaaattatc actaatccta cctaaatttt aaaattaacg ccttttattc ttcaatctcc 2280
   gaatcctaaa accttttaaa acctactctt aacaaattaa atctatattc cctaaaatcc 2340
   aaatacatat aattttcaaa cacaaaaaat caaaaaaaat ccataactaa ataacaataa 2400
   atactaaata tttccaaaac ccgttaccca ccgcccccac ctacaaataa aataattatc 2460
   cttttccccc acaaacttct cccctaaact ctttccattt aaaacacaca cacacacaca 2520
   cacacacaca cacacacact aaactctata tcaataaaac tcctttaaaa tcctatctat 2580
   cctccccact ccaacttcta ttttataaaa ataattatat tttaaacact taaaacatta 2640
   atttaataaa cacattatat ttactttcat atttaattat atatttttaa attattccac 2700
   acaaaataac aaataaaaac aaaaaaaaaa aataccataa aatatataaa aacgcacccc 2760
   ccaaaaacgc tcaacaatca atcaaaaatt cttaaaaaat aaaaacaact acgaaaccct 2820
   accccaaccc aatcaaaaca tcccacccga aaaaaaacga cttatttctt ttttctatta 2880
   aaaataaata ataaactaaa ttcgatttaa cgatattttc ttaactaact ctcgactaca 2940
   atcaaaccat tttcgaacac attcccatat aaataccaaa aatactctaa aaaaaactat 3000
   cgacgcctat tcaactaatt taaattttct ttatctaatt actataaaac ttaaaaaaaa 3060
   aaaaccgact caaaacaata aactaacact cgatctctat atataatatc caaacaaaat 3120
   ttaataaaaa aaacctactt ccactcaacc tcaaatcact atctaaaacc aaccccgacc 3180
   cacccccctc ccacataccc ctcccctccc cacccgccct ctctacatct acaaccccac 3240
   caacacactc aaccaaaaac aatcccccga cactccaaaa acacacaaaa ctcctaccgc 3300
   cccaaaaaac ctacctaaac tatacgacta taaaaactct ttacctaata tcttcctcct 3360
   aattactaca caacctccct ttctcctacc tcctacaact ccccaaactc cccaacaaac 3420
   acacccgtac acctcgatcc tttcaaccta aacgcaacga aatacccaac tctacctcct     3480
   atttaatccg ctaatctaca acaccttctt tctaaacctc ccccttcaac ccttaaaaaa     3540
   ccccgccaac tccgacctcc tactctcctc ctccctctcc caactcctat ccctaatcga 3600
   actacatttc cctacacgta taatccaata actaactatc gaatctcatt aaaaataatt 3660
   actcccgaaa acacatctct ctcaaaacct aactcttact taaaaaaaaa ttaaccacaa 3720
   ctaaaaaaaa atattctcga acaaaaatta actaaaacct aacacaattt taaataaaca     3780
   atattaaaac gaaaaatata aacctaataa caactacaca ccccttcaaa actaactata     3840
   aatataacca tctcgcttat ctcaaaatct ttctttttct ttctctttct ttcttttctt 3900
   ccttccttcc tttttctttt acactttttt cttacaaaac actaactaaa atacgaaaat 3960
   aaaaaaaaaa aataaaaaca aatacaaccc taaatcccat aaaatccaaa tttccaaaat 4020
   ctttaaaact taacctaaat ttctcaatat aaatccctct actcaaaatt ccgaaaacgt 4080
   ccacgatcgc caacgacccg caaccctacg acgaccccca atcgccctaa caattatcga 4140
   aaaacctaca aaccctacca aaaaaaaaaa aaattaacac gttaccttta aacgtctaac 4200
   accccgacgt ataactaaaa acgacaaaat ctaaacgaaa ttaacaaata aaatcaatat 4260
   cgaccctaaa aaaaacgacc aaaaaaactc cgaacaaccg cacccgcaac aacaccactc 4320
   gacccgacga accaaaaaaa aaaaaaaaac gcgaaaaaac gaaactaaat cctaaaataa 4380
   aacgcgaaac ttactacgaa aaacaaacaa aaaaaatatt tcataaaatc aaaaataatc 4440
   gaattttacg aaaaataaaa atcgtccccc taacaaaacc gaaataaatc tataatccct 4500
   caattaaaaa aaaaataaat aaaaaaaaaa aaaaaaaacc gcgaaaaaaa taaaaaacga 4560
   aaaaacccta aaaaacgaaa aacgaaataa aaaaaaaaaa cgaccgccaa atcgaaaaaa 4620
   aaaaaaataa acgcgacgac gcgaaacgaa aaaaaaacaa aacgaaataa aaaaaaaaaa 4680
   aaaaaaaaaa aaaacgaaaa aaacgaccga aaaaaacgaa aaaaaaaaaa aatccgaaaa 4740
   acaaaacgaa aaacgctcct aaaaaaaaac gtcgaactcg tccccgaaat ccaaaccgcg 4800
   atcccactcg cgaacgcaat cgcccgacgc cgaccgctcg accccgcgac ctaaaaaacc 4860
   gttacgcaaa acaaaacccg cgactcgaaa aaaaatacgc cgaacgttac ctcccgcccg 4920
   ccgccgccgc cgccgccaaa aaccccaaaa aaactaaaaa aaacgacgcc gaaacgctaa 4980
   cccgaaataa ccacgactac aacccgaacg acgaactaaa acgctcgcaa aacttctaaa 5040
   ccgaccccgc tccgacgcct ccgcttcccg cccgaaaccc gccctcgcga cccgacctcg 5100
   ctcccgaatc ccaaataacc accgaaaacg aaccgccgcc gaccccgctc cgccgcgcgt 5160
   acaacccgat ccccgacgcg ctccaaaccg ccctaataaa cccgccgccc gccgccgccg 5220
   ccgccgccgc cgccgccccg aaaaccacct cctcctcctc gtcgtcgtcc tccgcctcct 5280
   acgcctcgtc ctcgtcctcc tccaattcga ccaacgcccc ctcgactacc tacaaaaacg 5340
   cgaacgacga cgacgcgaac gccgaaaacg aaaacgccaa aaaaacgaac tcgaaactac 5400
   gacgacccga aaaaccgccc tactcgtaca tcgcgctcat cgtcataacc atccaaaact 5460
   cgcccaacaa acgcctaacg ctcaacgaaa tctaccaatt cctacaaacg cgcttcccct 5520
   tcttccgcga cgcctaccaa aactaaaaaa actcgatacg ccacaatctc tcgctcaacg 5580
   aatacttcat caaactacct aaaaacctcg aacgacccga caaaaaccac tactaaacca 5640
   tcgacccgac caacgaattc atattcgaaa aaaactcgtt ccgccgccga ccgcgcgact 5700
   tcaaacgaaa ataccaaacg ctcaaaccca tataccaccg cgtaataaac gacttaaact 5760
   tcgaaacgtc gctactaccc caaaacttcg acttccaaac gcccccgtcg acgccgctcg 5820
   actaccacaa ccaaaacgac tacgacgacc tcgacataat acccgcgaac tacgacgccg 5880
   acgcgaacgc ccccaaccac gcgcaccctc accaccacca ccaccaccac gtcccgcaca 5940
   tatcgcccaa cccgaattcc acctacataa ccaactaccc gatacccgcg aaacccgaaa 6000
   acgtcgatac gaccgaaaac gacgacgacg acgactacga accgaacaac aacaacaacc 6060
   cgataccctc gtccccgacc ataacgaacg ccatcgaata ccactcgccc tacacgaacc 6120
   ctacgacgca ctaaaactcg cctaacgcct cgccttacct caaacaaccg cctaccctaa 6180
   cgcccaacaa caaccccgcc gcctcgacaa acctacactc caacatatcc tcctactcgc 6240
   taaaacaaaa ctacttacac caaaacgctc gcgaaaacct ctcaaataac gcaacacgct 6300
   ccaacccaac caactaaacg caccgcttct aaacctccaa aactaacgac cactaccact 6360
   cccacaaaca aaaaccccga aactaaaaaa tctaaaaaca ctaaaaaaaa caaatactaa 6420
   aaaaaaccga catcttattc gtaaaaacaa aaatcaaaat cttactatcc tcccaatatc 6480
   cctaaccaac aaccgaaact tcaaaattat ctaactacta taaacgtaat ataaaatccc 6540
   ctaccttcct aaactcttta aaaatcccta tacaaaaaac tacaaatctc ccgttcaaaa 6600
   tctcacttaa cttaaaaaaa aaacatctaa cctttaaaaa acttaatcaa aaaactatat 6660
   ctaattcctt ttatcccttt ttttccttac gttcatttta ctatacttct atcccgaaat 6720
   aaatatcaaa atcaaatata ttctatatat taaaatatct acaacgaaat atttacaact 6780
   aaacaaatat taaaaaaaaa aaataaaaaa aacaaaacta aatcccaaaa tctttcacta 6840
   aaaaaacatt taccaataaa aaaaatcacc aaactattaa aaactcctcc ccttccctac 6900
   ctcccccccc gcccaacaat aaacccacat taataataca aatcctaaat aacaaaacct 6960
   aaaaaaccct actaaaactc ccctaaaaaa tacgcctacc ctataaatac cacttataat 7020
   ataactaatt tacattattc taattacaaa ttataaaaaa ttattaactt actaaaaacg 7080
   ataaaaaata aaaaaaaacg ataaaaataa aaaaaaaaat ttaaatattt aaccaaatta 7140
   atataaaaaa caaaaataaa cccttacatt aacccctcgc tcgaaacacc taaaactaca 7200
   aattaaaatc ccataaacct accaatcaaa aacaaaaccc caaactactc tctcacacta 7260
   acctcccaca acaataaaac aataacttaa aaaataatac atactttata tattcctaaa 7320
   ccgatataca acgaaaacac ccgaaaaaaa aaaaaaccca aaaataaaca aaaaatatta 7380
   attccctaaa cgataaaatt tcccaaataa accttttaaa tataaatctc aaaaattaac 7440
   caaacaaaaa caatctcgac aaaaccacac cgaaaaaaat tttaacctaa cttaacccac 7500
   taaaaacaaa atcttattca attttataaa aatttcaaca aataaccaat acaaacgacc 7560
   ttcccctctt atttttatat tcccttaaac cgactatcaa tcacacacac acacacacac 7620
   acacacacac acacacacac acacccctcg atacactaac cccttccctt ccccgcctac 7680
   ctactcctct cccaacctac caactaccct ccctactccc cttcttctcc tctcccgaca 7740
   aatccactca acttaaaccc gaactctaaa acttaacaac cttaaaaaaa ccgtactcta 7800
   aaaaaaaaaa aaaacacaca aaaaaaaaac tcctaaccct tcctcaaaac tactctcgcc 7860
   ccacctcata acactaaacg caaacacaaa ctcccaaaac ttcgaaatct aaatacatta 7920
   ctatcttaaa aaaaataaaa actacccaac aaaacaaacc gacctctata cccctaccca 7980
   acgacctccc gcgaccgacc ttccctaaaa acctcgcaaa acccccgacc gaatctaaaa 8040
   acgcccgaaa aaaacaataa ctcgaaccta acgcccgaca aaaaccctct acccttcctt 8100
   cctcgctatc ttactcccgt ccctataaaa tacccacccg accctattac catctcccta 8160
   aaacgccaac gataaaaact tcgaaaaaac cgaccgaata caccgaaacc ccgcgaaacc 8220
   taattaaaaa cgcgccgccc actaaaacct aaaaacccga cgaaacgcga aaataaccaa 8280
   aacaatctcc ccgacacaca aatcaacaca tatctacgcg cacacaccca acacgaccct 8340
   ccctccccca acacacccca aaaaccgcgc cctccgctcc taaacctaat aaccataatc 8400
   catatacccc taccgacgca aaaactaccc taaaattctc cattaaaccc tacgaacact 8460
   tcaaaaaaaa actcgaactt ctatcatccc gctcaacaca tcaaaacgcc gacgaccgcc 8520
   ttcgtaaaca aatataaacg ctcgccctct ctcctcccga acctactatt ttataatcca 8580
   aaacttacca tcaaaactta ctttaacgcg acgaaaaaac tcacctatat acaaaacgcc 8640
   gcgcacacat cgaaactcgc gaaacaaaac aactacgtta acgcaacgcc gaatatattc 8700
   gaataaatat tacgaacaaa aaaataaaca acgaccccta aacaaccgcc tcgctccgac 8760
   attacgaaaa cacgacgaaa ctaaaaccac gaaaacaaaa cccgaacccg acgaaccgta 8820
   attacgattt tcttacacta aaaaactaaa tccgacccga aacgacgtac actttataat 8880
   ccccacacca ctcgattaac taaaaaaaaa cccgaacgaa taaacctaac gcaacccgat 8940
   accgaaaaaa cccgatccaa caacctctaa aacccaatac gcaaaacact acgaaccgat 9000
   tacgccccga aaccaaaaaa cgccgaaaaa acaaaaacaa aaaccgacga cgaataaaaa 9060
   tcaaacgacc cgatacaaac cgaaaaccga aaaaccgccc cgaaaaaaca aaatccaaac 9120
   cttctctatc cccctccaac ctaaaactac ctcaaaaact cctcgcgcta ccaaatactt 9180
   cgaaccaaaa aacatttctc tacgccctaa aatcccgact tctctaaaaa aaatctaaaa 9240
   cgttcgaaac ccacgaaaat acctacgaaa aaacaaaaaa aaaaacgtac ttttctaccc 9300
   catcccttca tcttacgatt aaattatttt ctatcgtccc ctttaacaca aaaaatcctc 9360
   tacgctcgct actataaccg acccaattaa ttaaaaaaca aaactcaaaa aaaaaaccgc 9420
   aataatattt ctaaaataaa atacttaacc ctccctaaaa ccaaaactca aaacaacaca 9480
   aaccaaaaac tctaccgaaa aaaacccgaa accaaaatct aaatctaatt atccgatacc 9540
   tatactcatt ctaaaattaa aatacacgcc ccaacctcaa aaaataaaat taaaattcca 9600
   aaacataaaa atccccctcg aaaaaaaact tactctatat ttatacgaac atcaaaacaa 9660
   aacataataa ttccaaaaca attctataac gtacaattaa tatatttaaa ctacttctaa 9720
   aactaaactt tctctaccaa ctatctaaca atttactaaa aaacacaaca acacctttta 9780
   tactctaaaa caaaataaaa caccctacca tccactaaat aactttattt ctaaaaaaat 9840
   ttttttttct ttcttctttc aataaaacta ccccgttacc aacatcactc tactccaata     9900
   tataacaaaa aaaatttcgt cctcaacttc aataaaattt cttctttcca tccctcaact     9960
   aacgaatcgt attatcacca tcaccaccaa aacgtctatc aaaatattaa accctacgtc    10020
   atataaacga aaaaaaacca aacgataacc gctctctcct ctcccctcct caaaaaaaac    10080
   aaataaaaac taaaacgaat tcaaatcaca tacacgcgaa taacaataaa ccacacacct    10140
   accacttaac caaaataccc aaaatcgcgt taatcactat tatttaccta ctactaaaaa    10200
   aaaaacaacc gctaacaaaa taacgttccc caatctaaat acctacaaac tcccacataa    10260
   aaaaaaaaac aaactcaaat aaaaaaatat accatacgta aaacatcaac gtatatctat    10320
   aaaatcttcg ttaccttcaa taatcaaaat ataaaaaaaa caaacaaatt atatatatat    10380
   atatatatct aaaaaaactt atatactttt caaaaaaaca atactaaaca caaaatttca    10440
   aaaaaacctc ttcttattac ctaactaaat aaaaaaatca ttttccccaa acactacatt    10500
   taaatacaaa taccaaaaaa cattattaaa aaattattta aaaacaatat atctaattta    10560
   aaaaaataat tttcaatatt ataacaatac aacgttttta caaaattatt ttctaccacc    10620
   atattttaaa aatattttta taaccgtata tactcacact ttacttatat tttaaaaaaa    10680
   aaatatattt acacttatat atacttttac aatttaccaa aatattttat tataaaattt    10740
   tttttcaata aaatatatat aacaaataat tattttaaaa actaattttc tcttcgaaca    10800
   ccctaaaaac aataaatacc aatttctcct ccccgacaaa caactataca actctaccca    10860
   caccccctcc aataatataa ctaacataat ataaaatcta taccttaaat aacacaaacc    10920
   aaattctaat tataaatctt ctcaaaaaaa aaacaataaa aaaaacgttt tctattattt    10980
   ctttctttac ttcaccccaa ctttttctaa caaaacaaaa accaaaaaat aaaaaaaact    11040
   aaaaaaaaaa ccaaacaaaa taccgaccaa aatttaattt taaaaaaacc aattttatac    11100
   cccttctatt ttctttccta tacccgaaaa atattctacc ctttaccctc tctccctccc    11160
   gactaacata aattaacaaa tataaaacac aataataaac ccccctccaa aattaaatac    11220
   ccaaaaaaca acaccctact tcttcccttt acacgttaca attttattat aaataacgat    11280
   cacatactat caaaaataaa attcaaatct aaaaaataca aaaaaatccc taaactacaa    11340
   aaaacaaact cccaacaaaa aatttaactt ccttaaaaaa tttaatacaa aaactaaact    11400
   ccccacacgt tactctataa ctacgaccta tcccaccctc caattcaaaa cctaactaac    11460
   cctactcaac atcgacacat aacaaaccga ttcaaccctt ccttcctccc gacctcctct    11520
   ccccactcct cccttttaac caaaaattta acaaaaaaac ccctcaaccc ctttttctat    11580
   tcctactaaa tttcccaatc accaatatta caaacaaaac taataacgct cccgataaaa    11640
   aaacaaaata aaccaactaa acaaaaaaac taaccttcta tcccaacaac ccttcaccat    11700
   cacgaaccat attaaaaaca ataaatctaa aaaaaacaaa aaaattatct actccaatca    11760
   tactatacat tttcctctcc aattcaaaac taaaatttta tttttatttt ttactttttc 11820
   tttcttaatt aaaaaactca cctttatttc ttatccctta aaaaatcttt ctcccaatca 11880
   atcaccaaac tcttaatatt cgtaaaaaac tatttcacac cccaaaattt ttatattcta 11940
   aataaatttc actacaaaaa acgtctccta aataaaacat cttatttccg aaaatcttaa 12000
   ccatctttct aatctttaaa tttccttcct atatcttcta aaattaaaaa ataatttaaa 12060
   cattttttat ctctccatta tcatatttac aattaaataa aaccttttta ttttctttta 12120
   taataaacta ctcgaaaacc tactataaat tataaaatca atttcaaaat ttactccgac 12180
   cacaatacta tatttacatt ccttccgaaa caaccgacat aatcgtaatt tatatcctaa 12240
   acacttaaac gtaacttatt tatacaaaaa ataacaaaac ctcaaaaacg aaaactacgt 12300
   ctacctacct aaccgcatta aaaaacccaa cgcgccccct aataacgcga aaaaatccgc 12360
   ccaaaacgac tcaataattc cttcaaacta cgcaaaaact taacaaaata acccgtttta    12420
   taataccttc caataaaaac atttttaaat atctcacttt cataaattat aatttccaat    12480
   tttaaaaaat actttacttt cttctttctt cttcttcctt tttttttttt tttttaaaac 12540
   aaaatcgtaa tctattaccc aaactaaaat acaataacac gatctcaact cactacaacc 12600
   tccaccgcct aaactcaaat aatcctcccg cctcaacctc ctaaataact aaaaccacaa 12660
   acacgcacca ccatacctaa ctaatttttt aatttttata aaaacgaaat cttacctcgt 12720
   tacccaaacg taaacttttt tctattttac tcttttcgcc tactatctca tcctaaaacc 12780
   aaatcacaat tcaataaaaa caaacctaac catactcaac aaatactaaa accctaactt 12840
   actacgtata ttcttcattt ctacatttct ccccaaataa aactattatt ttatttcctt 12900
   ttaaatcaaa attatttctc tcctaactat ccctcaaaaa cacacacgta cacataaact 12960
   catttatatt cacccttttt tttttttttt taaaaaaaca tacaatactt aactttaaaa 13020
   attactcaaa tattaataat taaattaaaa aaaaatacta cacctttata aataaaaata 13080
   aataaactaa ctcaactaaa aataaatacc tcaactattt tttcgtcaaa taaattttaa 13140
   acttaaattt tctactcatt aacgaaataa aataatatat taaaaaattt tattattttc 13200
   tataataatt attatttttt attaatttca ttaaaactat atacataaaa acaaaaccaa 13260
   aaaactacta aacatcatac atcaaatact aaacaccgta actatcttac caaatctcac 13320
   acgactatct tacttaattc tatcaatatc cctctaaaat actaatatta accttattta 13380
   caaatctata ctaactccta actaaatcat aactttaaaa aatttaaccc atatcttcta 13440
   atttcaattt atttccctcg tttctccaac actacctcta aaaattttac aactaacact 13500
   acaaatcttt aaataacaaa aatattccct ctaaaacaac aattctcaca cttaaataca 13560
   catcaaaatc acctaaaaaa cttattcaac acaaactata aatccacccc accctccatt 13620
   tctaattcaa taaatccaaa attatattca aaaatatata ttcctaataa actaataaaa 13680
   aaaaataata ctaataatat taatcctaaa accacactct aaaaactaca actcttaaaa 13740
   aacaatttcg attacaaatc tttttataaa ttccaataaa taaatattaa aaataaccta 13800
   ttaattaatt aaacaaaaat ctaaaaaaat ttttcaaata atttatccat ctaaatacct 13860
   ctataattaa ttataaaccc ctaaaaaaat acaaaactta attttaaacc ctaaaccaaa 13920
   aaataaaatc tccccatttt tattcaaaac aataaaacct cttattttat tataataaac 13980
   attacaaaaa aaaatcaaat aaaatcatta aaatctacct acctcaataa aacaataaat 14040
   ctaaatactt acacacaaca accaatcaaa attctacaaa caaaaaaaaa caataataaa 14100
   aacaaacaaa aaaaaaaact ccattattaa ttataaaacc ttttaaaaat aaaacaaaaa 14160
   aaaaacaaaa aaaaaaaact ataatctcaa aaaataacaa taataatccc taaatataaa 14220
   taaataaaca aaaccgtaaa taaaaaaaaa aaaaaaacaa aaaaaaaaaa aaaaaaaaaa 14280
   aaaaaaaaaa aaaaaaaaat aaaaacaaaa aaaaaaaaaa aaaaaaaaaa aaataatcaa 14340
   aaaaaaaaaa aaaaaaaaaa aaaaaaaaaa aaaaaaaaaa aaaaaaaaaa aaaaaacaca 14400
   taaaaaaaaa aaaaaaaaaa aaacgaaaaa aaaaaaaaaa aaaaaaaaaa aaaaaaaaaa 14460
   taaaaaaaaa ataaaataaa ataaaaaaaa aacaaaaaaa aaaaaaaaac taaaaaaaaa 14520
   aaaaaaatac acaaaaaaaa aaaaaaaaaa aacaaaaata aataaaaaat ataaaaaaaa 14580
   aaaaaaaaaa aaaaaaaaaa aaaaaaacaa aaaaaaaaaa aaaataaaaa aaaaaaaaaa 14640
   aaaaaaaaca aaaaaaaaaa aataaaaaaa aaaaaaaatc taaaaaaaaa aaatctacaa 14700
   acatttccta aactcctatc cctaaaacta aaaaacaacc ctaaaaacta aattcctatc 14760
   ctctaaaacc accattctct ctccaaatcc ccaataccaa cccacgaact aaaaaaccca 14820
   taaacttcta ttttacttaa cttaacctaa attctaaaaa aataaataaa ttctctaatt 14880
   atacataatc aaattttcta aactaccaca acgacttata caaaattaaa aaaaacaatc 14940
   ttaacttaaa ttttaaaaaa taaaaatccg atttctaaaa aaaatcttca aatccctaaa 15000
   ataaaccacg atataacatt aaaaaaaatt aacaactcct tcccaaaaaa aacactctct 15060
   ttataaattc gtattatacc taattactct acatcataat aattaattca aaacaaacac 15120
   taacttactt aaaataaaaa tatattttaa tatattttat ccgaacgaaa atacatttta 15180
   ccaaacctac ctctataaaa cactatctta acaaaaaaaa acaccacaaa tataaacata 15240
   accaaaaaaa accaaaacac acaaacgtca aaatcattaa aaaaaaaata aaaatcctaa 15300
   aaattctttt taaattcaaa ataccccaaa tccacaacaa tccaactaat aaatattcat 15360
   ctctccatat cctaaaaaac gtatataaaa tacgaaacct taaccatccc acgactacat 15420
   cgccaaacct ataaacttaa ctatattcac gaaaacacaa acgaaacgaa caacgaaaca 15480
   actttccctc cgaaaaaccc attccaccct ccaacctaac aacgaaaact cgatcacaac 15540
   cgaacaataa ctcgtctacc gaataacacg acctcacctc cccaaactaa caaaacacct    15600
   ccccctcaaa tttaaaaacc gctataaatt aaataaaatc caaacctaaa atataaaaaa    15660
   aaaaaaacct acctcctcgc tcctccttca acaactccac gcaacacaaa acgaaaatat    15720
   cgataaaccg tattaattaa ctaactaaac cccaacgcgc cgta                     15764
<210> 30
   <211> 15764
   <212> DNA
   <213> Artificial Sequence
<220>
   <223> G to A (bis2 rc) rc
<400> 30
   tacggcgcgt tggggtttag ttagttaatt aatacggttt atcgatattt tcgttttgtg      60
   ttgcgtggag ttgttgaagg aggagcgagg aggtaggttt tttttttttt atattttagg     120
   tttggatttt atttaattta tagcggtttt taaatttgag ggggaggtgt tttgttagtt     180
   tggggaggtg aggtcgtgtt attcggtaga cgagttattg ttcggttgtg atcgagtttt     240
   cgttgttagg ttggagggtg gaatgggttt ttcggaggga aagttgtttc gttgttcgtt     300
   tcgtttgtgt tttcgtgaat atagttaagt ttataggttt ggcgatgtag tcgtgggatg     360
   gttaaggttt cgtattttat atacgttttt taggatatgg agagatgaat atttattagt     420
   tggattgttg tggatttggg gtattttgaa tttaaaaaga atttttagga tttttatttt     480
   ttttttaatg attttgacgt ttgtgtgttt tggttttttt tggttatgtt tatatttgtg        540
   gtgttttttt ttgttaagat agtgttttat agaggtaggt ttggtaaaat gtattttcgt      600
   tcggataaaa tatattaaaa tatattttta ttttaagtaa gttagtgttt gttttgaatt     660
   aattattatg atgtagagta attaggtata atacgaattt ataaagagag tgtttttttt      720
   gggaaggagt tgttaatttt ttttaatgtt atatcgtggt ttattttagg gatttgaaga     780
   ttttttttag aaatcggatt tttatttttt aaaatttaag ttaagattgt tttttttaat      840
   tttgtataag tcgttgtggt agtttagaaa atttgattat gtataattag agaatttatt     900
   tattttttta gaatttaggt taagttaagt aaaatagaag tttatgggtt ttttagttcg     960
   tgggttggta ttggggattt ggagagagaa tggtggtttt agaggatagg aatttagttt    1020
   ttagggttgt tttttagttt tagggatagg agtttaggaa atgtttgtag attttttttt    1080
   tttagatttt tttttttttt tatttttttt tttttgtttt tttttttttt ttttttttta     1140
   tttttttttt ttttttgttt tttttttttt tttttttttt tttttttttt ttatattttt    1200
   tatttatttt tgtttttttt tttttttttt ttgtgtattt tttttttttt tttagttttt     1260
   tttttttttt tgtttttttt ttattttatt ttattttttt tttatttttt tttttttttt      1320
   tttttttttt tttttttttc gttttttttt tttttttttt tttatgtgtt tttttttttt 1380
   tttttttttt tttttttttt tttttttttt tttttttttt ttttttgatt attttttttt 1440
   tttttttttt ttttttttgt ttttattttt tttttttttt tttttttttt tttttttttt 1500
   ttttttgttt tttttttttt tttatttacg gttttgttta tttatttata tttagggatt 1560
   attattgtta ttttttgaga ttatagtttt ttttttttgt tttttttttg ttttattttt 1620
   aaaaggtttt ataattaata atggagtttt tttttttgtt tgtttttatt attgtttttt 1680
   tttgtttgta gaattttgat tggttgttgt gtgtaagtat ttagatttat tgttttattg 1740
   aggtaggtag attttaatga ttttatttga tttttttttg taatgtttat tataataaaa 1800
   taagaggttt tattgttttg aataaaaatg gggagatttt attttttggt ttagggttta 1860
   aaattaagtt ttgtattttt ttaggggttt ataattaatt atagaggtat ttagatggat 1920
   aaattatttg aaaaattttt ttagattttt gtttaattaa ttaataggtt atttttaata 1980
   tttatttatt ggaatttata aaaagatttg taatcgaaat tgttttttaa gagttgtagt 2040
   ttttagagtg tggttttagg attaatatta ttagtattat ttttttttat tagtttatta 2100
   ggaatatata tttttgaata taattttgga tttattgaat tagaaatgga gggtggggtg 2160
   gatttatagt ttgtgttgaa taagtttttt aggtgatttt gatgtgtatt taagtgtgag 2220
   aattgttgtt ttagagggaa tatttttgtt atttaaagat ttgtagtgtt agttgtaaaa 2280
   tttttagagg tagtgttgga gaaacgaggg aaataaattg aaattagaag atatgggtta 2340
   aattttttaa agttatgatt tagttaggag ttagtataga tttgtaaata aggttaatat 2400
   tagtatttta gagggatatt gatagaatta agtaagatag tcgtgtgaga tttggtaaga 2460
   tagttacggt gtttagtatt tgatgtatga tgtttagtag ttttttggtt ttgtttttat 2520
   gtatatagtt ttaatgaaat taataaaaaa taataattat tatagaaaat aataaaattt 2580
   tttaatatat tattttattt cgttaatgag tagaaaattt aagtttaaaa tttatttgac 2640
   gaaaaaatag ttgaggtatt tatttttagt tgagttagtt tatttatttt tatttataaa 2700
   ggtgtagtat ttttttttaa tttaattatt aatatttgag taatttttaa agttaagtat 2760
   tgtatgtttt tttaaaaaaa aaaaaaaaag ggtgaatata aatgagttta tgtgtacgtg 2820
   tgtgtttttg agggatagtt aggagagaaa taattttgat ttaaaaggaa ataaaataat 2880
   agttttattt ggggagaaat gtagaaatga agaatatacg tagtaagtta gggttttagt 2940
   atttgttgag tatggttagg tttgttttta ttgaattgtg atttggtttt aggatgagat 3000
   agtaggcgaa aagagtaaaa tagaaaaaag tttacgtttg ggtaacgagg taagatttcg 3060
   tttttataaa aattaaaaaa ttagttaggt atggtggtgc gtgtttgtgg ttttagttat 3120
   ttaggaggtt gaggcgggag gattatttga gtttaggcgg tggaggttgt agtgagttga 3180
   gatcgtgtta ttgtatttta gtttgggtaa tagattacga ttttgtttta aaaaaaaaaa 3240
   aaaaaaggaa gaagaagaaa gaagaaagta aagtattttt taaaattgga aattataatt     3300
   tatgaaagtg agatatttaa aaatgttttt attggaaggt attataaaac gggttatttt     3360
   gttaagtttt tgcgtagttt gaaggaatta ttgagtcgtt ttgggcggat tttttcgcgt     3420
   tattaggggg cgcgttgggt tttttaatgc ggttaggtag gtagacgtag ttttcgtttt     3480
   tgaggttttg ttattttttg tataaataag ttacgtttaa gtgtttagga tataaattac     3540
   gattatgtcg gttgtttcgg aaggaatgta aatatagtat tgtggtcgga gtaaattttg     3600
   aaattgattt tataatttat agtaggtttt cgagtagttt attataaaag aaaataaaaa     3660
   ggttttattt aattgtaaat atgataatgg agagataaaa aatgtttaaa ttatttttta     3720
   attttagaag atataggaag gaaatttaaa gattagaaag atggttaaga ttttcggaaa     3780
   taagatgttt tatttaggag acgttttttg tagtgaaatt tatttagaat ataaaaattt     3840
   tggggtgtga aatagttttt tacgaatatt aagagtttgg tgattgattg ggagaaagat     3900
   tttttaaggg ataagaaata aaggtgagtt ttttaattaa gaaagaaaaa gtaaaaaata     3960
   aaaataaaat tttagttttg aattggagag gaaaatgtat agtatgattg gagtagataa     4020
   tttttttgtt ttttttagat ttattgtttt taatatggtt cgtgatggtg aagggttgtt     4080
   gggatagaag gttagttttt ttgtttagtt ggtttatttt gtttttttat cgggagcgtt     4140
   attagttttg tttgtaatat tggtgattgg gaaatttagt aggaatagaa aaaggggttg     4200
   aggggttttt ttgttaaatt tttggttaaa agggaggagt ggggagagga ggtcgggagg     4260
   aaggaagggt tgaatcggtt tgttatgtgt cgatgttgag tagggttagt taggttttga     4320
   attggagggt gggataggtc gtagttatag agtaacgtgt ggggagttta gtttttgtat     4380
   taaatttttt aaggaagtta aattttttgt tgggagtttg ttttttgtag tttagggatt     4440
   tttttgtatt ttttagattt gaattttatt tttgatagta tgtgatcgtt atttataata     4500
   aaattgtaac gtgtaaaggg aagaagtagg gtgttgtttt ttgggtattt aattttggag     4560
   gggggtttat tattgtgttt tatatttgtt aatttatgtt agtcgggagg gagagagggt     4620
   aaagggtaga atatttttcg ggtataggaa agaaaataga aggggtataa aattggtttt     4680
   tttaaaatta aattttggtc ggtattttgt ttggtttttt ttttagtttt ttttattttt     4740
   tggtttttgt tttgttagaa aaagttgggg tgaagtaaag aaagaaataa tagaaaacgt     4800
   tttttttatt gttttttttt tgagaagatt tataattaga atttggtttg tgttatttaa     4860
   ggtatagatt ttatattatg ttagttatat tattggaggg ggtgtgggta gagttgtata     4920
   gttgtttgtc ggggaggaga aattggtatt tattgttttt agggtgttcg aagagaaaat     4980
   tagtttttaa aataattatt tgttatatat attttattga aaaaaaattt tataataaaa     5040
   tattttggta aattgtaaaa gtatatataa gtgtaaatat attttttttt taaaatataa     5100
   gtaaagtgtg agtatatacg gttataaaaa tatttttaaa atatggtggt agaaaataat 5160
   tttgtaaaaa cgttgtattg ttataatatt gaaaattatt tttttaaatt agatatattg 5220
   tttttaaata attttttaat aatgtttttt ggtatttgta tttaaatgta gtgtttgggg 5280
   aaaatgattt ttttatttag ttaggtaata agaagaggtt tttttgaaat tttgtgttta 5340
   gtattgtttt tttgaaaagt atataagttt ttttagatat atatatatat atataatttg 5400
   tttgtttttt ttatattttg attattgaag gtaacgaaga ttttatagat atacgttgat 5460
   gttttacgta tggtatattt ttttatttga gtttgttttt ttttttatgt gggagtttgt 5520
   aggtatttag attggggaac gttattttgt tagcggttgt ttttttttta gtagtaggta 5580
   aataatagtg attaacgcga ttttgggtat tttggttaag tggtaggtgt gtggtttatt 5640
   gttattcgcg tgtatgtgat ttgaattcgt tttagttttt atttgttttt tttgaggagg 5700
   ggagaggaga gagcggttat cgtttggttt tttttcgttt atatgacgta gggtttaata 5760
   ttttgataga cgttttggtg gtgatggtga taatacgatt cgttagttga gggatggaaa 5820
   gaagaaattt tattgaagtt gaggacgaaa ttttttttgt tatatattgg agtagagtga 5880
   tgttggtaac ggggtagttt tattgaaaga agaaagaaaa aaaaattttt ttagaaataa 5940
   agttatttag tggatggtag ggtgttttat tttgttttag agtataaaag gtgttgttgt 6000
   gttttttagt aaattgttag atagttggta gagaaagttt agttttagaa gtagtttaaa 6060
   tatattaatt gtacgttata gaattgtttt ggaattatta tgttttgttt tgatgttcgt 6120
   ataaatatag agtaagtttt ttttcgaggg ggatttttat gttttggaat tttaatttta 6180
   ttttttgagg ttggggcgtg tattttaatt ttagaatgag tataggtatc ggataattag 6240
   atttagattt tggtttcggg tttttttcgg tagagttttt ggtttgtgtt gttttgagtt 6300
   ttggttttag ggagggttaa gtattttatt ttagaaatat tattgcggtt ttttttttga 6360
   gttttgtttt ttaattaatt gggtcggtta tagtagcgag cgtagaggat tttttgtgtt 6420
   aaaggggacg atagaaaata atttaatcgt aagatgaagg gatggggtag aaaagtacgt 6480
   tttttttttt gttttttcgt aggtattttc gtgggtttcg aacgttttag atttttttta 6540
   gagaagtcgg gattttaggg cgtagagaaa tgttttttgg ttcgaagtat ttggtagcgc 6600
   gaggagtttt tgaggtagtt ttaggttgga gggggataga gaaggtttgg attttgtttt 6660
   ttcggggcgg tttttcggtt ttcggtttgt atcgggtcgt ttgattttta ttcgtcgtcg 6720
   gtttttgttt ttgttttttc ggcgtttttt ggtttcgggg cgtaatcggt tcgtagtgtt 6780
   ttgcgtattg ggttttagag gttgttggat cgggtttttt cggtatcggg ttgcgttagg 6840
   tttattcgtt cgggtttttt tttagttaat cgagtggtgt ggggattata aagtgtacgt 6900
   cgtttcgggt cggatttagt tttttagtgt aagaaaatcg taattacggt tcgtcgggtt 6960
   cgggttttgt tttcgtggtt ttagtttcgt cgtgttttcg taatgtcgga gcgaggcggt 7020
   tgtttagggg tcgttgttta tttttttgtt cgtaatattt attcgaatat attcggcgtt 7080
   gcgttaacgt agttgttttg tttcgcgagt ttcgatgtgt gcgcggcgtt ttgtatatag 7140
   gtgagttttt tcgtcgcgtt aaagtaagtt ttgatggtaa gttttggatt ataaaatagt 7200
   aggttcggga ggagagaggg cgagcgttta tatttgttta cgaaggcggt cgtcggcgtt 7260
   ttgatgtgtt gagcgggatg atagaagttc gagttttttt ttgaagtgtt cgtagggttt 7320
   aatggagaat tttagggtag tttttgcgtc ggtaggggta tatggattat ggttattagg 7380
   tttaggagcg gagggcgcgg tttttggggt gtgttggggg agggagggtc gtgttgggtg 7440
   tgtgcgcgta gatatgtgtt gatttgtgtg tcggggagat tgttttggtt attttcgcgt 7500
   ttcgtcgggt ttttaggttt tagtgggcgg cgcgttttta attaggtttc gcggggtttc 7560
   ggtgtattcg gtcggttttt tcgaagtttt tatcgttggc gttttaggga gatggtaata 7620
   gggtcgggtg ggtattttat agggacggga gtaagatagc gaggaaggaa gggtagaggg 7680
   tttttgtcgg gcgttaggtt cgagttattg tttttttcgg gcgtttttag attcggtcgg 7740
   gggttttgcg aggtttttag ggaaggtcgg tcgcgggagg tcgttgggta ggggtataga 7800
   ggtcggtttg ttttgttggg tagtttttat tttttttaag atagtaatgt atttagattt 7860
   cgaagttttg ggagtttgtg tttgcgttta gtgttatgag gtggggcgag agtagttttg 7920
   aggaagggtt aggagttttt tttttgtgtg tttttttttt tttttagagt acggtttttt 7980
   taaggttgtt aagttttaga gttcgggttt aagttgagtg gatttgtcgg gagaggagaa 8040
   gaaggggagt agggagggta gttggtaggt tgggagagga gtaggtaggc ggggaaggga 8100
   aggggttagt gtatcgaggg gtgtgtgtgt gtgtgtgtgt gtgtgtgtgt gtgtgtgtgt 8160
   gtgattgata gtcggtttaa gggaatataa aaataagagg ggaaggtcgt ttgtattggt 8220
   tatttgttga aatttttata aaattgaata agattttgtt tttagtgggt taagttaggt 8280
   taaaattttt ttcggtgtgg ttttgtcgag attgtttttg tttggttaat ttttgagatt 8340
   tatatttaaa aggtttattt gggaaatttt atcgtttagg gaattaatat tttttgttta 8400
   tttttgggtt tttttttttt tcgggtgttt tcgttgtata tcggtttagg aatatataaa 8460
   gtatgtatta ttttttaagt tattgtttta ttgttgtggg aggttagtgt gagagagtag 8520
   tttggggttt tgtttttgat tggtaggttt atgggatttt aatttgtagt tttaggtgtt 8580
   tcgagcgagg ggttaatgta agggtttatt tttgtttttt atattaattt ggttaaatat 8640
   ttaaattttt ttttttattt ttatcgtttt tttttatttt ttatcgtttt tagtaagtta 8700
   ataatttttt ataatttgta attagaataa tgtaaattag ttatattata agtggtattt 8760
   atagggtagg cgtatttttt aggggagttt tagtagggtt ttttaggttt tgttatttag 8820
   gatttgtatt attaatgtgg gtttattgtt gggcgggggg ggaggtaggg aaggggagga 8880
   gtttttaata gtttggtgat tttttttatt ggtaaatgtt tttttagtga aagattttgg 8940
   gatttagttt tgtttttttt attttttttt tttaatattt gtttagttgt aaatatttcg 9000
   ttgtagatat tttaatatat agaatatatt tgattttgat atttatttcg ggatagaagt 9060
   atagtaaaat gaacgtaagg aaaaaaaggg ataaaaggaa ttagatatag ttttttgatt 9120
   aagtttttta aaggttagat gttttttttt taagttaagt gagattttga acgggagatt 9180
   tgtagttttt tgtataggga tttttaaaga gtttaggaag gtaggggatt ttatattacg 9240
   tttatagtag ttagataatt ttgaagtttc ggttgttggt tagggatatt gggaggatag 9300
   taagattttg atttttgttt ttacgaataa gatgtcggtt ttttttagta tttgtttttt 9360
   ttagtgtttt tagatttttt agtttcgggg tttttgtttg tgggagtggt agtggtcgtt 9420
   agttttggag gtttagaagc ggtgcgttta gttggttggg ttggagcgtg ttgcgttatt 9480
   tgagaggttt tcgcgagcgt tttggtgtaa gtagttttgt tttagcgagt aggaggatat 9540
   gttggagtgt aggtttgtcg aggcggcggg gttgttgttg ggcgttaggg taggcggttg 9600
   tttgaggtaa ggcgaggcgt taggcgagtt ttagtgcgtc gtagggttcg tgtagggcga 9660
   gtggtattcg atggcgttcg ttatggtcgg ggacgagggt atcgggttgt tgttgttgtt 9720
   cggttcgtag tcgtcgtcgt cgtcgttttc ggtcgtatcg acgttttcgg gtttcgcggg 9780
   tatcgggtag ttggttatgt aggtggaatt cgggttgggc gatatgtgcg ggacgtggtg 9840
   gtggtggtgg tggtgagggt gcgcgtggtt gggggcgttc gcgtcggcgt cgtagttcgc 9900
   gggtattatg tcgaggtcgt cgtagtcgtt ttggttgtgg tagtcgagcg gcgtcgacgg 9960
   gggcgtttgg aagtcgaagt tttggggtag tagcgacgtt tcgaagttta agtcgtttat 10020
   tacgcggtgg tatatgggtt tgagcgtttg gtattttcgt ttgaagtcgc gcggtcggcg 10080
   gcggaacgag tttttttcga atatgaattc gttggtcggg tcgatggttt agtagtggtt 10140
   tttgtcgggt cgttcgaggt ttttaggtag tttgatgaag tattcgttga gcgagagatt 10200
   gtggcgtatc gagttttttt agttttggta ggcgtcgcgg aagaagggga agcgcgtttg 10260
   taggaattgg tagatttcgt tgagcgttag gcgtttgttg ggcgagtttt ggatggttat 10320
   gacgatgagc gcgatgtacg agtagggcgg tttttcgggt cgtcgtagtt tcgagttcgt 10380
   ttttttggcg ttttcgtttt cggcgttcgc gtcgtcgtcg ttcgcgtttt tgtaggtagt 10440
   cgagggggcg ttggtcgaat tggaggagga cgaggacgag gcgtaggagg cggaggacga 10500
   cgacgaggag gaggaggtgg ttttcggggc ggcggcggcg gcggcggcgg cggcgggcgg 10560
   cgggtttatt agggcggttt ggagcgcgtc ggggatcggg ttgtacgcgc ggcggagcgg 10620
   ggtcggcggc ggttcgtttt cggtggttat ttgggattcg ggagcgaggt cgggtcgcga 10680
   gggcgggttt cgggcgggaa gcggaggcgt cggagcgggg tcggtttaga agttttgcga 10740
   gcgttttagt tcgtcgttcg ggttgtagtc gtggttattt cgggttagcg tttcggcgtc 10800
   gtttttttta gtttttttgg ggtttttggc ggcggcggcg gcggcgggcg ggaggtaacg 10860
   ttcggcgtat tttttttcga gtcgcgggtt ttgttttgcg taacggtttt ttaggtcgcg 10920
   gggtcgagcg gtcggcgtcg ggcgattgcg ttcgcgagtg ggatcgcggt ttggatttcg 10980
   gggacgagtt cgacgttttt ttttaggagc gtttttcgtt ttgtttttcg gatttttttt 11040
   ttttttcgtt tttttcggtc gtttttttcg tttttttttt tttttttttt ttttttattt 11100
   cgttttgttt ttttttcgtt tcgcgtcgtc gcgtttattt tttttttttt cgatttggcg 11160
   gtcgtttttt ttttttattt cgtttttcgt tttttagggt tttttcgttt tttatttttt 11220
   tcgcggtttt tttttttttt ttttatttat ttttttttta attgagggat tatagattta 11280
   tttcggtttt gttaggggga cgatttttat ttttcgtaaa attcgattat ttttgatttt 11340
   atgaaatatt ttttttgttt gtttttcgta gtaagtttcg cgttttattt taggatttag 11400
   tttcgttttt tcgcgttttt tttttttttt tggttcgtcg ggtcgagtgg tgttgttgcg 11460
   ggtgcggttg ttcggagttt ttttggtcgt tttttttagg gtcgatattg attttatttg 11520
   ttaatttcgt ttagattttg tcgtttttag ttatacgtcg gggtgttaga cgtttaaagg 11580
   taacgtgtta attttttttt tttttggtag ggtttgtagg tttttcgata attgttaggg 11640
   cgattggggg tcgtcgtagg gttgcgggtc gttggcgatc gtggacgttt tcggaatttt 11700
   gagtagaggg atttatattg agaaatttag gttaagtttt aaagattttg gaaatttgga 11760
   ttttatggga tttagggttg tatttgtttt tatttttttt ttttattttc gtattttagt 11820
   tagtgttttg taagaaaaaa gtgtaaaaga aaaaggaagg aaggaagaaa agaaagaaag 11880
   agaaagaaaa agaaagattt tgagataagc gagatggtta tatttatagt tagttttgaa 11940
   ggggtgtgta gttgttatta ggtttatatt tttcgtttta atattgttta tttaaaattg 12000
   tgttaggttt tagttaattt ttgttcgaga atattttttt ttagttgtgg ttaatttttt 12060
   tttaagtaag agttaggttt tgagagagat gtgttttcgg gagtaattat ttttaatgag 12120
   attcgatagt tagttattgg attatacgtg tagggaaatg tagttcgatt agggatagga 12180
   gttgggagag ggaggaggag agtaggaggt cggagttggc ggggtttttt aagggttgaa 12240
   gggggaggtt tagaaagaag gtgttgtaga ttagcggatt aaataggagg tagagttggg 12300
   tatttcgttg cgtttaggtt gaaaggatcg aggtgtacgg gtgtgtttgt tggggagttt 12360
   ggggagttgt aggaggtagg agaaagggag gttgtgtagt aattaggagg aagatattag 12420
   gtaaagagtt tttatagtcg tatagtttag gtaggttttt tggggcggta ggagttttgt 12480
   gtgtttttgg agtgtcgggg gattgttttt ggttgagtgt gttggtgggg ttgtagatgt 12540
   agagagggcg ggtggggagg ggaggggtat gtgggagggg ggtgggtcgg ggttggtttt 12600
   agatagtgat ttgaggttga gtggaagtag gtttttttta ttaaattttg tttggatatt 12660
   atatatagag atcgagtgtt agtttattgt tttgagtcgg tttttttttt ttaagtttta 12720
   tagtaattag ataaagaaaa tttaaattag ttgaataggc gtcgatagtt ttttttagag 12780
   tatttttggt atttatatgg gaatgtgttc gaaaatggtt tgattgtagt cgagagttag 12840
   ttaagaaaat atcgttaaat cgaatttagt ttattattta tttttaatag aaaaaagaaa 12900
   taagtcgttt tttttcgggt gggatgtttt gattgggttg gggtagggtt tcgtagttgt 12960
   ttttattttt taagaatttt tgattgattg ttgagcgttt ttggggggtg cgtttttata 13020
   tattttatgg tatttttttt ttttgttttt atttgttatt ttgtgtggaa taatttaaaa 13080
   atatataatt aaatatgaaa gtaaatataa tgtgtttatt aaattaatgt tttaagtgtt 13140
   taaaatataa ttatttttat aaaatagaag ttggagtggg gaggatagat aggattttaa 13200
   aggagtttta ttgatataga gtttagtgtg tgtgtgtgtg tgtgtgtgtg tgtgtgtgtg 13260
   ttttaaatgg aaagagttta ggggagaagt ttgtggggga aaaggataat tattttattt 13320
   gtaggtgggg gcggtgggta acgggttttg gaaatattta gtatttattg ttatttagtt 13380
   atggattttt tttgattttt tgtgtttgaa aattatatgt atttggattt tagggaatat 13440
   agatttaatt tgttaagagt aggttttaaa aggttttagg attcggagat tgaagaataa 13500
   aaggcgttaa ttttaaaatt taggtaggat tagtgataat tttttatatt agtagttttt 13560
   agttttgggg ttatggggtt attttttgag aatacgatgg aagttatgga gttagttttt 13620
   agaaaaatat ttttaatttg tttgagattt taggaagttt ataggtattt attaaaataa 13680
   ataaggaaat aagtaaaaaa ttttagattt ttttagtttt tattaattta gttggttaaa 13740
   ttatgaggga tttttttaaa aggttttttt ttttatttaa gtaatagtag tatttatttt 13800
   atttaaattt taaatttgta agaaatttta tttagagtgt ggtgagtgtt atgagggtcg 13860
   gttaggtcgt gggaaggtag tggatattgg agagtagtta gggttaggag gcggttagtt 13920
   gattggaagt tttttttttt tgtaggggtt tgtatattgt ttttgtagat tttgaatttt 13980
   agaaagataa gtagtttggg ggttttttta agttatatta gtaggaaggt ttttttttaa 14040
   aatttttttt tggatatttt atttttgttt ttggaaatta tttttatggt atagattatt 14100
   attacgttta tagtgattaa attttgtatt tgtttgttat agatttcgta ggtattattt 14160
   tgttatttat taagtggttt aggtattatt attttttttt tgaggtgagg aaattaaggt 14220
   ttagagattg ggtgattggt tataagttac gttggggtaa gtgataaagt tggttagtag 14280
   tttgggtttg gaattttaaa gttagtgttt tttttattgt attatagtaa agtttttttg 14340
   agttttgttt taattattga tatatataaa ttttttgagt aagaatgtat gtgatagtaa 14400
   attatgttaa tttttgtatt tttgggagga atattttaat ttttggttag tagatagggt 14460
   ttaaaaggaa agttgtgttt gtttttgaaa tagagttttg tttaggatgg tttttttatt 14520
   tttttattgg ggtttttttt tatgggggtg agttttttat ttatttttat tttttataaa 14580
   taatatgaat tttgatattt ttttttttac gggaagggaa ggtatggttt atataaatga 14640
   gtttagatta gtttaggtag tttttggtta attaaaatta tgtagttttc gtcgcgatgg 14700
   gttttttttt tatttcgttt tggttgattg tttcgtttgg gttgcgggtt tttttttttt 14760
   ttaatttttt aggttttatt tttgttttag cgtaagggaa tgtaaatttt aataagtttg 14820
   ggttttttcg attttgtttt gtaatcggcg ttttatttta ggaaattttt ttattattgc 14880
   ggtttaaatt ttgtgttttt agtatgtttt ttttgtttcg tgggttttat atgaaatggt 14940
   ttagattaaa ttagttatcg ataggagaga cgtattcgta tcgtattgtt tgcggttatt 15000
   taagacgtat ttttttttgt ttttcgtttg ggttcgggag gagggaagtt tttttttaat 15060
   ttaaaatgag attgattttt gttttcgttt tgtatttagg cgtaaagtta agtcgtgtag 15120
   aggattttcg aatttttgcg aggttttttt aaatgtggac ggcgagaatt tagtgttggg 15180
   gtttgggggt gttagcgtga ttgaggaaat gttggaagag tattagtttg tacgttgtag 15240
   aggttttttt agaaatttta tataatagta gaagatgcgg acgggaatag taataggagg 15300
   acgcgcggag gcgggggttg gagagagtaa ggtggtcggg agtgggaatt ttatattttt 15360
   tttggagttt gttttttttt ttaggtttag aaatttttag attttcgggt ttgaatatta 15420
   gttatttagt ttttagtgtt tatttggata gagtttttcg ttttttaaag gtttgtgttt 15480
   cggttagggt tttttttata gttattttta tgagagattt tttagtagtt ttagattttt 15540
   tggtttgggc ggtgggtacg attcgatgat ttattggtta ggtttgaggg tgtttgaagg 15600
   gggaaaaagg ggtttaattt acgagatttt taagcgaaga tcggttttat tttattttcg 15660
   tattttttaa attgtcgggc gcgggttttt tcggttgcga gattaaaatt ttgggatgat 15720
   tttaggagtt agtttagcgg tttgtaggtt tcgattttag tgcg 15764
<210> 31
   <211> 5764
   <212> DNA
   <213> Homo sapiens
<400> 31
   agcccgggcg gcgggctagg gcgctcgcag ggcttctggg ccgaccccgc tccggcgcct        60
   ccgcttcccg cccggggccc gccctcgcgg cccggcctcg ctcccgggtc ccagatgacc       120
   accgagggcg ggccgccgcc ggccccgctc cgccgcgcgt gcagcccggt ccccggcgcg       180
   ctccaggccg ccctgatgag cccgccgccc gccgccgccg ccgccgccgc cgccgccccg       240
   gagaccacct cctcctcctc gtcgtcgtcc tccgcctcct gcgcctcgtc ctcgtcctcc       300
   tccaattcgg ccagcgcccc ctcggctgcc tgcaagagcg cgggcggcgg cggcgcgggc       360
   gccgggagcg ggggcgccaa gaaggcgagc tcggggctgc ggcggcccga gaagccgccc       420
   tactcgtaca tcgcgctcat cgtcatggcc atccagagct cgcccagcaa gcgcctgacg       480
   ctcagcgaga tctaccagtt cctgcaggcg cgcttcccct tcttccgcgg cgcctaccag 540
   ggctggaaga actcggtgcg ccacaatctc tcgctcaacg agtgcttcat caagctgcct 600
   aagggcctcg ggcggcccgg caagggccac tactggacca tcgacccggc cagcgagttc 660
   atgttcgagg agggctcgtt ccgccgccgg ccgcgcggct tcaggcggaa gtgccaggcg 720
   ctcaagccca tgtaccaccg cgtggtgagc ggcttgggct tcggggcgtc gctgctgccc 780
   cagggcttcg acttccaggc gcccccgtcg gcgccgctcg gctgccacag ccagggcggc 840
   tacggcggcc tcgacatgat gcccgcgggc tacgacgccg gcgcgggcgc ccccagccac 900
   gcgcaccctc accaccacca ccaccaccac gtcccgcaca tgtcgcccaa cccgggttcc 960
   acctacatgg ccagctgccc ggtgcccgcg ggacccgggg gcgtcggtgc ggccgggggc 1020
   ggcggcggcg gcgactacgg gccggacagc agcagcagcc cggtaccctc gtccccggcc 1080
   atggcgagcg ccatcgaatg ccactcgccc tacacgagcc ctgcggcgca ctggagctcg 1140
   cctggcgcct cgccttacct caagcagccg cctgccctga cgcccagcag caaccccgcc 1200
   gcctcggcag gcctgcactc cagcatgtcc tcctactcgc tggagcagag ctacttgcac 1260
   cagaacgctc gcgaggacct ctcaggtaac gcagcacgct ccagcccagc cagctgggcg 1320
   caccgcttct aggcctccag ggctggcggc cactgccact cccacaaaca gggaccccga 1380
   agctaggagg tctgagggca ctgggaaaag cagatgctgg ggaaagccgg catcttattc 1440
   gtgggagcag ggatcagggt cttactgtcc tcccagtatc cctggccagc agccgaagct 1500
   tcagaattgt ctggctgctg tgagcgtggt gtaaagtccc ctgccttcct gggctctttg 1560
   gggatccctg tgcaggagac tgcaggtctc ccgttcagag tctcacttag cttggagaga 1620
   aggcatctgg cctttgggga acttggtcag gaagctgtgt ctagttcctt ttgtcccttt 1680
   ttttccttgc gttcattttg ctgtacttct gtcccgaagt aaatgtcaga atcagatatg 1740
   ttctgtatgt tggggtgtct gcagcgagat gtttgcagct gggcaagtgt taagaggagg 1800
   gggtgggagg gacagagctg gatcccagga tctttcacta aagggacatt tgccagtgga 1860
   gggggtcacc aaactgttgg agactcctcc ccttccctgc ctcccccccc gcccaacaat 1920
   ggacccacat tgatgataca ggtcctgggt ggcaggacct gaaggaccct gctaggactc 1980
   ccctgagaaa tgcgcctgcc ctgtggatgc cacttgtagt gtggctggtt tacattgttc 2040
   tggttgcaga ttatgaaaaa ttattgactt gctgaaggcg gtgaaaggtg ggaggggacg 2100
   gtagggatgg aagagggaat ttgaatattt gaccaggtta atgtgaagaa cagagataaa 2160
   cccttacatt aacccctcgc tcggagcacc tagggctgca gattaaaatc ccatgggcct 2220
   gccagtcaaa ggcaaggccc caagctactc tctcacactg gcctcccaca gcaatgagac 2280
   agtggcttgg agggtgatgc atgctttatg tgttcctgag ccggtgtgca gcgggagcac 2340
   ccggggagaa ggaaagccca gaaatgggca aaagatgttg gttccctgga cggtgaaatt 2400
   tcccaaatag gccttttaaa tgtgggtctc agaggttaac caaacaaaga cagtctcggc 2460
   aggaccacac cggagaggat tttagcctga cttaacccac tgagaacaga gtcttgttca 2520
   attttatggg aatttcaaca agtaaccaat acaggcggcc ttcccctctt atttttgtat 2580
   tcccttggac cggctgtcaa tcacacacac acacacacac acacacacac acacacacac 2640
   acacccctcg gtgcactggc cccttccctt ccccgcctgc ctgctcctct cccagcctgc 2700
   cagctgccct ccctgctccc cttcttctcc tctcccggca gatccactca acttgaaccc 2760
   ggactctgag gcttagcagc cttggagaaa ccgtactcta ggaagaaaaa aaagcacaca 2820
   ggaaagagac tcctgaccct tcctcaaggc tgctctcgcc ccacctcata acactgggcg 2880
   caggcacaag ctcccaaaac ttcggaatct ggatacatta ctgtcttgga gggggtgaaa 2940
   actgcccagc aaagcaagcc ggcctctgtg cccctaccca gcggcctccc gcggccggcc 3000
   ttccctggga gcctcgcaga acccccgacc gagtctggga acgcccgggg gagacaatag 3060
   ctcgggcctg acgcccgaca gggaccctct gcccttcctt cctcgctgtc ttgctcccgt 3120
   ccctatagga tacccacccg gccctgttgc catctccctg gagcgccagc ggtggaggct 3180
   tcggaggagc cggccggatg caccgggacc ccgcggggcc tagttgggag cgcgccgccc 3240
   actgaggcct gggggcccgg cgaggcgcgg gggtggccaa ggcagtctcc ccggcacaca 3300
   gatcagcaca tgtctacgcg cacacaccca gcacgaccct ccctccccca gcacacccca 3360
   agggccgcgc cctccgctcc tgggcctggt ggccatggtc catgtgcccc tgccggcgca 3420
   ggggctgccc tggagttctc cattaagccc tgcgagcact tcaggaagga gctcgggctt 3480
   ctgtcatccc gctcagcaca tcaaagcgcc gacggccgcc ttcgtgggca ggtgtgagcg 3540
   ctcgccctct ctcctcccga gcctgctatt ttgtggtcca ggacttgcca tcagaacttg 3600
   ctttaacgcg acgagggaac tcacctgtgt gcagagcgcc gcgcacacat cggggctcgc 3660
   gggacagggc agctgcgtta gcgcagcgcc gggtgtgttc gggtaggtgt tgcgggcaag 3720
   gaagtaggca gcggcccctg agcagccgcc tcgctccggc attgcgggga cacggcgggg 3780
   ctgaggccac gagagcaggg cccgagcccg gcgggccgtg gttacggttt tcttgcactg 3840
   aaaaactgaa tccggcccga agcgacgtgc actttatggt ccccacacca ctcggttaac 3900
   taagaaaaga cccgggcgaa tggacctaac gcagcccggt gccgagaggg cccggtccag 3960
   cagcctctgg ggcccagtgc gcagggcact gcgggccgat tgcgccccgg ggccaggagg 4020
   cgccgagaaa gcaaaagcaa aagccggcgg cgggtggagg tcaagcggcc cggtgcaggc 4080
   cggggaccgg ggagccgccc cgggagagca gggtccaggc cttctctgtc cccctccagc 4140
   ctggggctac ctcagggact cctcgcgctg ccagatgctt cggaccaaag ggcatttctc 4200
   tgcgccctga agtcccggct tctctagagg aagtctaaga cgttcgggac ccacgaagat 4260
   gcctgcggag gggcaggaga agaggcgtgc ttttctgccc catcccttca tcttgcgatt 4320
   gggttgtttt ctatcgtccc ctttggcaca gggagtcctc tgcgctcgct gctgtgaccg 4380
   gcccagttag ttgggaaaca aggctcagaa agaaggccgc agtggtgttt ctggggtagg 4440
   gtgcttggcc ctccctgaag ccagggctca gggcagcaca ggccaggagc tctgccggga 4500
   gagacccgga accagagtct gggtctagtt gtccggtacc tgtgctcatt ctgagattaa 4560
   agtgcacgcc ccagcctcag agaataaaat tggaattcca ggacataaga atccccctcg 4620
   agggggaact tgctctgtgt ttgtgcgagc atcaaaacaa aacataatga ttccaaagca 4680
   attctatgac gtgcagttgg tgtgtttgag ctacttctaa agctgggctt tctctaccag 4740
   ctgtctagca atttactaaa aggcacagca gcaccttttg tactctgagg caaaataaga 4800
   caccctgcca tccactggat gactttgttt ctgaagaggt ttttttttct ttcttctttc 4860
   agtgggactg ccccgttacc agcatcactc tactccagtg tgtgacagaa aagatttcgt 4920
   cctcaacttc aatgggattt cttctttcca tccctcagct agcgggtcgt attatcacca 4980
   tcaccaccag agcgtctgtc aggatattaa gccctgcgtc atgtgaacgg aaagaggcca 5040
   agcgatggcc gctctctcct ctcccctcct cagagggggc agatagaaac tgggacggat 5100
   tcaagtcaca tgcacgcgga tagcagtaag ccacacacct gccacttagc cagaatgccc 5160
   aggatcgcgt tggtcactgt tatttgccta ctgctggaag aaggacaacc gctggcaagg 5220
   tagcgttccc caatctgaat acctgcaggc tcccacatga gggagagggc agactcaggt 5280
   gggaagatgt gccatgcgta aggcatcaac gtgtatctgt gggatcttcg ttgccttcag 5340
   taatcagggt gtgaaaaaag cagacaagtt gtgtgtgtgt gtgtgtgtct aagaaaactt 5400
   gtgtgctttt caaaaaggca gtgctaagca caagatttca agaaagcctc ttcttgttgc 5460
   ctagctgagt gggagagtca ttttccccag acactacatt tggatacagg tgccaaagaa 5520
   cattattaag gaattattta gaaacaatgt gtctagttta agaaagtggt tttcagtatt 5580
   gtgacaatac aacgttttta caaggttgtt ttctaccacc atattttaaa gatattttta 5640
   tgaccgtgta tactcacact ttgcttgtat tttaaaagga ggatatattt gcacttatgt 5700
   atacttttac agtttgccaa aatattttgt tgtaaaattt tttttcaata aaatgtatat 5760
   aaca 5764
<210> 32
   <211> 5764
   <212> DNA
   <213> Artificial Sequence
<220>
   <223> C to T (bis1)
<400> 32
   agttcgggcg gcgggttagg gcgttcgtag ggtttttggg tcgatttcgt ttcggcgttt 60
   tcgtttttcg ttcggggttc gttttcgcgg ttcggtttcg ttttcgggtt ttagatgatt 120
   atcgagggcg ggtcgtcgtc ggtttcgttt cgtcgcgcgt gtagttcggt tttcggcgcg 180
   ttttaggtcg ttttgatgag ttcgtcgttc gtcgtcgtcg tcgtcgtcgt cgtcgtttcg 240
   gagattattt tttttttttc gtcgtcgttt ttcgtttttt gcgtttcgtt ttcgtttttt 300
   tttaattcgg ttagcgtttt ttcggttgtt tgtaagagcg cgggcggcgg cggcgcgggc 360
   gtcgggagcg ggggcgttaa gaaggcgagt tcggggttgc ggcggttcga gaagtcgttt 420
   tattcgtata tcgcgtttat cgttatggtt atttagagtt cgtttagtaa gcgtttgacg 480
   tttagcgaga tttattagtt tttgtaggcg cgtttttttt tttttcgcgg cgtttattag 540
   ggttggaaga attcggtgcg ttataatttt tcgtttaacg agtgttttat taagttgttt 600
   aagggtttcg ggcggttcgg taagggttat tattggatta tcgattcggt tagcgagttt 660
   atgttcgagg agggttcgtt tcgtcgtcgg tcgcgcggtt ttaggcggaa gtgttaggcg 720
   tttaagttta tgtattatcg cgtggtgagc ggtttgggtt tcggggcgtc gttgttgttt 780
   tagggtttcg atttttaggc gttttcgtcg gcgtcgttcg gttgttatag ttagggcggt 840
   tacggcggtt tcgatatgat gttcgcgggt tacgacgtcg gcgcgggcgt ttttagttac 900
   gcgtattttt attattatta ttattattac gtttcgtata tgtcgtttaa ttcgggtttt 960
   atttatatgg ttagttgttc ggtgttcgcg ggattcgggg gcgtcggtgc ggtcgggggc 1020
   ggcggcggcg gcgattacgg gtcggatagt agtagtagtt cggtattttc gttttcggtt 1080
   atggcgagcg ttatcgaatg ttattcgttt tatacgagtt ttgcggcgta ttggagttcg 1140
   tttggcgttt cgttttattt taagtagtcg tttgttttga cgtttagtag taatttcgtc 1200
   gtttcggtag gtttgtattt tagtatgttt ttttattcgt tggagtagag ttatttgtat 1260
   tagaacgttc gcgaggattt tttaggtaac gtagtacgtt ttagtttagt tagttgggcg 1320
   tatcgttttt aggtttttag ggttggcggt tattgttatt tttataaata gggatttcga 1380
   agttaggagg tttgagggta ttgggaaaag tagatgttgg ggaaagtcgg tattttattc 1440
   gtgggagtag ggattagggt tttattgttt ttttagtatt tttggttagt agtcgaagtt 1500
   ttagaattgt ttggttgttg tgagcgtggt gtaaagtttt ttgttttttt gggttttttg 1560
   gggatttttg tgtaggagat tgtaggtttt tcgtttagag ttttatttag tttggagaga 1620
   aggtatttgg tttttgggga atttggttag gaagttgtgt ttagtttttt ttgttttttt 1680
   ttttttttgc gtttattttg ttgtattttt gtttcgaagt aaatgttaga attagatatg 1740
   ttttgtatgt tggggtgttt gtagcgagat gtttgtagtt gggtaagtgt taagaggagg 1800
   gggtgggagg gatagagttg gattttagga ttttttatta aagggatatt tgttagtgga 1860
   gggggttatt aaattgttgg agattttttt ttttttttgt tttttttttc gtttaataat 1920
   ggatttatat tgatgatata ggttttgggt ggtaggattt gaaggatttt gttaggattt 1980
   ttttgagaaa tgcgtttgtt ttgtggatgt tatttgtagt gtggttggtt tatattgttt 2040
   tggttgtaga ttatgaaaaa ttattgattt gttgaaggcg gtgaaaggtg ggaggggacg 2100
   gtagggatgg aagagggaat ttgaatattt gattaggtta atgtgaagaa tagagataaa 2160
   tttttatatt aatttttcgt tcggagtatt tagggttgta gattaaaatt ttatgggttt 2220
   gttagttaaa ggtaaggttt taagttattt ttttatattg gttttttata gtaatgagat 2280
   agtggtttgg agggtgatgt atgttttatg tgtttttgag tcggtgtgta gcgggagtat 2340
   tcggggagaa ggaaagttta gaaatgggta aaagatgttg gttttttgga cggtgaaatt 2400
   ttttaaatag gttttttaaa tgtgggtttt agaggttaat taaataaaga tagtttcggt 2460
   aggattatat cggagaggat tttagtttga tttaatttat tgagaataga gttttgttta 2520
   attttatggg aattttaata agtaattaat ataggcggtt tttttttttt atttttgtat 2580
   ttttttggat cggttgttaa ttatatatat atatatatat atatatatat atatatatat 2640
   atatttttcg gtgtattggt tttttttttt tttcgtttgt ttgttttttt tttagtttgt 2700
   tagttgtttt ttttgttttt tttttttttt tttttcggta gatttattta atttgaattc 2760
   ggattttgag gtttagtagt tttggagaaa tcgtatttta ggaagaaaaa aaagtatata 2820
   ggaaagagat ttttgatttt tttttaaggt tgttttcgtt ttattttata atattgggcg 2880
   taggtataag tttttaaaat ttcggaattt ggatatatta ttgttttgga gggggtgaaa 2940
   attgtttagt aaagtaagtc ggtttttgtg tttttattta gcggtttttc gcggtcggtt 3000
   ttttttggga gtttcgtaga attttcgatc gagtttggga acgttcgggg gagataatag 3060
   ttcgggtttg acgttcgata gggatttttt gttttttttt tttcgttgtt ttgttttcgt 3120
   ttttatagga tatttattcg gttttgttgt tatttttttg gagcgttagc ggtggaggtt 3180
   tcggaggagt cggtcggatg tatcgggatt tcgcggggtt tagttgggag cgcgtcgttt 3240
   attgaggttt gggggttcgg cgaggcgcgg gggtggttaa ggtagttttt tcggtatata 3300
   gattagtata tgtttacgcg tatatattta gtacgatttt ttttttttta gtatatttta 3360
   agggtcgcgt ttttcgtttt tgggtttggt ggttatggtt tatgtgtttt tgtcggcgta 3420
   ggggttgttt tggagttttt tattaagttt tgcgagtatt ttaggaagga gttcgggttt 3480
   ttgttatttc gtttagtata ttaaagcgtc gacggtcgtt ttcgtgggta ggtgtgagcg 3540
   ttcgtttttt tttttttcga gtttgttatt ttgtggttta ggatttgtta ttagaatttg 3600
   ttttaacgcg acgagggaat ttatttgtgt gtagagcgtc gcgtatatat cggggttcgc 3660
   gggatagggt agttgcgtta gcgtagcgtc gggtgtgttc gggtaggtgt tgcgggtaag 3720
   gaagtaggta gcggtttttg agtagtcgtt tcgtttcggt attgcgggga tacggcgggg 3780
   ttgaggttac gagagtaggg ttcgagttcg gcgggtcgtg gttacggttt ttttgtattg 3840
   aaaaattgaa ttcggttcga agcgacgtgt attttatggt ttttatatta ttcggttaat 3900
   taagaaaaga ttcgggcgaa tggatttaac gtagttcggt gtcgagaggg ttcggtttag 3960
   tagtttttgg ggtttagtgc gtagggtatt gcgggtcgat tgcgtttcgg ggttaggagg 4020
   cgtcgagaaa gtaaaagtaa aagtcggcgg cgggtggagg ttaagcggtt cggtgtaggt 4080
   cggggatcgg ggagtcgttt cgggagagta gggtttaggt tttttttgtt tttttttagt 4140
   ttggggttat tttagggatt tttcgcgttg ttagatgttt cggattaaag ggtatttttt 4200
   tgcgttttga agtttcggtt tttttagagg aagtttaaga cgttcgggat ttacgaagat 4260
   gtttgcggag gggtaggaga agaggcgtgt ttttttgttt tattttttta ttttgcgatt 4320
   gggttgtttt ttatcgtttt ttttggtata gggagttttt tgcgttcgtt gttgtgatcg 4380
   gtttagttag ttgggaaata aggtttagaa agaaggtcgt agtggtgttt ttggggtagg 4440
   gtgtttggtt ttttttgaag ttagggttta gggtagtata ggttaggagt tttgtcggga 4500
   gagattcgga attagagttt gggtttagtt gttcggtatt tgtgtttatt ttgagattaa 4560
   agtgtacgtt ttagttttag agaataaaat tggaatttta ggatataaga atttttttcg 4620
   agggggaatt tgttttgtgt ttgtgcgagt attaaaataa aatataatga ttttaaagta 4680
   attttatgac gtgtagttgg tgtgtttgag ttatttttaa agttgggttt tttttattag 4740
   ttgtttagta atttattaaa aggtatagta gtattttttg tattttgagg taaaataaga 4800
   tattttgtta tttattggat gattttgttt ttgaagaggt tttttttttt tttttttttt 4860
   agtgggattg tttcgttatt agtattattt tattttagtg tgtgatagaa aagatttcgt 4920
   ttttaatttt aatgggattt ttttttttta ttttttagtt agcgggtcgt attattatta 4980
   ttattattag agcgtttgtt aggatattaa gttttgcgtt atgtgaacgg aaagaggtta 5040
   agcgatggtc gttttttttt tttttttttt tagagggggt agatagaaat tgggacggat 5100
   ttaagttata tgtacgcgga tagtagtaag ttatatattt gttatttagt tagaatgttt 5160
   aggatcgcgt tggttattgt tatttgttta ttgttggaag aaggataatc gttggtaagg 5220
   tagcgttttt taatttgaat atttgtaggt ttttatatga gggagagggt agatttaggt 5280
   gggaagatgt gttatgcgta aggtattaac gtgtatttgt gggattttcg ttgtttttag 5340
   taattagggt gtgaaaaaag tagataagtt gtgtgtgtgt gtgtgtgttt aagaaaattt 5400
   gtgtgttttt taaaaaggta gtgttaagta taagatttta agaaagtttt tttttgttgt 5460
   ttagttgagt gggagagtta ttttttttag atattatatt tggatatagg tgttaaagaa 5520
   tattattaag gaattattta gaaataatgt gtttagttta agaaagtggt ttttagtatt 5580
   gtgataatat aacgttttta taaggttgtt ttttattatt atattttaaa gatattttta 5640
   tgatcgtgta tatttatatt ttgtttgtat tttaaaagga ggatatattt gtatttatgt 5700
   atatttttat agtttgttaa aatattttgt tgtaaaattt ttttttaata aaatgtatat 5760
   aata 5764
<210> 33
   <211> 5764
   <212> DNA
   <213> Artificial Sequence
<220>
   <223> rc C to T (bis1)
<400> 33
   tattatatac attttattaa aaaaaaattt tacaacaaaa tattttaaca aactataaaa 60
   atatacataa atacaaatat atcctccttt taaaatacaa acaaaatata aatatacacg 120
   atcataaaaa tatctttaaa atataataat aaaaaacaac cttataaaaa cgttatatta 180
   tcacaatact aaaaaccact ttcttaaact aaacacatta tttctaaata attccttaat 240
   aatattcttt aacacctata tccaaatata atatctaaaa aaaataactc tcccactcaa 300
   ctaaacaaca aaaaaaaact ttcttaaaat cttatactta acactacctt tttaaaaaac 360
   acacaaattt tcttaaacac acacacacac acacaactta tctacttttt tcacacccta 420
   attactaaaa acaacgaaaa tcccacaaat acacgttaat accttacgca taacacatct 480
   tcccacctaa atctaccctc tccctcatat aaaaacctac aaatattcaa attaaaaaac 540
   gctaccttac caacgattat ccttcttcca acaataaaca aataacaata accaacgcga 600
   tcctaaacat tctaactaaa taacaaatat ataacttact actatccgcg tacatataac 660
   ttaaatccgt cccaatttct atctaccccc tctaaaaaaa aaaaaaaaaa aaacgaccat 720
   cgcttaacct ctttccgttc acataacgca aaacttaata tcctaacaaa cgctctaata 780
   ataataataa taatacgacc cgctaactaa aaaataaaaa aaaaaaatcc cattaaaatt 840
   aaaaacgaaa tcttttctat cacacactaa aataaaataa tactaataac gaaacaatcc 900
   cactaaaaaa aaaaaaaaaa aaaaacctct tcaaaaacaa aatcatccaa taaataacaa 960
   aatatcttat tttacctcaa aatacaaaaa atactactat accttttaat aaattactaa 1020
   acaactaata aaaaaaaccc aactttaaaa ataactcaaa cacaccaact acacgtcata 1080
   aaattacttt aaaatcatta tattttattt taatactcgc acaaacacaa aacaaattcc 1140
   ccctcgaaaa aaattcttat atcctaaaat tccaatttta ttctctaaaa ctaaaacgta 1200
   cactttaatc tcaaaataaa cacaaatacc gaacaactaa acccaaactc taattccgaa 1260
   tctctcccga caaaactcct aacctatact accctaaacc ctaacttcaa aaaaaaccaa 1320
   acaccctacc ccaaaaacac cactacgacc ttctttctaa accttatttc ccaactaact 1380
   aaaccgatca caacaacgaa cgcaaaaaac tccctatacc aaaaaaaacg ataaaaaaca 1440
   acccaatcgc aaaataaaaa aataaaacaa aaaaacacgc ctcttctcct acccctccgc 1500
   aaacatcttc gtaaatcccg aacgtcttaa acttcctcta aaaaaaccga aacttcaaaa 1560
   cgcaaaaaaa taccctttaa tccgaaacat ctaacaacgc gaaaaatccc taaaataacc 1620
   ccaaactaaa aaaaaacaaa aaaaacctaa accctactct cccgaaacga ctccccgatc 1680
   cccgacctac accgaaccgc ttaacctcca cccgccgccg acttttactt ttactttctc 1740
   gacgcctcct aaccccgaaa cgcaatcgac ccgcaatacc ctacgcacta aaccccaaaa 1800
   actactaaac cgaaccctct cgacaccgaa ctacgttaaa tccattcgcc cgaatctttt 1860
   cttaattaac cgaataatat aaaaaccata aaatacacgt cgcttcgaac cgaattcaat 1920
   ttttcaatac aaaaaaaccg taaccacgac ccgccgaact cgaaccctac tctcgtaacc 1980
   tcaaccccgc cgtatccccg caataccgaa acgaaacgac tactcaaaaa ccgctaccta 2040
   cttccttacc cgcaacacct acccgaacac acccgacgct acgctaacgc aactacccta 2100
   tcccgcgaac cccgatatat acgcgacgct ctacacacaa ataaattccc tcgtcgcgtt 2160
   aaaacaaatt ctaataacaa atcctaaacc acaaaataac aaactcgaaa aaaaaaaaaa 2220
   cgaacgctca cacctaccca cgaaaacgac cgtcgacgct ttaatatact aaacgaaata 2280
   acaaaaaccc gaactccttc ctaaaatact cgcaaaactt aataaaaaac tccaaaacaa 2340
   cccctacgcc gacaaaaaca cataaaccat aaccaccaaa cccaaaaacg aaaaacgcga 2400
   cccttaaaat atactaaaaa aaaaaaaatc gtactaaata tatacgcgta aacatatact 2460
   aatctatata ccgaaaaaac taccttaacc acccccgcgc ctcgccgaac ccccaaacct 2520
   caataaacga cgcgctccca actaaacccc gcgaaatccc gatacatccg accgactcct 2580
   ccgaaacctc caccgctaac gctccaaaaa aataacaaca aaaccgaata aatatcctat 2640
   aaaaacgaaa acaaaacaac gaaaaaaaaa aaacaaaaaa tccctatcga acgtcaaacc 2700
   cgaactatta tctcccccga acgttcccaa actcgatcga aaattctacg aaactcccaa 2760
   aaaaaaccga ccgcgaaaaa ccgctaaata aaaacacaaa aaccgactta ctttactaaa 2820
   caattttcac cccctccaaa acaataatat atccaaattc cgaaatttta aaaacttata 2880
   cctacgccca atattataaa ataaaacgaa aacaacctta aaaaaaaatc aaaaatctct 2940
   ttcctatata cttttttttc ttcctaaaat acgatttctc caaaactact aaacctcaaa 3000
   atccgaattc aaattaaata aatctaccga aaaaaaaaaa aaaaaaaaac aaaaaaaaca 3060
   actaacaaac taaaaaaaaa acaaacaaac gaaaaaaaaa aaaaaccaat acaccgaaaa 3120
   atatatatat atatatatat atatatatat atatatatat ataattaaca accgatccaa 3180
   aaaaatacaa aaataaaaaa aaaaaaccgc ctatattaat tacttattaa aattcccata 3240
   aaattaaaca aaactctatt ctcaataaat taaatcaaac taaaatcctc tccgatataa 3300
   tcctaccgaa actatcttta tttaattaac ctctaaaacc cacatttaaa aaacctattt 3360
   aaaaaatttc accgtccaaa aaaccaacat cttttaccca tttctaaact ttccttctcc 3420
   ccgaatactc ccgctacaca ccgactcaaa aacacataaa acatacatca ccctccaaac 3480
   cactatctca ttactataaa aaaccaatat aaaaaaataa cttaaaacct tacctttaac 3540
   taacaaaccc ataaaatttt aatctacaac cctaaatact ccgaacgaaa aattaatata 3600
   aaaatttatc tctattcttc acattaacct aatcaaatat tcaaattccc tcttccatcc 3660
   ctaccgtccc ctcccacctt tcaccgcctt caacaaatca ataatttttc ataatctaca 3720
   accaaaacaa tataaaccaa ccacactaca aataacatcc acaaaacaaa cgcatttctc 3780
   aaaaaaatcc taacaaaatc cttcaaatcc taccacccaa aacctatatc atcaatataa 3840
   atccattatt aaacgaaaaa aaaaacaaaa aaaaaaaaaa atctccaaca atttaataac 3900
   cccctccact aacaaatatc cctttaataa aaaatcctaa aatccaactc tatccctccc 3960
   accccctcct cttaacactt acccaactac aaacatctcg ctacaaacac cccaacatac 4020
   aaaacatatc taattctaac atttacttcg aaacaaaaat acaacaaaat aaacgcaaaa 4080
   aaaaaaaaaa acaaaaaaaa ctaaacacaa cttcctaacc aaattcccca aaaaccaaat 4140
   accttctctc caaactaaat aaaactctaa acgaaaaacc tacaatctcc tacacaaaaa 4200
   tccccaaaaa acccaaaaaa acaaaaaact ttacaccacg ctcacaacaa ccaaacaatt 4260
   ctaaaacttc gactactaac caaaaatact aaaaaaacaa taaaacccta atccctactc 4320
   ccacgaataa aataccgact ttccccaaca tctacttttc ccaataccct caaacctcct 4380
   aacttcgaaa tccctattta taaaaataac aataaccgcc aaccctaaaa acctaaaaac 4440
   gatacgccca actaactaaa ctaaaacgta ctacgttacc taaaaaatcc tcgcgaacgt 4500
   tctaatacaa ataactctac tccaacgaat aaaaaaacat actaaaatac aaacctaccg 4560
   aaacgacgaa attactacta aacgtcaaaa caaacgacta cttaaaataa aacgaaacgc 4620
   caaacgaact ccaatacgcc gcaaaactcg tataaaacga ataacattcg ataacgctcg 4680
   ccataaccga aaacgaaaat accgaactac tactactatc cgacccgtaa tcgccgccgc 4740
   cgccgccccc gaccgcaccg acgcccccga atcccgcgaa caccgaacaa ctaaccatat 4800
   aaataaaacc cgaattaaac gacatatacg aaacgtaata ataataataa taataaaaat 4860
   acgcgtaact aaaaacgccc gcgccgacgt cgtaacccgc gaacatcata tcgaaaccgc 4920
   cgtaaccgcc ctaactataa caaccgaacg acgccgacga aaacgcctaa aaatcgaaac 4980
   cctaaaacaa caacgacgcc ccgaaaccca aaccgctcac cacgcgataa tacataaact 5040
   taaacgccta acacttccgc ctaaaaccgc gcgaccgacg acgaaacgaa ccctcctcga 5100
   acataaactc gctaaccgaa tcgataatcc aataataacc cttaccgaac cgcccgaaac 5160
   ccttaaacaa cttaataaaa cactcgttaa acgaaaaatt ataacgcacc gaattcttcc 5220
   aaccctaata aacgccgcga aaaaaaaaaa aacgcgccta caaaaactaa taaatctcgc     5280
   taaacgtcaa acgcttacta aacgaactct aaataaccat aacgataaac gcgatatacg     5340
   aataaaacga cttctcgaac cgccgcaacc ccgaactcgc cttcttaacg cccccgctcc     5400
   cgacgcccgc gccgccgccg cccgcgctct tacaaacaac cgaaaaaacg ctaaccgaat     5460
   taaaaaaaaa cgaaaacgaa acgcaaaaaa cgaaaaacga cgacgaaaaa aaaaaaataa     5520
   tctccgaaac gacgacgacg acgacgacga cgacgaacga cgaactcatc aaaacgacct     5580
   aaaacgcgcc gaaaaccgaa ctacacgcgc gacgaaacga aaccgacgac gacccgccct     5640
   cgataatcat ctaaaacccg aaaacgaaac cgaaccgcga aaacgaaccc cgaacgaaaa     5700
   acgaaaacgc cgaaacgaaa tcgacccaaa aaccctacga acgccctaac ccgccgcccg     5760
   aact                                                                  5764
<210> 34
   <211> 5764
   <212> DNA
   <213> Artificial Sequence
<220>
   <223> G to A (bis2 rc)
<400> 34
   aacccgaacg acgaactaaa acgctcgcaa aacttctaaa ccgaccccgc tccgacgcct       60
   ccgcttcccg cccgaaaccc gccctcgcga cccgacctcg ctcccgaatc ccaaataacc      120
   accgaaaacg aaccgccgcc gaccccgctc cgccgcgcgt acaacccgat ccccgacgcg      180
   ctccaaaccg ccctaataaa cccgccgccc gccgccgccg ccgccgccgc cgccgccccg      240
   aaaaccacct cctcctcctc gtcgtcgtcc tccgcctcct acgcctcgtc ctcgtcctcc      300
   tccaattcga ccaacgcccc ctcgactacc tacaaaaacg cgaacgacga cgacgcgaac      360
   gccgaaaacg aaaacgccaa aaaaacgaac tcgaaactac gacgacccga aaaaccgccc      420
   tactcgtaca tcgcgctcat cgtcataacc atccaaaact cgcccaacaa acgcctaacg      480
   ctcaacgaaa tctaccaatt cctacaaacg cgcttcccct tcttccgcga cgcctaccaa      540
   aactaaaaaa actcgatacg ccacaatctc tcgctcaacg aatacttcat caaactacct      600
   aaaaacctcg aacgacccga caaaaaccac tactaaacca tcgacccgac caacgaattc      660
   atattcgaaa aaaactcgtt ccgccgccga ccgcgcgact tcaaacgaaa ataccaaacg      720
   ctcaaaccca tataccaccg cgtaataaac gacttaaact tcgaaacgtc gctactaccc      780
   caaaacttcg acttccaaac gcccccgtcg acgccgctcg actaccacaa ccaaaacgac      840
   tacgacgacc tcgacataat acccgcgaac tacgacgccg acgcgaacgc ccccaaccac      900
   gcgcaccctc accaccacca ccaccaccac gtcccgcaca tatcgcccaa cccgaattcc      960
   acctacataa ccaactaccc gatacccgcg aaacccgaaa acgtcgatac gaccgaaaac     1020
   gacgacgacg acgactacga accgaacaac aacaacaacc cgataccctc gtccccgacc 1080
   ataacgaacg ccatcgaata ccactcgccc tacacgaacc ctacgacgca ctaaaactcg 1140
   cctaacgcct cgccttacct caaacaaccg cctaccctaa cgcccaacaa caaccccgcc 1200
   gcctcgacaa acctacactc caacatatcc tcctactcgc taaaacaaaa ctacttacac 1260
   caaaacgctc gcgaaaacct ctcaaataac gcaacacgct ccaacccaac caactaaacg 1320
   caccgcttct aaacctccaa aactaacgac cactaccact cccacaaaca aaaaccccga 1380
   aactaaaaaa tctaaaaaca ctaaaaaaaa caaatactaa aaaaaaccga catcttattc 1440
   gtaaaaacaa aaatcaaaat cttactatcc tcccaatatc cctaaccaac aaccgaaact 1500
   tcaaaattat ctaactacta taaacgtaat ataaaatccc ctaccttcct aaactcttta 1560
   aaaatcccta tacaaaaaac tacaaatctc ccgttcaaaa tctcacttaa cttaaaaaaa 1620
   aaacatctaa cctttaaaaa acttaatcaa aaaactatat ctaattcctt ttatcccttt 1680
   ttttccttac gttcatttta ctatacttct atcccgaaat aaatatcaaa atcaaatata 1740
   ttctatatat taaaatatct acaacgaaat atttacaact aaacaaatat taaaaaaaaa 1800
   aaataaaaaa aacaaaacta aatcccaaaa tctttcacta aaaaaacatt taccaataaa 1860
   aaaaatcacc aaactattaa aaactcctcc ccttccctac ctcccccccc gcccaacaat 1920
   aaacccacat taataataca aatcctaaat aacaaaacct aaaaaaccct actaaaactc 1980
   ccctaaaaaa tacgcctacc ctataaatac cacttataat ataactaatt tacattattc 2040
   taattacaaa ttataaaaaa ttattaactt actaaaaacg ataaaaaata aaaaaaaacg 2100
   ataaaaataa aaaaaaaaat ttaaatattt aaccaaatta atataaaaaa caaaaataaa 2160
   cccttacatt aacccctcgc tcgaaacacc taaaactaca aattaaaatc ccataaacct 2220
   accaatcaaa aacaaaaccc caaactactc tctcacacta acctcccaca acaataaaac 2280
   aataacttaa aaaataatac atactttata tattcctaaa ccgatataca acgaaaacac 2340
   ccgaaaaaaa aaaaaaccca aaaataaaca aaaaatatta attccctaaa cgataaaatt 2400
   tcccaaataa accttttaaa tataaatctc aaaaattaac caaacaaaaa caatctcgac 2460
   aaaaccacac cgaaaaaaat tttaacctaa cttaacccac taaaaacaaa atcttattca 2520
   attttataaa aatttcaaca aataaccaat acaaacgacc ttcccctctt atttttatat 2580
   tcccttaaac cgactatcaa tcacacacac acacacacac acacacacac acacacacac 2640
   acacccctcg atacactaac cccttccctt ccccgcctac ctactcctct cccaacctac 2700
   caactaccct ccctactccc cttcttctcc tctcccgaca aatccactca acttaaaccc 2760
   gaactctaaa acttaacaac cttaaaaaaa ccgtactcta aaaaaaaaaa aaaacacaca 2820
   aaaaaaaaac tcctaaccct tcctcaaaac tactctcgcc ccacctcata acactaaacg 2880
   caaacacaaa ctcccaaaac ttcgaaatct aaatacatta ctatcttaaa aaaaataaaa 2940
   actacccaac aaaacaaacc gacctctata cccctaccca acgacctccc gcgaccgacc 3000
   ttccctaaaa acctcgcaaa acccccgacc gaatctaaaa acgcccgaaa aaaacaataa 3060
   ctcgaaccta acgcccgaca aaaaccctct acccttcctt cctcgctatc ttactcccgt 3120
   ccctataaaa tacccacccg accctattac catctcccta aaacgccaac gataaaaact 3180
   tcgaaaaaac cgaccgaata caccgaaacc ccgcgaaacc taattaaaaa cgcgccgccc 3240
   actaaaacct aaaaacccga cgaaacgcga aaataaccaa aacaatctcc ccgacacaca 3300
   aatcaacaca tatctacgcg cacacaccca acacgaccct ccctccccca acacacccca 3360
   aaaaccgcgc cctccgctcc taaacctaat aaccataatc catatacccc taccgacgca 3420
   aaaactaccc taaaattctc cattaaaccc tacgaacact tcaaaaaaaa actcgaactt 3480
   ctatcatccc gctcaacaca tcaaaacgcc gacgaccgcc ttcgtaaaca aatataaacg 3540
   ctcgccctct ctcctcccga acctactatt ttataatcca aaacttacca tcaaaactta 3600
   ctttaacgcg acgaaaaaac tcacctatat acaaaacgcc gcgcacacat cgaaactcgc 3660
   gaaacaaaac aactacgtta acgcaacgcc gaatatattc gaataaatat tacgaacaaa 3720
   aaaataaaca acgaccccta aacaaccgcc tcgctccgac attacgaaaa cacgacgaaa 3780
   ctaaaaccac gaaaacaaaa cccgaacccg acgaaccgta attacgattt tcttacacta 3840
   aaaaactaaa tccgacccga aacgacgtac actttataat ccccacacca ctcgattaac 3900
   taaaaaaaaa cccgaacgaa taaacctaac gcaacccgat accgaaaaaa cccgatccaa 3960
   caacctctaa aacccaatac gcaaaacact acgaaccgat tacgccccga aaccaaaaaa 4020
   cgccgaaaaa acaaaaacaa aaaccgacga cgaataaaaa tcaaacgacc cgatacaaac 4080
   cgaaaaccga aaaaccgccc cgaaaaaaca aaatccaaac cttctctatc cccctccaac 4140
   ctaaaactac ctcaaaaact cctcgcgcta ccaaatactt cgaaccaaaa aacatttctc 4200
   tacgccctaa aatcccgact tctctaaaaa aaatctaaaa cgttcgaaac ccacgaaaat 4260
   acctacgaaa aaacaaaaaa aaaaacgtac ttttctaccc catcccttca tcttacgatt 4320
   aaattatttt ctatcgtccc ctttaacaca aaaaatcctc tacgctcgct actataaccg 4380
   acccaattaa ttaaaaaaca aaactcaaaa aaaaaaccgc aataatattt ctaaaataaa 4440
   atacttaacc ctccctaaaa ccaaaactca aaacaacaca aaccaaaaac tctaccgaaa 4500
   aaaacccgaa accaaaatct aaatctaatt atccgatacc tatactcatt ctaaaattaa 4560
   aatacacgcc ccaacctcaa aaaataaaat taaaattcca aaacataaaa atccccctcg 4620
   aaaaaaaact tactctatat ttatacgaac atcaaaacaa aacataataa ttccaaaaca 4680
   attctataac gtacaattaa tatatttaaa ctacttctaa aactaaactt tctctaccaa 4740
   ctatctaaca atttactaaa aaacacaaca acacctttta tactctaaaa caaaataaaa 4800
   caccctacca tccactaaat aactttattt ctaaaaaaat ttttttttct ttcttctttc 4860
   aataaaacta ccccgttacc aacatcactc tactccaata tataacaaaa aaaatttcgt 4920
   cctcaacttc aataaaattt cttctttcca tccctcaact aacgaatcgt attatcacca 4980
   tcaccaccaa aacgtctatc aaaatattaa accctacgtc atataaacga aaaaaaacca 5040
   aacgataacc gctctctcct ctcccctcct caaaaaaaac aaataaaaac taaaacgaat 5100
   tcaaatcaca tacacgcgaa taacaataaa ccacacacct accacttaac caaaataccc 5160
   aaaatcgcgt taatcactat tatttaccta ctactaaaaa aaaaacaacc gctaacaaaa 5220
   taacgttccc caatctaaat acctacaaac tcccacataa aaaaaaaaac aaactcaaat 5280
   aaaaaaatat accatacgta aaacatcaac gtatatctat aaaatcttcg ttaccttcaa 5340
   taatcaaaat ataaaaaaaa caaacaaatt atatatatat atatatatct aaaaaaactt 5400
   atatactttt caaaaaaaca atactaaaca caaaatttca aaaaaacctc ttcttattac 5460
   ctaactaaat aaaaaaatca ttttccccaa acactacatt taaatacaaa taccaaaaaa 5520
   cattattaaa aaattattta aaaacaatat atctaattta aaaaaataat tttcaatatt 5580
   ataacaatac aacgttttta caaaattatt ttctaccacc atattttaaa aatattttta 5640
   taaccgtata tactcacact ttacttatat tttaaaaaaa aaatatattt acacttatat 5700
   atacttttac aatttaccaa aatattttat tataaaattt tttttcaata aaatatatat 5760
   aaca 5764
<210> 35
   <211> 5764
   <212> DNA
   <213> Artificial Sequence
<220>
   <223> G to A (bis2 rc) rc
<400> 35
   tgttatatat attttattga aaaaaaattt tataataaaa tattttggta aattgtaaaa        60
   gtatatataa gtgtaaatat attttttttt taaaatataa gtaaagtgtg agtatatacg       120
   gttataaaaa tatttttaaa atatggtggt agaaaataat tttgtaaaaa cgttgtattg       180
   ttataatatt gaaaattatt tttttaaatt agatatattg tttttaaata attttttaat      240
   aatgtttttt ggtatttgta tttaaatgta gtgtttgggg aaaatgattt ttttatttag       300
   ttaggtaata agaagaggtt tttttgaaat tttgtgttta gtattgtttt tttgaaaagt       360
   atataagttt ttttagatat atatatatat atataatttg tttgtttttt ttatattttg       420
   attattgaag gtaacgaaga ttttatagat atacgttgat gttttacgta tggtatattt       480
   ttttatttga gtttgttttt ttttttatgt gggagtttgt aggtatttag attggggaac        540
   gttattttgt tagcggttgt ttttttttta gtagtaggta aataatagtg attaacgcga 600
   ttttgggtat tttggttaag tggtaggtgt gtggtttatt gttattcgcg tgtatgtgat 660
   ttgaattcgt tttagttttt atttgttttt tttgaggagg ggagaggaga gagcggttat 720
   cgtttggttt tttttcgttt atatgacgta gggtttaata ttttgataga cgttttggtg 780
   gtgatggtga taatacgatt cgttagttga gggatggaaa gaagaaattt tattgaagtt 840
   gaggacgaaa ttttttttgt tatatattgg agtagagtga tgttggtaac ggggtagttt 900
   tattgaaaga agaaagaaaa aaaaattttt ttagaaataa agttatttag tggatggtag 960
   ggtgttttat tttgttttag agtataaaag gtgttgttgt gttttttagt aaattgttag 1020
   atagttggta gagaaagttt agttttagaa gtagtttaaa tatattaatt gtacgttata 1080
   gaattgtttt ggaattatta tgttttgttt tgatgttcgt ataaatatag agtaagtttt 1140
   ttttcgaggg ggatttttat gttttggaat tttaatttta ttttttgagg ttggggcgtg 1200
   tattttaatt ttagaatgag tataggtatc ggataattag atttagattt tggtttcggg 1260
   tttttttcgg tagagttttt ggtttgtgtt gttttgagtt ttggttttag ggagggttaa 1320
   gtattttatt ttagaaatat tattgcggtt ttttttttga gttttgtttt ttaattaatt 1380
   gggtcggtta tagtagcgag cgtagaggat tttttgtgtt aaaggggacg atagaaaata 1440
   atttaatcgt aagatgaagg gatggggtag aaaagtacgt tttttttttt gttttttcgt 1500
   aggtattttc gtgggtttcg aacgttttag atttttttta gagaagtcgg gattttaggg 1560
   cgtagagaaa tgttttttgg ttcgaagtat ttggtagcgc gaggagtttt tgaggtagtt 1620
   ttaggttgga gggggataga gaaggtttgg attttgtttt ttcggggcgg tttttcggtt 1680
   ttcggtttgt atcgggtcgt ttgattttta ttcgtcgtcg gtttttgttt ttgttttttc 1740
   ggcgtttttt ggtttcgggg cgtaatcggt tcgtagtgtt ttgcgtattg ggttttagag 1800
   gttgttggat cgggtttttt cggtatcggg ttgcgttagg tttattcgtt cgggtttttt 1860
   tttagttaat cgagtggtgt ggggattata aagtgtacgt cgtttcgggt cggatttagt 1920
   tttttagtgt aagaaaatcg taattacggt tcgtcgggtt cgggttttgt tttcgtggtt 1980
   ttagtttcgt cgtgttttcg taatgtcgga gcgaggcggt tgtttagggg tcgttgttta 2040
   tttttttgtt cgtaatattt attcgaatat attcggcgtt gcgttaacgt agttgttttg 2100
   tttcgcgagt ttcgatgtgt gcgcggcgtt ttgtatatag gtgagttttt tcgtcgcgtt 2160
   aaagtaagtt ttgatggtaa gttttggatt ataaaatagt aggttcggga ggagagaggg 2220
   cgagcgttta tatttgttta cgaaggcggt cgtcggcgtt ttgatgtgtt gagcgggatg 2280
   atagaagttc gagttttttt ttgaagtgtt cgtagggttt aatggagaat tttagggtag 2340
   tttttgcgtc ggtaggggta tatggattat ggttattagg tttaggagcg gagggcgcgg 2400
   tttttggggt gtgttggggg agggagggtc gtgttgggtg tgtgcgcgta gatatgtgtt 2460
   gatttgtgtg tcggggagat tgttttggtt attttcgcgt ttcgtcgggt ttttaggttt 2520
   tagtgggcgg cgcgttttta attaggtttc gcggggtttc ggtgtattcg gtcggttttt 2580
   tcgaagtttt tatcgttggc gttttaggga gatggtaata gggtcgggtg ggtattttat 2640
   agggacggga gtaagatagc gaggaaggaa gggtagaggg tttttgtcgg gcgttaggtt 2700
   cgagttattg tttttttcgg gcgtttttag attcggtcgg gggttttgcg aggtttttag 2760
   ggaaggtcgg tcgcgggagg tcgttgggta ggggtataga ggtcggtttg ttttgttggg 2820
   tagtttttat tttttttaag atagtaatgt atttagattt cgaagttttg ggagtttgtg 2880
   tttgcgttta gtgttatgag gtggggcgag agtagttttg aggaagggtt aggagttttt 2940
   tttttgtgtg tttttttttt tttttagagt acggtttttt taaggttgtt aagttttaga 3000
   gttcgggttt aagttgagtg gatttgtcgg gagaggagaa gaaggggagt agggagggta 3060
   gttggtaggt tgggagagga gtaggtaggc ggggaaggga aggggttagt gtatcgaggg 3120
   gtgtgtgtgt gtgtgtgtgt gtgtgtgtgt gtgtgtgtgt gtgattgata gtcggtttaa 3180
   gggaatataa aaataagagg ggaaggtcgt ttgtattggt tatttgttga aatttttata 3240
   aaattgaata agattttgtt tttagtgggt taagttaggt taaaattttt ttcggtgtgg 3300
   ttttgtcgag attgtttttg tttggttaat ttttgagatt tatatttaaa aggtttattt 3360
   gggaaatttt atcgtttagg gaattaatat tttttgttta tttttgggtt tttttttttt 3420
   tcgggtgttt tcgttgtata tcggtttagg aatatataaa gtatgtatta ttttttaagt 3480
   tattgtttta ttgttgtggg aggttagtgt gagagagtag tttggggttt tgtttttgat 3540
   tggtaggttt atgggatttt aatttgtagt tttaggtgtt tcgagcgagg ggttaatgta 3600
   agggtttatt tttgtttttt atattaattt ggttaaatat ttaaattttt ttttttattt 3660
   ttatcgtttt tttttatttt ttatcgtttt tagtaagtta ataatttttt ataatttgta 3720
   attagaataa tgtaaattag ttatattata agtggtattt atagggtagg cgtatttttt 3780
   aggggagttt tagtagggtt ttttaggttt tgttatttag gatttgtatt attaatgtgg 3840
   gtttattgtt gggcgggggg ggaggtaggg aaggggagga gtttttaata gtttggtgat 3900
   tttttttatt ggtaaatgtt tttttagtga aagattttgg gatttagttt tgtttttttt 3960
   attttttttt tttaatattt gtttagttgt aaatatttcg ttgtagatat tttaatatat 4020
   agaatatatt tgattttgat atttatttcg ggatagaagt atagtaaaat gaacgtaagg 4080
   aaaaaaaggg ataaaaggaa ttagatatag ttttttgatt aagtttttta aaggttagat 4140
   gttttttttt taagttaagt gagattttga acgggagatt tgtagttttt tgtataggga 4200
   tttttaaaga gtttaggaag gtaggggatt ttatattacg tttatagtag ttagataatt 4260
   ttgaagtttc ggttgttggt tagggatatt gggaggatag taagattttg atttttgttt 4320
   ttacgaataa gatgtcggtt ttttttagta tttgtttttt ttagtgtttt tagatttttt 4380
   agtttcgggg tttttgtttg tgggagtggt agtggtcgtt agttttggag gtttagaagc 4440
   ggtgcgttta gttggttggg ttggagcgtg ttgcgttatt tgagaggttt tcgcgagcgt 4500
   tttggtgtaa gtagttttgt tttagcgagt aggaggatat gttggagtgt aggtttgtcg 4560
   aggcggcggg gttgttgttg ggcgttaggg taggcggttg tttgaggtaa ggcgaggcgt 4620
   taggcgagtt ttagtgcgtc gtagggttcg tgtagggcga gtggtattcg atggcgttcg 4680
   ttatggtcgg ggacgagggt atcgggttgt tgttgttgtt cggttcgtag tcgtcgtcgt 4740
   cgtcgttttc ggtcgtatcg acgttttcgg gtttcgcggg tatcgggtag ttggttatgt 4800
   aggtggaatt cgggttgggc gatatgtgcg ggacgtggtg gtggtggtgg tggtgagggt 4860
   gcgcgtggtt gggggcgttc gcgtcggcgt cgtagttcgc gggtattatg tcgaggtcgt 4920
   cgtagtcgtt ttggttgtgg tagtcgagcg gcgtcgacgg gggcgtttgg aagtcgaagt 4980
   tttggggtag tagcgacgtt tcgaagttta agtcgtttat tacgcggtgg tatatgggtt 5040
   tgagcgtttg gtattttcgt ttgaagtcgc gcggtcggcg gcggaacgag tttttttcga 5100
   atatgaattc gttggtcggg tcgatggttt agtagtggtt tttgtcgggt cgttcgaggt 5160
   ttttaggtag tttgatgaag tattcgttga gcgagagatt gtggcgtatc gagttttttt 5220
   agttttggta ggcgtcgcgg aagaagggga agcgcgtttg taggaattgg tagatttcgt 5280
   tgagcgttag gcgtttgttg ggcgagtttt ggatggttat gacgatgagc gcgatgtacg 5340
   agtagggcgg tttttcgggt cgtcgtagtt tcgagttcgt ttttttggcg ttttcgtttt 5400
   cggcgttcgc gtcgtcgtcg ttcgcgtttt tgtaggtagt cgagggggcg ttggtcgaat 5460
   tggaggagga cgaggacgag gcgtaggagg cggaggacga cgacgaggag gaggaggtgg 5520
   ttttcggggc ggcggcggcg gcggcggcgg cggcgggcgg cgggtttatt agggcggttt 5580
   ggagcgcgtc ggggatcggg ttgtacgcgc ggcggagcgg ggtcggcggc ggttcgtttt 5640
   cggtggttat ttgggattcg ggagcgaggt cgggtcgcga gggcgggttt cgggcgggaa 5700
   gcggaggcgt cggagcgggg tcggtttaga agttttgcga gcgttttagt tcgtcgttcg 5760
   ggtt 5764
<210> 36
   <211> 2254
   <212> DNA
   <213> Homo sapiens
<400> 36
   cgaaaacgtc cacgatcgcc agcggcccgc aaccctgcgg cggcccccag tcgccctaac 60
   aattgtcggg gagcctgcag gccctgccag aaagagagag gattggcacg ttacctttgg 120
   gcgtctggca ccccggcgtg tggctgaggg cggcaaagtc taaacgaagt tgacagatgg 180
   agtcagtatc ggccctgggg agggcggcca gggaggctcc gagcagccgc acccgcagca 240
   gcaccactcg gcccggcggg ccaggaggag gaagggggcg cggaggggcg gggctgggtc 300
   ctgggatggg acgcggggct tgctgcgggg ggcaaacaga aagaatattt catgaagtca 360
   gaggtggtcg agttttgcga ggggtggggg tcgtccccct ggcagaaccg gagtgagtct 420
   gtgatccctc aattggaaag agggtgagtg agggaaaggg gaggaggccg cgagaggaat 480
   gaagagcgag gagaccctgg agagcgggga gcgagataag agagggaggc ggccgccagg 540
   tcgggagggg gaggagtgag cgcggcggcg cggagcgagg agggggcagg gcggggtggg 600
   gggggaggga gggagggagg gagcgaggag ggcggccggg gagggcgagg gagggaggaa 660
   gtccgagaga cagagcgagg agcgctcctg aagggagacg tcgggctcgt ccccggggtc 720
   caggccgcgg tcccactcgc gggcgcagtc gcccggcgcc ggccgctcgg ccccgcggcc 780
   tgggaggccg ttgcgcaagg cagggcccgc ggctcgggga gggatgcgcc gggcgttgcc 840
   tcccgcccgc cgccgccgcc gccgccagaa gccccagagg agctgaggga ggcgacgccg 900
   aagcgctggc ccggagtggc cacggctgca gcccgggcgg cgggctaggg cgctcgcagg 960
   gcttctgggc cgaccccgct ccggcgcctc cgcttcccgc ccggggcccg ccctcgcggc 1020
   ccggcctcgc tcccgggtcc cagatgacca ccgagggcgg gccgccgccg gccccgctcc 1080
   gccgcgcgtg cagcccggtc cccggcgcgc tccaggccgc cctgatgagc ccgccgcccg 1140
   ccgccgccgc cgccgccgcc gccgccccgg agaccacctc ctcctcctcg tcgtcgtcct 1200
   ccgcctcctg cgcctcgtcc tcgtcctcct ccaattcggc cagcgccccc tcggctgcct 1260
   gcaagagcgc gggcggcggc ggcgcgggcg ccgggagcgg gggcgccaag aaggcgagct 1320
   cggggctgcg gcggcccgag aagccgccct actcgtacat cgcgctcatc gtcatggcca 1380
   tccagagctc gcccagcaag cgcctgacgc tcagcgagat ctaccagttc ctgcaggcgc 1440
   gcttcccctt cttccgcggc gcctaccagg gctggaagaa ctcggtgcgc cacaatctct 1500
   cgctcaacga gtgcttcatc aagctgccta agggcctcgg gcggcccggc aagggccact 1560
   actggaccat cgacccggcc agcgagttca tgttcgagga gggctcgttc cgccgccggc 1620
   cgcgcggctt caggcggaag tgccaggcgc tcaagcccat gtaccaccgc gtggtgagcg 1680
   gcttgggctt cggggcgtcg ctgctgcccc agggcttcga cttccaggcg cccccgtcgg 1740
   cgccgctcgg ctgccacagc cagggcggct acggcggcct cgacatgatg cccgcgggct 1800
   acgacgccgg cgcgggcgcc cccagccacg cgcaccctca ccaccaccac caccaccacg 1860
   tcccgcacat gtcgcccaac ccgggttcca cctacatggc cagctgcccg gtgcccgcgg 1920
   gacccggggg cgtcggtgcg gccgggggcg gcggcggcgg cgactacggg ccggacagca 1980
   gcagcagccc ggtaccctcg tccccggcca tggcgagcgc catcgaatgc cactcgccct 2040
   acacgagccc tgcggcgcac tggagctcgc ctggcgcctc gccttacctc aagcagccgc 2100
   ctgccctgac gcccagcagc aaccccgccg cctcggcagg cctgcactcc agcatgtcct 2160
   cctactcgct ggagcagagc tacttgcacc agaacgctcg cgaggacctc tcaggtaacg 2220
   cagcacgctc cagcccagcc agctgggcgc accg 2254
<210> 37
   <211> 2254
   <212> DNA
   <213> Artificial Sequence
<220>
   <223> C to T (bis1)
<400> 37
   cgaaaacgtt tacgatcgtt agcggttcgt aattttgcgg cggtttttag tcgttttaat      60
   aattgtcggg gagtttgtag gttttgttag aaagagagag gattggtacg ttatttttgg     120
   gcgtttggta tttcggcgtg tggttgaggg cggtaaagtt taaacgaagt tgatagatgg     180
   agttagtatc ggttttgggg agggcggtta gggaggtttc gagtagtcgt attcgtagta     240
   gtattattcg gttcggcggg ttaggaggag gaagggggcg cggaggggcg gggttgggtt     300
   ttgggatggg acgcggggtt tgttgcgggg ggtaaataga aagaatattt tatgaagtta     360
   gaggtggtcg agttttgcga ggggtggggg tcgttttttt ggtagaatcg gagtgagttt     420
   gtgatttttt aattggaaag agggtgagtg agggaaaggg gaggaggtcg cgagaggaat     480
   gaagagcgag gagattttgg agagcgggga gcgagataag agagggaggc ggtcgttagg     540
   tcgggagggg gaggagtgag cgcggcggcg cggagcgagg aggggtaggg gcggggtggg      600
   gggggaggga gggagggagg gagcgaggag ggcggtcggg gagggcgagg gagggaggaa     660
   gttcgagaga tagagcgagg agcgtttttg aagggagacg tcgggttcgt tttcggggtt     720
   taggtcgcgg ttttattcgc gggcgtagtc gttcggcgtc ggtcgttcgg tttcgcggtt     780
   tgggaggtcg ttgcgtaagg tagggttcgc ggttcgggga gggatgcgtc gggcgttgtt     840
   tttcgttcgt cgtcgtcgtc gtcgttagaa gttttagagg agttgaggga ggcgacgtcg     900
   aagcgttggt tcggagtggt tacggttgta gttcgggcgg cgggttaggg cgttcgtagg     960
   gtttttgggt cgatttcgtt tcggcgtttt cgtttttcgt tcggggttcg ttttcgcggt    1020
   tcggtttcgt tttcgggttt tagatgatta tcgagggcgg gtcgtcgtcg gtttcgtttc    1080
   gtcgcgcgtg tagttcggtt ttcggcgcgt tttaggtcgt tttgatgagt tcgtcgttcg    1140
   tcgtcgtcgt cgtcgtcgtc gtcgtttcgg agattatttt ttttttttcg tcgtcgtttt     1200
   tcgttttttg cgtttcgttt tcgttttttt ttaattcggt tagcgttttt tcggttgttt      1260
   gtaagagcgc gggcggcggc ggcgcgggcg tcgggagcgg gggcgttaag aaggcgagtt    1320
   cggggttgcg gcggttcgag aagtcgtttt attcgtatat cgcgtttatc gttatggtta    1380
   tttagagttc gtttagtaag cgtttgacgt ttagcgagat ttattagttt ttgtaggcgc 1440
   gttttttttt ttttcgcggc gtttattagg gttggaagaa ttcggtgcgt tataattttt 1500
   cgtttaacga gtgttttatt aagttgttta agggtttcgg gcggttcggt aagggttatt 1560
   attggattat cgattcggtt agcgagttta tgttcgagga gggttcgttt cgtcgtcggt 1620
   cgcgcggttt taggcggaag tgttaggcgt ttaagtttat gtattatcgc gtggtgagcg 1680
   gtttgggttt cggggcgtcg ttgttgtttt agggtttcga tttttaggcg ttttcgtcgg 1740
   cgtcgttcgg ttgttatagt tagggcggtt acggcggttt cgatatgatg ttcgcgggtt 1800
   acgacgtcgg cgcgggcgtt tttagttacg cgtattttta ttattattat tattattacg 1860
   tttcgtatat gtcgtttaat tcgggtttta tttatatggt tagttgttcg gtgttcgcgg 1920
   gattcggggg cgtcggtgcg gtcgggggcg gcggcggcgg cgattacggg tcggatagta 1980
   gtagtagttc ggtattttcg ttttcggtta tggcgagcgt tatcgaatgt tattcgtttt 2040
   atacgagttt tgcggcgtat tggagttcgt ttggcgtttc gttttatttt aagtagtcgt 2100
   ttgttttgac gtttagtagt aatttcgtcg tttcggtagg tttgtatttt agtatgtttt 2160
   tttattcgtt ggagtagagt tatttgtatt agaacgttcg cgaggatttt ttaggtaacg 2220
   tagtacgttt tagtttagtt agttgggcgt atcg 2254
<210> 38
   <211> 2254
   <212> DNA
   <213> Artificial Sequence
<220>
   <223> rc C to T (bis1)
<400> 38
   cgatacgccc aactaactaa actaaaacgt actacgttac ctaaaaaatc ctcgcgaacg 60
   ttctaataca aataactcta ctccaacgaa taaaaaaaca tactaaaata caaacctacc 120
   gaaacgacga aattactact aaacgtcaaa acaaacgact acttaaaata aaacgaaacg 180
   ccaaacgaac tccaatacgc cgcaaaactc gtataaaacg aataacattc gataacgctc 240
   gccataaccg aaaacgaaaa taccgaacta ctactactat ccgacccgta atcgccgccg 300
   ccgccgcccc cgaccgcacc gacgcccccg aatcccgcga acaccgaaca actaaccata 360
   taaataaaac ccgaattaaa cgacatatac gaaacgtaat aataataata ataataaaaa 420
   tacgcgtaac taaaaacgcc cgcgccgacg tcgtaacccg cgaacatcat atcgaaaccg 480
   ccgtaaccgc cctaactata acaaccgaac gacgccgacg aaaacgccta aaaatcgaaa 540
   ccctaaaaca acaacgacgc cccgaaaccc aaaccgctca ccacgcgata atacataaac 600
   ttaaacgcct aacacttccg cctaaaaccg cgcgaccgac gacgaaacga accctcctcg 660
   aacataaact cgctaaccga atcgataatc caataataac ccttaccgaa ccgcccgaaa 720
   cccttaaaca acttaataaa acactcgtta aacgaaaaat tataacgcac cgaattcttc 780
   caaccctaat aaacgccgcg aaaaaaaaaa aaacgcgcct acaaaaacta ataaatctcg 840
   ctaaacgtca aacgcttact aaacgaactc taaataacca taacgataaa cgcgatatac 900
   gaataaaacg acttctcgaa ccgccgcaac cccgaactcg ccttcttaac gcccccgctc 960
   ccgacgcccg cgccgccgcc gcccgcgctc ttacaaacaa ccgaaaaaac gctaaccgaa 1020
   ttaaaaaaaa acgaaaacga aacgcaaaaa acgaaaaacg acgacgaaaa aaaaaaaata 1080
   atctccgaaa cgacgacgac gacgacgacg acgacgaacg acgaactcat caaaacgacc 1140
   taaaacgcgc cgaaaaccga actacacgcg cgacgaaacg aaaccgacga cgacccgccc 1200
   tcgataatca tctaaaaccc gaaaacgaaa ccgaaccgcg aaaacgaacc ccgaacgaaa 1260
   aacgaaaacg ccgaaacgaa atcgacccaa aaaccctacg aacgccctaa cccgccgccc 1320
   gaactacaac cgtaaccact ccgaaccaac gcttcgacgt cgcctccctc aactcctcta 1380
   aaacttctaa cgacgacgac gacgacgaac gaaaaacaac gcccgacgca tccctccccg 1440
   aaccgcgaac cctaccttac gcaacgacct cccaaaccgc gaaaccgaac gaccgacgcc 1500
   gaacgactac gcccgcgaat aaaaccgcga cctaaacccc gaaaacgaac ccgacgtctc 1560
   ccttcaaaaa cgctcctcgc tctatctctc gaacttcctc cctccctcgc cctccccgac 1620
   cgccctcctc gctccctccc tccctccctc cccccccacc ccgccctacc ccctcctcgc 1680
   tccgcgccgc cgcgctcact cctccccctc ccgacctaac gaccgcctcc ctctcttatc 1740
   tcgctccccg ctctccaaaa tctcctcgct cttcattcct ctcgcgacct cctccccttt 1800
   ccctcactca ccctctttcc aattaaaaaa tcacaaactc actccgattc taccaaaaaa 1860
   acgaccccca cccctcgcaa aactcgacca cctctaactt cataaaatat tctttctatt 1920
   taccccccgc aacaaacccc gcgtcccatc ccaaaaccca accccgcccc tccgcgcccc 1980
   cttcctcctc ctaacccgcc gaaccgaata atactactac gaatacgact actcgaaacc 2040
   tccctaaccg ccctccccaa aaccgatact aactccatct atcaacttcg tttaaacttt 2100
   accgccctca accacacgcc gaaataccaa acgcccaaaa ataacgtacc aatcctctct 2160
   ctttctaaca aaacctacaa actccccgac aattattaaa acgactaaaa accgccgcaa 2220
   aattacgaac cgctaacgat cgtaaacgtt ttcg 2254
<210> 39
   <211> 2254
   <212> DNA
   <213> Artificial Sequence
<220>
   <223> G to A (bis2 rc)
<400> 39
   cgaaaacgtc cacgatcgcc aacgacccgc aaccctacga cgacccccaa tcgccctaac 60
   aattatcgaa aaacctacaa accctaccaa aaaaaaaaaa aattaacacg ttacctttaa 120
   acgtctaaca ccccgacgta taactaaaaa cgacaaaatc taaacgaaat taacaaataa 180
   aatcaatatc gaccctaaaa aaaacgacca aaaaaactcc gaacaaccgc acccgcaaca 240
   acaccactcg acccgacgaa ccaaaaaaaa aaaaaaaacg cgaaaaaacg aaactaaatc 300
   ctaaaataaa acgcgaaact tactacgaaa aacaaacaaa aaaaatattt cataaaatca 360
   aaaataatcg aattttacga aaaataaaaa tcgtccccct aacaaaaccg aaataaatct 420
   ataatccctc aattaaaaaa aaaataaata aaaaaaaaaa aaaaaaaccg cgaaaaaaat 480
   aaaaaacgaa aaaaccctaa aaaacgaaaa acgaaataaa aaaaaaaaac gaccgccaaa 540
   tcgaaaaaaa aaaaaataaa cgcgacgacg cgaaacgaaa aaaaaacaaa acgaaataaa 600
   aaaaaaaaaa aaaaaaaaaa aaacgaaaaa aacgaccgaa aaaaacgaaa aaaaaaaaaa 660
   atccgaaaaa caaaacgaaa aacgctccta aaaaaaaacg tcgaactcgt ccccgaaatc 720
   caaaccgcga tcccactcgc gaacgcaatc gcccgacgcc gaccgctcga ccccgcgacc 780
   taaaaaaccg ttacgcaaaa caaaacccgc gactcgaaaa aaaatacgcc gaacgttacc 840
   tcccgcccgc cgccgccgcc gccgccaaaa accccaaaaa aactaaaaaa aacgacgccg 900
   aaacgctaac ccgaaataac cacgactaca acccgaacga cgaactaaaa cgctcgcaaa 960
   acttctaaac cgaccccgct ccgacgcctc cgcttcccgc ccgaaacccg ccctcgcgac 1020
   ccgacctcgc tcccgaatcc caaataacca ccgaaaacga accgccgccg accccgctcc 1080
   gccgcgcgta caacccgatc cccgacgcgc tccaaaccgc cctaataaac ccgccgcccg 1140
   ccgccgccgc cgccgccgcc gccgccccga aaaccacctc ctcctcctcg tcgtcgtcct 1200
   ccgcctccta cgcctcgtcc tcgtcctcct ccaattcgac caacgccccc tcgactacct 1260
   acaaaaacgc gaacgacgac gacgcgaacg ccgaaaacga aaacgccaaa aaaacgaact 1320
   cgaaactacg acgacccgaa aaaccgccct actcgtacat cgcgctcatc gtcataacca 1380
   tccaaaactc gcccaacaaa cgcctaacgc tcaacgaaat ctaccaattc ctacaaacgc 1440
   gcttcccctt cttccgcgac gcctaccaaa actaaaaaaa ctcgatacgc cacaatctct 1500
   cgctcaacga atacttcatc aaactaccta aaaacctcga acgacccgac aaaaaccact 1560
   actaaaccat cgacccgacc aacgaattca tattcgaaaa aaactcgttc cgccgccgac 1620
   cgcgcgactt caaacgaaaa taccaaacgc tcaaacccat ataccaccgc gtaataaacg 1680
   acttaaactt cgaaacgtcg ctactacccc aaaacttcga cttccaaacg cccccgtcga 1740
   cgccgctcga ctaccacaac caaaacgact acgacgacct cgacataata cccgcgaact 1800
   acgacgccga cgcgaacgcc cccaaccacg cgcaccctca ccaccaccac caccaccacg 1860
   tcccgcacat atcgcccaac ccgaattcca cctacataac caactacccg atacccgcga 1920
   aacccgaaaa cgtcgatacg accgaaaacg acgacgacga cgactacgaa ccgaacaaca 1980
   acaacaaccc gataccctcg tccccgacca taacgaacgc catcgaatac cactcgccct 2040
   acacgaaccc tacgacgcac taaaactcgc ctaacgcctc gccttacctc aaacaaccgc 2100
   ctaccctaac gcccaacaac aaccccgccg cctcgacaaa cctacactcc aacatatcct 2160
   cctactcgct aaaacaaaac tacttacacc aaaacgctcg cgaaaacctc tcaaataacg 2220
   caacacgctc caacccaacc aactaaacgc accg 2254
<210> 40
   <211> 2254
   <212> DNA
   <213> Artificial Sequence
<220>
   <223> G to A (bis2 rc) rc
<400> 40
   cggtgcgttt agttggttgg gttggagcgt gttgcgttat ttgagaggtt ttcgcgagcg 60
   ttttggtgta agtagttttg ttttagcgag taggaggata tgttggagtg taggtttgtc 120
   gaggcggcgg ggttgttgtt gggcgttagg gtaggcggtt gtttgaggta aggcgaggcg 180
   ttaggcgagt tttagtgcgt cgtagggttc gtgtagggcg agtggtattc gatggcgttc 240
   gttatggtcg gggacgaggg tatcgggttg ttgttgttgt tcggttcgta gtcgtcgtcg 300
   tcgtcgtttt cggtcgtatc gacgttttcg ggtttcgcgg gtatcgggta gttggttatg 360
   taggtggaat tcgggttggg cgatatgtgc gggacgtggt ggtggtggtg gtggtgaggg 420
   tgcgcgtggt tgggggcgtt cgcgtcggcg tcgtagttcg cgggtattat gtcgaggtcg 480
   tcgtagtcgt tttggttgtg gtagtcgagc ggcgtcgacg ggggcgtttg gaagtcgaag 540
   ttttggggta gtagcgacgt ttcgaagttt aagtcgttta ttacgcggtg gtatatgggt 600
   ttgagcgttt ggtattttcg tttgaagtcg cgcggtcggc ggcggaacga gtttttttcg 660
   aatatgaatt cgttggtcgg gtcgatggtt tagtagtggt ttttgtcggg tcgttcgagg 720
   tttttaggta gtttgatgaa gtattcgttg agcgagagat tgtggcgtat cgagtttttt 780
   tagttttggt aggcgtcgcg gaagaagggg aagcgcgttt gtaggaattg gtagatttcg 840
   ttgagcgtta ggcgtttgtt gggcgagttt tggatggtta tgacgatgag cgcgatgtac 900
   gagtagggcg gtttttcggg tcgtcgtagt ttcgagttcg tttttttggc gttttcgttt 960
   tcggcgttcg cgtcgtcgtc gttcgcgttt ttgtaggtag tcgagggggc gttggtcgaa 1020
   ttggaggagg acgaggacga ggcgtaggag gcggaggacg acgacgagga ggaggaggtg 1080
   gttttcgggg cggcggcggc ggcggcggcg gcggcgggcg gcgggtttat tagggcggtt 1140
   tggagcgcgt cggggatcgg gttgtacgcg cggcggagcg gggtcggcgg cggttcgttt 1200
   tcggtggtta tttgggattc gggagcgagg tcgggtcgcg agggcgggtt tcgggcggga 1260
   agcggaggcg tcggagcggg gtcggtttag aagttttgcg agcgttttag ttcgtcgttc 1320
   gggttgtagt cgtggttatt tcgggttagc gtttcggcgt cgtttttttt agtttttttg 1380
   gggtttttgg cggcggcggc ggcggcgggc gggaggtaac gttcggcgta ttttttttcg 1440
   agtcgcgggt tttgttttgc gtaacggttt tttaggtcgc ggggtcgagc ggtcggcgtc 1500
   gggcgattgc gttcgcgagt gggatcgcgg tttggatttc ggggacgagt tcgacgtttt 1560
   tttttaggag cgtttttcgt tttgtttttc ggattttttt tttttttcgt ttttttcggt 1620
   cgtttttttc gttttttttt tttttttttt tttttttatt tcgttttgtt tttttttcgt 1680
   ttcgcgtcgt cgcgtttatt tttttttttt tcgatttggc ggtcgttttt tttttttatt 1740
   tcgtttttcg ttttttaggg ttttttcgtt ttttattttt ttcgcggttt tttttttttt 1800
   tttttattta tttttttttt aattgaggga ttatagattt atttcggttt tgttaggggg 1860
   acgattttta tttttcgtaa aattcgatta tttttgattt tatgaaatat tttttttgtt 1920
   tgtttttcgt agtaagtttc gcgttttatt ttaggattta gtttcgtttt ttcgcgtttt 1980
   tttttttttt ttggttcgtc gggtcgagtg gtgttgttgc gggtgcggtt gttcggagtt 2040
   tttttggtcg ttttttttag ggtcgatatt gattttattt gttaatttcg tttagatttt 2100
   gtcgttttta gttatacgtc ggggtgttag acgtttaaag gtaacgtgtt aatttttttt 2160
   ttttttggta gggtttgtag gtttttcgat aattgttagg gcgattgggg gtcgtcgtag 2220
   ggttgcgggt cgttggcgat cgtggacgtt ttcg 2254
<210> 41
   <211> 1076
   <212> DNA
   <213> Homo sapiens
<400> 41
   cttctgggcc gaccccgctc cggcgcctcc gcttcccgcc cggggcccgc cctcgcggcc 60
   cggcctcgct cccgggtccc agatgaccac cgagggcggg ccgccgccgg ccccgctccg 120
   ccgcgcgtgc agcccggtcc ccggcgcgct ccaggccgcc ctgatgagcc cgccgcccgc 180
   cgccgccgcc gccgccgccg ccgccccgga gaccacctcc tcctcctcgt cgtcgtcctc 240
   cgcctcctgc gcctcgtcct cgtcctcctc caattcggcc agcgccccct cggctgcctg 300
   caagagcgcg ggcggcggcg gcgcgggcgc cgggagcggg ggcgccaaga aggcgagctc 360
   ggggctgcgg cggcccgaga agccgcccta ctcgtacatc gcgctcatcg tcatggccat 420
   ccagagctcg cccagcaagc gcctgacgct cagcgagatc taccagttcc tgcaggcgcg 480
   cttccccttc ttccgcggcg cctaccaggg ctggaagaac tcggtgcgcc acaatctctc 540
   gctcaacgag tgcttcatca agctgcctaa gggcctcggg cggcccggca agggccacta 600
   ctggaccatc gacccggcca gcgagttcat gttcgaggag ggctcgttcc gccgccggcc 660
   gcgcggcttc aggcggaagt gccaggcgct caagcccatg taccaccgcg tggtgagcgg 720
   cttgggcttc ggggcgtcgc tgctgcccca gggcttcgac ttccaggcgc ccccgtcggc 780
   gccgctcggc tgccacagcc agggcggcta cggcggcctc gacatgatgc ccgcgggcta 840
   cgacgccggc gcgggcgccc ccagccacgc gcaccctcac caccaccacc accaccacgt 900
   cccgcacatg tcgcccaacc cgggttccac ctacatggcc agctgcccgg tgcccgcggg 960
   acccgggggc gtcggtgcgg ccgggggcgg cggcggcggc gactacgggc cggacagcag 1020
   cagcagcccg gtaccctcgt ccccggccat ggcgagcgcc atcgaatgcc actcgc 1076
<210> 42
   <211> 1076
   <212> DNA
   <213> Artificial Sequence
<220>
   <223> C to T (bis1)
<400> 42
   tttttgggtc gatttcgttt cggcgttttc gtttttcgtt cggggttcgt tttcgcggtt        60
   cggtttcgtt ttcgggtttt agatgattat cgagggcggg tcgtcgtcgg tttcgtttcg       120
   tcgcgcgtgt agttcggttt tcggcgcgtt ttaggtcgtt ttgatgagtt cgtcgttcgt       180
   cgtcgtcgtc gtcgtcgtcg tcgtttcgga gattattttt tttttttcgt cgtcgttttt       240
   cgttttttgc gtttcgtttt cgtttttttt taattcggtt agcgtttttt cggttgtttg      300
   taagagcgcg ggcggcggcg gcgcgggcgt cgggagcggg ggcgttaaga aggcgagttc       360
   ggggttgcgg cggttcgaga agtcgtttta ttcgtatatc gcgtttatcg ttatggttat       420
   ttagagttcg tttagtaagc gtttgacgtt tagcgagatt tattagtttt tgtaggcgcg       480
   tttttttttt tttcgcggcg tttattaggg ttggaagaat tcggtgcgtt ataatttttc      540
   gtttaacgag tgttttatta agttgtttaa gggtttcggg cggttcggta agggttatta       600
   ttggattatc gattcggtta gcgagtttat gttcgaggag ggttcgtttc gtcgtcggtc       660
   gcgcggtttt aggcggaagt gttaggcgtt taagtttatg tattatcgcg tggtgagcgg       720
   tttgggtttc ggggcgtcgt tgttgtttta gggtttcgat ttttaggcgt tttcgtcggc       780
   gtcgttcggt tgttatagtt agggcggtta cggcggtttc gatatgatgt tcgcgggtta       840
   cgacgtcggc gcgggcgttt ttagttacgc gtatttttat tattattatt attattacgt      900
   ttcgtatatg tcgtttaatt cgggttttat ttatatggtt agttgttcgg tgttcgcggg       960
   attcgggggc gtcggtgcgg tcgggggcgg cggcggcggc gattacgggt cggatagtag      1020
   tagtagttcg gtattttcgt tttcggttat ggcgagcgtt atcgaatgtt attcgt          1076
<210> 43
   <211> 1076
   <212> DNA
   <213> Artificial Sequence
<220>
   <223> rc C to T (bis1)
<400> 43
   acgaataaca ttcgataacg ctcgccataa ccgaaaacga aaataccgaa ctactactac 60
   tatccgaccc gtaatcgccg ccgccgccgc ccccgaccgc accgacgccc ccgaatcccg 120
   cgaacaccga acaactaacc atataaataa aacccgaatt aaacgacata tacgaaacgt 180
   aataataata ataataataa aaatacgcgt aactaaaaac gcccgcgccg acgtcgtaac 240
   ccgcgaacat catatcgaaa ccgccgtaac cgccctaact ataacaaccg aacgacgccg 300
   acgaaaacgc ctaaaaatcg aaaccctaaa acaacaacga cgccccgaaa cccaaaccgc 360
   tcaccacgcg ataatacata aacttaaacg cctaacactt ccgcctaaaa ccgcgcgacc 420
   gacgacgaaa cgaaccctcc tcgaacataa actcgctaac cgaatcgata atccaataat 480
   aacccttacc gaaccgcccg aaacccttaa acaacttaat aaaacactcg ttaaacgaaa 540
   aattataacg caccgaattc ttccaaccct aataaacgcc gcgaaaaaaa aaaaaacgcg 600
   cctacaaaaa ctaataaatc tcgctaaacg tcaaacgctt actaaacgaa ctctaaataa 660
   ccataacgat aaacgcgata tacgaataaa acgacttctc gaaccgccgc aaccccgaac 720
   tcgccttctt aacgcccccg ctcccgacgc ccgcgccgcc gccgcccgcg ctcttacaaa 780
   caaccgaaaa aacgctaacc gaattaaaaa aaaacgaaaa cgaaacgcaa aaaacgaaaa 840
   acgacgacga aaaaaaaaaa ataatctccg aaacgacgac gacgacgacg acgacgacga 900
   acgacgaact catcaaaacg acctaaaacg cgccgaaaac cgaactacac gcgcgacgaa 960
   acgaaaccga cgacgacccg ccctcgataa tcatctaaaa cccgaaaacg aaaccgaacc 1020
   gcgaaaacga accccgaacg aaaaacgaaa acgccgaaac gaaatcgacc caaaaa 1076
<210> 44
   <211> 1076
   <212> DNA
   <213> Artificial Sequence
<220>
   <223> G to A (bis2 rc)
<400> 44
   cttctaaacc gaccccgctc cgacgcctcc gcttcccgcc cgaaacccgc cctcgcgacc 60
   cgacctcgct cccgaatccc aaataaccac cgaaaacgaa ccgccgccga ccccgctccg 120
   ccgcgcgtac aacccgatcc ccgacgcgct ccaaaccgcc ctaataaacc cgccgcccgc 180
   cgccgccgcc gccgccgccg ccgccccgaa aaccacctcc tcctcctcgt cgtcgtcctc 240
   cgcctcctac gcctcgtcct cgtcctcctc caattcgacc aacgccccct cgactaccta 300
   caaaaacgcg aacgacgacg acgcgaacgc cgaaaacgaa aacgccaaaa aaacgaactc 360
   gaaactacga cgacccgaaa aaccgcccta ctcgtacatc gcgctcatcg tcataaccat 420
   ccaaaactcg cccaacaaac gcctaacgct caacgaaatc taccaattcc tacaaacgcg 480
   cttccccttc ttccgcgacg cctaccaaaa ctaaaaaaac tcgatacgcc acaatctctc 540
   gctcaacgaa tacttcatca aactacctaa aaacctcgaa cgacccgaca aaaaccacta 600
   ctaaaccatc gacccgacca acgaattcat attcgaaaaa aactcgttcc gccgccgacc 660
   gcgcgacttc aaacgaaaat accaaacgct caaacccata taccaccgcg taataaacga 720
   cttaaacttc gaaacgtcgc tactacccca aaacttcgac ttccaaacgc ccccgtcgac 780
   gccgctcgac taccacaacc aaaacgacta cgacgacctc gacataatac ccgcgaacta 840
   cgacgccgac gcgaacgccc ccaaccacgc gcaccctcac caccaccacc accaccacgt 900
   cccgcacata tcgcccaacc cgaattccac ctacataacc aactacccga tacccgcgaa 960
   acccgaaaac gtcgatacga ccgaaaacga cgacgacgac gactacgaac cgaacaacaa 1020
   caacaacccg ataccctcgt ccccgaccat aacgaacgcc atcgaatacc actcgc 1076
<210> 45
   <211> 1076
   <212> DNA
   <213> Artificial Sequence
<220>
   <223> G to A (bis2 rc) rc
<400> 45
   gcgagtggta ttcgatggcg ttcgttatgg tcggggacga gggtatcggg ttgttgttgt 60
   tgttcggttc gtagtcgtcg tcgtcgtcgt tttcggtcgt atcgacgttt tcgggtttcg 120
   cgggtatcgg gtagttggtt atgtaggtgg aattcgggtt gggcgatatg tgcgggacgt 180
   ggtggtggtg gtggtggtga gggtgcgcgt ggttgggggc gttcgcgtcg gcgtcgtagt 240
   tcgcgggtat tatgtcgagg tcgtcgtagt cgttttggtt gtggtagtcg agcggcgtcg 300
   acgggggcgt ttggaagtcg aagttttggg gtagtagcga cgtttcgaag tttaagtcgt 360
   ttattacgcg gtggtatatg ggtttgagcg tttggtattt tcgtttgaag tcgcgcggtc 420
   ggcggcggaa cgagtttttt tcgaatatga attcgttggt cgggtcgatg gtttagtagt 480
   ggtttttgtc gggtcgttcg aggtttttag gtagtttgat gaagtattcg ttgagcgaga 540
   gattgtggcg tatcgagttt ttttagtttt ggtaggcgtc gcggaagaag gggaagcgcg 600
   tttgtaggaa ttggtagatt tcgttgagcg ttaggcgttt gttgggcgag ttttggatgg 660
   ttatgacgat gagcgcgatg tacgagtagg gcggtttttc gggtcgtcgt agtttcgagt 720
   tcgttttttt ggcgttttcg ttttcggcgt tcgcgtcgtc gtcgttcgcg tttttgtagg 780
   tagtcgaggg ggcgttggtc gaattggagg aggacgagga cgaggcgtag gaggcggagg 840
   acgacgacga ggaggaggag gtggttttcg gggcggcggc ggcggcggcg gcggcggcgg 900
   gcggcgggtt tattagggcg gtttggagcg cgtcggggat cgggttgtac gcgcggcgga 960
   gcggggtcgg cggcggttcg ttttcggtgg ttatttggga ttcgggagcg aggtcgggtc 1020
   gcgagggcgg gtttcgggcg ggaagcggag gcgtcggagc ggggtcggtt tagaag 1076
<210> 46
   <211> 128
   <212> DNA
   <213> Homo sapiens
<400> 46
   ctcagcgaga tctaccagtt cctgcaggcg cgcttcccct tcttccgcgg cgcctaccag 60
   ggctggaaga actcggtgcg ccacaatctc tcgctcaacg agtgcttcat caagctgcct 120
   aagggcct 128
<210> 47
   <211> 128
   <212> DNA
   <213> Artificial Sequence
<220>
   <223> C to T (bis1)
<400> 47
   tttagcgaga tttattagtt tttgtaggcg cgtttttttt tttttcgcgg cgtttattag 60
   ggttggaaga attcggtgcg ttataatttt tcgtttaacg agtgttttat taagttgttt 120
   aagggttt 128
<210> 48
   <211> 128
   <212> DNA
   <213> Artificial Sequence
<220>
   <223> rc C to T (bis1)
<400> 48
   aaacccttaa acaacttaat aaaacactcg ttaaacgaaa aattataacg caccgaattc 60
   ttccaaccct aataaacgcc gcgaaaaaaa aaaaaacgcg cctacaaaaa ctaataaatc 120
   tcgctaaa 128
<210> 49
   <211> 128
   <212> DNA
   <213> Artificial Sequence
<220>
   <223> G to A (bis2 rc)
<400> 49
   ctcaacgaaa tctaccaatt cctacaaacg cgcttcccct tcttccgcga cgcctaccaa 60
   aactaaaaaa actcgatacg ccacaatctc tcgctcaacg aatacttcat caaactacct 120
   aaaaacct 128
<210> 50
   <211> 128
   <212> DNA
   <213> Artificial Sequence
<220>
   <223> G to A (bis2 rc) rc
<400> 50
   aggtttttag gtagtttgat gaagtattcg ttgagcgaga gattgtggcg tatcgagttt 60
   ttttagtttt ggtaggcgtc gcggaagaag gggaagcgcg tttgtaggaa ttggtagatt 120
   tcgttgag 128
<210> 51
   <211> 76
   <212> DNA
   <213> Homo sapiens
<400> 51
   cctaccaggg ctggaagaac tcggtgcgcc acaatctctc gctcaacgag tgcttcatca 60
   agctgcctaa gggcct 76
<210> 52
   <211> 76
   <212> DNA
   <213> Artificial Sequence
<220>
   <223> C to T (bis1)
<400> 52
   tttattaggg ttggaagaat tcggtgcgtt ataatttttc gtttaacgag tgttttatta 60
   agttgtttaa gggttt 76
<210> 53
   <211> 76
   <212> DNA
   <213> Artificial Sequence
<220>
   <223> rc C to T (bis1)
<400> 53
   aaacccttaa acaacttaat aaaacactcg ttaaacgaaa aattataacg caccgaattc 60
   ttccaaccct aataaa 76
<210> 54
   <211> 76
   <212> DNA
   <213> Artificial Sequence
<220>
   <223> G to A (bis2 rc)
<400> 54
   cctaccaaaa ctaaaaaaac tcgatacgcc acaatctctc gctcaacgaa tacttcatca 60
   aactacctaa aaacct 76
<210> 55
   <211> 76
   <212> DNA
   <213> Artificial Sequence
<220>
   <223> G to A (bis2 rc) rc
<400> 55
   aggtttttag gtagtttgat gaagtattcg ttgagcgaga gattgtggcg tatcgagttt 60
   ttttagtttt ggtagg 76
<210> 56
   <211> 22
   <212> DNA
   <213> Artificial Sequence
<220>
   <223> Primer
<400> 56
   gtattttgag gtttttattg at 22
<210> 57
   <211> 19
   <212> DNA
   <213> Artificial Sequence
<220>
   <223> Primer
<400> 57
   atctaactcc taaccctaa 19
<210> 58
   <211> 39
   <212> DNA
   <213> Artificial Sequence
<220>
   <223> Blocker
<400> 58
   ttattgattg agtagtagtg ttttttgttg ggatttttg 39
<210> 59
   <211> 17
   <212> DNA
   <213> Artificial Sequence
<220>
   <223> Probe
<400> 59
   taacgcgcgt aacgcta 17
<210> 60
   <211> 21
   <212> DNA
   <213> Artificial Sequence
<220>
   <223> Primer
<400> 60
   tttattaggg ttggaagaat t 21
<210> 61
   <211> 22
   <212> DNA
   <213> Artificial Sequence
<220>
   <223> Primer
<400> 61
   aaacccttaa acaacttaat aa 22
<210> 62
   <211> 32
   <212> DNA
   <213> Artificial Sequence
<220>
   <223> Blocker
<400> 62
   gaatttggtg tgttataatt ttttgtttaa tg 32
<210> 63
   <211> 24
   <212> DNA
   <213> Artificial Sequence
<220>
   <223> Probe
<400> 63
   tcgttaaacg aaaaattata acgc 24